(19) Europäisches Patentamt
European Patent Office
Office européen des brevets

(11) **EP 3 249 402 A1**

(12) **EUROPEAN PATENT APPLICATION**

(43) Date of publication:
**29.11.2017 Bulletin 2017/48**

(51) Int Cl.:
***G01N 33/53*** (2006.01)

(21) Application number: **17175396.5**

(22) Date of filing: **06.10.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.10.2010 US 390999 P**

(62) Document number(s) of the earlier application(s) in accordance with Art. 76 EPC:
**11831575.3 / 2 625 524**

(71) Applicant: **Astute Medical, Inc.**
**San Diego, CA 92121 (US)**

(72) Inventors:
• **ANDERBERG, Joseph**
  **Encinitas, CA 92024 (US)**

• **GRAY, Jeff**
  **Solana Beach, CA 92075 (US)**
• **MCPHERSON, Paul**
  **Encinitas, CA 92024 (US)**
• **NAKAMURA, Kevin**
  **Cardiff by the Sea, CA 92007 (US)**
• **KAMPF, James Patrick**
  **San Diego, CA 92130 (US)**

(74) Representative: **Schiweck, Weinzierl & Koch**
**Patentanwälte Partnerschaft mbB**
**Landsberger Straße 98**
**80339 München (DE)**

Remarks:
This application was filed on 12-06-2017 as a divisional application to the application mentioned under INID code 62.

(54) **METHODS AND COMPOSITIONS FOR DIAGNOSIS AND PROGNOSIS OF RENAL INJURY AND RENAL FAILURE**

(57) The present invention relates to methods and compositions for monitoring, diagnosis, prognosis, and determination of treatment regimens in subjects suffering from or suspected of having a renal injury. In particular, the invention relates to using assays that detect Follistatin, and optionally further one or more biomarkers selected from the group consisting of Beta-nerve growth factor, Interleukin-17A, Follitropin subunit beta, Collagenase 3, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha as diagnostic and prognostic biomarker assays in renal injuries.

EP 3 249 402 A1

SEQUENCE LISTING

<110> ASTUTE MEDICAL, INC.

<120> METHODS AND COMPOSITIONS FOR DIAGNOSIS AND PROGNOSIS OF RENAL
INJURY AND RENAL FAILURE

<130> BLG14740PCTEPD1

<140> EP11831575.3
<141> 2011-10-06

<150> 61/390,999
<151> 2010-10-07

<160> 28

<170> PatentIn version 3.5

<210> 1
<211> 241
<212> PRT
<213> Homo sapiens

<400> 1
Met Ser Met Leu Phe Tyr Thr Leu Ile Thr Ala Phe Leu Ile Gly Ile
1               5                   10                  15


Gln Ala Glu Pro His Ser Glu Ser Asn Val Pro Ala Gly His Thr Ile
            20                  25                  30


Pro Gln Ala His Trp Thr Lys Leu Gln His Ser Leu Asp Thr Ala Leu
        35                  40                  45


Arg Arg Ala Arg Ser Ala Pro Ala Ala Ala Ile Ala Ala Arg Val Ala
    50                  55                  60


Gly Gln Thr Arg Asn Ile Thr Val Asp Pro Arg Leu Phe Lys Lys Arg
65                  70                  75                  80


Arg Leu Arg Ser Pro Arg Val Leu Phe Ser Thr Gln Pro Pro Arg Glu
                85                  90                  95


Ala Ala Asp Thr Gln Asp Leu Asp Phe Glu Val Gly Gly Ala Ala Pro
                100                 105                 110


Phe Asn Arg Thr His Arg Ser Lys Arg Ser Ser Ser His Pro Ile Phe
            115                 120                 125


His Arg Gly Glu Phe Ser Val Cys Asp Ser Val Ser Val Trp Val Gly
        130                 135                 140


Asp Lys Thr Thr Ala Thr Asp Ile Lys Gly Lys Glu Val Met Val Leu
145                 150                 155                 160

```
Gly Glu Val Asn Ile Asn Asn Ser Val Phe Lys Gln Tyr Phe Phe Glu
                165                 170                 175

Thr Lys Cys Arg Asp Pro Asn Pro Val Asp Ser Gly Cys Arg Gly Ile
                180                 185                 190

Asp Ser Lys His Trp Asn Ser Tyr Cys Thr Thr Thr His Thr Phe Val
                195                 200                 205

Lys Ala Leu Thr Met Asp Gly Lys Gln Ala Ala Trp Arg Phe Ile Arg
        210                 215                 220

Ile Asp Thr Ala Cys Val Cys Val Leu Ser Arg Lys Ala Val Arg Arg
225                 230                 235                 240

Ala
```

```
<210>  2
<211>  155
<212>  PRT
<213>  Homo sapiens
```

```
<400>  2
Met Thr Pro Gly Lys Thr Ser Leu Val Ser Leu Leu Leu Leu Leu Ser
1               5                   10                  15

Leu Glu Ala Ile Val Lys Ala Gly Ile Thr Ile Pro Arg Asn Pro Gly
                20                  25                  30

Cys Pro Asn Ser Glu Asp Lys Asn Phe Pro Arg Thr Val Met Val Asn
            35                  40                  45

Leu Asn Ile His Asn Arg Asn Thr Asn Thr Asn Pro Lys Arg Ser Ser
        50                  55                  60

Asp Tyr Tyr Asn Arg Ser Thr Ser Pro Trp Asn Leu His Arg Asn Glu
65                  70                  75                  80

Asp Pro Glu Arg Tyr Pro Ser Val Ile Trp Glu Ala Lys Cys Arg His
                85                  90                  95

Leu Gly Cys Ile Asn Ala Asp Gly Asn Val Asp Tyr His Met Asn Ser
            100                 105                 110

Val Pro Ile Gln Gln Glu Ile Leu Val Leu Arg Arg Glu Pro Pro His
        115                 120                 125
```

```
Cys Pro Asn Ser Phe Arg Leu Glu Lys Ile Leu Val Ser Val Gly Cys
    130                 135                 140
```

```
Thr Cys Val Thr Pro Ile Val His His Val Ala
145                 150                 155
```

```
<210> 3
<211> 129
<212> PRT
<213> Homo sapiens

<400> 3
Met Lys Thr Leu Gln Phe Phe Phe Leu Phe Cys Cys Trp Lys Ala Ile
1               5                   10                  15
```

```
Cys Cys Asn Ser Cys Glu Leu Thr Asn Ile Thr Ile Ala Ile Glu Lys
            20                  25                  30
```

```
Glu Glu Cys Arg Phe Cys Ile Ser Ile Asn Thr Thr Trp Cys Ala Gly
        35                  40                  45
```

```
Tyr Cys Tyr Thr Arg Asp Leu Val Tyr Lys Asp Pro Ala Arg Pro Lys
    50                  55                  60
```

```
Ile Gln Lys Thr Cys Thr Phe Lys Glu Leu Val Tyr Glu Thr Val Arg
65                  70                  75                  80
```

```
Val Pro Gly Cys Ala His His Ala Asp Ser Leu Tyr Thr Tyr Pro Val
            85                  90                  95
```

```
Ala Thr Gln Cys His Cys Gly Lys Cys Asp Ser Asp Ser Thr Asp Cys
            100                 105                 110
```

```
Thr Val Arg Gly Leu Gly Pro Ser Tyr Cys Ser Phe Gly Glu Met Lys
            115                 120                 125
```

```
Glu
```

```
<210> 4
<211> 471
<212> PRT
<213> Homo sapiens

<400> 4
Met His Pro Gly Val Leu Ala Ala Phe Leu Phe Leu Ser Trp Thr His
1               5                   10                  15
```

```
Cys Arg Ala Leu Pro Leu Pro Ser Gly Gly Asp Glu Asp Asp Leu Ser
            20                  25                  30
```

Glu Glu Asp Leu Gln Phe Ala Glu Arg Tyr Leu Arg Ser Tyr Tyr His
35                    40                    45

Pro Thr Asn Leu Ala Gly Ile Leu Lys Glu Asn Ala Ala Ser Ser Met
50                    55                    60

Thr Glu Arg Leu Arg Glu Met Gln Ser Phe Phe Gly Leu Glu Val Thr
65                    70                    75                    80

Gly Lys Leu Asp Asp Asn Thr Leu Asp Val Met Lys Lys Pro Arg Cys
85                    90                    95

Gly Val Pro Asp Val Gly Glu Tyr Asn Val Phe Pro Arg Thr Leu Lys
100                    105                    110

Trp Ser Lys Met Asn Leu Thr Tyr Arg Ile Val Asn Tyr Thr Pro Asp
115                    120                    125

Met Thr His Ser Glu Val Glu Lys Ala Phe Lys Lys Ala Phe Lys Val
130                    135                    140

Trp Ser Asp Val Thr Pro Leu Asn Phe Thr Arg Leu His Asp Gly Ile
145                    150                    155                    160

Ala Asp Ile Met Ile Ser Phe Gly Ile Lys Glu His Gly Asp Phe Tyr
165                    170                    175

Pro Phe Asp Gly Pro Ser Gly Leu Leu Ala His Ala Phe Pro Pro Gly
180                    185                    190

Pro Asn Tyr Gly Gly Asp Ala His Phe Asp Asp Asp Glu Thr Trp Thr
195                    200                    205

Ser Ser Ser Lys Gly Tyr Asn Leu Phe Leu Val Ala Ala His Glu Phe
210                    215                    220

Gly His Ser Leu Gly Leu Asp His Ser Lys Asp Pro Gly Ala Leu Met
225                    230                    235                    240

Phe Pro Ile Tyr Thr Tyr Thr Gly Lys Ser His Phe Met Leu Pro Asp
245                    250                    255

Asp Asp Val Gln Gly Ile Gln Ser Leu Tyr Gly Pro Gly Asp Glu Asp
260                    265                    270

Pro Asn Pro Lys His Pro Lys Thr Pro Asp Lys Cys Asp Pro Ser Leu

308

```
           275                    280                    285


    Ser Leu Asp Ala Ile Thr Ser Leu Arg Gly Glu Thr Met Ile Phe Lys
        290                    295                    300


    Asp Arg Phe Phe Trp Arg Leu His Pro Gln Gln Val Asp Ala Glu Leu
    305                    310                    315                    320


    Phe Leu Thr Lys Ser Phe Trp Pro Glu Leu Pro Asn Arg Ile Asp Ala
                    325                    330                    335


    Ala Tyr Glu His Pro Ser His Asp Leu Ile Phe Ile Phe Arg Gly Arg
                    340                    345                    350


    Lys Phe Trp Ala Leu Asn Gly Tyr Asp Ile Leu Glu Gly Tyr Pro Lys
            355                    360                    365


    Lys Ile Ser Glu Leu Gly Leu Pro Lys Glu Val Lys Lys Ile Ser Ala
        370                    375                    380


    Ala Val His Phe Glu Asp Thr Gly Lys Thr Leu Leu Phe Ser Gly Asn
    385                    390                    395                    400


    Gln Val Trp Arg Tyr Asp Asp Thr Asn His Ile Met Asp Lys Asp Tyr
                    405                    410                    415


    Pro Arg Leu Ile Glu Glu Asp Phe Pro Gly Ile Gly Asp Lys Val Asp
                    420                    425                    430


    Ala Val Tyr Glu Lys Asn Gly Tyr Ile Tyr Phe Phe Asn Gly Pro Ile
            435                    440                    445


    Gln Phe Glu Tyr Ser Ile Trp Ser Asn Arg Ile Val Arg Val Met Pro
        450                    455                    460


    Ala Asn Ser Ile Leu Trp Cys
    465                    470


    <210> 5
    <211> 344
    <212> PRT
    <213> Homo sapiens

    <400> 5
    Met Val Arg Ala Arg His Gln Pro Gly Gly Leu Cys Leu Leu Leu Leu
    1                   5                   10                   15


    Leu Leu Cys Gln Phe Met Glu Asp Arg Ser Ala Gln Ala Gly Asn Cys
                    20                   25                   30
```

Trp Leu Arg Gln Ala Lys Asn Gly Arg Cys Gln Val Leu Tyr Lys Thr
35                      40                      45

Glu Leu Ser Lys Glu Glu Cys Cys Ser Thr Gly Arg Leu Ser Thr Ser
50                      55                      60

Trp Thr Glu Glu Asp Val Asn Asp Asn Thr Leu Phe Lys Trp Met Ile
65                      70                      75                      80

Phe Asn Gly Gly Ala Pro Asn Cys Ile Pro Cys Lys Glu Thr Cys Glu
85                      90                      95

Asn Val Asp Cys Gly Pro Gly Lys Lys Cys Arg Met Asn Lys Lys Asn
100                      105                      110

Lys Pro Arg Cys Val Cys Ala Pro Asp Cys Ser Asn Ile Thr Trp Lys
115                      120                      125

Gly Pro Val Cys Gly Leu Asp Gly Lys Thr Tyr Arg Asn Glu Cys Ala
130                      135                      140

Leu Leu Lys Ala Arg Cys Lys Glu Gln Pro Glu Leu Glu Val Gln Tyr
145                      150                      155                      160

Gln Gly Arg Cys Lys Lys Thr Cys Arg Asp Val Phe Cys Pro Gly Ser
165                      170                      175

Ser Thr Cys Val Val Asp Gln Thr Asn Asn Ala Tyr Cys Val Thr Cys
180                      185                      190

Asn Arg Ile Cys Pro Glu Pro Ala Ser Ser Glu Gln Tyr Leu Cys Gly
195                      200                      205

Asn Asp Gly Val Thr Tyr Ser Ser Ala Cys His Leu Arg Lys Ala Thr
210                      215                      220

Cys Leu Leu Gly Arg Ser Ile Gly Leu Ala Tyr Glu Gly Lys Cys Ile
225                      230                      235                      240

Lys Ala Lys Ser Cys Glu Asp Ile Gln Cys Thr Gly Gly Lys Lys Cys
245                      250                      255

Leu Trp Asp Phe Lys Val Gly Arg Gly Arg Cys Ser Leu Cys Asp Glu
260                      265                      270

Leu Cys Pro Asp Ser Lys Ser Asp Glu Pro Val Cys Ala Ser Asp Asn

```
                275                      280                      285

        Ala Thr Tyr Ala Ser Glu Cys Ala Met Lys Glu Ala Ala Cys Ser Ser
            290                      295                      300

        Gly Val Leu Leu Glu Val Lys His Ser Gly Ser Cys Asn Ser Ile Ser
        305                      310                      315                      320

        Glu Asp Thr Glu Glu Glu Glu Glu Asp Glu Asp Gln Asp Tyr Ser Phe
                             325                      330                      335

        Pro Ile Ser Ser Ile Leu Glu Trp
                             340


        <210> 6
        <211> 474
        <212> PRT
        <213> Homo sapiens

        <400> 6
        Met Lys Arg Val Leu Val Leu Leu Leu Ala Val Ala Phe Gly His Ala
        1               5                       10                      15

        Leu Glu Arg Gly Arg Asp Tyr Glu Lys Asn Lys Val Cys Lys Glu Phe
                        20                      25                      30

        Ser His Leu Gly Lys Glu Asp Phe Thr Ser Leu Ser Leu Val Leu Tyr
                35                      40                      45

        Ser Arg Lys Phe Pro Ser Gly Thr Phe Glu Gln Val Ser Gln Leu Val
                50                      55                      60

        Lys Glu Val Val Ser Leu Thr Glu Ala Cys Cys Ala Glu Gly Ala Asp
        65                      70                      75                      80

        Pro Asp Cys Tyr Asp Thr Arg Thr Ser Ala Leu Ser Ala Lys Ser Cys
                        85                      90                      95

        Glu Ser Asn Ser Pro Phe Pro Val His Pro Gly Thr Ala Glu Cys Cys
                        100                     105                     110

        Thr Lys Glu Gly Leu Glu Arg Lys Leu Cys Met Ala Ala Leu Lys His
                115                     120                     125

        Gln Pro Gln Glu Phe Pro Thr Tyr Val Glu Pro Thr Asn Asp Glu Ile
                130                     135                     140

        Cys Glu Ala Phe Arg Lys Asp Pro Lys Glu Tyr Ala Asn Gln Phe Met
        145                     150                     155                     160
```

Trp Glu Tyr Ser Thr Asn Tyr Gly Gln Ala Pro Leu Ser Leu Leu Val
                165             170                 175

Ser Tyr Thr Lys Ser Tyr Leu Ser Met Val Gly Ser Cys Cys Thr Ser
                180             185                 190

Ala Ser Pro Thr Val Cys Phe Leu Lys Glu Arg Leu Gln Leu Lys His
            195                 200             205

Leu Ser Leu Leu Thr Thr Leu Ser Asn Arg Val Cys Ser Gln Tyr Ala
        210             215                 220

Ala Tyr Gly Glu Lys Lys Ser Arg Leu Ser Asn Leu Ile Lys Leu Ala
225                 230             235                 240

Gln Lys Val Pro Thr Ala Asp Leu Glu Asp Val Leu Pro Leu Ala Glu
                245             250                 255

Asp Ile Thr Asn Ile Leu Ser Lys Cys Cys Glu Ser Ala Ser Glu Asp
                260             265                 270

Cys Met Ala Lys Glu Leu Pro Glu His Thr Val Lys Leu Cys Asp Asn
            275             280                 285

Leu Ser Thr Lys Asn Ser Lys Phe Glu Asp Cys Cys Gln Glu Lys Thr
        290             295                 300

Ala Met Asp Val Phe Val Cys Thr Tyr Phe Met Pro Ala Ala Gln Leu
305                 310                 315                 320

Pro Glu Leu Pro Asp Val Glu Leu Pro Thr Asn Lys Asp Val Cys Asp
                325             330                 335

Pro Gly Asn Thr Lys Val Met Asp Lys Tyr Thr Phe Glu Leu Ser Arg
                340             345                 350

Arg Thr His Leu Pro Glu Val Phe Leu Ser Lys Val Leu Glu Pro Thr
            355             360                 365

Leu Lys Ser Leu Gly Glu Cys Cys Asp Val Glu Asp Ser Thr Thr Cys
        370             375                 380

Phe Asn Ala Lys Gly Pro Leu Leu Lys Lys Glu Leu Ser Ser Phe Ile
385             390                 395                 400

Asp Lys Gly Gln Glu Leu Cys Ala Asp Tyr Ser Glu Asn Thr Phe Thr

```
                        405                    410                    415


        Glu Tyr Lys Lys Lys Leu Ala Glu Arg Leu Lys Ala Lys Leu Pro Asp
                    420                    425                    430


        Ala Thr Pro Lys Glu Leu Ala Lys Leu Val Asn Lys Arg Ser Asp Phe
                    435                    440                    445


        Ala Ser Asn Cys Cys Ser Ile Asn Ser Pro Pro Leu Tyr Cys Asp Ser
                450                    455                    460


        Glu Ile Asp Ala Glu Leu Lys Asn Ile Leu
        465                    470


        <210> 7
        <211> 89
        <212> PRT
        <213> Homo sapiens

        <400> 7
        Met Gly Ile Leu Lys Leu Gln Val Phe Leu Ile Val Leu Ser Val Ala
        1                   5                    10                   15


        Leu Asn His Leu Lys Ala Thr Pro Ile Glu Ser His Gln Val Glu Lys
                    20                    25                    30


        Arg Lys Cys Asn Thr Ala Thr Cys Ala Thr Gln Arg Leu Ala Asn Phe
                    35                    40                    45


        Leu Val His Ser Ser Asn Asn Phe Gly Ala Ile Leu Ser Ser Thr Asn
                50                    55                    60


        Val Gly Ser Asn Thr Tyr Gly Lys Arg Asn Ala Val Glu Val Leu Lys
        65                    70                    75                    80


        Arg Glu Pro Leu Asn Tyr Leu Pro Leu
                            85


        <210> 8
        <211> 110
        <212> PRT
        <213> Homo sapiens

        <400> 8
        Met Ala Leu Trp Met Arg Leu Leu Pro Leu Leu Ala Leu Leu Ala Leu
        1                   5                    10                   15


        Trp Gly Pro Asp Pro Ala Ala Ala Phe Val Asn Gln His Leu Cys Gly
                    20                    25                    30
```

```
Ser His Leu Val Glu Ala Leu Tyr Leu Val Cys Gly Glu Arg Gly Phe
        35                  40              45

Phe Tyr Thr Pro Lys Thr Arg Arg Glu Ala Glu Asp Leu Gln Val Gly
        50                  55              60

Gln Val Glu Leu Gly Gly Gly Pro Gly Ala Gly Ser Leu Gln Pro Leu
65                  70              75                  80

Ala Leu Glu Gly Ser Leu Gln Lys Arg Gly Ile Val Glu Gln Cys Cys
                85                  90                  95

Thr Ser Ile Cys Ser Leu Tyr Gln Leu Glu Asn Tyr Cys Asn
            100                 105                 110
```

<210> 9
<211> 1231
<212> PRT
<213> Homo sapiens

<400> 9
```
Met Arg Leu Leu Ala Lys Ile Ile Cys Leu Met Leu Trp Ala Ile Cys
1               5                   10                  15

Val Ala Glu Asp Cys Asn Glu Leu Pro Pro Arg Arg Asn Thr Glu Ile
            20                  25                  30

Leu Thr Gly Ser Trp Ser Asp Gln Thr Tyr Pro Glu Gly Thr Gln Ala
            35                  40                  45

Ile Tyr Lys Cys Arg Pro Gly Tyr Arg Ser Leu Gly Asn Val Ile Met
        50                  55                  60

Val Cys Arg Lys Gly Glu Trp Val Ala Leu Asn Pro Leu Arg Lys Cys
65                  70                  75                  80

Gln Lys Arg Pro Cys Gly His Pro Gly Asp Thr Pro Phe Gly Thr Phe
                85                  90                  95

Thr Leu Thr Gly Gly Asn Val Phe Glu Tyr Gly Val Lys Ala Val Tyr
            100                 105                 110

Thr Cys Asn Glu Gly Tyr Gln Leu Leu Gly Glu Ile Asn Tyr Arg Glu
            115                 120                 125

Cys Asp Thr Asp Gly Trp Thr Asn Asp Ile Pro Ile Cys Glu Val Val
        130                 135                 140

Lys Cys Leu Pro Val Thr Ala Pro Glu Asn Gly Lys Ile Val Ser Ser
```

```
            145                    150                    155                    160


            Ala Met Glu Pro Asp Arg Glu Tyr His Phe Gly Gln Ala Val Arg Phe
                        165                170                175


            Val Cys Asn Ser Gly Tyr Lys Ile Glu Gly Asp Glu Glu Met His Cys
                    180                185                190


            Ser Asp Asp Gly Phe Trp Ser Lys Glu Lys Pro Lys Cys Val Glu Ile
                    195                200                205


            Ser Cys Lys Ser Pro Asp Val Ile Asn Gly Ser Pro Ile Ser Gln Lys
                210                215                220


            Ile Ile Tyr Lys Glu Asn Glu Arg Phe Gln Tyr Lys Cys Asn Met Gly
            225                230                235                240


            Tyr Glu Tyr Ser Glu Arg Gly Asp Ala Val Cys Thr Glu Ser Gly Trp
                        245                250                255


            Arg Pro Leu Pro Ser Cys Glu Glu Lys Ser Cys Asp Asn Pro Tyr Ile
                        260                265                270


            Pro Asn Gly Asp Tyr Ser Pro Leu Arg Ile Lys His Arg Thr Gly Asp
                    275                280                285


            Glu Ile Thr Tyr Gln Cys Arg Asn Gly Phe Tyr Pro Ala Thr Arg Gly
                290                295                300


            Asn Thr Ala Lys Cys Thr Ser Thr Gly Trp Ile Pro Ala Pro Arg Cys
            305                310                315                320


            Thr Leu Lys Pro Cys Asp Tyr Pro Asp Ile Lys His Gly Gly Leu Tyr
                        325                330                335


            His Glu Asn Met Arg Arg Pro Tyr Phe Pro Val Ala Val Gly Lys Tyr
                    340                345                350


            Tyr Ser Tyr Tyr Cys Asp Glu His Phe Glu Thr Pro Ser Gly Ser Tyr
                    355                360                365


            Trp Asp His Ile His Cys Thr Gln Asp Gly Trp Ser Pro Ala Val Pro
                370                375                380


            Cys Leu Arg Lys Cys Tyr Phe Pro Tyr Leu Glu Asn Gly Tyr Asn Gln
            385                390                395                400
```

```
Asn Tyr Gly Arg Lys Phe Val Gln Gly Lys Ser Ile Asp Val Ala Cys
            405             410             415

His Pro Gly Tyr Ala Leu Pro Lys Ala Gln Thr Thr Val Thr Cys Met
            420             425             430

Glu Asn Gly Trp Ser Pro Thr Pro Arg Cys Ile Arg Val Lys Thr Cys
            435             440             445

Ser Lys Ser Ser Ile Asp Ile Glu Asn Gly Phe Ile Ser Glu Ser Gln
            450             455             460

Tyr Thr Tyr Ala Leu Lys Glu Lys Ala Lys Tyr Gln Cys Lys Leu Gly
465             470             475             480

Tyr Val Thr Ala Asp Gly Glu Thr Ser Gly Ser Ile Thr Cys Gly Lys
            485             490             495

Asp Gly Trp Ser Ala Gln Pro Thr Cys Ile Lys Ser Cys Asp Ile Pro
            500             505             510

Val Phe Met Asn Ala Arg Thr Lys Asn Asp Phe Thr Trp Phe Lys Leu
            515             520             525

Asn Asp Thr Leu Asp Tyr Glu Cys His Asp Gly Tyr Glu Ser Asn Thr
            530             535             540

Gly Ser Thr Thr Gly Ser Ile Val Cys Gly Tyr Asn Gly Trp Ser Asp
545             550             555             560

Leu Pro Ile Cys Tyr Glu Arg Glu Cys Glu Leu Pro Lys Ile Asp Val
            565             570             575

His Leu Val Pro Asp Arg Lys Lys Asp Gln Tyr Lys Val Gly Glu Val
            580             585             590

Leu Lys Phe Ser Cys Lys Pro Gly Phe Thr Ile Val Gly Pro Asn Ser
            595             600             605

Val Gln Cys Tyr His Phe Gly Leu Ser Pro Asp Leu Pro Ile Cys Lys
            610             615             620

Glu Gln Val Gln Ser Cys Gly Pro Pro Pro Glu Leu Leu Asn Gly Asn
625             630             635             640

Val Lys Glu Lys Thr Lys Glu Glu Tyr Gly His Ser Glu Val Val Glu
            645             650             655
```

```
Tyr Tyr Cys Asn Pro Arg Phe Leu Met Lys Gly Pro Asn Lys Ile Gln
        660                 665                 670

Cys Val Asp Gly Glu Trp Thr Thr Leu Pro Val Cys Ile Val Glu Glu
        675                 680                 685

Ser Thr Cys Gly Asp Ile Pro Glu Leu Glu His Gly Trp Ala Gln Leu
        690                 695                 700

Ser Ser Pro Pro Tyr Tyr Tyr Gly Asp Ser Val Glu Phe Asn Cys Ser
705                 710                 715                 720

Glu Ser Phe Thr Met Ile Gly His Arg Ser Ile Thr Cys Ile His Gly
        725                 730                 735

Val Trp Thr Gln Leu Pro Gln Cys Val Ala Ile Asp Lys Leu Lys Lys
        740                 745                 750

Cys Lys Ser Ser Asn Leu Ile Ile Leu Glu Glu His Leu Lys Asn Lys
        755                 760                 765

Lys Glu Phe Asp His Asn Ser Asn Ile Arg Tyr Arg Cys Arg Gly Lys
        770                 775                 780

Glu Gly Trp Ile His Thr Val Cys Ile Asn Gly Arg Trp Asp Pro Glu
785                 790                 795                 800

Val Asn Cys Ser Met Ala Gln Ile Gln Leu Cys Pro Pro Pro Gln
        805                 810                 815

Ile Pro Asn Ser His Asn Met Thr Thr Thr Leu Asn Tyr Arg Asp Gly
        820                 825                 830

Glu Lys Val Ser Val Leu Cys Gln Glu Asn Tyr Leu Ile Gln Glu Gly
        835                 840                 845

Glu Glu Ile Thr Cys Lys Asp Gly Arg Trp Gln Ser Ile Pro Leu Cys
        850                 855                 860

Val Glu Lys Ile Pro Cys Ser Gln Pro Pro Gln Ile Glu His Gly Thr
865                 870                 875                 880

Ile Asn Ser Ser Arg Ser Ser Gln Glu Ser Tyr Ala His Gly Thr Lys
        885                 890                 895

Leu Ser Tyr Thr Cys Glu Gly Gly Phe Arg Ile Ser Glu Glu Asn Glu
        900                 905                 910
```

Thr Thr Cys Tyr Met Gly Lys Trp Ser Ser Pro Pro Gln Cys Glu Gly
    915          920          925

Leu Pro Cys Lys Ser Pro Pro Glu Ile Ser His Gly Val Val Ala His
    930          935          940

Met Ser Asp Ser Tyr Gln Tyr Gly Glu Glu Val Thr Tyr Lys Cys Phe
945          950          955          960

Glu Gly Phe Gly Ile Asp Gly Pro Ala Ile Ala Lys Cys Leu Gly Glu
    965          970          975

Lys Trp Ser His Pro Pro Ser Cys Ile Lys Thr Asp Cys Leu Ser Leu
    980          985          990

Pro Ser Phe Glu Asn Ala Ile Pro Met Gly Glu Lys Lys Asp Val Tyr
    995        1000       1005

Lys Ala Gly Glu Gln Val Thr Tyr Thr Cys Ala Thr Tyr Tyr Lys
    1010       1015       1020

Met Asp Gly Ala Ser Asn Val Thr Cys Ile Asn Ser Arg Trp Thr
    1025       1030       1035

Gly Arg Pro Thr Cys Arg Asp Thr Ser Cys Val Asn Pro Pro Thr
    1040       1045       1050

Val Gln Asn Ala Tyr Ile Val Ser Arg Gln Met Ser Lys Tyr Pro
    1055       1060       1065

Ser Gly Glu Arg Val Arg Tyr Gln Cys Arg Ser Pro Tyr Glu Met
    1070       1075       1080

Phe Gly Asp Glu Glu Val Met Cys Leu Asn Gly Asn Trp Thr Glu
    1085       1090       1095

Pro Pro Gln Cys Lys Asp Ser Thr Gly Lys Cys Gly Pro Pro Pro
    1100       1105       1110

Pro Ile Asp Asn Gly Asp Ile Thr Ser Phe Pro Leu Ser Val Tyr
    1115       1120       1125

Ala Pro Ala Ser Ser Val Glu Tyr Gln Cys Gln Asn Leu Tyr Gln
    1130       1135       1140

Leu Glu Gly Asn Lys Arg Ile Thr Cys Arg Asn Gly Gln Trp Ser

```
              1145                      1150                      1155


         Glu Pro  Pro Lys Cys Leu His  Pro Cys Val Ile Ser  Arg Glu Ile
              1160                 1165                 1170


         Met Glu  Asn Tyr Asn Ile Ala  Leu Arg Trp Thr Ala  Lys Gln Lys
              1175                 1180                 1185


         Leu Tyr  Ser Arg Thr Gly Glu  Ser Val Glu Phe Val  Cys Lys Arg
              1190                 1195                 1200


         Gly Tyr  Arg Leu Ser Ser Arg  Ser His Thr Leu Arg  Thr Thr Cys
              1205                 1210                 1215


         Trp Asp  Gly Lys Leu Glu Tyr  Pro Thr Cys Ala Lys  Arg
              1220                 1225                 1230


         <210> 10
         <211> 153
         <212> PRT
         <213> Homo sapiens

         <400> 10
         Met Val Ala Thr Lys Thr Phe Ala Leu Leu Leu Leu Ser Leu Phe Leu
         1                   5                   10                  15


         Ala Val Gly Leu Gly Glu Lys Lys Glu Gly His Phe Ser Ala Leu Pro
                     20                  25                  30


         Ser Leu Pro Val Gly Ser His Ala Lys Val Ser Ser Pro Gln Pro Arg
                 35                  40                  45


         Gly Pro Arg Tyr Ala Glu Gly Thr Phe Ile Ser Asp Tyr Ser Ile Ala
             50                  55                  60


         Met Asp Lys Ile His Gln Gln Asp Phe Val Asn Trp Leu Leu Ala Gln
         65                  70                  75                  80


         Lys Gly Lys Lys Asn Asp Trp Lys His Asn Ile Thr Gln Arg Glu Ala
                         85                  90                  95


         Arg Ala Leu Glu Leu Ala Ser Gln Ala Asn Arg Lys Glu Glu Glu Ala
                     100                 105                 110


         Val Glu Pro Gln Ser Ser Pro Ala Lys Asn Pro Ser Asp Glu Asp Leu
                 115                 120                 125


         Leu Arg Asp Leu Leu Ile Gln Glu Leu Leu Ala Cys Leu Leu Asp Gln
             130                 135                 140
```

319

Thr Asn Leu Cys Arg Leu Arg Ser Arg
145             150

<210> 11
<211> 4
<212> PRT
<213> Homo sapiens

<400> 11
Lys Thr Cys Ser
1

<210> 12
<211> 180
<212> PRT
<213> Homo sapiens

<400> 12
Met Lys Ser Ile Tyr Phe Val Ala Gly Leu Phe Val Met Leu Val Gln
1               5                   10                  15

Gly Ser Trp Gln Arg Ser Leu Gln Asp Thr Glu Glu Lys Ser Arg Ser
            20                  25                  30

Phe Ser Ala Ser Gln Ala Asp Pro Leu Ser Asp Pro Asp Gln Met Asn
        35                  40                  45

Glu Asp Lys Arg His Ser Gln Gly Thr Phe Thr Ser Asp Tyr Ser Lys
        50                  55                  60

Tyr Leu Asp Ser Arg Arg Ala Gln Asp Phe Val Gln Trp Leu Met Asn
65                  70                  75                  80

Thr Lys Arg Asn Arg Asn Asn Ile Ala Lys Arg His Asp Glu Phe Glu
                85                  90                  95

Arg His Ala Glu Gly Thr Phe Thr Ser Asp Val Ser Ser Tyr Leu Glu
            100                 105                 110

Gly Gln Ala Ala Lys Glu Phe Ile Ala Trp Leu Val Lys Gly Arg Gly
        115                 120                 125

Arg Arg Asp Phe Pro Glu Glu Val Ala Ile Val Glu Glu Leu Gly Arg
        130                 135                 140

Arg His Ala Asp Gly Ser Phe Ser Asp Glu Met Asn Thr Ile Leu Asp
145                 150                 155                 160

Asn Leu Ala Ala Arg Asp Phe Ile Asn Trp Leu Ile Gln Thr Lys Ile

Thr Asp Arg Lys
180

<210> 13
<211> 585
<212> PRT
<213> Homo sapiens

<400> 13
Met Ser Gln Gln His Thr Leu Pro Val Thr Leu Ser Pro Ala Leu Ser
1               5               10              15

Gln Glu Leu Leu Lys Thr Val Pro Pro Pro Val Asn Thr His Gln Glu
            20              25              30

Gln Met Lys Gln Pro Thr Pro Leu Pro Pro Pro Cys Gln Lys Val Pro
        35              40              45

Val Glu Leu Pro Val Glu Val Pro Ser Lys Gln Glu Glu Lys His Met
        50              55              60

Thr Ala Val Lys Gly Leu Pro Glu Gln Glu Cys Glu Gln Gln Gln Lys
65              70              75              80

Glu Pro Gln Glu Gln Glu Leu Gln Gln Gln His Trp Glu Gln His Glu
            85              90              95

Glu Tyr Gln Lys Ala Glu Asn Pro Glu Gln Gln Leu Lys Gln Glu Lys
            100             105             110

Thr Gln Arg Asp Gln Gln Leu Asn Lys Gln Leu Glu Glu Glu Lys Lys
        115             120             125

Leu Leu Asp Gln Gln Leu Asp Gln Glu Leu Val Lys Arg Asp Glu Gln
        130             135             140

Leu Gly Met Lys Lys Glu Gln Leu Leu Glu Leu Pro Glu Gln Gln Glu
145             150             155             160

Gly His Leu Lys His Leu Glu Gln Gln Glu Gly Gln Leu Lys His Pro
            165             170             175

Glu Gln Gln Glu Gly Gln Leu Glu Leu Pro Glu Gln Gln Glu Gly Gln
        180             185             190

Leu Glu Leu Pro Glu Gln Gln Glu Gly Gln Leu Glu Leu Pro Glu Gln
        195             200             205

Gln Glu Gly Gln Leu Glu Leu Pro Glu Gln Gln Glu Gly Gln Leu Glu
210                215                220

Leu Pro Glu Gln Gln Glu Gly Gln Leu Glu Leu Pro Gln Gln Gln Glu
225                230                235                240

Gly Gln Leu Glu Leu Ser Glu Gln Gln Glu Gly Gln Leu Glu Leu Ser
245                250                255

Glu Gln Gln Glu Gly Gln Leu Lys His Leu Glu His Gln Glu Gly Gln
260                265                270

Leu Glu Val Pro Glu Glu Gln Met Gly Gln Leu Lys Tyr Leu Glu Gln
275                280                285

Gln Glu Gly Gln Leu Lys His Leu Asp Gln Gln Glu Lys Gln Pro Glu
290                295                300

Leu Pro Glu Gln Gln Met Gly Gln Leu Lys His Leu Glu Gln Gln Glu
305                310                315                320

Gly Gln Pro Lys His Leu Glu Gln Gln Glu Gly Gln Leu Glu Gln Leu
325                330                335

Glu Glu Gln Glu Gly Gln Leu Lys His Leu Glu Gln Gln Glu Gly Gln
340                345                350

Leu Glu His Leu Glu His Gln Glu Gly Gln Leu Gly Leu Pro Glu Gln
355                360                365

Gln Val Leu Gln Leu Lys Gln Leu Glu Lys Gln Gln Gly Gln Pro Lys
370                375                380

His Leu Glu Glu Glu Glu Gly Gln Leu Lys His Leu Val Gln Gln Glu
385                390                395                400

Gly Gln Leu Lys His Leu Val Gln Gln Glu Gly Gln Leu Glu Gln Gln
405                410                415

Glu Arg Gln Val Glu His Leu Glu Gln Gln Val Gly Gln Leu Lys His
420                425                430

Leu Glu Glu Gln Glu Gly Gln Leu Lys His Leu Glu Gln Gln Gln Gly
435                440                445

Gln Leu Glu Val Pro Glu Gln Gln Val Gly Gln Pro Lys Asn Leu Glu

```
                450                      455                       460

      Gln Glu Glu Lys Gln Leu Glu Leu Pro Glu Gln Gln Glu Gly Gln Val
      465                 470                 475                 480


      Lys His Leu Glu Lys Gln Glu Ala Gln Leu Glu Leu Pro Glu Gln Gln
                      485                 490                 495


      Val Gly Gln Pro Lys His Leu Glu Gln Gln Glu Lys His Leu Glu His
                      500                 505                 510


      Pro Glu Gln Gln Asp Gly Gln Leu Lys His Leu Glu Gln Gln Glu Gly
                  515                 520                 525


      Gln Leu Lys Asp Leu Glu Gln Gln Lys Gly Gln Leu Glu Gln Pro Val
              530                 535                 540


      Phe Ala Pro Ala Pro Gly Gln Val Gln Asp Ile Gln Pro Ala Leu Pro
      545                 550                 555                 560


      Thr Lys Gly Glu Val Leu Leu Pro Val Glu His Gln Gln Gln Lys Gln
                      565                 570                 575


      Glu Val Gln Trp Pro Pro Lys His Lys
                      580                 585


      <210> 14
      <211> 644
      <212> PRT
      <213> Homo sapiens

      <400> 14
      Met Ser Arg Gln Phe Ser Ser Arg Ser Gly Tyr Arg Ser Gly Gly Gly
      1               5                   10                  15


      Phe Ser Ser Gly Ser Ala Gly Ile Ile Asn Tyr Gln Arg Arg Thr Thr
                      20                  25                  30


      Ser Ser Ser Thr Arg Arg Ser Gly Gly Gly Gly Gly Arg Phe Ser Ser
                  35                  40                  45


      Cys Gly Gly Gly Gly Gly Ser Phe Gly Ala Gly Gly Gly Phe Gly Ser
          50                  55                  60


      Arg Ser Leu Val Asn Leu Gly Gly Ser Lys Ser Ile Ser Ile Ser Val
      65                  70                  75                  80


      Ala Arg Gly Gly Gly Arg Gly Ser Gly Phe Gly Gly Gly Tyr Gly Gly
                      85                  90                  95
```

Gly Gly Phe Gly Gly Gly Gly Phe Gly Gly Gly Gly Phe Gly Gly Gly
                100                 105                 110

Gly Ile Gly Gly Gly Gly Phe Gly Gly Phe Gly Ser Gly Gly Gly Gly
                115                 120                 125

Phe Gly Gly Gly Gly Phe Gly Gly Gly Gly Tyr Gly Gly Gly Tyr Gly
                130                 135                 140

Pro Val Cys Pro Pro Gly Gly Ile Gln Glu Val Thr Ile Asn Gln Ser
145                 150                 155                 160

Leu Leu Gln Pro Leu Asn Val Glu Ile Asp Pro Glu Ile Gln Lys Val
                165                 170                 175

Lys Ser Arg Glu Arg Glu Gln Ile Lys Ser Leu Asn Asn Gln Phe Ala
                180                 185                 190

Ser Phe Ile Asp Lys Val Arg Phe Leu Glu Gln Gln Asn Gln Val Leu
                195                 200                 205

Gln Thr Lys Trp Glu Leu Leu Gln Gln Val Asp Thr Ser Thr Arg Thr
    210                 215                 220

His Asn Leu Glu Pro Tyr Phe Glu Ser Phe Ile Asn Asn Leu Arg Arg
225                 230                 235                 240

Arg Val Asp Gln Leu Lys Ser Asp Gln Ser Arg Leu Asp Ser Glu Leu
                245                 250                 255

Lys Asn Met Gln Asp Met Val Glu Asp Tyr Arg Asn Lys Tyr Glu Asp
                260                 265                 270

Glu Ile Asn Lys Arg Thr Asn Ala Glu Asn Glu Phe Val Thr Ile Lys
                275                 280                 285

Lys Asp Val Asp Gly Ala Tyr Met Thr Lys Val Asp Leu Gln Ala Lys
                290                 295                 300

Leu Asp Asn Leu Gln Gln Glu Ile Asp Phe Leu Thr Ala Leu Tyr Gln
305                 310                 315                 320

Ala Glu Leu Ser Gln Met Gln Thr Gln Ile Ser Glu Thr Asn Val Ile
                325                 330                 335

Leu Ser Met Asp Asn Asn Arg Ser Leu Asp Leu Asp Ser Ile Ile Ala

324

340          345          350

Glu Val Lys Ala Gln Tyr Glu Asp Ile Ala Gln Lys Ser Lys Ala Glu
     355             360             365

Ala Glu Ser Leu Tyr Gln Ser Lys Tyr Glu Glu Leu Gln Ile Thr Ala
   370             375             380

Gly Arg His Gly Asp Ser Val Arg Asn Ser Lys Ile Glu Ile Ser Glu
385              390          395             400

Leu Asn Arg Val Ile Gln Arg Leu Arg Ser Glu Ile Asp Asn Val Lys
         405             410             415

Lys Gln Ile Ser Asn Leu Gln Gln Ser Ile Ser Asp Ala Glu Gln Arg
         420             425             430

Gly Glu Asn Ala Leu Lys Asp Ala Lys Asn Lys Leu Asn Asp Leu Glu
     435             440             445

Asp Ala Leu Gln Gln Ala Lys Glu Asp Leu Ala Arg Leu Leu Arg Asp
    450              455             460

Tyr Gln Glu Leu Met Asn Thr Lys Leu Ala Leu Asp Leu Glu Ile Ala
465              470          475             480

Thr Tyr Arg Thr Leu Leu Glu Gly Glu Glu Ser Arg Met Ser Gly Glu
         485             490             495

Cys Ala Pro Asn Val Ser Val Ser Val Ser Thr Ser His Thr Thr Ile
         500             505             510

Ser Gly Gly Gly Ser Arg Gly Gly Gly Gly Gly Tyr Gly Ser Gly
     515             520             525

Gly Ser Ser Tyr Gly Ser Gly Gly Gly Ser Tyr Gly Ser Gly Gly Gly
     530             535             540

Gly Gly Gly Gly Arg Gly Ser Tyr Gly Ser Gly Gly Ser Ser Tyr Gly
545              550          555             560

Ser Gly Gly Gly Ser Tyr Gly Ser Gly Gly Gly Gly Gly His Gly
         565             570             575

Ser Tyr Gly Ser Gly Ser Ser Ser Gly Gly Tyr Arg Gly Gly Ser Gly
     580             585             590

```
Gly Gly Gly Gly Gly Ser Ser Gly Gly Arg Gly Ser Gly Gly Gly Ser
        595                 600             605

Ser Gly Gly Ser Ile Gly Gly Arg Gly Ser Ser Ser Gly Gly Val Lys
        610                 615             620

Ser Ser Gly Gly Ser Ser Ser Val Lys Phe Val Ser Thr Thr Tyr Ser
625             630             635             640

Gly Val Thr Arg
```

```
<210> 15
<211> 584
<212> PRT
<213> Homo sapiens

<400> 15
Met Ser Val Arg Tyr Ser Ser Ser Lys His Tyr Ser Ser Ser Arg Ser
1           5               10              15

Gly Gly Gly Gly Gly Gly Gly Gly Cys Gly Gly Gly Gly Gly Val Ser
            20              25              30

Ser Leu Arg Ile Ser Ser Ser Lys Gly Ser Leu Gly Gly Gly Phe Ser
        35              40              45

Ser Gly Gly Phe Ser Gly Gly Ser Phe Ser Arg Gly Ser Ser Gly Gly
        50              55              60

Gly Cys Phe Gly Gly Ser Ser Gly Gly Tyr Gly Gly Leu Gly Gly Phe
65              70              75              80

Gly Gly Gly Ser Phe Arg Gly Ser Tyr Gly Ser Ser Ser Phe Gly Gly
            85              90              95

Ser Tyr Gly Gly Ile Phe Gly Gly Gly Ser Phe Gly Gly Gly Ser Phe
            100             105             110

Gly Gly Gly Ser Phe Gly Gly Gly Gly Phe Gly Gly Gly Gly Phe Gly
        115             120             125

Gly Gly Phe Gly Gly Gly Phe Gly Gly Asp Gly Gly Leu Leu Ser Gly
        130             135             140

Asn Glu Lys Val Thr Met Gln Asn Leu Asn Asp Arg Leu Ala Ser Tyr
145             150             155             160

Leu Asp Lys Val Arg Ala Leu Glu Glu Ser Asn Tyr Glu Leu Glu Gly
```

165           170           175

Lys Ile Lys Glu Trp Tyr Glu Lys His Gly Asn Ser His Gln Gly Glu
180          185          190

Pro Arg Asp Tyr Ser Lys Tyr Tyr Lys Thr Ile Asp Asp Leu Lys Asn
195          200          205

Gln Ile Leu Asn Leu Thr Thr Asp Asn Ala Asn Ile Leu Leu Gln Ile
210          215          220

Asp Asn Ala Arg Leu Ala Ala Asp Asp Phe Arg Leu Lys Tyr Glu Asn
225          230          235          240

Glu Val Ala Leu Arg Gln Ser Val Glu Ala Asp Ile Asn Gly Leu Arg
245          250          255

Arg Val Leu Asp Glu Leu Thr Leu Thr Lys Ala Asp Leu Glu Met Gln
260          265          270

Ile Glu Ser Leu Thr Glu Glu Leu Ala Tyr Leu Lys Lys Asn His Glu
275          280          285

Glu Glu Met Lys Asp Leu Arg Asn Val Ser Thr Gly Asp Val Asn Val
290          295          300

Glu Met Asn Ala Ala Pro Gly Val Asp Leu Thr Gln Leu Leu Asn Asn
305          310          315          320

Met Arg Ser Gln Tyr Glu Gln Leu Ala Glu Gln Asn Arg Lys Asp Ala
325          330          335

Glu Ala Trp Phe Asn Glu Lys Ser Lys Glu Leu Thr Thr Glu Ile Asp
340          345          350

Asn Asn Ile Glu Gln Ile Ser Ser Tyr Lys Ser Glu Ile Thr Glu Leu
355          360          365

Arg Arg Asn Val Gln Ala Leu Glu Ile Glu Leu Gln Ser Gln Leu Ala
370          375          380

Leu Lys Gln Ser Leu Glu Ala Ser Leu Ala Glu Thr Glu Gly Arg Tyr
385          390          395          400

Cys Val Gln Leu Ser Gln Ile Gln Ala Gln Ile Ser Ala Leu Glu Glu
405          410          415

Gln Leu Gln Gln Ile Arg Ala Glu Thr Glu Cys Gln Asn Thr Glu Tyr
420 425 430

Gln Gln Leu Leu Asp Ile Lys Ile Arg Leu Glu Asn Glu Ile Gln Thr
435 440 445

Tyr Arg Ser Leu Leu Glu Gly Glu Gly Ser Ser Gly Gly Gly Gly Arg
450 455 460

Gly Gly Gly Ser Phe Gly Gly Gly Tyr Gly Gly Gly Ser Ser Gly Gly
465 470 475 480

Gly Ser Ser Gly Gly Gly His Gly Gly Gly His Gly Gly Ser Ser Gly
485 490 495

Gly Gly Tyr Gly Gly Gly Ser Ser Gly Gly Gly Ser Ser Gly Gly Gly
500 505 510

Tyr Gly Gly Gly Ser Ser Ser Gly Gly His Gly Gly Ser Ser Ser Gly
515 520 525

Gly Tyr Gly Gly Gly Ser Ser Gly Gly Gly Gly Gly Gly Tyr Gly Gly
530 535 540

Gly Ser Ser Gly Gly Gly Ser Ser Ser Gly Gly Gly Tyr Gly Gly Gly
545 550 555 560

Ser Ser Ser Gly Gly His Lys Ser Ser Ser Ser Gly Ser Val Gly Glu
565 570 575

Ser Ser Ser Lys Gly Pro Arg Tyr
580

```
<210> 16
<211> 564
<212> PRT
<213> Homo sapiens

<400> 16
```

Met Ala Ser Thr Ser Thr Thr Ile Arg Ser His Ser Ser Ser Arg Arg
1 5 10 15

Gly Phe Ser Ala Asn Ser Ala Arg Leu Pro Gly Val Ser Arg Ser Gly
20 25 30

Phe Ser Ser Val Ser Val Ser Arg Ser Arg Gly Ser Gly Gly Leu Gly
35 40 45

Gly Ala Cys Gly Gly Ala Gly Phe Gly Ser Arg Ser Leu Tyr Gly Leu

                50                          55                          60

Gly Gly Ser Lys Arg Ile Ser Ile Gly Gly Gly Ser Cys Ala Ile Ser
65              70              75              80

Gly Gly Tyr Gly Ser Arg Ala Gly Gly Ser Tyr Gly Phe Gly Gly Ala
        85              90              95

Gly Ser Gly Phe Gly Phe Gly Gly Gly Ala Gly Ile Gly Phe Gly Leu
        100             105             110

Gly Gly Gly Ala Gly Leu Ala Gly Gly Phe Gly Gly Pro Gly Phe Pro
        115             120             125

Val Cys Pro Pro Gly Gly Ile Gln Glu Val Thr Val Asn Gln Ser Leu
    130             135             140

Leu Thr Pro Leu Asn Leu Gln Ile Asp Pro Thr Ile Gln Arg Val Arg
145             150             155             160

Ala Glu Glu Arg Glu Gln Ile Lys Thr Leu Asn Asn Lys Phe Ala Ser
            165             170             175

Phe Ile Asp Lys Val Arg Phe Leu Glu Gln Gln Asn Lys Val Leu Glu
        180             185             190

Thr Lys Trp Thr Leu Leu Gln Glu Gln Gly Thr Lys Thr Val Arg Gln
        195             200             205

Asn Leu Glu Pro Leu Phe Glu Gln Tyr Ile Asn Asn Leu Arg Arg Gln
    210             215             220

Leu Asp Ser Ile Val Gly Glu Arg Gly Arg Leu Asp Ser Glu Leu Arg
225             230             235             240

Gly Met Gln Asp Leu Val Glu Asp Phe Lys Asn Lys Tyr Glu Asp Glu
        245             250             255

Ile Asn Lys Arg Thr Ala Ala Glu Asn Glu Phe Val Thr Leu Lys Lys
        260             265             270

Asp Val Asp Ala Ala Tyr Met Asn Lys Val Glu Leu Gln Ala Lys Ala
        275             280             285

Asp Thr Leu Thr Asp Glu Ile Asn Phe Leu Arg Ala Leu Tyr Asp Ala
    290             295             300

Glu Leu Ser Gln Met Gln Thr His Ile Ser Asp Thr Ser Val Val Leu
305                310                315                320

Ser Met Asp Asn Asn Arg Asn Leu Asp Leu Asp Ser Ile Ile Ala Glu
                325                330                335

Val Lys Ala Gln Tyr Glu Glu Ile Ala Gln Arg Ser Arg Ala Glu Ala
                340                345                350

Glu Ser Trp Tyr Gln Thr Lys Tyr Glu Glu Leu Gln Val Thr Ala Gly
                355                360                365

Arg His Gly Asp Asp Leu Arg Asn Thr Lys Gln Glu Ile Ala Glu Ile
        370                375                380

Asn Arg Met Ile Gln Arg Leu Arg Ser Glu Ile Asp His Val Lys Lys
385                390                395                400

Gln Cys Ala Asn Leu Gln Ala Ala Ile Ala Asp Ala Glu Gln Arg Gly
                405                410                415

Glu Met Ala Leu Lys Asp Ala Lys Asn Lys Leu Glu Gly Leu Glu Asp
                420                425                430

Ala Leu Gln Lys Ala Lys Gln Asp Leu Ala Arg Leu Leu Lys Glu Tyr
        435                440                445

Gln Glu Leu Met Asn Val Lys Leu Ala Leu Asp Val Glu Ile Ala Thr
        450                455                460

Tyr Arg Lys Leu Leu Glu Gly Glu Glu Cys Arg Leu Asn Gly Glu Gly
465                470                475                480

Val Gly Gln Val Asn Ile Ser Val Val Gln Ser Thr Val Ser Ser Gly
                485                490                495

Tyr Gly Gly Ala Ser Gly Val Gly Ser Gly Leu Gly Leu Gly Gly Gly
        500                505                510

Ser Ser Tyr Ser Tyr Gly Ser Gly Leu Gly Val Gly Gly Gly Phe Ser
        515                520                525

Ser Ser Ser Gly Arg Ala Ile Gly Gly Gly Leu Ser Ser Val Gly Gly
        530                535                540

Gly Ser Ser Thr Ile Lys Tyr Thr Thr Thr Ser Ser Ser Ser Arg Lys
545                550                555                560

Ser Tyr Lys His

<210> 17
<211> 564
<212> PRT
<213> Homo sapiens

<400> 17
Met Ala Ser Thr Ser Thr Thr Ile Arg Ser His Ser Ser Ser Arg Arg
1               5                   10                  15

Gly Phe Ser Ala Asn Ser Ala Arg Leu Pro Gly Val Ser Arg Ser Gly
            20                  25                  30

Phe Ser Ser Ile Ser Val Ser Arg Ser Arg Gly Ser Gly Gly Leu Gly
            35                  40                  45

Gly Ala Cys Gly Gly Ala Gly Phe Gly Ser Arg Ser Leu Tyr Gly Leu
        50                  55                  60

Gly Gly Ser Lys Arg Ile Ser Ile Gly Gly Gly Ser Cys Ala Ile Ser
65                  70                  75                  80

Gly Gly Tyr Gly Ser Arg Ala Gly Gly Ser Tyr Gly Phe Gly Gly Ala
                85                  90                  95

Gly Ser Gly Phe Gly Phe Gly Gly Gly Ala Gly Ile Gly Phe Gly Leu
            100                 105                 110

Gly Gly Gly Ala Gly Leu Ala Gly Gly Phe Gly Gly Pro Gly Phe Pro
            115                 120                 125

Val Cys Pro Pro Gly Gly Ile Gln Glu Val Thr Val Asn Gln Ser Leu
    130                 135                 140

Leu Thr Pro Leu Asn Leu Gln Ile Asp Pro Ala Ile Gln Arg Val Arg
145                 150                 155                 160

Ala Glu Glu Arg Glu Gln Ile Lys Thr Leu Asn Asn Lys Phe Ala Ser
                165                 170                 175

Phe Ile Asp Lys Val Arg Phe Leu Glu Gln Gln Asn Lys Val Leu Asp
            180                 185                 190

Thr Lys Trp Thr Leu Leu Gln Glu Gln Gly Thr Lys Thr Val Arg Gln
            195                 200                 205

331

Asn Leu Glu Pro Leu Phe Glu Gln Tyr Ile Asn Asn Leu Arg Arg Gln
210                215                220

Leu Asp Asn Ile Val Gly Glu Arg Gly Arg Leu Asp Ser Glu Leu Arg
225                230                235                240

Asn Met Gln Asp Leu Val Glu Asp Leu Lys Asn Lys Tyr Glu Asp Glu
245                250                255

Ile Asn Lys Arg Thr Ala Ala Glu Asn Glu Phe Val Thr Leu Lys Lys
260                265                270

Asp Val Asp Ala Ala Tyr Met Asn Lys Val Glu Leu Gln Ala Lys Ala
275                280                285

Asp Thr Leu Thr Asp Glu Ile Asn Phe Leu Arg Ala Leu Tyr Asp Ala
290                295                300

Glu Leu Ser Gln Met Gln Thr His Ile Ser Asp Thr Ser Val Val Leu
305                310                315                320

Ser Met Asp Asn Asn Arg Asn Leu Asp Leu Asp Ser Ile Ile Ala Glu
325                330                335

Val Lys Ala Gln Tyr Glu Glu Ile Ala Gln Arg Ser Arg Ala Glu Ala
340                345                350

Glu Ser Trp Tyr Gln Thr Lys Tyr Glu Glu Leu Gln Ile Thr Ala Gly
355                360                365

Arg His Gly Asp Asp Leu Arg Asn Thr Lys Gln Glu Ile Ala Glu Ile
370                375                380

Asn Arg Met Ile Gln Arg Leu Arg Ser Glu Ile Asp His Val Lys Lys
385                390                395                400

Gln Cys Ala Asn Leu Gln Ala Ala Ile Ala Asp Ala Glu Gln Arg Gly
405                410                415

Glu Met Ala Leu Lys Asp Ala Lys Asn Lys Leu Glu Gly Leu Glu Asp
420                425                430

Ala Leu Gln Lys Ala Lys Gln Asp Leu Ala Arg Leu Leu Lys Glu Tyr
435                440                445

Gln Glu Leu Met Asn Val Lys Leu Ala Leu Asp Val Glu Ile Ala Thr
450                455                460

```
Tyr Arg Lys Leu Leu Glu Gly Glu Glu Cys Arg Leu Asn Gly Glu Gly
465             470             475             480

Val Gly Gln Val Asn Ile Ser Val Val Gln Ser Thr Val Ser Ser Gly
                485             490             495

Tyr Gly Gly Ala Ser Gly Val Gly Ser Gly Leu Gly Leu Gly Gly Gly
        500             505             510

Ser Ser Tyr Ser Tyr Gly Ser Gly Leu Gly Val Gly Gly Gly Phe Ser
        515             520             525

Ser Ser Ser Gly Arg Ala Thr Gly Gly Gly Leu Ser Ser Val Gly Gly
        530             535             540

Gly Ser Ser Thr Ile Lys Tyr Thr Thr Thr Ser Ser Ser Ser Arg Lys
545             550             555             560

Ser Tyr Lys His


<210> 18
<211> 564
<212> PRT
<213> Homo sapiens

<400> 18
Met Ala Ser Thr Ser Thr Thr Ile Arg Ser His Ser Ser Ser Arg Arg
1               5               10              15

Gly Phe Ser Ala Asn Ser Ala Arg Leu Pro Gly Val Ser Arg Ser Gly
            20              25              30

Phe Ser Ser Ile Ser Val Ser Arg Ser Arg Gly Ser Gly Gly Leu Gly
            35              40              45

Gly Ala Cys Gly Gly Ala Gly Phe Gly Ser Arg Ser Leu Tyr Gly Leu
        50              55              60

Gly Gly Ser Lys Arg Ile Ser Ile Gly Gly Gly Ser Cys Ala Ile Ser
65              70              75              80

Gly Gly Tyr Gly Ser Arg Ala Gly Gly Ser Tyr Gly Phe Gly Gly Ala
                85              90              95

Gly Ser Gly Phe Gly Phe Gly Gly Gly Ala Gly Ile Gly Phe Gly Leu
            100             105             110
```

Gly Gly Gly Ala Gly Leu Ala Gly Gly Phe Gly Gly Pro Gly Phe Pro
        115             120             125

Val Cys Pro Pro Gly Gly Ile Gln Glu Val Thr Val Asn Gln Ser Leu
        130             135             140

Leu Thr Pro Leu Asn Leu Gln Ile Asp Pro Ala Ile Gln Arg Val Arg
145             150             155             160

Ala Glu Glu Arg Glu Gln Ile Lys Thr Leu Asn Asn Lys Phe Ala Ser
            165             170             175

Phe Ile Asp Lys Val Arg Phe Leu Glu Gln Gln Asn Lys Val Leu Asp
            180             185             190

Thr Lys Trp Thr Leu Leu Gln Glu Gln Gly Thr Lys Thr Val Arg Gln
        195             200             205

Asn Leu Glu Pro Leu Phe Glu Gln Tyr Ile Asn Asn Leu Arg Arg Gln
    210             215             220

Leu Asp Ser Ile Val Gly Glu Arg Gly Arg Leu Asp Ser Glu Leu Arg
225             230             235             240

Asn Met Gln Asp Leu Val Glu Asp Leu Lys Asn Lys Tyr Glu Asp Glu
            245             250             255

Ile Asn Lys Arg Thr Ala Ala Glu Asn Glu Phe Val Thr Leu Lys Lys
        260             265             270

Asp Val Asp Ala Ala Tyr Met Asn Lys Val Glu Leu Gln Ala Lys Ala
        275             280             285

Asp Thr Leu Thr Asp Glu Ile Asn Phe Leu Arg Ala Leu Tyr Asp Ala
    290             295             300

Glu Leu Ser Gln Met Gln Thr His Ile Ser Asp Thr Ser Val Val Leu
305             310             315             320

Ser Met Asp Asn Asn Arg Asn Leu Asp Leu Asp Ser Ile Ile Ala Glu
            325             330             335

Val Lys Ala Gln Tyr Glu Glu Ile Ala Gln Arg Ser Arg Ala Glu Ala
        340             345             350

Glu Ser Trp Tyr Gln Thr Lys Tyr Glu Glu Leu Gln Val Thr Ala Gly
        355             360             365

334

Arg His Gly Asp Asp Leu Arg Asn Thr Lys Gln Glu Ile Ala Glu Ile
    370             375             380

Asn Arg Met Ile Gln Arg Leu Arg Ser Glu Ile Asp His Val Lys Lys
385             390             395                 400

Gln Cys Ala Ser Leu Gln Ala Ala Ile Ala Asp Ala Glu Gln Arg Gly
            405             410             415

Glu Met Ala Leu Lys Asp Ala Lys Asn Lys Leu Glu Gly Leu Glu Asp
            420             425             430

Ala Leu Gln Lys Ala Lys Gln Asp Leu Ala Arg Leu Leu Lys Glu Tyr
        435             440             445

Gln Glu Leu Met Asn Val Lys Leu Ala Leu Asp Val Glu Ile Ala Thr
    450             455             460

Tyr Arg Lys Leu Leu Glu Gly Glu Glu Cys Arg Leu Asn Gly Glu Gly
465             470             475                 480

Val Gly Gln Val Asn Val Ser Val Val Gln Ser Thr Ile Ser Ser Gly
            485             490             495

Tyr Gly Gly Ala Ser Gly Val Gly Ser Gly Leu Gly Leu Gly Gly Gly
        500             505             510

Ser Ser Tyr Ser Tyr Gly Ser Gly Leu Gly Ile Gly Gly Gly Phe Ser
    515             520             525

Ser Ser Ser Gly Arg Ala Ile Gly Gly Gly Leu Ser Ser Val Gly Gly
    530             535             540

Gly Ser Ser Thr Ile Lys Tyr Thr Thr Thr Ser Ser Ser Ser Arg Lys
545             550             555                 560

Ser Tyr Lys His


<210> 19
<211> 100
<212> PRT
<213> Homo sapiens

<400> 19
Met Arg Ala Leu Thr Leu Leu Ala Leu Leu Ala Leu Ala Ala Leu Cys
1               5               10                  15

```
Ile Ala Gly Gln Ala Gly Ala Lys Pro Ser Gly Ala Glu Ser Ser Lys
            20                  25              30

Gly Ala Ala Phe Val Ser Lys Gln Glu Gly Ser Glu Val Val Lys Arg
            35                  40              45

Pro Arg Arg Tyr Leu Tyr Gln Trp Leu Gly Ala Pro Val Pro Tyr Pro
            50                  55              60

Asp Pro Leu Glu Pro Arg Arg Glu Val Cys Glu Leu Asn Pro Asp Cys
65                  70                  75                  80

Asp Glu Leu Ala Asp His Ile Gly Phe Gln Glu Ala Tyr Arg Arg Phe
                85                  90                  95

Tyr Gly Pro Val
                100


<210> 20
<211> 95
<212> PRT
<213> Homo sapiens

<400> 20
Met Ala Ala Ala Arg Leu Cys Leu Ser Leu Leu Leu Leu Ser Thr Cys
1               5                   10                  15

Val Ala Leu Leu Leu Gln Pro Leu Leu Gly Ala Gln Gly Ala Pro Leu
            20                  25                  30

Glu Pro Val Tyr Pro Gly Asp Asn Ala Thr Pro Glu Gln Met Ala Gln
            35                  40                  45

Tyr Ala Ala Asp Leu Arg Arg Tyr Ile Asn Met Leu Thr Arg Pro Arg
            50                  55                  60

Tyr Gly Lys Arg His Lys Glu Asp Thr Leu Ala Phe Ser Glu Trp Gly
65                  70                  75                  80

Ser Pro His Ala Ala Val Pro Arg Glu Leu Ser Pro Leu Asp Leu
                85                  90                  95


<210> 21
<211> 97
<212> PRT
<213> Homo sapiens

<400> 21
Met Val Phe Val Arg Arg Pro Trp Pro Ala Leu Thr Thr Val Leu Leu
1               5                   10                  15
```

```
Ala Leu Leu Val Cys Leu Gly Ala Leu Val Asp Ala Tyr Pro Ile Lys
            20                  25                  30

Pro Glu Ala Pro Gly Glu Asp Ala Ser Pro Glu Glu Leu Asn Arg Tyr
            35                  40                  45

Tyr Ala Ser Leu Arg His Tyr Leu Asn Leu Val Thr Arg Gln Arg Tyr
            50                  55                  60

Gly Lys Arg Asp Gly Pro Asp Thr Leu Leu Ser Lys Thr Phe Phe Pro
65                  70                  75                  80

Asp Gly Glu Asp Arg Pro Val Arg Ser Arg Ser Glu Gly Pro Asp Leu
                85                  90                  95

Trp


<210> 22
<211> 132
<212> PRT
<213> Homo sapiens

<400> 22
Met Leu Thr Ala Ala Val Leu Ser Cys Ala Leu Leu Leu Ala Leu Pro
1               5                   10                  15

Ala Thr Arg Gly Ala Gln Met Gly Leu Ala Pro Met Glu Gly Ile Arg
            20                  25                  30

Arg Pro Asp Gln Ala Leu Leu Pro Glu Leu Pro Gly Leu Gly Leu Arg
            35                  40                  45

Ala Pro Leu Lys Lys Thr Thr Ala Glu Gln Ala Glu Glu Asp Leu Leu
            50                  55                  60

Gln Glu Ala Gln Ala Leu Ala Glu Val Leu Asp Leu Gln Asp Arg Glu
65                  70                  75                  80

Pro Arg Ser Ser Arg Arg Cys Val Arg Leu His Glu Ser Cys Leu Gly
                85                  90                  95

Gln Gln Val Pro Cys Cys Asp Pro Cys Ala Thr Cys Tyr Cys Arg Phe
                100                 105                 110

Phe Asn Ala Phe Cys Tyr Cys Arg Lys Leu Gly Thr Ala Met Asn Pro
            115                 120                 125
```

337

Cys Ser Arg Thr
130

<210> 23
<211> 200
<212> PRT
<213> Homo sapiens

<400> 23
Met Ala Phe Thr Glu His Ser Pro Leu Thr Pro His Arg Arg Asp Leu
1               5                   10                  15

Cys Ser Arg Ser Ile Trp Leu Ala Arg Lys Ile Arg Ser Asp Leu Thr
            20                  25                  30

Ala Leu Thr Glu Ser Tyr Val Lys His Gln Gly Leu Asn Lys Asn Ile
            35                  40                  45

Asn Leu Asp Ser Ala Asp Gly Met Pro Val Ala Ser Thr Asp Gln Trp
        50                  55                  60

Ser Glu Leu Thr Glu Ala Glu Arg Leu Gln Glu Asn Leu Gln Ala Tyr
65                  70                  75                  80

Arg Thr Phe His Val Leu Leu Ala Arg Leu Leu Glu Asp Gln Gln Val
            85                  90                  95

His Phe Thr Pro Thr Glu Gly Asp Phe His Gln Ala Ile His Thr Leu
            100                 105                 110

Leu Leu Gln Val Ala Ala Phe Ala Tyr Gln Ile Glu Glu Leu Met Ile
            115                 120                 125

Leu Leu Glu Tyr Lys Ile Pro Arg Asn Glu Ala Asp Gly Met Pro Ile
            130                 135                 140

Asn Val Gly Asp Gly Gly Leu Phe Glu Lys Lys Leu Trp Gly Leu Lys
145                 150                 155                 160

Val Leu Gln Glu Leu Ser Gln Trp Thr Val Arg Ser Ile His Asp Leu
            165                 170                 175

Arg Phe Ile Ser Ser His Gln Thr Gly Ile Pro Ala Arg Gly Ser His
            180                 185                 190

Tyr Ile Ala Asn Asn Lys Lys Met
            195                 200

<210> 24

```
<211> 117
<212> PRT
<213> Homo sapiens

<400> 24
Met Pro Ser Pro Gly Thr Val Cys Ser Leu Leu Leu Gly Met Leu
1               5                   10                  15


Trp Leu Asp Leu Ala Met Ala Gly Ser Ser Phe Leu Ser Pro Glu His
            20                  25                  30


Gln Arg Val Gln Gln Arg Lys Glu Ser Lys Lys Pro Pro Ala Lys Leu
            35                  40                  45


Gln Pro Arg Ala Leu Ala Gly Trp Leu Arg Pro Glu Asp Gly Gly Gln
        50                  55                  60


Ala Glu Gly Ala Glu Asp Glu Leu Glu Val Arg Phe Asn Ala Pro Phe
65                  70                  75                  80


Asp Val Gly Ile Lys Leu Ser Gly Val Gln Tyr Gln Gln His Ser Gln
                85                  90                  95


Ala Leu Gly Lys Phe Leu Gln Asp Ile Leu Trp Glu Glu Ala Lys Glu
                100                 105                 110


Ala Pro Ala Asp Lys
            115


<210> 25
<211> 147
<212> PRT
<213> Homo sapiens

<400> 25
Met Ala Ser His Arg Leu Leu Leu Leu Cys Leu Ala Gly Leu Val Phe
1               5                   10                  15


Val Ser Glu Ala Gly Pro Thr Gly Thr Gly Glu Ser Lys Cys Pro Leu
            20                  25                  30


Met Val Lys Val Leu Asp Ala Val Arg Gly Ser Pro Ala Ile Asn Val
            35                  40                  45


Ala Val His Val Phe Arg Lys Ala Ala Asp Asp Thr Trp Glu Pro Phe
        50                  55                  60


Ala Ser Gly Lys Thr Ser Glu Ser Gly Glu Leu His Gly Leu Thr Thr
65                  70                  75                  80
```

EP 3 249 402 A1

```
Glu Glu Glu Phe Val Glu Gly Ile Tyr Lys Val Glu Ile Asp Thr Lys
            85                  90                  95

Ser Tyr Trp Lys Ala Leu Gly Ile Ser Pro Phe His Glu His Ala Glu
            100                 105                 110

Val Val Phe Thr Ala Asn Asp Ser Gly Pro Arg Arg Tyr Thr Ile Ala
            115                 120                 125

Ala Leu Leu Ser Pro Tyr Ser Tyr Ser Thr Thr Ala Val Val Thr Asn
            130                 135                 140

Pro Lys Glu
145


<210> 26
<211> 1242
<212> PRT
<213> Homo sapiens

<400> 26
Met Ala Ser Pro Pro Glu Ser Asp Gly Phe Ser Asp Val Arg Lys Val
1               5                   10                  15

Gly Tyr Leu Arg Lys Pro Lys Ser Met His Lys Arg Phe Phe Val Leu
            20                  25                  30

Arg Ala Ala Ser Glu Ala Gly Gly Pro Ala Arg Leu Glu Tyr Tyr Glu
            35                  40                  45

Asn Glu Lys Lys Trp Arg His Lys Ser Ser Ala Pro Lys Arg Ser Ile
            50                  55                  60

Pro Leu Glu Ser Cys Phe Asn Ile Asn Lys Arg Ala Asp Ser Lys Asn
65                  70                  75                  80

Lys His Leu Val Ala Leu Tyr Thr Arg Asp Glu His Phe Ala Ile Ala
            85                  90                  95

Ala Asp Ser Glu Ala Glu Gln Asp Ser Trp Tyr Gln Ala Leu Leu Gln
            100                 105                 110

Leu His Asn Arg Ala Lys Gly His His Asp Gly Ala Ala Ala Leu Gly
            115                 120                 125

Ala Gly Gly Gly Gly Gly Ser Cys Ser Gly Ser Ser Gly Leu Gly Glu
            130                 135                 140

Ala Gly Glu Asp Leu Ser Tyr Gly Asp Val Pro Pro Gly Pro Ala Phe
```

340

|  | 145 | | | | 150 | | | | 155 | | | | 160 | |
|---|---|---|---|---|---|---|---|---|---|---|---|---|---|---|

Lys Glu Val Trp Gln Val Ile Leu Lys Pro Lys Gly Leu Gly Gln Thr
             165             170             175

Lys Asn Leu Ile Gly Ile Tyr Arg Leu Cys Leu Thr Ser Lys Thr Ile
         180         185          190

Ser Phe Val Lys Leu Asn Ser Glu Ala Ala Ala Val Val Leu Gln Leu
         195         200         205

Met Asn Ile Arg Arg Cys Gly His Ser Glu Asn Phe Phe Phe Ile Glu
       210         215         220

Val Gly Arg Ser Ala Val Thr Gly Pro Gly Glu Phe Trp Met Gln Val
225             230         235         240

Asp Asp Ser Val Val Ala Gln Asn Met His Glu Thr Ile Leu Glu Ala
         245         250         255

Met Arg Ala Met Ser Asp Glu Phe Arg Pro Arg Ser Lys Ser Gln Ser
         260         265         270

Ser Ser Asn Cys Ser Asn Pro Ile Ser Val Pro Leu Arg Arg His His
         275         280         285

Leu Asn Asn Pro Pro Pro Ser Gln Val Gly Leu Thr Arg Arg Ser Arg
       290         295         300

Thr Glu Ser Ile Thr Ala Thr Ser Pro Ala Ser Met Val Gly Gly Lys
305             310         315         320

Pro Gly Ser Phe Arg Val Arg Ala Ser Ser Asp Gly Glu Gly Thr Met
         325         330         335

Ser Arg Pro Ala Ser Val Asp Gly Ser Pro Val Ser Pro Ser Thr Asn
         340         345         350

Arg Thr His Ala His Arg His Arg Gly Ser Ala Arg Leu His Pro Pro
         355         360         365

Leu Asn His Ser Arg Ser Ile Pro Met Pro Ala Ser Arg Cys Ser Pro
       370         375         380

Ser Ala Thr Ser Pro Val Ser Leu Ser Ser Ser Ser Thr Ser Gly His
385             390         395         400

Gly Ser Thr Ser Asp Cys Leu Phe Pro Arg Arg Ser Ser Ala Ser Val
            405             410             415

Ser Gly Ser Pro Ser Asp Gly Gly Phe Ile Ser Ser Asp Glu Tyr Gly
            420             425             430

Ser Ser Pro Cys Asp Phe Arg Ser Ser Phe Arg Ser Val Thr Pro Asp
            435             440             445

Ser Leu Gly His Thr Pro Pro Ala Arg Gly Glu Glu Glu Leu Ser Asn
    450             455             460

Tyr Ile Cys Met Gly Gly Lys Gly Pro Ser Thr Leu Thr Ala Pro Asn
465             470             475             480

Gly His Tyr Ile Leu Ser Arg Gly Gly Asn Gly His Arg Cys Thr Pro
            485             490             495

Gly Thr Gly Leu Gly Thr Ser Pro Ala Leu Ala Gly Asp Glu Ala Ala
            500             505             510

Ser Ala Ala Asp Leu Asp Asn Arg Phe Arg Lys Arg Thr His Ser Ala
    515             520             525

Gly Thr Ser Pro Thr Ile Thr His Gln Lys Thr Pro Ser Gln Ser Ser
    530             535             540

Val Ala Ser Ile Glu Glu Tyr Thr Glu Met Met Pro Ala Tyr Pro Pro
545             550             555             560

Gly Gly Gly Ser Gly Gly Arg Leu Pro Gly His Arg His Ser Ala Phe
            565             570             575

Val Pro Thr Arg Ser Tyr Pro Glu Glu Gly Leu Glu Met His Pro Leu
            580             585             590

Glu Arg Arg Gly Gly His His Arg Pro Asp Ser Ser Thr Leu His Thr
    595             600             605

Asp Asp Gly Tyr Met Pro Met Ser Pro Gly Val Ala Pro Val Pro Ser
    610             615             620

Gly Arg Lys Gly Ser Gly Asp Tyr Met Pro Met Ser Pro Lys Ser Val
625             630             635             640

Ser Ala Pro Gln Gln Ile Ile Asn Pro Ile Arg Arg His Pro Gln Arg
            645             650             655

342

```
Val Asp Pro Asn Gly Tyr Met Met Met Ser Pro Ser Gly Gly Cys Ser
        660             665         670

Pro Asp Ile Gly Gly Gly Pro Ser Ser Ser Ser Ser Ser Ser Asn Ala
        675             680         685

Val Pro Ser Gly Thr Ser Tyr Gly Lys Leu Trp Thr Asn Gly Val Gly
    690             695         700

Gly His His Ser His Val Leu Pro His Pro Lys Pro Pro Val Glu Ser
705             710         715             720

Ser Gly Gly Lys Leu Leu Pro Cys Thr Gly Asp Tyr Met Asn Met Ser
            725         730             735

Pro Val Gly Asp Ser Asn Thr Ser Ser Pro Ser Asp Cys Tyr Tyr Gly
        740             745         750

Pro Glu Asp Pro Gln His Lys Pro Val Leu Ser Tyr Tyr Ser Leu Pro
        755             760         765

Arg Ser Phe Lys His Thr Gln Arg Pro Gly Glu Pro Glu Glu Gly Ala
    770             775         780

Arg His Gln His Leu Arg Leu Ser Thr Ser Ser Gly Arg Leu Leu Tyr
785             790         795             800

Ala Ala Thr Ala Asp Asp Ser Ser Ser Ser Thr Ser Ser Asp Ser Leu
            805         810             815

Gly Gly Gly Tyr Cys Gly Ala Arg Leu Glu Pro Ser Leu Pro His Pro
        820             825         830

His His Gln Val Leu Gln Pro His Leu Pro Arg Lys Val Asp Thr Ala
        835             840         845

Ala Gln Thr Asn Ser Arg Leu Ala Arg Pro Thr Arg Leu Ser Leu Gly
    850             855         860

Asp Pro Lys Ala Ser Thr Leu Pro Arg Ala Arg Glu Gln Gln Gln Gln
865             870             875             880

Gln Gln Pro Leu Leu His Pro Pro Glu Pro Lys Ser Pro Gly Glu Tyr
            885             890             895

Val Asn Ile Glu Phe Gly Ser Asp Gln Ser Gly Tyr Leu Ser Gly Pro
        900             905             910
```

```
Val Ala Phe His Ser Ser Pro Ser Val Arg Cys Pro Ser Gln Leu Gln
        915                 920                 925

Pro Ala Pro Arg Glu Glu Glu Thr Gly Thr Glu Glu Tyr Met Lys Met
        930                 935                 940

Asp Leu Gly Pro Gly Arg Arg Ala Ala Trp Gln Glu Ser Thr Gly Val
945                 950                 955                 960

Glu Met Gly Arg Leu Gly Pro Ala Pro Pro Gly Ala Ala Ser Ile Cys
                965                 970                 975

Arg Pro Thr Arg Ala Val Pro Ser Ser Arg Gly Asp Tyr Met Thr Met
            980                 985                 990

Gln Met Ser Cys Pro Arg Gln Ser  Tyr Val Asp Thr Ser  Pro Ala Ala
        995                 1000                1005

Pro Val  Ser Tyr Ala Asp Met  Arg Thr Gly Ile  Ala  Ala Glu Glu
    1010                1015                1020

Val Ser  Leu Pro Arg Ala Thr  Met Ala Ala Ala  Ser  Ser Ser Ser
    1025                1030                1035

Ala Ala  Ser Ala Ser Pro Thr  Gly Pro Gln Gly Ala  Ala Glu Leu
    1040                1045                1050

Ala Ala  His Ser Ser Leu Leu  Gly Gly Pro Gln Gly  Pro Gly Gly
    1055                1060                1065

Met Ser  Ala Phe Thr Arg Val  Asn Leu Ser Pro Asn  Arg Asn Gln
    1070                1075                1080

Ser Ala  Lys Val Ile Arg Ala  Asp Pro Gln Gly Cys  Arg Arg Arg
    1085                1090                1095

His Ser  Ser Glu Thr Phe Ser  Ser Thr Pro Ser Ala  Thr Arg Val
    1100                1105                1110

Gly Asn  Thr Val Pro Phe Gly  Ala Gly Ala Ala Val  Gly Gly Gly
    1115                1120                1125

Gly Gly  Ser Ser Ser Ser Ser  Glu Asp Val Lys Arg  His Ser Ser
    1130                1135                1140

Ala Ser  Phe Glu Asn Val Trp  Leu Arg Pro Gly Glu  Leu Gly Gly
```

```
                    1145                        1150                        1155


          Ala Pro  Lys Glu Pro Ala Lys  Leu Cys Gly Ala Ala  Gly Gly Leu
              1160                   1165                1170


          Glu Asn  Gly Leu Asn Tyr Ile  Asp Leu Asp Leu Val  Lys Asp Phe
              1175                   1180                1185


          Lys Gln  Cys Pro Gln Glu Cys  Thr Pro Glu Pro Gln  Pro Pro Pro
              1190                   1195                1200


          Pro Pro  Pro Pro His Gln Pro  Leu Gly Ser Gly Glu  Ser Ser Ser
              1205                   1210                1215


          Thr Arg  Arg Ser Ser Glu Asp  Leu Ser Ala Tyr Ala  Ser Ile Ser
              1220                   1225                1230


          Phe Gln  Lys Gln Pro Glu Asp  Arg Gln
              1235                   1240


          <210> 27
          <211> 317
          <212> PRT
          <213> Homo sapiens

          <400> 27
          Met Phe Gln Ala Ala Glu Arg Pro Gln Glu Trp Ala Met Glu Gly Pro
          1                 5                     10                      15


          Arg Asp Gly Leu Lys Lys Glu Arg Leu Leu Asp Asp Arg His Asp Ser
                      20                    25                    30


          Gly Leu Asp Ser Met Lys Asp Glu Glu Tyr Glu Gln Met Val Lys Glu
                  35                    40                    45


          Leu Gln Glu Ile Arg Leu Glu Pro Gln Glu Val Pro Arg Gly Ser Glu
              50                    55                    60


          Pro Trp Lys Gln Gln Leu Thr Glu Asp Gly Asp Ser Phe Leu His Leu
          65                    70                    75                      80


          Ala Ile Ile His Glu Glu Lys Ala Leu Thr Met Glu Val Ile Arg Gln
                            85                    90                      95


          Val Lys Gly Asp Leu Ala Phe Leu Asn Phe Gln Asn Asn Leu Gln Gln
                      100                   105                   110


          Thr Pro Leu His Leu Ala Val Ile Thr Asn Gln Pro Glu Ile Ala Glu
                  115                   120                   125
```

```
Ala Leu Leu Gly Ala Gly Cys Asp Pro Glu Leu Arg Asp Phe Arg Gly
    130                 135             140

Asn Thr Pro Leu His Leu Ala Cys Glu Gln Gly Cys Leu Ala Ser Val
145                 150                 155                 160

Gly Val Leu Thr Gln Ser Cys Thr Thr Pro His Leu His Ser Ile Leu
                165             170                 175

Lys Ala Thr Asn Tyr Asn Gly His Thr Cys Leu His Leu Ala Ser Ile
            180             185                 190

His Gly Tyr Leu Gly Ile Val Glu Leu Leu Val Ser Leu Gly Ala Asp
        195             200             205

Val Asn Ala Gln Glu Pro Cys Asn Gly Arg Thr Ala Leu His Leu Ala
210                 215             220

Val Asp Leu Gln Asn Pro Asp Leu Val Ser Leu Leu Leu Lys Cys Gly
225             230             235                 240

Ala Asp Val Asn Arg Val Thr Tyr Gln Gly Tyr Ser Pro Tyr Gln Leu
            245             250             255

Thr Trp Gly Arg Pro Ser Thr Arg Ile Gln Gln Gln Leu Gly Gln Leu
        260             265             270

Thr Leu Glu Asn Leu Gln Met Leu Pro Glu Ser Glu Asp Glu Glu Ser
    275             280             285

Tyr Asp Thr Glu Ser Glu Phe Thr Glu Phe Thr Glu Asp Glu Leu Pro
    290             295             300

Tyr Asp Asp Cys Val Phe Gly Gly Gln Arg Leu Thr Leu
305             310             315
```

```
<210> 28
<211> 4
<212> PRT
<213> Homo sapiens

<400> 28
Ser Phe Thr Leu
1
```

**Description**

The present application claims priority to U.S. Provisional Patent Application 61/390,999 filed October 7, 2010, which is hereby incorporated in its entirety including all tables, figures, and claims.

BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830, which are hereby incorporated by reference in their entirety. Renal disease and/or injury may be acute or chronic. Acute and chronic kidney disease are described as follows (from Current Medical Diagnosis & Treatment 2008, 47th Ed, McGraw Hill, New York, pages 785-815, which are hereby incorporated by reference in their entirety): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week)reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222, and which is hereby incorporated by reference in their entirety:

| Type | Risk Factors |
|---|---|
| **Prerenal** | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |

(continued)

| Intrinsic Renal | |
|---|---|
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |
| Acute tubulo interstitial nephritis | Drug reaction (eg, β-lactams, NSAIDs, sulfonamides, ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |
| Postrenal | |
| Tubular precipitation | Uric acid (tumor lysis), sulfonamides, triamterene, acyclovir, indinavir, methotrexate, ethylene glycol |
| | ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370, 2005, which, with the references listed therein, are hereby incorporated by reference in their entirety. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004, hereby incorporated by reference in its entirety) investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004, which is hereby incorporated by reference in its entirety, proposes the following classifications for stratifying AKI patients:

"Risk": serum creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.

"ESRD": end stage renal disease-the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, each hereby incorporated by reference in its entirety, the RIFLE criteria provide a uniform definition of AKI which has been validated in numerous studies.

More recently, Mehta et al., Crit. Care 11:R31 (doi:10.1186.cc5713), 2007, hereby incorporated by reference in its entirety, proposes the following similar classifications for stratifying AKI patients, which have been modified from RIFLE:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR serum creatinine $\geq$ 354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006; 7(4):177-197, hereby incorporated by reference in its entirety) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

BRIEF SUMMARY OF THE INVENTION

It is an object of the invention to provide methods and compositions for evaluating renal function in a subject. As described herein, measurement of one or more biomarkers selected from the group consisting of Beta-nerve growth factor, Interleukin-17A, Follitropin subunit beta, Collagenase 3, Follistatin, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha (referred to herein as a "kidney injury marker") can be used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury).

The kidney injury markers of the present invention may be used, individually or in panels comprising a plurality of kidney injury markers, for risk stratification (that is, to identify subjects at risk for a future injury to renal function, for future progression to reduced renal function, for future progression to ARF, for future improvement in renal function, etc.); for diagnosis of existing disease (that is, to identify subjects who have suffered an injury to renal function, who have progressed to reduced renal function, who have progressed to ARF, etc.); for monitoring for deterioration or improvement of renal function; and for predicting a future medical outcome, such as improved or worsening renal function, a decreased or increased mortality risk, a decreased or increased risk that a subject will require renal replacement therapy (i.e., hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, etc.

In a first aspect, the present invention relates to methods for evaluating renal status in a subject. These methods comprise performing an assay method that is configured to detect one or more biomarkers selected from the group consisting of Beta-nerve growth factor, Interleukin-17A, Follitropin subunit beta, Collagenase 3, Follistatin, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha in a body fluid sample obtained from the subject. The assay result(s), for example measured concentration(s) of one or more biomarkers selected from the group consisting of Beta-nerve growth factor, Interleukin-17A, Follitropin subunit beta, Collagenase 3, Follistatin, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha is/are then correlated to the renal status of the subject. This correlation to renal status may include correlating the assay result(s) to one or more of risk stratification, diagnosis, prognosis, staging, classifying and monitoring of the subject as described herein. Thus, the present invention utilizes one or more kidney injury markers of the present invention for the evaluation of renal injury.

In certain embodiments, the methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments, these methods comprise determining a subject's risk for future reduced renal function, and the assay result(s) is/are correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

In other embodiments, the methods for evaluating renal status described herein are methods for diagnosing a renal injury in the subject; that is, assessing whether or not a subject has suffered from an injury to renal function, reduced renal function, or ARF. In these embodiments, the assay result(s), for example measured concentration(s) of one or more biomarkers selected from the group consisting of Beta-nerve growth factor, Interleukin-17A, Follitropin subunit beta, Collagenase 3, Follistatin, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha is/are correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the assay result(s) is/are correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic embodiments, these methods comprise diagnosing the occurrence or nonoccurrence of reduced renal function, and the assay result(s) is/are correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold). For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic embodiments, these methods comprise diagnosing the occurrence or nonoccurrence of ARF, and the assay result(s) is/are correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic embodiments, these methods comprise diagnosing a subject as being in need of renal replacement therapy, and the assay result(s) is/are correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic embodiments, these methods comprise diagnosing a subject as being in need of renal transplantation, and the assay result(s0 is/are correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the likelihood assigned when the measured concentration is below the threshold).

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay result(s), for example measured concentration(s) of one or more biomarkers selected from the group consisting of Beta-nerve growth factor, Interleukin-17A, Follitropin subunit beta, Collagenase 3, Follistatin, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha is/are correlated to the occurrence or nonoccurrence of a change in renal status. The following are preferred monitoring embodiments.

In preferred monitoring embodiments, these methods comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the assay result(s), for example measured concentration(s) of one or more biomarkers selected from the group consisting of Beta-nerve growth factor, Interleukin-17A, Follitropin subunit beta, Collagenase 3, Follistatin, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha is/are correlated to a particular class and/or subclass. The following are preferred classification embodiments.

In preferred classification embodiments, these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, *etc.),* by selecting a concentration representing the 75th, 85th, 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers of the present invention must be compared to corresponding individual thresholds. Methods for combining assay results can comprise the use of multivariate logistical regression, loglinear modeling, neural network analysis, n-of-m analysis, decision tree analysis, calculating ratios of markers, *etc.* This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity; a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/-5% of a given measurement.

Multiple thresholds may also be used to assess renal status in a subject. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, the present invention also relates to devices and kits for performing the methods described herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, *etc.*) as well as molecules that may be indirectly detected by production of a detectable reaction product (*e.g.*, enzymes such as horseradish peroxidase, alkaline phosphatase, *etc.)* or through the use of a specific binding molecule which itself may be detectable (*e.g.*, a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, *etc.* In certain of these methods, the solid phase antibody is coupled to a transducer (*e.g.*, a diffraction grating, electrochemical sensor, etc) for generation of a signal, while in others, a signal is generated by a transducer that is spatially separate from the solid phase antibody (*e.g.*, a fluorometer that employs an excitation light source and an optical detector). This list is not meant to be limiting. Antibody-based biosensors may also be employed to determine the presence or amount of analytes that optionally eliminate the need for a labeled molecule.

DETAILED DESCRIPTION OF THE INVENTION

The present invention relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers. In various embodiments, a measured concentration of one or more biomarkers selected from the group consisting of Beta-nerve growth factor, Interleukin-17A, Follitropin subunit beta, Collagenase 3, Follistatin, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha or one or more markers related thereto, are correlated to the renal status of the subject.

For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq$ 8.8 $\mu$mol/L), a percentage increase in serum creatinine of greater than or equal to 20% (1.2-fold from baseline), or a reduction in urine output (documented oliguria of less than 0. 5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq$ 26.4 $\mu$mol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

As used herein, the term "Beta-nerve growth factor" refers to one or more polypeptides present in a biological sample that are derived from the Beta-nerve growth factor precursor (Swiss-Prot P01138 (SEQ ID NO: 1)).

```
          10         20         30         40         50         60

MSMLFYTLIT AFLIGIQAEP HSESNVPAGH TIPQAHWTKL QHSLDTALRR ARSAPAAAIA

          70         80         90        100        110        120

ARVAGQTRNI TVDPRLFKKR RLRSPRVLFS TQPPREAADT QDLDFEVGGA APFNRTHRSK

         130        140        150        160        170        180

RSSSHPIFHR GEFSVCDSVS VWVGDKTTAT DIKGKEVMVL GEVNINNSVF KQYFFETKCR

         190        200        210        220        230        240

DPNPVDSGCR GIDSKHWNSY CTTTHTFVKA LTMDGKQAAW RFIRIDTACV CVLSRKAVRR

241

A
```

The following domains have been identified in Beta-nerve growth factor:

| Residues | Length | Domain ID |
|---|---|---|
| 1-18 | 18 | signal sequence |
| 19-121 | 103 | propeptide |
| 122-241 | 120 | mature Beta-nerve growth factor |

As used herein, the term "Interleukin-17A" refers to one or more polypeptides present in a biological sample that are derived from the Interleukin-17A precursor (Swiss-Prot Q16552 (SEQ ID NO: 2)).

```
          10         20         30         40         50         60

MTPGKTSLVS LLLLLLSLEAI VKAGITIPRN PGCPNSEDKN FPRTVMVNLN IHNRNTNTNP

          70         80         90        100        110        120

KRSSDYYNRS TSPWNLHRNE DPERYPSVIW EAKCRHLGCI NADGNVDYHM NSVPIQQEIL

         130        140        150

VLRREPPHCP NSFRLEKILV SVGCTCVTPI VHHVA
```

The following domains have been identified in Interleukin-17A:

| Residues | Length | Domain ID |
|---|---|---|
| 1-23 | 23 | signal sequence |
| 24-155 | 132 | mature Interleukin-17A |

As used herein, the term "Follitropin subunit beta" refers to one or more polypeptides present in a biological sample that are derived from the Follitropin subunit beta precursor (Swiss-Prot P01225 (SEQ ID NO: 3)).

```
         10         20         30         40         50         60
   MKTLQFFFLF CCWKAICCNS CELTNITIAI EKEECRFCIS INTTWCAGYC YTRDLVYKDP

         70         80         90        100        110        120
   ARPKIQKTCT FKELVYETVR VPGCAHHADS LYTYPVATQC HCGKCDSDST DCTVRGLGPS

   YCSFGEMKE
```

The following domains have been identified in Follitropin subunit beta:

| Residues | Length | Domain ID |
|----------|--------|-----------|
| 1-18 | 18 | signal sequence |
| 19-129 | 111 | mature Follitropin subunit beta |

As used herein, the term "Collagenase 3" refers to one or more polypeptides present in a biological sample that are derived from the Collagenase 3 precursor (Swiss-Prot P45452 (SEQ ID NO: 4)).

```
         10         20         30         40         50         60
   MHPGVLAAFL FLSWTHCRAL PLPSGGDEDD LSEEDLQFAE RYLRSYYHPT NLAGILKENA

         70         80         90        100        110        120
   ASSMTERLRE MQSFFGLEVT GKLDDNTLDV MKKPRCGVPD VGEYNVFPRT LKWSKMNLTY

        130        140        150        160        170        180
   RIVNYTPDMT HSEVEKAFKK AFKVWSDVTP LNFTRLHDGI ADIMISFGIK EHGDFYPFDG

        190        200        210        220        230        240
   PSGLLAHAFP PGPNYGGDAH FDDDETWTSS SKGYNLFLVA AHEFGHSLGL DHSKDPGALM

        250        260        270        280        290        300
   FPIYTYTGKS HFMLPDDDVQ GIQSLYGPGD EDPNPKHPKT PDKCDPSLSL DAITSLRGET

        310        320        330        340        350        360
   MIFKDRFFWR LHPQQVDAEL FLTKSFWPEL PNRIDAAYEH PSHDLIFIFR GRKFWALNGY

        370        380        390        400        410        420
   DILEGYPKKI SELGLPKEVK KISAAVHFED TGKTLLFSGN QVWRYDDTNH IMDKDYPRLI

        430        440        450        460        470
   EEDFPGIGDK VDAVYEKNGY IYFFNGPIQF EYSIWSNRIV RVMPANSILW C
```

The following domains have been identified in Collagenase 3:

| Residues | Length | Domain ID |
|----------|--------|-----------|
| 1-19 | 19 | signal sequence |
| 20-103 | 84 | Activation peptide |
| 104-471 | 368 | mature Collagenase 3 |

As used herein, the term "Follistatin" refers to one or more polypeptides present in a biological sample that are derived from the Follistatin precursor (Swiss-Prot P19883 (SEQ ID NO: 5)).

```
           10         20         30         40         50         60
    MVRARHQPGG LCLLLLLLCQ FMEDRSAQAG NCWLRQAKNG RCQVLYKTEL SKEECCSTGR

           70         80         90        100        110        120
    LSTSWTEEDV NDNTLFKWMI FNGGAPNCIP CKETCENVDC GPGKKCRMNK KNKPRCVCAP

          130        140        150        160        170        180
    DCSNITWKGP VCGLDGKTYR NECALLKARC KEQPELEVQY QGRCKKTCRD VFCPGSSTCV

          190        200        210        220        230        240
    VDQTNNAYCV TCNRICPEPA SSEQYLCGND GVTYSSACHL RKATCLLGRS IGLAYEGKCI

          250        260        270        280        290        300
    KAKSCEDIQC TGGKKCLWDF KVGRGRCSLC DELCPDSKSD EPVCASDNAT YASECAMKEA

          310        320        330        340
    ACSSGVLLEV KHSGSCNSIS EDTEEEEEDE DQDYSFPISS ILEW
```

The following domains have been identified in Follistatin:

| Residues | Length | Domain ID |
| --- | --- | --- |
| 1-29 | 29 | signal sequence |
| 30-344 | 315 | mature Follistatin |
| 318-344 | 27 | missing in isoform 2 |
| 318-323 | 6 | missing in isoform 3 |

As used herein, the term "Vitamin D Binding Protein" refers to one or more polypeptides present in a biological sample that are derived from the Vitamin D Binding Protein precursor (Swiss-Prot P02774 (SEQ ID NO: 6)).

```
           10         20         30         40         50         60
    MKRVLVLLLA VAFGHALERG RDYEKNKVCK EFSHLGKEDF TSLSLVLYSR KFPSGTFEQV
```

```
        70         80         90        100        110        120
SQLVKEVVSL TEACCAEGAD PDCYDTRTSA LSAKSCESNS PFPVHPGTAE CCTKEGLERK

       130        140        150        160        170        180
LCMAALKHQP QEFPTYVEPT NDEICEAFRK DPKEYANQFM WEYSTNYGQA PLSLLVSYTK

       190        200        210        220        230        240
SYLSMVGSCC TSASPTVCFL KERLQLKHLS LLTTLSNRVC SQYAAYGEKK SRLSNLIKLA

       250        260        270        280        290        300
QKVPTADLED VLPLAEDITN ILSKCCESAS EDCMAKELPE HTVKLCDNLS TKNSKFEDCC

       310        320        330        340        350        360
QEKTAMDVFV CTYFMPAAQL PELPDVELPT NKDVCDPGNT KVMDKYTFEL SRRTHLPEVF

       370        380        390        400        410        420
LSKVLEPTLK SLGECCDVED STTCFNAKGP LLKKELSSFI DKGQELCADY SENTFTEYKK

       430        440        450        460        470
KLAERLKAKL PDATPKELAK LVNKRSDFAS NCCSINSPPL YCDSEIDAEL KNIL
```

The following domains have been identified in Vitamin D Binding Protein:

| Residues | Length | Domain ID |
|---|---|---|
| 1-16 | 16 | signal sequence |
| 17-474 | 458 | mature Vitamin D Binding Protein |
| 1-122 | 122 | missing in isoform 2 |

As used herein, the term "Islet amyloid polypeptide" refers to one or more polypeptides present in a biological sample that are derived from the Islet amyloid polypeptide precursor (Swiss-Prot P10997 (SEQ ID NO: 7)).

```
        10         20         30         40         50         60
MGILKLQVFL IVLSVALNHL KATPIESHQV EKRKCNTATC ATQRLANFLV HSSNNFGAIL

        70         80
SSTNVGSNTY GKRNAVEVLK REPLNYLPL
```

The following domains have been identified in Islet amyloid polypeptide:

| Residues | Length | Domain ID |
|---|---|---|
| 1-22 | 22 | signal sequence |
| 23-31 | 9 | propeptide |
| 34-70 | 37 | mature Islet amyloid polypeptide |
| 74-89 | 16 | propeptide |

As used herein, the term "Insulin C-peptide" refers to one or more polypeptides present in a biological sample that are derived from the insulin precursor (Swiss-Prot P01308 (SEQ ID NO: 8)) and which comprise all or a portion of the C-peptide sequence.

```
            10         20         30         40         50         60
    MALWMRLLPL LALLALWGPD PAAAFVNQHL CGSHLVEALY LVCGERGFFY TPKTRREAED

            70         80         90        100        110
    LQVGQVELGG GPGAGSLQPL ALEGSLQKRG IVEQCCTSIC SLYQLENYCN
```

The following domains have been identified in the Insulin precursor:

| Residues | Length | Domain ID |
|----------|--------|-----------|
| 1-24 | 24 | signal sequence |
| 25-54 | 30 | Insulin B chain |
| 57-87 | 31 | Insulin C-peptide |
| 90-110 | 21 | Insulin A chain |

As used herein, the term "Complement Factor H" refers to one or more polypeptides present in a biological sample that are derived from the Complement Factor H precursor (Swiss-Prot P08603 (SEQ ID NO: 9)).

```
            10         20         30         40         50         60
    MRLLAKIICL MLWAICVAED CNELPPRRNT EILTGSWSDQ TYPEGTQAIY KCRPGYRSLG

            70         80         90        100        110        120
    NVIMVCRKGE WVALNPLRKC QKRPCGHPGD TPFGTFTLTG GNVFEYGVKA VYTCNEGYQL

           130        140        150        160        170        180
    LGEINYRECD TDGWTNDIPI CEVVKCLPVT APENGKIVSS AMEPDREYHF GQAVRFVCNS

           190        200        210        220        230        240
    GYKIEGDEEM HCSDDGFWSK EKPKCVEISC KSPDVINGSP ISQKIIYKEN ERFQYKCNMG

           250        260        270        280        290        300
    YEYSERGDAV CTESGWRPLP SCEEKSCDNP YIPNGDYSPL RIKHRTGDEI TYQCRNGFYP

           310        320        330        340        350        360
    ATRGNTAKCT STGWIPAPRC TLKPCDYPDI KHGGLYHENM RRPYFPVAVG KYYSYYCDEH

           370        380        390        400        410        420
    FETPSGSYWD HIHCTQDGWS PAVPCLRKCY FPYLENGYNQ NYGRKFVQGK SIDVACHPGY
```

```
        430        440        450        460        470        480
   ALPKAQTTVT CMENGWSPTP RCIRVKTCSK SSIDIENGFI SESQYTYALK EKAKYQCKLG

        490        500        510        520        530        540
   YVTADGETSG SITCGKDGWS AQPTCIKSCD IPVFMNARTK NDFTWFKLND TLDYECHDGY

        550        560        570        580        590        600
   ESNTGSTTGS IVCGYNGWSD LPICYERECE LPKIDVHLVP DRKKDQYKVG EVLKFSCKPG

        610        620        630        640        650        660
   FTIVGPNSVQ CYHFGLSPDL PICKEQVQSC GPPPELLNGN VKEKTKEEYG HSEVVEYYCN

        670        680        690        700        710        720
   PRFLMKGPNK IQCVDGEWTT LPVCIVEEST CGDIPELEHG WAQLSSPPYY YGDSVEFNCS

        730        740        750        760        770        780
   ESFTMIGHRS ITCIHGVWTQ LPQCVAIDKL KKCKSSNLII LEEHLKNKKE FDHNSNIRYR

        790        800        810        820        830        840
   CRGKEGWIHT VCINGRWDPE VNCSMAQIQL CPPPPQIPNS HNMTTTLNYR DGEKVSVLCQ

        850        860        870        880        890        900
   ENYLIQEGEE ITCKDGRWQS IPLCVEKIPC SQPPQIEHGT INSSRSSQES YAHGTKLSYT

        910        920        930        940        950        960
   CEGGFRISEE NETTCYMGKW SSPPQCEGLP CKSPPEISHG VVAHMSDSYQ YGEEVTYKCF

        970        980        990       1000       1010       1020
   EGFGIDGPAI AKCLGEKWSH PPSCIKTDCL SLPSFENAIP MGEKKDVYKA GEQVTYTCAT

       1030       1040       1050       1060       1070       1080
   YYKMDGASNV TCINSRWTGR PTCRDTSCVN PPTVQNAYIV SRQMSKYPSG ERVRYQCRSP

       1090       1100       1110       1120       1130       1140
   YEMFGDEEVM CLNGNWTEPP QCKDSTGKCG PPPPIDNGDI TSFPLSVYAP ASSVEYQCQN

       1150       1160       1170       1180       1190       1200
   LYQLEGNKRI TCRNGQWSEP PKCLHPCVIS REIMENYNIA LRWTAKQKLY SRTGESVEFV

       1210       1220       1230
   CKRGYRLSSR SHTLRTTCWD GKLEYPTCAK R
```

The following domains have been identified in Complement Factor H:

| Residues | Length | Domain ID |
|---|---|---|
| 1-18 | 18 | signal sequence |
| 19-1231 | 1213 | mature Complement Factor H |
| 446-449 | | KTCS → SFTL in isoform 2 |
| 450-1231 | | missing in isoform 2 |

As used herein, the term "Gastric inhibitory polypeptide" refers to one or more polypeptides present in a biological sample that are derived from the Gastric inhibitory polypeptide precursor (Swiss-Prot P09681 (SEQ ID NO: 10)).

```
        10          20          30          40          50          60
MVATKTFALL  LLSLFLAVGL  GEKKEGHFSA  LPSLPVGSHA  KVSSPQPRGP  RYAEGTFISD

        70          80          90         100         110         120
YSIAMDKIHQ  QDFVNWLLAQ  KGKKNDWKHN  ITQREARALE  LASQANRKEE  EAVEPQSSPA

       130         140         150
KNPSDEDLLR  DLLIQELLAC  LLDQTNLCRL  RSR
```

The following domains have been identified in Gastric inhibitory polypeptide:

| Residues | Length | Domain ID |
|---|---|---|
| 1-21 | 21 | signal sequence |
| 22-50 | 29 | propeptide |
| 52-93 | 42 | mature Gastric inhibitory polypeptide |
| 95-153 | 59 | propeptide |

As used herein, the term "Glucagon" refers to one or more polypeptides present in a biological sample that are derived from the Glucagon precursor (Swiss-Prot P01275 (SEQ ID NO: 12)) and which comprises all or a portion of mature Glucagon. Similarly, the term "Glucagon-like peptide 1" refers to one or more polypeptides present in a biological sample that are derived from the Glucagon precursor (Swiss-Prot P01275 (SEQ ID NO: 12)) and which comprises all or a portion of Glucagon-like peptide 1.

```
        10          20          30          40          50          60
MKSIYFVAGL  FVMLVQGSWQ  RSLQDTEEKS  RSFSASQADP  LSDPDQMNED  KRHSQGTFTS

        70          80          90         100         110         120
DYSKYLDSRR  AQDFVQWLMN  TKRNRNNIAK  RHDEFERHAE  GTFTSDVSSY  LEGQAAKEFI

       130         140         150         160         170         180
AWLVKGRGRR  DFPEEVAIVE  ELGRRHADGS  FSDEMNTILD  NLAARDFINW  LIQTKITDRK
```

The following domains have been identified in Glucagon:

| Residues | Length | Domain ID |
|---|---|---|
| 1-20 | 20 | signal sequence |
| 21-89 | 69 | glicentin |
| 21-50 | 30 | glicentin-related polypeptide |
| 53-89 | 37 | oxyntomodulin |
| 53-81 | 29 | mature Glucagon |
| 84-89 | 6 | propeptide |
| 92-128 | 37 | Glucagon-like peptide 1 |
| 98-128 | 31 | Glucagon-like peptide 1(7-37) |
| 98-127 | 30 | Glucagon-like peptide 1(7-36) |
| 131-145 | 15 | propeptide |
| 146-178 | 33 | glucagon-like peptide 2 |

As used herein, the term "Involucrin" refers to one or more polypeptides present in a biological sample that are derived from the Involucrin precursor (Swiss-Prot P07476 (SEQ ID NO: 13)).

```
        10         20         30         40         50         60
MSQQHTLPVT LSPALSQELL KTVPPPVNTH QEQMKQPTPL PPPCQKVPVE LPVEVPSKQE

        70         80         90        100        110        120
EKHMTAVKGL PEQECEQQQK EPQEQELQQQ HWEQHEEYQK AENPEQQLKQ EKTQRDQQLN

       130        140        150        160        170        180
KQLEEEKKLL DQQLDQELVK RDEQLGMKKE QLLELPEQQE GHLKHLEQQE GQLKHPEQQE

       190        200        210        220        230        240
GQLELPEQQE GQLELPEQQE GQLELPEQQE GQLELPEQQE GQLELPEQQE GQLELPQQQE

       250        260        270        280        290        300
GQLELSEQQE GQLELSEQQE GQLKHLEHQE GQLEVPEEQM GQLKYLEQQE GQLKHLDQQE

       310        320        330        340        350        360
KQPELPEQQM GQLKHLEQQE GQPKHLEQQE GQLEQLEEQE GQLKHLEQQE GQLEHLEHQE

       370        380        390        400        410        420
GQLGLPEQQV LQLKQLEKQQ GQPKHLEEEE GQLKHLVQQE GQLKHLVQQE GQLEQQERQV

       430        440        450        460        470        480
EHLEQQVGQL KHLEEQEGQL KHLEQQQGQL EVPEQQVGQP KNLEQEEKQL ELPEQQEGQV

       490        500        510        520        530        540
KHLEKQEAQL ELPEQQVGQP KHLEQQEKHL EHPEQQDGQL KHLEQQEGQL KDLEQQKGQL

       550        560        570        580
EQPVFAPAPG QVQDIQPALP TKGEVLLPVE HQQQKQEVQW PPKHK
```

As used herein, the term "type II cytoskeletal Keratin-1/10" refers to one or more polypeptides present in a biological sample that are derived from the type II cytoskeletal Keratin-1 precursor (Swiss-Prot P04264 (SEQ ID NO: 14))

```
            10          20          30          40          50          60
   MSRQFSSRSG  YRSGGGFSSG  SAGIINYQRR  TTSSSTRRSG  GGGGRFSSCG  GGGGSFGAGG

            70          80          90         100         110         120
   GFGSRSLVNL  GGSKSISISV  ARGGGRGSGF  GGGYGGGGFG  GGGFGGGGFG  GGGIGGGGFG

           130         140         150         160         170         180
   GFGSGGGGFG  GGGFGGGGYG  GGYGPVCPPG  GIQEVTINQS  LLQPLNVEID  PEIQKVKSRE

           190         200         210         220         230         240
   REQIKSLNNQ  FASFIDKVRF  LEQQNQVLQT  KWELLQQVDT  STRTHNLEPY  FESFINNLRR

           250         260         270         280         290         300
   RVDQLKSDQS  RLDSELKNMQ  DMVEDYRNKY  EDEINKRTNA  ENEFVTIKKD  VDGAYMTKVD

           310         320         330         340         350         360
   LQAKLDNLQQ  EIDFLTALYQ  AELSQMQTQI  SETNVILSMD  NNRSLDLDSI  IAEVKAQYED

           370         380         390         400         410         420
   IAQKSKAEAE  SLYQSKYEEL  QITAGRHGDS  VRNSKIEISE  LNRVIQRLRS  EIDNVKKQIS

           430         440         450         460         470         480
   NLQQSISDAE  QRGENALKDA  KNKLNDLEDA  LQQAKEDLAR  LLRDYQELMN  TKLALDLEIA

           490         500         510         520         530         540
   TYRTLLEGEE  SRMSGECAPN  VSVSVSTSHT  TISGGGSRGG  GGGGYGSGGS  SYGSGGGSYG

           550         560         570         580         590         600
   SGGGGGGGRG  SYGSGGSSYG  SGGGSYGSGG  GGGGHGSYGS  GSSSGGYRGG  SGGGGGGSSG

           610         620         630         640
   GRGSGGGSSG  GSIGGRGSSS  GGVKSSGGSS  SVKFVSTTYS  GVTR
```

or from the type II cytoskeletal Keratin-10 precursor (Swiss-Prot P13645 (SEQ ID NO: 15)).

```
            10          20          30          40          50          60
   MSVRYSSSKH  YSSSRSGGGG  GGGGCGGGGG  VSSLRISSSK  GSLGGGFSSG  GFSGGSFSRG

            70          80          90         100         110         120
   SSGGGCFGGS  SGGYGGLGGF  GGGSFRGSYG  SSSFGGSYGG  IFGGGSFGGG  SFGGGSFGGG

           130         140         150         160         170         180
   GFGGGGFGGG  FGGGFGGDGG  LLSGNEKVTM  QNLNDRLASY  LDKVRALEES  NYELEGKIKE

           190         200         210         220         230         240
   WYEKHGNSHQ  GEPRDYSKYY  KTIDDLKNQI  LNLTTDNANI  LLQIDNARLA  ADDFRLKYEN
```

```
        250        260        270        280        290        300
   EVALRQSVEA DINGLRRVLD ELTLTKADLE MQIESLTEEL AYLKKNHEEE MKDLRNVSTG

        310        320        330        340        350        360
   DVNVEMNAAP GVDLTQLLNN MRSQYEQLAE QNRKDAEAWF NEKSKELTTE IDNNIEQISS

        370        380        390        400        410        420
   YKSEITELRR NVQALEIELQ SQLALKQSLE ASLAETEGRY CVQLSQIQAQ ISALEEQLQQ

        430        440        450        460        470        480
   IRAETECQNT EYQQLLDIKI RLENEIQTYR SLLEGEGSSG GGGRGGGSFG GGYGGGSSGG

        490        500        510        520        530        540
   GSSGGGHGGG HGGSSGGGYG GGSSGGGSSG GGYGGGSSSG GHGGSSSGGY GGGSSGGGGG

        550        560        570        580
   GYGGGSSGGG SSSGGGYGGG SSSGGHKSSS SGSVGESSSK GPRY
```

Preferred assays detect both type II cytoskeletal Keratin-1 and type II cytoskeletal Keratin-10 sequences.

As used herein, the term "type II cytoskeletal Keratin-6A/6B/6C" refers to one or more polypeptides present in a biological sample that are derived from the type II cytoskeletal Keratin-6A precursor (Swiss-Prot P02538 (SEQ ID NO: 16))

```
         10         20         30         40         50         60
   MASTSTTIRS HSSSRRGFSA NSARLPGVSR SGFSSVSVSR SRGSGGLGGA CGGAGFGSRS

         70         80         90        100        110        120
   LYGLGGSKRI SIGGGSCAIS GGYGSRAGGS YGFGGAGSGF GFGGGAGIGF GLGGGAGLAG

        130        140        150        160        170        180
   GFGGPGFPVC PPGGIQEVTV NQSLLTPLNL QIDPTIQRVR AEEREQIKTL NNKFASFIDK

        190        200        210        220        230        240
   VRFLEQQNKV LETKWTLLQE QGTKTVRQNL EPLFEQYINN LRRQLDSIVG ERGRLDSELR

        250        260        270        280        290        300
   GMQDLVEDFK NKYEDEINKR TAAENEFVTL KKDVDAAYMN KVELQAKADT LTDEINFLRA

        310        320        330        340        350        360
   LYDAELSQMQ THISDTSVVL SMDNNRNLDL DSIIAEVKAQ YEEIAQRSRA EAESWYQTKY

        370        380        390        400        410        420
   EELQVTAGRH GDDLRNTKQE IAEINRMIQR LRSEIDHVKK QCANLQAAIA DAEQRGEMAL

        430        440        450        460        470        480
   KDAKNKLEGL EDALQKAKQD LARLLKEYQE LMNVKLALDV EIATYRKLLE GEECRLNGEG

        490        500        510        520        530        540
   VGQVNISVVQ STVSSGYGGA SGVGSGLGLG GGSSYSYGSG LGVGGGFSSS SGRAIGGGLS

        550        560
   SVGGGSSTIK YTTTSSSSRK SYKH
```

or from the type II cytoskeletal Keratin-6B precursor (Swiss-Prot P04259 (SEQ ID NO: 17)).

```
            10         20         30         40         50         60
     MASTSTTIRS HSSSRRGFSA NSARLPGVSR SGFSSISVSR SRGSGGLGGA CGGAGFGSRS

            70         80         90        100        110        120
     LYGLGGSKRI SIGGGSCAIS GGYGSRAGGS YGFGGAGSGF GFGGGAGIGF GLGGGAGLAG

           130        140        150        160        170        180
     GFGGPGFPVC PPGGIQEVTV NQSLLTPLNL QIDPAIQRVR AEEREQIKTL NNKFASFIDK

           190        200        210        220        230        240
     VRFLEQQNKV LDTKWTLLQE QGTKTVRQNL EPLFEQYINN LRRQLDNIVG ERGRLDSELR

           250        260        270        280        290        300
     NMQDLVEDLK NKYEDEINKR TAAENEFVTL KKDVDAAYMN KVELQAKADT LTDEINFLRA

           310        320        330        340        350        360
     LYDAELSQMQ THISDTSVVL SMDNNRNLDL DSIIAEVKAQ YEEIAQRSRA EAESWYQTKY

           370        380        390        400        410        420
     EELQITAGRH GDDLRNTKQE IAEINRMIQR LRSEIDHVKK QCANLQAAIA DAEQRGEMAL

           430        440        450        460        470        480
     KDAKNKLEGL EDALQKAKQD LARLLKEYQE LMNVKLALDV EIATYRKLLE GEECRLNGEG

           490        500        510        520        530        540
     VGQVNISVVQ STVSSGYGGA SGVGSGLGLG GGSSYSYGSG LGVGGGFSSS SGRATGGGLS

           550        560
     SVGGGSSTIK YTTTSSSSRK SYKH
```

or from the type II cytoskeletal Keratin-6C precursor (Swiss-Prot P48668 (SEQ ID NO: 18)).

```
            10         20         30         40         50         60
     MASTSTTIRS HSSSRRGFSA NSARLPGVSR SGFSSISVSR SRGSGGLGGA CGGAGFGSRS

            70         80         90        100        110        120
     LYGLGGSKRI SIGGGSCAIS GGYGSRAGGS YGFGGAGSGF GFGGGAGIGF GLGGGAGLAG

           130        140        150        160        170        180
     GFGGPGFPVC PPGGIQEVTV NQSLLTPLNL QIDPAIQRVR AEEREQIKTL NNKFASFIDK

           190        200        210        220        230        240
     VRFLEQQNKV LDTKWTLLQE QGTKTVRQNL EPLFEQYINN LRRQLDSIVG ERGRLDSELR

           250        260        270        280        290        300
     NMQDLVEDLK NKYEDEINKR TAAENEFVTL KKDVDAAYMN KVELQAKADT LTDEINFLRA
```

```
         310        320        330        340        350        360
    LYDAELSQMQ THISDTSVVL SMDNNRNLDL DSIIAEVKAQ YEEIAQRSRA EAESWYQTKY

         370        380        390        400        410        420
    EELQVTAGRH GDDLRNTKQE IAEINRMIQR LRSEIDHVKK QCASLQAAIA DAEQRGEMAL

         430        440        450        460        470        480
    KDAKNKLEGL EDALQKAKQD LARLLKEYQE LMNVKLALDV EIATYRKLLE GEECRLNGEG

         490        500        510        520        530        540
    VGQVNVSVVQ STISSGYGGA SGVGSGLGLG GGSSYSYGSG LGIGGGFSSS SGRAIGGGLS

         550        560
    SVGGGSSTIK YTTTSSSSRK SYKH
```

Preferred assays detect each of the type II cytoskeletal Keratin-6A, -6B, and 6C sequences.
As used herein, the term "Osteocalcin" refers to one or more polypeptides present in a biological sample that are derived from the Osteocalcin precursor (Swiss-Prot P02818 (SEQ ID NO: 19)).

```
          10         20         30         40         50         60
    MRALTLLALL ALAALCIAGQ AGAKPSGAES SKGAAFVSKQ EGSEVVKRPR RYLYQWLGAP

          70         80         90        100
    VPYPDPLEPR REVCELNPDC DELADHIGFQ EAYRRFYGPV
```

The following domains have been identified in Osteocalcin:

| Residues | Length | Domain ID |
|---|---|---|
| 1-23 | 23 | signal sequence |
| 24-51 | 28 | propeptide |
| 52-100 | 49 | mature Osteocalcin |

As used herein, the term "Lipopolysaccharide" refers to a cell wall constituent of gram-negative organisms such as *Escherichia coli, Klebsiella, Proteus or Pseudomonas.* Circulating LPS and, in particular, its constituent lipid A, provokes a wide range of systemic reactions. Activation of circulating monocytes and macrophages leads to release of cytokines such as IL- 6, IL-12, IL-15, IL-18, TNF-$\alpha$, macrophage migration inhibitory factor (MIF), and cytokine-like molecules such as high mobility group B1 (HMGB1), which, in turn activate neutrophils, lymphocytes and vascular endothelium, up-regulate cell adhesion molecules, and induce prostaglandins, nitric oxide synthase and acute- phase proteins. Release of platelet activating factor (PAF), prostaglandins, leukotrienes and thromboxane activates vascular endothelium, regulates vascular tone and activates the extrinsic coagulation cascade. Assays for the measurement of LPS in biological samples, *e.g.,* the Limulus amoebocyte lysate (LAL), are known in the art.
As used herein, the term "Pancreatic prohormone" refers to one or more polypeptides present in a biological sample that are derived from the Pancreatic prohormone precursor (Swiss-Prot P01298 (SEQ ID NO: 20)).

```
          10         20         30         40         50         60
    MAAARLCLSL LLLSTCVALL LQPLLGAQGA PLEPVYPGDN ATPEQMAQYA ADLRRYINML

          70         80         90
    TRPRYGKRHK EDTLAFSEWG SPHAAVPREL SPLDL
```

The following domains have been identified in Pancreatic prohormone:

| Residues | Length | Domain ID |
|---|---|---|
| 1-29 | 29 | signal sequence |

(continued)

| Residues | Length | Domain ID |
|----------|--------|-----------|
| 30-65 | 36 | mature Pancreatic prohormone |
| 69-88 | 20 | Pancreatic icosapeptide |
| 89-95 | 7 | propeptide |

As used herein, the term "Peptide YY" refers to one or more polypeptides present in a biological sample that are derived from the Peptide YY precursor (Swiss-Prot P10082 (SEQ ID NO: 21)).

```
            10          20          30          40          50          60
    MVFVRRPWPA  LTTVLLALLV  CLGALVDAYP  IKPEAPGEDA  SPEELNRYYA  SLRHYLNLVT

            70          80          90
    RQRYGKRDGP  DTLLSKTFFP  DGEDRPVRSR  SEGPDLW
```

The following domains have been identified in Peptide YY:

| Residues | Length | Domain ID |
|----------|--------|-----------|
| 1-28 | 28 | signal sequence |
| 29-64 | 36 | mature Peptide YY |
| 31-64 | 34 | Peptide YY(3-36) |
| 68-97 | 30 | propeptide |
| 91-97 | 7 | missing in isoform 2 |

As used herein, the term "Agouti-related protein" refers to one or more polypeptides present in a biological sample that are derived from the Agouti-related protein precursor (Swiss-Prot 000253 (SEQ ID NO: 22)).

```
            10          20          30          40          50          60
    MLTAAVLSCA  LLLALPATRG  AQMGLAPMEG  IRRPDQALLP  ELPGLGLRAP  LKKTTAEQAE

            70          80          90         100         110         120
    EDLLQEAQAL  AEVLDLQDRE  PRSSRRCVRL  HESCLGQQVP  CCDPCATCYC  RFFNAFCYCR

           130
    KLGTAMNPCS  RT
```

The following domains have been identified in Agouti-related protein:

| Residues | Length | Domain ID |
|----------|--------|-----------|
| 1-20 | 20 | signal sequence |
| 21-132 | 112 | mature Agouti-related protein |

As used herein, the term "Ciliary neurotrophic factor" refers to one or more polypeptides present in a biological sample that are derived from the Ciliary neurotrophic factor precursor (Swiss-Prot P26441 (SEQ ID NO: 23)).

```
         10         20         30         40         50         60
MAFTEHSPLT PHRRDLCSRS IWLARKIRSD LTALTESYVK HQGLNKNINL DSADGMPVAS

         70         80         90        100        110        120
TDQWSELTEA ERLQENLQAY RTFHVLLARL LEDQQVHFTP TEGDFHQAIH TLLLQVAAFA

        130        140        150        160        170        180
YQIEELMILL EYKIPRNEAD GMPINVGDGG LFEKKLWGLK VLQELSQWTV RSIHDLRFIS

        190        200
SHQTGIPARG SHYIANNKKM
```

As used herein, the term "Appetite-regulating hormone" refers to one or more polypeptides present in a biological sample that are derived from the Appetite-regulating hormone precursor (Swiss-Prot Q9UBU3 (SEQ ID NO: 24)).

```
         10         20         30         40         50         60
MPSPGTVCSL LLLGMLWLDL AMAGSSFLSP EHQRVQQRKE SKKPPAKLQP RALAGWLRPE

         70         80         90        100        110
DGGQAEGAED ELEVRFNAPF DVGIKLSGVQ YQQHSQALGK FLQDILWEEA KEAPADK
```

The following domains have been identified in Appetite-regulating hormone:

| Residues | Length | Domain ID |
| --- | --- | --- |
| 1-23 | 23 | signal sequence |
| 24-51 | 28 | Ghrelin-28 |
| 24-50 | 27 | Ghrelin-27 |
| 52-75 | 24 | propeptide |
| 76-98 | 23 | obestatin |
| 99-117 | 19 | propeptide |
| 37 | 1 | missing in isoform 2 |

As used herein, the term "Transthyretin" refers to one or more polypeptides present in a biological sample that are derived from the Transthyretin precursor (Swiss-Prot P02766 (SEQ ID NO: 25)).

```
         10         20         30         40         50         60
MASHRLLLLC LAGLVFVSEA GPTGTGESKC PLMVKVLDAV RGSPAINVAV HVFRKAADDT

         70         80         90        100        110        120
WEPFASGKTS ESGELHGLTT EEEFVEGIYK VEIDTKSYWK ALGISPFHEH AEVVFTANDS

        130        140
GPRRYTIAAL LSPYSYSTTA VVTNPKE
```

The following domains have been identified in Transthyretin:

| Residues | Length | Domain ID |
| --- | --- | --- |
| 1-20 | 20 | signal sequence |
| 21-147 | 127 | mature Transthyretin |

As used herein, the term "Insulin receptor substrate 1" refers to one or more polypeptides present in a biological sample that are derived from the Insulin receptor substrate 1 precursor (Swiss-Prot P35568 (SEQ ID NO: 26)).

```
          1 0         2 0         3 0         4 0         5 0         6 0
MASPPESDGF SDVRKVGYLR KPKSMHKRFF VLRAASEAGG PARLEYYENE KKWRHKSSAP

          7 0         8 0         9 0        10 0        11 0        12 0
KRSIPLESCF NINKRADSKN KHLVALYTRD EHFAIAADSE AEQDSWYQAL LQLHNRAKGH

         13 0        14 0        15 0        16 0        17 0        18 0
HDGAAALGAG GGGGSCSGSS GLGEAGEDLS YGDVPPGPAF KEVWQVILKP KGLGQTKNLI
```

```
        190        200        210        220        230        240
GIYRLCLTSK TISFVKLNSE AAAVVLQLMN IRRCGHSENF FFIEVGRSAV TGPGEFWMQV

        250        260        270        280        290        300
DDSVVAQNMH ETILEAMRAM SDEFRPRSKS QSSSNCSNPI SVPLRRHHLN NPPPSQVGLT

        310        320        330        340        350        360
RRSRTESITA TSPASMVGGK PGSFRVRASS DGEGTMSRPA SVDGSPVSPS TNRTHAHRHR

        370        380        390        400        410        420
GSARLHPPLN HSRSIPMPAS RCSPSATSPV SLSSSSTSGH GSTSDCLFPR RSSASVSGSP

        430        440        450        460        470        480
SDGGFISSDE YGSSPCDFRS SFRSVTPDSL GHTPPARGEE ELSNYICMGG KGPSTLTAPN

        490        500        510        520        530        540
GHYILSRGGN GHRCTPGTGL GTSPALAGDE AASAADLDNR FRKRTHSAGT SPTITHQKTP

        550        560        570        580        590        600
SQSSVASIEE YTEMMPAYPP GGGSGGRLPG HRHSAFVPTR SYPEEGLEMH PLERRGGHHR

        610        620        630        640        650        660
PDSSTLHTDD GYMPMSPGVA PVPSGRKGSG DYMPMSPKSV SAPQQIINPI RRHPQRVDPN

        670        680        690        700        710        720
GYMMMSPSGG CSPDIGGGPS SSSSSSNAVP SGTSYGKLWT NGVGGHHSHV LPHPKPPVES

        730        740        750        760        770        780
SGGKLLPCTG DYMNMSPVGD SNTSSPSDCY YGPEDPQHKP VLSYYSLPRS FKHTQRPGEP

        790        800        810        820        830        840
EEGARHQHLR LSTSSGRLLY AATADDSSSS TSSDSLGGGY CGARLEPSLP HPHHQVLQPH

        850        860        870        880        890        900
LPRKVDTAAQ TNSRLARPTR LSLGDPKAST LPRAREQQQQ QQPLLHPPEP KSPGEYVNIE

        910        920        930        940        950        960
FGSDQSGYLS GPVAFHSSPS VRCPSQLQPA PREEETGTEE YMKMDLGPGR RAAWQESTGV

        970        980        990       1000       1010       1020
EMGRLGPAPP GAASICRPTR AVPSSRGDYM TMQMSCPRQS YVDTSPAAPV SYADMRTGIA

       1030       1040       1050       1060       1070       1080
AEEVSLPRAT MAAASSSSAA SASPTGPQGA AELAAHSSLL GGPQGPGGMS AFTRVNLSPN

       1090       1100       1110       1120       1130       1140
RNQSAKVIRA DPQGCRRHS SETFSSTPSA TRVGNTVPFG AGAAVGGGGG SSSSSEDVKR

       1150       1160       1170       1180       1190       1200
HSSASFENVW LRPGELGGAP KEPAKLCGAA GGLENGLNYI DLDLVKDFKQ CPQECTPEPQ

       1210       1220       1230       1240
PPPPPPPHQP LGSGESSSTR RSSEDLSAYA SISFQKQPED RQ
```

As used herein, the term "and NF-kappa-B inhibitor alpha" refers to one or more polypeptides present in a biological sample that are derived from the precursor (Swiss-Prot P25963 (SEQ ID NO: 27)).

```
         10         20         30         40         50         60
   MFQAAERPQE WAMEGPRDGL KKERLLDDRH DSGLDSMKDE EYEQMVKELQ EIRLEPQEVP

         70         80         90        100        110        120
   RGSEPWKQQL TEDGDSFLHL AIIHEEKALT MEVIRQVKGD LAFLNFQNNL QQTPLHLAVI

        130        140        150        160        170        180
   TNQPEIAEAL LGAGCDPELR DFRGNTPLHL ACEQGCLASV GVLTQSCTTP HLHSILKATN

        190        200        210        220        230        240
   YNGHTCLHLA SIHGYLGIVE LLVSLGADVN AQEPCNGRTA LHLAVDLQNP DLVSLLLKCG

        250        260        270        280        290        300
   ADVNRVTYQG YSPYQLTWGR PSTRIQQQLG QLTLENLQML PESEDEESYD TESEFTEFTE

        310
   DELPYDDCVF GGQRLTL
```

As used herein, the term "relating a signal to the presence or amount" of an analyte reflects the following understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, *etc.*

In this regard, the skilled artisan will understand that the signals obtained from an immunoassay are a direct result of complexes formed between one or more antibodies and the target biomolecule (*i. e.,* the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc.)* and nucleic acid measurements (mRNA quatitation). This list is not meant to be limiting.

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker of the present invention, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994, each of which is hereby incorporated by reference in its entirety, including all tables, figures and claims.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377, each of which is hereby incorporated by reference in its entirety, including all tables, figures and claims. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (e.g., fluorescent moieties, electrochemical labels, metal chelates, *etc.)* as well as molecules that may be indirectly detected by production of a detectable reaction product (*e.g.*, enzymes such as horseradish peroxidase, alkaline phosphatase, *etc.)* or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homo functional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, the present invention provides kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker of the present invention. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ $M^{-1}$, and preferably between about $10^8$ $M^{-1}$ to about $10^9$ $M^{-1}$, about $10^9$ $M^{-1}$ to about $10^{10}$ $M^{-1}$, or about $10^{10}$ $M^{-1}$ to about $10^{12}$ $M^{-1}$.

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098, which is hereby incorporated in its entirety, including all tables, figures, and claims.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs *(e.g.,* in sandwich assays) may interfere with one another sterically, *etc.,* assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection theory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s) of the present invention. These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Ceruloplasmin (P00450); Clusterin (P10909); Complement C3 (P01024); Cysteine-rich protein (CYR61, 000622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-1alpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltransferase (P21673); TGF-Beta1 (P01137); Transferrin (P02787); Trefoil factor 3 (TFF3, Q07654); Toll-Like protein 4 (000206); Total protein; Tubulointerstitial nephritis antigen (Q9UJW2); Uromodulin (Tamm-Horsfall protein, P07911).

For purposes of risk stratification, Adiponectin (Q15848); Alkaline phosphatase (P05186); Aminopeptidase N (P15144); CalbindinD28k (P05937); Cystatin C (P01034); 8 subunit of FIFO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, 000458); Isovaleryl-CoA dehydrogenase (IVD, P26440); I-TAC/CXCL11 (014625); Keratin 19 (P08727); Kim-1 (Hepatitis A virus cellular receptor 1, 043656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (014788); P8 protein (060356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61); RT1.B-1 (alpha) chain of the integral membrane protein (Q5Y7A8); Soluble tumor necrosis factor receptor superfamily member 1A (sTNFR-I, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s) of the present invention.

Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (*e.g.*, blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 $m^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present invention may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:

Inclusion Criteria

males and females 18 years of age or older;

undergoing a radiographic / angiographic procedure (such as a CT scan or coronary intervention) involving the intravascular administration of contrast media;

expected to be hospitalized for at least 48 hours after contrast administration.

able and willing to provide written informed consent for study participation and to comply with all study procedures.

Exclusion Criteria

renal transplant recipients;

acutely worsening renal function prior to the contrast procedure;

already receiving dialysis (either acute or chronic) or in imminent need of dialysis at enrollment;

expected to undergo a major surgical procedure (such as involving cardiopulmonary bypass) or an additional imaging procedure with contrast media with significant risk for further renal insult within the 48 hrs following contrast administration;

participation in an interventional clinical study with an experimental therapy within the previous 30 days;

known infection with human immunodeficiency virus (HIV) or a hepatitis virus.

Immediately prior to the first contrast administration (and after any pre-procedure hydration), an EDTA anticoagulated blood sample (10 mL) and a urine sample (10 mL) are collected from each patient. Blood and urine samples are then collected at 4 ($\pm$0.5), 8 ($\pm$1), 24 ($\pm$2) 48 ($\pm$2), and 72 ($\pm$2) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Serum creatinine is assessed at the site immediately prior to the first contrast administration (after any pre-procedure hydration) and at 4 ($\pm$0.5), 8 ($\pm$1), 24 ($\pm$2) and 48 ($\pm$2)), and 72 ($\pm$2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 m$^2$ = 2 points, 20-40 mL/min/1.73 m$^2$ = 4 points, < 20 mL/min/1.73 m$^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1%, risk of dialysis - 1.09%; >16 total points = risk of CIN - 57.3%, risk of dialysis - 12.8%.

Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:

Inclusion Criteria

males and females 18 years of age or older;

undergoing cardiovascular surgery;

Toronto/Ottawa Predictive Risk Index for Renal Replacement risk score of at least 2 (Wijeysundera et al., JAMA 297: 1801-9, 2007); and

able and willing to provide written informed consent for study participation and to comply with all study procedures.

Exclusion Criteria

known pregnancy;

previous renal transplantation;

acutely worsening renal function prior to enrollment (e.g., any category of RIFLE criteria);

already receiving dialysis (either acute or chronic) or in imminent need of dialysis at enrollment;

currently enrolled in another clinical study or expected to be enrolled in another clinical study within 7 days of cardiac surgery that involves drug infusion or a therapeutic intervention for AKI;

known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 ($\pm 0.5$), 6 ($\pm 0.5$), 12 ($\pm 1$), 24 ($\pm 2$) and 48 ($\pm 2$) hrs following the procedure and then daily on days 3 through 7 if the subject remains in the hospital. Blood is collected via direct venipuncture or via other available venous access, such as an existing femoral sheath, central venous line, peripheral intravenous line or hep-lock. These study blood samples are frozen and shipped to Astute Medical, Inc., San Diego, CA. The study urine samples are frozen and shipped to Astute Medical, Inc.

Example 3: Acutely ill subject sample collection

The objective of this study is to collect samples from acutely ill patients. Approximately 900 adults expected to be in the ICU for at least 48 hours will be enrolled. To be enrolled in the study, each patient must meet all of the following inclusion criteria and none of the following exclusion criteria:

Inclusion Criteria

males and females 18 years of age or older;

Study population 1: approximately 300 patients that have at least one of:

shock (SBP < 90 mmHg and/or need for vasopressor support to maintain MAP > 60 mmHg and/or documented drop in SBP of at least 40 mmHg); and

sepsis;

Study population 2: approximately 300 patients that have at least one of:

IV antibiotics ordered in computerized physician order entry (CPOE) within 24 hours of enrollment;

contrast media exposure within 24 hours of enrollment;

increased Intra-Abdominal Pressure with acute decompensated heart failure; and severe trauma as the primary reason for ICU admission and likely to be hospitalized in the ICU for 48 hours after enrollment;

Study population 3: approximately 300 patients expected to be hospitalized through acute care setting (ICU or ED) with a known risk factor for acute renal injury (e.g. sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment.

Exclusion Criteria

known pregnancy;

institutionalized individuals;

previous renal transplantation;

known acutely worsening renal function prior to enrollment (e.g., any category of RIFLE criteria);

received dialysis (either acute or chronic) within 5 days prior to enrollment or in imminent need of dialysis at the time of enrollment;

known infection with human immunodeficiency virus (HIV) or a hepatitis virus;

meets only the SBP < 90 mmHg inclusion criterion set forth above, and does not have shock in the attending physician's or principal investigator's opinion.

After providing informed consent, an EDTA anti-coagulated blood sample (10 mL) and a urine sample (25-30 mL) are collected from each patient. Blood and urine samples are then collected at 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 4. Immunoassay format

Analytes are measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.

Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

Example 6. Use of Kidney Injury Markers for evaluating renal status in patients

Patients from the intensive care unit (ICU) were enrolled in the following study. Each patient was classified by kidney status as non-injury (0), risk of injury (R), injury (I), and failure (F) according to the maximum stage reached within 7 days of enrollment as determined by the RIFLE criteria. EDTA anti-coagulated blood samples (10 mL) and a urine samples (25-30 mL) were collected from each patient at enrollment, 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Markers were each measured by standard immunoassay methods using commercially available assay reagents in the urine samples and the plasma component of the blood samples collected.

Two cohorts were defined to represent a "diseased" and a "normal" population. While these terms are used for convenience, "diseased" and "normal" simply represent two cohorts for comparison (say RIFLE 0 vs RIFLE R, I and F; RIFLE 0 vs RIFLE R; RIFLE 0 and R vs RIFLE I and F; etc.). The time "prior max stage" represents the time at which a sample is collected, relative to the time a particular patient reaches the lowest disease stage as defined for that cohort, binned into three groups which are +/- 12 hours. For example, "24 hr prior" which uses 0 vs R, I, F as the two cohorts would mean 24 hr (+/- 12 hours) prior to reaching stage R (or I if no sample at R, or F if no sample at R or I).

A receiver operating characteristic (ROC) curve was generated for each biomarker measured and the area under each ROC curve (AUC) is determined. Patients in Cohort 2 were also separated according to the reason for adjudication to cohort 2 as being based on serum creatinine measurements (sCr), being based on urine output (UO), or being based on either serum creatinine measurements or urine output. Using the same example discussed above (0 vs R, I, F), for those patients adjudicated to stage R, I, or F on the basis of serum creatinine measurements alone, the stage 0 cohort may include patients adjudicated to stage R, I, or F on the basis of urine output; for those patients adjudicated to stage R, I, or F on the basis of urine output alone, the stage 0 cohort may include patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, in the data for patients adjudicated on the basis of serum creatinine measurements or urine output, the adjudication method which yielded the most severe RIFLE stage is used.

The ability to distinguish cohort 1 from Cohort 2 was determined using ROC analysis. SE is the standard error of the AUC, n is the number of sample or individual patients ("pts," as indicated). Standard errors are calculated as described in Hanley, J. A., and McNeil, B.J., The meaning and use of the area under a receiver operating characteristic (ROC) curve. Radiology (1982) 143: 29-36; p values are calculated with a two-tailed Z-test. An AUC < 0.5 is indicative of a negative going marker for the comparison, and an AUC > 0.5 is indicative of a positive going marker for the comparison.

Various threshold (or "cutoff") concentrations were selected, and the associated sensitivity and specificity for distinguishing cohort 1 from cohort 2 are determined. OR is the odds ratio calculated for the particular cutoff concentration, and 95% CI is the confidence interval for the odds ratio.

Table 1: Comparison of marker levels in urine samples collected from Cohort 1 (patients that did not progress beyond RIFLE stage 0) and in urine samples collected from subjects at 0, 24 hours, and 48 hours prior to reaching stage R, I or F in Cohort 2.

**Agouti-related protein**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.792 | 0.977 | 0.792 | 0.771 | 0.792 | 1.20 |
| Average | 1.27 | 1.71 | 1.27 | 1.42 | 1.27 | 1.19 |
| Stdev | 2.03 | 2.57 | 2.03 | 2.06 | 2.03 | 0.736 |
| p(t-test) | | 0.075 | | 0.56 | | 0.88 |
| Min | 0.00318 | 0.00745 | 0.00318 | 0.00952 | 0.00318 | 0.141 |
| Max | 20.0 | 20.0 | 20.0 | 14.5 | 20.0 | 2.41 |
| n (Samp) | 237 | 125 | 237 | 82 | 237 | 15 |
| n (Patient) | 109 | 125 | 109 | 82 | 109 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.935 | 0.687 | 0.935 | 0.816 | 0.935 | 1.04 |
| Average | 1.52 | 0.901 | 1.52 | 1.11 | 1.52 | 1.09 |
| Stdev | 2.16 | 0.772 | 2.16 | 1.18 | 2.16 | 0.770 |
| p(t-test) | | 0.077 | | 0.29 | | 0.38 |
| Min | 0.00318 | 0.0333 | 0.00318 | 0.00952 | 0.00318 | 0.141 |
| Max | 20.0 | 3.30 | 20.0 | 6.53 | 20.0 | 3.07 |
| n (Samp) | 592 | 38 | 592 | 31 | 592 | 19 |
| n (Patient) | 229 | 38 | 229 | 31 | 229 | 19 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.747 | 1.21 | 0.747 | 0.762 | 0.747 | 0.736 |
| Average | 1.13 | 1.91 | 1.13 | 1.50 | 1.13 | 1.00 |
| Stdev | 1.81 | 2.68 | 1.81 | 2.09 | 1.81 | 0.763 |
| p(t-test) | | 8.7E-4 | | 0.11 | | 0.78 |
| Min | 0.00198 | 0.00745 | 0.00198 | 0.0261 | 0.00198 | 0.141 |
| Max | 20.0 | 20.0 | 20.0 | 14.5 | 20.0 | 2.41 |
| n (Samp) | 295 | 110 | 295 | 84 | 295 | 16 |
| n (Patient) | 124 | 110 | 124 | 84 | 124 | 16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.58 | 0.42 | 0.65 | 0.54 | 0.46 | 0.57 | 0.59 | 0.49 | 0.54 |
| SE | 0.032 | 0.050 | 0.032 | 0.037 | 0.054 | 0.036 | 0.079 | 0.068 | 0.076 |
| p | 0.012 | 0.095 | 3.7E-6 | 0.31 | 0.49 | 0.043 | 0.27 | 0.86 | 0.56 |
| nCohort 1 | 237 | 592 | 295 | 237 | 592 | 295 | 237 | 592 | 295 |
| nCohort 2 | 125 | 38 | 110 | 82 | 31 | 84 | 15 | 19 | 16 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 1 | 0.481 | 0.379 | 0.694 | 0.469 | 0.599 | 0.488 | 0.617 | 0.420 | 0.378 |
| Sens 1 | 70% | 71% | 70% | 71% | 71% | 70% | 73% | 74% | 75% |
| Spec 1 | 38% | 24% | 48% | 38% | 36% | 41% | 43% | 26% | 35% |
| Cutoff 2 | 0.390 | 0.228 | 0.453 | 0.316 | 0.399 | 0.378 | 0.419 | 0.300 | 0.355 |
| Sens 2 | 80% | 82% | 80% | 80% | 81% | 81% | 80% | 84% | 81% |
| Spec 2 | 33% | 15% | 39% | 30% | 25% | 35% | 34% | 21% | 33% |
| Cutoff 3 | 0.211 | 0.159 | 0.308 | 0.208 | 0.175 | 0.262 | 0.228 | 0.173 | 0.229 |
| Sens 3 | 90% | 92% | 90% | 90% | 90% | 90% | 93% | 95% | 94% |
| Spec 3 | 19% | 11% | 32% | 19% | 12% | 28% | 21% | 12% | 24% |
| Cutoff 4 | 1.37 | 1.59 | 1.26 | 1.37 | 1.59 | 1.26 | 1.37 | 1.59 | 1.26 |
| Sens 4 | 38% | 13% | 47% | 32% | 26% | 35% | 47% | 21% | 31% |
| Spec 4 | 70% | 70% | 70% | 70% | 70% | 70% | 70% | 70% | 70% |
| Cutoff 5 | 1.77 | 2.26 | 1.62 | 1.77 | 2.26 | 1.62 | 1.77 | 2.26 | 1.62 |
| Sens 5 | 29% | 11% | 35% | 23% | 6% | 25% | 33% | 5% | 31% |
| Spec 5 | 80% | 80% | 80% | 80% | 80% | 80% | 80% | 80% | 80% |
| Cutoff 6 | 2.71 | 3.28 | 2.52 | 2.71 | 3.28 | 2.52 | 2.71 | 3.28 | 2.52 |
| Sens 6 | 18% | 3% | 25% | 10% | 3% | 12% | 0% | 0% | 0% |
| Spec 6 | 90% | 90% | 90% | 90% | 90% | 90% | 90% | 90% | 90% |
| OR Quart 2 | 2.4 | 2.1 | 3.5 | 2.9 | 1.4 | 4.8 | 1.5 | 4.7 | 3.1 |
| p Value | 0.0077 | 0.19 | 0.0018 | 0.0056 | 0.56 | 1.4E-4 | 0.65 | 0.050 | 0.17 |
| 95% CI of OR Quart2 | 1.3 / 4.7 | 0.69 / 6.2 | 1.6 / 7.7 | 1.4 / 6.1 | 0.44 / 4.6 | 2.1 / 11 | 0.25 / 9.5 | 1.0 / 22 | 0.61 / 16 |
| OR Quart 3 | 2.1 | 2.3 | 4.4 | 1.3 | 2.5 | 1.9 | 2.6 | 1.5 | 1.5 |
| p Value | 0.033 | 0.13 | 1.7E-4 | 0.56 | 0.090 | 0.14 | 0.26 | 0.65 | 0.66 |
| 95% CI of OR Quart3 | 1.1 / 4.0 | 0.78 / 6.8 | 2.0 / 9.6 | 0.57 / 2.8 | 0.86 / 7.3 | 0.80 / 4.6 | 0.49 / 14 | 0.25 / 9.2 | 0.24 / 9.2 |
| OR Quart 4 | 2.7 | 2.5 | 5.6 | 2.2 | 1.4 | 3.8 | 2.6 | 2.6 | 2.6 |
| p Value | 0.0032 | 0.088 | 9.6E-6 | 0.046 | 0.55 | 0.0016 | 0.26 | 0.26 | 0.27 |
| 95% CI of OR Quart4 | 1.4 / 5.2 | 0.87 / 7.4 | 2.6 / 12 | 1.0 / 4.7 | 0.44 / 4.6 | 1.7 / 8.5 | 0.49 / 14 | 0.49 / 13 | 0.48 / 14 |

## Osteocalcin

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 4920 | 6940 | 4920 | 6040 | 4920 | 4990 |
| Average | 6250 | 8840 | 6250 | 7730 | 6250 | 7600 |
| Stdev | 5910 | 8110 | 5910 | 6950 | 5910 | 6240 |
| p(t-test) |  | 1.6E-5 |  | 0.015 |  | 0.15 |
| Min | 76.8 | 473 | 76.8 | 103 | 76.8 | 520 |
| Max | 83000 | 67900 | 83000 | 49400 | 83000 | 23400 |
| n (Samp) | 385 | 191 | 385 | 144 | 385 | 45 |
| n (Patient) | 191 | 191 | 191 | 144 | 191 | 45 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 6050 | 6400 | 6050 | 5680 | 6050 | 4940 |
| Average | 7660 | 9720 | 7660 | 9050 | 7660 | 7320 |
| Stdev | 7660 | 9080 | 7660 | 9090 | 7660 | 6010 |
| p(t-test) |  | 0.050 |  | 0.20 |  | 0.79 |
| Min | 76.8 | 714 | 76.8 | 584 | 76.8 | 1030 |
| Max | 83500 | 48000 | 83500 | 42300 | 83500 | 23400 |
| n (Samp) | 991 | 58 | 991 | 55 | 991 | 35 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 386 | 58 | 386 | 55 | 386 | 35 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5010 | 7240 | 5010 | 6230 | 5010 | 6500 |
| Average | 6940 | 8760 | 6940 | 7940 | 6940 | 8790 |
| Stdev | 7020 | 7510 | 7020 | 7160 | 7020 | 8580 |
| p(t-test) | | 0.0044 | | 0.15 | | 0.11 |
| Min | 73.4 | 473 | 73.4 | 103 | 73.4 | 475 |
| Max | 83000 | 67900 | 83000 | 49400 | 83000 | 42300 |
| n (Samp) | 456 | 174 | 456 | 136 | 456 | 42 |
| n (Patient) | 202 | 174 | 202 | 136 | 202 | 42 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.55 | 0.61 | 0.57 | 0.51 | 0.56 | 0.54 | 0.47 | 0.54 |
| SE | 0.025 | 0.040 | 0.026 | 0.028 | 0.040 | 0.029 | 0.046 | 0.050 | 0.047 |
| p | 6.7E-6 | 0.20 | 4.3E-5 | 0.022 | 0.85 | 0.035 | 0.39 | 0.61 | 0.43 |
| nCohort 1 | 385 | 991 | 456 | 385 | 991 | 456 | 385 | 991 | 456 |
| nCohort 2 | 191 | 58 | 174 | 144 | 55 | 136 | 45 | 35 | 42 |
| Cutoff 1 | 4440 | 4190 | 4770 | 3480 | 3200 | 4120 | 3270 | 3320 | 3200 |
| Sens 1 | 70% | 71% | 70% | 70% | 71% | 71% | 71% | 71% | 71% |
| Spec 1 | 45% | 33% | 46% | 33% | 23% | 38% | 30% | 24% | 28% |
| Cutoff 2 | 3350 | 3110 | 3350 | 2710 | 2290 | 2980 | 3000 | 3080 | 2430 |
| Sens 2 | 80% | 81% | 80% | 81% | 80% | 80% | 80% | 80% | 81% |
| Spec 2 | 31% | 22% | 29% | 24% | 14% | 26% | 28% | 21% | 20% |
| Cutoff 3 | 2180 | 1810 | 2600 | 1620 | 1330 | 1820 | 1030 | 2610 | 1030 |
| Sens 3 | 90% | 91% | 90% | 90% | 91% | 90% | 91% | 91% | 90% |
| Spec 3 | 17% | 9% | 21% | 11% | 6% | 12% | 5% | 16% | 5% |
| Cutoff 4 | 7680 | 8750 | 7910 | 7680 | 8750 | 7910 | 7680 | 8750 | 7910 |
| Sens 4 | 44% | 40% | 44% | 40% | 38% | 42% | 38% | 26% | 43% |
| Spec 4 | 70% | 70% | 70% | 70% | 70% | 70% | 70% | 70% | 70% |
| Cutoff 5 | 8960 | 10400 | 9630 | 8960 | 10400 | 9630 | 8960 | 10400 | 9630 |
| Sens 5 | 35% | 36% | 32% | 32% | 33% | 29% | 31% | 20% | 36% |
| Spec 5 | 80% | 80% | 80% | 80% | 80% | 80% | 80% | 80% | 80% |
| Cutoff 6 | 11300 | 14300 | 13600 | 11300 | 14300 | 13600 | 11300 | 14300 | 13600 |
| Sens 6 | 25% | 21% | 18% | 20% | 18% | 11% | 29% | 14% | 24% |
| Spec 6 | 90% | 90% | 90% | 90% | 90% | 90% | 90% | 90% | 90% |
| OR Quart 2 | 1.3 | 1.0 | 1.2 | 0.96 | 0.63 | 1.0 | 1.5 | 0.88 | 0.99 |
| p Value | 0.28 | 1.0 | 0.60 | 0.88 | 0.24 | 0.88 | 0.38 | 0.80 | 0.99 |
| 95% CI of OR Quart2 | 0.79 2.3 | 0.47 2.1 | 0.67 2.0 | 0.54 1.7 | 0.29 1.4 | 0.59 1.9 | 0.61 3.6 | 0.31 2.4 | 0.40 2.5 |
| OR Quart 3 | 1.8 | 0.56 | 2.0 | 1.1 | 0.51 | 1.2 | 0.88 | 1.1 | 0.58 |
| p Value | 0.028 | 0.20 | 0.0073 | 0.67 | 0.11 | 0.47 | 0.80 | 0.81 | 0.31 |
| 95% CI of OR Quart3 | 1.1 3.0 | 0.23 1.4 | 1.2 3.4 | 0.65 2.0 | 0.22 1.2 | 0.70 2.2 | 0.33 2.4 | 0.43 3.0 | 0.20 1.6 |
| OR Quart 4 | 2.9 | 1.6 | 2.5 | 1.9 | 1.1 | 1.9 | 1.8 | 1.4 | 1.7 |
| p Value | 3.4E-5 | 0.17 | 4.0E-4 | 0.019 | 0.87 | 0.017 | 0.21 | 0.48 | 0.23 |
| 95% CI of OR Quart4 | 1.8 4.9 | 0.81 3.2 | 1.5 4.2 | 1.1 3.2 | 0.53 2.1 | 1.1 3.3 | 0.73 4.2 | 0.55 3.5 | 0.73 3.8 |

## Complement factor H

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.47 | 8.09 | 9.47 | 9.33 | 9.47 | 5.77 |
| Average | 17.9 | 16.1 | 17.9 | 19.8 | 17.9 | 13.7 |
| Stdev | 27.2 | 26.7 | 27.2 | 30.4 | 27.2 | 17.5 |
| p(t-test) | | 0.54 | | 0.54 | | 0.36 |
| Min | 0.132 | 0.0818 | 0.132 | 0.133 | 0.132 | 0.0620 |
| Max | 282 | 223 | 282 | 208 | 282 | 67.2 |
| n (Samp) | 271 | 140 | 271 | 107 | 271 | 38 |
| n (Patient) | 151 | 140 | 151 | 107 | 151 | 38 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.31 | 10.7 | 9.31 | 9.05 | 9.31 | 10.3 |
| Average | 18.1 | 19.3 | 18.1 | 17.7 | 18.1 | 15.3 |
| Stdev | 28.2 | 25.3 | 28.2 | 23.5 | 28.2 | 15.6 |
| p(t-test) | | 0.79 | | 0.93 | | 0.63 |
| Min | 0.0620 | 1.20 | 0.0620 | 1.53 | 0.0620 | 0.832 |
| Max | 282 | 142 | 282 | 97.5 | 282 | 67.2 |
| n (Samp) | 667 | 40 | 667 | 39 | 667 | 24 |
| n (Patient) | 290 | 40 | 290 | 39 | 290 | 24 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.0 | 7.77 | 10.0 | 9.03 | 10.0 | 4.98 |
| Average | 20.5 | 15.0 | 20.5 | 18.2 | 20.5 | 11.1 |
| Stdev | 49.9 | 26.9 | 49.9 | 29.2 | 49.9 | 13.9 |
| p(t-test) | | 0.25 | | 0.67 | | 0.27 |
| Min | 0.132 | 0.0818 | 0.132 | 0.133 | 0.132 | 0.0620 |
| Max | 772 | 223 | 772 | 208 | 772 | 59.1 |
| n (Samp) | 316 | 127 | 316 | 104 | 316 | 35 |
| n (Patient) | 155 | 127 | 155 | 104 | 155 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.55 | 0.44 | 0.49 | 0.49 | 0.46 | 0.40 | 0.51 | 0.36 |
| SE | 0.030 | 0.048 | 0.031 | 0.033 | 0.048 | 0.033 | 0.051 | 0.060 | 0.053 |
| p | 0.30 | 0.30 | 0.048 | 0.77 | 0.81 | 0.24 | 0.052 | 0.85 | 0.0075 |
| nCohort 1 | 271 | 667 | 316 | 271 | 667 | 316 | 271 | 667 | 316 |
| nCohort 2 | 140 | 40 | 127 | 107 | 39 | 104 | 38 | 24 | 35 |
| Cutoff 1 | 5.87 | 6.66 | 5.86 | 5.67 | 5.87 | 4.62 | 3.03 | 5.59 | 3.03 |
| Sens 1 | 70% | 70% | 70% | 70% | 72% | 70% | 71% | 71% | 71% |
| Spec 1 | 28% | 36% | 26% | 25% | 29% | 20% | 13% | 27% | 12% |
| Cutoff 2 | 4.41 | 6.00 | 4.11 | 3.61 | 3.03 | 3.33 | 2.30 | 4.19 | 2.34 |
| Sens 2 | 80% | 80% | 80% | 80% | 82% | 81% | 82% | 83% | 80% |
| Spec 2 | 20% | 30% | 17% | 15% | 13% | 13% | 11% | 20% | 10% |
| Cutoff 3 | 2.81 | 3.94 | 2.81 | 2.52 | 2.10 | 2.31 | 1.69 | 3.30 | 1.70 |
| Sens 3 | 90% | 90% | 91% | 91% | 92% | 90% | 92% | 92% | 91% |
| Spec 3 | 12% | 18% | 11% | 11% | 9% | 10% | 8% | 14% | 7% |
| Cutoff 4 | 17.2 | 16.4 | 18.1 | 17.2 | 16.4 | 18.1 | 17.2 | 16.4 | 18.1 |
| Sens 4 | 25% | 38% | 21% | 27% | 26% | 23% | 26% | 33% | 23% |
| Spec 4 | 70% | 70% | 70% | 70% | 70% | 70% | 70% | 70% | 70% |
| Cutoff 5 | 25.1 | 23.6 | 24.8 | 25.1 | 23.6 | 24.8 | 25.1 | 23.6 | 24.8 |
| Sens 5 | 16% | 20% | 14% | 18% | 18% | 16% | 16% | 17% | 9% |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Spec 5 | 80% | 80% | 80% | 80% | 80% | 80% | 80% | 80% | 80% |
| Cutoff 6 | 38.0 | 40.5 | 41.6 | 38.0 | 40.5 | 41.6 | 38.0 | 40.5 | 41.6 |
| Sens 6 | 9% | 10% | 4% | 14% | 13% | 11% | 13% | 8% | 6% |
| Spec 6 | 90% | 90% | 90% | 90% | 90% | 90% | 90% | 90% | 90% |
| OR Quart 2 | 0.91 | 1.3 | 1.1 | 0.87 | 1.1 | 1.5 | 0.53 | 0.65 | 1.2 |
| p Value | 0.77 | 0.62 | 0.75 | 0.66 | 0.80 | 0.25 | 0.28 | 0.52 | 0.76 |
| 95% CI of | 0.51 | 0.47 | 0.60 | 0.46 | 0.45 | 0.76 | 0.17 | 0.18 | 0.36 |
| OR Quart2 | 1.6 | 3.6 | 2.0 | 1.6 | 2.8 | 2.8 | 1.7 | 2.4 | 4.1 |
| OR Quart 3 | 1.2 | 1.6 | 1.6 | 0.81 | 1.1 | 1.1 | 0.77 | 1.2 | 1.2 |
| p Value | 0.56 | 0.34 | 0.14 | 0.52 | 0.81 | 0.86 | 0.62 | 0.79 | 0.76 |
| 95% CI of | 0.67 | 0.61 | 0.87 | 0.43 | 0.44 | 0.54 | 0.27 | 0.38 | 0.36 |
| OR Quart3 | 2.1 | 4.2 | 2.8 | 1.5 | 2.8 | 2.1 | 2.2 | 3.5 | 4.1 |
| OR Quart 4 | 1.1 | 1.9 | 1.7 | 1.1 | 1.1 | 1.8 | 2.2 | 1.2 | 4.3 |
| p Value | 0.73 | 0.18 | 0.094 | 0.72 | 0.80 | 0.060 | 0.084 | 0.79 | 0.0058 |
| 95% CI of | 0.62 | 0.74 | 0.92 | 0.60 | 0.45 | 0.98 | 0.90 | 0.38 | 1.5 |
| OR Quart4 | 2.0 | 4.9 | 3.0 | 2.1 | 2.8 | 3.4 | 5.2 | 3.5 | 12 |

**Ciliary neurotrophic factor**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.147 | 0.0325 | 0.147 | 0.0688 | 0.147 | 0.0551 |
| Average | 0.200 | 0.196 | 0.200 | 0.384 | 0.200 | 0.136 |
| Stdev | 0.278 | 0.594 | 0.278 | 1.56 | 0.278 | 0.166 |
| p(t-test) |  | 0.95 |  | 0.081 |  | 0.39 |
| Min | 0.000103 | 0.000103 | 0.000103 | 0.000103 | 0.000103 | 0.000103 |
| Max | 2.35 | 6.11 | 2.35 | 14.0 | 2.35 | 0.551 |
| n (Samp) | 237 | 125 | 237 | 82 | 237 | 15 |
| n (Patient) | 109 | 125 | 109 | 82 | 109 | 15 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0452 | 0.0765 | 0.0452 | 0.0678 | 0.0452 | 0.000166 |
| Average | 0.221 | 0.253 | 0.221 | 0.226 | 0.221 | 0.106 |
| Stdev | 0.743 | 0.502 | 0.743 | 0.406 | 0.743 | 0.151 |
| p(t-test) |  | 0.79 |  | 0.97 |  | 0.50 |
| Min | 0.000103 | 0.000103 | 0.000103 | 0.000118 | 0.000103 | 0.000119 |
| Max | 14.0 | 2.28 | 14.0 | 2.11 | 14.0 | 0.403 |
| n (Samp) | 592 | 38 | 592 | 31 | 592 | 19 |
| n (Patient) | 229 | 38 | 229 | 31 | 229 | 19 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0876 | 0.0260 | 0.0876 | 0.0698 | 0.0876 | 0.101 |
| Average | 0.209 | 0.225 | 0.209 | 0.392 | 0.209 | 0.178 |
| Stdev | 0.383 | 0.660 | 0.383 | 1.54 | 0.383 | 0.203 |
| p(t-test) |  | 0.77 |  | 0.065 |  | 0.75 |
| Min | 0.000103 | 0.000103 | 0.000103 | 0.000103 | 0.000103 | 0.000103 |
| Max | 4.30 | 6.11 | 4.30 | 14.0 | 4.30 | 0.609 |
| n (Samp) | 295 | 110 | 295 | 84 | 295 | 16 |
| n (Patient) | 124 | 110 | 124 | 84 | 124 | 16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.43 | 0.54 | 0.44 | 0.51 | 0.55 | 0.53 | 0.45 | 0.44 | 0.53 |
| SE | 0.032 | 0.049 | 0.033 | 0.037 | 0.055 | 0.036 | 0.079 | 0.069 | 0.075 |
| p | 0.027 | 0.42 | 0.082 | 0.89 | 0.32 | 0.34 | 0.53 | 0.39 | 0.69 |
| nCohort 1 | 237 | 592 | 295 | 237 | 592 | 295 | 237 | 592 | 295 |
| nCohort 2 | 125 | 38 | 110 | 82 | 31 | 84 | 15 | 19 | 16 |
| Cutoff 1 | 0.000123 | 0.00333 | 0.000123 | 0.000167 | 0.000161 | 0.00333 | 0.00333 | 0.000121 | 0.00333 |
| Sens 1 | 74% | 71% | 73% | 71% | 77% | 70% | 73% | 74% | 75% |
| Spec 1 | 20% | 41% | 23% | 28% | 30% | 34% | 30% | 22% | 34% |
| Cutoff 2 | 0.000121 | 0.000123 | 0.000119 | 0.000123 | 0.000123 | 0.000123 | 0.000123 | 0.000119 | 0.000167 |
| Sens 2 | 80% | 82% | 85% | 80% | 84% | 82% | 80% | 84% | 81% |
| Spec 2 | 17% | 27% | 14% | 20% | 27% | 23% | 20% | 18% | 31% |
| Cutoff 3 | 0.000103 | 0.000103 | 0.000103 | 0.000119 | 0.000119 | 0.000118 | 0.000121 | 0.000118 | 0.000121 |
| Sens 3 | 92% | 92% | 94% | 90% | 94% | 90% | 93% | 100% | 94% |
| Spec 3 | 3% | 5% | 3% | 14% | 18% | 7% | 17% | 11% | 18% |
| Cutoff 4 | 0.290 | 0.229 | 0.245 | 0.290 | 0.229 | 0.245 | 0.290 | 0.229 | 0.245 |
| Sens 4 | 21% | 26% | 22% | 33% | 29% | 40% | 20% | 21% | 25% |
| Spec 4 | 71% | 70% | 71% | 71% | 70% | 71% | 71% | 70% | 71% |
| Cutoff 5 | 0.348 | 0.327 | 0.348 | 0.348 | 0.327 | 0.348 | 0.348 | 0.327 | 0.348 |
| Sens 5 | 19% | 18% | 20% | 26% | 23% | 29% | 13% | 21% | 25% |
| Spec 5 | 81% | 80% | 81% | 81% | 80% | 81% | 81% | 80% | 81% |
| Cutoff 6 | 0.457 | 0.433 | 0.459 | 0.457 | 0.433 | 0.459 | 0.457 | 0.433 | 0.459 |
| Sens 6 | 9% | 11% | 11% | 12% | 16% | 14% | 7% | 0% | 12% |
| Spec 6 | 91% | 90% | 92% | 91% | 90% | 92% | 91% | 90% | 92% |
| OR Quart 2 | 1.0 | 1.4 | 0.96 | 1.0 | 2.6 | 1.1 | 1.4 | 0.74 | 1.7 |
| p Value | 0.96 | 0.46 | 0.90 | 0.90 | 0.12 | 0.76 | 0.70 | 0.70 | 0.48 |
| 95% CI of OR Quart2 | 0.53 1.9 | 0.54 3.9 | 0.49 1.9 | 0.52 2.1 | 0.79 8.4 | 0.57 2.2 | 0.29 6.3 | 0.16 3.4 | 0.39 7.3 |
| OR Quart 3 | 1.6 | 1.8 | 1.7 | 0.55 | 2.0 | 0.60 | 1.4 | 1.0 | 1.3 |
| p Value | 0.16 | 0.24 | 0.080 | 0.12 | 0.25 | 0.18 | 0.70 | 1.0 | 0.71 |
| 95% CI of OR Quart3 | 0.84 2.9 | 0.68 4.6 | 0.94 3.2 | 0.26 1.2 | 0.60 6.9 | 0.28 1.3 | 0.29 6.3 | 0.25 4.1 | 0.29 6.2 |
| OR Quart 4 | 1.8 | 1.3 | 1.5 | 1.0 | 2.3 | 1.3 | 1.4 | 2.1 | 1.3 |
| p Value | 0.056 | 0.62 | 0.19 | 0.90 | 0.17 | 0.42 | 0.70 | 0.24 | 0.71 |
| 95% CI of OR Quart4 | 0.98 3.4 | 0.47 3.6 | 0.81 2.9 | 0.52 2.1 | 0.70 7.7 | 0.68 2.5 | 0.29 6.3 | 0.61 7.0 | 0.29 6.2 |

**Follitropin subunit beta**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 13.7 | 12.2 | 13.7 | 18.7 | 13.7 | 5.88 |
| Average | 31.7 | 23.9 | 31.7 | 30.9 | 31.7 | 33.3 |
| Stdev | 41.9 | 34.4 | 41.9 | 40.4 | 41.9 | 46.9 |
| p(t-test) | | 0.073 | | 0.87 | | 0.89 |
| Min | 0.0800 | 0.209 | 0.0800 | 0.385 | 0.0800 | 0.474 |
| Max | 200 | 256 | 200 | 239 | 200 | 128 |
| n (Samp) | 237 | 125 | 237 | 82 | 237 | 15 |
| n (Patient) | 109 | 125 | 109 | 82 | 109 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 13.6 | 7.94 | 13.6 | 10.5 | 13.6 | 7.46 |
| Average | 31.4 | 18.7 | 31.4 | 22.1 | 31.4 | 19.4 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 43.1 | 28.6 | 43.1 | 27.3 | 43.1 | 28.3 |
| p(t-test) | | 0.076 | | 0.24 | | 0.23 |
| Min | 0.0800 | 0.209 | 0.0800 | 0.838 | 0.0800 | 0.474 |
| Max | 300 | 158 | 300 | 108 | 300 | 108 |
| n (Samp) | 592 | 38 | 592 | 31 | 592 | 19 |
| n (Patient) | 229 | 38 | 229 | 31 | 229 | 19 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 11.7 | 13.4 | 11.7 | 18.8 | 11.7 | 24.2 |
| Average | 28.2 | 25.1 | 28.2 | 32.6 | 28.2 | 39.2 |
| Stdev | 39.2 | 34.5 | 39.2 | 41.8 | 39.2 | 44.2 |
| p(t-test) | | 0.46 | | 0.37 | | 0.28 |
| Min | 0.0800 | 0.345 | 0.0800 | 0.385 | 0.0800 | 0.209 |
| Max | 200 | 256 | 200 | 239 | 200 | 128 |
| n (Samp) | 295 | 110 | 295 | 84 | 295 | 16 |
| n (Patient) | 124 | 110 | 124 | 84 | 124 | 16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.40 | 0.53 | 0.52 | 0.44 | 0.58 | 0.44 | 0.40 | 0.57 |
| SE | 0.032 | 0.050 | 0.033 | 0.037 | 0.055 | 0.036 | 0.079 | 0.070 | 0.076 |
| p | 0.29 | 0.040 | 0.39 | 0.54 | 0.27 | 0.027 | 0.41 | 0.14 | 0.37 |
| nCohort 1 | 237 | 592 | 295 | 237 | 592 | 295 | 237 | 592 | 295 |
| nCohort 2 | 125 | 38 | 110 | 82 | 31 | 84 | 15 | 19 | 16 |
| Cutoff 1 | 6.94 | 4.89 | 7.15 | 8.26 | 5.33 | 9.43 | 3.42 | 3.76 | 7.66 |
| Sens 1 | 70% | 71% | 70% | 71% | 71% | 70% | 73% | 74% | 75% |
| Spec 1 | 30% | 23% | 39% | 34% | 25% | 47% | 19% | 17% | 40% |
| Cutoff 2 | 4.75 | 3.19 | 5.43 | 4.89 | 2.59 | 6.22 | 1.86 | 1.86 | 5.45 |
| Sens 2 | 80% | 82% | 80% | 80% | 81% | 81% | 80% | 84% | 81% |
| Spec 2 | 24% | 14% | 31% | 25% | 12% | 34% | 11% | 8% | 32% |
| Cutoff 3 | 2.07 | 0.903 | 3.09 | 3.48 | 1.86 | 4.42 | 0.570 | 0.903 | 0.329 |
| Sens 3 | 90% | 92% | 90% | 90% | 90% | 90% | 93% | 95% | 94% |
| Spec 3 | 11% | 2% | 20% | 19% | 8% | 28% | 1% | 2% | 1% |
| Cutoff 4 | 32.5 | 31.3 | 28.3 | 32.5 | 31.3 | 28.3 | 32.5 | 31.3 | 28.3 |
| Sens 4 | 22% | 18% | 25% | 28% | 23% | 33% | 27% | 11% | 50% |
| Spec 4 | 70% | 70% | 70% | 70% | 70% | 70% | 70% | 70% | 70% |
| Cutoff 5 | 51.3 | 47.1 | 44.7 | 51.3 | 47.1 | 44.7 | 51.3 | 47.1 | 44.7 |
| Sens 5 | 10% | 11% | 15% | 17% | 16% | 19% | 20% | 11% | 25% |
| Spec 5 | 80% | 80% | 80% | 80% | 80% | 80% | 80% | 80% | 80% |
| Cutoff 6 | 80.6 | 82.6 | 74.5 | 80.6 | 82.6 | 74.5 | 80.6 | 82.6 | 74.5 |
| Sens 6 | 6% | 3% | 8% | 10% | 6% | 13% | 20% | 5% | 19% |
| Spec 6 | 90% | 90% | 90% | 90% | 90% | 90% | 90% | 90% | 90% |
| OR Quart 2 | 1.9 | 1.4 | 2.5 | 1.2 | 1.2 | 3.0 | 0.48 | 3.6 | 0.99 |
| p Value | 0.037 | 0.58 | 0.0067 | 0.60 | 0.78 | 0.0093 | 0.41 | 0.11 | 0.99 |
| 95% CI of OR Quart2 | 1.0 – 3.7 | 0.46 – 4.0 | 1.3 – 4.9 | 0.58 – 2.5 | 0.39 – 3.6 | 1.3 – 6.9 | 0.085 – 2.7 | 0.74 – 18 | 0.19 – 5.0 |
| OR Quart 3 | 1.7 | 2.1 | 2.4 | 1.7 | 1.5 | 4.1 | 0.74 | 1.5 | 1.7 |
| p Value | 0.11 | 0.15 | 0.010 | 0.17 | 0.43 | 6.1E-4 | 0.70 | 0.65 | 0.48 |
| 95% CI of OR Quart3 | 0.89 – 3.1 | 0.76 – 5.7 | 1.2 – 4.7 | 0.81 – 3.4 | 0.53 – 4.4 | 1.8 – 9.4 | 0.16 – 3.4 | 0.25 – 9.2 | 0.39 – 7.3 |
| OR Quart 4 | 1.4 | 2.1 | 1.7 | 1.2 | 1.5 | 3.0 | 1.6 | 3.6 | 1.7 |
| p Value | 0.31 | 0.15 | 0.13 | 0.60 | 0.42 | 0.0093 | 0.51 | 0.11 | 0.48 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.74 | 0.77 | 0.85 | 0.58 | 0.54 | 1.3 | 0.42 | 0.74 | 0.39 |
| OR Quart4 | 2.6 | 5.7 | 3.4 | 2.5 | 4.4 | 6.9 | 5.8 | 18 | 7.3 |

**Follistatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.38 | 8.68 | 6.38 | 8.68 | 6.38 | 8.40 |
| Average | 44.7 | 15.3 | 44.7 | 28.4 | 44.7 | 15.9 |
| Stdev | 382 | 22.7 | 382 | 80.0 | 382 | 25.4 |
| p(t-test) | | 0.33 | | 0.62 | | 0.58 |
| Min | 0.0191 | 0.0191 | 0.0191 | 0.0191 | 0.0191 | 0.0191 |
| Max | 8290 | 217 | 8290 | 840 | 8290 | 160 |
| n (Samp) | 509 | 162 | 509 | 141 | 509 | 53 |
| n (Patient) | 242 | 162 | 242 | 141 | 242 | 53 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 7.02 | 8.42 | 7.02 | 10.5 | 7.02 | 6.69 |
| Average | 29.2 | 19.0 | 29.2 | 24.7 | 29.2 | 27.1 |
| Stdev | 256 | 33.0 | 256 | 46.1 | 256 | 49.5 |
| p(t-test) | | 0.78 | | 0.90 | | 0.96 |
| Min | 0.000347 | 0.0425 | 0.000347 | 0.0191 | 0.000347 | 0.0191 |
| Max | 8290 | 217 | 8290 | 310 | 8290 | 225 |
| n (Samp) | 1153 | 51 | 1153 | 50 | 1153 | 33 |
| n (Patient) | 423 | 51 | 423 | 50 | 423 | 33 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.65 | 9.29 | 6.65 | 9.13 | 6.65 | 9.05 |
| Average | 44.1 | 29.2 | 44.1 | 38.5 | 44.1 | 14.0 |
| Stdev | 379 | 166 | 379 | 136 | 379 | 15.8 |
| p(t-test) | | 0.64 | | 0.87 | | 0.58 |
| Min | 0.00280 | 0.0191 | 0.00280 | 0.0412 | 0.00280 | 0.0287 |
| Max | 8290 | 2010 | 8290 | 1300 | 8290 | 65.1 |
| n (Samp) | 517 | 147 | 517 | 136 | 517 | 48 |
| n (Patient) | 205 | 147 | 205 | 136 | 205 | 48 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.56 | 0.54 | 0.55 | 0.58 | 0.55 | 0.52 | 0.52 | 0.52 |
| SE | 0.026 | 0.042 | 0.027 | 0.028 | 0.043 | 0.028 | 0.042 | 0.052 | 0.044 |
| p | 0.17 | 0.16 | 0.16 | 0.094 | 0.072 | 0.100 | 0.58 | 0.69 | 0.65 |
| nCohort 1 | 509 | 1153 | 517 | 509 | 1153 | 517 | 509 | 1153 | 517 |
| nCohort 2 | 162 | 51 | 147 | 141 | 50 | 136 | 53 | 33 | 48 |
| Cutoff 1 | 4.89 | 5.33 | 5.23 | 3.57 | 4.60 | 3.83 | 3.21 | 2.87 | 3.93 |
| Sens 1 | 70% | 71% | 70% | 70% | 70% | 71% | 72% | 73% | 71% |
| Spec 1 | 40% | 41% | 40% | 31% | 36% | 31% | 28% | 25% | 32% |
| Cutoff 2 | 2.37 | 3.83 | 2.25 | 2.49 | 3.09 | 2.49 | 2.12 | 1.55 | 2.12 |
| Sens 2 | 80% | 80% | 80% | 80% | 80% | 80% | 81% | 82% | 81% |
| Spec 2 | 22% | 31% | 18% | 23% | 26% | 21% | 20% | 14% | 17% |
| Cutoff 3 | 1.15 | 2.74 | 1.000 | 0.386 | 1.55 | 0.386 | 1.000 | 1.02 | 1.13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 90% | 90% | 90% | 90% | 90% | 90% | 91% | 91% | 92% |
| Spec 3 | 10% | 24% | 8% | 7% | 14% | 6% | 9% | 10% | 9% |
| Cutoff 4 | 12.5 | 12.9 | 13.4 | 12.5 | 12.9 | 13.4 | 12.5 | 12.9 | 13.4 |
| Sens 4 | 33% | 33% | 33% | 40% | 44% | 42% | 34% | 42% | 31% |
| Spec 4 | 70% | 70% | 70% | 70% | 70% | 70% | 70% | 70% | 70% |
| Cutoff 5 | 22.1 | 21.0 | 23.7 | 22.1 | 21.0 | 23.7 | 22.1 | 21.0 | 23.7 |
| Sens 5 | 19% | 25% | 20% | 26% | 32% | 25% | 21% | 27% | 17% |
| Spec 5 | 80% | 80% | 80% | 80% | 80% | 80% | 80% | 80% | 80% |
| Cutoff 6 | 47.8 | 41.3 | 45.9 | 47.8 | 41.3 | 45.9 | 47.8 | 41.3 | 45.9 |
| Sens 6 | 9% | 12% | 11% | 13% | 20% | 15% | 8% | 15% | 6% |
| Spec 6 | 90% | 90% | 90% | 90% | 90% | 90% | 90% | 90% | 90% |
| OR Quart 2 | 0.80 | 2.5 | 0.69 | 0.84 | 1.2 | 0.60 | 0.47 | 0.69 | 0.36 |
| p Value | 0.40 | 0.044 | 0.20 | 0.55 | 0.66 | 0.084 | 0.12 | 0.46 | 0.060 |
| 95% CI of OR Quart2 | 0.47 | 1.0 | 0.39 | 0.48 | 0.50 | 0.34 | 0.18 | 0.26 | 0.13 |
| | 1.4 | 6.2 | 1.2 | 1.5 | 3.0 | 1.1 | 1.2 | 1.8 | 1.0 |
| OR Quart 3 | 1.5 | 1.6 | 1.5 | 1.2 | 1.3 | 0.96 | 1.1 | 0.49 | 1.3 |
| p Value | 0.089 | 0.34 | 0.098 | 0.59 | 0.51 | 0.89 | 0.84 | 0.20 | 0.56 |
| 95% CI of OR Quart3 | 0.94 | 0.61 | 0.92 | 0.68 | 0.56 | 0.57 | 0.50 | 0.17 | 0.58 |
| | 2.5 | 4.2 | 2.5 | 2.0 | 3.2 | 1.6 | 2.3 | 1.5 | 2.7 |
| OR Quart 4 | 1.2 | 2.4 | 1.3 | 1.5 | 2.1 | 1.3 | 1.2 | 1.1 | 1.1 |
| p Value | 0.46 | 0.063 | 0.36 | 0.097 | 0.084 | 0.26 | 0.58 | 0.83 | 0.85 |
| 95% CI of OR Quart4 | 0.73 | 0.96 | 0.76 | 0.92 | 0.91 | 0.81 | 0.58 | 0.46 | 0.49 |
| | 2.0 | 5.8 | 2.1 | 2.6 | 4.7 | 2.2 | 2.6 | 2.6 | 2.4 |

**Vitamin D-binding protein**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 152 | 258 | 152 | 272 | 152 | 159 |
| Average | 5740 | 15900 | 5740 | 11100 | 5740 | 778 |
| Stdev | 33600 | 52300 | 33600 | 46600 | 33600 | 1650 |
| p(t-test) | | 0.036 | | 0.28 | | 0.58 |
| Min | 1.43 | 0.430 | 1.43 | 5.41 | 1.43 | 0.612 |
| Max | 352000 | 379000 | 352000 | 365000 | 352000 | 5950 |
| n (Samp) | 203 | 113 | 203 | 78 | 203 | 14 |
| n (Patient) | 97 | 113 | 97 | 78 | 97 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 208 | 306 | 208 | 342 | 208 | 176 |
| Average | 7170 | 3800 | 7170 | 20900 | 7170 | 28700 |
| Stdev | 33700 | 10200 | 33700 | 77400 | 33700 | 92200 |
| p(t-test) | | 0.58 | | 0.066 | | 0.029 |
| Min | 0.892 | 0.430 | 0.892 | 1.50 | 0.892 | 0.612 |
| Max | 365000 | 43100 | 365000 | 379000 | 365000 | 347000 |
| n (Samp) | 505 | 31 | 505 | 26 | 505 | 14 |
| n (Patient) | 210 | 31 | 210 | 26 | 210 | 14 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 164 | 261 | 164 | 283 | 164 | 276 |
| Average | 6350 | 17800 | 6350 | 11800 | 6350 | 2380 |
| Stdev | 32800 | 55300 | 32800 | 46100 | 32800 | 5220 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.017 | | 0.25 | | 0.63 |
| Min | 0.242 | 1.50 | 0.242 | 10.6 | 0.242 | 5.54 |
| Max | 352000 | 379000 | 352000 | 365000 | 352000 | 20700 |
| n (Samp) | 251 | 100 | 251 | 80 | 251 | 16 |
| n (Patient) | 109 | 100 | 109 | 80 | 109 | 16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.52 | 0.63 | 0.59 | 0.55 | 0.58 | 0.47 | 0.47 | 0.56 |
| SE | 0.034 | 0.054 | 0.034 | 0.039 | 0.059 | 0.038 | 0.081 | 0.080 | 0.077 |
| p | 4.7E-4 | 0.74 | 2.3E-4 | 0.017 | 0.39 | 0.028 | 0.72 | 0.72 | 0.40 |
| nCohort 1 | 203 | 505 | 251 | 203 | 505 | 251 | 203 | 505 | 251 |
| nCohort 2 | 113 | 31 | 100 | 78 | 26 | 80 | 14 | 14 | 16 |
| Cutoff 1 | 125 | 90.2 | 164 | 108 | 105 | 117 | 62.7 | 80.0 | 104 |
| Sens 1 | 71% | 71% | 70% | 71% | 73% | 70% | 71% | 71% | 75% |
| Spec 1 | 46% | 29% | 50% | 42% | 32% | 41% | 30% | 27% | 39% |
| Cutoff 2 | 83.0 | 71.7 | 108 | 78.5 | 78.7 | 79.9 | 7.96 | 10.1 | 62.7 |
| Sens 2 | 81% | 81% | 80% | 81% | 81% | 80% | 86% | 86% | 81% |
| Spec 2 | 36% | 23% | 40% | 36% | 27% | 34% | 6% | 5% | 27% |
| Cutoff 3 | 31.9 | 21.7 | 43.1 | 40.3 | 9.08 | 43.1 | 3.54 | 0.892 | 9.75 |
| Sens 3 | 90% | 90% | 90% | 91% | 92% | 90% | 93% | 93% | 94% |
| Spec 3 | 15% | 8% | 20% | 19% | 4% | 20% | 5% | 0% | 6% |
| Cutoff 4 | 346 | 457 | 438 | 346 | 457 | 438 | 346 | 457 | 438 |
| Sens 4 | 43% | 32% | 43% | 40% | 46% | 34% | 29% | 36% | 38% |
| Spec 4 | 70% | 70% | 70% | 70% | 70% | 70% | 70% | 70% | 70% |
| Cutoff 5 | 795 | 1270 | 944 | 795 | 1270 | 944 | 795 | 1270 | 944 |
| Sens 5 | 34% | 16% | 36% | 27% | 31% | 26% | 14% | 21% | 31% |
| Spec 5 | 80% | 80% | 80% | 80% | 80% | 80% | 80% | 80% | 80% |
| Cutoff 6 | 4700 | 7480 | 5930 | 4700 | 7480 | 5930 | 4700 | 7480 | 5930 |
| Sens 6 | 19% | 13% | 22% | 13% | 12% | 15% | 7% | 21% | 12% |
| Spec 6 | 90% | 90% | 90% | 90% | 90% | 90% | 90% | 90% | 90% |
| OR Quart 2 | 1.6 | 0.85 | 2.0 | 1.4 | 1.2 | 1.6 | 1.4 | 1.3 | 0.98 |
| p Value | 0.21 | 0.78 | 0.076 | 0.41 | 0.77 | 0.26 | 0.68 | 0.70 | 0.98 |
| 95% CI of OR Quart2 | 0.77 3.2 | 0.28 2.6 | 0.93 4.1 | 0.62 3.2 | 0.36 4.0 | 0.72 3.4 | 0.30 6.5 | 0.29 6.1 | 0.19 5.1 |
| OR Quart 3 | 2.3 | 1.5 | 2.1 | 2.4 | 0.99 | 2.4 | 1.0 | 1.0 | 1.3 |
| p Value | 0.018 | 0.45 | 0.053 | 0.025 | 0.99 | 0.021 | 0.98 | 1.0 | 0.71 |
| 95% CI of OR Quart3 | 1.2 4.6 | 0.54 4.0 | 0.99 4.3 | 1.1 5.3 | 0.28 3.5 | 1.1 5.1 | 0.20 5.3 | 0.20 5.0 | 0.29 6.2 |
| OR Quart 4 | 3.1 | 1.2 | 3.8 | 2.1 | 2.1 | 1.9 | 1.4 | 1.4 | 2.1 |
| p Value | 0.0011 | 0.79 | 2.5E-4 | 0.062 | 0.20 | 0.097 | 0.68 | 0.69 | 0.32 |
| 95% CI of OR Quart4 | 1.6 6.2 | 0.41 3.3 | 1.9 7.7 | 0.96 4.6 | 0.69 6.2 | 0.89 4.1 | 0.30 6.5 | 0.30 6.2 | 0.49 8.6 |

**Glucagon**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 238 | 238 | 238 | 347 | 238 | 347 |
| Average | 1510 | 1480 | 1510 | 2220 | 1510 | 2980 |
| Stdev | 2880 | 3090 | 2880 | 3660 | 2880 | 3990 |
| p(t-test) | | 0.95 | | 0.17 | | 0.030 |
| Min | 2.40 | 1.53 | 2.40 | 4.46 | 2.40 | 3.67 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 9330 | 9330 | 9330 | 9330 | 9330 | 9330 |
| n (Samp) | 122 | 47 | 122 | 50 | 122 | 26 |
| n (Patient) | 98 | 47 | 98 | 50 | 98 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 238 | 238 | 238 | 347 | 238 | 2420 |
| Average | 1680 | 2140 | 1680 | 3800 | 1680 | 4230 |
| Stdev | 3080 | 3900 | 3080 | 4400 | 3080 | 4290 |
| p(t-test) | | 0.60 | | 0.0056 | | 0.0047 |
| Min | 0.0247 | 4.37 | 0.0247 | 6.86 | 0.0247 | 3.67 |
| Max | 9330 | 9330 | 9330 | 9330 | 9330 | 9330 |
| n (Samp) | 261 | 14 | 261 | 19 | 261 | 13 |
| n (Patient) | 159 | 14 | 159 | 19 | 159 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 238 | 238 | 238 | 238 | 238 | 347 |
| Average | 1250 | 1830 | 1250 | 1900 | 1250 | 2340 |
| Stdev | 2480 | 3370 | 2480 | 3500 | 2480 | 3790 |
| p(t-test) | | 0.24 | | 0.19 | | 0.085 |
| Min | 2.40 | 1.53 | 2.40 | 4.46 | 2.40 | 6.71 |
| Max | 9330 | 9330 | 9330 | 9330 | 9330 | 9330 |
| n (Samp) | 111 | 44 | 111 | 46 | 111 | 23 |
| n (Patient) | 84 | 44 | 84 | 46 | 84 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.52 | 0.49 | 0.56 | 0.58 | 0.66 | 0.56 | 0.72 | 0.70 | 0.68 |
| SE | 0.050 | 0.080 | 0.052 | 0.049 | 0.070 | 0.051 | 0.060 | 0.083 | 0.066 |
| p | 0.73 | 0.92 | 0.28 | 0.086 | 0.024 | 0.27 | 2.1E-4 | 0.016 | 0.0057 |
| nCohort 1 | 122 | 261 | 111 | 122 | 261 | 111 | 122 | 261 | 111 |
| nCohort 2 | 47 | 14 | 44 | 50 | 19 | 46 | 26 | 13 | 23 |
| Cutoff 1 | 98.8 | 166 | 101 | 46.8 | 174 | 19.3 | 275 | 275 | 238 |
| Sens 1 | 70% | 71% | 70% | 70% | 74% | 72% | 88% | 85% | 78% |
| Spec 1 | 42% | 34% | 41% | 40% | 35% | 38% | 61% | 54% | 61% |
| Cutoff 2 | 10.2 | 10.2 | 10.8 | 16.2 | 75.1 | 10.8 | 275 | 275 | 168 |
| Sens 2 | 81% | 86% | 82% | 80% | 84% | 80% | 88% | 85% | 83% |
| Spec 2 | 37% | 25% | 35% | 38% | 33% | 35% | 61% | 54% | 41% |
| Cutoff 3 | 6.53 | 8.98 | 6.52 | 6.86 | 11.7 | 6.71 | 169 | 4.46 | 98.8 |
| Sens 3 | 91% | 93% | 93% | 90% | 95% | 91% | 92% | 92% | 91% |
| Spec 3 | 19% | 22% | 19% | 20% | 27% | 20% | 43% | 7% | 41% |
| Cutoff 4 | 347 | 347 | 347 | 347 | 347 | 347 | 347 | 347 | 347 |
| Sens 4 | 19% | 21% | 25% | 30% | 47% | 24% | 42% | 62% | 30% |
| Spec 4 | 73% | 72% | 74% | 73% | 72% | 74% | 73% | 72% | 74% |
| Cutoff 5 | 2420 | 2420 | 2420 | 2420 | 2420 | 2420 | 2420 | 2420 | 2420 |
| Sens 5 | 13% | 21% | 16% | 20% | 37% | 17% | 27% | 38% | 22% |
| Spec 5 | 88% | 86% | 91% | 88% | 86% | 91% | 88% | 86% | 91% |
| Cutoff 6 | 9330 | 9330 | 2420 | 9330 | 9330 | 2420 | 9330 | 9330 | 2420 |
| Sens 6 | 0% | 0% | 16% | 0% | 0% | 17% | 0% | 0% | 22% |
| Spec 6 | 100% | 100% | 91% | 100% | 100% | 91% | 100% | 100% | 91% |
| OR Quart 2 | 2.6 | 0.32 | 3.0 | 2.6 | 4.2 | 2.9 | 2.1 | 0 | 4.3 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value | 0.057 | 0.33 | 0.049 | 0.058 | 0.21 | 0.048 | 0.56 | na | 0.21 |
| 95% CI of | 0.97 | 0.033 | 1.0 | 0.97 | 0.46 | 1.0 | 0.18 | na | 0.45 |
| OR Quart2 | 7.0 | 3.2 | 8.9 | 6.9 | 38 | 8.1 | 24 | na | 40 |
| OR Quart 3 | 2.1 | 2.9 | 2.7 | 2.3 | 6.5 | 2.3 | 22 | 2.6 | 18 |
| p Value | 0.14 | 0.13 | 0.080 | 0.092 | 0.088 | 0.12 | 0.0039 | 0.26 | 0.0070 |
| 95% CI of | 0.78 | 0.73 | 0.89 | 0.87 | 0.76 | 0.80 | 2.7 | 0.49 | 2.2 |
| OR Quart3 | 5.8 | 11 | 8.0 | 6.3 | 55 | 6.6 | 180 | 14 | 150 |
| OR Quart 4 | 1.1 | 0.67 | 2.1 | 1.5 | 8.9 | 1.7 | 12 | 3.1 | 6.9 |
| p Value | 0.83 | 0.66 | 0.19 | 0.44 | 0.042 | 0.31 | 0.024 | 0.17 | 0.083 |
| 95% CI of | 0.39 | 0.11 | 0.69 | 0.54 | 1.1 | 0.59 | 1.4 | 0.61 | 0.78 |
| OR Quart4 | 3.3 | 4.1 | 6.4 | 4.2 | 73 | 5.1 | 97 | 16 | 60 |

**Glucagon-like peptide 1**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 5.53 | 3.10 | 5.53 | 3.10 | 5.53 | 1.20 |
| Average | 9.04 | 5.05 | 9.04 | 6.48 | 9.04 | 22.4 |
| Stdev | 43.4 | 3.51 | 43.4 | 15.1 | 43.4 | 101 |
| p(t-test) |  | 0.53 |  | 0.68 |  | 0.28 |
| Min | 0.407 | 0.407 | 0.407 | 0.407 | 0.407 | 0.407 |
| Max | 482 | 9.60 | 482 | 107 | 482 | 519 |
| n (Samp) | 122 | 47 | 122 | 50 | 122 | 26 |
| n (Patient) | 98 | 47 | 98 | 50 | 98 | 26 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 3.10 | 5.53 | 3.10 | 3.10 | 3.10 | 2.30 |
| Average | 9.65 | 5.97 | 9.65 | 4.04 | 9.65 | 3.63 |
| Stdev | 45.3 | 3.59 | 45.3 | 3.81 | 45.3 | 3.70 |
| p(t-test) |  | 0.76 |  | 0.59 |  | 0.63 |
| Min | 0.407 | 0.407 | 0.407 | 0.407 | 0.407 | 0.407 |
| Max | 519 | 9.60 | 519 | 10.1 | 519 | 11.8 |
| n (Samp) | 261 | 14 | 261 | 19 | 261 | 13 |
| n (Patient) | 159 | 14 | 159 | 19 | 159 | 13 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 5.53 | 3.10 | 5.53 | 3.10 | 5.53 | 0.685 |
| Average | 9.53 | 4.60 | 9.53 | 7.43 | 9.53 | 25.0 |
| Stdev | 45.4 | 3.51 | 45.4 | 16.3 | 45.4 | 108 |
| p(t-test) |  | 0.47 |  | 0.76 |  | 0.27 |
| Min | 0.407 | 0.407 | 0.407 | 0.407 | 0.407 | 0.407 |
| Max | 482 | 9.60 | 482 | 107 | 482 | 519 |
| n (Samp) | 111 | 44 | 111 | 46 | 111 | 23 |
| n (Patient) | 84 | 44 | 84 | 46 | 84 | 23 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.49 | 0.60 | 0.44 | 0.43 | 0.45 | 0.43 | 0.30 | 0.42 | 0.30 |
| SE | 0.050 | 0.082 | 0.052 | 0.049 | 0.070 | 0.051 | 0.061 | 0.085 | 0.065 |
| p | 0.90 | 0.20 | 0.24 | 0.17 | 0.45 | 0.15 | 0.0013 | 0.36 | 0.0025 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| nCohort 1 | 122 | 261 | 111 | 122 | 261 | 111 | 122 | 261 | 111 |
| nCohort 2 | 47 | 14 | 44 | 50 | 19 | 46 | 26 | 13 | 23 |
| Cutoff 1 | 2.30 | 2.30 | 1.71 | 0.685 | 0.407 | 0.685 | 0 | 0.407 | 0 |
| Sens 1 | 74% | 79% | 70% | 72% | 84% | 72% | 100% | 85% | 100% |
| Spec 1 | 28% | 36% | 23% | 12% | 15% | 10% | 0% | 15% | 0% |
| Cutoff 2 | 0.685 | 1.75 | 0.407 | 0.407 | 0.407 | 0 | 0 | 0.407 | 0 |
| Sens 2 | 83% | 86% | 84% | 82% | 84% | 100% | 100% | 85% | 100% |
| Spec 2 | 12% | 33% | 9% | 10% | 15% | 0% | 0% | 15% | 0% |
| Cutoff 3 | 0 | 0.407 | 0 | 0 | 0 | 0 | 0 | 0 | 0 |
| Sens 3 | 100% | 93% | 100% | 100% | 100% | 100% | 100% | 100% | 100% |
| Spec 3 | 0% | 15% | 0% | 0% | 0% | 0% | 0% | 0% | 0% |
| Cutoff 4 | 5.53 | 5.53 | 8.07 | 5.53 | 5.53 | 8.07 | 5.53 | 5.53 | 8.07 |
| Sens 4 | 34% | 43% | 27% | 26% | 26% | 22% | 15% | 15% | 9% |
| Spec 4 | 72% | 73% | 70% | 72% | 73% | 70% | 72% | 73% | 70% |
| Cutoff 5 | 9.60 | 9.60 | 9.60 | 9.60 | 9.60 | 9.60 | 9.60 | 9.60 | 9.60 |
| Sens 5 | 0% | 0% | 0% | 10% | 11% | 11% | 8% | 15% | 4% |
| Spec 5 | 94% | 94% | 92% | 94% | 94% | 92% | 94% | 94% | 92% |
| Cutoff 6 | 9.60 | 9.60 | 9.60 | 9.60 | 9.60 | 9.60 | 9.60 | 9.60 | 9.60 |
| Sens 6 | 0% | 0% | 0% | 10% | 11% | 11% | 8% | 15% | 4% |
| Spec 6 | 94% | 94% | 92% | 94% | 94% | 92% | 94% | 94% | 92% |
| OR Quart 2 | 0.56 | 1.5 | 1.1 | 0.68 | 0.38 | 1.4 | 1.4 | 1.5 | 4.3 |
| p Value | 0.25 | 0.66 | 0.79 | 0.45 | 0.26 | 0.56 | 0.69 | 0.64 | 0.084 |
| 95% CI of | 0.21 | 0.24 | 0.41 | 0.25 | 0.072 | 0.49 | 0.29 | 0.25 | 0.82 |
| OR Quart2 | 1.5 | 9.3 | 3.2 | 1.8 | 2.0 | 3.7 | 6.6 | 9.6 | 23 |
| OR Quart 3 | 0.93 | 1.5 | 1.5 | 1.1 | 0.79 | 1.2 | 1.4 | 2.1 | 1.0 |
| p Value | 0.87 | 0.66 | 0.44 | 0.81 | 0.73 | 0.74 | 0.69 | 0.41 | 1.0 |
| 95% CI of | 0.37 | 0.24 | 0.54 | 0.44 | 0.20 | 0.42 | 0.29 | 0.36 | 0.13 |
| OR Quart3 | 2.3 | 9.3 | 4.1 | 2.8 | 3.1 | 3.3 | 6.6 | 12 | 7.5 |
| OR Quart 4 | 0.74 | 3.1 | 1.7 | 1.5 | 1.7 | 2.4 | 7.7 | 2.1 | 9.1 |
| p Value | 0.52 | 0.17 | 0.28 | 0.36 | 0.39 | 0.080 | 0.0030 | 0.40 | 0.0065 |
| 95% CI of | 0.29 | 0.61 | 0.64 | 0.62 | 0.52 | 0.90 | 2.0 | 0.37 | 1.9 |
| OR Quart4 | 1.9 | 16 | 4.7 | 3.8 | 5.4 | 6.4 | 30 | 12 | 45 |

**Appetite-regulating hormone**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.560 | 0.232 | 0.560 | 0.232 | 0.560 | 0.396 |
| Average | 0.919 | 0.539 | 0.919 | 2.03 | 0.919 | 1.33 |
| Stdev | 1.31 | 0.732 | 1.31 | 9.23 | 1.31 | 1.61 |
| p(t-test) |  | 0.062 |  | 0.19 |  | 0.16 |
| Min | 0.0793 | 0.0793 | 0.0793 | 0.0793 | 0.0793 | 0.0793 |
| Max | 8.07 | 2.40 | 8.07 | 65.5 | 8.07 | 4.69 |
| n (Samp) | 122 | 47 | 122 | 50 | 122 | 26 |
| n (Patient) | 98 | 47 | 98 | 50 | 98 | 26 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.232 | 0.232 | 0.232 | 0.232 | 0.232 | 0.560 |
| Average | 1.13 | 0.661 | 1.13 | 0.883 | 1.13 | 1.17 |
| Stdev | 4.19 | 0.857 | 4.19 | 0.994 | 4.19 | 1.45 |
| p(t-test) |  | 0.68 |  | 0.80 |  | 0.97 |
| Min | 0.0793 | 0.0793 | 0.0793 | 0.0793 | 0.0793 | 0.0793 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 65.5 | 2.40 | 65.5 | 2.92 | 65.5 | 4.69 |
| n (Samp) | 261 | 14 | 261 | 19 | 261 | 13 |
| n (Patient) | 159 | 14 | 159 | 19 | 159 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.232 | 0.232 | 0.232 | 0.232 | 0.232 | 0.560 |
| Average | 0.975 | 0.527 | 0.975 | 2.12 | 0.975 | 1.15 |
| Stdev | 1.37 | 0.726 | 1.37 | 9.63 | 1.37 | 1.49 |
| p(t-test) | | 0.042 | | 0.22 | | 0.58 |
| Min | 0.0793 | 0.0793 | 0.0793 | 0.0793 | 0.0793 | 0.0793 |
| Max | 8.07 | 2.40 | 8.07 | 65.5 | 8.07 | 4.55 |
| n (Samp) | 111 | 44 | 111 | 46 | 111 | 23 |
| n (Patient) | 84 | 44 | 84 | 46 | 84 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.40 | 0.45 | 0.38 | 0.44 | 0.49 | 0.42 | 0.51 | 0.49 | 0.49 |
| SE | 0.050 | 0.081 | 0.051 | 0.049 | 0.069 | 0.051 | 0.063 | 0.083 | 0.067 |
| p | 0.048 | 0.57 | 0.016 | 0.19 | 0.84 | 0.14 | 0.94 | 0.88 | 0.93 |
| nCohort 1 | 122 | 261 | 111 | 122 | 261 | 111 | 122 | 261 | 111 |
| nCohort 2 | 47 | 14 | 44 | 50 | 19 | 46 | 26 | 13 | 23 |
| Cutoff 1 | 0.0995 | 0.0995 | 0.0995 | 0.108 | 0 | 0.0995 | 0.0995 | 0 | 0.108 |
| Sens 1 | 81% | 79% | 75% | 70% | 100% | 78% | 73% | 100% | 74% |
| Spec 1 | 16% | 20% | 14% | 29% | 0% | 14% | 16% | 0% | 29% |
| Cutoff 2 | 0.0995 | 0 | 0.0793 | 0.0793 | 0 | 0.0793 | 0 | 0 | 0 |
| Sens 2 | 81% | 100% | 84% | 82% | 100% | 83% | 100% | 100% | 100% |
| Spec 2 | 16% | 0% | 6% | 10% | 0% | 6% | 0% | 0% | 0% |
| Cutoff 3 | 0 | 0 | 0 | 0 | 0 | 0 | 0 | 0 | 0 |
| Sens 3 | 100% | 100% | 100% | 100% | 100% | 100% | 100% | 100% | 100% |
| Spec 3 | 0% | 0% | 0% | 0% | 0% | 0% | 0% | 0% | 0% |
| Cutoff 4 | 0.560 | 0.560 | 0.560 | 0.560 | 0.560 | 0.560 | 0.560 | 0.560 | 0.560 |
| Sens 4 | 17% | 21% | 18% | 22% | 37% | 17% | 46% | 38% | 39% |
| Spec 4 | 75% | 73% | 72% | 75% | 73% | 72% | 75% | 73% | 72% |
| Cutoff 5 | 1.75 | 1.75 | 1.75 | 1.75 | 1.75 | 1.75 | 1.75 | 1.75 | 1.75 |
| Sens 5 | 9% | 14% | 7% | 14% | 16% | 11% | 19% | 23% | 13% |
| Spec 5 | 83% | 85% | 80% | 83% | 85% | 80% | 83% | 85% | 80% |
| Cutoff 6 | 2.67 | 2.67 | 2.67 | 2.67 | 2.67 | 2.67 | 2.67 | 2.67 | 2.67 |
| Sens 6 | 0% | 0% | 0% | 8% | 5% | 9% | 15% | 8% | 13% |
| Spec 6 | 93% | 93% | 92% | 93% | 93% | 92% | 93% | 93% | 92% |
| OR Quart 2 | 0.88 | 1.0 | 0.57 | 0.66 | 1.0 | 1.4 | 1.0 | 0.39 | 1.6 |
| p Value | 0.81 | 1.0 | 0.37 | 0.44 | 1.0 | 0.55 | 1.0 | 0.27 | 0.49 |
| 95% CI of OR Quart2 | 0.29 2.7 | 0.19 5.1 | 0.17 1.9 | 0.24 1.9 | 0.31 3.3 | 0.48 4.0 | 0.31 3.2 | 0.073 2.1 | 0.44 5.5 |
| OR Quart 3 | 4.4 | 1.7 | 2.2 | 2.1 | 0.15 | 2.5 | 0.24 | 0.19 | 1.0 |
| p Value | 0.0031 | 0.47 | 0.14 | 0.11 | 0.088 | 0.075 | 0.094 | 0.13 | 1.0 |
| 95% CI of OR Quart3 | 1.6 12 | 0.39 7.5 | 0.79 6.0 | 0.84 5.2 | 0.018 1.3 | 0.91 6.9 | 0.047 1.3 | 0.021 1.7 | 0.26 3.8 |
| OR Quart 4 | 1.6 | 1.0 | 3.1 | 1.3 | 1.0 | 2.0 | 1.6 | 1.0 | 1.3 |
| p Value | 0.40 | 0.99 | 0.026 | 0.63 | 1.0 | 0.18 | 0.41 | 0.98 | 0.70 |
| 95% CI of OR Quart4 | 0.55 4.4 | 0.20 5.2 | 1.1 8.6 | 0.49 3.2 | 0.31 3.3 | 0.72 5.6 | 0.53 4.8 | 0.28 3.7 | 0.35 4.7 |

**Islet amyloid polypeptide**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.282 | 0.227 | 0.282 | 0.0802 | 0.282 | 0.0786 |
| Average | 2.08 | 2.54 | 2.08 | 2.95 | 2.08 | 0.425 |
| Stdev | 9.81 | 12.3 | 9.81 | 10.8 | 9.81 | 0.939 |
| p(t-test) | | 0.80 | | 0.61 | | 0.39 |
| Min | 0.00151 | 0.00151 | 0.00151 | 0.00151 | 0.00151 | 0.00151 |
| Max | 102 | 84.9 | 102 | 57.5 | 102 | 4.44 |
| n (Samp) | 122 | 47 | 122 | 50 | 122 | 26 |
| n (Patient) | 98 | 47 | 98 | 50 | 98 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.201 | 0.901 | 0.201 | 0.00945 | 0.201 | 0.0527 |
| Average | 1.88 | 7.06 | 1.88 | 3.60 | 1.88 | 10.9 |
| Stdev | 8.86 | 22.4 | 8.86 | 12.0 | 8.86 | 36.6 |
| p(t-test) | | 0.059 | | 0.43 | | 0.0064 |
| Min | 0.00151 | 0.00151 | 0.00151 | 0.00151 | 0.00151 | 0.00151 |
| Max | 102 | 84.9 | 102 | 51.4 | 102 | 133 |
| n (Samp) | 261 | 14 | 261 | 19 | 261 | 13 |
| n (Patient) | 159 | 14 | 159 | 19 | 159 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.359 | 0.261 | 0.359 | 0.205 | 0.359 | 0.0527 |
| Average | 1.32 | 1.30 | 1.32 | 2.39 | 1.32 | 4.02 |
| Stdev | 3.70 | 4.10 | 3.70 | 9.00 | 3.70 | 17.6 |
| p(t-test) | | 0.98 | | 0.29 | | 0.14 |
| Min | 0.00151 | 0.00151 | 0.00151 | 0.00151 | 0.00151 | 0.00151 |
| Max | 30.4 | 26.4 | 30.4 | 57.5 | 30.4 | 84.9 |
| n (Samp) | 111 | 44 | 111 | 46 | 111 | 23 |
| n (Patient) | 84 | 44 | 84 | 46 | 84 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | 0.62 | 0.47 | 0.45 | 0.37 | 0.46 | 0.39 | 0.43 | 0.39 |
| SE | 0.050 | 0.082 | 0.052 | 0.049 | 0.071 | 0.051 | 0.064 | 0.085 | 0.067 |
| p | 0.97 | 0.13 | 0.53 | 0.28 | 0.058 | 0.38 | 0.078 | 0.38 | 0.11 |
| nCohort 1 | 122 | 261 | 111 | 122 | 261 | 111 | 122 | 261 | 111 |
| nCohort 2 | 47 | 14 | 44 | 50 | 19 | 46 | 26 | 13 | 23 |
| Cutoff 1 | 0.00550 | 0.0527 | 0.00571 | 0.00369 | 0 | 0.00369 | 0.00151 | 0.00151 | 0.00187 |
| Sens 1 | 77% | 71% | 70% | 76% | 100% | 78% | 81% | 85% | 74% |
| Spec 1 | 29% | 44% | 25% | 23% | 0% | 14% | 7% | 8% | 9% |
| Cutoff 2 | 0.00353 | 0.00550 | 0.00353 | 0.00187 | 0 | 0.00353 | 0.00151 | 0.00151 | 0.00151 |
| Sens 2 | 83% | 86% | 82% | 80% | 100% | 80% | 81% | 85% | 83% |
| Spec 2 | 23% | 29% | 14% | 11% | 0% | 14% | 7% | 8% | 4% |
| Cutoff 3 | 0.00151 | 0.00369 | 0.00151 | 0.00151 | 0 | 0.00151 | 0 | 0 | 0 |
| Sens 3 | 96% | 93% | 98% | 90% | 100% | 93% | 100% | 100% | 100% |
| Spec 3 | 7% | 23% | 4% | 7% | 0% | 4% | 0% | 0% | 0% |
| Cutoff 4 | 0.976 | 0.618 | 1.06 | 0.976 | 0.618 | 1.06 | 0.976 | 0.618 | 1.06 |
| Sens 4 | 28% | 50% | 20% | 22% | 16% | 24% | 12% | 23% | 17% |
| Spec 4 | 70% | 70% | 72% | 70% | 70% | 72% | 70% | 70% | 72% |
| Cutoff 5 | 1.47 | 1.20 | 1.47 | 1.47 | 1.20 | 1.47 | 1.47 | 1.20 | 1.47 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 5 | 19% | 50% | 16% | 18% | 16% | 20% | 8% | 23% | 13% |
| Spec 5 | 80% | 80% | 80% | 80% | 80% | 80% | 80% | 80% | 80% |
| Cutoff 6 | 2.36 | 2.21 | 2.35 | 2.36 | 2.21 | 2.35 | 2.36 | 2.21 | 2.35 |
| Sens 6 | 11% | 29% | 11% | 12% | 16% | 11% | 4% | 23% | 4% |
| Spec 6 | 90% | 90% | 91% | 90% | 90% | 91% | 90% | 90% | 91% |
| OR Quart 2 | 1.0 | 2.0 | 1.5 | 1.0 | 0.32 | 0.91 | 2.2 | 0.67 | 1.3 |
| p Value | 0.95 | 0.42 | 0.44 | 1.0 | 0.33 | 0.85 | 0.29 | 0.66 | 0.69 |
| 95% CI of OR Quart2 | 0.39 | 0.36 | 0.54 | 0.37 | 0.033 | 0.33 | 0.50 | 0.11 | 0.33 |
|  | 2.7 | 11 | 4.1 | 2.7 | 3.2 | 2.5 | 9.5 | 4.1 | 5.5 |
| OR Quart 3 | 2.0 | 0.49 | 1.3 | 2.4 | 2.9 | 0.79 | 3.6 | 0.66 | 1.9 |
| p Value | 0.15 | 0.56 | 0.60 | 0.069 | 0.13 | 0.65 | 0.070 | 0.65 | 0.33 |
| 95% CI of OR Quart3 | 0.79 | 0.043 | 0.47 | 0.94 | 0.73 | 0.29 | 0.90 | 0.11 | 0.51 |
|  | 5.0 | 5.5 | 3.6 | 6.0 | 11 | 2.2 | 15 | 4.1 | 7.4 |
| OR Quart 4 | 0.68 | 3.7 | 1.5 | 1.3 | 2.5 | 1.8 | 3.1 | 2.1 | 2.0 |
| p Value | 0.47 | 0.11 | 0.40 | 0.62 | 0.20 | 0.21 | 0.11 | 0.30 | 0.30 |
| 95% CI of OR Quart4 | 0.24 | 0.75 | 0.56 | 0.48 | 0.61 | 0.71 | 0.76 | 0.51 | 0.53 |
|  | 1.9 | 19 | 4.2 | 3.4 | 10 | 4.7 | 13 | 8.9 | 7.7 |

**Interleukin-17A**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.00337 | 0.00303 | 0.00337 | 0.00468 | 0.00337 | 0.00327 |
| Average | 1.11 | 0.643 | 1.11 | 0.788 | 1.11 | 0.364 |
| Stdev | 8.62 | 3.07 | 8.62 | 3.13 | 8.62 | 1.30 |
| p(t-test) |  | 0.44 |  | 0.62 |  | 0.50 |
| Min | 0.000344 | 0.000344 | 0.000344 | 0.000344 | 0.000344 | 0.000381 |
| Max | 133 | 29.0 | 133 | 25.7 | 133 | 9.17 |
| n (Samp) | 624 | 214 | 624 | 180 | 624 | 60 |
| n (Patient) | 282 | 214 | 282 | 180 | 282 | 60 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.00337 | 0.00362 | 0.00337 | 0.00362 | 0.00337 | 0.00283 |
| Average | 1.11 | 0.299 | 1.11 | 0.455 | 1.11 | 0.132 |
| Stdev | 8.18 | 1.04 | 8.18 | 1.86 | 8.18 | 0.320 |
| p(t-test) |  | 0.41 |  | 0.52 |  | 0.43 |
| Min | 0.000344 | 0.000344 | 0.000344 | 0.000344 | 0.000344 | 0.000344 |
| Max | 157 | 7.91 | 157 | 14.5 | 157 | 1.43 |
| n (Samp) | 1484 | 69 | 1484 | 66 | 1484 | 43 |
| n (Patient) | 514 | 69 | 514 | 66 | 514 | 43 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.00337 | 0.00283 | 0.00337 | 0.00492 | 0.00337 | 0.00360 |
| Average | 0.925 | 1.04 | 0.925 | 1.10 | 0.925 | 0.343 |
| Stdev | 7.93 | 4.24 | 7.93 | 3.73 | 7.93 | 1.35 |
| p(t-test) |  | 0.85 |  | 0.78 |  | 0.59 |
| Min | 0.000344 | 0.000344 | 0.000344 | 0.000344 | 0.000344 | 0.000381 |
| Max | 133 | 33.1 | 133 | 25.7 | 133 | 9.17 |
| n (Samp) | 659 | 195 | 659 | 170 | 659 | 55 |
| n (Patient) | 251 | 195 | 251 | 170 | 251 | 55 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.50 | 0.48 | 0.56 | 0.50 | 0.58 | 0.52 | 0.45 | 0.54 |
| SE | 0.023 | 0.036 | 0.024 | 0.025 | 0.036 | 0.025 | 0.039 | 0.046 | 0.041 |
| p | 0.16 | 0.97 | 0.36 | 0.018 | 0.97 | 9.5E-4 | 0.70 | 0.26 | 0.31 |
| nCohort 1 | 624 | 1484 | 659 | 624 | 1484 | 659 | 624 | 1484 | 659 |
| nCohort 2 | 214 | 69 | 195 | 180 | 66 | 170 | 60 | 43 | 55 |
| Cutoff 1 | 0.000662 | 0.00182 | 0.000662 | 0.00275 | 0.00182 | 0.00291 | 0.00263 | 0.000747 | 0.00263 |
| Sens 1 | 72% | 71% | 70% | 72% | 74% | 70% | 70% | 77% | 73% |
| Spec 1 | 14% | 29% | 18% | 38% | 29% | 44% | 38% | 21% | 41% |
| Cutoff 2 | 0.000597 | 0.000662 | 0.000597 | 0.00182 | 0.000662 | 0.00182 | 0.00174 | 0.000662 | 0.00189 |
| Sens 2 | 83% | 81% | 83% | 81% | 80% | 81% | 83% | 84% | 82% |
| Spec 2 | 8% | 17% | 11% | 26% | 17% | 30% | 26% | 17% | 34% |
| Cutoff 3 | 0.000381 | 0.000410 | 0.000381 | 0.000410 | 0.000380 | 0.000410 | 0.000734 | 0.000597 | 0.000654 |
| Sens 3 | 92% | 93% | 91% | 91% | 92% | 93% | 90% | 91% | 91% |
| Spec 3 | 4% | 7% | 5% | 5% | 4% | 6% | 15% | 10% | 16% |
| Cutoff 4 | 0.00875 | 0.00913 | 0.00875 | 0.00875 | 0.00913 | 0.00875 | 0.00875 | 0.00913 | 0.00875 |
| Sens 4 | 32% | 32% | 32% | 37% | 35% | 37% | 32% | 30% | 27% |
| Spec 4 | 71% | 70% | 72% | 71% | 70% | 72% | 71% | 70% | 72% |
| Cutoff 5 | 0.106 | 0.150 | 0.0652 | 0.106 | 0.150 | 0.0652 | 0.106 | 0.150 | 0.0652 |
| Sens 5 | 21% | 20% | 23% | 29% | 26% | 31% | 22% | 19% | 18% |
| Spec 5 | 80% | 80% | 80% | 80% | 80% | 80% | 80% | 80% | 80% |
| Cutoff 6 | 0.608 | 0.830 | 0.606 | 0.608 | 0.830 | 0.606 | 0.608 | 0.830 | 0.606 |
| Sens 6 | 11% | 9% | 13% | 14% | 11% | 17% | 12% | 7% | 9% |
| Spec 6 | 90% | 90% | 90% | 90% | 90% | 90% | 90% | 90% | 90% |
| OR Quart 2 | 0.91 | 0.59 | 0.80 | 1.0 | 0.81 | 1.2 | 2.6 | 0.59 | 2.5 |
| p Value | 0.67 | 0.15 | 0.36 | 0.90 | 0.58 | 0.43 | 0.014 | 0.32 | 0.034 |
| 95% CI of OR Quart2 | 0.58 1.4 | 0.28 1.2 | 0.50 1.3 | 0.62 1.7 | 0.40 1.7 | 0.73 2.1 | 1.2 5.7 | 0.21 1.6 | 1.1 5.9 |
| OR Quart 3 | 0.63 | 0.95 | 0.77 | 1.6 | 1.0 | 1.6 | 0.89 | 1.3 | 2.0 |
| p Value | 0.058 | 0.87 | 0.28 | 0.070 | 1.0 | 0.059 | 0.81 | 0.53 | 0.14 |
| 95% CI of OR Quart3 | 0.40 1.0 | 0.50 1.8 | 0.48 1.2 | 0.96 2.5 | 0.50 2.0 | 0.98 2.7 | 0.35 2.3 | 0.57 3.0 | 0.81 4.7 |
| OR Quart 4 | 1.7 | 0.89 | 1.5 | 1.8 | 1.1 | 2.1 | 1.8 | 1.4 | 1.7 |
| p Value | 0.018 | 0.73 | 0.076 | 0.018 | 0.87 | 0.0031 | 0.16 | 0.41 | 0.27 |
| 95% CI of OR Quart4 | 1.1 2.5 | 0.46 1.7 | 0.96 2.3 | 1.1 2.8 | 0.54 2.1 | 1.3 3.4 | 0.79 4.0 | 0.62 3.2 | 0.67 4.1 |

**Insulin receptor substrate 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000546 | 1.04E-5 | 0.000546 | 0.00110 | 0.000546 | 1.04E-5 |
| Average | 0.00176 | 0.00292 | 0.00176 | 0.00630 | 0.00176 | 0.00173 |
| Stdev | 0.00612 | 0.0145 | 0.00612 | 0.0324 | 0.00612 | 0.00415 |
| p(t-test) | | 0.29 | | 0.040 | | 0.99 |
| Min | 1.25E-9 | 1.25E-9 | 1.25E-9 | 1.25E-9 | 1.25E-9 | 1.25E-9 |
| Max | 0.0712 | 0.123 | 0.0712 | 0.282 | 0.0712 | 0.0163 |
| n (Samp) | 237 | 125 | 237 | 82 | 237 | 15 |
| n (Patient) | 108 | 125 | 108 | 82 | 108 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.78E-5 | 2.78E-5 | 2.78E-5 | 0.00110 | 2.78E-5 | 0.00137 |
| Average | 0.00355 | 0.00106 | 0.00355 | 0.00626 | 0.00355 | 0.00210 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 0.0332 | 0.00146 | 0.0332 | 0.0219 | 0.0332 | 0.00360 |
| p(t-test) | | 0.64 | | 0.65 | | 0.85 |
| Min | 1.25E-9 | 1.25E-9 | 1.25E-9 | 7.25E-7 | 1.25E-9 | 9.90E-7 |
| Max | 0.735 | 0.00563 | 0.735 | 0.123 | 0.735 | 0.0163 |
| n (Samp) | 591 | 38 | 591 | 31 | 591 | 19 |
| n (Patient) | 229 | 38 | 229 | 31 | 229 | 19 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.78E-5 | 1.04E-5 | 2.78E-5 | 0.00110 | 2.78E-5 | 3.41E-6 |
| Average | 0.00177 | 0.00317 | 0.00177 | 0.00574 | 0.00177 | 0.000563 |
| Stdev | 0.00574 | 0.0155 | 0.00574 | 0.0320 | 0.00574 | 0.000973 |
| p(t-test) | | 0.19 | | 0.044 | | 0.40 |
| Min | 1.25E-9 | 1.25E-9 | 1.25E-9 | 1.25E-9 | 1.25E-9 | 1.25E-9 |
| Max | 0.0712 | 0.123 | 0.0712 | 0.282 | 0.0712 | 0.00243 |
| n (Samp) | 294 | 110 | 294 | 84 | 294 | 16 |
| n (Patient) | 123 | 110 | 123 | 84 | 123 | 16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.43 | 0.50 | 0.44 | 0.57 | 0.64 | 0.54 | 0.43 | 0.64 | 0.39 |
| SE | 0.032 | 0.048 | 0.033 | 0.037 | 0.055 | 0.036 | 0.079 | 0.070 | 0.077 |
| p | 0.031 | 0.93 | 0.047 | 0.077 | 0.014 | 0.32 | 0.41 | 0.038 | 0.15 |
| nCohort 1 | 237 | 591 | 294 | 237 | 591 | 294 | 237 | 591 | 294 |
| nCohort 2 | 125 | 38 | 110 | 82 | 31 | 84 | 15 | 19 | 16 |
| Cutoff 1 | 1.78E-6 | 3.73E-6 | 1.78E-6 | 5.14E-6 | 5.14E-6 | 2.32E-6 | 1.50E-6 | 4.40E-5 | 1.50E-6 |
| Sens 1 | 71% | 71% | 71% | 73% | 81% | 75% | 73% | 79% | 75% |
| Spec 1 | 24% | 35% | 24% | 36% | 40% | 32% | 17% | 53% | 17% |
| Cutoff 2 | 7.25E-7 | 7.25E-7 | 7.25E-7 | 1.78E-6 | 5.14E-6 | 1.50E-6 | 9.90E-7 | 1.78E-6 | 9.90E-7 |
| Sens 2 | 84% | 82% | 85% | 80% | 81% | 82% | 87% | 89% | 88% |
| Spec 2 | 8% | 12% | 8% | 24% | 40% | 17% | 13% | 28% | 13% |
| Cutoff 3 | 1.25E-9 | 0 | 1.25E-9 | 7.25E-7 | 7.25E-7 | 1.25E-9 | 1.25E-9 | 7.25E-7 | 1.25E-9 |
| Sens 3 | 90% | 100% | 92% | 91% | 94% | 92% | 93% | 100% | 94% |
| Spec 3 | 6% | 0% | 5% | 8% | 12% | 5% | 6% | 12% | 5% |
| Cutoff 4 | 0.00229 | 0.00110 | 0.00163 | 0.00229 | 0.00110 | 0.00163 | 0.00229 | 0.00110 | 0.00163 |
| Sens 4 | 15% | 34% | 20% | 32% | 48% | 33% | 20% | 53% | 19% |
| Spec 4 | 76% | 71% | 70% | 76% | 71% | 70% | 76% | 71% | 70% |
| Cutoff 5 | 0.00243 | 0.00229 | 0.00243 | 0.00243 | 0.00229 | 0.00243 | 0.00243 | 0.00229 | 0.00243 |
| Sens 5 | 10% | 16% | 10% | 22% | 35% | 15% | 13% | 32% | 0% |
| Spec 5 | 89% | 81% | 85% | 89% | 81% | 85% | 89% | 81% | 85% |
| Cutoff 6 | 0.00271 | 0.00271 | 0.00281 | 0.00271 | 0.00271 | 0.00281 | 0.00271 | 0.00271 | 0.00281 |
| Sens 6 | 9% | 8% | 7% | 16% | 26% | 10% | 7% | 11% | 0% |
| Spec 6 | 90% | 90% | 90% | 90% | 90% | 90% | 90% | 90% | 90% |
| OR Quart 2 | 1.6 | 0.53 | 2.3 | 1.1 | 0.99 | 1.1 | 1.0 | 0.99 | 1.0 |
| p Value | 0.13 | 0.22 | 0.015 | 0.88 | 0.99 | 0.74 | 1.0 | 0.99 | 0.99 |
| 95% CI of OR Quart2 | 0.86 | 0.19 | 1.2 | 0.50 | 0.28 | 0.55 | 0.19 | 0.14 | 0.20 |
| | 3.1 | 1.5 | 4.5 | 2.2 | 3.5 | 2.3 | 5.2 | 7.1 | 5.2 |
| OR Quart 3 | 1.8 | 1.0 | 2.1 | 1.3 | 1.4 | 1.4 | 1.0 | 3.6 | 1.4 |
| p Value | 0.080 | 0.99 | 0.032 | 0.48 | 0.56 | 0.38 | 1.0 | 0.11 | 0.70 |
| 95% CI of OR Quart3 | 0.93 | 0.42 | 1.1 | 0.62 | 0.44 | 0.68 | 0.19 | 0.74 | 0.29 |
| | 3.3 | 2.4 | 4.1 | 2.7 | 4.6 | 2.7 | 5.2 | 18 | 6.2 |
| OR Quart 4 | 2.0 | 0.91 | 2.2 | 1.8 | 3.0 | 1.3 | 2.1 | 4.1 | 2.1 |
| p Value | 0.036 | 0.83 | 0.022 | 0.12 | 0.042 | 0.40 | 0.31 | 0.076 | 0.30 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of OR Quart4 | 1.0 3.7 | 0.37 2.2 | 1.1 4.3 | 0.86 3.6 | 1.0 8.4 | 0.67 2.7 | 0.50 8.8 | 0.86 20 | 0.51 8.8 |

**Involucrin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.385 | 0.607 | 0.385 | 0.493 | 0.385 | 0.414 |
| Average | 1.04 | 1.30 | 1.04 | 1.44 | 1.04 | 0.846 |
| Stdev | 2.89 | 2.31 | 2.89 | 3.36 | 2.89 | 1.10 |
| p(t-test) | | 0.29 | | 0.17 | | 0.63 |
| Min | 0.0119 | 0.0181 | 0.0119 | 0.0117 | 0.0119 | 0.0176 |
| Max | 47.1 | 19.2 | 47.1 | 31.3 | 47.1 | 5.04 |
| n (Samp) | 475 | 173 | 475 | 139 | 475 | 52 |
| n (Patient) | 253 | 173 | 253 | 139 | 253 | 52 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.499 | 0.628 | 0.499 | 0.502 | 0.499 | 0.589 |
| Average | 1.38 | 1.48 | 1.38 | 1.60 | 1.38 | 0.948 |
| Stdev | 4.19 | 2.75 | 4.19 | 3.17 | 4.19 | 1.32 |
| p(t-test) | | 0.86 | | 0.71 | | 0.53 |
| Min | 0.0119 | 0.0214 | 0.0119 | 0.0117 | 0.0119 | 0.0176 |
| Max | 102 | 19.2 | 102 | 15.4 | 102 | 7.39 |
| n (Samp) | 1031 | 58 | 1031 | 53 | 1031 | 37 |
| n (Patient) | 424 | 58 | 424 | 53 | 424 | 37 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.368 | 0.647 | 0.368 | 0.516 | 0.368 | 0.376 |
| Average | 1.07 | 1.37 | 1.07 | 1.61 | 1.07 | 1.05 |
| Stdev | 3.03 | 2.01 | 3.03 | 3.52 | 3.03 | 1.43 |
| p(t-test) | | 0.26 | | 0.078 | | 0.95 |
| Min | 0.00244 | 0.0181 | 0.00244 | 0.0378 | 0.00244 | 0.0268 |
| Max | 47.1 | 15.5 | 47.1 | 31.3 | 47.1 | 6.03 |
| n (Samp) | 486 | 156 | 486 | 135 | 486 | 46 |
| n (Patient) | 216 | 156 | 216 | 135 | 216 | 46 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.54 | 0.64 | 0.58 | 0.51 | 0.61 | 0.52 | 0.51 | 0.54 |
| SE | 0.026 | 0.040 | 0.026 | 0.028 | 0.041 | 0.028 | 0.043 | 0.049 | 0.045 |
| p | 3.7E-5 | 0.31 | 8.2E-8 | 0.0066 | 0.84 | 1.1E-4 | 0.61 | 0.83 | 0.38 |
| nCohort 1 | 475 | 1031 | 486 | 475 | 1031 | 486 | 475 | 1031 | 486 |
| nCohort 2 | 173 | 58 | 156 | 139 | 53 | 135 | 52 | 37 | 46 |
| Cutoff 1 | 0.364 | 0.308 | 0.424 | 0.322 | 0.276 | 0.350 | 0.202 | 0.393 | 0.261 |
| Sens 1 | 71% | 71% | 71% | 71% | 72% | 70% | 71% | 70% | 72% |
| Spec 1 | 49% | 34% | 55% | 44% | 32% | 48% | 30% | 42% | 38% |
| Cutoff 2 | 0.236 | 0.230 | 0.290 | 0.216 | 0.212 | 0.250 | 0.167 | 0.204 | 0.171 |
| Sens 2 | 80% | 81% | 80% | 81% | 81% | 80% | 81% | 81% | 80% |
| Spec 2 | 35% | 27% | 42% | 32% | 25% | 37% | 23% | 24% | 24% |
| Cutoff 3 | 0.138 | 0.126 | 0.142 | 0.111 | 0.0592 | 0.157 | 0.0499 | 0.0373 | 0.0892 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 90% | 91% | 90% | 91% | 91% | 90% | 90% | 92% | 91% |
| Spec 3 | 17% | 12% | 19% | 14% | 3% | 21% | 4% | 2% | 10% |
| Cutoff 4 | 0.701 | 0.962 | 0.715 | 0.701 | 0.962 | 0.715 | 0.701 | 0.962 | 0.715 |
| Sens 4 | 42% | 36% | 47% | 40% | 32% | 41% | 35% | 27% | 37% |
| Spec 4 | 70% | 70% | 70% | 70% | 70% | 70% | 70% | 70% | 70% |
| Cutoff 5 | 1.08 | 1.58 | 1.11 | 1.08 | 1.58 | 1.11 | 1.08 | 1.58 | 1.11 |
| Sens 5 | 28% | 24% | 33% | 27% | 23% | 29% | 27% | 11% | 30% |
| Spec 5 | 80% | 80% | 80% | 80% | 80% | 80% | 80% | 80% | 80% |
| Cutoff 6 | 2.02 | 2.84 | 1.96 | 2.02 | 2.84 | 1.96 | 2.02 | 2.84 | 1.96 |
| Sens 6 | 17% | 16% | 20% | 17% | 13% | 19% | 10% | 8% | 13% |
| Spec 6 | 90% | 90% | 90% | 90% | 90% | 90% | 90% | 90% | 90% |
| OR Quart 2 | 1.4 | 1.4 | 1.6 | 1.6 | 1.2 | 1.9 | 0.90 | 1.1 | 1.3 |
| p Value | 0.21 | 0.42 | 0.11 | 0.091 | 0.69 | 0.038 | 0.81 | 0.81 | 0.51 |
| 95% CI of | 0.82 | 0.62 | 0.90 | 0.92 | 0.53 | 1.0 | 0.38 | 0.43 | 0.56 |
| OR Quart2 | 2.5 | 3.1 | 3.0 | 2.9 | 2.6 | 3.5 | 2.1 | 3.0 | 3.2 |
| OR Quart 3 | 2.5 | 1.4 | 2.8 | 1.6 | 1.1 | 2.1 | 1.1 | 1.8 | 0.79 |
| p Value | 7.1E-4 | 0.42 | 3.4E-4 | 0.11 | 0.84 | 0.014 | 0.85 | 0.20 | 0.63 |
| 95% CI of | 1.5 | 0.62 | 1.6 | 0.90 | 0.49 | 1.2 | 0.47 | 0.74 | 0.30 |
| OR Quart3 | 4.1 | 3.1 | 5.0 | 2.8 | 2.4 | 3.8 | 2.5 | 4.3 | 2.1 |
| OR Quart 4 | 2.3 | 1.6 | 3.4 | 2.1 | 1.2 | 2.7 | 1.4 | 0.74 | 1.6 |
| p Value | 0.0016 | 0.25 | 1.7E-5 | 0.0072 | 0.69 | 0.0011 | 0.44 | 0.59 | 0.30 |
| 95% CI of | 1.4 | 0.72 | 2.0 | 1.2 | 0.53 | 1.5 | 0.62 | 0.25 | 0.68 |
| OR Quart4 | 3.9 | 3.4 | 6.0 | 3.8 | 2.6 | 4.8 | 3.0 | 2.2 | 3.6 |

**Keratin, type II cytoskeletal 1; type1 cytoskeletal 10 (Keratin-1,-10 mix)**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0174 | 0.0218 | 0.0174 | 0.0602 | 0.0174 | 0.00541 |
| Average | 3.87 | 1.53 | 3.87 | 4.35 | 3.87 | 0.704 |
| Stdev | 20.3 | 3.65 | 20.3 | 26.4 | 20.3 | 2.36 |
| p(t-test) | | 0.13 | | 0.82 | | 0.26 |
| Min | 0.000410 | 0.000410 | 0.000410 | 0.000410 | 0.000410 | 0.000410 |
| Max | 367 | 28.7 | 367 | 309 | 367 | 15.4 |
| n (Samp) | 475 | 173 | 475 | 139 | 475 | 52 |
| n (Patient) | 253 | 173 | 253 | 139 | 253 | 52 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0174 | 0.0174 | 0.0174 | 0.0153 | 0.0174 | 0.0744 |
| Average | 3.28 | 2.11 | 3.28 | 2.20 | 3.28 | 1.12 |
| Stdev | 18.7 | 5.58 | 18.7 | 5.26 | 18.7 | 2.19 |
| p(t-test) | | 0.64 | | 0.68 | | 0.48 |
| Min | 0.000410 | 0.000410 | 0.000410 | 0.000410 | 0.000410 | 0.000410 |
| Max | 367 | 28.7 | 367 | 27.8 | 367 | 11.8 |
| n (Samp) | 1031 | 58 | 1031 | 53 | 1031 | 37 |
| n (Patient) | 424 | 58 | 424 | 53 | 424 | 37 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0218 | 0.0196 | 0.0218 | 0.0602 | 0.0218 | 0.00896 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 3.17 | 1.62 | 3.17 | 4.20 | 3.17 | 0.804 |
| Stdev | 12.1 | 4.30 | 12.1 | 26.7 | 12.1 | 2.56 |
| p(t-test) | | 0.12 | | 0.52 | | 0.19 |
| Min | 0.000410 | 0.000410 | 0.000410 | 0.000410 | 0.000410 | 0.000410 |
| Max | 113 | 42.0 | 113 | 309 | 113 | 15.4 |
| n (Samp) | 486 | 156 | 486 | 135 | 486 | 46 |
| n (Patient) | 216 | 156 | 216 | 135 | 216 | 46 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | 0.49 | 0.48 | 0.52 | 0.49 | 0.51 | 0.31 | 0.47 | 0.33 |
| SE | 0.026 | 0.039 | 0.027 | 0.028 | 0.041 | 0.028 | 0.042 | 0.049 | 0.045 |
| p | 0.94 | 0.89 | 0.37 | 0.39 | 0.89 | 0.61 | 5.5E-6 | 0.49 | 2.1E-4 |
| nCohort 1 | 475 | 1031 | 486 | 475 | 1031 | 486 | 475 | 1031 | 486 |
| nCohort 2 | 173 | 58 | 156 | 139 | 53 | 135 | 52 | 37 | 46 |
| Cutoff 1 | 0.00562 | 0.00562 | 0.00521 | 0.00892 | 0.00686 | 0.00892 | 0 | 0.00408 | 0 |
| Sens 1 | 71% | 72% | 73% | 71% | 72% | 72% | 100% | 70% | 100% |
| Spec 1 | 23% | 27% | 21% | 34% | 29% | 33% | 0% | 12% | 0% |
| Cutoff 2 | 0.00474 | 0.00408 | 0.00474 | 0.00474 | 0.000693 | 0.00508 | 0 | 0 | 0 |
| Sens 2 | 82% | 84% | 81% | 81% | 85% | 80% | 100% | 100% | 100% |
| Spec 2 | 13% | 12% | 14% | 13% | 6% | 18% | 0% | 0% | 0% |
| Cutoff 3 | 0.000693 | 0.000693 | 0.000693 | 0 | 0 | 0 | 0 | 0 | 0 |
| Sens 3 | 94% | 93% | 92% | 100% | 100% | 100% | 100% | 100% | 100% |
| Spec 3 | 4% | 6% | 3% | 0% | 0% | 0% | 0% | 0% | 0% |
| Cutoff 4 | 0.981 | 1.21 | 1.21 | 0.981 | 1.21 | 1.21 | 0.981 | 1.21 | 1.21 |
| Sens 4 | 35% | 28% | 33% | 37% | 28% | 35% | 13% | 30% | 11% |
| Spec 4 | 71% | 70% | 70% | 71% | 70% | 70% | 71% | 70% | 70% |
| Cutoff 5 | 2.40 | 2.40 | 2.41 | 2.40 | 2.40 | 2.41 | 2.40 | 2.40 | 2.41 |
| Sens 5 | 21% | 22% | 19% | 24% | 21% | 21% | 8% | 14% | 9% |
| Spec 5 | 80% | 80% | 80% | 80% | 80% | 80% | 80% | 80% | 80% |
| Cutoff 6 | 6.14 | 5.06 | 6.14 | 6.14 | 5.06 | 6.14 | 6.14 | 5.06 | 6.14 |
| Sens 6 | 3% | 10% | 4% | 11% | 11% | 9% | 2% | 5% | 2% |
| Spec 6 | 91% | 90% | 91% | 91% | 90% | 91% | 91% | 90% | 91% |
| OR Quart 2 | 1.0 | 0.93 | 0.88 | 0.61 | 1.0 | 0.89 | 1.5 | 2.1 | 1.6 |
| p Value | 0.90 | 0.86 | 0.62 | 0.084 | 1.0 | 0.67 | 0.43 | 0.13 | 0.40 |
| 95% CI of OR Quart2 | 0.64 1.7 | 0.44 2.0 | 0.52 1.5 | 0.34 1.1 | 0.45 2.2 | 0.51 1.5 | 0.53 4.4 | 0.82 5.2 | 0.52 5.1 |
| OR Quart 3 | 0.53 | 0.93 | 0.75 | 0.96 | 1.0 | 1.0 | 1.9 | 0.42 | 2.8 |
| p Value | 0.018 | 0.86 | 0.29 | 0.89 | 1.0 | 0.89 | 0.22 | 0.21 | 0.060 |
| 95% CI of OR Quart3 | 0.31 0.90 | 0.44 2.0 | 0.44 1.3 | 0.57 1.6 | 0.45 2.2 | 0.60 1.8 | 0.68 5.3 | 0.11 1.6 | 0.96 8.0 |
| OR Quart 4 | 1.2 | 1.0 | 1.3 | 1.1 | 1.1 | 1.2 | 5.2 | 1.9 | 4.5 |
| p Value | 0.47 | 0.99 | 0.30 | 0.62 | 0.84 | 0.52 | 4.8E-4 | 0.18 | 0.0034 |
| 95% CI of OR Quart4 | 0.74 1.9 | 0.48 2.1 | 0.79 2.1 | 0.68 1.9 | 0.50 2.3 | 0.70 2.0 | 2.1 13 | 0.75 4.8 | 1.6 12 |

**Keratin, type II cytoskeletal 6 (6A, -6B, -6C mix)**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0252 | 0.111 | 0.0252 | 0.0491 | 0.0252 | 0.0134 |
| Average | 2.82 | 2.64 | 2.82 | 2.58 | 2.82 | 2.78 |
| Stdev | 8.19 | 6.71 | 8.19 | 6.03 | 8.19 | 8.48 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.80 | | 0.74 | | 0.97 |
| Min | 8.79E-5 | 8.79E-5 | 8.79E-5 | 8.79E-5 | 8.79E-5 | 8.79E-5 |
| Max | 89.9 | 48.4 | 89.9 | 38.6 | 89.9 | 46.7 |
| n (Samp) | 475 | 173 | 475 | 139 | 475 | 52 |
| n (Patient) | 253 | 173 | 253 | 139 | 253 | 52 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0252 | 0.0801 | 0.0252 | 0.0462 | 0.0252 | 0.129 |
| Average | 3.10 | 3.18 | 3.10 | 2.70 | 3.10 | 3.66 |
| Stdev | 13.8 | 7.61 | 13.8 | 6.98 | 13.8 | 8.81 |
| p(t-test) | | 0.96 | | 0.83 | | 0.80 |
| Min | 8.79E-5 | 8.79E-5 | 8.79E-5 | 8.79E-5 | 8.79E-5 | 8.79E-5 |
| Max | 265 | 48.4 | 265 | 38.6 | 265 | 46.7 |
| n (Samp) | 1031 | 58 | 1031 | 53 | 1031 | 37 |
| n (Patient) | 424 | 58 | 424 | 53 | 424 | 37 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0252 | 0.111 | 0.0252 | 0.111 | 0.0252 | 0.0177 |
| Average | 2.34 | 2.31 | 2.34 | 2.39 | 2.34 | 1.98 |
| Stdev | 6.98 | 5.78 | 6.98 | 5.19 | 6.98 | 6.11 |
| p(t-test) | | 0.96 | | 0.94 | | 0.74 |
| Min | 8.79E-5 | 8.79E-5 | 8.79E-5 | 8.79E-5 | 8.79E-5 | 8.79E-5 |
| Max | 89.9 | 37.9 | 89.9 | 33.8 | 89.9 | 34.5 |
| n (Samp) | 486 | 156 | 486 | 135 | 486 | 46 |
| n (Patient) | 216 | 156 | 216 | 135 | 216 | 46 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | 0.52 | 0.49 | 0.48 | 0.50 | 0.51 | 0.35 | 0.50 | 0.38 |
| SE | 0.026 | 0.039 | 0.027 | 0.028 | 0.041 | 0.028 | 0.043 | 0.048 | 0.046 |
| p | 0.70 | 0.59 | 0.76 | 0.55 | 0.91 | 0.85 | 3.7E-4 | 0.97 | 0.0093 |
| nCohort 1 | 475 | 1031 | 486 | 475 | 1031 | 486 | 475 | 1031 | 486 |
| nCohort 2 | 173 | 58 | 156 | 139 | 53 | 135 | 52 | 37 | 46 |
| Cutoff 1 | 0.0134 | 0.0134 | 0.0134 | 0.0134 | 0.0134 | 0.0134 | 0.000154 | 0.0127 | 0.000220 |
| Sens 1 | 73% | 71% | 71% | 72% | 72% | 72% | 71% | 70% | 72% |
| Spec 1 | 25% | 29% | 28% | 25% | 29% | 28% | 3% | 23% | 7% |
| Cutoff 2 | 0.0106 | 0.00903 | 0.0106 | 0.00903 | 0.0106 | 0.00903 | 0 | 0.00821 | 0 |
| Sens 2 | 83% | 84% | 81% | 83% | 81% | 84% | 100% | 81% | 100% |
| Spec 2 | 16% | 15% | 16% | 12% | 18% | 13% | 0% | 12% | 0% |
| Cutoff 3 | 0.000220 | 0.00533 | 0.000154 | 0 | 0 | 0.000154 | 0 | 0 | 0 |
| Sens 3 | 92% | 93% | 94% | 100% | 100% | 90% | 100% | 100% | 100% |
| Spec 3 | 6% | 9% | 3% | 0% | 0% | 3% | 0% | 0% | 0% |
| Cutoff 4 | 0.758 | 0.440 | 0.547 | 0.758 | 0.440 | 0.547 | 0.758 | 0.440 | 0.547 |
| Sens 4 | 28% | 33% | 28% | 28% | 26% | 30% | 21% | 46% | 20% |
| Spec 4 | 70% | 70% | 71% | 70% | 70% | 71% | 70% | 70% | 71% |
| Cutoff 5 | 3.08 | 2.93 | 2.77 | 3.08 | 2.93 | 2.77 | 3.08 | 2.93 | 2.77 |
| Sens 5 | 19% | 28% | 20% | 21% | 19% | 21% | 15% | 22% | 13% |
| Spec 5 | 80% | 80% | 80% | 80% | 80% | 80% | 80% | 80% | 80% |
| Cutoff 6 | 7.99 | 8.01 | 6.75 | 7.99 | 8.01 | 6.75 | 7.99 | 8.01 | 6.75 |
| Sens 6 | 12% | 12% | 12% | 12% | 9% | 13% | 8% | 11% | 9% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 6 | 90% | 90% | 90% | 90% | 90% | 90% | 90% | 90% | 90% |
| OR Quart 2 | 1.4 | 0.57 | 1.5 | 1.2 | 1.5 | 0.69 | 0.79 | 0.57 | 1.4 |
| p Value | 0.17 | 0.17 | 0.15 | 0.48 | 0.33 | 0.20 | 0.63 | 0.25 | 0.48 |
| 95% CI of | 0.86 | 0.26 | 0.87 | 0.71 | 0.68 | 0.39 | 0.30 | 0.22 | 0.55 |
| OR Quart2 | 2.3 | 1.3 | 2.4 | 2.0 | 3.3 | 1.2 | 2.1 | 1.5 | 3.6 |
| OR Quart 3 | 0.84 | 0.81 | 0.96 | 0.85 | 1.2 | 1.1 | 0.79 | 0.49 | 0.74 |
| p Value | 0.51 | 0.58 | 0.89 | 0.57 | 0.68 | 0.69 | 0.63 | 0.16 | 0.58 |
| 95% CI of | 0.50 | 0.39 | 0.57 | 0.49 | 0.52 | 0.66 | 0.30 | 0.18 | 0.25 |
| OR Quart3 | 1.4 | 1.7 | 1.6 | 1.5 | 2.7 | 1.9 | 2.1 | 1.3 | 2.2 |
| OR Quart 4 | 1.2 | 1.00 | 1.2 | 1.1 | 1.2 | 1.0 | 3.0 | 1.0 | 2.9 |
| p Value | 0.38 | 0.99 | 0.41 | 0.76 | 0.68 | 0.92 | 0.0051 | 1.0 | 0.014 |
| 95% CI of | 0.76 | 0.50 | 0.74 | 0.64 | 0.52 | 0.61 | 1.4 | 0.44 | 1.2 |
| OR Quart4 | 2.0 | 2.0 | 2.1 | 1.9 | 2.7 | 1.7 | 6.6 | 2.3 | 6.9 |

**NF-kappa-B inhibitor alpha**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000108 | 3.20E-5 | 0.000108 | 9.81E-5 | 0.000108 | 2.25E-5 |
| Average | 0.00480 | 0.00307 | 0.00480 | 0.00666 | 0.00480 | 0.00602 |
| Stdev | 0.0236 | 0.0194 | 0.0236 | 0.0330 | 0.0236 | 0.0201 |
| p(t-test) | | 0.48 | | 0.58 | | 0.85 |
| Min | 3.52E-7 | 3.52E-7 | 3.52E-7 | 3.52E-7 | 3.52E-7 | 3.80E-7 |
| Max | 0.211 | 0.203 | 0.211 | 0.245 | 0.211 | 0.0782 |
| n (Samp) | 237 | 125 | 237 | 82 | 237 | 15 |
| n (Patient) | 108 | 125 | 108 | 82 | 108 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.81E-5 | 0.000176 | 9.81E-5 | 0.000108 | 9.81E-5 | 6.66E-6 |
| Average | 0.00443 | 0.00236 | 0.00443 | 0.00796 | 0.00443 | 0.00985 |
| Stdev | 0.0230 | 0.0117 | 0.0230 | 0.0363 | 0.0230 | 0.0396 |
| p(t-test) | | 0.58 | | 0.42 | | 0.33 |
| Min | 3.52E-7 | 3.52E-7 | 3.52E-7 | 3.65E-7 | 3.52E-7 | 3.65E-7 |
| Max | 0.245 | 0.0725 | 0.245 | 0.203 | 0.245 | 0.173 |
| n (Samp) | 591 | 38 | 591 | 31 | 591 | 19 |
| n (Patient) | 229 | 38 | 229 | 31 | 229 | 19 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000190 | 3.20E-5 | 0.000190 | 9.81E-5 | 0.000190 | 1.32E-5 |
| Average | 0.00460 | 0.00341 | 0.00460 | 0.00638 | 0.00460 | 0.00527 |
| Stdev | 0.0218 | 0.0206 | 0.0218 | 0.0327 | 0.0218 | 0.0195 |
| p(t-test) | | 0.62 | | 0.56 | | 0.90 |
| Min | 3.52E-7 | 3.52E-7 | 3.52E-7 | 3.52E-7 | 3.52E-7 | 3.80E-7 |
| Max | 0.211 | 0.203 | 0.211 | 0.245 | 0.211 | 0.0782 |
| n (Samp) | 294 | 110 | 294 | 84 | 294 | 16 |
| n (Patient) | 123 | 110 | 123 | 84 | 123 | 16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.44 | 0.51 | 0.43 | 0.50 | 0.55 | 0.47 | 0.40 | 0.45 | 0.38 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| SE | 0.032 | 0.049 | 0.033 | 0.037 | 0.055 | 0.036 | 0.079 | 0.069 | 0.077 |
| p | 0.081 | 0.90 | 0.026 | 0.94 | 0.34 | 0.45 | 0.22 | 0.46 | 0.13 |
| nCohort 1 | 237 | 591 | 294 | 237 | 591 | 294 | 237 | 591 | 294 |
| nCohort 2 | 125 | 38 | 110 | 82 | 31 | 84 | 15 | 19 | 16 |
| Cutoff 1 | 1.03E-6 | 3.95E-6 | 1.03E-6 | 3.95E-6 | 2.18E-5 | 1.17E-6 | 4.18E-7 | 5.05E-7 | 4.18E-7 |
| Sens 1 | 73% | 71% | 70% | 72% | 71% | 73% | 73% | 79% | 81% |
| Spec 1 | 22% | 33% | 21% | 28% | 36% | 26% | 13% | 19% | 13% |
| Cutoff 2 | 4.18E-7 | 1.03E-6 | 4.18E-7 | 1.03E-6 | 1.03E-6 | 5.23E-7 | 4.17E-7 | 4.18E-7 | 4.18E-7 |
| Sens 2 | 82% | 82% | 81% | 80% | 84% | 83% | 80% | 84% | 81% |
| Spec 2 | 13% | 28% | 13% | 22% | 28% | 18% | 9% | 19% | 13% |
| Cutoff 3 | 3.80E-7 | 3.65E-7 | 3.80E-7 | 3.80E-7 | 3.80E-7 | 3.65E-7 | 3.52E-7 | 3.80E-7 | 3.65E-7 |
| Sens 3 | 90% | 92% | 92% | 90% | 97% | 90% | 100% | 95% | 100% |
| Spec 3 | 5% | 5% | 4% | 5% | 8% | 3% | 3% | 8% | 3% |
| Cutoff 4 | 0.000613 | 0.000532 | 0.000614 | 0.000613 | 0.000532 | 0.000614 | 0.000613 | 0.000532 | 0.000614 |
| Sens 4 | 22% | 32% | 18% | 29% | 39% | 27% | 27% | 26% | 19% |
| Spec 4 | 71% | 70% | 72% | 71% | 70% | 72% | 71% | 70% | 72% |
| Cutoff 5 | 0.00113 | 0.00109 | 0.00128 | 0.00113 | 0.00109 | 0.00128 | 0.00113 | 0.00109 | 0.00128 |
| Sens 5 | 13% | 18% | 12% | 23% | 29% | 19% | 27% | 26% | 19% |
| Spec 5 | 81% | 80% | 80% | 81% | 80% | 80% | 81% | 80% | 80% |
| Cutoff 6 | 0.00284 | 0.00274 | 0.00384 | 0.00284 | 0.00274 | 0.00384 | 0.00284 | 0.00274 | 0.00384 |
| Sens 6 | 7% | 5% | 5% | 15% | 16% | 12% | 13% | 11% | 12% |
| Spec 6 | 90% | 90% | 90% | 90% | 90% | 90% | 90% | 90% | 90% |
| OR Quart 2 | 1.4 | 1.3 | 1.6 | 0.77 | 2.6 | 1.0 | 0.24 | 0.60 | 0.33 |
| p Value | 0.32 | 0.61 | 0.18 | 0.47 | 0.12 | 0.97 | 0.20 | 0.48 | 0.34 |
| 95% CI of OR Quart2 | 0.74 – 2.6 | 0.47 – 3.6 | 0.81 – 3.1 | 0.37 – 1.6 | 0.79 – 8.4 | 0.50 – 2.1 | 0.026 – 2.2 | 0.14 – 2.5 | 0.033 – 3.2 |
| OR Quart 3 | 1.0 | 1.8 | 1.7 | 0.94 | 1.8 | 1.4 | 0.74 | 1.0 | 1.7 |
| p Value | 1.0 | 0.24 | 0.10 | 0.86 | 0.36 | 0.30 | 0.70 | 1.0 | 0.47 |
| 95% CI of OR Quart3 | 0.53 – 1.9 | 0.68 – 4.6 | 0.90 – 3.4 | 0.47 – 1.9 | 0.51 – 6.2 | 0.72 – 2.8 | 0.16 – 3.4 | 0.28 – 3.5 | 0.39 – 7.4 |
| OR Quart 4 | 1.7 | 1.4 | 2.3 | 0.95 | 2.6 | 1.2 | 1.8 | 1.2 | 2.5 |
| p Value | 0.080 | 0.46 | 0.012 | 0.90 | 0.12 | 0.69 | 0.35 | 0.75 | 0.20 |
| 95% CI of OR Quart4 | 0.94 – 3.2 | 0.54 – 3.9 | 1.2 – 4.4 | 0.47 – 1.9 | 0.79 – 8.4 | 0.57 – 2.3 | 0.51 – 6.6 | 0.36 – 4.1 | 0.62 – 10 |

**Beta-nerve growth factor**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 2.78 | 1.90 | 2.78 | 2.30 | 2.78 | 1.12 |
| Average | 4.11 | 3.29 | 4.11 | 3.54 | 4.11 | 3.40 |
| Stdev | 5.11 | 4.60 | 5.11 | 4.26 | 5.11 | 7.81 |
| p(t-test) |  | 0.13 |  | 0.37 |  | 0.61 |
| Min | 0.000201 | 0.000201 | 0.000201 | 0.000499 | 0.000201 | 0.000201 |
| Max | 44.0 | 35.5 | 44.0 | 25.2 | 44.0 | 32.2 |
| n (Samp) | 238 | 124 | 238 | 82 | 238 | 16 |
| n (Patient) | 111 | 124 | 111 | 82 | 111 | 16 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 2.40 | 1.89 | 2.40 | 2.83 | 2.40 | 1.04 |
| Average | 3.90 | 3.10 | 3.90 | 3.12 | 3.90 | 4.21 |
| Stdev | 5.26 | 2.83 | 5.26 | 2.80 | 5.26 | 7.95 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.36 | | 0.42 | | 0.80 |
| Min | 0.000201 | 0.000201 | 0.000201 | 0.000506 | 0.000201 | 0.000201 |
| Max | 46.1 | 10.5 | 46.1 | 13.2 | 46.1 | 32.2 |
| n (Samp) | 593 | 37 | 593 | 31 | 593 | 19 |
| n (Patient) | 231 | 37 | 231 | 31 | 231 | 19 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.59 | 1.95 | 2.59 | 2.30 | 2.59 | 1.79 |
| Average | 3.80 | 3.34 | 3.80 | 3.68 | 3.80 | 3.18 |
| Stdev | 4.62 | 4.77 | 4.62 | 4.27 | 4.62 | 3.50 |
| p(t-test) | | 0.38 | | 0.82 | | 0.60 |
| Min | 0.000201 | 0.000201 | 0.000201 | 0.000499 | 0.000201 | 0.000201 |
| Max | 32.2 | 35.5 | 32.2 | 25.2 | 32.2 | 11.7 |
| n (Samp) | 296 | 110 | 296 | 84 | 296 | 16 |
| n (Patient) | 126 | 110 | 126 | 84 | 126 | 16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.44 | 0.49 | 0.46 | 0.46 | 0.50 | 0.49 | 0.33 | 0.38 | 0.46 |
| SE | 0.032 | 0.049 | 0.033 | 0.037 | 0.053 | 0.036 | 0.076 | 0.070 | 0.076 |
| p | 0.051 | 0.80 | 0.19 | 0.26 | 0.93 | 0.80 | 0.027 | 0.099 | 0.56 |
| nCohort 1 | 238 | 593 | 296 | 238 | 593 | 296 | 238 | 593 | 296 |
| nCohort 2 | 124 | 37 | 110 | 82 | 31 | 84 | 16 | 19 | 16 |
| Cutoff 1 | 0.931 | 1.43 | 0.931 | 1.12 | 1.33 | 1.34 | 0.685 | 0.397 | 0.931 |
| Sens 1 | 71% | 70% | 71% | 71% | 71% | 70% | 75% | 74% | 75% |
| Spec 1 | 20% | 36% | 22% | 24% | 34% | 32% | 17% | 13% | 22% |
| Cutoff 2 | 0.552 | 0.365 | 0.596 | 0.731 | 0.834 | 0.771 | 0.397 | 0.000499 | 0.685 |
| Sens 2 | 81% | 81% | 80% | 80% | 81% | 81% | 81% | 89% | 81% |
| Spec 2 | 15% | 12% | 17% | 17% | 22% | 19% | 12% | 3% | 18% |
| Cutoff 3 | 0.0589 | 0.000506 | 0.261 | 0.397 | 0.111 | 0.397 | 0.0368 | 0.000244 | 0.0368 |
| Sens 3 | 90% | 95% | 90% | 90% | 90% | 90% | 94% | 95% | 94% |
| Spec 3 | 9% | 4% | 11% | 12% | 9% | 12% | 8% | 1% | 8% |
| Cutoff 4 | 4.36 | 3.90 | 4.02 | 4.36 | 3.90 | 4.02 | 4.36 | 3.90 | 4.02 |
| Sens 4 | 23% | 32% | 25% | 22% | 26% | 27% | 12% | 21% | 31% |
| Spec 4 | 70% | 70% | 70% | 70% | 70% | 70% | 70% | 70% | 70% |
| Cutoff 5 | 6.12 | 5.41 | 5.24 | 6.12 | 5.41 | 5.24 | 6.12 | 5.41 | 5.24 |
| Sens 5 | 13% | 19% | 15% | 16% | 13% | 21% | 6% | 16% | 25% |
| Spec 5 | 80% | 80% | 80% | 80% | 80% | 80% | 80% | 80% | 80% |
| Cutoff 6 | 9.10 | 9.08 | 8.06 | 9.10 | 9.08 | 8.06 | 9.10 | 9.08 | 8.06 |
| Sens 6 | 6% | 3% | 10% | 7% | 3% | 14% | 6% | 16% | 12% |
| Spec 6 | 90% | 90% | 90% | 90% | 90% | 90% | 90% | 90% | 90% |
| OR Quart 2 | 1.3 | 1.0 | 0.91 | 1.2 | 1.0 | 0.94 | 1.0 | 0.49 | 0.19 |
| p Value | 0.39 | 0.99 | 0.78 | 0.71 | 1.0 | 0.86 | 0.99 | 0.42 | 0.13 |
| 95% CI of OR Quart2 | 0.70 | 0.39 | 0.49 | 0.55 | 0.34 | 0.46 | 0.14 | 0.089 | 0.022 |
| | 2.5 | 2.6 | 1.7 | 2.4 | 2.9 | 1.9 | 7.4 | 2.7 | 1.7 |
| OR Quart 3 | 1.4 | 1.0 | 0.85 | 1.4 | 1.5 | 1.3 | 2.1 | 1.3 | 1.0 |
| p Value | 0.34 | 1.0 | 0.63 | 0.36 | 0.46 | 0.39 | 0.41 | 0.74 | 1.0 |
| 95% CI of OR Quart3 | 0.72 | 0.39 | 0.45 | 0.68 | 0.54 | 0.68 | 0.36 | 0.33 | 0.28 |
| | 2.6 | 2.6 | 1.6 | 2.9 | 3.9 | 2.6 | 12 | 4.8 | 3.6 |
| OR Quart 4 | 1.9 | 1.1 | 1.4 | 1.6 | 1.0 | 1.0 | 4.5 | 2.1 | 1.0 |
| p Value | 0.038 | 0.80 | 0.26 | 0.21 | 1.0 | 1.0 | 0.064 | 0.25 | 1.0 |
| 95% CI of | 1.0 | 0.44 | 0.77 | 0.77 | 0.34 | 0.50 | 0.92 | 0.61 | 0.28 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart4 | 3.6 | 2.9 | 2.6 | 3.3 | 2.9 | 2.0 | 22 | 7.0 | 3.6 |

**Pancreatic prohormone**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.15 | 21.0 | 1.15 | 18.5 | 1.15 | 19.3 |
| Average | 87.8 | 106 | 87.8 | 70.5 | 87.8 | 53.3 |
| Stdev | 190 | 209 | 190 | 134 | 190 | 125 |
| p(t-test) |  | 0.59 |  | 0.56 |  | 0.38 |
| Min | 0.117 | 0.117 | 0.117 | 0.117 | 0.117 | 0.117 |
| Max | 950 | 1020 | 950 | 690 | 950 | 613 |
| n (Samp) | 122 | 47 | 122 | 50 | 122 | 26 |
| n (Patient) | 98 | 47 | 98 | 50 | 98 | 26 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 5.31 | 23.4 | 5.31 | 17.2 | 5.31 | 13.4 |
| Average | 87.2 | 87.5 | 87.2 | 109 | 87.2 | 79.2 |
| Stdev | 190 | 131 | 190 | 183 | 190 | 138 |
| p(t-test) |  | 0.99 |  | 0.63 |  | 0.88 |
| Min | 0.117 | 0.122 | 0.117 | 0.117 | 0.117 | 0.117 |
| Max | 1220 | 396 | 1220 | 728 | 1220 | 448 |
| n (Samp) | 261 | 14 | 261 | 19 | 261 | 13 |
| n (Patient) | 159 | 14 | 159 | 19 | 159 | 13 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.15 | 24.8 | 1.15 | 23.0 | 1.15 | 23.3 |
| Average | 82.5 | 109 | 82.5 | 87.7 | 82.5 | 72.3 |
| Stdev | 179 | 209 | 179 | 155 | 179 | 149 |
| p(t-test) |  | 0.44 |  | 0.86 |  | 0.80 |
| Min | 0.117 | 0.117 | 0.117 | 0.117 | 0.117 | 0.117 |
| Max | 950 | 1020 | 950 | 690 | 950 | 613 |
| n (Samp) | 111 | 44 | 111 | 46 | 111 | 23 |
| n (Patient) | 84 | 44 | 84 | 46 | 84 | 23 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.59 | 0.56 | 0.60 | 0.55 | 0.52 | 0.56 | 0.52 | 0.50 | 0.52 |
| SE | 0.050 | 0.081 | 0.052 | 0.049 | 0.069 | 0.051 | 0.063 | 0.082 | 0.067 |
| p | 0.058 | 0.45 | 0.050 | 0.28 | 0.77 | 0.23 | 0.73 | 0.96 | 0.72 |
| nCohort 1 | 122 | 261 | 111 | 122 | 261 | 111 | 122 | 261 | 111 |
| nCohort 2 | 47 | 14 | 44 | 50 | 19 | 46 | 26 | 13 | 23 |
| Cutoff 1 | 1.15 | 1.15 | 3.15 | 0.635 | 0.122 | 1.15 | 0.293 | 0.134 | 0.293 |
| Sens 1 | 79% | 71% | 70% | 76% | 74% | 72% | 77% | 77% | 74% |
| Spec 1 | 45% | 39% | 52% | 36% | 16% | 39% | 36% | 23% | 29% |
| Cutoff 2 | 0.293 | 0.122 | 1.15 | 0.134 | 0.117 | 0.134 | 0.117 | 0.117 | 0.117 |
| Sens 2 | 81% | 93% | 82% | 80% | 89% | 83% | 88% | 92% | 87% |
| Spec 2 | 36% | 16% | 39% | 22% | 9% | 20% | 7% | 9% | 6% |
| Cutoff 3 | 0.122 | 0.122 | 0.122 | 0.117 | 0 | 0.117 | 0 | 0.117 | 0 |
| Sens 3 | 91% | 93% | 93% | 92% | 100% | 91% | 100% | 92% | 100% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 15% | 16% | 11% | 7% | 0% | 6% | 0% | 9% | 0% |
| Cutoff 4 | 35.9 | 39.2 | 41.8 | 35.9 | 39.2 | 41.8 | 35.9 | 39.2 | 41.8 |
| Sens 4 | 43% | 36% | 36% | 36% | 42% | 39% | 27% | 31% | 26% |
| Spec 4 | 70% | 70% | 71% | 70% | 70% | 71% | 70% | 70% | 71% |
| Cutoff 5 | 96.3 | 89.0 | 101 | 96.3 | 89.0 | 101 | 96.3 | 89.0 | 101 |
| Sens 5 | 21% | 29% | 23% | 20% | 37% | 22% | 12% | 31% | 13% |
| Spec 5 | 80% | 80% | 80% | 80% | 80% | 80% | 80% | 80% | 80% |
| Cutoff 6 | 310 | 310 | 286 | 310 | 310 | 286 | 310 | 310 | 286 |
| Sens 6 | 13% | 7% | 11% | 6% | 11% | 11% | 4% | 8% | 9% |
| Spec 6 | 90% | 90% | 90% | 90% | 90% | 90% | 90% | 90% | 90% |
| OR Quart 2 | 0.73 | 0.24 | 1.1 | 0.66 | 0.48 | 0.53 | 0.46 | 0.98 | 0.28 |
| p Value | 0.58 | 0.20 | 0.82 | 0.44 | 0.31 | 0.27 | 0.29 | 0.99 | 0.14 |
| 95% CI of OR Quart2 | 0.25 | 0.026 | 0.37 | 0.24 | 0.11 | 0.17 | 0.10 | 0.19 | 0.052 |
| | 2.2 | 2.2 | 3.5 | 1.9 | 2.0 | 1.6 | 2.0 | 5.1 | 1.5 |
| OR Quart 3 | 2.8 | 0.98 | 3.1 | 2.3 | 0.31 | 2.5 | 2.5 | 1.0 | 2.2 |
| p Value | 0.038 | 0.98 | 0.034 | 0.073 | 0.16 | 0.062 | 0.11 | 1.0 | 0.16 |
| 95% CI of OR Quart3 | 1.1 | 0.24 | 1.1 | 0.93 | 0.061 | 0.96 | 0.82 | 0.19 | 0.72 |
| | 7.2 | 4.1 | 8.7 | 5.7 | 1.6 | 6.5 | 7.5 | 5.1 | 7.1 |
| OR Quart 4 | 1.6 | 1.2 | 2.2 | 1.1 | 1.4 | 1.2 | 0.81 | 1.3 | 0.60 |
| p Value | 0.36 | 0.75 | 0.14 | 0.81 | 0.57 | 0.67 | 0.74 | 0.71 | 0.46 |
| 95% CI of OR Quart4 | 0.59 | 0.32 | 0.77 | 0.43 | 0.45 | 0.46 | 0.22 | 0.29 | 0.15 |
| | 4.2 | 4.9 | 6.4 | 2.9 | 4.2 | 3.3 | 2.9 | 6.2 | 2.4 |

**Transthyretin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 20.7 | 29.7 | 20.7 | 25.4 | 20.7 | 17.9 |
| Average | 43.5 | 55.9 | 43.5 | 50.7 | 43.5 | 28.3 |
| Stdev | 68.4 | 87.0 | 68.4 | 79.2 | 68.4 | 30.6 |
| p(t-test) | | 0.11 | | 0.38 | | 0.18 |
| Min | 0.0796 | 0.486 | 0.0796 | 0.773 | 0.0796 | 0.000148 |
| Max | 668 | 677 | 668 | 551 | 668 | 145 |
| n (Samp) | 271 | 140 | 271 | 107 | 271 | 38 |
| n (Patient) | 151 | 140 | 151 | 107 | 151 | 38 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.3 | 36.1 | 24.3 | 31.4 | 24.3 | 23.0 |
| Average | 50.7 | 71.9 | 50.7 | 87.9 | 50.7 | 48.7 |
| Stdev | 126 | 159 | 126 | 144 | 126 | 66.6 |
| p(t-test) | | 0.31 | | 0.076 | | 0.94 |
| Min | 0.000148 | 1.04 | 0.000148 | 2.72 | 0.000148 | 0.773 |
| Max | 2000 | 997 | 2000 | 648 | 2000 | 241 |
| n (Samp) | 667 | 40 | 667 | 39 | 667 | 24 |
| n (Patient) | 290 | 40 | 290 | 39 | 290 | 24 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 23.0 | 30.3 | 23.0 | 26.9 | 23.0 | 20.0 |
| Average | 47.3 | 59.4 | 47.3 | 45.7 | 47.3 | 31.2 |
| Stdev | 72.7 | 90.9 | 72.7 | 59.9 | 72.7 | 34.5 |
| p(t-test) | | 0.14 | | 0.83 | | 0.20 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.0796 | 0.486 | 0.0796 | 0.773 | 0.0796 | 0.000148 |
| Max | 668 | 677 | 668 | 396 | 668 | 182 |
| n (Samp) | 316 | 127 | 316 | 104 | 316 | 35 |
| n (Patient) | 155 | 127 | 155 | 104 | 155 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.56 | 0.56 | 0.55 | 0.58 | 0.53 | 0.46 | 0.48 | 0.47 |
| SE | 0.030 | 0.048 | 0.031 | 0.033 | 0.049 | 0.033 | 0.051 | 0.061 | 0.052 |
| p | 0.022 | 0.20 | 0.062 | 0.17 | 0.089 | 0.39 | 0.42 | 0.75 | 0.51 |
| nCohort 1 | 271 | 667 | 316 | 271 | 667 | 316 | 271 | 667 | 316 |
| nCohort 2 | 140 | 40 | 127 | 107 | 39 | 104 | 38 | 24 | 35 |
| Cutoff 1 | 16.1 | 17.5 | 16.4 | 14.1 | 18.9 | 14.1 | 10.3 | 8.99 | 13.5 |
| Sens 1 | 70% | 70% | 70% | 70% | 72% | 70% | 71% | 71% | 71% |
| Spec 1 | 40% | 39% | 37% | 37% | 42% | 33% | 27% | 20% | 32% |
| Cutoff 2 | 10.2 | 13.6 | 10.8 | 10.7 | 9.87 | 11.7 | 8.81 | 7.39 | 9.58 |
| Sens 2 | 80% | 80% | 80% | 80% | 82% | 81% | 82% | 83% | 80% |
| Spec 2 | 27% | 30% | 26% | 28% | 22% | 28% | 25% | 16% | 24% |
| Cutoff 3 | 5.50 | 6.53 | 5.37 | 6.06 | 6.13 | 7.62 | 4.25 | 3.99 | 8.58 |
| Sens 3 | 90% | 90% | 91% | 91% | 92% | 90% | 92% | 92% | 91% |
| Spec 3 | 16% | 15% | 14% | 18% | 14% | 20% | 13% | 9% | 22% |
| Cutoff 4 | 41.3 | 45.6 | 47.3 | 41.3 | 45.6 | 47.3 | 41.3 | 45.6 | 47.3 |
| Sens 4 | 39% | 38% | 37% | 31% | 36% | 30% | 21% | 33% | 17% |
| Spec 4 | 70% | 70% | 70% | 70% | 70% | 70% | 70% | 70% | 70% |
| Cutoff 5 | 63.4 | 63.8 | 66.2 | 63.4 | 63.8 | 66.2 | 63.4 | 63.8 | 66.2 |
| Sens 5 | 21% | 20% | 22% | 21% | 28% | 18% | 13% | 29% | 11% |
| Spec 5 | 80% | 80% | 80% | 80% | 80% | 80% | 80% | 80% | 80% |
| Cutoff 6 | 92.5 | 98.6 | 109 | 92.5 | 98.6 | 109 | 92.5 | 98.6 | 109 |
| Sens 6 | 14% | 15% | 12% | 14% | 23% | 10% | 5% | 12% | 3% |
| Spec 6 | 90% | 90% | 90% | 90% | 90% | 90% | 90% | 90% | 90% |
| OR Quart 2 | 1.1 | 1.1 | 1.0 | 1.3 | 0.74 | 1.6 | 1.3 | 0.42 | 3.3 |
| p Value | 0.79 | 0.81 | 0.90 | 0.42 | 0.58 | 0.14 | 0.58 | 0.21 | 0.045 |
| 95% CI of | 0.59 | 0.42 | 0.56 | 0.68 | 0.25 | 0.85 | 0.47 | 0.11 | 1.0 |
| OR Quart2 | 2.0 | 3.0 | 1.9 | 2.5 | 2.2 | 3.1 | 3.8 | 1.6 | 11 |
| OR Quart 3 | 1.8 | 1.4 | 1.9 | 1.8 | 1.8 | 1.6 | 2.1 | 0.71 | 3.0 |
| p Value | 0.047 | 0.49 | 0.033 | 0.076 | 0.19 | 0.14 | 0.15 | 0.56 | 0.069 |
| 95% CI of | 1.0 | 0.55 | 1.1 | 0.94 | 0.74 | 0.85 | 0.77 | 0.22 | 0.92 |
| OR Quart3 | 3.2 | 3.5 | 3.4 | 3.4 | 4.4 | 3.1 | 5.5 | 2.3 | 9.8 |
| OR Quart 4 | 1.4 | 1.5 | 1.4 | 1.5 | 1.4 | 1.4 | 1.3 | 1.3 | 2.1 |
| p Value | 0.25 | 0.37 | 0.31 | 0.26 | 0.49 | 0.32 | 0.58 | 0.60 | 0.23 |
| 95% CI of | 0.78 | 0.61 | 0.75 | 0.75 | 0.55 | 0.72 | 0.47 | 0.48 | 0.62 |
| OR Quart4 | 2.5 | 3.8 | 2.5 | 2.8 | 3.5 | 2.7 | 3.8 | 3.6 | 7.3 |

**C-peptide Urine**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 25100 | 20600 | 25100 | 14300 | 25100 | 19800 |
| Average | 36100 | 27800 | 36100 | 27800 | 36100 | 30800 |
| Stdev | 30900 | 26000 | 30900 | 28800 | 30900 | 26800 |
| p(t-test) | | 0.0067 | | 0.016 | | 0.31 |
| Min | 0.00978 | 83.6 | 0.00978 | 53.8 | 0.00978 | 1500 |
| Max | 102000 | 100000 | 102000 | 103000 | 102000 | 93300 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 271 | 140 | 271 | 108 | 271 | 38 |
| n (Patient) | 151 | 140 | 151 | 108 | 151 | 38 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22200 | 12200 | 22200 | 18000 | 22200 | 26300 |
| Average | 32800 | 26900 | 32800 | 25600 | 32800 | 35700 |
| Stdev | 29800 | 28600 | 29800 | 26000 | 29800 | 28800 |
| p(t-test) | | 0.23 | | 0.14 | | 0.63 |
| Min | 0.00978 | 0.0614 | 0.00978 | 0.0955 | 0.00978 | 53.8 |
| Max | 114000 | 100000 | 114000 | 100000 | 114000 | 100000 |
| n (Samp) | 666 | 40 | 666 | 39 | 666 | 25 |
| n (Patient) | 290 | 40 | 290 | 39 | 290 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24400 | 21400 | 24400 | 13400 | 24400 | 16400 |
| Average | 34800 | 28500 | 34800 | 25700 | 34800 | 25300 |
| Stdev | 29800 | 26400 | 29800 | 28500 | 29800 | 24600 |
| p(t-test) | | 0.039 | | 0.0067 | | 0.071 |
| Min | 0.00978 | 83.6 | 0.00978 | 53.8 | 0.00978 | 1500 |
| Max | 102000 | 100000 | 102000 | 103000 | 102000 | 93300 |
| n (Samp) | 316 | 127 | 316 | 105 | 316 | 35 |
| n (Patient) | 155 | 127 | 155 | 105 | 155 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.42 | 0.42 | 0.44 | 0.41 | 0.43 | 0.39 | 0.45 | 0.54 | 0.40 |
| SE | 0.030 | 0.048 | 0.031 | 0.033 | 0.049 | 0.033 | 0.051 | 0.060 | 0.053 |
| p | 0.011 | 0.11 | 0.044 | 0.0073 | 0.16 | 7.6E-4 | 0.32 | 0.49 | 0.057 |
| nCohort 1 | 271 | 666 | 316 | 271 | 666 | 316 | 271 | 666 | 316 |
| nCohort 2 | 140 | 40 | 127 | 108 | 39 | 105 | 38 | 25 | 35 |
| Cutoff 1 | 9360 | 6880 | 9360 | 7850 | 7340 | 7820 | 9280 | 11700 | 7780 |
| Sens 1 | 70% | 70% | 70% | 70% | 72% | 70% | 71% | 72% | 71% |
| Spec 1 | 22% | 18% | 22% | 18% | 20% | 19% | 22% | 31% | 19% |
| Cutoff 2 | 6520 | 4890 | 6490 | 6490 | 5860 | 6440 | 5690 | 10400 | 5700 |
| Sens 2 | 80% | 80% | 80% | 81% | 82% | 80% | 82% | 80% | 80% |
| Spec 2 | 16% | 13% | 16% | 15% | 15% | 16% | 14% | 28% | 14% |
| Cutoff 3 | 2730 | 2000 | 2160 | 4320 | 4980 | 2670 | 3080 | 7180 | 3080 |
| Sens 3 | 90% | 90% | 91% | 91% | 92% | 90% | 92% | 92% | 91% |
| Spec 3 | 7% | 6% | 5% | 11% | 13% | 5% | 9% | 19% | 8% |
| Cutoff 4 | 47300 | 39400 | 44200 | 47300 | 39400 | 44200 | 47300 | 39400 | 44200 |
| Sens 4 | 18% | 28% | 20% | 19% | 21% | 19% | 29% | 44% | 20% |
| Spec 4 | 70% | 70% | 70% | 70% | 70% | 70% | 70% | 70% | 70% |
| Cutoff 5 | 64500 | 59500 | 60800 | 64500 | 59500 | 60800 | 64500 | 59500 | 60800 |
| Sens 5 | 12% | 15% | 13% | 13% | 10% | 14% | 11% | 20% | 14% |
| Spec 5 | 80% | 80% | 80% | 80% | 80% | 80% | 80% | 80% | 80% |
| Cutoff 6 | 89700 | 85000 | 87200 | 89700 | 85000 | 87200 | 89700 | 85000 | 87200 |
| Sens 6 | 4% | 8% | 5% | 7% | 5% | 9% | 3% | 4% | 3% |
| Spec 6 | 90% | 90% | 90% | 90% | 90% | 90% | 90% | 90% | 90% |
| OR Quart 2 | 2.2 | 1.6 | 1.7 | 1.3 | 1.9 | 1.2 | 1.7 | 2.7 | 1.2 |
| p Value | 0.012 | 0.33 | 0.096 | 0.39 | 0.22 | 0.57 | 0.31 | 0.14 | 0.77 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of OR Quart2 | 1.2 4.0 | 0.61 4.3 | 0.91 3.1 | 0.68 2.6 | 0.69 5.3 | 0.61 2.5 | 0.62 4.6 | 0.71 10 | 0.38 3.7 |
| OR Quart 3 | 1.5 | 0.85 | 1.3 | 1.3 | 1.2 | 1.7 | 1.0 | 2.0 | 1.4 |
| p Value | 0.21 | 0.78 | 0.43 | 0.39 | 0.77 | 0.12 | 0.98 | 0.32 | 0.58 |
| 95% CI of OR Quart3 | 0.80 2.8 | 0.28 2.6 | 0.69 2.4 | 0.68 2.6 | 0.39 3.6 | 0.87 3.3 | 0.34 3.0 | 0.50 8.2 | 0.45 4.1 |
| OR Quart 4 | 2.4 | 2.4 | 2.0 | 2.5 | 2.7 | 2.9 | 2.1 | 2.7 | 2.6 |
| p Value | 0.0044 | 0.057 | 0.024 | 0.0044 | 0.049 | 0.0012 | 0.15 | 0.14 | 0.061 |
| 95% CI of OR Quart4 | 1.3 4.4 | 0.97 6.1 | 1.1 3.6 | 1.3 4.8 | 1.0 7.0 | 1.5 5.5 | 0.77 5.5 | 0.71 10 | 0.96 7.2 |

**Gastric inhibitory polypeptide Urine**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.372 | 0.344 | 0.372 | 0.411 | 0.372 | 0.470 |
| Average | 19.8 | 14.0 | 19.8 | 69.4 | 19.8 | 81.9 |
| Stdev | 236 | 66.2 | 236 | 404 | 236 | 373 |
| p(t-test) | | 0.77 | | 0.14 | | 0.16 |
| Min | 0.000104 | 9.60E-5 | 0.000104 | 9.60E-5 | 0.000104 | 0.000161 |
| Max | 3840 | 513 | 3840 | 3180 | 3840 | 2220 |
| n (Samp) | 271 | 140 | 271 | 108 | 271 | 38 |
| n (Patient) | 151 | 140 | 151 | 108 | 151 | 38 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.386 | 0.354 | 0.386 | 0.605 | 0.386 | 0.612 |
| Average | 20.3 | 19.2 | 20.3 | 87.0 | 20.3 | 123 |
| Stdev | 204 | 71.0 | 204 | 423 | 204 | 447 |
| p(t-test) | | 0.97 | | 0.068 | | 0.020 |
| Min | 9.60E-5 | 9.60E-5 | 9.60E-5 | 0.000136 | 9.60E-5 | 0.000599 |
| Max | 3840 | 421 | 3840 | 2590 | 3840 | 2220 |
| n (Samp) | 667 | 40 | 667 | 39 | 667 | 25 |
| n (Patient) | 290 | 40 | 290 | 39 | 290 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.424 | 0.360 | 0.424 | 0.305 | 0.424 | 0.427 |
| Average | 34.6 | 10.5 | 34.6 | 52.2 | 34.6 | 33.9 |
| Stdev | 290 | 57.5 | 290 | 329 | 290 | 129 |
| p(t-test) | | 0.35 | | 0.60 | | 0.99 |
| Min | 0.000104 | 9.60E-5 | 0.000104 | 9.60E-5 | 0.000104 | 9.60E-5 |
| Max | 3840 | 513 | 3840 | 3180 | 3840 | 647 |
| n (Samp) | 316 | 127 | 316 | 105 | 316 | 35 |
| n (Patient) | 155 | 127 | 155 | 105 | 155 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | 0.51 | 0.46 | 0.49 | 0.60 | 0.45 | 0.53 | 0.59 | 0.48 |
| SE | 0.030 | 0.047 | 0.031 | 0.033 | 0.049 | 0.033 | 0.051 | 0.061 | 0.052 |
| p | 0.55 | 0.76 | 0.17 | 0.68 | 0.034 | 0.11 | 0.50 | 0.13 | 0.75 |
| nCohort 1 | 271 | 667 | 316 | 271 | 667 | 316 | 271 | 667 | 316 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 2 | 140 | 40 | 127 | 108 | 39 | 105 | 38 | 25 | 35 |
| Cutoff 1 | 0.134 | 0.186 | 0.125 | 0.0726 | 0.347 | 0.0484 | 0.246 | 0.246 | 0.164 |
| Sens 1 | 70% | 70% | 70% | 70% | 72% | 71% | 71% | 72% | 71% |
| Spec 1 | 30% | 34% | 26% | 24% | 47% | 18% | 39% | 40% | 29% |
| Cutoff 2 | 0.0260 | 0.119 | 0.00355 | 0.00218 | 0.108 | 0.00355 | 0.0384 | 0.136 | 0.00982 |
| Sens 2 | 80% | 80% | 80% | 81% | 82% | 80% | 82% | 80% | 80% |
| Spec 2 | 20% | 29% | 16% | 19% | 28% | 16% | 21% | 30% | 16% |
| Cutoff 3 | 0.000161 | 0.000599 | 0.000104 | 0.000232 | 0.00221 | 0.000232 | 0.000599 | 0.00511 | 0.000520 |
| Sens 3 | 91% | 90% | 93% | 92% | 92% | 91% | 92% | 92% | 91% |
| Spec 3 | 6% | 14% | 3% | 7% | 18% | 7% | 15% | 19% | 8% |
| Cutoff 4 | 0.828 | 0.828 | 0.969 | 0.828 | 0.828 | 0.969 | 0.828 | 0.828 | 0.969 |
| Sens 4 | 30% | 32% | 27% | 31% | 38% | 27% | 29% | 40% | 26% |
| Spec 4 | 70% | 70% | 70% | 70% | 70% | 70% | 70% | 70% | 70% |
| Cutoff 5 | 1.56 | 1.64 | 2.13 | 1.56 | 1.64 | 2.13 | 1.56 | 1.64 | 2.13 |
| Sens 5 | 20% | 18% | 17% | 20% | 31% | 18% | 24% | 24% | 20% |
| Spec 5 | 80% | 80% | 80% | 80% | 80% | 80% | 80% | 80% | 80% |
| Cutoff 6 | 5.24 | 4.81 | 7.60 | 5.24 | 4.81 | 7.60 | 5.24 | 4.81 | 7.60 |
| Sens 6 | 9% | 12% | 9% | 12% | 21% | 10% | 8% | 20% | 9% |
| Spec 6 | 90% | 90% | 90% | 90% | 90% | 90% | 90% | 90% | 90% |
| OR Quart 2 | 0.88 | 2.1 | 1.0 | 0.90 | 1.5 | 0.91 | 1.1 | 1.5 | 1.3 |
| p Value | 0.66 | 0.13 | 1.0 | 0.75 | 0.44 | 0.77 | 0.80 | 0.52 | 0.62 |
| 95% CI of OR Quart2 | 0.49 – 1.6 | 0.82 – 5.3 | 0.55 – 1.8 | 0.48 – 1.7 | 0.53 – 4.4 | 0.48 – 1.7 | 0.42 – 3.1 | 0.42 – 5.5 | 0.48 – 3.4 |
| OR Quart 3 | 1.1 | 1.6 | 0.95 | 0.61 | 1.7 | 0.81 | 1.4 | 1.8 | 1.0 |
| p Value | 0.77 | 0.34 | 0.88 | 0.14 | 0.31 | 0.53 | 0.46 | 0.36 | 1.0 |
| 95% CI of OR Quart3 | 0.61 – 1.9 | 0.61 – 4.2 | 0.53 – 1.7 | 0.31 – 1.2 | 0.61 – 4.8 | 0.42 – 1.6 | 0.54 – 3.8 | 0.51 – 6.2 | 0.36 – 2.8 |
| OR Quart 4 | 1.1 | 1.1 | 1.4 | 1.2 | 2.4 | 1.7 | 1.3 | 2.0 | 1.2 |
| p Value | 0.84 | 0.80 | 0.23 | 0.61 | 0.075 | 0.088 | 0.64 | 0.25 | 0.78 |
| 95% CI of OR Quart4 | 0.60 – 1.9 | 0.41 – 3.2 | 0.80 – 2.5 | 0.64 – 2.2 | 0.91 – 6.5 | 0.93 – 3.1 | 0.47 – 3.4 | 0.61 – 6.9 | 0.42 – 3.1 |

**Peptide YY  Urine**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.33 | 7.08 | 4.33 | 10.6 | 4.33 | 2.38 |
| Average | 36.3 | 49.5 | 36.3 | 64.7 | 36.3 | 59.4 |
| Stdev | 89.9 | 123 | 89.9 | 181 | 89.9 | 173 |
| p(t-test) | | 0.22 | | 0.043 | | 0.20 |
| Min | 0.00976 | 0.00976 | 0.00976 | 0.00976 | 0.00976 | 0.00976 |
| Max | 936 | 762 | 936 | 1200 | 936 | 959 |
| n (Samp) | 271 | 140 | 271 | 107 | 271 | 38 |
| n (Patient) | 151 | 140 | 151 | 107 | 151 | 38 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.26 | 10.6 | 3.26 | 22.3 | 3.26 | 11.4 |
| Average | 44.9 | 53.6 | 44.9 | 47.5 | 44.9 | 49.4 |
| Stdev | 123 | 114 | 123 | 81.6 | 123 | 96.5 |
| p(t-test) | | 0.66 | | 0.90 | | 0.86 |
| Min | 0.00976 | 0.00976 | 0.00976 | 0.00976 | 0.00976 | 0.0188 |
| Max | 1200 | 502 | 1200 | 392 | 1200 | 450 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 665 | 40 | 665 | 39 | 665 | 25 |
| n (Patient) | 290 | 40 | 290 | 39 | 290 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.1 | 7.08 | 10.1 | 9.24 | 10.1 | 2.92 |
| Average | 38.2 | 48.1 | 38.2 | 67.2 | 38.2 | 63.9 |
| Stdev | 87.3 | 119 | 87.3 | 185 | 87.3 | 183 |
| p(t-test) | | 0.33 | | 0.031 | | 0.15 |
| Min | 0.00976 | 0.00976 | 0.00976 | 0.00976 | 0.00976 | 0.00976 |
| Max | 936 | 762 | 936 | 1200 | 936 | 959 |
| n (Samp) | 316 | 127 | 316 | 104 | 316 | 35 |
| n (Patient) | 155 | 127 | 155 | 104 | 155 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | 0.54 | 0.48 | 0.52 | 0.61 | 0.50 | 0.49 | 0.58 | 0.47 |
| SE | 0.030 | 0.048 | 0.030 | 0.033 | 0.049 | 0.033 | 0.050 | 0.061 | 0.052 |
| p | 0.94 | 0.36 | 0.59 | 0.46 | 0.031 | 0.95 | 0.86 | 0.20 | 0.57 |
| nCohort 1 | 271 | 665 | 316 | 271 | 665 | 316 | 271 | 665 | 316 |
| nCohort 2 | 140 | 40 | 127 | 107 | 39 | 104 | 38 | 25 | 35 |
| Cutoff 1 | 0.220 | 0.363 | 0.220 | 0.677 | 0.963 | 0.220 | 0.0196 | 0.817 | 0.0196 |
| Sens 1 | 71% | 72% | 70% | 72% | 72% | 71% | 82% | 72% | 80% |
| Spec 1 | 25% | 32% | 26% | 32% | 44% | 26% | 22% | 41% | 24% |
| Cutoff 2 | 0.0151 | 0.220 | 0.0151 | 0.0188 | 0.677 | 0.0151 | 0.0196 | 0.677 | 0.0196 |
| Sens 2 | 85% | 80% | 84% | 80% | 82% | 82% | 82% | 80% | 80% |
| Spec 2 | 15% | 30% | 15% | 18% | 36% | 15% | 22% | 36% | 24% |
| Cutoff 3 | 0.0101 | 0.0128 | 0.0101 | 0.0101 | 0.0151 | 0.0101 | 0.0128 | 0.0151 | 0.0128 |
| Sens 3 | 94% | 90% | 93% | 93% | 95% | 93% | 95% | 100% | 94% |
| Spec 3 | 5% | 11% | 5% | 5% | 18% | 5% | 8% | 18% | 8% |
| Cutoff 4 | 26.8 | 27.4 | 35.3 | 26.8 | 27.4 | 35.3 | 26.8 | 27.4 | 35.3 |
| Sens 4 | 32% | 38% | 24% | 35% | 41% | 26% | 26% | 36% | 20% |
| Spec 4 | 70% | 70% | 70% | 70% | 70% | 70% | 70% | 70% | 70% |
| Cutoff 5 | 42.3 | 42.7 | 48.2 | 42.3 | 42.7 | 48.2 | 42.3 | 42.7 | 48.2 |
| Sens 5 | 19% | 25% | 20% | 24% | 31% | 23% | 21% | 28% | 20% |
| Spec 5 | 80% | 80% | 80% | 80% | 80% | 80% | 80% | 80% | 80% |
| Cutoff 6 | 86.8 | 101 | 97.4 | 86.8 | 101 | 97.4 | 86.8 | 101 | 97.4 |
| Sens 6 | 12% | 12% | 10% | 15% | 10% | 16% | 11% | 16% | 11% |
| Spec 6 | 90% | 90% | 90% | 90% | 90% | 90% | 90% | 90% | 90% |
| OR Quart 2 | 1.3 | 1.3 | 1.3 | 0.80 | 2.6 | 0.81 | 1.1 | 1.5 | 1.2 |
| p Value | 0.38 | 0.63 | 0.45 | 0.49 | 0.11 | 0.52 | 0.78 | 0.53 | 0.79 |
| 95% CI of OR Quart2 | 0.73 | 0.49 | 0.69 | 0.42 | 0.80 | 0.43 | 0.44 | 0.42 | 0.40 |
| | 2.3 | 3.3 | 2.3 | 1.5 | 8.4 | 1.5 | 3.0 | 5.4 | 3.3 |
| OR Quart 3 | 0.87 | 1.1 | 1.1 | 0.90 | 3.1 | 0.91 | 0.89 | 1.8 | 1.7 |
| p Value | 0.65 | 0.80 | 0.65 | 0.75 | 0.051 | 0.75 | 0.82 | 0.36 | 0.32 |
| 95% CI of OR Quart3 | 0.48 | 0.43 | 0.63 | 0.48 | 0.99 | 0.49 | 0.32 | 0.51 | 0.61 |
| | 1.6 | 3.0 | 2.1 | 1.7 | 10.0 | 1.7 | 2.4 | 6.2 | 4.5 |
| OR Quart 4 | 1.3 | 1.7 | 1.4 | 1.0 | 3.4 | 0.91 | 1.3 | 2.0 | 1.3 |
| p Value | 0.43 | 0.27 | 0.28 | 0.91 | 0.034 | 0.75 | 0.61 | 0.25 | 0.58 |
| 95% CI of OR Quart4 | 0.71 | 0.67 | 0.77 | 0.56 | 1.1 | 0.49 | 0.50 | 0.60 | 0.47 |
| | 2.2 | 4.1 | 2.5 | 1.9 | 11 | 1.7 | 3.3 | 6.9 | 3.8 |

Table 2: Comparison of marker levels in urine samples collected from Cohort 1 (patients that did not progress beyond RIFLE stage 0 or R) and in urine samples collected from subjects at 0, 24 hours, and 48 hours prior to reaching stage I or F in Cohort 2.

**Agouti-related protein**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.818 | 1.47 | 0.818 | 0.888 | 0.818 | 0.805 |
| Average | 1.41 | 1.95 | 1.41 | 1.29 | 1.41 | 1.12 |
| Stdev | 2.23 | 1.57 | 2.23 | 1.18 | 2.23 | 0.811 |
| p(t-test) | | 0.083 | | 0.65 | | 0.47 |
| Min | 0.00318 | 0.00757 | 0.00318 | 0.0343 | 0.00318 | 0.00498 |
| Max | 20.0 | 6.53 | 20.0 | 6.12 | 20.0 | 2.60 |
| n (Samp) | 516 | 55 | 516 | 68 | 516 | 30 |
| n (Patient) | 222 | 55 | 222 | 68 | 222 | 30 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.879 | 0.811 | 0.879 | 0.794 | 0.879 | 0.892 |
| Average | 1.44 | 1.14 | 1.44 | 1.22 | 1.44 | 1.13 |
| Stdev | 2.00 | 1.02 | 2.00 | 1.46 | 2.00 | 0.744 |
| p(t-test) | | 0.58 | | 0.64 | | 0.59 |
| Min | 0.00318 | 0.0556 | 0.00318 | 0.180 | 0.00318 | 0.186 |
| Max | 20.0 | 3.30 | 20.0 | 6.53 | 20.0 | 2.33 |
| n (Samp) | 736 | 14 | 736 | 18 | 736 | 12 |
| n (Patient) | 280 | 14 | 280 | 18 | 280 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.812 | 1.54 | 0.812 | 0.945 | 0.812 | 0.735 |
| Average | 1.36 | 2.05 | 1.36 | 1.37 | 1.36 | 1.06 |
| Stdev | 2.14 | 1.62 | 2.14 | 1.21 | 2.14 | 0.875 |
| p(t-test) | | 0.030 | | 0.97 | | 0.49 |
| Min | 0.00198 | 0.00757 | 0.00198 | 0.0343 | 0.00198 | 0.00498 |
| Max | 20.0 | 6.53 | 20.0 | 6.12 | 20.0 | 2.60 |
| n (Samp) | 559 | 48 | 559 | 63 | 559 | 25 |
| n (Patient) | 225 | 48 | 225 | 63 | 225 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.65 | 0.48 | 0.67 | 0.54 | 0.48 | 0.57 | 0.52 | 0.52 | 0.49 |
| SE | 0.042 | 0.079 | 0.044 | 0.038 | 0.070 | 0.039 | 0.055 | 0.085 | 0.059 |
| p | 3.6E-4 | 0.78 | 1.2E-4 | 0.27 | 0.77 | 0.089 | 0.67 | 0.82 | 0.90 |
| nCohort 1 | 516 | 736 | 559 | 516 | 736 | 559 | 516 | 736 | 559 |
| nCohort 2 | 55 | 14 | 48 | 68 | 18 | 63 | 30 | 12 | 25 |
| Cutoff 1 | 0.867 | 0.364 | 0.935 | 0.574 | 0.600 | 0.574 | 0.579 | 0.579 | 0.505 |
| Sens 1 | 71% | 71% | 71% | 71% | 72% | 71% | 70% | 75% | 72% |
| Spec 1 | 52% | 24% | 55% | 38% | 37% | 38% | 39% | 36% | 35% |
| Cutoff 2 | 0.562 | 0.289 | 0.637 | 0.364 | 0.295 | 0.366 | 0.465 | 0.444 | 0.218 |
| Sens 2 | 80% | 86% | 81% | 81% | 83% | 81% | 80% | 83% | 80% |
| Spec 2 | 38% | 20% | 41% | 27% | 20% | 27% | 32% | 28% | 17% |
| Cutoff 3 | 0.235 | 0.154 | 0.241 | 0.211 | 0.183 | 0.258 | 0.173 | 0.420 | 0.140 |
| Sens 3 | 91% | 93% | 92% | 91% | 94% | 90% | 90% | 92% | 92% |
| Spec 3 | 18% | 11% | 18% | 16% | 13% | 20% | 14% | 27% | 10% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 1.38 | 1.54 | 1.37 | 1.38 | 1.54 | 1.37 | 1.38 | 1.54 | 1.37 |
| Sens 4 | 53% | 29% | 56% | 38% | 28% | 44% | 40% | 42% | 36% |
| Spec 4 | 70% | 70% | 70% | 70% | 70% | 70% | 70% | 70% | 70% |
| Cutoff 5 | 1.87 | 2.15 | 1.82 | 1.87 | 2.15 | 1.82 | 1.87 | 2.15 | 1.82 |
| Sens 5 | 42% | 21% | 46% | 21% | 6% | 29% | 20% | 17% | 28% |
| Spec 5 | 80% | 80% | 80% | 80% | 80% | 80% | 80% | 80% | 80% |
| Cutoff 6 | 2.99 | 3.13 | 2.93 | 2.99 | 3.13 | 2.93 | 2.99 | 3.13 | 2.93 |
| Sens 6 | 24% | 7% | 29% | 9% | 6% | 10% | 0% | 0% | 0% |
| Spec 6 | 90% | 90% | 90% | 90% | 90% | 90% | 90% | 90% | 90% |
| OR Quart 2 | 0.99 | 1.0 | 0.99 | 1.4 | 1.7 | 1.1 | 2.1 | 5.1 | 0.10 |
| p Value | 0.99 | 0.99 | 0.99 | 0.34 | 0.47 | 0.85 | 0.20 | 0.14 | 0.034 |
| 95% CI of | 0.36 | 0.20 | 0.31 | 0.68 | 0.40 | 0.48 | 0.69 | 0.59 | 0.013 |
| OR Quart2 | 2.7 | 5.0 | 3.2 | 3.1 | 7.2 | 2.5 | 6.2 | 44 | 0.84 |
| OR Quart 3 | 2.0 | 1.0 | 2.1 | 1.3 | 2.0 | 1.4 | 0.79 | 3.0 | 1.0 |
| p Value | 0.14 | 1.0 | 0.16 | 0.56 | 0.32 | 0.43 | 0.74 | 0.34 | 1.0 |
| 95% CI of | 0.80 | 0.20 | 0.76 | 0.58 | 0.50 | 0.63 | 0.21 | 0.31 | 0.39 |
| OR Quart3 | 4.8 | 5.0 | 5.7 | 2.7 | 8.3 | 3.0 | 3.0 | 29 | 2.6 |
| OR Quart 4 | 3.4 | 1.7 | 4.5 | 1.7 | 1.3 | 2.0 | 2.3 | 3.0 | 0.65 |
| p Value | 0.0044 | 0.47 | 0.0014 | 0.15 | 0.70 | 0.076 | 0.14 | 0.34 | 0.43 |
| 95% CI of | 1.5 | 0.40 | 1.8 | 0.83 | 0.30 | 0.93 | 0.77 | 0.31 | 0.23 |
| OR Quart4 | 7.8 | 7.2 | 11 | 3.6 | 6.1 | 4.1 | 6.8 | 29 | 1.9 |

**Osteocalcin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5840 | 6360 | 5840 | 7250 | 5840 | 6280 |
| Average | 7330 | 8630 | 7330 | 8860 | 7330 | 8330 |
| Stdev | 6430 | 8820 | 6430 | 8710 | 6430 | 8630 |
| p(t-test) | | 0.071 | | 0.032 | | 0.28 |
| Min | 76.8 | 736 | 76.8 | 473 | 76.8 | 103 |
| Max | 83000 | 67800 | 83000 | 49400 | 83000 | 42300 |
| n (Samp) | 848 | 98 | 848 | 99 | 848 | 53 |
| n (Patient) | 374 | 98 | 374 | 99 | 374 | 53 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6120 | 7200 | 6120 | 6050 | 6120 | 5900 |
| Average | 7910 | 10000 | 7910 | 8190 | 7910 | 8880 |
| Stdev | 7800 | 6900 | 7800 | 5970 | 7800 | 8730 |
| p(t-test) | | 0.19 | | 0.85 | | 0.55 |
| Min | 76.8 | 1450 | 76.8 | 1330 | 76.8 | 1250 |
| Max | 83500 | 26800 | 83500 | 22900 | 83500 | 33400 |
| n (Samp) | 1223 | 23 | 1223 | 27 | 1223 | 24 |
| n (Patient) | 472 | 23 | 472 | 27 | 472 | 24 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5920 | 6040 | 5920 | 7100 | 5920 | 6280 |
| Average | 7580 | 8220 | 7580 | 8560 | 7580 | 8280 |
| Stdev | 6760 | 8910 | 6760 | 8820 | 6760 | 8420 |
| p(t-test) | | 0.41 | | 0.21 | | 0.50 |
| Min | 73.4 | 736 | 73.4 | 473 | 73.4 | 103 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 83000 | 67800 | 83000 | 49400 | 83000 | 42300 |
| n (Samp) | 883 | 87 | 883 | 88 | 883 | 45 |
| n (Patient) | 361 | 87 | 361 | 88 | 361 | 45 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.62 | 0.51 | 0.55 | 0.54 | 0.53 | 0.49 | 0.51 | 0.49 |
| SE | 0.031 | 0.063 | 0.033 | 0.031 | 0.057 | 0.033 | 0.041 | 0.060 | 0.044 |
| p | 0.17 | 0.064 | 0.70 | 0.14 | 0.49 | 0.44 | 0.86 | 0.88 | 0.85 |
| nCohort 1 | 848 | 1223 | 883 | 848 | 1223 | 883 | 848 | 1223 | 883 |
| nCohort 2 | 98 | 23 | 87 | 99 | 27 | 88 | 53 | 24 | 45 |
| Cutoff 1 | 4470 | 4590 | 4020 | 4300 | 4900 | 3790 | 3090 | 4430 | 3090 |
| Sens 1 | 70% | 74% | 70% | 71% | 70% | 70% | 72% | 71% | 71% |
| Spec 1 | 38% | 38% | 31% | 36% | 40% | 30% | 22% | 36% | 22% |
| Cutoff 2 | 3410 | 4510 | 2720 | 3370 | 4310 | 3240 | 2700 | 3250 | 2450 |
| Sens 2 | 81% | 83% | 80% | 81% | 81% | 81% | 81% | 83% | 80% |
| Spec 2 | 27% | 37% | 18% | 26% | 35% | 23% | 18% | 24% | 15% |
| Cutoff 3 | 2300 | 3520 | 2190 | 1920 | 1630 | 1920 | 1030 | 2700 | 584 |
| Sens 3 | 91% | 91% | 91% | 91% | 93% | 91% | 91% | 92% | 91% |
| Spec 3 | 15% | 27% | 13% | 11% | 8% | 11% | 4% | 18% | 1% |
| Cutoff 4 | 8640 | 8880 | 8700 | 8640 | 8880 | 8700 | 8640 | 8880 | 8700 |
| Sens 4 | 35% | 43% | 31% | 37% | 33% | 35% | 26% | 25% | 31% |
| Spec 4 | 70% | 70% | 70% | 70% | 70% | 70% | 70% | 70% | 70% |
| Cutoff 5 | 10400 | 11100 | 10800 | 10400 | 11100 | 10800 | 10400 | 11100 | 10800 |
| Sens 5 | 27% | 39% | 23% | 23% | 22% | 18% | 26% | 21% | 27% |
| Spec 5 | 80% | 80% | 80% | 80% | 80% | 80% | 80% | 80% | 80% |
| Cutoff 6 | 14600 | 15300 | 15300 | 14600 | 15300 | 15300 | 14600 | 15300 | 15300 |
| Sens 6 | 11% | 17% | 9% | 12% | 11% | 8% | 17% | 12% | 11% |
| Spec 6 | 90% | 90% | 90% | 90% | 90% | 90% | 90% | 90% | 90% |
| OR Quart 2 | 1.3 | 3.5 | 1.1 | 1.3 | 2.5 | 0.89 | 1.1 | 1.4 | 0.92 |
| p Value | 0.35 | 0.12 | 0.76 | 0.43 | 0.12 | 0.73 | 0.84 | 0.57 | 0.84 |
| 95% CI of OR Quart2 | 0.72 2.5 | 0.73 17 | 0.59 2.1 | 0.68 2.4 | 0.79 8.2 | 0.46 1.7 | 0.51 2.3 | 0.44 4.5 | 0.41 2.1 |
| OR Quart 3 | 1.4 | 2.0 | 1.1 | 1.5 | 1.5 | 1.4 | 0.49 | 1.4 | 0.37 |
| p Value | 0.34 | 0.42 | 0.75 | 0.17 | 0.53 | 0.23 | 0.13 | 0.57 | 0.064 |
| 95% CI of OR Quart3 | 0.72 2.5 | 0.37 11 | 0.59 2.1 | 0.83 2.8 | 0.42 5.4 | 0.79 2.6 | 0.19 1.2 | 0.44 4.5 | 0.13 1.1 |
| OR Quart 4 | 1.6 | 5.1 | 1.2 | 1.5 | 1.8 | 1.1 | 1.2 | 1.00 | 1.2 |
| p Value | 0.13 | 0.036 | 0.64 | 0.17 | 0.37 | 0.76 | 0.57 | 1.00 | 0.70 |
| 95% CI of OR Quart4 | 0.87 2.9 | 1.1 24 | 0.62 2.2 | 0.83 2.8 | 0.51 6.1 | 0.59 2.1 | 0.59 2.6 | 0.29 3.5 | 0.54 2.5 |

**Complement factor H**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.31 | 7.92 | 9.31 | 8.75 | 9.31 | 8.05 |
| Average | 18.9 | 15.8 | 18.9 | 18.5 | 18.9 | 14.0 |
| Stdev | 41.1 | 28.0 | 41.1 | 29.6 | 41.1 | 20.0 |
| p(t-test) | | 0.54 | | 0.93 | | 0.45 |
| Min | 0.0620 | 0.113 | 0.0620 | 0.343 | 0.0620 | 0.121 |
| Max | 772 | 193 | 772 | 142 | 772 | 92.1 |
| n (Samp) | 599 | 70 | 599 | 79 | 599 | 40 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 281 | 70 | 281 | 79 | 281 | 40 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.33 | 7.92 | 9.33 | 12.5 | 9.33 | 12.3 |
| Average | 18.6 | 16.4 | 18.6 | 25.4 | 18.6 | 17.0 |
| Stdev | 37.7 | 20.4 | 37.7 | 35.9 | 37.7 | 14.6 |
| p(t-test) | | 0.85 | | 0.45 | | 0.86 |
| Min | 0.0620 | 3.87 | 0.0620 | 2.72 | 0.0620 | 0.832 |
| Max | 772 | 70.6 | 772 | 142 | 772 | 59.2 |
| n (Samp) | 829 | 10 | 829 | 18 | 829 | 16 |
| n (Patient) | 353 | 10 | 353 | 18 | 353 | 16 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 8.90 | 8.38 | 8.90 | 8.03 | 8.90 | 7.25 |
| Average | 18.6 | 16.7 | 18.6 | 16.1 | 18.6 | 12.7 |
| Stdev | 40.7 | 29.7 | 40.7 | 25.2 | 40.7 | 19.7 |
| p(t-test) | | 0.71 | | 0.60 | | 0.39 |
| Min | 0.0620 | 0.113 | 0.0620 | 0.343 | 0.0620 | 0.121 |
| Max | 772 | 193 | 772 | 131 | 772 | 92.1 |
| n (Samp) | 608 | 66 | 608 | 74 | 608 | 35 |
| n (Patient) | 266 | 66 | 266 | 74 | 266 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.50 | 0.48 | 0.47 | 0.61 | 0.46 | 0.43 | 0.58 | 0.40 |
| SE | 0.037 | 0.092 | 0.038 | 0.035 | 0.071 | 0.036 | 0.049 | 0.075 | 0.052 |
| p | 0.41 | 0.99 | 0.51 | 0.45 | 0.14 | 0.25 | 0.14 | 0.32 | 0.049 |
| nCohort 1 | 599 | 829 | 608 | 599 | 829 | 608 | 599 | 829 | 608 |
| nCohort 2 | 70 | 10 | 66 | 79 | 18 | 74 | 40 | 16 | 35 |
| Cutoff 1 | 5.90 | 6.66 | 5.27 | 4.91 | 9.39 | 4.95 | 4.28 | 8.64 | 4.04 |
| Sens 1 | 70% | 70% | 71% | 71% | 72% | 70% | 70% | 75% | 71% |
| Spec 1 | 30% | 36% | 25% | 25% | 50% | 24% | 21% | 48% | 18% |
| Cutoff 2 | 4.11 | 6.40 | 4.11 | 3.87 | 6.57 | 3.87 | 3.03 | 6.01 | 3.03 |
| Sens 2 | 80% | 80% | 80% | 81% | 83% | 81% | 80% | 81% | 80% |
| Spec 2 | 20% | 34% | 20% | 17% | 36% | 17% | 13% | 31% | 13% |
| Cutoff 3 | 3.03 | 3.87 | 2.60 | 2.26 | 4.58 | 2.26 | 2.52 | 2.41 | 2.54 |
| Sens 3 | 90% | 90% | 91% | 91% | 94% | 91% | 90% | 94% | 91% |
| Spec 3 | 13% | 17% | 10% | 9% | 22% | 9% | 10% | 10% | 10% |
| Cutoff 4 | 16.1 | 15.9 | 15.6 | 16.1 | 15.9 | 15.6 | 16.1 | 15.9 | 15.6 |
| Sens 4 | 23% | 30% | 23% | 28% | 39% | 28% | 20% | 44% | 17% |
| Spec 4 | 70% | 70% | 70% | 70% | 70% | 70% | 70% | 70% | 70% |
| Cutoff 5 | 24.0 | 22.8 | 23.5 | 24.0 | 22.8 | 23.5 | 24.0 | 22.8 | 23.5 |
| Sens 5 | 13% | 20% | 14% | 14% | 22% | 16% | 15% | 31% | 11% |
| Spec 5 | 80% | 80% | 80% | 80% | 80% | 80% | 80% | 80% | 80% |
| Cutoff 6 | 40.5 | 40.3 | 40.3 | 40.5 | 40.3 | 40.3 | 40.5 | 40.3 | 40.3 |
| Sens 6 | 6% | 10% | 6% | 8% | 11% | 5% | 8% | 6% | 6% |
| Spec 6 | 90% | 90% | 90% | 90% | 90% | 90% | 90% | 90% | 90% |
| OR Quart 2 | 1.3 | 0 | 1.5 | 1.1 | 0.66 | 1.2 | 1.7 | 1.5 | 2.3 |
| p Value | 0.45 | na | 0.26 | 0.72 | 0.65 | 0.57 | 0.31 | 0.65 | 0.17 |
| 95% CI of | 0.64 | na | 0.73 | 0.58 | 0.11 | 0.60 | 0.61 | 0.25 | 0.70 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart2 | 2.8 | na | 3.2 | 2.2 | 4.0 | 2.5 | 4.8 | 9.1 | 7.7 |
| OR Quart 3 | 1.5 | 1.7 | 1.3 | 1.0 | 2.0 | 1.3 | 1.9 | 3.1 | 2.1 |
| p Value | 0.27 | 0.48 | 0.56 | 1.0 | 0.32 | 0.47 | 0.22 | 0.17 | 0.25 |
| 95% CI of OR Quart3 | 0.73 | 0.40 | 0.58 | 0.50 | 0.50 | 0.64 | 0.68 | 0.61 | 0.61 |
|  | 3.1 | 7.1 | 2.7 | 2.0 | 8.2 | 2.7 | 5.3 | 15 | 7.0 |
| OR Quart 4 | 1.3 | 0.67 | 1.4 | 1.3 | 2.4 | 1.5 | 2.3 | 2.5 | 3.8 |
| p Value | 0.45 | 0.66 | 0.34 | 0.39 | 0.22 | 0.22 | 0.10 | 0.27 | 0.022 |
| 95% CI of OR Quart4 | 0.64 | 0.11 | 0.68 | 0.69 | 0.60 | 0.77 | 0.85 | 0.48 | 1.2 |
|  | 2.8 | 4.0 | 3.0 | 2.6 | 9.3 | 3.1 | 6.2 | 13 | 12 |

**Ciliary neurotrophic factor**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0787 | 0.000166 | 0.0787 | 0.0325 | 0.0787 | 0.0501 |
| Average | 0.270 | 0.130 | 0.270 | 0.162 | 0.270 | 0.166 |
| Stdev | 0.799 | 0.319 | 0.799 | 0.207 | 0.799 | 0.287 |
| p(t-test) |  | 0.20 |  | 0.27 |  | 0.48 |
| Min | 0.000103 | 0.000103 | 0.000103 | 0.000103 | 0.000103 | 0.000103 |
| Max | 14.0 | 2.14 | 14.0 | 0.866 | 14.0 | 1.42 |
| n (Samp) | 516 | 55 | 516 | 68 | 516 | 30 |
| n (Patient) | 222 | 55 | 222 | 68 | 222 | 30 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0452 | 0.00883 | 0.0452 | 0.0185 | 0.0452 | 0.0894 |
| Average | 0.233 | 0.0538 | 0.233 | 0.253 | 0.233 | 0.120 |
| Stdev | 0.709 | 0.0906 | 0.709 | 0.526 | 0.709 | 0.133 |
| p(t-test) |  | 0.35 |  | 0.90 |  | 0.58 |
| Min | 0.000103 | 0.000119 | 0.000103 | 0.000119 | 0.000103 | 0.000123 |
| Max | 14.0 | 0.290 | 14.0 | 2.14 | 14.0 | 0.360 |
| n (Samp) | 736 | 14 | 736 | 18 | 736 | 12 |
| n (Patient) | 280 | 14 | 280 | 18 | 280 | 12 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0551 | 0.00951 | 0.0551 | 0.0325 | 0.0551 | 0.0551 |
| Average | 0.249 | 0.148 | 0.249 | 0.158 | 0.249 | 0.192 |
| Stdev | 0.774 | 0.338 | 0.774 | 0.187 | 0.774 | 0.315 |
| p(t-test) |  | 0.37 |  | 0.35 |  | 0.71 |
| Min | 0.000103 | 0.000103 | 0.000103 | 0.000103 | 0.000103 | 0.000103 |
| Max | 14.0 | 2.14 | 14.0 | 0.778 | 14.0 | 1.42 |
| n (Samp) | 559 | 48 | 559 | 63 | 559 | 25 |
| n (Patient) | 225 | 48 | 225 | 63 | 225 | 25 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.37 | 0.41 | 0.41 | 0.47 | 0.51 | 0.49 | 0.46 | 0.52 | 0.51 |
| SE | 0.042 | 0.081 | 0.045 | 0.038 | 0.069 | 0.039 | 0.055 | 0.085 | 0.059 |
| p | 0.0021 | 0.24 | 0.052 | 0.37 | 0.91 | 0.88 | 0.49 | 0.85 | 0.93 |
| nCohort 1 | 516 | 736 | 559 | 516 | 736 | 559 | 516 | 736 | 559 |
| nCohort 2 | 55 | 14 | 48 | 68 | 18 | 63 | 30 | 12 | 25 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 1 | 0.000121 | 0.000161 | 0.000121 | 0.000123 | 0.000161 | 0.000161 | 0.000161 | 0.000161 | 0.000123 |
| Sens 1 | 73% | 71% | 71% | 72% | 72% | 71% | 70% | 83% | 76% |
| Spec 1 | 18% | 29% | 19% | 21% | 29% | 28% | 25% | 29% | 24% |
| Cutoff 2 | 0.000119 | 0.000121 | 0.000119 | 0.000119 | 0.000121 | 0.000119 | 0.000123 | 0.000161 | 0.000121 |
| Sens 2 | 82% | 93% | 81% | 85% | 89% | 86% | 80% | 83% | 80% |
| Spec 2 | 15% | 22% | 15% | 15% | 22% | 15% | 21% | 29% | 19% |
| Cutoff 3 | 0.000103 | 0.000121 | 0 | 0.000103 | 0.000119 | 0.000103 | 0.000119 | 0.000123 | 0.000118 |
| Sens 3 | 91% | 93% | 100% | 96% | 94% | 95% | 93% | 92% | 96% |
| Spec 3 | 5% | 22% | 0% | 5% | 18% | 4% | 15% | 26% | 10% |
| Cutoff 4 | 0.290 | 0.232 | 0.245 | 0.290 | 0.232 | 0.245 | 0.290 | 0.232 | 0.245 |
| Sens 4 | 15% | 7% | 17% | 25% | 28% | 32% | 23% | 17% | 28% |
| Spec 4 | 71% | 71% | 71% | 71% | 71% | 71% | 71% | 71% | 71% |
| Cutoff 5 | 0.360 | 0.346 | 0.360 | 0.360 | 0.346 | 0.360 | 0.360 | 0.346 | 0.360 |
| Sens 5 | 11% | 0% | 12% | 18% | 22% | 16% | 13% | 8% | 20% |
| Spec 5 | 81% | 80% | 82% | 81% | 80% | 82% | 81% | 80% | 82% |
| Cutoff 6 | 0.467 | 0.459 | 0.467 | 0.467 | 0.459 | 0.467 | 0.467 | 0.459 | 0.467 |
| Sens 6 | 5% | 0% | 6% | 9% | 17% | 6% | 7% | 0% | 12% |
| Spec 6 | 90% | 90% | 91% | 90% | 90% | 91% | 90% | 90% | 91% |
| OR Quart 2 | 1.3 | 3.0 | 1.5 | 0.86 | 2.7 | 0.94 | 0.71 | >6.2 | 1.0 |
| p Value | 0.63 | 0.34 | 0.36 | 0.70 | 0.14 | 0.86 | 0.57 | <0.093 | 1.0 |
| 95% CI of | 0.49 | 0.31 | 0.61 | 0.40 | 0.71 | 0.45 | 0.22 | >0.74 | 0.31 |
| OR Quart2 | 3.3 | 30 | 3.9 | 1.8 | 10 | 2.0 | 2.3 | na | 3.2 |
| OR Quart 3 | 1.8 | 9.4 | 1.4 | 1.1 | 0.66 | 0.93 | 1.3 | >4.1 | 1.0 |
| p Value | 0.19 | 0.034 | 0.48 | 0.85 | 0.65 | 0.85 | 0.61 | <0.21 | 1.0 |
| 95% CI of | 0.74 | 1.2 | 0.55 | 0.52 | 0.11 | 0.44 | 0.47 | >0.45 | 0.31 |
| OR Quart3 | 4.5 | 75 | 3.6 | 2.2 | 4.0 | 2.0 | 3.6 | na | 3.2 |
| OR Quart 4 | 3.3 | 1.0 | 2.3 | 1.4 | 1.7 | 1.1 | 1.3 | >2.0 | 1.2 |
| p Value | 0.0059 | 1.00 | 0.064 | 0.38 | 0.48 | 0.84 | 0.60 | <0.57 | 0.78 |
| 95% CI of | 1.4 | 0.062 | 0.95 | 0.68 | 0.39 | 0.52 | 0.48 | >0.18 | 0.39 |
| OR Quart4 | 7.6 | 16 | 5.5 | 2.7 | 7.1 | 2.2 | 3.6 | na | 3.6 |

**Follitropin subunit beta**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 13.2 | 11.9 | 13.2 | 15.7 | 13.2 | 17.4 |
| Average | 30.3 | 22.7 | 30.3 | 27.2 | 30.3 | 31.0 |
| Stdev | 43.2 | 28.3 | 43.2 | 29.8 | 43.2 | 40.2 |
| p(t-test) |  | 0.20 |  | 0.57 |  | 0.93 |
| Min | 0.0800 | 1.24 | 0.0800 | 0.345 | 0.0800 | 0.474 |
| Max | 300 | 128 | 300 | 123 | 300 | 158 |
| n (Samp) | 516 | 55 | 516 | 68 | 516 | 30 |
| n (Patient) | 222 | 55 | 222 | 68 | 222 | 30 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 13.8 | 4.96 | 13.8 | 6.67 | 13.8 | 6.63 |
| Average | 29.5 | 6.97 | 29.5 | 16.8 | 29.5 | 24.2 |
| Stdev | 40.1 | 6.17 | 40.1 | 27.0 | 40.1 | 45.3 |
| p(t-test) |  | 0.036 |  | 0.18 |  | 0.65 |
| Min | 0.0800 | 1.68 | 0.0800 | 0.979 | 0.0800 | 1.58 |
| Max | 300 | 21.0 | 300 | 109 | 300 | 158 |
| n (Samp) | 736 | 14 | 736 | 18 | 736 | 12 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 280 | 14 | 280 | 18 | 280 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 11.9 | 12.8 | 11.9 | 15.5 | 11.9 | 21.5 |
| Average | 28.4 | 24.6 | 28.4 | 28.2 | 28.4 | 36.6 |
| Stdev | 42.0 | 29.7 | 42.0 | 30.5 | 42.0 | 42.2 |
| p(t-test) | | 0.55 | | 0.97 | | 0.34 |
| Min | 0.0800 | 1.24 | 0.0800 | 0.345 | 0.0800 | 0.474 |
| Max | 300 | 128 | 300 | 123 | 300 | 158 |
| n (Samp) | 559 | 48 | 559 | 63 | 559 | 25 |
| n (Patient) | 225 | 48 | 225 | 63 | 225 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | 0.26 | 0.53 | 0.52 | 0.35 | 0.55 | 0.52 | 0.38 | 0.60 |
| SE | 0.041 | 0.077 | 0.044 | 0.038 | 0.071 | 0.039 | 0.055 | 0.087 | 0.061 |
| p | 0.68 | 0.0022 | 0.55 | 0.58 | 0.035 | 0.16 | 0.68 | 0.16 | 0.11 |
| nCohort 1 | 516 | 736 | 559 | 516 | 736 | 559 | 516 | 736 | 559 |
| nCohort 2 | 55 | 14 | 48 | 68 | 18 | 63 | 30 | 12 | 25 |
| Cutoff 1 | 7.02 | 3.63 | 7.15 | 7.07 | 3.58 | 7.23 | 6.91 | 3.43 | 10.9 |
| Sens 1 | 71% | 71% | 71% | 71% | 72% | 71% | 70% | 75% | 72% |
| Spec 1 | 33% | 16% | 38% | 34% | 15% | 38% | 32% | 15% | 48% |
| Cutoff 2 | 4.50 | 2.07 | 4.63 | 5.45 | 1.90 | 5.56 | 5.33 | 3.19 | 6.41 |
| Sens 2 | 80% | 86% | 81% | 81% | 83% | 81% | 80% | 83% | 80% |
| Spec 2 | 22% | 9% | 26% | 27% | 9% | 30% | 26% | 13% | 35% |
| Cutoff 3 | 2.63 | 1.75 | 3.66 | 1.57 | 1.28 | 1.72 | 3.42 | 1.90 | 4.11 |
| Sens 3 | 91% | 93% | 92% | 91% | 94% | 90% | 90% | 92% | 92% |
| Spec 3 | 14% | 8% | 21% | 8% | 4% | 10% | 17% | 9% | 23% |
| Cutoff 4 | 29.2 | 29.3 | 26.6 | 29.2 | 29.3 | 26.6 | 29.2 | 29.3 | 26.6 |
| Sens 4 | 20% | 0% | 31% | 32% | 22% | 33% | 30% | 17% | 44% |
| Spec 4 | 70% | 70% | 70% | 70% | 70% | 70% | 70% | 70% | 70% |
| Cutoff 5 | 45.6 | 44.1 | 40.9 | 45.6 | 44.1 | 40.9 | 45.6 | 44.1 | 40.9 |
| Sens 5 | 11% | 0% | 15% | 18% | 11% | 29% | 20% | 17% | 32% |
| Spec 5 | 80% | 80% | 80% | 80% | 80% | 80% | 80% | 80% | 80% |
| Cutoff 6 | 76.8 | 76.0 | 75.3 | 76.8 | 76.0 | 75.3 | 76.8 | 76.0 | 75.3 |
| Sens 6 | 7% | 0% | 8% | 12% | 6% | 13% | 13% | 8% | 16% |
| Spec 6 | 90% | 90% | 90% | 90% | 90% | 90% | 90% | 90% | 90% |
| OR Quart 2 | 2.0 | >2.0 | 2.0 | 1.3 | 1.5 | 3.1 | 1.2 | 1.0 | 1.0 |
| p Value | 0.11 | <0.56 | 0.14 | 0.44 | 0.65 | 0.013 | 0.79 | 1.0 | 1.0 |
| 95% CI of OR Quart2 | 0.86 | >0.18 | 0.80 | 0.63 | 0.25 | 1.3 | 0.38 | 0.14 | 0.25 |
| | 4.7 | na | 4.8 | 2.9 | 9.2 | 7.6 | 3.6 | 7.2 | 4.1 |
| OR Quart 3 | 1.9 | >4.1 | 2.0 | 1.5 | 2.5 | 2.4 | 1.5 | 1.0 | 2.3 |
| p Value | 0.15 | <0.21 | 0.14 | 0.26 | 0.27 | 0.058 | 0.43 | 1.0 | 0.17 |
| 95% CI of OR Quart3 | 0.80 | >0.45 | 0.80 | 0.73 | 0.49 | 0.97 | 0.53 | 0.14 | 0.70 |
| | 4.4 | na | 4.8 | 3.2 | 13 | 6.1 | 4.4 | 7.2 | 7.8 |
| OR Quart 4 | 1.5 | >8.4 | 1.3 | 1.5 | 4.2 | 3.1 | 1.3 | 3.1 | 2.1 |
| p Value | 0.37 | <0.046 | 0.64 | 0.26 | 0.074 | 0.013 | 0.59 | 0.17 | 0.25 |
| 95% CI of OR Quart4 | 0.62 | >1.0 | 0.48 | 0.73 | 0.87 | 1.3 | 0.45 | 0.61 | 0.61 |
| | 3.6 | na | 3.3 | 3.2 | 20 | 7.6 | 4.0 | 15 | 7.0 |

**Follistatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 7.38 | 6.85 | 7.38 | 9.97 | 7.38 | 5.88 |
| Average | 31.8 | 13.6 | 31.8 | 29.5 | 31.8 | 19.1 |
| Stdev | 269 | 17.1 | 269 | 89.8 | 269 | 35.4 |
| p(t-test) | | 0.54 | | 0.93 | | 0.73 |
| Min | 0.000347 | 0.0191 | 0.000347 | 0.0191 | 0.000347 | 0.0191 |
| Max | 8290 | 98.9 | 8290 | 840 | 8290 | 225 |
| n (Samp) | 1035 | 84 | 1035 | 92 | 1035 | 53 |
| n (Patient) | 397 | 84 | 397 | 92 | 397 | 53 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 7.59 | 13.9 | 7.59 | 9.74 | 7.59 | 16.4 |
| Average | 28.2 | 22.2 | 28.2 | 13.1 | 28.2 | 34.9 |
| Stdev | 232 | 29.4 | 232 | 12.7 | 232 | 50.1 |
| p(t-test) | | 0.92 | | 0.75 | | 0.89 |
| Min | 0.000347 | 2.94 | 0.000347 | 0.0191 | 0.000347 | 0.0191 |
| Max | 8290 | 125 | 8290 | 49.9 | 8290 | 222 |
| n (Samp) | 1406 | 16 | 1406 | 24 | 1406 | 22 |
| n (Patient) | 496 | 16 | 496 | 24 | 496 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 7.46 | 6.94 | 7.46 | 11.5 | 7.46 | 6.64 |
| Average | 32.1 | 38.9 | 32.1 | 46.4 | 32.1 | 18.7 |
| Stdev | 278 | 225 | 278 | 164 | 278 | 35.7 |
| p(t-test) | | 0.83 | | 0.64 | | 0.74 |
| Min | 0.000347 | 0.0191 | 0.000347 | 0.0412 | 0.000347 | 0.0767 |
| Max | 8290 | 2010 | 8290 | 1300 | 8290 | 225 |
| n (Samp) | 967 | 79 | 967 | 87 | 967 | 46 |
| n (Patient) | 338 | 79 | 338 | 87 | 338 | 46 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | 0.63 | 0.50 | 0.56 | 0.54 | 0.58 | 0.48 | 0.63 | 0.48 |
| SE | 0.033 | 0.075 | 0.034 | 0.032 | 0.061 | 0.033 | 0.041 | 0.064 | 0.044 |
| p | 0.92 | 0.076 | 0.90 | 0.057 | 0.48 | 0.014 | 0.57 | 0.042 | 0.71 |
| nCohort 1 | 1035 | 1406 | 967 | 1035 | 1406 | 967 | 1035 | 1406 | 967 |
| nCohort 2 | 84 | 16 | 79 | 92 | 24 | 87 | 53 | 22 | 46 |
| Cutoff 1 | 4.11 | 5.90 | 3.64 | 5.00 | 6.66 | 5.46 | 2.49 | 5.84 | 2.49 |
| Sens 1 | 70% | 75% | 71% | 71% | 71% | 70% | 72% | 73% | 72% |
| Spec 1 | 32% | 42% | 30% | 37% | 46% | 40% | 21% | 42% | 21% |
| Cutoff 2 | 2.80 | 5.88 | 2.31 | 3.10 | 3.98 | 3.10 | 1.55 | 4.77 | 1.64 |
| Sens 2 | 81% | 81% | 81% | 80% | 83% | 80% | 81% | 82% | 80% |
| Spec 2 | 23% | 42% | 19% | 25% | 31% | 25% | 14% | 36% | 14% |
| Cutoff 3 | 0.765 | 4.11 | 0.541 | 1.24 | 0.418 | 1.26 | 0.820 | 3.36 | 0.820 |
| Sens 3 | 90% | 94% | 91% | 90% | 92% | 91% | 91% | 91% | 91% |
| Spec 3 | 9% | 32% | 7% | 11% | 7% | 10% | 9% | 27% | 8% |
| Cutoff 4 | 14.1 | 14.1 | 14.1 | 14.1 | 14.1 | 14.1 | 14.1 | 14.1 | 14.1 |
| Sens 4 | 30% | 50% | 29% | 41% | 29% | 45% | 32% | 50% | 35% |
| Spec 4 | 70% | 70% | 70% | 70% | 70% | 70% | 70% | 70% | 70% |
| Cutoff 5 | 23.6 | 22.6 | 22.7 | 23.6 | 22.6 | 22.7 | 23.6 | 22.6 | 22.7 |
| Sens 5 | 14% | 31% | 18% | 26% | 17% | 32% | 23% | 45% | 20% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 5 | 80% | 80% | 80% | 80% | 80% | 80% | 80% | 80% | 80% |
| Cutoff 6 | 45.8 | 43.6 | 43.2 | 45.8 | 43.6 | 43.2 | 45.8 | 43.6 | 43.2 |
| Sens 6 | 7% | 6% | 11% | 17% | 8% | 22% | 13% | 27% | 13% |
| Spec 6 | 90% | 90% | 90% | 90% | 90% | 90% | 90% | 90% | 90% |
| OR Quart 2 | 1.1 | 5.0 | 1.1 | 1.1 | 2.0 | 0.94 | 0.42 | 3.0 | 0.61 |
| p Value | 0.74 | 0.14 | 0.86 | 0.75 | 0.33 | 0.85 | 0.062 | 0.18 | 0.27 |
| 95% CI of | 0.58 | 0.59 | 0.55 | 0.58 | 0.50 | 0.47 | 0.17 | 0.61 | 0.25 |
| OR Quart2 | 2.1 | 43 | 2.0 | 2.1 | 8.1 | 1.9 | 1.0 | 15 | 1.5 |
| OR Quart 3 | 1.3 | 4.0 | 1.1 | 1.1 | 3.4 | 1.1 | 0.74 | 1.5 | 0.68 |
| p Value | 0.35 | 0.21 | 0.74 | 0.75 | 0.065 | 0.86 | 0.44 | 0.66 | 0.39 |
| 95% CI of | 0.72 | 0.45 | 0.58 | 0.58 | 0.93 | 0.54 | 0.34 | 0.25 | 0.29 |
| OR Quart3 | 2.5 | 36 | 2.1 | 2.1 | 12 | 2.1 | 1.6 | 9.1 | 1.6 |
| OR Quart 4 | 1.0 | 6.1 | 1.0 | 1.7 | 1.7 | 1.9 | 1.1 | 5.6 | 1.3 |
| p Value | 0.99 | 0.096 | 0.99 | 0.080 | 0.48 | 0.030 | 0.72 | 0.025 | 0.56 |
| 95% CI of | 0.52 | 0.73 | 0.52 | 0.94 | 0.40 | 1.1 | 0.57 | 1.2 | 0.59 |
| OR Quart4 | 1.9 | 51 | 1.9 | 3.1 | 7.0 | 3.5 | 2.3 | 26 | 2.7 |

**Vitamin D-binding protein**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 200 | 296 | 200 | 391 | 200 | 159 |
| Average | 7290 | 14000 | 7290 | 14000 | 7290 | 17600 |
| Stdev | 34200 | 47100 | 34200 | 57800 | 34200 | 67900 |
| p(t-test) | | 0.21 | | 0.19 | | 0.16 |
| Min | 0.259 | 1.50 | 0.259 | 1.39 | 0.259 | 0.337 |
| Max | 365000 | 283000 | 365000 | 379000 | 365000 | 347000 |
| n (Samp) | 456 | 49 | 456 | 63 | 456 | 26 |
| n (Patient) | 202 | 49 | 202 | 63 | 202 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 217 | 291 | 217 | 567 | 217 | 357 |
| Average | 7890 | 7050 | 7890 | 1570 | 7890 | 40400 |
| Stdev | 35900 | 13100 | 35900 | 3000 | 35900 | 115000 |
| p(t-test) | | 0.95 | | 0.50 | | 0.011 |
| Min | 0.259 | 6.04 | 0.259 | 54.6 | 0.259 | 0.612 |
| Max | 379000 | 34100 | 379000 | 11000 | 379000 | 347000 |
| n (Samp) | 634 | 8 | 634 | 15 | 634 | 9 |
| n (Patient) | 259 | 8 | 259 | 15 | 259 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 203 | 278 | 203 | 381 | 203 | 173 |
| Average | 8460 | 14000 | 8460 | 14600 | 8460 | 4310 |
| Stdev | 37700 | 49000 | 37700 | 59200 | 37700 | 11100 |
| p(t-test) | | 0.36 | | 0.27 | | 0.60 |
| Min | 0.200 | 1.50 | 0.200 | 1.39 | 0.200 | 0.337 |
| Max | 365000 | 283000 | 365000 | 379000 | 365000 | 51700 |
| n (Samp) | 482 | 45 | 482 | 60 | 482 | 23 |
| n (Patient) | 202 | 45 | 202 | 60 | 202 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.51 | 0.55 | 0.59 | 0.61 | 0.59 | 0.49 | 0.53 | 0.49 |
| SE | 0.045 | 0.10 | 0.046 | 0.040 | 0.078 | 0.041 | 0.059 | 0.099 | 0.062 |
| p | 0.11 | 0.92 | 0.25 | 0.018 | 0.17 | 0.031 | 0.82 | 0.77 | 0.92 |
| nCohort 1 | 456 | 634 | 482 | 456 | 634 | 482 | 456 | 634 | 482 |
| nCohort 2 | 49 | 8 | 45 | 63 | 15 | 60 | 26 | 9 | 23 |
| Cutoff 1 | 158 | 69.4 | 143 | 152 | 124 | 156 | 36.6 | 80.3 | 70.1 |
| Sens 1 | 71% | 75% | 71% | 71% | 73% | 70% | 73% | 78% | 74% |
| Spec 1 | 43% | 22% | 40% | 41% | 36% | 42% | 12% | 27% | 23% |
| Cutoff 2 | 69.4 | 15.6 | 101 | 94.1 | 111 | 98.6 | 11.7 | 0.892 | 33.9 |
| Sens 2 | 82% | 88% | 80% | 81% | 80% | 80% | 81% | 89% | 83% |
| Spec 2 | 23% | 7% | 32% | 31% | 33% | 31% | 6% | 1% | 11% |
| Cutoff 3 | 6.72 | 5.93 | 6.72 | 53.2 | 79.5 | 52.2 | 0.892 | 0.611 | 9.75 |
| Sens 3 | 92% | 100% | 91% | 90% | 93% | 90% | 92% | 100% | 91% |
| Spec 3 | 4% | 4% | 5% | 20% | 26% | 18% | 1% | 0% | 5% |
| Cutoff 4 | 476 | 498 | 498 | 476 | 498 | 498 | 476 | 498 | 498 |
| Sens 4 | 39% | 38% | 31% | 46% | 53% | 43% | 38% | 44% | 39% |
| Spec 4 | 70% | 70% | 70% | 70% | 70% | 70% | 70% | 70% | 70% |
| Cutoff 5 | 1080 | 1530 | 1490 | 1080 | 1530 | 1490 | 1080 | 1530 | 1490 |
| Sens 5 | 27% | 25% | 22% | 32% | 13% | 25% | 38% | 22% | 35% |
| Spec 5 | 80% | 80% | 80% | 80% | 80% | 80% | 80% | 80% | 80% |
| Cutoff 6 | 9170 | 10300 | 13600 | 9170 | 10300 | 13600 | 9170 | 10300 | 13600 |
| Sens 6 | 16% | 25% | 11% | 13% | 7% | 12% | 15% | 22% | 9% |
| Spec 6 | 90% | 90% | 90% | 90% | 90% | 90% | 90% | 90% | 90% |
| OR Quart 2 | 0.48 | 0 | 0.65 | 2.0 | 4.1 | 1.8 | 0.19 | 0.99 | 0.36 |
| p Value | 0.19 | na | 0.42 | 0.14 | 0.21 | 0.19 | 0.034 | 0.99 | 0.14 |
| 95% CI of | 0.16 | na | 0.22 | 0.81 | 0.45 | 0.74 | 0.040 | 0.14 | 0.094 |
| OR Quart2 | 1.4 | na | 1.9 | 4.8 | 37 | 4.5 | 0.88 | 7.1 | 1.4 |
| OR Quart 3 | 2.2 | 1.0 | 2.1 | 2.4 | 4.1 | 2.3 | 0.48 | 0.49 | 0.61 |
| p Value | 0.056 | 1.0 | 0.076 | 0.046 | 0.21 | 0.064 | 0.19 | 0.57 | 0.41 |
| 95% CI of | 0.98 | 0.20 | 0.92 | 1.0 | 0.45 | 0.95 | 0.16 | 0.044 | 0.20 |
| OR Quart3 | 4.9 | 5.0 | 5.0 | 5.8 | 37 | 5.5 | 1.4 | 5.5 | 1.9 |
| OR Quart 4 | 1.4 | 0.66 | 1.4 | 3.1 | 6.2 | 2.9 | 0.90 | 2.0 | 0.88 |
| p Value | 0.40 | 0.65 | 0.51 | 0.0094 | 0.094 | 0.014 | 0.83 | 0.42 | 0.80 |
| 95% CI of | 0.61 | 0.11 | 0.55 | 1.3 | 0.73 | 1.2 | 0.35 | 0.36 | 0.31 |
| OR Quart4 | 3.4 | 4.0 | 3.3 | 7.2 | 52 | 6.8 | 2.3 | 11 | 2.5 |

**Glucagon**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 238 | 347 | 238 | 238 | 238 | 347 |
| Average | 1460 | 3460 | 1460 | 1550 | 1460 | 3800 |
| Stdev | 2840 | 4170 | 2840 | 3090 | 2840 | 4460 |
| p(t-test) | | 0.0027 | | 0.87 | | 0.0023 |
| Min | 1.53 | 6.86 | 1.53 | 0.0247 | 1.53 | 6.71 |
| Max | 9330 | 9330 | 9330 | 9330 | 9330 | 9330 |
| n (Samp) | 248 | 22 | 248 | 32 | 248 | 16 |
| n (Patient) | 160 | 22 | 160 | 32 | 160 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 238 | 206 | 238 | 9330 |
| Average | nd | nd | 1720 | 536 | 1720 | 7060 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | nd | nd | 3110 | 930 | 3110 | 3930 |
| p(t-test) | nd | nd | | 0.35 | | 1.1E-5 |
| Min | nd | nd | 0.0247 | 9.15 | 0.0247 | 347 |
| Max | nd | nd | 9330 | 2420 | 9330 | 9330 |
| n (Samp) | nd | nd | 314 | 6 | 314 | 7 |
| n (Patient) | nd | nd | 187 | 6 | 187 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 238 | 347 | 238 | 238 | 238 | 347 |
| Average | 1380 | 3460 | 1380 | 1620 | 1380 | 3280 |
| Stdev | 2700 | 4170 | 2700 | 3220 | 2700 | 4430 |
| p(t-test) | | 0.0013 | | 0.65 | | 0.013 |
| Min | 1.53 | 6.86 | 1.53 | 0.0247 | 1.53 | 6.71 |
| Max | 9330 | 9330 | 9330 | 9330 | 9330 | 9330 |
| n (Samp) | 216 | 22 | 216 | 29 | 216 | 15 |
| n (Patient) | 133 | 22 | 133 | 29 | 133 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.73 | nd | 0.73 | 0.47 | 0.45 | 0.46 | 0.71 | 0.87 | 0.66 |
| SE | 0.063 | nd | 0.063 | 0.055 | 0.12 | 0.058 | 0.074 | 0.087 | 0.079 |
| p | 3.6E-4 | nd | 3.6E-4 | 0.55 | 0.70 | 0.51 | 0.0043 | 2.1E-5 | 0.040 |
| nCohort 1 | 248 | nd | 216 | 248 | 314 | 216 | 248 | 314 | 216 |
| nCohort 2 | 22 | nd | 22 | 32 | 6 | 29 | 16 | 7 | 15 |
| Cutoff 1 | 275 | nd | 238 | 8.98 | 30.4 | 8.64 | 275 | 4790 | 174 |
| Sens 1 | 82% | nd | 82% | 72% | 83% | 72% | 75% | 71% | 87% |
| Spec 1 | 58% | nd | 57% | 23% | 29% | 23% | 58% | 87% | 38% |
| Cutoff 2 | 275 | nd | 238 | 7.71 | 30.4 | 7.20 | 174 | 677 | 174 |
| Sens 2 | 82% | nd | 82% | 81% | 83% | 83% | 94% | 86% | 87% |
| Spec 2 | 58% | nd | 57% | 20% | 29% | 17% | 39% | 73% | 38% |
| Cutoff 3 | 174 | nd | 174 | 5.98 | 8.98 | 5.82 | 174 | 275 | 30.4 |
| Sens 3 | 95% | nd | 95% | 91% | 100% | 93% | 94% | 100% | 93% |
| Spec 3 | 39% | nd | 38% | 12% | 21% | 11% | 39% | 55% | 31% |
| Cutoff 4 | 347 | nd | 347 | 347 | 347 | 347 | 347 | 347 | 347 |
| Sens 4 | 45% | nd | 45% | 25% | 17% | 24% | 44% | 86% | 33% |
| Spec 4 | 74% | nd | 74% | 74% | 72% | 74% | 74% | 72% | 74% |
| Cutoff 5 | 2420 | nd | 2420 | 2420 | 2420 | 2420 | 2420 | 2420 | 2420 |
| Sens 5 | 32% | nd | 32% | 12% | 0% | 14% | 38% | 71% | 33% |
| Spec 5 | 89% | nd | 90% | 89% | 86% | 90% | 89% | 86% | 90% |
| Cutoff 6 | 9330 | nd | 4790 | 9330 | 9330 | 4790 | 9330 | 9330 | 4790 |
| Sens 6 | 0% | nd | 32% | 0% | 0% | 14% | 0% | 0% | 33% |
| Spec 6 | 100% | nd | 91% | 100% | 100% | 91% | 100% | 100% | 91% |
| OR Quart 2 | 3.0 | nd | 3.1 | 0.73 | 1.0 | 0.70 | 1.0 | >0 | 4.1 |
| p Value | 0.34 | nd | 0.34 | 0.57 | 1.0 | 0.56 | 1.0 | <na | 0.21 |
| 95% CI of | 0.31 | nd | 0.31 | 0.24 | 0.061 | 0.21 | 0.061 | >na | 0.45 |
| OR Quart2 | 30 | nd | 30 | 2.2 | 16 | 2.3 | 16 | na | 38 |
| OR Quart 3 | 10 | nd | 9.1 | 0.86 | 3.1 | 1.0 | 7.7 | >1.0 | 5.3 |
| p Value | 0.030 | nd | 0.041 | 0.78 | 0.33 | 0.97 | 0.060 | <0.99 | 0.13 |
| 95% CI of | 1.3 | nd | 1.1 | 0.29 | 0.31 | 0.33 | 0.92 | >0.062 | 0.60 |
| OR Quart3 | 83 | nd | 75 | 2.5 | 30 | 3.1 | 65 | na | 47 |
| OR Quart 4 | 10 | nd | 12 | 1.4 | 1.0 | 1.5 | 7.7 | >6.4 | 5.3 |
| p Value | 0.031 | nd | 0.022 | 0.46 | 1.0 | 0.41 | 0.060 | <0.089 | 0.13 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of OR Quart4 | 1.2 82 | nd nd | 1.4 94 | 0.54 3.8 | 0.061 16 | 0.55 4.4 | 0.92 65 | >0.75 na | 0.60 47 |

**Glucagon-like peptide 1**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 3.10 | 2.30 | 3.10 | 5.53 | 3.10 | 1.20 |
| Average | 9.80 | 3.72 | 9.80 | 9.62 | 9.80 | 3.20 |
| Stdev | 46.0 | 3.46 | 46.0 | 19.0 | 46.0 | 3.66 |
| p(t-test) |  | 0.54 |  | 0.98 |  | 0.57 |
| Min | 0.407 | 0.407 | 0.407 | 0.407 | 0.407 | 0.407 |
| Max | 519 | 9.60 | 519 | 107 | 519 | 9.60 |
| n (Samp) | 248 | 22 | 248 | 32 | 248 | 16 |
| n (Patient) | 160 | 22 | 160 | 32 | 160 | 16 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 3.10 | 5.53 | 3.10 | 2.30 |
| Average | nd | nd | 9.04 | 5.71 | 9.04 | 3.67 |
| Stdev | nd | nd | 41.4 | 3.73 | 41.4 | 4.10 |
| p(t-test) | nd | nd |  | 0.84 |  | 0.73 |
| Min | nd | nd | 0.407 | 0.407 | 0.407 | 0.407 |
| Max | nd | nd | 519 | 10.1 | 519 | 11.8 |
| n (Samp) | nd | nd | 314 | 6 | 314 | 7 |
| n (Patient) | nd | nd | 187 | 6 | 187 | 7 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 3.10 | 2.70 | 3.10 | 5.53 | 3.10 | 0.685 |
| Average | 10.4 | 4.04 | 10.4 | 9.74 | 10.4 | 3.36 |
| Stdev | 49.3 | 3.46 | 49.3 | 20.0 | 49.3 | 3.76 |
| p(t-test) |  | 0.55 |  | 0.94 |  | 0.58 |
| Min | 0.407 | 0.407 | 0.407 | 0.407 | 0.407 | 0.407 |
| Max | 519 | 9.60 | 519 | 107 | 519 | 9.60 |
| n (Samp) | 216 | 22 | 216 | 29 | 216 | 15 |
| n (Patient) | 133 | 22 | 133 | 29 | 133 | 15 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.39 | nd | 0.43 | 0.56 | 0.59 | 0.54 | 0.34 | 0.40 | 0.36 |
| SE | 0.066 | nd | 0.066 | 0.055 | 0.12 | 0.058 | 0.076 | 0.11 | 0.079 |
| p | 0.11 | nd | 0.29 | 0.30 | 0.47 | 0.51 | 0.038 | 0.41 | 0.080 |
| nCohort 1 | 248 | nd | 216 | 248 | 314 | 216 | 248 | 314 | 216 |
| nCohort 2 | 22 | nd | 22 | 32 | 6 | 29 | 16 | 7 | 15 |
| Cutoff 1 | 0.407 | nd | 0.685 | 2.30 | 2.30 | 0.685 | 0 | 0.685 | 0 |
| Sens 1 | 73% | nd | 73% | 72% | 83% | 79% | 100% | 71% | 100% |
| Spec 1 | 13% | nd | 17% | 31% | 35% | 17% | 0% | 20% | 0% |
| Cutoff 2 | 0 | nd | 0 | 0.685 | 2.30 | 0.407 | 0 | 0.407 | 0 |
| Sens 2 | 100% | nd | 100% | 81% | 83% | 90% | 100% | 86% | 100% |
| Spec 2 | 0% | nd | 0% | 17% | 35% | 14% | 0% | 14% | 0% |
| Cutoff 3 | 0 | nd | 0 | 0.407 | 0 | 0 | 0 | 0 | 0 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 3 | 100% | nd | 100% | 91% | 100% | 100% | 100% | 100% | 100% |
| Spec 3 | 0% | nd | 0% | 13% | 0% | 0% | 0% | 0% | 0% |
| Cutoff 4 | 5.53 | nd | 5.53 | 5.53 | 5.53 | 5.53 | 5.53 | 5.53 | 5.53 |
| Sens 4 | 32% | nd | 36% | 34% | 33% | 31% | 25% | 29% | 27% |
| Spec 4 | 71% | nd | 71% | 71% | 70% | 71% | 71% | 70% | 71% |
| Cutoff 5 | 9.60 | nd | 9.60 | 9.60 | 9.60 | 9.60 | 9.60 | 9.60 | 9.60 |
| Sens 5 | 0% | nd | 0% | 16% | 17% | 14% | 0% | 14% | 0% |
| Spec 5 | 93% | nd | 93% | 93% | 94% | 93% | 93% | 94% | 93% |
| Cutoff 6 | 9.60 | nd | 9.60 | 9.60 | 9.60 | 9.60 | 9.60 | 9.60 | 9.60 |
| Sens 6 | 0% | nd | 0% | 16% | 17% | 14% | 0% | 14% | 0% |
| Spec 6 | 93% | nd | 93% | 93% | 94% | 93% | 93% | 94% | 93% |
| OR Quart 2 | 2.1 | nd | 2.6 | 0.41 | 1.0 | 0.84 | 0.66 | 1.0 | 0.65 |
| p Value | 0.30 | nd | 0.19 | 0.16 | 1.0 | 0.77 | 0.65 | 0.99 | 0.65 |
| 95% CI of OR Quart2 | 0.51 | nd | 0.63 | 0.12 | 0.061 | 0.27 | 0.11 | 0.062 | 0.11 |
|  | 8.9 | nd | 10 | 1.4 | 16 | 2.7 | 4.1 | 16 | 4.1 |
| OR Quart 3 | 1.0 | nd | 1.4 | 0.87 | 2.0 | 1.0 | 0.66 | 2.1 | 0.65 |
| p Value | 1.0 | nd | 0.70 | 0.80 | 0.57 | 1.0 | 0.65 | 0.56 | 0.65 |
| 95% CI of OR Quart3 | 0.19 | nd | 0.29 | 0.32 | 0.18 | 0.33 | 0.11 | 0.18 | 0.11 |
|  | 5.1 | nd | 6.3 | 2.4 | 23 | 3.0 | 4.1 | 23 | 4.1 |
| OR Quart 4 | 3.8 | nd | 3.0 | 1.3 | 2.0 | 1.3 | 3.3 | 3.1 | 3.0 |
| p Value | 0.051 | nd | 0.12 | 0.63 | 0.57 | 0.62 | 0.083 | 0.33 | 0.12 |
| 95% CI of OR Quart4 | 1.00 | nd | 0.75 | 0.49 | 0.18 | 0.45 | 0.86 | 0.32 | 0.75 |
|  | 14 | nd | 12 | 3.3 | 23 | 3.8 | 13 | 31 | 12 |

**Appetite-regulating hormone**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.232 | 0.170 | 0.232 | 0.560 | 0.232 | 0.108 |
| Average | 0.890 | 0.997 | 0.890 | 2.97 | 0.890 | 0.599 |
| Stdev | 1.25 | 1.15 | 1.25 | 11.5 | 1.25 | 0.855 |
| p(t-test) |  | 0.70 |  | 0.0061 |  | 0.36 |
| Min | 0.0793 | 0.0793 | 0.0793 | 0.0793 | 0.0793 | 0.0793 |
| Max | 8.07 | 4.38 | 8.07 | 65.5 | 8.07 | 2.67 |
| n (Samp) | 248 | 22 | 248 | 32 | 248 | 16 |
| n (Patient) | 160 | 22 | 160 | 32 | 160 | 16 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 0.232 | 1.15 | 0.232 | 0.0793 |
| Average | nd | nd | 1.07 | 1.36 | 1.07 | 0.688 |
| Stdev | nd | nd | 3.84 | 1.05 | 3.84 | 1.07 |
| p(t-test) | nd | nd |  | 0.85 |  | 0.79 |
| Min | nd | nd | 0.0793 | 0.232 | 0.0793 | 0.0793 |
| Max | nd | nd | 65.5 | 2.67 | 65.5 | 2.67 |
| n (Samp) | nd | nd | 314 | 6 | 314 | 7 |
| n (Patient) | nd | nd | 187 | 6 | 187 | 7 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.232 | 0.131 | 0.232 | 0.560 | 0.232 | 0.108 |
| Average | 0.890 | 1.05 | 0.890 | 3.08 | 0.890 | 0.471 |
| Stdev | 1.28 | 1.38 | 1.28 | 12.1 | 1.28 | 0.671 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.57 | | 0.0099 | | 0.21 |
| Min | 0.0793 | 0.0793 | 0.0793 | 0.0793 | 0.0793 | 0.0793 |
| Max | 8.07 | 4.55 | 8.07 | 65.5 | 8.07 | 1.75 |
| n (Samp) | 216 | 22 | 216 | 29 | 216 | 15 |
| n (Patient) | 133 | 22 | 133 | 29 | 133 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.43 | nd | 0.42 | 0.55 | 0.72 | 0.53 | 0.35 | 0.29 | 0.34 |
| SE | 0.066 | nd | 0.066 | 0.055 | 0.12 | 0.058 | 0.076 | 0.11 | 0.079 |
| p | 0.32 | nd | 0.23 | 0.41 | 0.062 | 0.65 | 0.044 | 0.057 | 0.040 |
| nCohort 1 | 248 | nd | 216 | 248 | 314 | 216 | 248 | 314 | 216 |
| nCohort 2 | 22 | nd | 22 | 32 | 6 | 29 | 16 | 7 | 15 |
| Cutoff 1 | 0 | nd | 0 | 0.193 | 0.398 | 0.0995 | 0 | 0 | 0 |
| Sens 1 | 100% | nd | 100% | 72% | 83% | 79% | 100% | 100% | 100% |
| Spec 1 | 0% | nd | 0% | 34% | 56% | 17% | 0% | 0% | 0% |
| Cutoff 2 | 0 | nd | 0 | 0.0995 | 0.398 | 0.0793 | 0 | 0 | 0 |
| Sens 2 | 100% | nd | 100% | 81% | 83% | 86% | 100% | 100% | 100% |
| Spec 2 | 0% | nd | 0% | 17% | 56% | 8% | 0% | 0% | 0% |
| Cutoff 3 | 0 | nd | 0 | 0 | 0.193 | 0 | 0 | 0 | 0 |
| Sens 3 | 100% | nd | 100% | 100% | 100% | 100% | 100% | 100% | 100% |
| Spec 3 | 0% | nd | 0% | 0% | 38% | 0% | 0% | 0% | 0% |
| Cutoff 4 | 0.560 | nd | 0.560 | 0.560 | 0.560 | 0.560 | 0.560 | 0.560 | 0.560 |
| Sens 4 | 45% | nd | 41% | 31% | 50% | 28% | 25% | 29% | 20% |
| Spec 4 | 73% | nd | 74% | 73% | 73% | 74% | 73% | 73% | 74% |
| Cutoff 5 | 1.75 | nd | 1.75 | 1.75 | 1.75 | 1.75 | 1.75 | 1.75 | 1.75 |
| Sens 5 | 9% | nd | 14% | 22% | 33% | 17% | 6% | 14% | 0% |
| Spec 5 | 83% | nd | 83% | 83% | 84% | 83% | 83% | 84% | 83% |
| Cutoff 6 | 2.67 | nd | 2.67 | 2.67 | 2.67 | 2.67 | 2.67 | 2.67 | 2.67 |
| Sens 6 | 5% | nd | 9% | 6% | 0% | 7% | 0% | 0% | 0% |
| Spec 6 | 93% | nd | 93% | 93% | 94% | 93% | 93% | 94% | 93% |
| OR Quart 2 | 0 | nd | 0.27 | 1.2 | >1.0 | 0.84 | 0.24 | 0 | 0.32 |
| p Value | na | nd | 0.11 | 0.77 | <0.99 | 0.77 | 0.21 | na | 0.33 |
| 95% CI of | na | nd | 0.053 | 0.38 | >0.062 | 0.27 | 0.026 | na | 0.032 |
| OR Quart2 | na | nd | 1.3 | 3.7 | na | 2.7 | 2.2 | na | 3.2 |
| OR Quart 3 | 0.18 | nd | 0.40 | 1.6 | >2.1 | 1.2 | 1.3 | 0 | 1.0 |
| p Value | 0.029 | nd | 0.20 | 0.42 | <0.56 | 0.78 | 0.73 | na | 1.0 |
| 95% CI of | 0.037 | nd | 0.098 | 0.53 | >0.18 | 0.39 | 0.33 | na | 0.19 |
| OR Quart3 | 0.84 | nd | 1.6 | 4.7 | na | 3.4 | 5.0 | na | 5.2 |
| OR Quart 4 | 1.0 | nd | 1.5 | 1.8 | >3.1 | 1.1 | 1.6 | 2.6 | 3.0 |
| p Value | 0.97 | nd | 0.41 | 0.29 | <0.33 | 0.81 | 0.51 | 0.26 | 0.12 |
| 95% CI of | 0.39 | nd | 0.55 | 0.61 | >0.32 | 0.39 | 0.42 | 0.50 | 0.75 |
| OR Quart4 | 2.6 | nd | 4.4 | 5.2 | na | 3.4 | 5.8 | 14 | 12 |

**Islet amyloid polypeptide**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.227 | 0.0924 | 0.227 | 0.336 | 0.227 | 0.00270 |
| Average | 2.46 | 0.398 | 2.46 | 1.52 | 2.46 | 0.129 |
| Stdev | 10.5 | 0.690 | 10.5 | 4.16 | 10.5 | 0.222 |
| p(t-test) | | 0.36 | | 0.61 | | 0.38 |
| Min | 0.00151 | 0.00151 | 0.00151 | 0.00151 | 0.00151 | 0.00151 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 102 | 2.41 | 102 | 23.2 | 102 | 0.618 |
| n (Samp) | 248 | 22 | 248 | 32 | 248 | 16 |
| n (Patient) | 160 | 22 | 160 | 32 | 160 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.214 | 1.56 | 0.214 | 0.00187 |
| Average | nd | nd | 3.09 | 5.04 | 3.09 | 0.176 |
| Stdev | nd | nd | 14.4 | 9.05 | 14.4 | 0.218 |
| p(t-test) | nd | nd | | 0.74 | | 0.59 |
| Min | nd | nd | 0.00151 | 0.00945 | 0.00151 | 0.00151 |
| Max | nd | nd | 136 | 23.2 | 136 | 0.444 |
| n (Samp) | nd | nd | 314 | 6 | 314 | 7 |
| n (Patient) | nd | nd | 187 | 6 | 187 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.252 | 0.0924 | 0.252 | 0.227 | 0.252 | 0.00571 |
| Average | 2.21 | 0.404 | 2.21 | 0.733 | 2.21 | 1.66 |
| Stdev | 8.89 | 0.688 | 8.89 | 1.23 | 8.89 | 5.96 |
| p(t-test) | | 0.34 | | 0.37 | | 0.81 |
| Min | 0.00151 | 0.00151 | 0.00151 | 0.00151 | 0.00151 | 0.00151 |
| Max | 84.9 | 2.41 | 84.9 | 5.59 | 84.9 | 23.2 |
| n (Samp) | 216 | 22 | 216 | 29 | 216 | 15 |
| n (Patient) | 133 | 22 | 133 | 29 | 133 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.42 | nd | 0.42 | 0.52 | 0.66 | 0.46 | 0.26 | 0.28 | 0.31 |
| SE | 0.066 | nd | 0.066 | 0.055 | 0.12 | 0.058 | 0.073 | 0.11 | 0.078 |
| p | 0.23 | nd | 0.23 | 0.74 | 0.18 | 0.51 | 8.0E-4 | 0.047 | 0.013 |
| nCohort 1 | 248 | nd | 216 | 248 | 314 | 216 | 248 | 314 | 216 |
| nCohort 2 | 22 | nd | 22 | 32 | 6 | 29 | 16 | 7 | 15 |
| Cutoff 1 | 0.00571 | nd | 0.00571 | 0.00571 | 0.0290 | 0.00369 | 0 | 0 | 0.00151 |
| Sens 1 | 73% | nd | 77% | 75% | 83% | 76% | 100% | 100% | 73% |
| Spec 1 | 30% | nd | 27% | 30% | 38% | 15% | 0% | 0% | 5% |
| Cutoff 2 | 0.00151 | nd | 0.00151 | 0.00369 | 0.0290 | 0.00187 | 0 | 0 | 0 |
| Sens 2 | 91% | nd | 91% | 81% | 83% | 86% | 100% | 100% | 100% |
| Spec 2 | 6% | nd | 5% | 19% | 38% | 11% | 0% | 0% | 0% |
| Cutoff 3 | 0.00151 | nd | 0.00151 | 0.00187 | 0.00571 | 0.00151 | 0 | 0 | 0 |
| Sens 3 | 91% | nd | 91% | 91% | 100% | 93% | 100% | 100% | 100% |
| Spec 3 | 6% | nd | 5% | 11% | 32% | 5% | 0% | 0% | 0% |
| Cutoff 4 | 0.914 | nd | 1.04 | 0.914 | 0.631 | 1.04 | 0.914 | 0.631 | 1.04 |
| Sens 4 | 9% | nd | 9% | 22% | 50% | 17% | 0% | 0% | 7% |
| Spec 4 | 70% | nd | 70% | 70% | 70% | 70% | 70% | 70% | 70% |
| Cutoff 5 | 1.50 | nd | 1.50 | 1.50 | 1.42 | 1.50 | 1.50 | 1.42 | 1.50 |
| Sens 5 | 9% | nd | 9% | 19% | 50% | 14% | 0% | 0% | 7% |
| Spec 5 | 80% | nd | 80% | 80% | 80% | 80% | 80% | 80% | 80% |
| Cutoff 6 | 2.46 | nd | 2.35 | 2.46 | 2.41 | 2.35 | 2.46 | 2.41 | 2.35 |
| Sens 6 | 0% | nd | 5% | 16% | 50% | 10% | 0% | 0% | 7% |
| Spec 6 | 90% | nd | 91% | 90% | 90% | 91% | 90% | 90% | 91% |
| OR Quart 2 | 3.8 | nd | 3.3 | 1.3 | >3.1 | 2.0 | >3.1 | >3.2 | 2.0 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value | 0.10 | nd | 0.16 | 0.60 | <0.33 | 0.25 | <0.33 | <0.32 | 0.57 |
| 95% CI of | 0.77 | nd | 0.63 | 0.47 | >0.32 | 0.62 | >0.32 | >0.32 | 0.18 |
| OR Quart2 | 19 | nd | 17 | 3.8 | na | 6.3 | na | na | 23 |
| OR Quart 3 | 3.8 | nd | 5.1 | 1.3 | >0 | 1.2 | >4.3 | >0 | 5.4 |
| p Value | 0.10 | nd | 0.043 | 0.60 | <na | 0.73 | <0.20 | <na | 0.13 |
| 95% CI of | 0.76 | nd | 1.1 | 0.47 | >na | 0.36 | >0.46 | >na | 0.61 |
| OR Quart3 | 19 | nd | 25 | 3.8 | na | 4.3 | na | na | 48 |
| OR Quart 4 | 3.2 | nd | 2.7 | 1.0 | >3.1 | 2.0 | >10 | >4.3 | 8.0 |
| p Value | 0.16 | nd | 0.25 | 1.0 | <0.33 | 0.25 | <0.028 | <0.20 | 0.056 |
| 95% CI of | 0.63 | nd | 0.50 | 0.33 | >0.32 | 0.62 | >1.3 | >0.47 | 0.95 |
| OR Quart4 | 17 | nd | 14 | 3.0 | na | 6.3 | na | na | 67 |

**Interleukin-17A**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.00360 | 0.00320 | 0.00360 | 0.00444 | 0.00360 | 0.00332 |
| Average | 1.16 | 0.678 | 1.16 | 0.969 | 1.16 | 0.321 |
| Stdev | 8.66 | 2.08 | 8.66 | 4.28 | 8.66 | 1.27 |
| p(t-test) |  | 0.56 |  | 0.82 |  | 0.44 |
| Min | 0.000344 | 0.000344 | 0.000344 | 0.000344 | 0.000344 | 0.000380 |
| Max | 157 | 13.4 | 157 | 39.1 | 157 | 9.17 |
| n (Samp) | 1288 | 112 | 1288 | 113 | 1288 | 65 |
| n (Patient) | 488 | 112 | 488 | 113 | 488 | 65 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.00360 | 0.00409 | 0.00360 | 0.00360 | 0.00360 | 0.00233 |
| Average | 0.983 | 0.852 | 0.983 | 0.190 | 0.983 | 0.938 |
| Stdev | 7.46 | 2.27 | 7.46 | 0.458 | 7.46 | 2.59 |
| p(t-test) |  | 0.92 |  | 0.54 |  | 0.97 |
| Min | 0.000344 | 0.000344 | 0.000344 | 0.000380 | 0.000344 | 0.000380 |
| Max | 157 | 9.48 | 157 | 2.17 | 157 | 10.2 |
| n (Samp) | 1805 | 29 | 1805 | 33 | 1805 | 30 |
| n (Patient) | 610 | 29 | 610 | 33 | 610 | 30 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.00337 | 0.00320 | 0.00337 | 0.00492 | 0.00337 | 0.00332 |
| Average | 1.10 | 1.20 | 1.10 | 1.45 | 1.10 | 0.348 |
| Stdev | 8.55 | 4.21 | 8.55 | 4.99 | 8.55 | 1.37 |
| p(t-test) |  | 0.91 |  | 0.69 |  | 0.51 |
| Min | 0.000344 | 0.000344 | 0.000344 | 0.000344 | 0.000344 | 0.000380 |
| Max | 157 | 33.1 | 157 | 39.1 | 157 | 9.17 |
| n (Samp) | 1247 | 100 | 1247 | 102 | 1247 | 55 |
| n (Patient) | 430 | 100 | 430 | 102 | 430 | 55 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.49 | 0.55 | 0.51 | 0.52 | 0.46 | 0.56 | 0.46 | 0.46 | 0.47 |
| SE | 0.029 | 0.055 | 0.030 | 0.029 | 0.052 | 0.030 | 0.037 | 0.054 | 0.040 |
| p | 0.78 | 0.41 | 0.66 | 0.47 | 0.47 | 0.063 | 0.25 | 0.47 | 0.41 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| nCohort 1 | 1288 | 1805 | 1247 | 1288 | 1805 | 1247 | 1288 | 1805 | 1247 |
| nCohort 2 | 112 | 29 | 100 | 113 | 33 | 102 | 65 | 30 | 55 |
| Cutoff 1 | 0.000747 | 0.00172 | 0.00126 | 0.00174 | 0.000734 | 0.00190 | 0.000747 | 0.000734 | 0.000747 |
| Sens 1 | 71% | 72% | 70% | 71% | 73% | 71% | 71% | 70% | 73% |
| Spec 1 | 19% | 26% | 25% | 26% | 19% | 33% | 19% | 19% | 23% |
| Cutoff 2 | 0.000603 | 0.000662 | 0.000603 | 0.000603 | 0.000410 | 0.000603 | 0.000603 | 0.000662 | 0.000603 |
| Sens 2 | 80% | 86% | 81% | 81% | 82% | 81% | 80% | 80% | 80% |
| Spec 2 | 13% | 17% | 15% | 13% | 6% | 15% | 13% | 17% | 15% |
| Cutoff 3 | 0.000381 | 0.000380 | 0.000410 | 0.000381 | 0.000380 | 0.000381 | 0.000410 | 0.000410 | 0.000410 |
| Sens 3 | 91% | 97% | 90% | 92% | 91% | 92% | 92% | 93% | 93% |
| Spec 3 | 4% | 3% | 7% | 4% | 3% | 5% | 5% | 6% | 7% |
| Cutoff 4 | 0.00875 | 0.00875 | 0.00875 | 0.00875 | 0.00875 | 0.00875 | 0.00875 | 0.00875 | 0.00875 |
| Sens 4 | 38% | 41% | 38% | 36% | 27% | 41% | 28% | 37% | 24% |
| Spec 4 | 71% | 70% | 71% | 71% | 70% | 71% | 71% | 70% | 71% |
| Cutoff 5 | 0.111 | 0.143 | 0.121 | 0.111 | 0.143 | 0.121 | 0.111 | 0.143 | 0.121 |
| Sens 5 | 27% | 28% | 28% | 30% | 24% | 33% | 20% | 27% | 16% |
| Spec 5 | 80% | 80% | 80% | 80% | 80% | 80% | 80% | 80% | 80% |
| Cutoff 6 | 0.748 | 0.819 | 0.791 | 0.748 | 0.819 | 0.791 | 0.748 | 0.819 | 0.791 |
| Sens 6 | 16% | 21% | 18% | 11% | 6% | 15% | 8% | 13% | 7% |
| Spec 6 | 90% | 90% | 90% | 90% | 90% | 90% | 90% | 90% | 90% |
| OR Quart 2 | 0.55 | 0.62 | 0.68 | 0.49 | 1.0 | 0.53 | 0.70 | 0.22 | 1.2 |
| p Value | 0.037 | 0.40 | 0.19 | 0.021 | 1.00 | 0.059 | 0.35 | 0.053 | 0.68 |
| 95% CI of OR Quart2 | 0.31 0.97 | 0.20 1.9 | 0.38 1.2 | 0.27 0.90 | 0.37 2.7 | 0.28 1.0 | 0.33 1.5 | 0.047 1.0 | 0.54 2.6 |
| OR Quart 3 | 0.57 | 0.75 | 0.48 | 0.84 | 0.62 | 0.84 | 0.82 | 1.0 | 1.0 |
| p Value | 0.053 | 0.59 | 0.023 | 0.50 | 0.41 | 0.56 | 0.59 | 1.0 | 1.0 |
| 95% CI of OR Quart3 | 0.33 1.0 | 0.26 2.2 | 0.25 0.90 | 0.49 1.4 | 0.20 1.9 | 0.47 1.5 | 0.40 1.7 | 0.39 2.5 | 0.44 2.3 |
| OR Quart 4 | 1.0 | 1.3 | 1.1 | 1.1 | 1.5 | 1.4 | 1.3 | 1.1 | 1.4 |
| p Value | 0.90 | 0.64 | 0.61 | 0.81 | 0.37 | 0.19 | 0.41 | 0.81 | 0.34 |
| 95% CI of OR Quart4 | 0.63 1.7 | 0.49 3.2 | 0.68 1.9 | 0.65 1.8 | 0.61 3.7 | 0.84 2.4 | 0.69 2.5 | 0.45 2.8 | 0.68 3.1 |

**Insulin receptor substrate 1**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 2.78E-5 | 4.51E-6 | 2.78E-5 | 0.000827 | 2.78E-5 | 1.91E-5 |
| Average | 0.00323 | 0.00112 | 0.00323 | 0.00901 | 0.00323 | 0.000954 |
| Stdev | 0.0331 | 0.00267 | 0.0331 | 0.0379 | 0.0331 | 0.00137 |
| p(t-test) |  | 0.64 |  | 0.18 |  | 0.71 |
| Min | 1.25E-9 | 1.25E-9 | 1.25E-9 | 1.25E-9 | 1.25E-9 | 1.25E-9 |
| Max | 0.735 | 0.0142 | 0.735 | 0.282 | 0.735 | 0.00563 |
| n (Samp) | 515 | 55 | 515 | 68 | 515 | 30 |
| n (Patient) | 222 | 55 | 222 | 68 | 222 | 30 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 2.78E-5 | 0.00144 | 2.78E-5 | 0.00137 | 2.78E-5 | 0.00101 |
| Average | 0.00330 | 0.00249 | 0.00330 | 0.00152 | 0.00330 | 0.00144 |
| Stdev | 0.0301 | 0.00302 | 0.0301 | 0.00169 | 0.0301 | 0.00172 |
| p(t-test) |  | 0.92 |  | 0.80 |  | 0.83 |
| Min | 1.25E-9 | 7.25E-7 | 1.25E-9 | 7.25E-7 | 1.25E-9 | 9.90E-7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 0.735 | 0.00914 | 0.735 | 0.00552 | 0.735 | 0.00563 |
| n (Samp) | 734 | 14 | 734 | 18 | 734 | 12 |
| n (Patient) | 280 | 14 | 280 | 18 | 280 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.40E-5 | 2.32E-6 | 4.40E-5 | 4.40E-5 | 4.40E-5 | 4.51E-6 |
| Average | 0.00308 | 0.000702 | 0.00308 | 0.00956 | 0.00308 | 0.000724 |
| Stdev | 0.0318 | 0.00226 | 0.0318 | 0.0393 | 0.0318 | 0.00135 |
| p(t-test) | | 0.61 | | 0.14 | | 0.71 |
| Min | 1.25E-9 | 1.25E-9 | 1.25E-9 | 1.25E-9 | 1.25E-9 | 1.25E-9 |
| Max | 0.735 | 0.0142 | 0.735 | 0.282 | 0.735 | 0.00563 |
| n (Samp) | 558 | 48 | 558 | 63 | 558 | 25 |
| n (Patient) | 225 | 48 | 225 | 63 | 225 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.40 | 0.62 | 0.34 | 0.56 | 0.59 | 0.55 | 0.47 | 0.58 | 0.41 |
| SE | 0.042 | 0.081 | 0.044 | 0.038 | 0.071 | 0.039 | 0.055 | 0.087 | 0.061 |
| p | 0.013 | 0.15 | 4.2E-4 | 0.13 | 0.20 | 0.23 | 0.54 | 0.38 | 0.15 |
| nCohort 1 | 515 | 734 | 558 | 515 | 734 | 558 | 515 | 734 | 558 |
| nCohort 2 | 55 | 14 | 48 | 68 | 18 | 63 | 30 | 12 | 25 |
| Cutoff 1 | 9.90E-7 | 1.04E-5 | 9.90E-7 | 2.32E-6 | 3.73E-6 | 1.78E-6 | 1.78E-6 | 3.73E-6 | 1.50E-6 |
| Sens 1 | 75% | 71% | 73% | 71% | 72% | 76% | 73% | 75% | 76% |
| Spec 1 | 15% | 46% | 15% | 32% | 34% | 25% | 24% | 34% | 19% |
| Cutoff 2 | 7.25E-7 | 9.90E-7 | 1.25E-9 | 1.50E-6 | 1.78E-6 | 1.50E-6 | 1.50E-6 | 1.78E-6 | 7.25E-7 |
| Sens 2 | 80% | 86% | 85% | 84% | 83% | 84% | 80% | 83% | 88% |
| Spec 2 | 10% | 16% | 7% | 18% | 27% | 19% | 18% | 27% | 11% |
| Cutoff 3 | 0 | 1.25E-9 | 0 | 1.25E-9 | 7.25E-7 | 1.25E-9 | 7.25E-7 | 9.90E-7 | 1.25E-9 |
| Sens 3 | 100% | 100% | 100% | 96% | 94% | 95% | 90% | 92% | 92% |
| Spec 3 | 0% | 8% | 0% | 7% | 12% | 7% | 10% | 16% | 7% |
| Cutoff 4 | 0.00148 | 0.00110 | 0.00148 | 0.00148 | 0.00110 | 0.00148 | 0.00148 | 0.00110 | 0.00148 |
| Sens 4 | 18% | 50% | 12% | 41% | 50% | 37% | 23% | 50% | 12% |
| Spec 4 | 72% | 72% | 72% | 72% | 72% | 72% | 72% | 72% | 72% |
| Cutoff 5 | 0.00243 | 0.00229 | 0.00229 | 0.00243 | 0.00229 | 0.00229 | 0.00243 | 0.00229 | 0.00229 |
| Sens 5 | 13% | 29% | 6% | 21% | 28% | 35% | 10% | 17% | 12% |
| Spec 5 | 88% | 81% | 80% | 88% | 81% | 80% | 88% | 81% | 80% |
| Cutoff 6 | 0.00281 | 0.00281 | 0.00281 | 0.00281 | 0.00281 | 0.00281 | 0.00281 | 0.00281 | 0.00281 |
| Sens 6 | 13% | 29% | 6% | 15% | 11% | 16% | 7% | 8% | 8% |
| Spec 6 | 92% | 91% | 91% | 92% | 91% | 91% | 92% | 91% | 91% |
| OR Quart 2 | 1.1 | 0.66 | 1.4 | 0.86 | 1.3 | 0.80 | 1.0 | 1.5 | 2.0 |
| p Value | 0.80 | 0.65 | 0.58 | 0.69 | 0.70 | 0.56 | 0.99 | 0.66 | 0.32 |
| 95% CI of OR Quart2 | 0.44 | 0.11 | 0.46 | 0.40 | 0.30 | 0.37 | 0.34 | 0.25 | 0.50 |
| | 2.9 | 4.0 | 4.0 | 1.8 | 6.1 | 1.7 | 3.0 | 9.1 | 8.3 |
| OR Quart 3 | 1.6 | 1.0 | 2.3 | 0.72 | 1.3 | 0.73 | 1.2 | 1.0 | 2.8 |
| p Value | 0.28 | 1.0 | 0.11 | 0.42 | 0.70 | 0.43 | 0.78 | 1.0 | 0.14 |
| 95% CI of OR Quart3 | 0.68 | 0.20 | 0.84 | 0.33 | 0.30 | 0.33 | 0.41 | 0.14 | 0.72 |
| | 3.9 | 5.0 | 6.2 | 1.6 | 6.1 | 1.6 | 3.3 | 7.2 | 11 |
| OR Quart 4 | 2.7 | 2.0 | 3.9 | 1.7 | 2.4 | 1.4 | 1.2 | 2.5 | 2.8 |
| p Value | 0.016 | 0.32 | 0.0042 | 0.10 | 0.21 | 0.31 | 0.78 | 0.27 | 0.14 |
| 95% CI of OR Quart4 | 1.2 | 0.50 | 1.5 | 0.89 | 0.61 | 0.72 | 0.41 | 0.48 | 0.72 |
| | 6.2 | 8.3 | 10 | 3.4 | 9.4 | 2.8 | 3.3 | 13 | 11 |

**Involucrin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.447 | 0.942 | 0.447 | 0.943 | 0.447 | 0.422 |
| Average | 1.28 | 1.95 | 1.28 | 1.70 | 1.28 | 0.802 |
| Stdev | 4.30 | 2.64 | 4.30 | 2.50 | 4.30 | 1.12 |
| p(t-test) | | 0.15 | | 0.35 | | 0.41 |
| Min | 0.00244 | 0.0214 | 0.00244 | 0.0181 | 0.00244 | 0.0268 |
| Max | 102 | 17.3 | 102 | 17.0 | 102 | 6.07 |
| n (Samp) | 938 | 88 | 938 | 97 | 938 | 56 |
| n (Patient) | 414 | 88 | 414 | 97 | 414 | 56 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.506 | 0.544 | 0.506 | 0.473 | 0.506 | 0.933 |
| Average | 1.35 | 0.888 | 1.35 | 1.86 | 1.35 | 2.34 |
| Stdev | 3.89 | 0.881 | 3.89 | 3.14 | 3.89 | 3.25 |
| p(t-test) | | 0.61 | | 0.50 | | 0.22 |
| Min | 0.00244 | 0.0214 | 0.00244 | 0.0181 | 0.00244 | 0.0378 |
| Max | 102 | 3.40 | 102 | 14.3 | 102 | 14.5 |
| n (Samp) | 1263 | 19 | 1263 | 26 | 1263 | 24 |
| n (Patient) | 496 | 19 | 496 | 26 | 496 | 24 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.455 | 0.963 | 0.455 | 0.976 | 0.455 | 0.395 |
| Average | 1.31 | 2.07 | 1.31 | 1.88 | 1.31 | 0.743 |
| Stdev | 4.39 | 2.77 | 4.39 | 2.65 | 4.39 | 1.15 |
| p(t-test) | | 0.12 | | 0.23 | | 0.38 |
| Min | 0.00244 | 0.0411 | 0.00244 | 0.0508 | 0.00244 | 0.0214 |
| Max | 102 | 17.3 | 102 | 17.0 | 102 | 6.07 |
| n (Samp) | 899 | 81 | 899 | 90 | 899 | 46 |
| n (Patient) | 357 | 81 | 357 | 90 | 357 | 46 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.65 | 0.51 | 0.65 | 0.65 | 0.55 | 0.66 | 0.49 | 0.62 | 0.44 |
| SE | 0.033 | 0.067 | 0.034 | 0.031 | 0.059 | 0.032 | 0.040 | 0.062 | 0.045 |
| p | 6.0E-6 | 0.87 | 2.2E-5 | 2.7E-6 | 0.43 | 6.4E-7 | 0.71 | 0.052 | 0.21 |
| nCohort 1 | 938 | 1263 | 899 | 938 | 1263 | 899 | 938 | 1263 | 899 |
| nCohort 2 | 88 | 19 | 81 | 97 | 26 | 90 | 56 | 24 | 46 |
| Cutoff 1 | 0.407 | 0.308 | 0.407 | 0.485 | 0.285 | 0.513 | 0.246 | 0.320 | 0.193 |
| Sens 1 | 70% | 74% | 70% | 70% | 73% | 70% | 71% | 71% | 72% |
| Spec 1 | 47% | 34% | 47% | 53% | 32% | 54% | 31% | 35% | 23% |
| Cutoff 2 | 0.345 | 0.234 | 0.345 | 0.388 | 0.230 | 0.392 | 0.161 | 0.255 | 0.140 |
| Sens 2 | 81% | 84% | 80% | 80% | 81% | 80% | 80% | 83% | 80% |
| Spec 2 | 42% | 27% | 41% | 46% | 26% | 45% | 20% | 29% | 15% |
| Cutoff 3 | 0.142 | 0.0373 | 0.157 | 0.185 | 0.192 | 0.185 | 0.112 | 0.178 | 0.0808 |
| Sens 3 | 91% | 95% | 90% | 91% | 92% | 90% | 91% | 92% | 91% |
| Spec 3 | 17% | 2% | 18% | 24% | 22% | 22% | 12% | 21% | 7% |
| Cutoff 4 | 0.867 | 0.971 | 0.916 | 0.867 | 0.971 | 0.916 | 0.867 | 0.971 | 0.916 |
| Sens 4 | 53% | 37% | 51% | 52% | 38% | 53% | 23% | 50% | 17% |
| Spec 4 | 70% | 70% | 70% | 70% | 70% | 70% | 70% | 70% | 70% |
| Cutoff 5 | 1.35 | 1.56 | 1.35 | 1.35 | 1.56 | 1.35 | 1.35 | 1.56 | 1.35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 5 | 41% | 16% | 43% | 37% | 23% | 41% | 16% | 46% | 13% |
| Spec 5 | 80% | 80% | 80% | 80% | 80% | 80% | 80% | 80% | 80% |
| Cutoff 6 | 2.41 | 2.85 | 2.34 | 2.41 | 2.85 | 2.34 | 2.41 | 2.85 | 2.34 |
| Sens 6 | 27% | 5% | 30% | 22% | 23% | 26% | 5% | 29% | 7% |
| Spec 6 | 90% | 90% | 90% | 90% | 90% | 90% | 90% | 90% | 90% |
| OR Quart 2 | 1.5 | 2.0 | 1.4 | 1.7 | 2.5 | 1.5 | 1.8 | 2.0 | 2.6 |
| p Value | 0.27 | 0.33 | 0.44 | 0.19 | 0.12 | 0.31 | 0.17 | 0.33 | 0.051 |
| 95% CI of | 0.72 | 0.50 | 0.63 | 0.78 | 0.79 | 0.67 | 0.79 | 0.50 | 1.00 |
| OR Quart2 | 3.2 | 8.1 | 2.9 | 3.6 | 8.2 | 3.5 | 3.9 | 8.1 | 6.9 |
| OR Quart 3 | 1.6 | 1.7 | 1.4 | 2.8 | 1.3 | 2.8 | 1.5 | 1.3 | 1.7 |
| p Value | 0.20 | 0.48 | 0.44 | 0.0044 | 0.74 | 0.0075 | 0.31 | 0.71 | 0.31 |
| 95% CI of | 0.77 | 0.40 | 0.63 | 1.4 | 0.33 | 1.3 | 0.67 | 0.30 | 0.61 |
| OR Quart3 | 3.4 | 7.1 | 2.9 | 5.8 | 4.7 | 5.9 | 3.5 | 6.0 | 4.8 |
| OR Quart 4 | 3.6 | 1.7 | 3.5 | 4.0 | 1.8 | 4.4 | 1.4 | 3.7 | 2.6 |
| p Value | 1.7E-4 | 0.48 | 3.4E-4 | 9.4E-5 | 0.37 | 5.1E-5 | 0.40 | 0.044 | 0.051 |
| 95% CI of | 1.9 | 0.40 | 1.8 | 2.0 | 0.51 | 2.2 | 0.62 | 1.0 | 1.00 |
| OR Quart4 | 7.1 | 7.1 | 6.8 | 8.0 | 6.1 | 9.1 | 3.3 | 14 | 6.9 |

**Keratin, type II cytoskeletal 1; type1 cytoskeletal 10 (Keratin-1,-10 mix)**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0174 | 0.0113 | 0.0174 | 0.0502 | 0.0174 | 0.0113 |
| Average | 3.30 | 1.41 | 3.30 | 5.02 | 3.30 | 0.810 |
| Stdev | 17.2 | 2.88 | 17.2 | 31.4 | 17.2 | 1.80 |
| p(t-test) | | 0.31 | | 0.39 | | 0.28 |
| Min | 0.000410 | 0.000410 | 0.000410 | 0.000410 | 0.000410 | 0.000410 |
| Max | 367 | 16.3 | 367 | 309 | 367 | 10.3 |
| n (Samp) | 938 | 88 | 938 | 97 | 938 | 56 |
| n (Patient) | 414 | 88 | 414 | 97 | 414 | 56 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0174 | 0.0113 | 0.0174 | 0.0502 | 0.0174 | 0.00962 |
| Average | 3.12 | 1.66 | 3.12 | 2.52 | 3.12 | 1.14 |
| Stdev | 17.3 | 3.10 | 17.3 | 5.71 | 17.3 | 2.23 |
| p(t-test) | | 0.71 | | 0.86 | | 0.58 |
| Min | 0.000410 | 0.000410 | 0.000410 | 0.000410 | 0.000410 | 0.000410 |
| Max | 367 | 10.9 | 367 | 28.5 | 367 | 10.3 |
| n (Samp) | 1263 | 19 | 1263 | 26 | 1263 | 24 |
| n (Patient) | 496 | 19 | 496 | 26 | 496 | 24 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0174 | 0.0113 | 0.0174 | 0.0474 | 0.0174 | 0.0120 |
| Average | 2.82 | 1.86 | 2.82 | 5.15 | 2.82 | 0.771 |
| Stdev | 12.5 | 5.28 | 12.5 | 32.5 | 12.5 | 1.43 |
| p(t-test) | | 0.49 | | 0.17 | | 0.27 |
| Min | 0.000410 | 0.000410 | 0.000410 | 0.000410 | 0.000410 | 0.000410 |
| Max | 217 | 42.0 | 217 | 309 | 217 | 6.14 |
| n (Samp) | 899 | 81 | 899 | 90 | 899 | 46 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 357 | 81 | 357 | 90 | 357 | 46 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.44 | 0.45 | 0.45 | 0.52 | 0.56 | 0.52 | 0.39 | 0.41 | 0.40 |
| SE | 0.033 | 0.069 | 0.034 | 0.031 | 0.059 | 0.032 | 0.041 | 0.062 | 0.045 |
| p | 0.069 | 0.44 | 0.12 | 0.54 | 0.34 | 0.47 | 0.0087 | 0.16 | 0.021 |
| nCohort 1 | 938 | 1263 | 899 | 938 | 1263 | 899 | 938 | 1263 | 899 |
| nCohort 2 | 88 | 19 | 81 | 97 | 26 | 90 | 56 | 24 | 46 |
| Cutoff 1 | 0.00508 | 0.00474 | 0.00508 | 0.00694 | 0.00926 | 0.00787 | 0.00408 | 0.00408 | 0.00471 |
| Sens 1 | 70% | 74% | 72% | 71% | 77% | 70% | 73% | 71% | 72% |
| Spec 1 | 19% | 15% | 19% | 27% | 39% | 31% | 12% | 12% | 14% |
| Cutoff 2 | 0.000693 | 0.00408 | 0.000693 | 0.00508 | 0.00686 | 0.00508 | 0.000693 | 0 | 0 |
| Sens 2 | 85% | 84% | 84% | 81% | 85% | 83% | 80% | 100% | 100% |
| Spec 2 | 5% | 12% | 5% | 19% | 28% | 19% | 5% | 0% | 0% |
| Cutoff 3 | 0 | 0 | 0 | 0.000693 | 0.000693 | 0.000693 | 0 | 0 | 0 |
| Sens 3 | 100% | 100% | 100% | 94% | 96% | 93% | 100% | 100% | 100% |
| Spec 3 | 0% | 0% | 0% | 5% | 6% | 5% | 0% | 0% | 0% |
| Cutoff 4 | 1.18 | 1.39 | 1.18 | 1.18 | 1.39 | 1.18 | 1.18 | 1.39 | 1.18 |
| Sens 4 | 32% | 26% | 33% | 35% | 35% | 36% | 21% | 29% | 26% |
| Spec 4 | 70% | 72% | 70% | 70% | 72% | 70% | 70% | 72% | 70% |
| Cutoff 5 | 2.41 | 2.41 | 2.41 | 2.41 | 2.41 | 2.41 | 2.41 | 2.41 | 2.41 |
| Sens 5 | 20% | 21% | 22% | 21% | 27% | 21% | 9% | 8% | 11% |
| Spec 5 | 81% | 81% | 81% | 81% | 81% | 81% | 81% | 81% | 81% |
| Cutoff 6 | 5.76 | 5.20 | 5.20 | 5.76 | 5.20 | 5.20 | 5.76 | 5.20 | 5.20 |
| Sens 6 | 8% | 11% | 9% | 9% | 15% | 10% | 4% | 4% | 2% |
| Spec 6 | 90% | 90% | 90% | 90% | 90% | 90% | 90% | 90% | 90% |
| OR Quart 2 | 0.66 | 0.40 | 0.68 | 0.59 | 2.0 | 0.69 | 1.0 | 0.66 | 0.77 |
| p Value | 0.24 | 0.27 | 0.29 | 0.11 | 0.25 | 0.26 | 0.99 | 0.53 | 0.62 |
| 95% CI of OR Quart2 | 0.34 1.3 | 0.077 2.1 | 0.34 1.4 | 0.31 1.1 | 0.60 6.8 | 0.36 1.3 | 0.41 2.5 | 0.19 2.4 | 0.28 2.1 |
| OR Quart 3 | 0.85 | 1.2 | 0.79 | 1.0 | 1.5 | 0.95 | 1.2 | 0.66 | 1.1 |
| p Value | 0.63 | 0.76 | 0.49 | 0.90 | 0.53 | 0.88 | 0.66 | 0.53 | 0.81 |
| 95% CI of OR Quart3 | 0.45 1.6 | 0.36 4.0 | 0.40 1.6 | 0.59 1.8 | 0.42 5.4 | 0.52 1.7 | 0.51 2.9 | 0.19 2.4 | 0.45 2.8 |
| OR Quart 4 | 1.5 | 1.2 | 1.6 | 1.1 | 2.0 | 1.1 | 2.6 | 1.7 | 2.3 |
| p Value | 0.15 | 0.76 | 0.11 | 0.79 | 0.26 | 0.78 | 0.015 | 0.31 | 0.039 |
| 95% CI of OR Quart4 | 0.86 2.7 | 0.36 4.0 | 0.90 2.9 | 0.62 1.9 | 0.60 6.8 | 0.61 2.0 | 1.2 5.5 | 0.61 4.7 | 1.0 5.3 |

**NF-kappa-B inhibitor alpha**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.81E-5 | 0.000146 | 9.81E-5 | 0.000262 | 9.81E-5 | 2.56E-6 |
| Average | 0.00524 | 0.00595 | 0.00524 | 0.00158 | 0.00524 | 0.00414 |
| Stdev | 0.0265 | 0.0259 | 0.0265 | 0.00361 | 0.0265 | 0.0158 |
| p(t-test) | | 0.85 | | 0.26 | | 0.82 |
| Min | 3.52E-7 | 3.52E-7 | 3.52E-7 | 3.52E-7 | 3.52E-7 | 3.52E-7 |
| Max | 0.268 | 0.178 | 0.268 | 0.0167 | 0.268 | 0.0782 |
| n (Samp) | 515 | 55 | 515 | 68 | 515 | 30 |
| n (Patient) | 222 | 55 | 222 | 68 | 222 | 30 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.81E-5 | 0.000264 | 9.81E-5 | 0.000251 | 9.81E-5 | 1.06E-6 |
| Average | 0.00548 | 0.00775 | 0.00548 | 0.000656 | 0.00548 | 0.000443 |
| Stdev | 0.0279 | 0.0197 | 0.0279 | 0.00109 | 0.0279 | 0.00103 |
| p(t-test) | | 0.76 | | 0.46 | | 0.53 |
| Min | 3.52E-7 | 3.80E-7 | 3.52E-7 | 4.17E-7 | 3.52E-7 | 4.17E-7 |
| Max | 0.326 | 0.0725 | 0.326 | 0.00446 | 0.326 | 0.00353 |
| n (Samp) | 734 | 14 | 734 | 18 | 734 | 12 |
| n (Patient) | 280 | 14 | 280 | 18 | 280 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000103 | 0.000127 | 0.000103 | 0.000213 | 0.000103 | 2.25E-5 |
| Average | 0.00512 | 0.00581 | 0.00512 | 0.00574 | 0.00512 | 0.00495 |
| Stdev | 0.0257 | 0.0268 | 0.0257 | 0.0324 | 0.0257 | 0.0172 |
| p(t-test) | | 0.86 | | 0.86 | | 0.97 |
| Min | 3.52E-7 | 3.52E-7 | 3.52E-7 | 3.52E-7 | 3.52E-7 | 3.52E-7 |
| Max | 0.268 | 0.178 | 0.268 | 0.257 | 0.268 | 0.0782 |
| n (Samp) | 558 | 48 | 558 | 63 | 558 | 25 |
| n (Patient) | 225 | 48 | 225 | 63 | 225 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.57 | 0.46 | 0.56 | 0.57 | 0.57 | 0.38 | 0.37 | 0.44 |
| SE | 0.042 | 0.080 | 0.044 | 0.038 | 0.071 | 0.039 | 0.056 | 0.087 | 0.061 |
| p | 0.47 | 0.40 | 0.38 | 0.10 | 0.36 | 0.097 | 0.034 | 0.14 | 0.34 |
| nCohort 1 | 515 | 734 | 558 | 515 | 734 | 558 | 515 | 734 | 558 |
| nCohort 2 | 55 | 14 | 48 | 68 | 18 | 63 | 30 | 12 | 25 |
| Cutoff 1 | 5.23E-7 | 0.000132 | 5.23E-7 | 2.25E-5 | 2.94E-5 | 2.25E-5 | 4.18E-7 | 5.05E-7 | 1.03E-6 |
| Sens 1 | 71% | 71% | 71% | 74% | 72% | 71% | 77% | 75% | 72% |
| Spec 1 | 20% | 52% | 21% | 37% | 42% | 38% | 15% | 19% | 26% |
| Cutoff 2 | 4.17E-7 | 3.80E-7 | 4.17E-7 | 9.58E-7 | 2.18E-5 | 9.58E-7 | 4.17E-7 | 3.80E-7 | 5.05E-7 |
| Sens 2 | 82% | 86% | 83% | 82% | 83% | 81% | 80% | 100% | 84% |
| Spec 2 | 11% | 8% | 12% | 23% | 37% | 24% | 11% | 8% | 16% |
| Cutoff 3 | 3.52E-7 | 3.65E-7 | 0 | 4.17E-7 | 5.05E-7 | 4.17E-7 | 3.80E-7 | 3.80E-7 | 3.52E-7 |
| Sens 3 | 91% | 100% | 100% | 96% | 94% | 95% | 90% | 100% | 92% |
| Spec 3 | 4% | 6% | 0% | 11% | 19% | 12% | 7% | 8% | 4% |
| Cutoff 4 | 0.000532 | 0.000566 | 0.000532 | 0.000532 | 0.000566 | 0.000532 | 0.000532 | 0.000566 | 0.000532 |
| Sens 4 | 27% | 36% | 25% | 44% | 33% | 44% | 20% | 17% | 24% |
| Spec 4 | 71% | 71% | 71% | 71% | 71% | 71% | 71% | 71% | 71% |
| Cutoff 5 | 0.00101 | 0.00113 | 0.00102 | 0.00101 | 0.00113 | 0.00102 | 0.00101 | 0.00113 | 0.00102 |
| Sens 5 | 20% | 29% | 19% | 28% | 17% | 30% | 13% | 8% | 12% |
| Spec 5 | 80% | 80% | 80% | 80% | 80% | 80% | 80% | 80% | 80% |
| Cutoff 6 | 0.00351 | 0.00371 | 0.00351 | 0.00351 | 0.00371 | 0.00351 | 0.00351 | 0.00371 | 0.00351 |
| Sens 6 | 11% | 29% | 8% | 10% | 6% | 13% | 7% | 0% | 8% |
| Spec 6 | 90% | 90% | 90% | 90% | 90% | 90% | 90% | 90% | 90% |
| OR Quart 2 | 1.5 | 0.33 | 1.7 | 0.85 | 2.5 | 1.2 | 1.8 | 1.0 | 2.8 |
| p Value | 0.33 | 0.34 | 0.22 | 0.68 | 0.27 | 0.69 | 0.36 | 1.00 | 0.14 |
| 95% CI of | 0.68 | 0.034 | 0.74 | 0.39 | 0.49 | 0.54 | 0.52 | 0.14 | 0.72 |
| OR Quart2 | 3.1 | 3.2 | 3.8 | 1.8 | 13 | 2.5 | 6.3 | 7.2 | 11 |
| OR Quart 3 | 0.29 | 1.7 | 0.69 | 1.1 | 4.1 | 0.92 | 1.5 | 1.0 | 2.4 |
| p Value | 0.033 | 0.48 | 0.46 | 0.72 | 0.075 | 0.83 | 0.51 | 1.0 | 0.21 |
| 95% CI of | 0.091 | 0.40 | 0.25 | 0.55 | 0.87 | 0.40 | 0.42 | 0.14 | 0.61 |
| OR Quart3 | 0.91 | 7.2 | 1.9 | 2.4 | 20 | 2.1 | 5.6 | 7.2 | 9.5 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 4 | 1.6 | 1.7 | 1.6 | 1.6 | 1.5 | 1.9 | 3.5 | 3.1 | 2.4 |
| p Value | 0.19 | 0.48 | 0.29 | 0.17 | 0.65 | 0.083 | 0.032 | 0.17 | 0.21 |
| 95% CI of | 0.78 | 0.40 | 0.68 | 0.81 | 0.25 | 0.92 | 1.1 | 0.61 | 0.61 |
| OR Quart4 | 3.4 | 7.2 | 3.6 | 3.2 | 9.1 | 3.9 | 11 | 15 | 9.5 |

**Beta-nerve growth factor**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 2.58 | 1.85 | 2.58 | 2.88 | 2.58 | 1.89 |
| Average | 3.97 | 3.46 | 3.97 | 5.10 | 3.97 | 2.69 |
| Stdev | 5.00 | 4.55 | 5.00 | 7.55 | 5.00 | 2.80 |
| p(t-test) |  | 0.47 |  | 0.10 |  | 0.16 |
| Min | 0.000201 | 0.000201 | 0.000201 | 0.000506 | 0.000201 | 0.000201 |
| Max | 44.0 | 22.1 | 44.0 | 46.1 | 44.0 | 11.7 |
| n (Samp) | 516 | 55 | 516 | 68 | 516 | 30 |
| n (Patient) | 224 | 55 | 224 | 68 | 224 | 30 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 2.52 | 1.29 | 2.52 | 2.16 | 2.52 | 1.33 |
| Average | 3.97 | 3.17 | 3.97 | 2.45 | 3.97 | 2.08 |
| Stdev | 5.18 | 5.73 | 5.18 | 2.04 | 5.18 | 2.41 |
| p(t-test) |  | 0.57 |  | 0.21 |  | 0.21 |
| Min | 0.000201 | 0.000201 | 0.000201 | 0.000244 | 0.000201 | 0.000506 |
| Max | 46.1 | 22.1 | 46.1 | 6.35 | 46.1 | 8.38 |
| n (Samp) | 736 | 14 | 736 | 18 | 736 | 12 |
| n (Patient) | 282 | 14 | 282 | 18 | 282 | 12 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 2.32 | 1.78 | 2.32 | 3.08 | 2.32 | 1.99 |
| Average | 3.66 | 3.19 | 3.66 | 5.38 | 3.66 | 2.94 |
| Stdev | 4.54 | 3.95 | 4.54 | 7.77 | 4.54 | 2.96 |
| p(t-test) |  | 0.49 |  | 0.0095 |  | 0.43 |
| Min | 0.000201 | 0.000506 | 0.000201 | 0.000506 | 0.000201 | 0.000201 |
| Max | 35.5 | 20.4 | 35.5 | 46.1 | 35.5 | 11.7 |
| n (Samp) | 559 | 48 | 559 | 63 | 559 | 25 |
| n (Patient) | 227 | 48 | 227 | 63 | 227 | 25 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.45 | 0.38 | 0.46 | 0.52 | 0.43 | 0.55 | 0.42 | 0.37 | 0.47 |
| SE | 0.042 | 0.081 | 0.044 | 0.038 | 0.071 | 0.039 | 0.056 | 0.087 | 0.060 |
| p | 0.20 | 0.13 | 0.33 | 0.58 | 0.35 | 0.17 | 0.18 | 0.13 | 0.62 |
| nCohort 1 | 516 | 736 | 559 | 516 | 736 | 559 | 516 | 736 | 559 |
| nCohort 2 | 55 | 14 | 48 | 68 | 18 | 63 | 30 | 12 | 25 |
| Cutoff 1 | 0.886 | 0.292 | 0.825 | 1.22 | 1.24 | 1.21 | 1.04 | 0.825 | 1.40 |
| Sens 1 | 71% | 71% | 71% | 71% | 72% | 71% | 70% | 75% | 72% |
| Spec 1 | 22% | 11% | 21% | 29% | 31% | 31% | 25% | 21% | 36% |
| Cutoff 2 | 0.462 | 0.223 | 0.261 | 0.717 | 0.135 | 0.781 | 0.494 | 0.135 | 0.495 |
| Sens 2 | 80% | 86% | 83% | 82% | 83% | 81% | 80% | 83% | 80% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 2 | 13% | 10% | 11% | 17% | 9% | 21% | 14% | 9% | 15% |
| Cutoff 3 | 0.000506 | 0.111 | 0.000506 | 0.397 | 0.000499 | 0.462 | 0.161 | 0.000605 | 0.161 |
| Sens 3 | 93% | 93% | 94% | 91% | 94% | 90% | 90% | 92% | 92% |
| Spec 3 | 4% | 9% | 4% | 11% | 2% | 14% | 9% | 6% | 10% |
| Cutoff 4 | 4.17 | 4.06 | 3.86 | 4.17 | 4.06 | 3.86 | 4.17 | 4.06 | 3.86 |
| Sens 4 | 25% | 21% | 25% | 32% | 22% | 37% | 20% | 17% | 28% |
| Spec 4 | 70% | 70% | 70% | 70% | 70% | 70% | 70% | 70% | 70% |
| Cutoff 5 | 5.67 | 5.66 | 5.25 | 5.67 | 5.66 | 5.25 | 5.67 | 5.66 | 5.25 |
| Sens 5 | 16% | 14% | 17% | 25% | 11% | 29% | 17% | 8% | 20% |
| Spec 5 | 80% | 80% | 80% | 80% | 80% | 80% | 80% | 80% | 80% |
| Cutoff 6 | 9.07 | 9.25 | 8.31 | 9.07 | 9.25 | 8.31 | 9.07 | 9.25 | 8.31 |
| Sens 6 | 13% | 7% | 12% | 15% | 0% | 19% | 3% | 0% | 8% |
| Spec 6 | 90% | 90% | 90% | 90% | 90% | 90% | 90% | 90% | 90% |
| OR Quart 2 | 0.75 | 1.5 | 0.90 | 0.75 | 3.6 | 0.66 | 1.0 | 2.0 | 1.0 |
| p Value | 0.52 | 0.65 | 0.82 | 0.45 | 0.11 | 0.31 | 0.99 | 0.57 | 1.0 |
| 95% CI of OR Quart2 | 0.32 1.8 | 0.25 9.2 | 0.37 2.2 | 0.36 1.6 | 0.74 18 | 0.30 1.5 | 0.28 3.6 | 0.18 22 | 0.28 3.5 |
| OR Quart 3 | 1.2 | 1.5 | 1.1 | 0.88 | 2.0 | 1.0 | 2.5 | 5.1 | 1.9 |
| p Value | 0.69 | 0.65 | 0.83 | 0.72 | 0.42 | 1.0 | 0.089 | 0.14 | 0.28 |
| 95% CI of OR Quart3 | 0.54 2.6 | 0.25 9.1 | 0.47 2.6 | 0.43 1.8 | 0.37 11 | 0.48 2.1 | 0.87 7.4 | 0.59 44 | 0.61 5.7 |
| OR Quart 4 | 1.4 | 3.1 | 1.4 | 1.1 | 2.6 | 1.3 | 1.6 | 4.1 | 1.2 |
| p Value | 0.43 | 0.17 | 0.40 | 0.73 | 0.27 | 0.49 | 0.39 | 0.21 | 0.76 |
| 95% CI of OR Quart4 | 0.63 2.9 | 0.61 15 | 0.63 3.2 | 0.57 2.2 | 0.49 13 | 0.64 2.6 | 0.53 5.2 | 0.45 37 | 0.36 4.1 |

**Pancreatic prohormone**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.69 | 15.7 | 5.69 | 6.89 | 5.69 | 0.491 |
| Average | 89.0 | 96.7 | 89.0 | 83.4 | 89.0 | 27.0 |
| Stdev | 183 | 224 | 183 | 164 | 183 | 58.1 |
| p(t-test) | | 0.85 | | 0.87 | | 0.18 |
| Min | 0.117 | 0.117 | 0.117 | 0.117 | 0.117 | 0.117 |
| Max | 1220 | 1020 | 1220 | 690 | 1220 | 225 |
| n (Samp) | 248 | 22 | 248 | 32 | 248 | 16 |
| n (Patient) | 160 | 22 | 160 | 32 | 160 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 4.35 | 98.1 | 4.35 | 33.7 |
| Average | nd | nd | 85.1 | 249 | 85.1 | 82.5 |
| Stdev | nd | nd | 181 | 316 | 181 | 105 |
| p(t-test) | nd | nd | | 0.032 | | 0.97 |
| Min | nd | nd | 0.117 | 0.134 | 0.117 | 0.122 |
| Max | nd | nd | 1220 | 728 | 1220 | 234 |
| n (Samp) | nd | nd | 314 | 6 | 314 | 7 |
| n (Patient) | nd | nd | 187 | 6 | 187 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 11.7 | 15.7 | 11.7 | 9.57 | 11.7 | 0.293 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 91.2 | 96.6 | 91.2 | 68.8 | 91.2 | 54.0 |
| Stdev | 185 | 224 | 185 | 145 | 185 | 145 |
| p(t-test) | | 0.90 | | 0.53 | | 0.45 |
| Min | 0.117 | 0.117 | 0.117 | 0.117 | 0.117 | 0.117 |
| Max | 1220 | 1020 | 1220 | 690 | 1220 | 567 |
| n (Samp) | 216 | 22 | 216 | 29 | 216 | 15 |
| n (Patient) | 133 | 22 | 133 | 29 | 133 | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | nd | 0.48 | 0.49 | 0.68 | 0.47 | 0.35 | 0.54 | 0.35 |
| SE | 0.065 | nd | 0.065 | 0.055 | 0.12 | 0.058 | 0.077 | 0.11 | 0.079 |
| p | 0.92 | nd | 0.73 | 0.87 | 0.14 | 0.56 | 0.048 | 0.73 | 0.059 |
| nCohort 1 | 248 | nd | 216 | 248 | 314 | 216 | 248 | 314 | 216 |
| nCohort 2 | 22 | nd | 22 | 32 | 6 | 29 | 16 | 7 | 15 |
| Cutoff 1 | 0.117 | nd | 0.117 | 0.134 | 0.635 | 0.134 | 0.117 | 0.635 | 0.117 |
| Sens 1 | 82% | nd | 82% | 75% | 83% | 76% | 75% | 71% | 73% |
| Spec 1 | 5% | nd | 5% | 21% | 32% | 18% | 5% | 32% | 5% |
| Cutoff 2 | 0.117 | nd | 0.117 | 0.122 | 0.635 | 0.122 | 0 | 0.117 | 0 |
| Sens 2 | 82% | nd | 82% | 84% | 83% | 83% | 100% | 100% | 100% |
| Spec 2 | 5% | nd | 5% | 11% | 32% | 8% | 0% | 8% | 0% |
| Cutoff 3 | 0 | nd | 0 | 0.117 | 0.122 | 0.117 | 0 | 0.117 | 0 |
| Sens 3 | 100% | nd | 100% | 94% | 100% | 93% | 100% | 100% | 100% |
| Spec 3 | 0% | nd | 0% | 5% | 15% | 5% | 0% | 8% | 0% |
| Cutoff 4 | 40.9 | nd | 46.0 | 40.9 | 40.4 | 46.0 | 40.9 | 40.4 | 46.0 |
| Sens 4 | 45% | nd | 41% | 34% | 67% | 28% | 12% | 43% | 20% |
| Spec 4 | 70% | nd | 70% | 70% | 70% | 70% | 70% | 70% | 70% |
| Cutoff 5 | 118 | nd | 118 | 118 | 96.3 | 118 | 118 | 96.3 | 118 |
| Sens 5 | 14% | nd | 14% | 22% | 50% | 17% | 6% | 29% | 7% |
| Spec 5 | 80% | nd | 80% | 80% | 80% | 80% | 80% | 80% | 80% |
| Cutoff 6 | 338 | nd | 347 | 338 | 310 | 347 | 338 | 310 | 347 |
| Sens 6 | 9% | nd | 9% | 6% | 33% | 3% | 0% | 0% | 7% |
| Spec 6 | 90% | nd | 90% | 90% | 90% | 90% | 90% | 90% | 90% |
| OR Quart 2 | 0.60 | nd | 0.47 | 0.56 | 1.0 | 1.0 | 2.6 | 0.49 | 1.4 |
| p Value | 0.40 | nd | 0.24 | 0.29 | 1.0 | 0.97 | 0.26 | 0.57 | 0.70 |
| 95% CI of OR Quart2 | 0.19 2.0 | nd | 0.13 1.7 | 0.19 1.6 | 0.061 16 | 0.33 3.1 | 0.49 14 | 0.044 5.6 | 0.29 6.4 |
| OR Quart 3 | 0.23 | nd | 0.34 | 0.67 | 0 | 0.70 | 1.0 | 0.49 | 0 |
| p Value | 0.068 | nd | 0.13 | 0.44 | na | 0.56 | 1.0 | 0.57 | na |
| 95% CI of OR Quart3 | 0.046 1.1 | nd | 0.086 1.4 | 0.24 1.9 | na na | 0.21 2.3 | 0.14 7.3 | 0.044 5.6 | na na |
| OR Quart 4 | 0.88 | nd | 0.88 | 0.89 | 4.2 | 1.5 | 3.8 | 1.5 | 3.0 |
| p Value | 0.81 | nd | 0.81 | 0.81 | 0.21 | 0.41 | 0.10 | 0.66 | 0.12 |
| 95% CI of OR Quart4 | 0.30 2.6 | nd | 0.30 2.6 | 0.34 2.3 | 0.45 38 | 0.55 4.4 | 0.76 19 | 0.24 9.2 | 0.75 12 |

**Transthyretin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 23.4 | 33.6 | 23.4 | 39.7 | 23.4 | 17.6 |
| Average | 45.4 | 62.2 | 45.4 | 86.7 | 45.4 | 36.6 |
| Stdev | 68.4 | 125 | 68.4 | 234 | 68.4 | 48.2 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.082 | | 7.8E-4 | | 0.42 |
| Min | 0.000148 | 3.93 | 0.000148 | 2.10 | 0.000148 | 0.773 |
| Max | 668 | 997 | 668 | 2000 | 668 | 241 |
| n (Samp) | 599 | 70 | 599 | 79 | 599 | 40 |
| n (Patient) | 281 | 70 | 281 | 79 | 281 | 40 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.8 | 53.3 | 24.8 | 33.0 | 24.8 | 42.5 |
| Average | 52.0 | 84.7 | 52.0 | 102 | 52.0 | 89.4 |
| Stdev | 120 | 76.1 | 120 | 229 | 120 | 104 |
| p(t-test) | | 0.39 | | 0.089 | | 0.22 |
| Min | 0.000148 | 17.5 | 0.000148 | 3.17 | 0.000148 | 0.773 |
| Max | 2000 | 229 | 2000 | 997 | 2000 | 380 |
| n (Samp) | 829 | 10 | 829 | 18 | 829 | 16 |
| n (Patient) | 353 | 10 | 353 | 18 | 353 | 16 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.1 | 33.0 | 24.1 | 43.0 | 24.1 | 17.7 |
| Average | 46.9 | 59.1 | 46.9 | 90.0 | 46.9 | 35.8 |
| Stdev | 69.3 | 126 | 69.3 | 241 | 69.3 | 39.8 |
| p(t-test) | | 0.22 | | 6.9E-4 | | 0.35 |
| Min | 0.000148 | 3.93 | 0.000148 | 2.10 | 0.000148 | 1.67 |
| Max | 668 | 997 | 668 | 2000 | 668 | 182 |
| n (Samp) | 608 | 66 | 608 | 74 | 608 | 35 |
| n (Patient) | 266 | 66 | 266 | 74 | 266 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.58 | 0.73 | 0.55 | 0.60 | 0.57 | 0.60 | 0.46 | 0.63 | 0.47 |
| SE | 0.037 | 0.091 | 0.038 | 0.035 | 0.071 | 0.036 | 0.048 | 0.076 | 0.051 |
| p | 0.039 | 0.011 | 0.17 | 0.0046 | 0.31 | 0.0059 | 0.47 | 0.083 | 0.57 |
| nCohort 1 | 599 | 829 | 608 | 599 | 829 | 608 | 599 | 829 | 608 |
| nCohort 2 | 70 | 10 | 66 | 79 | 18 | 74 | 40 | 16 | 35 |
| Cutoff 1 | 20.3 | 46.1 | 16.1 | 18.9 | 18.9 | 20.8 | 13.7 | 26.8 | 13.9 |
| Sens 1 | 70% | 70% | 71% | 71% | 72% | 70% | 70% | 75% | 71% |
| Spec 1 | 45% | 69% | 35% | 42% | 41% | 44% | 31% | 53% | 30% |
| Cutoff 2 | 12.3 | 34.6 | 11.0 | 12.1 | 8.67 | 13.9 | 9.49 | 15.4 | 9.58 |
| Sens 2 | 80% | 80% | 80% | 81% | 83% | 81% | 80% | 81% | 80% |
| Spec 2 | 29% | 62% | 24% | 28% | 19% | 30% | 22% | 33% | 21% |
| Cutoff 3 | 7.87 | 26.9 | 7.20 | 8.67 | 4.98 | 9.87 | 5.71 | 4.36 | 8.58 |
| Sens 3 | 90% | 90% | 91% | 91% | 94% | 91% | 90% | 94% | 91% |
| Spec 3 | 20% | 53% | 16% | 21% | 9% | 22% | 14% | 8% | 20% |
| Cutoff 4 | 46.2 | 46.7 | 47.0 | 46.2 | 46.7 | 47.0 | 46.2 | 46.7 | 47.0 |
| Sens 4 | 33% | 60% | 32% | 43% | 39% | 43% | 18% | 50% | 17% |
| Spec 4 | 70% | 70% | 70% | 70% | 70% | 70% | 70% | 70% | 70% |
| Cutoff 5 | 63.1 | 64.9 | 63.8 | 63.1 | 64.9 | 63.8 | 63.1 | 64.9 | 63.8 |
| Sens 5 | 26% | 40% | 24% | 28% | 22% | 30% | 15% | 44% | 17% |
| Spec 5 | 80% | 80% | 80% | 80% | 80% | 80% | 80% | 80% | 80% |
| Cutoff 6 | 100 | 105 | 106 | 100 | 105 | 106 | 100 | 105 | 106 |
| Sens 6 | 13% | 30% | 11% | 15% | 22% | 15% | 8% | 31% | 6% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 6 | 90% | 90% | 90% | 90% | 90% | 90% | 90% | 90% | 90% |
| OR Quart 2 | 0.75 | >1.0 | 0.77 | 0.92 | 0.49 | 1.4 | 2.1 | 0.33 | 1.7 |
| p Value | 0.52 | <1.0 | 0.52 | 0.83 | 0.42 | 0.42 | 0.15 | 0.34 | 0.31 |
| 95% CI of | 0.32 | >0.062 | 0.34 | 0.42 | 0.089 | 0.62 | 0.76 | 0.034 | 0.61 |
| OR Quart2 | 1.8 | na | 1.7 | 2.0 | 2.7 | 3.1 | 5.7 | 3.2 | 4.8 |
| OR Quart 3 | 2.3 | >5.1 | 1.8 | 2.2 | 2.0 | 2.5 | 1.9 | 1.7 | 1.7 |
| p Value | 0.021 | <0.14 | 0.088 | 0.023 | 0.25 | 0.016 | 0.22 | 0.48 | 0.31 |
| 95% CI of | 1.1 | >0.59 | 0.91 | 1.1 | 0.60 | 1.2 | 0.68 | 0.40 | 0.61 |
| OR Quart3 | 4.6 | na | 3.7 | 4.3 | 6.8 | 5.2 | 5.3 | 7.1 | 4.8 |
| OR Quart 4 | 1.6 | >4.1 | 1.2 | 1.8 | 1.00 | 2.2 | 1.9 | 2.4 | 1.5 |
| p Value | 0.21 | <0.21 | 0.58 | 0.092 | 0.99 | 0.035 | 0.21 | 0.22 | 0.42 |
| 95% CI of | 0.77 | >0.45 | 0.59 | 0.91 | 0.25 | 1.1 | 0.69 | 0.60 | 0.54 |
| OR Quart4 | 3.3 | na | 2.6 | 3.7 | 4.0 | 4.8 | 5.3 | 9.3 | 4.4 |

**C-peptide Urine**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22500 | 22700 | 22500 | 19900 | 22500 | 21000 |
| Average | 33600 | 31100 | 33600 | 27000 | 33600 | 26600 |
| Stdev | 30100 | 30400 | 30100 | 25900 | 30100 | 24800 |
| p(t-test) | | 0.51 | | 0.062 | | 0.14 |
| Min | 0.00978 | 55.3 | 0.00978 | 133 | 0.00978 | 53.8 |
| Max | 104000 | 114000 | 104000 | 100000 | 104000 | 100000 |
| n (Samp) | 599 | 70 | 599 | 79 | 599 | 41 |
| n (Patient) | 281 | 70 | 281 | 79 | 281 | 41 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22000 | 7840 | 22000 | 24900 | 22000 | 7680 |
| Average | 32100 | 20400 | 32100 | 35200 | 32100 | 19700 |
| Stdev | 29300 | 26000 | 29300 | 32500 | 29300 | 27300 |
| p(t-test) | | 0.21 | | 0.66 | | 0.093 |
| Min | 0.00978 | 240 | 0.00978 | 558 | 0.00978 | 180 |
| Max | 114000 | 83800 | 114000 | 100000 | 114000 | 100000 |
| n (Samp) | 829 | 10 | 829 | 18 | 829 | 16 |
| n (Patient) | 353 | 10 | 353 | 18 | 353 | 16 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22900 | 22300 | 22900 | 19300 | 22900 | 19200 |
| Average | 33700 | 31800 | 33700 | 24900 | 33700 | 24400 |
| Stdev | 29800 | 31100 | 29800 | 24900 | 29800 | 22700 |
| p(t-test) | | 0.62 | | 0.015 | | 0.067 |
| Min | 0.00978 | 55.3 | 0.00978 | 133 | 0.00978 | 53.8 |
| Max | 104000 | 114000 | 104000 | 100000 | 104000 | 81500 |
| n (Samp) | 608 | 66 | 608 | 74 | 608 | 36 |
| n (Patient) | 266 | 66 | 266 | 74 | 266 | 36 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.35 | 0.47 | 0.44 | 0.52 | 0.41 | 0.43 | 0.32 | 0.41 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.037 | 0.095 | 0.038 | 0.035 | 0.070 | 0.036 | 0.048 | 0.075 | 0.051 |
| p | 0.35 | 0.11 | 0.39 | 0.073 | 0.74 | 0.011 | 0.16 | 0.017 | 0.080 |
| nCohort 1 | 599 | 829 | 608 | 599 | 829 | 608 | 599 | 829 | 608 |
| nCohort 2 | 70 | 10 | 66 | 79 | 18 | 74 | 41 | 16 | 36 |
| Cutoff 1 | 8450 | 6690 | 8710 | 9680 | 12500 | 7110 | 7980 | 2440 | 6130 |
| Sens 1 | 70% | 70% | 71% | 71% | 72% | 70% | 71% | 75% | 72% |
| Spec 1 | 23% | 18% | 23% | 25% | 34% | 17% | 22% | 6% | 14% |
| Cutoff 2 | 4980 | 1340 | 4690 | 5480 | 8820 | 5070 | 5200 | 2160 | 5200 |
| Sens 2 | 80% | 80% | 80% | 81% | 83% | 81% | 80% | 81% | 81% |
| Spec 2 | 12% | 3% | 11% | 13% | 25% | 12% | 13% | 6% | 12% |
| Cutoff 3 | 1340 | 512 | 1240 | 1340 | 1540 | 1340 | 2370 | 800 | 1970 |
| Sens 3 | 90% | 90% | 91% | 91% | 94% | 91% | 90% | 94% | 92% |
| Spec 3 | 2% | 2% | 2% | 2% | 3% | 2% | 5% | 2% | 5% |
| Cutoff 4 | 43100 | 39300 | 43400 | 43100 | 39300 | 43400 | 43100 | 39300 | 43400 |
| Sens 4 | 23% | 10% | 24% | 20% | 33% | 18% | 22% | 25% | 19% |
| Spec 4 | 70% | 70% | 70% | 70% | 70% | 70% | 70% | 70% | 70% |
| Cutoff 5 | 60800 | 58100 | 59800 | 60800 | 58100 | 59800 | 60800 | 58100 | 59800 |
| Sens 5 | 17% | 10% | 18% | 10% | 28% | 11% | 15% | 6% | 14% |
| Spec 5 | 80% | 80% | 80% | 80% | 80% | 80% | 80% | 80% | 80% |
| Cutoff 6 | 87200 | 83400 | 87100 | 87200 | 83400 | 87100 | 87200 | 83400 | 87100 |
| Sens 6 | 7% | 10% | 9% | 5% | 17% | 4% | 2% | 6% | 0% |
| Spec 6 | 90% | 90% | 90% | 90% | 90% | 90% | 90% | 90% | 90% |
| OR Quart 2 | 1.5 | 3.0 | 1.1 | 1.2 | 1.2 | 1.4 | 1.3 | 1.0 | 1.2 |
| p Value | 0.28 | 0.34 | 0.84 | 0.58 | 0.74 | 0.44 | 0.63 | 1.00 | 0.79 |
| 95% CI of OR Quart2 | 0.73 | 0.31 | 0.52 | 0.60 | 0.33 | 0.63 | 0.49 | 0.14 | 0.41 |
| | 3.0 | 29 | 2.3 | 2.5 | 4.7 | 2.9 | 3.3 | 7.2 | 3.3 |
| OR Quart 3 | 0.72 | 0 | 1.0 | 1.4 | 1.00 | 1.6 | 1.3 | 1.5 | 1.2 |
| p Value | 0.42 | na | 1.0 | 0.39 | 0.99 | 0.20 | 0.63 | 0.65 | 0.79 |
| 95% CI of OR Quart3 | 0.32 | na | 0.47 | 0.68 | 0.25 | 0.77 | 0.49 | 0.25 | 0.41 |
| | 1.6 | na | 2.1 | 2.7 | 4.0 | 3.4 | 3.3 | 9.2 | 3.3 |
| OR Quart 4 | 1.6 | 6.2 | 1.4 | 1.5 | 1.2 | 2.0 | 1.7 | 4.7 | 1.9 |
| p Value | 0.17 | 0.093 | 0.36 | 0.23 | 0.74 | 0.057 | 0.26 | 0.050 | 0.17 |
| 95% CI of OR Quart4 | 0.82 | 0.74 | 0.68 | 0.77 | 0.33 | 0.98 | 0.68 | 1.00 | 0.75 |
| | 3.2 | 52 | 2.8 | 3.0 | 4.7 | 4.1 | 4.2 | 22 | 5.0 |

**Gastric inhibitory polypeptide Urine**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.369 | 0.426 | 0.369 | 0.386 | 0.369 | 0.464 |
| Average | 29.4 | 10.6 | 29.4 | 21.2 | 29.4 | 1.72 |
| Stdev | 252 | 62.6 | 252 | 119 | 252 | 3.31 |
| p(t-test) | | 0.53 | | 0.77 | | 0.48 |
| Min | 9.60E-5 | 9.60E-5 | 9.60E-5 | 0.000104 | 9.60E-5 | 0.000104 |
| Max | 3840 | 513 | 3840 | 1040 | 3840 | 13.1 |
| n (Samp) | 600 | 70 | 600 | 79 | 600 | 41 |
| n (Patient) | 281 | 70 | 281 | 79 | 281 | 41 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.397 | 0.260 | 0.397 | 0.728 | 0.397 | 0.734 |
| Average | 25.1 | 1.84 | 25.1 | 44.6 | 25.1 | 11.3 |
| Stdev | 219 | 4.60 | 219 | 143 | 219 | 28.7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.74 | | 0.71 | | 0.80 |
| Min | 9.60E-5 | 9.60E-5 | 9.60E-5 | 0.000520 | 9.60E-5 | 0.0460 |
| Max | 3840 | 14.9 | 3840 | 606 | 3840 | 114 |
| n (Samp) | 830 | 10 | 830 | 18 | 830 | 16 |
| n (Patient) | 353 | 10 | 353 | 18 | 353 | 16 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.385 | 0.376 | 0.385 | 0.334 | 0.385 | 0.446 |
| Average | 30.7 | 11.0 | 30.7 | 21.7 | 30.7 | 1.43 |
| Stdev | 251 | 64.4 | 251 | 123 | 251 | 2.57 |
| p(t-test) | | 0.53 | | 0.76 | | 0.48 |
| Min | 9.60E-5 | 0.000104 | 9.60E-5 | 0.000104 | 9.60E-5 | 0.000104 |
| Max | 3840 | 513 | 3840 | 1040 | 3840 | 13.1 |
| n (Samp) | 609 | 66 | 609 | 74 | 609 | 36 |
| n (Patient) | 266 | 66 | 266 | 74 | 266 | 36 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | 0.45 | 0.48 | 0.50 | 0.64 | 0.48 | 0.52 | 0.63 | 0.50 |
| SE | 0.037 | 0.094 | 0.038 | 0.035 | 0.071 | 0.036 | 0.047 | 0.076 | 0.050 |
| p | 0.94 | 0.61 | 0.56 | 0.90 | 0.050 | 0.60 | 0.67 | 0.074 | 0.92 |
| nCohort 1 | 600 | 830 | 609 | 600 | 830 | 609 | 600 | 830 | 609 |
| nCohort 2 | 70 | 10 | 66 | 79 | 18 | 74 | 41 | 16 | 36 |
| Cutoff 1 | 0.221 | 0.186 | 0.221 | 0.143 | 0.348 | 0.143 | 0.142 | 0.254 | 0.142 |
| Sens 1 | 70% | 70% | 71% | 72% | 72% | 70% | 71% | 75% | 72% |
| Spec 1 | 37% | 34% | 36% | 31% | 47% | 30% | 31% | 40% | 30% |
| Cutoff 2 | 0.0766 | 0.0381 | 0.0507 | 0.0484 | 0.259 | 0.0484 | 0.108 | 0.227 | 0.108 |
| Sens 2 | 80% | 80% | 80% | 81% | 83% | 81% | 83% | 81% | 83% |
| Spec 2 | 26% | 21% | 22% | 23% | 41% | 22% | 28% | 38% | 27% |
| Cutoff 3 | 0.000161 | 0.000273 | 0.000104 | 0.000273 | 0.0269 | 0.000273 | 0.00116 | 0.0589 | 0.00104 |
| Sens 3 | 90% | 90% | 92% | 91% | 94% | 91% | 90% | 94% | 92% |
| Spec 3 | 7% | 10% | 4% | 9% | 20% | 10% | 16% | 23% | 15% |
| Cutoff 4 | 0.908 | 0.835 | 0.956 | 0.908 | 0.835 | 0.956 | 0.908 | 0.835 | 0.956 |
| Sens 4 | 21% | 30% | 20% | 27% | 39% | 24% | 29% | 50% | 31% |
| Spec 4 | 70% | 70% | 70% | 70% | 70% | 70% | 70% | 70% | 70% |
| Cutoff 5 | 1.73 | 1.58 | 2.00 | 1.73 | 1.58 | 2.00 | 1.73 | 1.58 | 2.00 |
| Sens 5 | 14% | 10% | 14% | 19% | 33% | 16% | 24% | 31% | 25% |
| Spec 5 | 80% | 80% | 80% | 80% | 80% | 80% | 80% | 80% | 80% |
| Cutoff 6 | 6.24 | 5.24 | 7.60 | 6.24 | 5.24 | 7.60 | 6.24 | 5.24 | 7.60 |
| Sens 6 | 9% | 10% | 8% | 13% | 33% | 11% | 7% | 25% | 3% |
| Spec 6 | 90% | 90% | 90% | 90% | 90% | 90% | 90% | 90% | 90% |
| OR Quart 2 | 2.2 | 3.0 | 2.1 | 1.2 | 2.5 | 1.5 | 1.8 | 1.5 | 1.9 |
| p Value | 0.036 | 0.34 | 0.048 | 0.62 | 0.27 | 0.29 | 0.24 | 0.66 | 0.22 |
| 95% CI of OR Quart2 | 1.1 4.5 | 0.31 29 | 1.0 4.6 | 0.61 2.3 | 0.49 13 | 0.72 2.9 | 0.68 4.6 | 0.25 9.1 | 0.68 5.3 |
| OR Quart 3 | 1.8 | 3.0 | 1.5 | 1.2 | 2.0 | 1.5 | 1.5 | 2.5 | 1.5 |
| p Value | 0.14 | 0.34 | 0.32 | 0.51 | 0.42 | 0.29 | 0.46 | 0.27 | 0.43 |
| 95% CI of OR Quart3 | 0.83 3.7 | 0.31 29 | 0.68 3.3 | 0.64 2.4 | 0.37 11 | 0.72 2.9 | 0.54 3.9 | 0.49 13 | 0.53 4.4 |
| OR Quart 4 | 1.2 | 3.0 | 1.6 | 0.99 | 3.6 | 1.2 | 1.8 | 3.0 | 1.7 |
| p Value | 0.67 | 0.34 | 0.23 | 0.99 | 0.11 | 0.70 | 0.25 | 0.18 | 0.32 |
| 95% CI of | 0.53 | 0.31 | 0.73 | 0.50 | 0.74 | 0.56 | 0.67 | 0.61 | 0.60 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart4 | 2.7 | 29 | 3.6 | 2.0 | 17 | 2.4 | 4.6 | 15 | 4.8 |

**Peptide YY  Urine**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 5.17 | 1.51 | 5.17 | 10.6 | 5.17 | 6.51 |
| Average | 46.8 | 31.6 | 46.8 | 43.9 | 46.8 | 21.8 |
| Stdev | 128 | 70.2 | 128 | 83.5 | 128 | 47.7 |
| p(t-test) |  | 0.33 |  | 0.85 |  | 0.21 |
| Min | 0.00976 | 0.00976 | 0.00976 | 0.00976 | 0.00976 | 0.00976 |
| Max | 1200 | 353 | 1200 | 478 | 1200 | 290 |
| n (Samp) | 598 | 70 | 598 | 79 | 598 | 41 |
| n (Patient) | 281 | 70 | 281 | 79 | 281 | 41 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 4.33 | 4.16 | 4.33 | 48.6 | 4.33 | 21.8 |
| Average | 44.3 | 17.6 | 44.3 | 98.9 | 44.3 | 57.6 |
| Stdev | 117 | 29.9 | 117 | 138 | 117 | 89.4 |
| p(t-test) |  | 0.47 |  | 0.052 |  | 0.65 |
| Min | 0.00976 | 0.00976 | 0.00976 | 0.00976 | 0.00976 | 0.0188 |
| Max | 1200 | 82.2 | 1200 | 554 | 1200 | 353 |
| n (Samp) | 828 | 10 | 828 | 18 | 828 | 16 |
| n (Patient) | 353 | 10 | 353 | 18 | 353 | 16 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 7.35 | 1.51 | 7.35 | 11.0 | 7.35 | 7.18 |
| Average | 47.0 | 31.8 | 47.0 | 40.8 | 47.0 | 23.8 |
| Stdev | 126 | 71.7 | 126 | 80.7 | 126 | 52.5 |
| p(t-test) |  | 0.34 |  | 0.68 |  | 0.27 |
| Min | 0.00976 | 0.00976 | 0.00976 | 0.00976 | 0.00976 | 0.00976 |
| Max | 1200 | 353 | 1200 | 478 | 1200 | 290 |
| n (Samp) | 607 | 66 | 607 | 74 | 607 | 36 |
| n (Patient) | 266 | 66 | 266 | 74 | 266 | 36 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.45 | 0.41 | 0.45 | 0.51 | 0.70 | 0.51 | 0.47 | 0.65 | 0.48 |
| SE | 0.037 | 0.095 | 0.038 | 0.035 | 0.070 | 0.036 | 0.047 | 0.075 | 0.050 |
| p | 0.21 | 0.34 | 0.21 | 0.71 | 0.0053 | 0.84 | 0.55 | 0.053 | 0.63 |
| nCohort 1 | 598 | 828 | 607 | 598 | 828 | 607 | 598 | 828 | 607 |
| nCohort 2 | 70 | 10 | 66 | 79 | 18 | 74 | 41 | 16 | 36 |
| Cutoff 1 | 0.0196 | 0.0151 | 0.0196 | 0.0196 | 16.0 | 0.0196 | 0.0196 | 6.01 | 0.0196 |
| Sens 1 | 71% | 70% | 71% | 72% | 72% | 72% | 78% | 75% | 81% |
| Spec 1 | 23% | 17% | 25% | 23% | 61% | 25% | 23% | 51% | 25% |
| Cutoff 2 | 0.0131 | 0.0128 | 0.0131 | 0.0151 | 0.677 | 0.0151 | 0.0188 | 0.817 | 0.0196 |
| Sens 2 | 81% | 90% | 80% | 82% | 83% | 82% | 80% | 81% | 81% |
| Spec 2 | 13% | 11% | 14% | 15% | 35% | 16% | 18% | 39% | 25% |
| Cutoff 3 | 0.00976 | 0.0128 | 0.00976 | 0.0101 | 0.0151 | 0.0101 | 0.0131 | 0.0151 | 0.0131 |
| Sens 3 | 96% | 90% | 97% | 91% | 94% | 92% | 93% | 100% | 92% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 3% | 11% | 3% | 6% | 17% | 7% | 13% | 17% | 14% |
| Cutoff 4 | 25.8 | 27.7 | 27.9 | 25.8 | 27.7 | 27.9 | 25.8 | 27.7 | 27.9 |
| Sens 4 | 27% | 20% | 26% | 41% | 61% | 35% | 27% | 44% | 28% |
| Spec 4 | 70% | 70% | 70% | 70% | 70% | 70% | 70% | 70% | 70% |
| Cutoff 5 | 44.2 | 44.4 | 48.2 | 44.2 | 44.4 | 48.2 | 44.2 | 44.4 | 48.2 |
| Sens 5 | 20% | 20% | 15% | 25% | 56% | 20% | 12% | 38% | 11% |
| Spec 5 | 80% | 80% | 80% | 80% | 80% | 80% | 80% | 80% | 80% |
| Cutoff 6 | 103 | 104 | 106 | 103 | 104 | 106 | 103 | 104 | 106 |
| Sens 6 | 9% | 0% | 9% | 11% | 33% | 8% | 2% | 12% | 6% |
| Spec 6 | 90% | 90% | 90% | 90% | 90% | 90% | 90% | 90% | 90% |
| OR Quart 2 | 1.2 | 1.5 | 1.4 | 0.62 | 1.00 | 0.69 | 1.8 | 1.0 | 1.9 |
| p Value | 0.58 | 0.65 | 0.44 | 0.17 | 1.00 | 0.29 | 0.19 | 1.0 | 0.22 |
| 95% CI of | 0.59 | 0.25 | 0.63 | 0.31 | 0.14 | 0.34 | 0.74 | 0.14 | 0.68 |
| OR Quart2 | 2.5 | 9.2 | 2.9 | 1.2 | 7.1 | 1.4 | 4.5 | 7.2 | 5.3 |
| OR Quart 3 | 1.1 | 0.50 | 1.4 | 0.80 | 2.0 | 0.95 | 1.0 | 2.5 | 2.1 |
| p Value | 0.71 | 0.57 | 0.44 | 0.51 | 0.42 | 0.87 | 1.0 | 0.27 | 0.15 |
| 95% CI of | 0.55 | 0.045 | 0.63 | 0.42 | 0.37 | 0.49 | 0.37 | 0.49 | 0.76 |
| OR Quart3 | 2.4 | 5.5 | 2.9 | 1.5 | 11 | 1.8 | 2.7 | 13 | 5.7 |
| OR Quart 4 | 1.4 | 2.0 | 1.5 | 0.94 | 5.2 | 0.83 | 1.4 | 3.6 | 1.2 |
| p Value | 0.37 | 0.42 | 0.26 | 0.86 | 0.035 | 0.60 | 0.47 | 0.11 | 0.77 |
| 95% CI of | 0.68 | 0.37 | 0.73 | 0.50 | 1.1 | 0.43 | 0.55 | 0.74 | 0.39 |
| OR Quart4 | 2.8 | 11 | 3.2 | 1.8 | 24 | 1.6 | 3.6 | 17 | 3.6 |

Table 3: Comparison of marker levels in urine samples collected within 12 hours of reaching stage R from Cohort 1 (patients that reached, but did not progress beyond, RIFLE stage R) and from Cohort 2 (patients that reached RIFLE stage I or F).

**Osteocalcin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Mcdian | 7300 | 6390 | 6330 | 10500 | 7370 | 6390 |
| Average | 8940 | 9590 | 9850 | 11400 | 8960 | 8600 |
| Stdev | 7960 | 9540 | 10200 | 8940 | 7390 | 8200 |
| p(t-test) |  | 0.57 |  | 0.56 |  | 0.75 |
| Min | 475 | 473 | 475 | 714 | 1210 | 473 |
| Max | 67900 | 48000 | 52600 | 29500 | 67900 | 42300 |
| n (Samp) | 160 | 85 | 57 | 20 | 138 | 67 |
| n (Patient) | 160 | 85 | 57 | 20 | 138 | 67 |

|  | At Enrollment | | |
|---|---|---|---|
|  | sCr or UO | sCr only | UO only |
| AUC | 0.48 | 0.55 | 0.45 |
| SE | 0.039 | 0.076 | 0.043 |
| p | 0.55 | 0.49 | 0.25 |
| nCohort 1 | 160 | 57 | 138 |
| nCohort 2 | 85 | 20 | 67 |
| Cutoff 1 | 4390 | 6000 | 4540 |
| Sens 1 | 71% | 70% | 70% |
| Spec 1 | 29% | 46% | 29% |
| Cutoff 2 | 3220 | 1810 | 3320 |
| Sens 2 | 80% | 80% | 81% |

|  | At Enrollment |  |  |
|---|---|---|---|
|  | sCr or UO | sCr only | UO only |
| Spec 2 | 17% | 5% | 18% |
| Cutoff 3 | 1810 | 1120 | 2380 |
| Sens 3 | 91% | 90% | 91% |
| Spec 3 | 7% | 4% | 10% |
| Cutoff 4 | 10400 | 12300 | 10500 |
| Sens 4 | 26% | 40% | 18% |
| Spec 4 | 70% | 70% | 70% |
| Cutoff 5 | 12600 | 14500 | 13500 |
| Sens 5 | 20% | 25% | 13% |
| Spec 5 | 80% | 81% | 80% |
| Cutoff 6 | 15900 | 20500 | 17400 |
| Sens 6 | 15% | 25% | 9% |
| Spec 6 | 90% | 91% | 91% |
| OR Quart 2 | 1.2 | 0.33 | 1.8 |
| p Value | 0.65 | 0.22 | 0.17 |
| 95% CI of | 0.56 | 0.055 | 0.77 |
| OR Quart2 | 2.5 | 2.0 | 4.3 |
| OR Quart 3 | 1.1 | 2.0 | 2.0 |
| p Value | 0.80 | 0.31 | 0.12 |
| 95% CI of | 0.52 | 0.52 | 0.84 |
| OR Quart3 | 2.4 | 8.0 | 4.7 |
| OR Quart 4 | 1.6 | 0.93 | 1.8 |
| p Value | 0.23 | 0.93 | 0.17 |
| 95% CI of | 0.75 | 0.22 | 0.77 |
| OR Quart4 | 3.3 | 3.9 | 4.3 |

**Follistatin**

|  | sCr or UO |  | sCr only |  | UO only |  |
|---|---|---|---|---|---|---|
|  | Cohort 1 | Cohort 2 | Cohort 1 | Cohort 2 | Cohort 1 | Cohort 2 |
| Median | 7.30 | 9.61 | 8.08 | 11.5 | 8.01 | 8.94 |
| Average | 17.3 | 18.6 | 24.9 | 21.5 | 15.1 | 18.9 |
| Stdev | 42.6 | 20.8 | 52.4 | 21.2 | 32.3 | 22.0 |
| p(t-test) |  | 0.81 |  | 0.80 |  | 0.41 |
| Min | 0.0425 | 0.0615 | 0.0425 | 2.82 | 0.0432 | 0.0615 |
| Max | 321 | 104 | 321 | 66.2 | 310 | 104 |
| n (Samp) | 137 | 77 | 54 | 17 | 114 | 61 |
| n (Patient) | 137 | 77 | 54 | 17 | 114 | 61 |

|  | At Enrollment |  |  |
|---|---|---|---|
|  | sCr or UO | sCr only | UO only |
| AUC | 0.59 | 0.58 | 0.58 |
| SE | 0.041 | 0.082 | 0.046 |
| p | 0.038 | 0.30 | 0.085 |
| nCohort 1 | 137 | 54 | 114 |
| nCohort 2 | 77 | 17 | 61 |
| Cutoff 1 | 4.89 | 9.06 | 4.89 |
| Sens 1 | 70% | 71% | 70% |
| Spec 1 | 35% | 56% | 36% |
| Cutoff 2 | 3.82 | 3.97 | 3.64 |
| Sens 2 | 81% | 82% | 80% |
| Spec 2 | 29% | 28% | 30% |
| Cutoff 3 | 1.81 | 3.30 | 1.68 |

|  | At Enrollment | | |
|---|---|---|---|
|  | sCr or UO | sCr only | UO only |
| Sens 3 | 91% | 94% | 90% |
| Spec 3 | 19% | 20% | 18% |
| Cutoff 4 | 11.6 | 21.0 | 11.6 |
| Sens 4 | 45% | 29% | 43% |
| Spec 4 | 70% | 70% | 70% |
| Cutoff 5 | 19.6 | 31.4 | 18.7 |
| Sens 5 | 30% | 29% | 33% |
| Spec 5 | 80% | 81% | 81% |
| Cutoff 6 | 33.2 | 47.8 | 40.3 |
| Sens 6 | 21% | 18% | 16% |
| Spec 6 | 91% | 91% | 90% |
| OR Quart 2 | 1.5 | 0.58 | 2.0 |
| p Value | 0.34 | 0.58 | 0.13 |
| 95% CI of | 0.66 | 0.085 | 0.81 |
| OR Quart2 | 3.4 | 4.0 | 5.0 |
| OR Quart 3 | 1.3 | 3.0 | 0.97 |
| p Value | 0.53 | 0.17 | 0.95 |
| 95% CI of | 0.57 | 0.62 | 0.37 |
| OR Quart3 | 3.0 | 14 | 2.6 |
| OR Quart 4 | 2.0 | 1.8 | 2.7 |
| p Value | 0.085 | 0.48 | 0.034 |
| 95% CI of | 0.91 | 0.36 | 1.1 |
| OR Quart4 | 4.5 | 9.1 | 6.6 |

**Islet amyloid polypeptide**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.228 | 0.359 | 0.545 | 1.19 | 0.424 | 0.00758 |
| Average | 3.62 | 1.44 | 5.21 | 5.04 | 2.98 | 0.231 |
| Stdev | 14.7 | 4.78 | 19.3 | 8.99 | 11.0 | 0.330 |
| p(t-test) |  | 0.49 |  | 0.98 |  | 0.32 |
| Min | 0.00151 | 0.00151 | 0.00151 | 0.359 | 0.00151 | 0.00151 |
| Max | 84.9 | 23.2 | 84.9 | 23.2 | 68.1 | 0.880 |
| n (Samp) | 53 | 23 | 19 | 6 | 43 | 16 |
| n (Patient) | 53 | 23 | 19 | 6 | 43 | 16 |

|  | At Enrollment | | |
|---|---|---|---|
|  | sCr or UO | sCr only | UO only |
| AUC | 0.45 | 0.69 | 0.32 |
| SE | 0.073 | 0.13 | 0.083 |
| p | 0.53 | 0.15 | 0.035 |
| nCohort 1 | 53 | 19 | 43 |
| nCohort 2 | 23 | 6 | 16 |
| Cutoff 1 | 0.00353 | 0.359 | 0.00187 |
| Sens 1 | 78% | 83% | 81% |
| Spec 1 | 13% | 47% | 9% |
| Cutoff 2 | 0.00187 | 0.359 | 0.00187 |
| Sens 2 | 91% | 83% | 81% |
| Spec 2 | 9% | 47% | 9% |
| Cutoff 3 | 0.00187 | 0.0802 | 0.00151 |
| Sens 3 | 91% | 100% | 94% |
| Spec 3 | 9% | 47% | 5% |

|  | At Enrollment | | |
|---|---|---|---|
|  | sCr or UO | sCr only | UO only |
| Cutoff 4 | 1.16 | 1.52 | 1.16 |
| Sens 4 | 13% | 50% | 0% |
| Spec 4 | 75% | 74% | 77% |
| Cutoff 5 | 1.50 | 2.03 | 1.50 |
| Sens 5 | 13% | 33% | 0% |
| Spec 5 | 81% | 84% | 81% |
| Cutoff 6 | 2.31 | 2.49 | 3.12 |
| Sens 6 | 9% | 33% | 0% |
| Spec 6 | 91% | 95% | 91% |
| OR Quart 2 | 4.8 | >3.0 | 7.0 |
| p Value | 0.044 | <0.43 | 0.097 |
| 95% CI of | 1.0 | >0.20 | 0.71 |
| OR Quart2 | 22 | na | 69 |
| OR Quart 3 | 1.4 | >3.0 | 5.1 |
| p Value | 0.68 | <0.43 | 0.17 |
| 95% CI of | 0.27 | >0.20 | 0.50 |
| OR Quart3 | 7.4 | na | 52 |
| OR Quart 4 | 3.1 | >2.4 | 10 |
| p Value | 0.15 | <0.52 | 0.044 |
| 95% CI of | 0.66 | >0.16 | 1.1 |
| OR Quart4 | 15 | na | 100 |

**Involucrin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.602 | 0.686 | 0.619 | 0.696 | 0.639 | 0.743 |
| Average | 1.36 | 1.70 | 1.40 | 2.17 | 1.34 | 1.63 |
| Stdev | 2.69 | 2.71 | 2.75 | 3.82 | 2.36 | 2.23 |
| p(t-test) |  | 0.37 |  | 0.33 |  | 0.41 |
| Min | 0.0226 | 0.0181 | 0.0226 | 0.0244 | 0.0326 | 0.0181 |
| Max | 19.2 | 17.0 | 19.2 | 17.0 | 15.5 | 15.1 |
| n (Samp) | 144 | 80 | 55 | 21 | 123 | 62 |
| n (Patient) | 144 | 80 | 55 | 21 | 123 | 62 |

|  | At Enrollment | | |
|---|---|---|---|
|  | sCr or UO | sCr only | UO only |
| AUC | 0.58 | 0.54 | 0.58 |
| SE | 0.040 | 0.075 | 0.045 |
| p | 0.044 | 0.64 | 0.066 |
| nCohort 1 | 144 | 55 | 123 |
| nCohort 2 | 80 | 21 | 62 |
| Cutoff 1 | 0.438 | 0.353 | 0.438 |
| Sens 1 | 70% | 71% | 71% |
| Spec 1 | 42% | 33% | 38% |
| Cutoff 2 | 0.297 | 0.228 | 0.360 |
| Sens 2 | 80% | 81% | 81% |
| Spec 2 | 33% | 20% | 32% |
| Cutoff 3 | 0.189 | 0.156 | 0.191 |
| Sens 3 | 90% | 90% | 90% |
| Spec 3 | 19% | 15% | 18% |
| Cutoff 4 | 0.945 | 1.17 | 1.11 |
| Sens 4 | 44% | 33% | 44% |

|  | At Enrollment | | |
|---|---|---|---|
|  | sCr or UO | sCr only | UO only |
| Spec 4 | 70% | 71% | 71% |
| Cutoff 5 | 1.55 | 1.81 | 1.56 |
| Sens 5 | 35% | 29% | 39% |
| Spec 5 | 81% | 80% | 80% |
| Cutoff 6 | 2.58 | 3.64 | 2.48 |
| Sens 6 | 20% | 19% | 23% |
| Spec 6 | 90% | 91% | 90% |
| OR Quart 2 | 1.5 | 0.58 | 3.2 |
| p Value | 0.32 | 0.46 | 0.016 |
| 95% CI of | 0.68 | 0.13 | 1.2 |
| OR Quart2 | 3.3 | 2.5 | 8.0 |
| OR Quart 3 | 0.91 | 0.77 | 1.1 |
| p Value | 0.83 | 0.72 | 0.80 |
| 95% CI of | 0.40 | 0.19 | 0.42 |
| OR Quart3 | 2.1 | 3.2 | 3.1 |
| OR Quart 4 | 2.5 | 1.0 | 3.9 |
| p Value | 0.022 | 1.0 | 0.0037 |
| 95% CI of | 1.1 | 0.25 | 1.6 |
| OR Quart4 | 5.5 | 3.9 | 9.9 |

**Collagenase 3**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 3.82 | 1.55 | 3.33 | 0.202 | 4.58 | 6.79 |
| Average | 12.5 | 9.78 | 8.05 | 4.46 | 14.3 | 10.9 |
| Stdev | 24.4 | 15.6 | 12.8 | 13.1 | 27.1 | 13.1 |
| p(t-test) |  | 0.46 |  | 0.42 |  | 0.43 |
| Min | 0.00479 | 0.00479 | 0.00479 | 0.00479 | 0.00479 | 0.00479 |
| Max | 182 | 80.1 | 48.1 | 45.8 | 182 | 52.2 |
| n (Samp) | 86 | 57 | 32 | 12 | 73 | 44 |
| n (Patient) | 86 | 57 | 32 | 12 | 73 | 44 |

|  | At Enrollment | | |
|---|---|---|---|
|  | sCr or UO | sCr only | UO only |
| AUC | 0.45 | 0.31 | 0.50 |
| SE | 0.050 | 0.095 | 0.055 |
| p | 0.31 | 0.042 | 0.99 |
| nCohort 1 | 86 | 32 | 73 |
| nCohort 2 | 57 | 12 | 44 |
| Cutoff 1 | 0.0466 | 0.00623 | 0.202 |
| Sens 1 | 74% | 75% | 73% |
| Spec 1 | 19% | 3% | 26% |
| Cutoff 2 | 0.00623 | 0.00588 | 0.0198 |
| Sens 2 | 86% | 83% | 84% |
| Spec 2 | 8% | 3% | 14% |
| Cutoff 3 | 0.00519 | 0.00479 | 0.00519 |
| Sens 3 | 93% | 92% | 95% |
| Spec 3 | 5% | 3% | 7% |
| Cutoff 4 | 14.2 | 6.71 | 17.4 |
| Sens 4 | 23% | 8% | 25% |
| Spec 4 | 71% | 72% | 71% |
| Cutoff 5 | 20.3 | 15.2 | 21.9 |

|  | At Enrollment | | |
|---|---|---|---|
|  | sCr or UO | sCr only | UO only |
| Sens 5 | 18% | 8% | 20% |
| Spec 5 | 80% | 81% | 81% |
| Cutoff 6 | 30.4 | 21.2 | 30.4 |
| Sens 6 | 9% | 8% | 9% |
| Spec 6 | 91% | 91% | 90% |
| OR Quart 2 | 1.1 | 2.2 | 0.54 |
| p Value | 0.81 | 0.54 | 0.27 |
| 95% CI of | 0.43 | 0.17 | 0.18 |
| OR Quart2 | 2.9 | 29 | 1.6 |
| OR Quart 3 | 0.78 | 8.3 | 1.2 |
| p Value | 0.62 | 0.080 | 0.79 |
| 95% CI of | 0.29 | 0.78 | 0.41 |
| OR Quart3 | 2.1 | 89 | 3.3 |
| OR Quart 4 | 2.1 | 5.7 | 0.82 |
| p Value | 0.13 | 0.15 | 0.71 |
| 95% CI of | 0.81 | 0.52 | 0.29 |
| OR Quart4 | 5.4 | 63 | 2.3 |

**Pancreatic prohormone**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 8.76 | 1.15 | 1.15 | 132 | 8.76 | 7.38 |
| Average | 110 | 61.8 | 71.3 | 182 | 115 | 45.8 |
| Stdev | 227 | 132 | 132 | 207 | 241 | 81.2 |
| p(t-test) |  | 0.34 |  | 0.13 |  | 0.27 |
| Min | 0.122 | 0.117 | 0.122 | 1.15 | 0.122 | 0.117 |
| Max | 1220 | 567 | 396 | 567 | 1220 | 310 |
| n (Samp) | 53 | 23 | 19 | 6 | 43 | 16 |
| n (Patient) | 53 | 23 | 19 | 6 | 43 | 16 |

|  | At Enrollment | | |
|---|---|---|---|
|  | sCr or UO | sCr only | UO only |
| AUC | 0.44 | 0.76 | 0.45 |
| SE | 0.073 | 0.12 | 0.086 |
| p | 0.42 | 0.034 | 0.54 |
| nCohort 1 | 53 | 19 | 43 |
| nCohort 2 | 23 | 6 | 16 |
| Cutoff 1 | 0.134 | 6.59 | 0.134 |
| Sens 1 | 78% | 83% | 75% |
| Spec 1 | 25% | 63% | 21% |
| Cutoff 2 | 0.122 | 6.59 | 0.122 |
| Sens 2 | 87% | 83% | 81% |
| Spec 2 | 6% | 63% | 5% |
| Cutoff 3 | 0.117 | 0.293 | 0 |
| Sens 3 | 91% | 100% | 100% |
| Spec 3 | 0% | 47% | 0% |
| Cutoff 4 | 40.4 | 36.4 | 40.4 |
| Sens 4 | 30% | 67% | 38% |
| Spec 4 | 72% | 74% | 72% |
| Cutoff 5 | 200 | 200 | 183 |
| Sens 5 | 9% | 33% | 6% |
| Spec 5 | 81% | 84% | 81% |

| | At Enrollment | | |
|---|---|---|---|
| | sCr or UO | sCr only | UO only |
| Cutoff 6 | 396 | 338 | 434 |
| Sens 6 | 4% | 17% | 0% |
| Spec 6 | 91% | 95% | 91% |
| OR Quart 2 | 0.58 | >1.2 | 1.0 |
| p Value | 0.46 | <0.91 | 1.0 |
| 95% CI of | 0.13 | >0.059 | 0.20 |
| OR Quart2 | 2.5 | na | 5.0 |
| OR Quart 3 | 1.3 | >3.0 | 0.69 |
| p Value | 0.73 | <0.43 | 0.67 |
| 95% CI of | 0.33 | >0.20 | 0.12 |
| OR Quart3 | 4.8 | na | 3.8 |
| OR Quart 4 | 1.0 | >4.5 | 1.5 |
| p Value | 1.0 | <0.26 | 0.60 |
| 95% CI of | 0.25 | >0.34 | 0.31 |
| OR Quart4 | 3.9 | na | 7.4 |

**Transthyretin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 23.4 | 42.0 | 26.4 | 40.0 | 26.0 | 52.7 |
| Average | 54.7 | 80.7 | 49.5 | 129 | 53.1 | 94.8 |
| Stdev | 86.1 | 120 | 65.3 | 266 | 86.0 | 132 |
| p(t-test) | | 0.094 | | 0.078 | | 0.021 |
| Min | 0.430 | 2.55 | 1.04 | 5.86 | 0.430 | 2.55 |
| Max | 648 | 677 | 355 | 997 | 648 | 677 |
| n (Samp) | 113 | 67 | 42 | 13 | 96 | 53 |
| n (Patient) | 113 | 67 | 42 | 13 | 96 | 53 |

| | At Enrollment | | |
|---|---|---|---|
| | sCr or UO | sCr only | UO only |
| AUC | 0.61 | 0.59 | 0.66 |
| SE | 0.044 | 0.093 | 0.048 |
| p | 0.010 | 0.33 | 6.0E-4 |
| nCohort 1 | 113 | 42 | 96 |
| nCohort 2 | 67 | 13 | 53 |
| Cutoff 1 | 26.3 | 26.9 | 27.3 |
| Sens 1 | 70% | 77% | 72% |
| Spec 1 | 52% | 52% | 52% |
| Cutoff 2 | 16.7 | 16.1 | 22.6 |
| Sens 2 | 81% | 85% | 81% |
| Spec 2 | 36% | 31% | 48% |
| Cutoff 3 | 8.43 | 6.53 | 11.7 |
| Sens 3 | 91% | 92% | 91% |
| Spec 3 | 23% | 14% | 27% |
| Cutoff 4 | 52.9 | 51.2 | 52.2 |
| Sens 4 | 39% | 31% | 51% |
| Spec 4 | 71% | 71% | 71% |
| Cutoff 5 | 75.4 | 64.2 | 64.8 |
| Sens 5 | 27% | 31% | 36% |
| Spec 5 | 81% | 81% | 80% |
| Cutoff 6 | 119 | 98.6 | 131 |
| Sens 6 | 16% | 31% | 15% |

|  | At Enrollment | | |
|---|---|---|---|
|  | sCr or UO | sCr only | UO only |
| Spec 6 | 90% | 90% | 91% |
| OR Quart 2 | 1.5 | 1.5 | 1.4 |
| p Value | 0.35 | 0.69 | 0.57 |
| 95% CI of | 0.62 | 0.21 | 0.45 |
| OR Quart2 | 3.9 | 11 | 4.2 |
| OR Quart 3 | 3.0 | 2.2 | 4.1 |
| p Value | 0.018 | 0.42 | 0.0086 |
| 95% CI of | 1.2 | 0.33 | 1.4 |
| OR Quart3 | 7.2 | 15 | 12 |
| OR Quart 4 | 2.3 | 2.2 | 4.3 |
| p Value | 0.076 | 0.42 | 0.0061 |
| 95% CI of | 0.92 | 0.33 | 1.5 |
| OR Quart4 | 5.6 | 15 | 12 |

**C-peptide Urine**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 19300 | 17200 | 14900 | 6130 | 24100 | 17200 |
| Average | 29000 | 24100 | 25200 | 17000 | 32400 | 23800 |
| Stdev | 26300 | 23400 | 25500 | 27800 | 26800 | 23900 |
| p(t-test) |  | 0.21 |  | 0.33 |  | 0.052 |
| Min | 0.0955 | 180 | 0.0614 | 558 | 0.0955 | 180 |
| Max | 100000 | 100000 | 100000 | 94700 | 100000 | 100000 |
| n (Samp) | 113 | 68 | 42 | 13 | 96 | 54 |
| n (Patient) | 113 | 68 | 42 | 13 | 96 | 54 |

|  | At Enrollment | | |
|---|---|---|---|
|  | sCr or UO | sCr only | UO only |
| AUC | 0.45 | 0.32 | 0.40 |
| SE | 0.045 | 0.090 | 0.049 |
| p | 0.22 | 0.042 | 0.032 |
| nCohort 1 | 113 | 42 | 96 |
| nCohort 2 | 68 | 13 | 54 |
| Cutoff 1 | 8450 | 940 | 8070 |
| Sens 1 | 71% | 77% | 70% |
| Spec 1 | 26% | 2% | 18% |
| Cutoff 2 | 6130 | 936 | 5600 |
| Sens 2 | 81% | 85% | 81% |
| Spec 2 | 14% | 2% | 11% |
| Cutoff 3 | 1110 | 558 | 1340 |
| Sens 3 | 91% | 92% | 91% |
| Spec 3 | 2% | 2% | 3% |
| Cutoff 4 | 38000 | 27100 | 42400 |
| Sens 4 | 19% | 15% | 13% |
| Spec 4 | 71% | 71% | 71% |
| Cutoff 5 | 48200 | 43100 | 55400 |
| Sens 5 | 16% | 15% | 11% |
| Spec 5 | 81% | 81% | 80% |
| Cutoff 6 | 70200 | 62400 | 71300 |
| Sens 6 | 4% | 8% | 6% |
| Spec 6 | 90% | 90% | 91% |
| OR Quart 2 | 2.0 | 1.0 | 2.9 |

| | At Enrollment | | |
|---|---|---|---|
| | sCr or UO | sCr only | UO only |
| p Value | 0.11 | 1.0 | 0.043 |
| 95% CI of OR Quart2 | 0.85 | 0.12 | 1.0 |
| | 4.8 | 8.3 | 7.9 |
| OR Quart 3 | 1.5 | 1.6 | 1.7 |
| p Value | 0.34 | 0.62 | 0.30 |
| 95% CI of OR Quart3 | 0.64 | 0.23 | 0.61 |
| | 3.7 | 12 | 4.9 |
| OR Quart 4 | 1.7 | 5.1 | 3.6 |
| p Value | 0.24 | 0.083 | 0.014 |
| 95% CI of OR Quart4 | 0.70 | 0.81 | 1.3 |
| | 4.1 | 33 | 9.8 |

**Peptide YY Urine**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 10.7 | 3.92 | 22.6 | 49.5 | 8.01 | 0.837 |
| Average | 50.6 | 29.4 | 56.2 | 78.0 | 49.4 | 17.3 |
| Stdev | 126 | 66.7 | 111 | 83.2 | 130 | 27.7 |
| p(t-test) | | 0.20 | | 0.52 | | 0.076 |
| Min | 0.00976 | 0.00976 | 0.0101 | 0.0196 | 0.00976 | 0.00976 |
| Max | 762 | 478 | 502 | 278 | 762 | 113 |
| n (Samp) | 113 | 68 | 42 | 13 | 96 | 54 |
| n (Patient) | 113 | 68 | 42 | 13 | 96 | 54 |

| | At Enrollment | | |
|---|---|---|---|
| | sCr or UO | sCr only | UO only |
| AUC | 0.44 | 0.66 | 0.42 |
| SE | 0.044 | 0.091 | 0.049 |
| p | 0.16 | 0.078 | 0.11 |
| nCohort 1 | 113 | 42 | 96 |
| nCohort 2 | 68 | 13 | 54 |
| Cutoff 1 | 0.0188 | 16.6 | 0.0188 |
| Sens 1 | 72% | 77% | 72% |
| Spec 1 | 17% | 45% | 18% |
| Cutoff 2 | 0.0131 | 10.3 | 0.0131 |
| Sens 2 | 85% | 85% | 83% |
| Spec 2 | 9% | 45% | 10% |
| Cutoff 3 | 0.0101 | 2.77 | 0.0101 |
| Sens 3 | 93% | 92% | 91% |
| Spec 3 | 4% | 40% | 5% |
| Cutoff 4 | 28.2 | 36.9 | 26.8 |
| Sens 4 | 31% | 62% | 26% |
| Spec 4 | 71% | 71% | 71% |
| Cutoff 5 | 50.0 | 60.1 | 35.6 |
| Sens 5 | 16% | 38% | 19% |
| Spec 5 | 81% | 81% | 80% |
| Cutoff 6 | 110 | 157 | 95.4 |
| Sens 6 | 6% | 15% | 2% |
| Spec 6 | 90% | 90% | 91% |
| OR Quart 2 | 0.85 | 4.8 | 1.0 |
| p Value | 0.71 | 0.19 | 0.94 |
| 95% CI of | 0.35 | 0.46 | 0.39 |

|  | At Enrollment |  |  |
|---|---|---|---|
|  | sCr or UO | sCr only | UO only |
| OR Quart2 | 2.0 | 50 | 2.7 |
| OR Quart 3 | 0.76 | 3.3 | 1.1 |
| p Value | 0.55 | 0.33 | 0.81 |
| 95% CI of | 0.31 | 0.29 | 0.43 |
| OR Quart3 | 1.8 | 36 | 2.9 |
| OR Quart 4 | 2.3 | 6.7 | 1.8 |
| p Value | 0.048 | 0.11 | 0.20 |
| 95% CI of | 1.0 | 0.66 | 0.72 |
| OR Quart4 | 5.5 | 67 | 4.7 |

Table 4: Comparison of the maximum marker levels in urine samples collected from Cohort 1 (patients that did not progress beyond RIFLE stage 0) and the maximum values in urine samples collected from subjects between enrollment and 0, 24 hours, and 48 hours prior to reaching stage F in Cohort 2.

**Agouti-related protein**

| sCr or UO | 0hr prior to AKI stage |  | 24hr prior to AKI stage |  | 48hr prior to AKI stage |  |
|---|---|---|---|---|---|---|
|  | Cohort 1 | Cohort 2 | Cohort 1 | Cohort 2 | Cohort 1 | Cohort 2 |
| Median | 1.34 | 1.80 | 1.34 | 1.78 | 1.34 | 1.63 |
| Average | 1.94 | 2.28 | 1.94 | 2.28 | 1.94 | 2.06 |
| Stdev | 2.71 | 1.77 | 2.71 | 1.77 | 2.71 | 1.45 |
| p(t-test) |  | 0.54 |  | 0.55 |  | 0.87 |
| Min | 0.0195 | 0.213 | 0.0195 | 0.213 | 0.0195 | 0.496 |
| Max | 20.0 | 6.53 | 20.0 | 6.53 | 20.0 | 5.30 |
| n (Samp) | 109 | 26 | 109 | 25 | 109 | 14 |
| n (Patient) | 109 | 26 | 109 | 25 | 109 | 14 |

| sCr only | 0hr prior to AKI stage |  | 24hr prior to AKI stage |  | 48hr prior to AKI stage |  |
|---|---|---|---|---|---|---|
|  | Cohort 1 | Cohort 2 | Cohort 1 | Cohort 2 | Cohort 1 | Cohort 2 |
| Median | 1.39 | 2.35 | 1.39 | 2.35 | 1.39 | 2.35 |
| Average | 2.36 | 2.52 | 2.36 | 2.48 | 2.36 | 2.38 |
| Stdev | 3.00 | 1.96 | 3.00 | 1.92 | 3.00 | 1.55 |
| p(t-test) |  | 0.84 |  | 0.89 |  | 0.99 |
| Min | 0.0195 | 0.213 | 0.0195 | 0.213 | 0.0195 | 0.496 |
| Max | 20.0 | 6.53 | 20.0 | 6.53 | 20.0 | 5.30 |
| n (Samp) | 229 | 14 | 229 | 14 | 229 | 10 |
| n (Patient) | 229 | 14 | 229 | 14 | 229 | 10 |

| UO only | 0hr prior to AKI stage |  | 24hr prior to AKI stage |  | 48hr prior to AKI stage |  |
|---|---|---|---|---|---|---|
|  | Cohort 1 | Cohort 2 | Cohort 1 | Cohort 2 | Cohort 1 | Cohort 2 |
| Median | 1.28 | 1.30 | 1.28 | 1.44 | 1.28 | 0.962 |
| Average | 1.80 | 1.86 | 1.80 | 1.88 | 1.80 | 1.37 |
| Stdev | 2.52 | 1.47 | 2.52 | 1.51 | 2.52 | 1.05 |
| p(t-test) |  | 0.92 |  | 0.90 |  | 0.64 |
| Min | 0.0195 | 0.496 | 0.0195 | 0.496 | 0.0195 | 0.496 |
| Max | 20.0 | 6.12 | 20.0 | 6.12 | 20.0 | 3.44 |
| n (Samp) | 124 | 16 | 124 | 15 | 124 | 8 |
| n (Patient) | 124 | 16 | 124 | 15 | 124 | 8 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.60 | 0.56 | 0.56 | 0.60 | 0.56 | 0.56 | 0.58 | 0.58 | 0.45 |
| SE | 0.064 | 0.082 | 0.079 | 0.065 | 0.082 | 0.081 | 0.084 | 0.096 | 0.11 |
| p | 0.13 | 0.45 | 0.43 | 0.14 | 0.47 | 0.45 | 0.34 | 0.40 | 0.66 |
| nCohort 1 | 109 | 229 | 124 | 109 | 229 | 124 | 109 | 229 | 124 |
| nCohort 2 | 26 | 14 | 16 | 25 | 14 | 15 | 14 | 10 | 8 |
| Cutoff 1 | 0.965 | 0.935 | 0.965 | 0.965 | 0.935 | 0.965 | 0.888 | 1.08 | 0.755 |
| Sens 1 | 73% | 71% | 75% | 72% | 71% | 73% | 71% | 70% | 75% |
| Spec 1 | 35% | 29% | 37% | 35% | 29% | 37% | 32% | 36% | 28% |
| Cutoff 2 | 0.855 | 0.600 | 0.880 | 0.855 | 0.600 | 0.880 | 0.755 | 0.935 | 0.672 |
| Sens 2 | 81% | 86% | 81% | 80% | 86% | 80% | 86% | 80% | 88% |
| Spec 2 | 32% | 19% | 34% | 32% | 19% | 34% | 27% | 29% | 27% |
| Cutoff 3 | 0.587 | 0.478 | 0.672 | 0.587 | 0.478 | 0.672 | 0.678 | 0.885 | 0.478 |
| Sens 3 | 92% | 93% | 94% | 92% | 93% | 93% | 93% | 90% | 100% |
| Spec 3 | 24% | 15% | 27% | 24% | 15% | 27% | 26% | 28% | 22% |
| Cutoff 4 | 1.93 | 2.58 | 1.82 | 1.93 | 2.58 | 1.82 | 1.93 | 2.58 | 1.82 |
| Sens 4 | 46% | 43% | 38% | 44% | 43% | 33% | 50% | 40% | 25% |
| Spec 4 | 71% | 70% | 70% | 71% | 70% | 70% | 71% | 70% | 70% |
| Cutoff 5 | 2.63 | 3.33 | 2.58 | 2.63 | 3.33 | 2.58 | 2.63 | 3.33 | 2.58 |
| Sens 5 | 31% | 36% | 19% | 32% | 36% | 20% | 29% | 30% | 12% |
| Spec 5 | 81% | 80% | 81% | 81% | 80% | 81% | 81% | 80% | 81% |
| Cutoff 6 | 3.36 | 4.50 | 3.28 | 3.36 | 4.50 | 3.28 | 3.36 | 4.50 | 3.28 |
| Sens 6 | 27% | 14% | 19% | 28% | 14% | 20% | 21% | 10% | 12% |
| Spec 6 | 91% | 90% | 90% | 91% | 90% | 90% | 91% | 90% | 90% |
| OR Quart 2 | 1.9 | 0.98 | 3.4 | 1.6 | 0.98 | 6.8 | 2.7 | 3.1 | 0 |
| p Value | 0.35 | 0.98 | 0.15 | 0.53 | 0.98 | 0.083 | 0.26 | 0.34 | na |
| 95% CI of | 0.49 | 0.19 | 0.64 | 0.40 | 0.19 | 0.78 | 0.48 | 0.31 | na |
| OR Quart2 | 7.1 | 5.1 | 18 | 6.1 | 5.1 | 60 | 15 | 30 | na |
| OR Quart 3 | 1.2 | 0.64 | 1.5 | 1.3 | 0.64 | 4.3 | 0.47 | 2.0 | 2.8 |
| p Value | 0.76 | 0.64 | 0.64 | 0.72 | 0.64 | 0.21 | 0.54 | 0.58 | 0.25 |
| 95% CI of | 0.30 | 0.10 | 0.24 | 0.31 | 0.10 | 0.45 | 0.040 | 0.18 | 0.50 |
| OR Quart3 | 5.1 | 4.0 | 9.9 | 5.3 | 4.0 | 40 | 5.4 | 23 | 15 |
| OR Quart 4 | 3.0 | 2.1 | 2.8 | 3.0 | 2.1 | 4.3 | 3.4 | 4.1 | 0.48 |
| p Value | 0.090 | 0.32 | 0.25 | 0.090 | 0.32 | 0.21 | 0.16 | 0.21 | 0.56 |
| 95% CI of | 0.84 | 0.49 | 0.50 | 0.84 | 0.49 | 0.45 | 0.62 | 0.45 | 0.042 |
| OR Quart4 | 11 | 8.7 | 15 | 11 | 8.7 | 40 | 18 | 38 | 5.6 |

**Osteocalcin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 6840 | 8560 | 6840 | 8230 | 6840 | 8260 |
| Average | 7910 | 11600 | 7910 | 10100 | 7910 | 9960 |
| Stdev | 7360 | 9240 | 7360 | 8680 | 7360 | 6940 |
| p(t-test) |  | 0.0054 |  | 0.097 |  | 0.20 |
| Min | 146 | 1080 | 146 | 1080 | 146 | 2630 |
| Max | 83000 | 49400 | 83000 | 49400 | 83000 | 29500 |
| n (Samp) | 191 | 42 | 191 | 40 | 191 | 24 |
| n (Patient) | 191 | 42 | 191 | 40 | 191 | 24 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 7620 | 10400 | 7620 | 9740 | 7620 | 9960 |
| Average | 9420 | 12000 | 9420 | 10400 | 9420 | 12100 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 8790 | 8520 | 8790 | 7100 | 8790 | 7940 |
| p(t-test) | | 0.20 | | 0.63 | | 0.28 |
| Min | 146 | 1080 | 146 | 1080 | 146 | 4220 |
| Max | 83500 | 29500 | 83500 | 29500 | 83500 | 29500 |
| n (Samp) | 386 | 20 | 386 | 20 | 386 | 13 |
| n (Patient) | 386 | 20 | 386 | 20 | 386 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 7010 | 7530 | 7010 | 6640 | 7010 | 5340 |
| Average | 8660 | 10700 | 8660 | 9600 | 8660 | 7990 |
| Stdev | 8580 | 9770 | 8580 | 9820 | 8580 | 6660 |
| p(t-test) | | 0.25 | | 0.60 | | 0.74 |
| Min | 146 | 2240 | 146 | 2240 | 146 | 2630 |
| Max | 83000 | 49400 | 83000 | 49400 | 83000 | 29500 |
| n (Samp) | 202 | 29 | 202 | 27 | 202 | 18 |
| n (Patient) | 202 | 29 | 202 | 27 | 202 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | 0.60 | 0.57 | 0.60 | 0.57 | 0.52 | 0.61 | 0.63 | 0.47 |
| SE | 0.050 | 0.068 | 0.059 | 0.051 | 0.068 | 0.060 | 0.064 | 0.084 | 0.072 |
| p | 0.0051 | 0.16 | 0.25 | 0.060 | 0.34 | 0.77 | 0.094 | 0.13 | 0.70 |
| nCohort 1 | 191 | 386 | 202 | 191 | 386 | 202 | 191 | 386 | 202 |
| nCohort 2 | 42 | 20 | 29 | 40 | 20 | 27 | 24 | 13 | 18 |
| Cutoff 1 | 5800 | 5920 | 5040 | 5500 | 5920 | 4620 | 5500 | 5920 | 4440 |
| Sens 1 | 71% | 70% | 72% | 70% | 70% | 70% | 71% | 77% | 72% |
| Spec 1 | 40% | 33% | 35% | 39% | 33% | 33% | 39% | 33% | 30% |
| Cutoff 2 | 4620 | 5130 | 4440 | 4540 | 5130 | 4190 | 4440 | 5500 | 3390 |
| Sens 2 | 81% | 80% | 83% | 80% | 80% | 81% | 83% | 85% | 83% |
| Spec 2 | 35% | 28% | 30% | 34% | 28% | 27% | 32% | 30% | 19% |
| Cutoff 3 | 4210 | 4210 | 2760 | 3800 | 4210 | 2760 | 3390 | 4990 | 2760 |
| Sens 3 | 90% | 90% | 93% | 90% | 90% | 93% | 92% | 92% | 94% |
| Spec 3 | 29% | 21% | 13% | 25% | 21% | 13% | 21% | 27% | 13% |
| Cutoff 4 | 8850 | 10400 | 9010 | 8850 | 10400 | 9010 | 8850 | 10400 | 9010 |
| Sens 4 | 48% | 50% | 38% | 45% | 45% | 33% | 46% | 46% | 28% |
| Spec 4 | 70% | 70% | 70% | 70% | 70% | 70% | 70% | 70% | 70% |
| Cutoff 5 | 10400 | 12300 | 11200 | 10400 | 12300 | 11200 | 10400 | 12300 | 11200 |
| Sens 5 | 40% | 35% | 28% | 32% | 25% | 19% | 33% | 38% | 17% |
| Spec 5 | 80% | 80% | 80% | 80% | 80% | 80% | 80% | 80% | 80% |
| Cutoff 6 | 14100 | 17200 | 15900 | 14100 | 17200 | 15900 | 14100 | 17200 | 15900 |
| Sens 6 | 31% | 20% | 21% | 18% | 10% | 11% | 21% | 15% | 11% |
| Spec 6 | 90% | 90% | 90% | 90% | 90% | 90% | 90% | 90% | 90% |
| OR Quart 2 | 4.8 | 1.7 | 3.8 | 2.4 | 1.7 | 2.0 | 2.1 | 3.0 | 1.0 |
| p Value | 0.021 | 0.48 | 0.055 | 0.12 | 0.48 | 0.26 | 0.32 | 0.34 | 1.0 |
| 95% CI of OR Quart2 | 1.3 | 0.39 | 0.97 | 0.79 | 0.39 | 0.61 | 0.49 | 0.31 | 0.24 |
| | 18 | 7.2 | 14 | 7.5 | 7.2 | 6.2 | 8.8 | 30 | 4.2 |
| OR Quart 3 | 3.4 | 1.3 | 2.5 | 2.2 | 2.1 | 1.2 | 2.1 | 3.0 | 1.6 |
| p Value | 0.081 | 0.70 | 0.21 | 0.18 | 0.32 | 0.75 | 0.32 | 0.34 | 0.51 |
| 95% CI of OR Quart3 | 0.86 | 0.29 | 0.61 | 0.69 | 0.50 | 0.35 | 0.49 | 0.31 | 0.42 |
| | 13 | 6.2 | 10 | 6.8 | 8.5 | 4.3 | 8.8 | 30 | 5.9 |
| OR Quart 4 | 8.0 | 2.8 | 3.3 | 3.3 | 2.0 | 1.4 | 3.3 | 6.3 | 1.0 |
| p Value | 0.0015 | 0.14 | 0.085 | 0.033 | 0.32 | 0.56 | 0.084 | 0.092 | 1.0 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of | 2.2 | 0.72 | 0.85 | 1.1 | 0.50 | 0.43 | 0.85 | 0.74 | 0.24 |
| OR Quart4 | 29 | 11 | 13 | 9.9 | 8.4 | 4.8 | 13 | 53 | 4.2 |

**Complement factor H**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 12.3 | 10.4 | 12.3 | 11.0 | 12.3 | 18.5 |
| Average | 23.3 | 26.3 | 23.3 | 26.9 | 23.3 | 25.5 |
| Stdev | 34.1 | 35.4 | 34.1 | 36.0 | 34.1 | 25.2 |
| p(t-test) |  | 0.67 |  | 0.62 |  | 0.80 |
| Min | 0.132 | 2.76 | 0.132 | 2.52 | 0.132 | 2.83 |
| Max | 282 | 142 | 282 | 142 | 282 | 99.4 |
| n (Samp) | 151 | 28 | 151 | 27 | 151 | 16 |
| n (Patient) | 151 | 28 | 151 | 27 | 151 | 16 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 13.7 | 9.74 | 13.7 | 9.74 | 13.7 | 20.2 |
| Average | 25.3 | 29.2 | 25.3 | 29.0 | 25.3 | 29.2 |
| Stdev | 35.7 | 39.9 | 35.7 | 40.1 | 35.7 | 29.7 |
| p(t-test) |  | 0.68 |  | 0.70 |  | 0.72 |
| Min | 0.0956 | 2.76 | 0.0956 | 2.76 | 0.0956 | 2.52 |
| Max | 282 | 142 | 282 | 142 | 282 | 99.4 |
| n (Samp) | 290 | 15 | 290 | 15 | 290 | 11 |
| n (Patient) | 290 | 15 | 290 | 15 | 290 | 11 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 14.1 | 9.35 | 14.1 | 9.74 | 14.1 | 10.4 |
| Average | 29.3 | 19.5 | 29.3 | 20.3 | 29.3 | 13.9 |
| Stdev | 68.9 | 27.0 | 68.9 | 27.6 | 68.9 | 9.50 |
| p(t-test) |  | 0.55 |  | 0.60 |  | 0.48 |
| Min | 0.132 | 2.76 | 0.132 | 2.52 | 0.132 | 2.83 |
| Max | 772 | 118 | 772 | 118 | 772 | 29.8 |
| n (Samp) | 155 | 18 | 155 | 17 | 155 | 10 |
| n (Patient) | 155 | 18 | 155 | 17 | 155 | 10 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.51 | 0.49 | 0.43 | 0.52 | 0.48 | 0.44 | 0.57 | 0.56 | 0.42 |
| SE | 0.060 | 0.077 | 0.074 | 0.061 | 0.077 | 0.076 | 0.078 | 0.091 | 0.097 |
| p | 0.86 | 0.95 | 0.33 | 0.80 | 0.84 | 0.43 | 0.35 | 0.54 | 0.43 |
| nCohort 1 | 151 | 290 | 155 | 151 | 290 | 155 | 151 | 290 | 155 |
| nCohort 2 | 28 | 15 | 18 | 27 | 15 | 17 | 16 | 11 | 10 |
| Cutoff 1 | 7.66 | 7.96 | 6.43 | 7.66 | 7.34 | 7.34 | 9.47 | 9.47 | 8.52 |
| Sens 1 | 71% | 73% | 72% | 70% | 73% | 71% | 75% | 73% | 70% |
| Spec 1 | 30% | 29% | 21% | 30% | 27% | 25% | 39% | 38% | 30% |
| Cutoff 2 | 6.14 | 7.34 | 6.00 | 6.03 | 6.03 | 6.00 | 8.52 | 7.34 | 7.34 |
| Sens 2 | 82% | 80% | 83% | 81% | 80% | 82% | 81% | 82% | 80% |
| Spec 2 | 25% | 27% | 19% | 25% | 20% | 19% | 36% | 27% | 25% |
| Cutoff 3 | 3.56 | 3.56 | 2.81 | 3.56 | 3.56 | 2.81 | 3.56 | 3.56 | 3.56 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 93% | 93% | 94% | 93% | 93% | 94% | 94% | 91% | 90% |
| Spec 3 | 13% | 10% | 8% | 13% | 10% | 8% | 13% | 10% | 10% |
| Cutoff 4 | 23.9 | 24.3 | 24.8 | 23.9 | 24.3 | 24.8 | 23.9 | 24.3 | 24.8 |
| Sens 4 | 32% | 33% | 28% | 33% | 33% | 29% | 38% | 45% | 20% |
| Spec 4 | 70% | 70% | 70% | 70% | 70% | 70% | 70% | 70% | 70% |
| Cutoff 5 | 29.8 | 34.9 | 31.5 | 29.8 | 34.9 | 31.5 | 29.8 | 34.9 | 31.5 |
| Sens 5 | 29% | 20% | 17% | 30% | 20% | 18% | 31% | 27% | 0% |
| Spec 5 | 80% | 80% | 80% | 80% | 80% | 80% | 80% | 80% | 80% |
| Cutoff 6 | 52.5 | 51.2 | 60.5 | 52.5 | 51.2 | 60.5 | 52.5 | 51.2 | 60.5 |
| Sens 6 | 11% | 13% | 6% | 11% | 13% | 6% | 12% | 18% | 0% |
| Spec 6 | 90% | 90% | 90% | 90% | 90% | 90% | 90% | 90% | 90% |
| OR Quart 2 | 1.6 | 0.19 | 0.75 | 1.4 | 0.19 | 0.73 | 2.6 | 1.5 | 3.2 |
| p Value | 0.43 | 0.14 | 0.72 | 0.59 | 0.14 | 0.69 | 0.26 | 0.65 | 0.32 |
| 95% CI of | 0.51 | 0.022 | 0.16 | 0.43 | 0.022 | 0.15 | 0.48 | 0.25 | 0.32 |
| OR Quart2 | 4.9 | 1.7 | 3.6 | 4.3 | 1.7 | 3.5 | 14 | 9.4 | 32 |
| OR Quart 3 | 0.79 | 1.2 | 1.3 | 0.81 | 1.0 | 1.3 | 2.1 | 0.49 | 4.4 |
| p Value | 0.72 | 0.74 | 0.70 | 0.75 | 0.98 | 0.73 | 0.42 | 0.57 | 0.19 |
| 95% CI of | 0.22 | 0.36 | 0.33 | 0.23 | 0.28 | 0.32 | 0.35 | 0.044 | 0.47 |
| OR Quart3 | 2.8 | 4.2 | 5.3 | 2.9 | 3.7 | 5.1 | 12 | 5.6 | 41 |
| OR Quart 4 | 1.4 | 0.59 | 1.6 | 1.4 | 0.80 | 1.3 | 2.6 | 2.6 | 2.1 |
| p Value | 0.59 | 0.48 | 0.48 | 0.59 | 0.75 | 0.73 | 0.26 | 0.27 | 0.55 |
| 95% CI of | 0.43 | 0.14 | 0.42 | 0.43 | 0.21 | 0.32 | 0.48 | 0.48 | 0.18 |
| OR Quart4 | 4.3 | 2.6 | 6.2 | 4.3 | 3.1 | 5.1 | 14 | 14 | 24 |

**Follistatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.97 | 27.7 | 9.97 | 23.1 | 9.97 | 27.7 |
| Average | 70.8 | 60.1 | 70.8 | 58.6 | 70.8 | 34.0 |
| Stdev | 543 | 137 | 543 | 139 | 543 | 24.7 |
| p(t-test) | | 0.91 | | 0.90 | | 0.76 |
| Min | 0.0191 | 3.84 | 0.0191 | 3.84 | 0.0191 | 1.87 |
| Max | 8290 | 840 | 8290 | 840 | 8290 | 98.2 |
| n (Samp) | 242 | 36 | 242 | 35 | 242 | 20 |
| n (Patient) | 242 | 36 | 242 | 35 | 242 | 20 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 11.8 | 28.3 | 11.8 | 23.1 | 11.8 | 25.1 |
| Average | 55.5 | 37.6 | 55.5 | 33.2 | 55.5 | 31.7 |
| Stdev | 413 | 32.3 | 413 | 27.0 | 413 | 27.7 |
| p(t-test) | | 0.85 | | 0.81 | | 0.83 |
| Min | 0.0191 | 3.84 | 0.0191 | 3.84 | 0.0191 | 1.87 |
| Max | 8290 | 125 | 8290 | 98.2 | 8290 | 98.2 |
| n (Samp) | 423 | 19 | 423 | 19 | 423 | 14 |
| n (Patient) | 423 | 19 | 423 | 19 | 423 | 14 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 11.0 | 37.5 | 11.0 | 26.9 | 11.0 | 27.1 |
| Average | 79.5 | 152 | 79.5 | 125 | 79.5 | 35.6 |
| Stdev | 589 | 420 | 589 | 300 | 589 | 21.5 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.55 | | 0.71 | | 0.79 |
| Min | 0.0191 | 4.96 | 0.0191 | 4.96 | 0.0191 | 4.79 |
| Max | 8290 | 2010 | 8290 | 1300 | 8290 | 69.9 |
| n (Samp) | 205 | 25 | 205 | 24 | 205 | 13 |
| n (Patient) | 205 | 25 | 205 | 24 | 205 | 13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.72 | 0.68 | 0.75 | 0.71 | 0.66 | 0.74 | 0.69 | 0.62 | 0.72 |
| SE | 0.050 | 0.069 | 0.059 | 0.052 | 0.070 | 0.060 | 0.068 | 0.081 | 0.082 |
| p | 8.8E-6 | 0.010 | 2.3E-5 | 5.6E-5 | 0.025 | 9.6E-5 | 0.0048 | 0.14 | 0.0066 |
| nCohort 1 | 242 | 423 | 205 | 242 | 423 | 205 | 242 | 423 | 205 |
| nCohort 2 | 36 | 19 | 25 | 35 | 19 | 24 | 20 | 14 | 13 |
| Cutoff 1 | 18.6 | 18.6 | 19.9 | 15.0 | 15.2 | 19.9 | 20.4 | 16.8 | 21.4 |
| Sens 1 | 72% | 74% | 72% | 71% | 74% | 71% | 70% | 71% | 77% |
| Spec 1 | 67% | 63% | 67% | 64% | 59% | 67% | 70% | 61% | 69% |
| Cutoff 2 | 14.5 | 12.0 | 14.4 | 14.5 | 12.0 | 12.1 | 18.6 | 4.69 | 19.9 |
| Sens 2 | 81% | 84% | 80% | 80% | 84% | 83% | 80% | 86% | 85% |
| Spec 2 | 63% | 51% | 59% | 63% | 51% | 53% | 67% | 21% | 67% |
| Cutoff 3 | 9.57 | 4.09 | 9.34 | 9.57 | 3.96 | 9.34 | 4.53 | 3.79 | 8.95 |
| Sens 3 | 92% | 95% | 92% | 91% | 95% | 92% | 90% | 93% | 92% |
| Spec 3 | 49% | 18% | 45% | 49% | 17% | 45% | 25% | 16% | 43% |
| Cutoff 4 | 21.8 | 23.9 | 23.7 | 21.8 | 23.9 | 23.7 | 21.8 | 23.9 | 23.7 |
| Sens 4 | 61% | 53% | 60% | 54% | 47% | 54% | 65% | 50% | 62% |
| Spec 4 | 70% | 70% | 70% | 70% | 70% | 70% | 70% | 70% | 70% |
| Cutoff 5 | 33.9 | 39.0 | 34.9 | 33.9 | 39.0 | 34.9 | 33.9 | 39.0 | 34.9 |
| Sens 5 | 42% | 32% | 52% | 34% | 32% | 46% | 30% | 29% | 38% |
| Spec 5 | 80% | 80% | 80% | 80% | 80% | 80% | 80% | 80% | 80% |
| Cutoff 6 | 68.2 | 71.8 | 64.8 | 68.2 | 71.8 | 64.8 | 68.2 | 71.8 | 64.8 |
| Sens 6 | 22% | 11% | 32% | 20% | 11% | 29% | 10% | 7% | 15% |
| Spec 6 | 90% | 90% | 90% | 90% | 90% | 90% | 90% | 90% | 90% |
| OR Quart 2 | 0.65 | 0.99 | 4.1 | 0.66 | 0.99 | 4.2 | 0 | 0 | 0.98 |
| p Value | 0.64 | 0.99 | 0.21 | 0.65 | 0.99 | 0.20 | na | na | 0.99 |
| 95% CI of | 0.10 | 0.14 | 0.45 | 0.11 | 0.14 | 0.46 | na | na | 0.060 |
| OR Quart2 | 4.0 | 7.2 | 38 | 4.1 | 7.2 | 39 | na | na | 16 |
| OR Quart 3 | 6.6 | 4.2 | 7.8 | 7.2 | 4.8 | 9.1 | 3.3 | 2.4 | 5.4 |
| p Value | 0.0039 | 0.072 | 0.058 | 0.0025 | 0.048 | 0.040 | 0.083 | 0.21 | 0.13 |
| 95% CI of | 1.8 | 0.88 | 0.93 | 2.0 | 1.0 | 1.1 | 0.86 | 0.61 | 0.61 |
| OR Quart3 | 24 | 20 | 66 | 26 | 23 | 76 | 13 | 9.6 | 48 |
| OR Quart 4 | 6.0 | 3.6 | 16 | 5.0 | 3.1 | 13 | 2.9 | 1.3 | 6.5 |
| p Value | 0.0065 | 0.11 | 0.0084 | 0.015 | 0.17 | 0.015 | 0.14 | 0.71 | 0.089 |
| 95% CI of | 1.7 | 0.74 | 2.0 | 1.4 | 0.61 | 1.6 | 0.72 | 0.29 | 0.75 |
| OR Quart4 | 22 | 18 | 130 | 18 | 16 | 110 | 11 | 6.1 | 56 |

**Vitamin D-binding protein**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 218 | 4340 | 218 | 1900 | 218 | 5870 |
| Average | 9990 | 55000 | 9990 | 46300 | 9990 | 80600 |
| Stdev | 46300 | 113000 | 46300 | 110000 | 46300 | 147000 |
| p(t-test) | | 0.0029 | | 0.015 | | 5.0E-4 |
| Min | 1.61 | 138 | 1.61 | 95.4 | 1.61 | 1.09 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 352000 | 401000 | 352000 | 401000 | 352000 | 401000 |
| n (Samp) | 97 | 24 | 97 | 23 | 97 | 12 |
| n (Patient) | 97 | 24 | 97 | 23 | 97 | 12 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 344 | 11700 | 344 | 10400 | 344 | 11700 |
| Average | 12800 | 80800 | 12800 | 78200 | 12800 | 107000 |
| Stdev | 46000 | 139000 | 46000 | 141000 | 46000 | 162000 |
| p(t-test) | | 2.5E-5 | | 5.1E-5 | | 8.9E-7 |
| Min | 1.61 | 223 | 1.61 | 95.4 | 1.61 | 1.09 |
| Max | 365000 | 401000 | 365000 | 401000 | 365000 | 401000 |
| n (Samp) | 210 | 13 | 210 | 13 | 210 | 9 |
| n (Patient) | 210 | 13 | 210 | 13 | 210 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 245 | 2710 | 245 | 1640 | 245 | 1270 |
| Average | 12400 | 45500 | 12400 | 32900 | 12400 | 59300 |
| Stdev | 47400 | 113000 | 47400 | 106000 | 47400 | 151000 |
| p(t-test) | | 0.043 | | 0.20 | | 0.039 |
| Min | 6.72 | 138 | 6.72 | 138 | 6.72 | 190 |
| Max | 352000 | 401000 | 352000 | 401000 | 352000 | 401000 |
| n (Samp) | 109 | 15 | 109 | 14 | 109 | 7 |
| n (Patient) | 109 | 15 | 109 | 14 | 109 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.80 | 0.80 | 0.72 | 0.76 | 0.74 | 0.71 | 0.74 | 0.73 | 0.70 |
| SE | 0.057 | 0.075 | 0.078 | 0.062 | 0.080 | 0.081 | 0.085 | 0.097 | 0.11 |
| p | 1.3E-7 | 4.8E-5 | 0.0039 | 2.4E-5 | 0.0023 | 0.012 | 0.0040 | 0.016 | 0.076 |
| nCohort 1 | 97 | 210 | 109 | 97 | 210 | 109 | 97 | 210 | 109 |
| nCohort 2 | 24 | 13 | 15 | 23 | 13 | 14 | 12 | 9 | 7 |
| Cutoff 1 | 1080 | 1150 | 967 | 954 | 968 | 967 | 464 | 1150 | 464 |
| Sens 1 | 71% | 77% | 73% | 74% | 77% | 71% | 75% | 78% | 71% |
| Spec 1 | 76% | 70% | 72% | 74% | 68% | 72% | 68% | 70% | 62% |
| Cutoff 2 | 528 | 968 | 464 | 464 | 528 | 190 | 274 | 218 | 274 |
| Sens 2 | 83% | 85% | 80% | 83% | 85% | 86% | 83% | 89% | 86% |
| Spec 2 | 69% | 68% | 62% | 68% | 61% | 39% | 57% | 37% | 54% |
| Cutoff 3 | 218 | 528 | 186 | 186 | 218 | 186 | 218 | 0 | 186 |
| Sens 3 | 92% | 92% | 93% | 91% | 92% | 93% | 92% | 100% | 100% |
| Spec 3 | 51% | 61% | 39% | 44% | 37% | 39% | 51% | 0% | 39% |
| Cutoff 4 | 715 | 1500 | 954 | 715 | 1500 | 954 | 715 | 1500 | 954 |
| Sens 4 | 79% | 62% | 73% | 74% | 54% | 71% | 67% | 67% | 57% |
| Spec 4 | 70% | 70% | 71% | 70% | 70% | 71% | 70% | 70% | 71% |
| Cutoff 5 | 3200 | 5060 | 4570 | 3200 | 5060 | 4570 | 3200 | 5060 | 4570 |
| Sens 5 | 50% | 62% | 40% | 43% | 54% | 36% | 50% | 67% | 29% |
| Spec 5 | 80% | 80% | 81% | 80% | 80% | 81% | 80% | 80% | 81% |
| Cutoff 6 | 13000 | 29700 | 29300 | 13000 | 29700 | 29300 | 13000 | 29700 | 29300 |
| Sens 6 | 29% | 38% | 13% | 22% | 31% | 7% | 25% | 33% | 14% |
| Spec 6 | 91% | 90% | 91% | 91% | 90% | 91% | 91% | 90% | 91% |
| OR Quart 2 | >3.3 | >1.0 | >3.3 | 3.2 | 0.98 | >3.2 | 1.0 | 0.98 | >1.0 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | <0.31 | <1.0 | <0.31 | 0.32 | 0.99 | <0.32 | 1.0 | 0.99 | <0.98 |
| 95% CI of | >0.33 | >0.061 | >0.33 | 0.32 | 0.060 | >0.32 | 0.059 | 0.060 | >0.062 |
| OR Quart2 | na | na | na | 33 | 16 | na | 17 | 16 | na |
| OR Quart 3 | >13 | >4.2 | >6.0 | 12 | 4.2 | >5.8 | 4.5 | 0.98 | >4.6 |
| p Value | <0.019 | <0.20 | <0.11 | 0.021 | 0.21 | <0.12 | 0.19 | 0.99 | <0.18 |
| 95% CI of | >1.5 | >0.46 | >0.65 | 1.5 | 0.45 | >0.63 | 0.47 | 0.060 | >0.49 |
| OR Quart3 | na | na | na | 110 | 38 | na | 43 | 16 | na |
| OR Quart 4 | >19 | >9.2 | >9.0 | 14 | 7.7 | >7.2 | 7.1 | 6.5 | >2.1 |
| p Value | <0.0065 | <0.040 | <0.046 | 0.014 | 0.060 | <0.076 | 0.080 | 0.089 | <0.54 |
| 95% CI of | >2.3 | >1.1 | >1.0 | 1.7 | 0.92 | >0.81 | 0.79 | 0.75 | >0.18 |
| OR Quart4 | na | na | na | 120 | 65 | na | 63 | 56 | na |

**Glucagon**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 238 | 9330 | 238 | 9330 | 238 | 9330 |
| Average | 1810 | 5710 | 1810 | 5510 | 1810 | 6750 |
| Stdev | 3130 | 4510 | 3130 | 4720 | 3130 | 4410 |
| p(t-test) | | 1.8E-4 | | 4.1E-4 | | 1.5E-4 |
| Min | 2.40 | 45.3 | 2.40 | 31.5 | 2.40 | 238 |
| Max | 9330 | 9330 | 9330 | 9330 | 9330 | 9330 |
| n (Samp) | 98 | 12 | 98 | 12 | 98 | 7 |
| n (Patient) | 98 | 12 | 98 | 12 | 98 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 347 | 9330 | 347 | 9330 | nd | nd |
| Average | 2050 | 6650 | 2050 | 6260 | nd | nd |
| Stdev | 3360 | 4210 | 3360 | 4770 | nd | nd |
| p(t-test) | | 0.0013 | | 0.0034 | nd | nd |
| Min | 1.98 | 174 | 1.98 | 31.5 | nd | nd |
| Max | 9330 | 9330 | 9330 | 9330 | nd | nd |
| n (Samp) | 159 | 6 | 159 | 6 | nd | nd |
| n (Patient) | 159 | 6 | 159 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 238 | 9330 | 238 | 9330 | 238 | 9330 |
| Average | 1570 | 5910 | 1570 | 5910 | 1570 | 6320 |
| Stdev | 2760 | 4720 | 2760 | 4720 | 2760 | 4670 |
| p(t-test) | | 1.5E-4 | | 1.5E-4 | | 2.1E-4 |
| Min | 2.40 | 45.3 | 2.40 | 45.3 | 2.40 | 238 |
| Max | 9330 | 9330 | 9330 | 9330 | 9330 | 9330 |
| n (Samp) | 84 | 8 | 84 | 8 | 84 | 6 |
| n (Patient) | 84 | 8 | 84 | 8 | 84 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.76 | 0.79 | 0.77 | 0.72 | 0.70 | 0.77 | 0.82 | nd | 0.81 |
| SE | 0.084 | 0.11 | 0.10 | 0.087 | 0.12 | 0.10 | 0.099 | nd | 0.11 |
| p | 0.0020 | 0.011 | 0.0082 | 0.011 | 0.100 | 0.0082 | 0.0011 | nd | 0.0044 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 98 | 159 | 84 | 98 | 159 | 84 | 98 | nd | 84 |
| nCohort 2 | 12 | 6 | 8 | 12 | 6 | 8 | 7 | nd | 6 |
| Cutoff 1 | 275 | 677 | 238 | 174 | 98.8 | 238 | 4790 | nd | 238 |
| Sens 1 | 75% | 83% | 75% | 75% | 83% | 75% | 71% | nd | 83% |
| Spec 1 | 56% | 69% | 55% | 37% | 28% | 55% | 87% | nd | 55% |
| Cutoff 2 | 174 | 677 | 98.8 | 98.8 | 98.8 | 98.8 | 275 | nd | 238 |
| Sens 2 | 83% | 83% | 88% | 83% | 83% | 88% | 86% | nd | 83% |
| Spec 2 | 37% | 69% | 33% | 37% | 28% | 33% | 56% | nd | 55% |
| Cutoff 3 | 98.8 | 98.8 | 41.7 | 41.7 | 16.2 | 41.7 | 98.8 | nd | 98.8 |
| Sens 3 | 92% | 100% | 100% | 92% | 100% | 100% | 100% | nd | 100% |
| Spec 3 | 37% | 28% | 32% | 35% | 25% | 32% | 37% | nd | 33% |
| Cutoff 4 | 2420 | 2420 | 2420 | 2420 | 2420 | 2420 | 2420 | nd | 2420 |
| Sens 4 | 58% | 67% | 62% | 58% | 67% | 62% | 71% | nd | 67% |
| Spec 4 | 85% | 82% | 88% | 85% | 82% | 88% | 85% | nd | 88% |
| Cutoff 5 | 2420 | 2420 | 2420 | 2420 | 2420 | 2420 | 2420 | nd | 2420 |
| Sens 5 | 58% | 67% | 62% | 58% | 67% | 62% | 71% | nd | 67% |
| Spec 5 | 85% | 82% | 88% | 85% | 82% | 88% | 85% | nd | 88% |
| Cutoff 6 | 9330 | 9330 | 4790 | 9330 | 9330 | 4790 | 9330 | nd | 4790 |
| Sens 6 | 0% | 0% | 62% | 0% | 0% | 62% | 0% | nd | 67% |
| Spec 6 | 100% | 100% | 90% | 100% | 100% | 90% | 100% | nd | 90% |
| OR Quart 2 | >3.2 | >1.0 | >2.2 | >4.5 | 1.0 | >2.2 | >1.0 | nd | >1.0 |
| p Value | <0.32 | <0.99 | <0.53 | <0.19 | 1.0 | <0.53 | <0.98 | nd | <1.0 |
| 95% CI of | >0.32 | >0.062 | >0.18 | >0.47 | 0.060 | >0.18 | >0.062 | nd | >0.059 |
| OR Quart2 | na | na | na | na | 17 | na | na | nd | na |
| OR Quart 3 | >1.0 | >1.0 | >1.0 | >1.0 | 0 | >1.0 | >1.0 | nd | >1.0 |
| p Value | <0.98 | <0.99 | <0.98 | <0.98 | na | <0.98 | <0.98 | nd | <0.97 |
| 95% CI of | >0.062 | >0.062 | >0.062 | >0.062 | na | >0.062 | >0.062 | nd | >0.061 |
| OR Quart3 | na | na | na | na | na | na | na | nd | na |
| OR Quart 4 | >11 | >4.3 | >6.4 | >9.0 | 4.2 | >6.4 | >5.9 | nd | >4.6 |
| p Value | <0.031 | <0.20 | <0.10 | <0.047 | 0.21 | <0.10 | <0.12 | nd | <0.19 |
| 95% CI of | >1.2 | >0.46 | >0.68 | >1.0 | 0.45 | >0.68 | >0.64 | nd | >0.48 |
| OR Quart4 | na | na | na | na | 39 | na | na | nd | na |

**Glucagon-like peptide 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.53 | 7.46 | 5.53 | 7.46 | 5.53 | 9.60 |
| Average | 10.3 | 16.1 | 10.3 | 16.1 | 10.3 | 10.2 |
| Stdev | 48.3 | 29.4 | 48.3 | 29.4 | 48.3 | 7.43 |
| p(t-test) | | 0.68 | | 0.68 | | 1.00 |
| Min | 0.407 | 0.685 | 0.407 | 0.685 | 0.407 | 0.685 |
| Max | 482 | 107 | 482 | 107 | 482 | 24.9 |
| n (Samp) | 98 | 12 | 98 | 12 | 98 | 7 |
| n (Patient) | 98 | 12 | 98 | 12 | 98 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.53 | 4.31 | 5.53 | 4.31 | nd | nd |
| Average | 13.8 | 4.83 | 13.8 | 4.83 | nd | nd |
| Stdev | 57.7 | 4.04 | 57.7 | 4.04 | nd | nd |
| p(t-test) | | 0.71 | | 0.71 | nd | nd |
| Min | 0.407 | 0.685 | 0.407 | 0.685 | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 519 | 9.60 | 519 | 9.60 | nd | nd |
| n (Samp) | 159 | 6 | 159 | 6 | nd | nd |
| n (Patient) | 159 | 6 | 159 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.53 | 9.50 | 5.53 | 9.50 | 5.53 | 9.50 |
| Average | 11.2 | 21.8 | 11.2 | 21.8 | 11.2 | 10.3 |
| Stdev | 52.2 | 35.2 | 52.2 | 35.2 | 52.2 | 8.14 |
| p(t-test) | | 0.57 | | 0.57 | | 0.97 |
| Min | 0.407 | 0.685 | 0.407 | 0.685 | 0.407 | 0.685 |
| Max | 482 | 107 | 482 | 107 | 482 | 24.9 |
| n (Samp) | 84 | 8 | 84 | 8 | 84 | 6 |
| n (Patient) | 84 | 8 | 84 | 8 | 84 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.43 | 0.70 | 0.63 | 0.43 | 0.70 | 0.72 | nd | 0.68 |
| SE | 0.091 | 0.12 | 0.11 | 0.091 | 0.12 | 0.11 | 0.11 | nd | 0.12 |
| p | 0.15 | 0.57 | 0.061 | 0.15 | 0.57 | 0.061 | 0.054 | nd | 0.15 |
| nCohort 1 | 98 | 159 | 84 | 98 | 159 | 84 | 98 | nd | 84 |
| nCohort 2 | 12 | 6 | 8 | 12 | 6 | 8 | 7 | nd | 6 |
| Cutoff 1 | 5.14 | 0.407 | 5.14 | 5.14 | 0.407 | 5.14 | 8.07 | nd | 5.14 |
| Sens 1 | 75% | 100% | 88% | 75% | 100% | 88% | 71% | nd | 83% |
| Spec 1 | 44% | 8% | 46% | 44% | 8% | 46% | 72% | nd | 46% |
| Cutoff 2 | 2.30 | 0.407 | 5.14 | 2.30 | 0.407 | 5.14 | 5.14 | nd | 5.14 |
| Sens 2 | 83% | 100% | 88% | 83% | 100% | 88% | 86% | nd | 83% |
| Spec 2 | 28% | 8% | 46% | 28% | 8% | 46% | 44% | nd | 46% |
| Cutoff 3 | 0.407 | 0.407 | 0.407 | 0.407 | 0.407 | 0.407 | 0.407 | nd | 0.407 |
| Sens 3 | 100% | 100% | 100% | 100% | 100% | 100% | 100% | nd | 100% |
| Spec 3 | 9% | 8% | 8% | 9% | 8% | 8% | 9% | nd | 8% |
| Cutoff 4 | 6.54 | 9.39 | 9.60 | 6.54 | 9.39 | 9.60 | 6.54 | nd | 9.60 |
| Sens 4 | 50% | 17% | 38% | 50% | 17% | 38% | 71% | nd | 33% |
| Spec 4 | 71% | 70% | 90% | 71% | 70% | 90% | 71% | nd | 90% |
| Cutoff 5 | 9.60 | 9.60 | 9.60 | 9.60 | 9.60 | 9.60 | 9.60 | nd | 9.60 |
| Sens 5 | 25% | 0% | 38% | 25% | 0% | 38% | 29% | nd | 33% |
| Spec 5 | 93% | 91% | 90% | 93% | 91% | 90% | 93% | nd | 90% |
| Cutoff 6 | 9.60 | 9.60 | 9.60 | 9.60 | 9.60 | 9.60 | 9.60 | nd | 9.60 |
| Sens 6 | 25% | 0% | 38% | 25% | 0% | 38% | 29% | nd | 33% |
| Spec 6 | 93% | 91% | 90% | 93% | 91% | 90% | 93% | nd | 90% |
| OR Quart 2 | 0.46 | >3.3 | 0 | 0.46 | >3.3 | 0 | 1.0 | nd | 0.95 |
| p Value | 0.54 | <0.31 | na | 0.54 | <0.31 | na | 1.0 | nd | 0.97 |
| 95% CI of OR Quart2 | 0.039 | >0.33 | na | 0.039 | >0.33 | na | 0.059 | nd | 0.056 |
| | 5.4 | na | na | 5.4 | na | na | 17 | nd | 16 |
| OR Quart 3 | 2.8 | >1.0 | 4.6 | 2.8 | >1.0 | 4.6 | 2.1 | nd | 2.1 |
| p Value | 0.24 | <0.97 | 0.19 | 0.24 | <0.97 | 0.19 | 0.56 | nd | 0.56 |
| 95% CI of OR Quart3 | 0.50 | >0.064 | 0.48 | 0.50 | >0.064 | 0.48 | 0.18 | nd | 0.18 |
| | 16 | na | 45 | 16 | na | 45 | 25 | nd | 25 |
| OR Quart 4 | 2.1 | >2.2 | 3.3 | 2.1 | >2.2 | 3.3 | 3.1 | nd | 2.0 |
| p Value | 0.42 | <0.54 | 0.32 | 0.42 | <0.54 | 0.32 | 0.34 | nd | 0.58 |
| 95% CI of OR Quart4 | 0.35 | >0.19 | 0.32 | 0.35 | >0.19 | 0.32 | 0.30 | nd | 0.17 |
| | 12 | na | 34 | 12 | na | 34 | 32 | nd | 24 |

**Appetite-regulating hormone**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.560 | 0.560 | 0.560 | 0.560 | 0.560 | 0.108 |
| Average | 1.04 | 6.38 | 1.04 | 6.25 | 1.04 | 1.09 |
| Stdev | 1.42 | 18.7 | 1.42 | 18.7 | 1.42 | 1.81 |
| p(t-test) | | 0.0052 | | 0.0064 | | 0.93 |
| Min | 0.0793 | 0.0793 | 0.0793 | 0.0793 | 0.0793 | 0.0793 |
| Max | 8.07 | 65.5 | 8.07 | 65.5 | 8.07 | 4.96 |
| n (Samp) | 98 | 12 | 98 | 12 | 98 | 7 |
| n (Patient) | 98 | 12 | 98 | 12 | 98 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.560 | 0.560 | 0.560 | 0.396 | nd | nd |
| Average | 1.55 | 0.597 | 1.55 | 0.345 | nd | nd |
| Stdev | 5.30 | 0.610 | 5.30 | 0.242 | nd | nd |
| p(t-test) | | 0.66 | | 0.58 | nd | nd |
| Min | 0.0793 | 0.0793 | 0.0793 | 0.0793 | nd | nd |
| Max | 65.5 | 1.75 | 65.5 | 0.560 | nd | nd |
| n (Samp) | 159 | 6 | 159 | 6 | nd | nd |
| n (Patient) | 159 | 6 | 159 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.560 | 0.560 | 0.560 | 0.560 | 0.560 | 0.334 |
| Average | 1.15 | 9.20 | 1.15 | 9.20 | 1.15 | 1.25 |
| Stdev | 1.51 | 22.8 | 1.51 | 22.8 | 1.51 | 1.92 |
| p(t-test) | | 0.0012 | | 0.0012 | | 0.87 |
| Min | 0.0793 | 0.0793 | 0.0793 | 0.0793 | 0.0793 | 0.0793 |
| Max | 8.07 | 65.5 | 8.07 | 65.5 | 8.07 | 4.96 |
| n (Samp) | 84 | 8 | 84 | 8 | 84 | 6 |
| n (Patient) | 84 | 8 | 84 | 8 | 84 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.52 | 0.40 | 0.51 | 0.48 | 0.35 | 0.51 | 0.41 | nd | 0.42 |
| SE | 0.089 | 0.12 | 0.11 | 0.089 | 0.12 | 0.11 | 0.12 | nd | 0.13 |
| p | 0.86 | 0.44 | 0.89 | 0.85 | 0.21 | 0.89 | 0.45 | nd | 0.54 |
| nCohort 1 | 98 | 159 | 84 | 98 | 159 | 84 | 98 | nd | 84 |
| nCohort 2 | 12 | 6 | 8 | 12 | 6 | 8 | 7 | nd | 6 |
| Cutoff 1 | 0.0995 | 0 | 0.0995 | 0.0995 | 0 | 0.0995 | 0.0995 | nd | 0 |
| Sens 1 | 75% | 100% | 75% | 75% | 100% | 75% | 71% | nd | 100% |
| Spec 1 | 16% | 0% | 13% | 16% | 0% | 13% | 16% | nd | 0% |
| Cutoff 2 | 0 | 0 | 0 | 0 | 0 | 0 | 0 | nd | 0 |
| Sens 2 | 100% | 100% | 100% | 100% | 100% | 100% | 100% | nd | 100% |
| Spec 2 | 0% | 0% | 0% | 0% | 0% | 0% | 0% | nd | 0% |
| Cutoff 3 | 0 | 0 | 0 | 0 | 0 | 0 | 0 | nd | 0 |
| Sens 3 | 100% | 100% | 100% | 100% | 100% | 100% | 100% | nd | 100% |
| Spec 3 | 0% | 0% | 0% | 0% | 0% | 0% | 0% | nd | 0% |
| Cutoff 4 | 0.560 | 1.75 | 1.75 | 0.560 | 1.75 | 1.75 | 0.560 | nd | 1.75 |
| Sens 4 | 33% | 0% | 25% | 25% | 0% | 25% | 29% | nd | 17% |
| Spec 4 | 70% | 76% | 74% | 70% | 76% | 74% | 70% | nd | 74% |
| Cutoff 5 | 2.40 | 2.40 | 2.67 | 2.40 | 2.40 | 2.67 | 2.40 | nd | 2.67 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 5 | 17% | 0% | 25% | 17% | 0% | 25% | 14% | nd | 17% |
| Spec 5 | 83% | 82% | 89% | 83% | 82% | 89% | 83% | nd | 89% |
| Cutoff 6 | 2.67 | 2.92 | 2.92 | 2.67 | 2.92 | 2.92 | 2.67 | nd | 2.92 |
| Sens 6 | 17% | 0% | 25% | 17% | 0% | 25% | 14% | nd | 17% |
| Spec 6 | 91% | 91% | 90% | 91% | 91% | 90% | 91% | nd | 90% |
| OR Quart 2 | 0.96 | >1.0 | 0 | 0 | >3.3 | 0 | 0 | nd | 2.2 |
| p Value | 0.96 | <0.97 | na | na | <0.31 | na | na | nd | 0.53 |
| 95% CI of OR Quart2 | 0.21 / 4.3 | >0.064 / na | na / na | na / na | >0.33 / na | na / na | na / na | nd / nd | 0.19 / 26 |
| OR Quart 3 | 0.22 | >3.3 | 1.0 | 1.8 | >1.0 | 1.0 | 1.0 | nd | 1.0 |
| p Value | 0.19 | <0.31 | 1.0 | 0.45 | <0.97 | 1.0 | 0.97 | nd | 1.0 |
| 95% CI of OR Quart3 | 0.023 / 2.1 | >0.33 / na | 0.18 / 5.6 | 0.39 / 8.4 | >0.064 / na | 0.18 / 5.6 | 0.14 / 8.0 | nd / nd | 0.059 / 17 |
| OR Quart 4 | 0.69 | >2.2 | 0.63 | 1.4 | >2.2 | 0.63 | 1.6 | nd | 2.2 |
| p Value | 0.65 | <0.54 | 0.64 | 0.65 | <0.54 | 0.64 | 0.61 | nd | 0.53 |
| 95% CI of OR Quart4 | 0.14 / 3.4 | >0.19 / na | 0.096 / 4.2 | 0.29 / 7.2 | >0.19 / na | 0.096 / 4.2 | 0.25 / 11 | nd / nd | 0.19 / 26 |

**Islet amyloid polypeptide**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.444 | 1.14 | 0.444 | 1.14 | 0.444 | 0.618 |
| Average | 2.51 | 3.59 | 2.51 | 3.59 | 2.51 | 1.16 |
| Stdev | 10.9 | 6.46 | 10.9 | 6.46 | 10.9 | 1.53 |
| p(t-test) | | 0.74 | | 0.74 | | 0.74 |
| Min | 0.00151 | 0.00151 | 0.00151 | 0.00151 | 0.00151 | 0.00151 |
| Max | 102 | 23.2 | 102 | 23.2 | 102 | 4.11 |
| n (Samp) | 98 | 12 | 98 | 12 | 98 | 7 |
| n (Patient) | 98 | 12 | 98 | 12 | 98 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.467 | 3.48 | 0.467 | 3.48 | nd | nd |
| Average | 2.89 | 5.71 | 2.89 | 5.71 | nd | nd |
| Stdev | 11.2 | 8.76 | 11.2 | 8.76 | nd | nd |
| p(t-test) | | 0.54 | | 0.54 | nd | nd |
| Min | 0.00151 | 0.00151 | 0.00151 | 0.00151 | nd | nd |
| Max | 102 | 23.2 | 102 | 23.2 | nd | nd |
| n (Samp) | 159 | 6 | 159 | 6 | nd | nd |
| n (Patient) | 159 | 6 | 159 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.506 | 0.802 | 0.506 | 0.802 | 0.506 | 0.413 |
| Average | 1.66 | 1.62 | 1.66 | 1.62 | 1.66 | 0.955 |
| Stdev | 4.19 | 2.08 | 4.19 | 2.08 | 4.19 | 1.57 |
| p(t-test) | | 0.98 | | 0.98 | | 0.68 |
| Min | 0.00151 | 0.00151 | 0.00151 | 0.00151 | 0.00151 | 0.00151 |
| Max | 30.4 | 5.59 | 30.4 | 5.59 | 30.4 | 4.11 |
| n (Samp) | 84 | 8 | 84 | 8 | 84 | 6 |
| n (Patient) | 84 | 8 | 84 | 8 | 84 | 6 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.63 | 0.62 | 0.56 | 0.63 | 0.62 | 0.56 | 0.55 | nd | 0.45 |
| SE | 0.091 | 0.12 | 0.11 | 0.091 | 0.12 | 0.11 | 0.12 | nd | 0.13 |
| p | 0.17 | 0.32 | 0.61 | 0.17 | 0.32 | 0.61 | 0.67 | nd | 0.71 |
| nCohort 1 | 98 | 159 | 84 | 98 | 159 | 84 | 98 | nd | 84 |
| nCohort 2 | 12 | 6 | 8 | 12 | 6 | 8 | 7 | nd | 6 |
| Cutoff 1 | 0.156 | 0 | 0.156 | 0.156 | 0 | 0.156 | 0.156 | nd | 0.0802 |
| Sens 1 | 75% | 100% | 75% | 75% | 100% | 75% | 71% | nd | 83% |
| Spec 1 | 40% | 0% | 35% | 40% | 0% | 35% | 40% | nd | 30% |
| Cutoff 2 | 0.0802 | 0 | 0.0802 | 0.0802 | 0 | 0.0802 | 0.0802 | nd | 0.0802 |
| Sens 2 | 83% | 100% | 88% | 83% | 100% | 88% | 86% | nd | 83% |
| Spec 2 | 37% | 0% | 30% | 37% | 0% | 30% | 37% | nd | 30% |
| Cutoff 3 | 0 | 0 | 0 | 0 | 0 | 0 | 0 | nd | 0 |
| Sens 3 | 100% | 100% | 100% | 100% | 100% | 100% | 100% | nd | 100% |
| Spec 3 | 0% | 0% | 0% | 0% | 0% | 0% | 0% | nd | 0% |
| Cutoff 4 | 1.06 | 1.16 | 1.16 | 1.06 | 1.16 | 1.16 | 1.06 | nd | 1.16 |
| Sens 4 | 50% | 67% | 38% | 50% | 67% | 38% | 29% | nd | 17% |
| Spec 4 | 70% | 72% | 71% | 70% | 72% | 71% | 70% | nd | 71% |
| Cutoff 5 | 1.61 | 1.65 | 1.64 | 1.61 | 1.65 | 1.64 | 1.61 | nd | 1.64 |
| Sens 5 | 42% | 67% | 25% | 42% | 67% | 25% | 29% | nd | 17% |
| Spec 5 | 81% | 81% | 81% | 81% | 81% | 81% | 81% | nd | 81% |
| Cutoff 6 | 3.74 | 3.39 | 2.66 | 3.74 | 3.39 | 2.66 | 3.74 | nd | 2.66 |
| Sens 6 | 33% | 50% | 25% | 33% | 50% | 25% | 14% | nd | 17% |
| Spec 6 | 91% | 91% | 90% | 91% | 91% | 90% | 91% | nd | 90% |
| OR Quart 2 | 0.96 | 0 | 2.1 | 0.96 | 0 | 2.1 | 2.1 | nd | 2.2 |
| p Value | 0.97 | na | 0.56 | 0.97 | na | 0.56 | 0.56 | nd | 0.53 |
| 95% CI of | 0.13 | na | 0.18 | 0.13 | na | 0.18 | 0.18 | nd | 0.19 |
| OR Quart2 | 7.4 | na | 25 | 7.4 | na | 25 | 25 | nd | 26 |
| OR Quart 3 | 1.6 | 0 | 3.3 | 1.6 | 0 | 3.3 | 2.1 | nd | 2.1 |
| p Value | 0.64 | na | 0.32 | 0.64 | na | 0.32 | 0.56 | nd | 0.56 |
| 95% CI of | 0.24 | na | 0.32 | 0.24 | na | 0.32 | 0.18 | nd | 0.18 |
| OR Quart3 | 10 | na | 34 | 10 | na | 34 | 25 | nd | 25 |
| OR Quart 4 | 2.7 | 2.1 | 2.1 | 2.7 | 2.1 | 2.1 | 2.0 | nd | 1.0 |
| p Value | 0.26 | 0.42 | 0.56 | 0.26 | 0.42 | 0.56 | 0.58 | nd | 0.97 |
| 95% CI of | 0.48 | 0.35 | 0.18 | 0.48 | 0.35 | 0.18 | 0.17 | nd | 0.061 |
| OR Quart4 | 15 | 12 | 25 | 15 | 12 | 25 | 23 | nd | 18 |

**Interleukin-17A**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.00492 | 0.00492 | 0.00492 | 0.00492 | 0.00492 | 0.00847 |
| Average | 1.98 | 1.41 | 1.98 | 1.29 | 1.98 | 1.34 |
| Stdev | 12.2 | 3.55 | 12.2 | 3.12 | 12.2 | 2.93 |
| p(t-test) |  | 0.74 |  | 0.70 |  | 0.79 |
| Min | 0.000344 | 0.000380 | 0.000344 | 0.000380 | 0.000344 | 0.000603 |
| Max | 133 | 17.7 | 133 | 13.0 | 133 | 13.0 |
| n (Samp) | 282 | 48 | 282 | 47 | 282 | 27 |
| n (Patient) | 282 | 48 | 282 | 47 | 282 | 27 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.00847 | 0.00492 | 0.00847 | 0.00492 | 0.00847 | 0.00483 |
| Average | 2.11 | 2.19 | 2.11 | 0.575 | 2.11 | 0.818 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 11.9 | 8.00 | 11.9 | 1.11 | 11.9 | 1.30 |
| p(t-test) | | 0.98 | | 0.53 | | 0.66 |
| Min | 0.000344 | 0.000410 | 0.000344 | 0.000381 | 0.000344 | 0.000603 |
| Max | 157 | 39.4 | 157 | 4.03 | 157 | 4.03 |
| n (Samp) | 514 | 24 | 514 | 24 | 514 | 16 |
| n (Patient) | 514 | 24 | 514 | 24 | 514 | 16 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00827 | 0.00740 | 0.00827 | 0.00492 | 0.00827 | 0.00847 |
| Average | 1.86 | 2.69 | 1.86 | 2.13 | 1.86 | 1.39 |
| Stdev | 12.2 | 6.77 | 12.2 | 4.69 | 12.2 | 3.32 |
| p(t-test) | | 0.70 | | 0.90 | | 0.86 |
| Min | 0.000344 | 0.000380 | 0.000344 | 0.000380 | 0.000344 | 0.000747 |
| Max | 133 | 33.1 | 133 | 18.8 | 133 | 13.0 |
| n (Samp) | 251 | 34 | 251 | 33 | 251 | 20 |
| n (Patient) | 251 | 34 | 251 | 33 | 251 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.50 | 0.59 | 0.53 | 0.48 | 0.55 | 0.57 | 0.49 | 0.60 |
| SE | 0.046 | 0.060 | 0.054 | 0.046 | 0.061 | 0.055 | 0.060 | 0.074 | 0.069 |
| p | 0.25 | 0.98 | 0.11 | 0.57 | 0.76 | 0.32 | 0.27 | 0.94 | 0.16 |
| nCohort 1 | 282 | 514 | 251 | 282 | 514 | 251 | 282 | 514 | 251 |
| nCohort 2 | 48 | 24 | 34 | 47 | 24 | 33 | 27 | 16 | 20 |
| Cutoff 1 | 0.00361 | 0.00336 | 0.00392 | 0.00337 | 0.00336 | 0.00361 | 0.00336 | 0.00206 | 0.00392 |
| Sens 1 | 71% | 75% | 71% | 70% | 71% | 73% | 74% | 75% | 70% |
| Spec 1 | 46% | 33% | 44% | 40% | 33% | 42% | 40% | 20% | 44% |
| Cutoff 2 | 0.00206 | 0.000662 | 0.00303 | 0.000747 | 0.000662 | 0.00263 | 0.00206 | 0.000662 | 0.00336 |
| Sens 2 | 81% | 83% | 82% | 81% | 83% | 82% | 81% | 81% | 85% |
| Spec 2 | 21% | 9% | 35% | 10% | 9% | 31% | 21% | 9% | 39% |
| Cutoff 3 | 0.000521 | 0.000521 | 0.000662 | 0.000597 | 0.000521 | 0.000654 | 0.000603 | 0.000521 | 0.00303 |
| Sens 3 | 94% | 96% | 91% | 91% | 96% | 91% | 93% | 100% | 90% |
| Spec 3 | 2% | 3% | 11% | 2% | 3% | 9% | 4% | 3% | 35% |
| Cutoff 4 | 0.113 | 0.214 | 0.121 | 0.113 | 0.214 | 0.121 | 0.113 | 0.214 | 0.121 |
| Sens 4 | 42% | 29% | 44% | 38% | 29% | 39% | 44% | 38% | 40% |
| Spec 4 | 70% | 70% | 71% | 70% | 70% | 71% | 70% | 70% | 71% |
| Cutoff 5 | 0.418 | 0.665 | 0.464 | 0.418 | 0.665 | 0.464 | 0.418 | 0.665 | 0.464 |
| Sens 5 | 31% | 25% | 32% | 30% | 25% | 30% | 33% | 38% | 30% |
| Spec 5 | 80% | 80% | 80% | 80% | 80% | 80% | 80% | 80% | 80% |
| Cutoff 6 | 1.30 | 1.90 | 1.30 | 1.30 | 1.90 | 1.30 | 1.30 | 1.90 | 1.30 |
| Sens 6 | 21% | 17% | 24% | 19% | 12% | 21% | 26% | 19% | 20% |
| Spec 6 | 90% | 90% | 90% | 90% | 90% | 90% | 90% | 90% | 90% |
| OR Quart 2 | 1.3 | 0.56 | 2.7 | 1.2 | 0.42 | 2.2 | 1.2 | 0.16 | 3.7 |
| p Value | 0.52 | 0.37 | 0.079 | 0.66 | 0.21 | 0.14 | 0.77 | 0.094 | 0.11 |
| 95% CI of | 0.55 | 0.16 | 0.89 | 0.51 | 0.11 | 0.78 | 0.38 | 0.019 | 0.75 |
| OR Quart2 | 3.2 | 2.0 | 8.1 | 2.9 | 1.7 | 6.2 | 3.7 | 1.4 | 19 |
| OR Quart 3 | 0.89 | 1.0 | 0.79 | 0.80 | 1.2 | 0.65 | 0.82 | 0.66 | 2.6 |
| p Value | 0.81 | 1.0 | 0.73 | 0.63 | 0.79 | 0.51 | 0.75 | 0.52 | 0.27 |
| 95% CI of | 0.34 | 0.34 | 0.20 | 0.31 | 0.41 | 0.17 | 0.24 | 0.18 | 0.48 |
| OR Quart3 | 2.3 | 2.9 | 3.1 | 2.0 | 3.3 | 2.4 | 2.8 | 2.4 | 14 |
| OR Quart 4 | 1.7 | 0.86 | 2.9 | 1.3 | 0.86 | 2.0 | 1.5 | 0.83 | 3.1 |
| p Value | 0.22 | 0.79 | 0.055 | 0.54 | 0.79 | 0.20 | 0.43 | 0.77 | 0.17 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of OR Quart4 | 0.73 | 0.28 | 0.98 | 0.56 | 0.28 | 0.69 | 0.52 | 0.25 | 0.61 |
|  | 4.1 | 2.6 | 8.6 | 3.1 | 2.6 | 5.7 | 4.6 | 2.8 | 16 |

**Insulin receptor substrate 1**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.00227 | 0.00243 | 0.00227 | 0.00243 | 0.00227 | 0.00257 |
| Average | 0.00318 | 0.0143 | 0.00318 | 0.0145 | 0.00318 | 0.00330 |
| Stdev | 0.00880 | 0.0546 | 0.00880 | 0.0558 | 0.00880 | 0.00268 |
| p(t-test) |  | 0.045 |  | 0.044 |  | 0.96 |
| Min | 1.25E-9 | 7.25E-7 | 1.25E-9 | 7.25E-7 | 1.25E-9 | 4.51E-6 |
| Max | 0.0712 | 0.282 | 0.0712 | 0.282 | 0.0712 | 0.00914 |
| n (Samp) | 108 | 26 | 108 | 25 | 108 | 14 |
| n (Patient) | 108 | 26 | 108 | 25 | 108 | 14 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.00163 | 0.00243 | 0.00163 | 0.00243 | 0.00163 | 0.00257 |
| Average | 0.00828 | 0.00303 | 0.00828 | 0.00283 | 0.00828 | 0.00296 |
| Stdev | 0.0530 | 0.00242 | 0.0530 | 0.00253 | 0.0530 | 0.00294 |
| p(t-test) |  | 0.71 |  | 0.70 |  | 0.75 |
| Min | 1.25E-9 | 4.51E-6 | 1.25E-9 | 4.51E-6 | 1.25E-9 | 4.51E-6 |
| Max | 0.735 | 0.00914 | 0.735 | 0.00914 | 0.735 | 0.00914 |
| n (Samp) | 229 | 14 | 229 | 14 | 229 | 10 |
| n (Patient) | 229 | 14 | 229 | 14 | 229 | 10 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.00224 | 0.00243 | 0.00224 | 0.00243 | 0.00224 | 0.00243 |
| Average | 0.00335 | 0.0208 | 0.00335 | 0.0218 | 0.00335 | 0.00256 |
| Stdev | 0.00844 | 0.0697 | 0.00844 | 0.0720 | 0.00844 | 0.00225 |
| p(t-test) |  | 0.0081 |  | 0.0065 |  | 0.79 |
| Min | 1.25E-9 | 7.25E-7 | 1.25E-9 | 7.25E-7 | 1.25E-9 | 4.51E-6 |
| Max | 0.0712 | 0.282 | 0.0712 | 0.282 | 0.0712 | 0.00601 |
| n (Samp) | 123 | 16 | 123 | 15 | 123 | 8 |
| n (Patient) | 123 | 16 | 123 | 15 | 123 | 8 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.68 | 0.65 | 0.62 | 0.65 | 0.61 | 0.60 | 0.68 | 0.60 | 0.59 |
| SE | 0.062 | 0.082 | 0.079 | 0.065 | 0.082 | 0.081 | 0.083 | 0.097 | 0.11 |
| p | 0.0031 | 0.061 | 0.14 | 0.021 | 0.17 | 0.21 | 0.032 | 0.29 | 0.40 |
| nCohort 1 | 108 | 229 | 123 | 108 | 229 | 123 | 108 | 229 | 123 |
| nCohort 2 | 26 | 14 | 16 | 25 | 14 | 15 | 14 | 10 | 8 |
| Cutoff 1 | 0.00224 | 0.00224 | 0.00110 | 0.00110 | 0.00110 | 0.00110 | 0.00229 | 0.00110 | 0.00110 |
| Sens 1 | 73% | 71% | 75% | 76% | 71% | 73% | 71% | 70% | 75% |
| Spec 1 | 50% | 55% | 45% | 43% | 47% | 45% | 58% | 47% | 45% |
| Cutoff 2 | 0.00110 | 0.000646 | 2.78E-5 | 0.000646 | 0.000610 | 2.78E-5 | 2.32E-6 | 2.32E-6 | 2.78E-5 |
| Sens 2 | 81% | 86% | 88% | 80% | 86% | 87% | 100% | 100% | 88% |
| Spec 2 | 43% | 34% | 28% | 29% | 31% | 28% | 15% | 13% | 28% |
| Cutoff 3 | 2.32E-6 | 2.32E-6 | 2.32E-6 | 2.32E-6 | 2.32E-6 | 2.32E-6 | 2.32E-6 | 2.32E-6 | 2.32E-6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 96% | 100% | 94% | 96% | 100% | 93% | 100% | 100% | 100% |
| Spec 3 | 15% | 13% | 15% | 15% | 13% | 15% | 15% | 13% | 15% |
| Cutoff 4 | 0.00243 | 0.00243 | 0.00243 | 0.00243 | 0.00243 | 0.00243 | 0.00243 | 0.00243 | 0.00243 |
| Sens 4 | 46% | 43% | 38% | 40% | 36% | 33% | 50% | 50% | 25% |
| Spec 4 | 81% | 77% | 73% | 81% | 77% | 73% | 81% | 77% | 73% |
| Cutoff 5 | 0.00243 | 0.00271 | 0.00281 | 0.00243 | 0.00271 | 0.00281 | 0.00243 | 0.00271 | 0.00281 |
| Sens 5 | 46% | 36% | 38% | 40% | 36% | 33% | 50% | 40% | 25% |
| Spec 5 | 81% | 81% | 82% | 81% | 81% | 82% | 81% | 81% | 82% |
| Cutoff 6 | 0.00334 | 0.00576 | 0.00563 | 0.00334 | 0.00576 | 0.00563 | 0.00334 | 0.00576 | 0.00563 |
| Sens 6 | 38% | 7% | 19% | 36% | 7% | 20% | 36% | 10% | 12% |
| Spec 6 | 92% | 91% | 90% | 92% | 91% | 90% | 92% | 91% | 90% |
| OR Quart 2 | 0.70 | 0.98 | 2.1 | 2.2 | 2.0 | 2.1 | 0.30 | 0.32 | 2.0 |
| p Value | 0.66 | 0.99 | 0.42 | 0.29 | 0.42 | 0.42 | 0.31 | 0.33 | 0.58 |
| 95% CI of OR Quart2 | 0.14 | 0.13 | 0.35 | 0.51 | 0.36 | 0.35 | 0.029 | 0.032 | 0.17 |
| | 3.4 | 7.2 | 12 | 9.8 | 12 | 12 | 3.1 | 3.1 | 23 |
| OR Quart 3 | 2.0 | 2.0 | 2.1 | 2.2 | 1.5 | 2.1 | 1.0 | 0.32 | 3.1 |
| p Value | 0.33 | 0.42 | 0.42 | 0.29 | 0.66 | 0.40 | 1.0 | 0.33 | 0.34 |
| 95% CI of OR Quart3 | 0.51 | 0.36 | 0.35 | 0.51 | 0.24 | 0.36 | 0.19 | 0.032 | 0.31 |
| | 7.4 | 12 | 12 | 9.8 | 9.3 | 13 | 5.4 | 3.1 | 31 |
| OR Quart 4 | 4.0 | 3.2 | 3.3 | 4.2 | 2.6 | 2.7 | 2.6 | 1.7 | 2.0 |
| p Value | 0.032 | 0.17 | 0.16 | 0.045 | 0.27 | 0.26 | 0.20 | 0.48 | 0.58 |
| 95% CI of OR Quart4 | 1.1 | 0.61 | 0.62 | 1.0 | 0.48 | 0.48 | 0.61 | 0.39 | 0.17 |
| | 14 | 16 | 18 | 17 | 14 | 15 | 11 | 7.4 | 23 |

**Involucrin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.582 | 1.50 | 0.582 | 1.36 | 0.582 | 1.05 |
| Average | 1.43 | 3.41 | 1.43 | 3.06 | 1.43 | 2.26 |
| Stdev | 3.66 | 4.66 | 3.66 | 4.61 | 3.66 | 3.57 |
| p(t-test) | | 0.0032 | | 0.016 | | 0.31 |
| Min | 0.0119 | 0.0767 | 0.0119 | 0.0767 | 0.0119 | 0.309 |
| Max | 47.1 | 19.7 | 47.1 | 19.7 | 47.1 | 17.0 |
| n (Samp) | 253 | 37 | 253 | 36 | 253 | 22 |
| n (Patient) | 253 | 37 | 253 | 36 | 253 | 22 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.763 | 1.15 | 0.763 | 1.10 | 0.763 | 1.15 |
| Average | 2.14 | 2.60 | 2.14 | 2.40 | 2.14 | 2.64 |
| Stdev | 6.06 | 3.29 | 6.06 | 3.36 | 6.06 | 3.61 |
| p(t-test) | | 0.74 | | 0.85 | | 0.75 |
| Min | 0.0119 | 0.0767 | 0.0119 | 0.0767 | 0.0119 | 0.226 |
| Max | 102 | 14.5 | 102 | 14.5 | 102 | 14.5 |
| n (Samp) | 424 | 19 | 424 | 19 | 424 | 15 |
| n (Patient) | 424 | 19 | 424 | 19 | 424 | 15 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.622 | 2.24 | 0.622 | 2.18 | 0.622 | 1.02 |
| Average | 1.62 | 4.36 | 1.62 | 4.00 | 1.62 | 2.81 |
| Stdev | 4.08 | 5.31 | 4.08 | 5.31 | 4.08 | 4.40 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.0020 | | 0.0083 | | 0.29 |
| Min | 0.0224 | 0.226 | 0.0224 | 0.226 | 0.0224 | 0.309 |
| Max | 47.1 | 19.7 | 47.1 | 19.7 | 47.1 | 17.0 |
| n (Samp) | 216 | 26 | 216 | 25 | 216 | 14 |
| n (Patient) | 216 | 26 | 216 | 25 | 216 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.73 | 0.65 | 0.75 | 0.69 | 0.58 | 0.73 | 0.70 | 0.65 | 0.66 |
| SE | 0.049 | 0.070 | 0.058 | 0.051 | 0.070 | 0.059 | 0.064 | 0.078 | 0.082 |
| p | 5.0E-6 | 0.037 | 2.0E-5 | 1.6E-4 | 0.23 | 1.1E-4 | 0.0022 | 0.053 | 0.051 |
| nCohort 1 | 253 | 424 | 216 | 253 | 424 | 216 | 253 | 424 | 216 |
| nCohort 2 | 37 | 19 | 26 | 36 | 19 | 25 | 22 | 15 | 14 |
| Cutoff 1 | 0.794 | 0.750 | 0.941 | 0.720 | 0.697 | 0.941 | 0.720 | 0.906 | 0.688 |
| Sens 1 | 70% | 74% | 73% | 72% | 74% | 72% | 73% | 73% | 71% |
| Spec 1 | 62% | 50% | 62% | 60% | 47% | 62% | 60% | 55% | 53% |
| Cutoff 2 | 0.697 | 0.697 | 0.731 | 0.605 | 0.357 | 0.731 | 0.697 | 0.750 | 0.522 |
| Sens 2 | 81% | 84% | 81% | 81% | 84% | 80% | 82% | 80% | 86% |
| Spec 2 | 58% | 47% | 55% | 54% | 25% | 55% | 58% | 50% | 41% |
| Cutoff 3 | 0.356 | 0.357 | 0.418 | 0.298 | 0.188 | 0.378 | 0.522 | 0.697 | 0.418 |
| Sens 3 | 92% | 95% | 92% | 92% | 95% | 92% | 91% | 93% | 93% |
| Spec 3 | 32% | 25% | 32% | 26% | 13% | 30% | 46% | 47% | 32% |
| Cutoff 4 | 1.13 | 1.60 | 1.23 | 1.13 | 1.60 | 1.23 | 1.13 | 1.60 | 1.23 |
| Sens 4 | 59% | 47% | 65% | 56% | 42% | 64% | 45% | 47% | 43% |
| Spec 4 | 70% | 70% | 70% | 70% | 70% | 70% | 70% | 70% | 70% |
| Cutoff 5 | 1.68 | 2.42 | 1.73 | 1.68 | 2.42 | 1.73 | 1.68 | 2.42 | 1.73 |
| Sens 5 | 43% | 37% | 54% | 39% | 32% | 52% | 27% | 27% | 36% |
| Spec 5 | 80% | 80% | 80% | 80% | 80% | 80% | 80% | 80% | 80% |
| Cutoff 6 | 2.59 | 3.90 | 2.68 | 2.59 | 3.90 | 2.68 | 2.59 | 3.90 | 2.68 |
| Sens 6 | 38% | 16% | 42% | 33% | 16% | 40% | 23% | 13% | 29% |
| Spec 6 | 90% | 90% | 90% | 90% | 90% | 90% | 90% | 90% | 90% |
| OR Quart 2 | 1.3 | 2.0 | 1.5 | 0.74 | 0.99 | 1.5 | 2.0 | 3.0 | 2.0 |
| p Value | 0.71 | 0.42 | 0.66 | 0.70 | 0.99 | 0.65 | 0.58 | 0.34 | 0.58 |
| 95% CI of OR Quart2 | 0.29 | 0.36 | 0.24 | 0.16 | 0.24 | 0.25 | 0.18 | 0.31 | 0.18 |
| | 6.2 | 11 | 9.3 | 3.4 | 4.1 | 9.5 | 23 | 30 | 23 |
| OR Quart 3 | 4.6 | 3.1 | 3.8 | 3.7 | 1.2 | 3.8 | 13 | 6.2 | 6.6 |
| p Value | 0.023 | 0.17 | 0.10 | 0.027 | 0.74 | 0.10 | 0.016 | 0.093 | 0.086 |
| 95% CI of OR Quart3 | 1.2 | 0.61 | 0.76 | 1.2 | 0.33 | 0.76 | 1.6 | 0.74 | 0.77 |
| | 17 | 16 | 19 | 12 | 4.8 | 19 | 100 | 53 | 57 |
| OR Quart 4 | 7.5 | 3.6 | 8.6 | 4.8 | 1.5 | 7.9 | 8.8 | 5.1 | 5.3 |
| p Value | 0.0019 | 0.11 | 0.0058 | 0.0078 | 0.53 | 0.0086 | 0.043 | 0.14 | 0.13 |
| 95% CI of OR Quart4 | 2.1 | 0.74 | 1.9 | 1.5 | 0.42 | 1.7 | 1.1 | 0.59 | 0.60 |
| | 27 | 18 | 40 | 15 | 5.5 | 37 | 72 | 45 | 47 |

**Collagenase 3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.74 | 5.62 | 6.74 | 7.51 | 6.74 | 16.2 |
| Average | 20.4 | 16.2 | 20.4 | 16.7 | 20.4 | 27.9 |
| Stdev | 41.3 | 24.0 | 41.3 | 24.3 | 41.3 | 29.1 |
| p(t-test) | | 0.64 | | 0.68 | | 0.55 |
| Min | 0.00479 | 0.00479 | 0.00479 | 0.00479 | 0.00479 | 0.202 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 340 | 80.1 | 340 | 80.1 | 340 | 80.1 |
| n (Samp) | 98 | 24 | 98 | 23 | 98 | 12 |
| n (Patient) | 98 | 24 | 98 | 23 | 98 | 12 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 11.1 | 13.5 | 11.1 | 13.5 | 11.1 | 18.3 |
| Average | 21.4 | 24.2 | 21.4 | 24.2 | 21.4 | 34.9 |
| Stdev | 37.5 | 29.7 | 37.5 | 29.7 | 37.5 | 30.1 |
| p(t-test) | | 0.79 | | 0.79 | | 0.29 |
| Min | 0.00479 | 0.00588 | 0.00479 | 0.00479 | 0.00479 | 0.202 |
| Max | 340 | 80.1 | 340 | 80.1 | 340 | 80.1 |
| n (Samp) | 210 | 13 | 210 | 13 | 210 | 9 |
| n (Patient) | 210 | 13 | 210 | 13 | 210 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 7.62 | 3.73 | 7.62 | 6.42 | 7.62 | 13.5 |
| Average | 21.7 | 9.64 | 21.7 | 9.88 | 21.7 | 12.8 |
| Stdev | 40.3 | 12.3 | 40.3 | 12.2 | 40.3 | 15.1 |
| p(t-test) | | 0.25 | | 0.28 | | 0.56 |
| Min | 0.00479 | 0.00479 | 0.00479 | 0.00479 | 0.00479 | 0.202 |
| Max | 340 | 41.9 | 340 | 41.9 | 340 | 41.9 |
| n (Samp) | 111 | 15 | 111 | 14 | 111 | 7 |
| n (Patient) | 111 | 15 | 111 | 14 | 111 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.45 | 0.51 | 0.38 | 0.46 | 0.51 | 0.40 | 0.61 | 0.67 | 0.44 |
| SE | 0.067 | 0.083 | 0.081 | 0.068 | 0.083 | 0.084 | 0.091 | 0.10 | 0.12 |
| p | 0.43 | 0.87 | 0.13 | 0.57 | 0.88 | 0.22 | 0.24 | 0.085 | 0.60 |
| nCohort 1 | 98 | 210 | 111 | 98 | 210 | 111 | 98 | 210 | 111 |
| nCohort 2 | 24 | 13 | 15 | 23 | 13 | 14 | 12 | 9 | 7 |
| Cutoff 1 | 0.0425 | 0.0425 | 0.0466 | 0.0425 | 0.0425 | 0.0466 | 5.86 | 13.0 | 0.0466 |
| Sens 1 | 88% | 92% | 87% | 91% | 92% | 93% | 75% | 78% | 100% |
| Spec 1 | 4% | 7% | 6% | 4% | 7% | 6% | 50% | 55% | 6% |
| Cutoff 2 | 0.0425 | 0.0425 | 0.0466 | 0.0425 | 0.0425 | 0.0466 | 0.0425 | 5.86 | 0.0466 |
| Sens 2 | 88% | 92% | 87% | 91% | 92% | 93% | 100% | 89% | 100% |
| Spec 2 | 4% | 7% | 6% | 4% | 7% | 6% | 4% | 41% | 6% |
| Cutoff 3 | 0.00479 | 0.0425 | 0 | 0.0425 | 0.0425 | 0.0466 | 0.0425 | 0.0425 | 0.0466 |
| Sens 3 | 92% | 92% | 100% | 91% | 92% | 93% | 100% | 100% | 100% |
| Spec 3 | 1% | 7% | 0% | 4% | 7% | 6% | 4% | 7% | 6% |
| Cutoff 4 | 20.0 | 24.1 | 20.4 | 20.0 | 24.1 | 20.4 | 20.0 | 24.1 | 20.4 |
| Sens 4 | 21% | 31% | 13% | 22% | 31% | 14% | 33% | 44% | 14% |
| Spec 4 | 70% | 70% | 70% | 70% | 70% | 70% | 70% | 70% | 70% |
| Cutoff 5 | 34.6 | 33.2 | 36.7 | 34.6 | 33.2 | 36.7 | 34.6 | 33.2 | 36.7 |
| Sens 5 | 17% | 31% | 7% | 17% | 31% | 7% | 33% | 44% | 14% |
| Spec 5 | 81% | 80% | 80% | 81% | 80% | 80% | 81% | 80% | 80% |
| Cutoff 6 | 52.0 | 51.3 | 57.0 | 52.0 | 51.3 | 57.0 | 52.0 | 51.3 | 57.0 |
| Sens 6 | 12% | 31% | 0% | 13% | 31% | 0% | 25% | 44% | 0% |
| Spec 6 | 91% | 90% | 90% | 91% | 90% | 90% | 91% | 90% | 90% |
| OR Quart 2 | 2.5 | 0.47 | 7.4 | 2.5 | 0.47 | 7.4 | 0.30 | 0.98 | 3.3 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value | 0.18 | 0.40 | 0.071 | 0.18 | 0.40 | 0.071 | 0.31 | 0.99 | 0.31 |
| 95% CI of | 0.65 | 0.083 | 0.84 | 0.65 | 0.083 | 0.84 | 0.029 | 0.060 | 0.33 |
| OR Quart2 | 9.2 | 2.7 | 66 | 9.2 | 2.7 | 66 | 3.0 | 16 | 34 |
| OR Quart 3 | 0.72 | 0.72 | 1.0 | 0.75 | 0.72 | 1.0 | 1.4 | 3.1 | 0 |
| p Value | 0.69 | 0.68 | 1.0 | 0.72 | 0.68 | 0.98 | 0.69 | 0.34 | na |
| 95% CI of | 0.15 | 0.15 | 0.060 | 0.15 | 0.15 | 0.062 | 0.28 | 0.31 | na |
| OR Quart3 | 3.5 | 3.4 | 17 | 3.7 | 3.4 | 17 | 6.9 | 30 | na |
| OR Quart 4 | 2.9 | 0.98 | 9.0 | 2.5 | 0.98 | 7.4 | 1.3 | 4.2 | 3.3 |
| p Value | 0.11 | 0.98 | 0.046 | 0.18 | 0.98 | 0.071 | 0.72 | 0.21 | 0.31 |
| 95% CI of | 0.78 | 0.23 | 1.0 | 0.65 | 0.23 | 0.84 | 0.27 | 0.45 | 0.33 |
| OR Quart4 | 11 | 4.1 | 79 | 9.2 | 4.1 | 66 | 6.6 | 38 | 34 |

**NF-kappa-B inhibitor alpha**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.000527 | 0.000580 | 0.000527 | 0.000523 | 0.000527 | 0.000330 |
| Average | 0.00895 | 0.0153 | 0.00895 | 0.0159 | 0.00895 | 0.0205 |
| Stdev | 0.0334 | 0.0514 | 0.0334 | 0.0523 | 0.0334 | 0.0684 |
| p(t-test) |  | 0.44 |  | 0.41 |  | 0.29 |
| Min | 4.17E-7 | 3.65E-7 | 4.17E-7 | 3.65E-7 | 4.17E-7 | 1.17E-6 |
| Max | 0.211 | 0.257 | 0.211 | 0.257 | 0.211 | 0.257 |
| n (Samp) | 108 | 26 | 108 | 25 | 108 | 14 |
| n (Patient) | 108 | 26 | 108 | 25 | 108 | 14 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.000493 | 0.000330 | 0.000493 | 0.000330 | 0.000493 | 0.000330 |
| Average | 0.00927 | 0.00714 | 0.00927 | 0.00714 | 0.00927 | 0.00291 |
| Stdev | 0.0338 | 0.0191 | 0.0338 | 0.0191 | 0.0338 | 0.00758 |
| p(t-test) |  | 0.82 |  | 0.82 |  | 0.55 |
| Min | 4.17E-7 | 1.17E-6 | 4.17E-7 | 1.17E-6 | 4.17E-7 | 1.17E-6 |
| Max | 0.245 | 0.0695 | 0.245 | 0.0695 | 0.245 | 0.0244 |
| n (Samp) | 229 | 14 | 229 | 14 | 229 | 10 |
| n (Patient) | 229 | 14 | 229 | 14 | 229 | 10 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.000566 | 0.000602 | 0.000566 | 0.000566 | 0.000566 | 0.000426 |
| Average | 0.00908 | 0.0187 | 0.00908 | 0.0199 | 0.00908 | 0.0325 |
| Stdev | 0.0319 | 0.0638 | 0.0319 | 0.0658 | 0.0319 | 0.0908 |
| p(t-test) |  | 0.32 |  | 0.28 |  | 0.090 |
| Min | 4.17E-7 | 3.65E-7 | 4.17E-7 | 3.65E-7 | 4.17E-7 | 2.62E-5 |
| Max | 0.211 | 0.257 | 0.211 | 0.257 | 0.211 | 0.257 |
| n (Samp) | 123 | 16 | 123 | 15 | 123 | 8 |
| n (Patient) | 123 | 16 | 123 | 15 | 123 | 8 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.54 | 0.48 | 0.54 | 0.53 | 0.48 | 0.54 | 0.48 | 0.45 | 0.46 |
| SE | 0.064 | 0.081 | 0.078 | 0.065 | 0.081 | 0.080 | 0.083 | 0.096 | 0.11 |
| p | 0.55 | 0.76 | 0.61 | 0.59 | 0.76 | 0.65 | 0.80 | 0.58 | 0.70 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| nCohort 1 | 108 | 229 | 123 | 108 | 229 | 123 | 108 | 229 | 123 |
| nCohort 2 | 26 | 14 | 16 | 25 | 14 | 15 | 14 | 10 | 8 |
| Cutoff 1 | 3.20E-5 | 3.20E-5 | 0.000219 | 3.20E-5 | 3.20E-5 | 0.000219 | 0.000193 | 0.000243 | 0.000193 |
| Sens 1 | 77% | 71% | 75% | 76% | 71% | 73% | 71% | 70% | 75% |
| Spec 1 | 31% | 31% | 36% | 31% | 31% | 36% | 39% | 43% | 35% |
| Cutoff 2 | 2.62E-5 | 2.25E-5 | 3.20E-5 | 2.62E-5 | 2.25E-5 | 3.20E-5 | 2.62E-5 | 2.62E-5 | 3.20E-5 |
| Sens 2 | 81% | 93% | 81% | 80% | 93% | 80% | 86% | 80% | 88% |
| Spec 2 | 18% | 11% | 28% | 18% | 11% | 28% | 18% | 15% | 28% |
| Cutoff 3 | 2.25E-5 | 2.25E-5 | 2.25E-5 | 2.25E-5 | 2.25E-5 | 2.25E-5 | 2.25E-5 | 2.25E-5 | 2.25E-5 |
| Sens 3 | 92% | 93% | 94% | 92% | 93% | 93% | 93% | 90% | 100% |
| Spec 3 | 11% | 11% | 11% | 11% | 11% | 11% | 11% | 11% | 11% |
| Cutoff 4 | 0.00128 | 0.00130 | 0.00140 | 0.00128 | 0.00130 | 0.00140 | 0.00128 | 0.00130 | 0.00140 |
| Sens 4 | 35% | 21% | 31% | 32% | 21% | 33% | 21% | 20% | 12% |
| Spec 4 | 70% | 70% | 72% | 70% | 70% | 72% | 70% | 70% | 72% |
| Cutoff 5 | 0.00274 | 0.00274 | 0.00274 | 0.00274 | 0.00274 | 0.00274 | 0.00274 | 0.00274 | 0.00274 |
| Sens 5 | 27% | 14% | 31% | 28% | 14% | 33% | 14% | 10% | 12% |
| Spec 5 | 81% | 80% | 80% | 81% | 80% | 80% | 81% | 80% | 80% |
| Cutoff 6 | 0.00704 | 0.0142 | 0.0127 | 0.00704 | 0.0142 | 0.0127 | 0.00704 | 0.0142 | 0.0127 |
| Sens 6 | 23% | 14% | 12% | 24% | 14% | 13% | 14% | 10% | 12% |
| Spec 6 | 91% | 90% | 90% | 91% | 90% | 90% | 91% | 90% | 90% |
| OR Quart 2 | 1.2 | 1.0 | 1.3 | 1.2 | 1.0 | 1.3 | 1.0 | 1.0 | 2.1 |
| p Value | 0.80 | 1.0 | 0.72 | 0.76 | 1.0 | 0.72 | 0.97 | 1.0 | 0.56 |
| 95% CI of OR Quart2 | 0.35 3.9 | 0.19 5.2 | 0.28 6.5 | 0.36 4.1 | 0.19 5.2 | 0.28 6.5 | 0.19 5.6 | 0.14 7.3 | 0.18 24 |
| OR Quart 3 | 0.80 | 1.7 | 1.3 | 0.62 | 1.7 | 1.0 | 1.8 | 1.5 | 4.4 |
| p Value | 0.74 | 0.47 | 0.72 | 0.49 | 0.47 | 1.0 | 0.45 | 0.65 | 0.20 |
| 95% CI of OR Quart3 | 0.22 2.9 | 0.39 7.6 | 0.28 6.5 | 0.16 2.4 | 0.39 7.6 | 0.19 5.3 | 0.39 8.3 | 0.25 9.5 | 0.47 42 |
| OR Quart 4 | 1.4 | 1.0 | 1.7 | 1.4 | 1.0 | 1.7 | 1.0 | 1.6 | 1.0 |
| p Value | 0.59 | 0.98 | 0.48 | 0.59 | 0.98 | 0.48 | 0.97 | 0.64 | 0.98 |
| 95% CI of OR Quart4 | 0.42 4.5 | 0.20 5.3 | 0.38 7.8 | 0.42 4.5 | 0.20 5.3 | 0.38 7.8 | 0.19 5.6 | 0.25 9.7 | 0.062 17 |

**Beta-nerve growth factor**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 4.27 | 4.34 | 4.27 | 4.42 | 4.27 | 4.53 |
| Average | 6.24 | 6.70 | 6.24 | 6.66 | 6.24 | 8.34 |
| Stdev | 6.49 | 6.86 | 6.49 | 7.04 | 6.49 | 8.77 |
| p(t-test) |  | 0.75 |  | 0.77 |  | 0.28 |
| Min | 0.00207 | 0.000506 | 0.00207 | 0.000506 | 0.00207 | 0.902 |
| Max | 44.0 | 25.2 | 44.0 | 25.2 | 44.0 | 25.2 |
| n (Samp) | 111 | 26 | 111 | 25 | 111 | 14 |
| n (Patient) | 111 | 26 | 111 | 25 | 111 | 14 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 3.81 | 5.50 | 3.81 | 5.50 | 3.81 | 5.76 |
| Average | 6.06 | 7.65 | 6.06 | 7.43 | 6.06 | 8.71 |
| Stdev | 6.74 | 7.09 | 6.74 | 7.25 | 6.74 | 8.21 |
| p(t-test) |  | 0.39 |  | 0.46 |  | 0.23 |
| Min | 0.000499 | 1.34 | 0.000499 | 0.943 | 0.000499 | 1.04 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 46.1 | 25.2 | 46.1 | 25.2 | 46.1 | 25.2 |
| n (Samp) | 231 | 14 | 231 | 14 | 231 | 10 |
| n (Patient) | 231 | 14 | 231 | 14 | 231 | 10 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.81 | 3.38 | 3.81 | 3.79 | 3.81 | 2.51 |
| Average | 5.80 | 6.34 | 5.80 | 6.46 | 5.80 | 8.00 |
| Stdev | 5.90 | 7.72 | 5.90 | 7.95 | 5.90 | 10.4 |
| p(t-test) | | 0.74 | | 0.69 | | 0.33 |
| Min | 0.000506 | 0.000506 | 0.000506 | 0.000506 | 0.000506 | 0.902 |
| Max | 32.2 | 25.2 | 32.2 | 25.2 | 32.2 | 25.2 |
| n (Samp) | 126 | 16 | 126 | 15 | 126 | 8 |
| n (Patient) | 126 | 16 | 126 | 15 | 126 | 8 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.52 | 0.61 | 0.47 | 0.51 | 0.58 | 0.48 | 0.55 | 0.63 | 0.46 |
| SE | 0.064 | 0.082 | 0.078 | 0.064 | 0.082 | 0.080 | 0.084 | 0.097 | 0.11 |
| p | 0.79 | 0.17 | 0.72 | 0.90 | 0.31 | 0.78 | 0.57 | 0.18 | 0.69 |
| nCohort 1 | 111 | 231 | 126 | 111 | 231 | 126 | 111 | 231 | 126 |
| nCohort 2 | 26 | 14 | 16 | 25 | 14 | 15 | 14 | 10 | 8 |
| Cutoff 1 | 2.63 | 4.24 | 1.92 | 2.63 | 4.07 | 2.29 | 2.61 | 4.36 | 1.89 |
| Sens 1 | 73% | 71% | 75% | 72% | 71% | 73% | 71% | 70% | 75% |
| Spec 1 | 30% | 54% | 22% | 30% | 53% | 28% | 29% | 56% | 22% |
| Cutoff 2 | 2.29 | 2.63 | 1.77 | 2.29 | 2.61 | 1.77 | 1.77 | 4.07 | 1.77 |
| Sens 2 | 81% | 86% | 81% | 80% | 86% | 80% | 86% | 80% | 88% |
| Spec 2 | 25% | 32% | 22% | 25% | 31% | 22% | 20% | 53% | 22% |
| Cutoff 3 | 0.950 | 2.61 | 0.771 | 0.771 | 1.49 | 0.223 | 1.04 | 2.61 | 0.771 |
| Sens 3 | 92% | 93% | 94% | 92% | 93% | 93% | 93% | 90% | 100% |
| Spec 3 | 9% | 31% | 9% | 8% | 16% | 3% | 10% | 31% | 9% |
| Cutoff 4 | 7.11 | 6.46 | 6.50 | 7.11 | 6.46 | 6.50 | 7.11 | 6.46 | 6.50 |
| Sens 4 | 27% | 36% | 31% | 28% | 36% | 27% | 36% | 50% | 25% |
| Spec 4 | 71% | 71% | 71% | 71% | 71% | 71% | 71% | 71% | 71% |
| Cutoff 5 | 8.14 | 8.14 | 7.78 | 8.14 | 8.14 | 7.78 | 8.14 | 8.14 | 7.78 |
| Sens 5 | 19% | 21% | 25% | 20% | 21% | 27% | 29% | 30% | 25% |
| Spec 5 | 80% | 80% | 80% | 80% | 80% | 80% | 80% | 80% | 80% |
| Cutoff 6 | 13.7 | 13.7 | 13.5 | 13.7 | 13.7 | 13.5 | 13.7 | 13.7 | 13.5 |
| Sens 6 | 12% | 14% | 12% | 12% | 14% | 13% | 21% | 20% | 25% |
| Spec 6 | 90% | 90% | 90% | 90% | 90% | 90% | 90% | 90% | 90% |
| OR Quart 2 | 1.2 | 2.0 | 0.75 | 1.0 | 1.0 | 0.75 | 1.0 | 1.0 | 0.50 |
| p Value | 0.76 | 0.57 | 0.72 | 1.0 | 1.0 | 0.72 | 1.0 | 1.0 | 0.58 |
| 95% CI of OR Quart2 | 0.36 4.1 | 0.18 23 | 0.16 3.6 | 0.29 3.5 | 0.14 7.3 | 0.16 3.6 | 0.19 5.4 | 0.061 16 | 0.043 5.8 |
| OR Quart 3 | 1.0 | 7.8 | 1.0 | 1.2 | 3.2 | 1.0 | 1.4 | 4.2 | 1.0 |
| p Value | 1.0 | 0.059 | 1.0 | 0.76 | 0.16 | 0.97 | 0.69 | 0.20 | 1.0 |
| 95% CI of OR Quart3 | 0.29 3.5 | 0.93 65 | 0.23 4.3 | 0.36 4.1 | 0.62 17 | 0.24 4.5 | 0.28 6.8 | 0.46 39 | 0.13 7.5 |
| OR Quart 4 | 1.2 | 4.1 | 1.3 | 1.0 | 2.0 | 1.0 | 1.3 | 4.1 | 1.6 |
| p Value | 0.80 | 0.21 | 0.69 | 1.0 | 0.42 | 0.97 | 0.72 | 0.21 | 0.62 |
| 95% CI of OR Quart4 | 0.35 3.9 | 0.45 38 | 0.33 5.4 | 0.29 3.5 | 0.36 12 | 0.24 4.5 | 0.27 6.5 | 0.45 38 | 0.25 10 |

**Pancreatic prohormone**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.98 | 57.1 | 2.98 | 57.1 | 2.98 | 29.5 |
| Average | 98.8 | 236 | 98.8 | 236 | 98.8 | 207 |
| Stdev | 201 | 317 | 201 | 317 | 201 | 320 |
| p(t-test) | | 0.040 | | 0.040 | | 0.19 |
| Min | 0.117 | 0.122 | 0.117 | 0.122 | 0.117 | 0.122 |
| Max | 950 | 832 | 950 | 832 | 950 | 832 |
| n (Samp) | 98 | 12 | 98 | 12 | 98 | 7 |
| n (Patient) | 98 | 12 | 98 | 12 | 98 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 13.4 | 508 | 13.4 | 508 | nd | nd |
| Average | 111 | 429 | 111 | 429 | nd | nd |
| Stdev | 220 | 357 | 220 | 357 | nd | nd |
| p(t-test) | | 8.5E-4 | | 8.5E-4 | nd | nd |
| Min | 0.117 | 0.122 | 0.117 | 0.122 | nd | nd |
| Max | 1220 | 832 | 1220 | 832 | nd | nd |
| n (Samp) | 159 | 6 | 159 | 6 | nd | nd |
| n (Patient) | 159 | 6 | 159 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.39 | 14.9 | 9.39 | 14.9 | 9.39 | 14.9 |
| Average | 98.0 | 87.7 | 98.0 | 87.7 | 98.0 | 103 |
| Stdev | 194 | 154 | 194 | 154 | 194 | 178 |
| p(t-test) | | 0.88 | | 0.88 | | 0.95 |
| Min | 0.117 | 0.122 | 0.117 | 0.122 | 0.117 | 0.122 |
| Max | 950 | 448 | 950 | 448 | 950 | 448 |
| n (Samp) | 84 | 8 | 84 | 8 | 84 | 6 |
| n (Patient) | 84 | 8 | 84 | 8 | 84 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | 0.66 | 0.44 | 0.56 | 0.66 | 0.44 | 0.54 | nd | 0.44 |
| SE | 0.091 | 0.12 | 0.11 | 0.091 | 0.12 | 0.11 | 0.12 | nd | 0.13 |
| p | 0.48 | 0.21 | 0.62 | 0.48 | 0.21 | 0.62 | 0.73 | nd | 0.63 |
| nCohort 1 | 98 | 159 | 84 | 98 | 159 | 84 | 98 | nd | 84 |
| nCohort 2 | 12 | 6 | 8 | 12 | 6 | 8 | 7 | nd | 6 |
| Cutoff 1 | 0.134 | 0.117 | 0.134 | 0.134 | 0.117 | 0.134 | 0.134 | nd | 0.117 |
| Sens 1 | 75% | 100% | 75% | 75% | 100% | 75% | 71% | nd | 100% |
| Spec 1 | 18% | 4% | 15% | 18% | 4% | 15% | 18% | nd | 4% |
| Cutoff 2 | 0.117 | 0.117 | 0.117 | 0.117 | 0.117 | 0.117 | 0.117 | nd | 0.117 |
| Sens 2 | 100% | 100% | 100% | 100% | 100% | 100% | 100% | nd | 100% |
| Spec 2 | 5% | 4% | 4% | 5% | 4% | 4% | 5% | nd | 4% |
| Cutoff 3 | 0.117 | 0.117 | 0.117 | 0.117 | 0.117 | 0.117 | 0.117 | nd | 0.117 |
| Sens 3 | 100% | 100% | 100% | 100% | 100% | 100% | 100% | nd | 100% |
| Spec 3 | 5% | 4% | 4% | 5% | 4% | 4% | 5% | nd | 4% |
| Cutoff 4 | 53.6 | 67.5 | 67.5 | 53.6 | 67.5 | 67.5 | 53.6 | nd | 67.5 |
| Sens 4 | 50% | 67% | 38% | 50% | 67% | 38% | 43% | nd | 33% |
| Spec 4 | 70% | 70% | 70% | 70% | 70% | 70% | 70% | nd | 70% |
| Cutoff 5 | 137 | 142 | 142 | 137 | 142 | 142 | 137 | nd | 142 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 5 | 42% | 67% | 12% | 42% | 67% | 12% | 43% | nd | 17% |
| Spec 5 | 81% | 81% | 81% | 81% | 81% | 81% | 81% | nd | 81% |
| Cutoff 6 | 415 | 440 | 347 | 415 | 440 | 347 | 415 | nd | 347 |
| Sens 6 | 33% | 67% | 12% | 33% | 67% | 12% | 29% | nd | 17% |
| Spec 6 | 91% | 91% | 90% | 91% | 91% | 90% | 91% | nd | 90% |
| OR Quart 2 | 0.21 | 0 | 1.0 | 0.21 | 0 | 1.0 | 0 | nd | 0.50 |
| p Value | 0.18 | na | 1.0 | 0.18 | na | 1.0 | na | nd | 0.58 |
| 95% CI of | 0.022 | na | 0.13 | 0.022 | na | 0.13 | na | nd | 0.042 |
| OR Quart2 | 2.0 | na | 7.8 | 2.0 | na | 7.8 | na | nd | 5.9 |
| OR Quart 3 | 0.46 | 0 | 0 | 0.46 | 0 | 0 | 0.31 | nd | 0 |
| p Value | 0.40 | na | na | 0.40 | na | na | 0.32 | nd | na |
| 95% CI of | 0.077 | na | na | 0.077 | na | na | 0.030 | nd | na |
| OR Quart3 | 2.8 | na | na | 2.8 | na | na | 3.2 | nd | na |
| OR Quart 4 | 1.2 | 2.1 | 2.2 | 1.2 | 2.1 | 2.2 | 0.96 | nd | 1.7 |
| p Value | 0.76 | 0.42 | 0.39 | 0.76 | 0.42 | 0.39 | 0.96 | nd | 0.60 |
| 95% CI of | 0.30 | 0.35 | 0.36 | 0.30 | 0.35 | 0.36 | 0.18 | nd | 0.25 |
| OR Quart4 | 5.3 | 12 | 13 | 5.3 | 12 | 13 | 5.2 | nd | 11 |

**Transthyretin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.8 | 90.5 | 24.8 | 74.2 | 24.8 | 70.4 |
| Average | 56.1 | 126 | 56.1 | 99.3 | 56.1 | 107 |
| Stdev | 86.2 | 112 | 86.2 | 86.9 | 86.2 | 109 |
| p(t-test) | | 2.4E-4 | | 0.018 | | 0.031 |
| Min | 0.164 | 3.29 | 0.164 | 3.29 | 0.164 | 11.6 |
| Max | 668 | 461 | 668 | 396 | 668 | 396 |
| n (Samp) | 151 | 28 | 151 | 27 | 151 | 16 |
| n (Patient) | 151 | 28 | 151 | 27 | 151 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 35.2 | 123 | 35.2 | 76.8 | 35.2 | 76.8 |
| Average | 72.2 | 133 | 72.2 | 119 | 72.2 | 117 |
| Stdev | 145 | 111 | 145 | 112 | 145 | 122 |
| p(t-test) | | 0.11 | | 0.22 | | 0.31 |
| Min | 0.164 | 3.29 | 0.164 | 3.29 | 0.164 | 11.6 |
| Max | 2000 | 396 | 2000 | 396 | 2000 | 396 |
| n (Samp) | 290 | 15 | 290 | 15 | 290 | 11 |
| n (Patient) | 290 | 15 | 290 | 15 | 290 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 32.6 | 90.5 | 32.6 | 74.2 | 32.6 | 70.4 |
| Average | 66.2 | 133 | 66.2 | 103 | 66.2 | 109 |
| Stdev | 95.3 | 123 | 95.3 | 89.7 | 95.3 | 117 |
| p(t-test) | | 0.0075 | | 0.13 | | 0.18 |
| Min | 0.411 | 13.8 | 0.411 | 13.8 | 0.411 | 13.8 |
| Max | 668 | 461 | 668 | 396 | 668 | 396 |
| n (Samp) | 155 | 18 | 155 | 17 | 155 | 10 |
| n (Patient) | 155 | 18 | 155 | 17 | 155 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.77 | 0.72 | 0.75 | 0.73 | 0.68 | 0.72 | 0.70 | 0.66 | 0.68 |
| SE | 0.055 | 0.076 | 0.069 | 0.058 | 0.078 | 0.072 | 0.076 | 0.091 | 0.096 |
| p | 9.2E-7 | 0.0035 | 2.2E-4 | 5.1E-5 | 0.021 | 0.0020 | 0.0073 | 0.086 | 0.064 |
| nCohort 1 | 151 | 290 | 155 | 151 | 290 | 155 | 151 | 290 | 155 |
| nCohort 2 | 28 | 15 | 18 | 27 | 15 | 17 | 16 | 11 | 10 |
| Cutoff 1 | 50.3 | 48.3 | 66.2 | 48.3 | 46.2 | 56.7 | 29.3 | 32.6 | 48.3 |
| Sens 1 | 71% | 73% | 72% | 70% | 73% | 71% | 75% | 73% | 70% |
| Spec 1 | 69% | 58% | 71% | 68% | 58% | 65% | 54% | 48% | 61% |
| Cutoff 2 | 41.3 | 46.2 | 48.3 | 31.2 | 31.2 | 48.3 | 25.6 | 26.5 | 29.3 |
| Sens 2 | 82% | 80% | 83% | 81% | 80% | 82% | 81% | 82% | 80% |
| Spec 2 | 64% | 58% | 61% | 56% | 47% | 61% | 52% | 41% | 49% |
| Cutoff 3 | 25.6 | 26.5 | 29.3 | 18.2 | 18.6 | 29.3 | 13.2 | 18.6 | 18.2 |
| Sens 3 | 93% | 93% | 94% | 93% | 93% | 94% | 94% | 91% | 90% |
| Spec 3 | 52% | 41% | 49% | 44% | 32% | 49% | 32% | 32% | 37% |
| Cutoff 4 | 51.9 | 67.7 | 64.9 | 51.9 | 67.7 | 64.9 | 51.9 | 67.7 | 64.9 |
| Sens 4 | 68% | 60% | 72% | 63% | 53% | 65% | 56% | 55% | 60% |
| Spec 4 | 70% | 70% | 70% | 70% | 70% | 70% | 70% | 70% | 70% |
| Cutoff 5 | 77.7 | 93.5 | 91.5 | 77.7 | 93.5 | 91.5 | 77.7 | 93.5 | 91.5 |
| Sens 5 | 50% | 53% | 50% | 44% | 47% | 41% | 38% | 36% | 40% |
| Spec 5 | 80% | 80% | 80% | 80% | 80% | 80% | 80% | 80% | 80% |
| Cutoff 6 | 123 | 145 | 187 | 123 | 145 | 187 | 123 | 145 | 187 |
| Sens 6 | 39% | 33% | 22% | 30% | 27% | 12% | 31% | 27% | 20% |
| Spec 6 | 90% | 90% | 90% | 90% | 90% | 90% | 90% | 90% | 90% |
| OR Quart 2 | 3.1 | 2.0 | 1.0 | 4.2 | 3.1 | 1.0 | >4.3 | 3.1 | >3.2 |
| p Value | 0.34 | 0.57 | 1.0 | 0.21 | 0.33 | 1.0 | <0.20 | 0.33 | <0.32 |
| 95% CI of OR Quart2 | 0.31 | 0.18 | 0.061 | 0.45 | 0.31 | 0.061 | >0.46 | 0.31 | >0.32 |
| | 31 | 23 | 17 | 39 | 30 | 17 | na | 30 | na |
| OR Quart 3 | 12 | 4.2 | 8.2 | 13 | 4.2 | 8.2 | >4.3 | 2.0 | >3.2 |
| p Value | 0.019 | 0.21 | 0.055 | 0.018 | 0.21 | 0.055 | <0.20 | 0.57 | <0.32 |
| 95% CI of OR Quart3 | 1.5 | 0.45 | 0.96 | 1.5 | 0.45 | 0.96 | >0.46 | 0.18 | >0.32 |
| | 100 | 38 | 70 | 100 | 38 | 70 | na | 23 | na |
| OR Quart 4 | 19 | 8.7 | 11 | 16 | 7.5 | 9.6 | >9.6 | 5.2 | >4.3 |
| p Value | 0.0052 | 0.044 | 0.027 | 0.0099 | 0.063 | 0.037 | <0.037 | 0.14 | <0.20 |
| 95% CI of OR Quart4 | 2.4 | 1.1 | 1.3 | 1.9 | 0.90 | 1.1 | >1.1 | 0.59 | >0.46 |
| | 160 | 71 | 89 | 130 | 63 | 81 | na | 46 | na |

**C-peptide Urine**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 37100 | 14300 | 37100 | 10800 | 37100 | 21600 |
| Average | 45400 | 26400 | 45400 | 26300 | 45400 | 37100 |
| Stdev | 31300 | 29400 | 31300 | 29900 | 31300 | 34400 |
| p(t-test) | | 0.0032 | | 0.0037 | | 0.32 |
| Min | 0.0119 | 940 | 0.0119 | 940 | 0.0119 | 1470 |
| Max | 102000 | 100000 | 102000 | 100000 | 102000 | 100000 |
| n (Samp) | 151 | 28 | 151 | 27 | 151 | 16 |
| n (Patient) | 151 | 28 | 151 | 27 | 151 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 35500 | 21000 | 35500 | 21000 | 35500 | 52900 |
| Average | 43700 | 36300 | 43700 | 35700 | 43700 | 45600 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 31500 | 34000 | 31500 | 34500 | 31500 | 35500 |
| p(t-test) | | 0.38 | | 0.34 | | 0.85 |
| Min | 0.0119 | 3420 | 0.0119 | 3420 | 0.0119 | 4640 |
| Max | 114000 | 100000 | 114000 | 100000 | 114000 | 100000 |
| n (Samp) | 290 | 15 | 290 | 15 | 290 | 11 |
| n (Patient) | 290 | 15 | 290 | 15 | 290 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 38100 | 7740 | 38100 | 8290 | 38100 | 13700 |
| Average | 44900 | 23200 | 44900 | 23300 | 44900 | 31600 |
| Stdev | 30700 | 31500 | 30700 | 32200 | 30700 | 39600 |
| p(t-test) | | 0.0053 | | 0.0070 | | 0.19 |
| Min | 0.0119 | 940 | 0.0119 | 940 | 0.0119 | 1470 |
| Max | 102000 | 122000 | 102000 | 122000 | 102000 | 122000 |
| n (Samp) | 155 | 18 | 155 | 17 | 155 | 10 |
| n (Patient) | 155 | 18 | 155 | 17 | 155 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.30 | 0.41 | 0.26 | 0.29 | 0.41 | 0.25 | 0.40 | 0.51 | 0.33 |
| SE | 0.058 | 0.079 | 0.069 | 0.059 | 0.079 | 0.071 | 0.078 | 0.089 | 0.096 |
| p | 5.3E-4 | 0.28 | 4.0E-4 | 5.2E-4 | 0.24 | 5.5E-4 | 0.22 | 0.94 | 0.078 |
| nCohort 1 | 151 | 290 | 155 | 151 | 290 | 155 | 151 | 290 | 155 |
| nCohort 2 | 28 | 15 | 18 | 27 | 15 | 17 | 16 | 11 | 10 |
| Cutoff 1 | 4980 | 10500 | 4270 | 5980 | 8240 | 4980 | 7180 | 20600 | 7180 |
| Sens 1 | 71% | 73% | 72% | 70% | 73% | 71% | 75% | 73% | 70% |
| Spec 1 | 6% | 17% | 5% | 6% | 12% | 5% | 9% | 32% | 8% |
| Cutoff 2 | 3420 | 8240 | 2020 | 4270 | 7180 | 2950 | 4980 | 8240 | 4980 |
| Sens 2 | 82% | 80% | 83% | 81% | 80% | 82% | 81% | 82% | 80% |
| Spec 2 | 4% | 12% | 2% | 5% | 10% | 3% | 6% | 12% | 5% |
| Cutoff 3 | 1930 | 4360 | 1630 | 1930 | 4360 | 1630 | 1630 | 5550 | 1630 |
| Sens 3 | 93% | 93% | 94% | 93% | 93% | 94% | 94% | 91% | 90% |
| Spec 3 | 2% | 6% | 2% | 2% | 6% | 2% | 2% | 7% | 2% |
| Cutoff 4 | 62300 | 60500 | 61000 | 62300 | 60500 | 61000 | 62300 | 60500 | 61000 |
| Sens 4 | 11% | 27% | 17% | 11% | 27% | 18% | 19% | 36% | 30% |
| Spec 4 | 70% | 70% | 70% | 70% | 70% | 70% | 70% | 70% | 70% |
| Cutoff 5 | 76500 | 74400 | 75900 | 76500 | 74400 | 75900 | 76500 | 74400 | 75900 |
| Sens 5 | 7% | 13% | 6% | 7% | 13% | 6% | 12% | 18% | 10% |
| Spec 5 | 80% | 80% | 80% | 80% | 80% | 80% | 80% | 80% | 80% |
| Cutoff 6 | 98500 | 100000 | 97900 | 98500 | 100000 | 97900 | 98500 | 100000 | 97900 |
| Sens 6 | 7% | 0% | 6% | 7% | 0% | 6% | 12% | 0% | 10% |
| Spec 6 | 90% | 99% | 90% | 90% | 99% | 90% | 90% | 99% | 90% |
| OR Quart 2 | 3.3 | 2.1 | 0.50 | 3.4 | 2.1 | 0.49 | 2.7 | 0.66 | 2.1 |
| p Value | 0.16 | 0.41 | 0.58 | 0.15 | 0.41 | 0.56 | 0.25 | 0.65 | 0.55 |
| 95% CI of | 0.63 | 0.37 | 0.044 | 0.65 | 0.37 | 0.043 | 0.49 | 0.11 | 0.18 |
| OR Quart2 | 17 | 12 | 5.7 | 18 | 12 | 5.6 | 15 | 4.1 | 24 |
| OR Quart 3 | 3.3 | 1.0 | 2.8 | 2.1 | 1.0 | 2.1 | 1.5 | 1.4 | 2.1 |
| p Value | 0.16 | 0.99 | 0.24 | 0.41 | 0.99 | 0.41 | 0.65 | 0.70 | 0.55 |
| 95% CI of | 0.63 | 0.14 | 0.51 | 0.36 | 0.14 | 0.36 | 0.24 | 0.29 | 0.18 |
| OR Quart3 | 17 | 7.4 | 15 | 12 | 7.4 | 12 | 9.7 | 6.3 | 24 |
| OR Quart 4 | 10 | 3.8 | 6.4 | 11 | 3.8 | 6.2 | 3.4 | 0.65 | 5.7 |
| p Value | 0.0036 | 0.10 | 0.022 | 0.0023 | 0.10 | 0.024 | 0.15 | 0.64 | 0.12 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of OR Quart4 | 2.1 47 | 0.76 19 | 1.3 31 | 2.4 52 | 0.76 19 | 1.3 30 | 0.65 18 | 0.11 4.0 | 0.64 51 |

**Gastric inhibitory polypeptide  Urine**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.599 | 1.40 | 0.599 | 1.39 | 0.599 | 1.24 |
| Average | 33.3 | 77.7 | 33.3 | 80.3 | 33.3 | 19.3 |
| Stdev | 315 | 225 | 315 | 229 | 315 | 62.2 |
| p(t-test) |  | 0.48 |  | 0.46 |  | 0.86 |
| Min | 0.000104 | 0.000165 | 0.000104 | 0.00873 | 0.000104 | 0.228 |
| Max | 3840 | 1040 | 3840 | 1040 | 3840 | 252 |
| n (Samp) | 151 | 28 | 151 | 27 | 151 | 16 |
| n (Patient) | 151 | 28 | 151 | 27 | 151 | 16 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.649 | 2.75 | 0.649 | 2.19 | 0.649 | 2.19 |
| Average | 41.1 | 64.5 | 41.1 | 64.1 | 41.1 | 27.5 |
| Stdev | 306 | 163 | 306 | 163 | 306 | 74.6 |
| p(t-test) |  | 0.77 |  | 0.77 |  | 0.88 |
| Min | 0.000104 | 0.00873 | 0.000104 | 0.00873 | 0.000104 | 0.375 |
| Max | 3840 | 606 | 3840 | 606 | 3840 | 252 |
| n (Samp) | 290 | 15 | 290 | 15 | 290 | 11 |
| n (Patient) | 290 | 15 | 290 | 15 | 290 | 11 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.711 | 0.963 | 0.711 | 0.990 | 0.711 | 0.773 |
| Average | 52.3 | 81.3 | 52.3 | 86.1 | 52.3 | 26.2 |
| Stdev | 373 | 248 | 373 | 255 | 373 | 79.3 |
| p(t-test) |  | 0.75 |  | 0.72 |  | 0.83 |
| Min | 0.000104 | 0.000165 | 0.000104 | 0.0429 | 0.000104 | 0.228 |
| Max | 3840 | 1040 | 3840 | 1040 | 3840 | 252 |
| n (Samp) | 155 | 18 | 155 | 17 | 155 | 10 |
| n (Patient) | 155 | 18 | 155 | 17 | 155 | 10 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.66 | 0.72 | 0.56 | 0.67 | 0.71 | 0.59 | 0.67 | 0.73 | 0.54 |
| SE | 0.060 | 0.076 | 0.074 | 0.060 | 0.077 | 0.076 | 0.077 | 0.088 | 0.096 |
| p | 0.0077 | 0.0031 | 0.45 | 0.0042 | 0.0076 | 0.25 | 0.026 | 0.010 | 0.69 |
| nCohort 1 | 151 | 290 | 155 | 151 | 290 | 155 | 151 | 290 | 155 |
| nCohort 2 | 28 | 15 | 18 | 27 | 15 | 17 | 16 | 11 | 10 |
| Cutoff 1 | 0.629 | 1.09 | 0.372 | 0.835 | 0.935 | 0.529 | 0.629 | 1.09 | 0.372 |
| Sens 1 | 71% | 73% | 72% | 70% | 73% | 71% | 75% | 73% | 70% |
| Spec 1 | 52% | 63% | 35% | 61% | 60% | 44% | 52% | 63% | 35% |
| Cutoff 2 | 0.372 | 0.935 | 0.292 | 0.529 | 0.897 | 0.320 | 0.599 | 0.935 | 0.320 |
| Sens 2 | 82% | 80% | 83% | 81% | 80% | 82% | 81% | 82% | 80% |
| Spec 2 | 40% | 60% | 30% | 47% | 59% | 32% | 50% | 60% | 32% |
| Cutoff 3 | 0.0384 | 0.372 | 0.0384 | 0.227 | 0.372 | 0.227 | 0.292 | 0.629 | 0.292 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 3 | 93% | 93% | 94% | 93% | 93% | 94% | 94% | 91% | 90% |
| Spec 3 | 16% | 36% | 12% | 29% | 36% | 25% | 35% | 50% | 30% |
| Cutoff 4 | 1.38 | 1.65 | 2.13 | 1.38 | 1.65 | 2.13 | 1.38 | 1.65 | 2.13 |
| Sens 4 | 54% | 60% | 33% | 52% | 53% | 35% | 50% | 55% | 30% |
| Spec 4 | 70% | 70% | 70% | 70% | 70% | 70% | 70% | 70% | 70% |
| Cutoff 5 | 3.75 | 3.58 | 5.24 | 3.75 | 3.58 | 5.24 | 3.75 | 3.58 | 5.24 |
| Sens 5 | 36% | 47% | 17% | 33% | 40% | 18% | 31% | 36% | 10% |
| Spec 5 | 80% | 80% | 80% | 80% | 80% | 80% | 80% | 80% | 80% |
| Cutoff 6 | 10.3 | 10.3 | 15.3 | 10.3 | 10.3 | 15.3 | 10.3 | 10.3 | 15.3 |
| Sens 6 | 25% | 33% | 17% | 26% | 33% | 18% | 25% | 36% | 10% |
| Spec 6 | 90% | 90% | 90% | 90% | 90% | 90% | 90% | 90% | 90% |
| OR Quart 2 | 2.1 | 2.0 | 1.8 | 3.2 | 2.0 | 2.7 | >5.5 | >2.1 | 4.3 |
| p Value | 0.32 | 0.57 | 0.46 | 0.17 | 0.57 | 0.25 | <0.13 | <0.56 | 0.20 |
| 95% CI of OR Quart2 | 0.49 9.0 | 0.18 23 | 0.39 7.9 | 0.62 17 | 0.18 23 | 0.49 15 | >0.62 na | >0.18 na | 0.46 40 |
| OR Quart 3 | 2.5 | 4.2 | 1.4 | 4.7 | 5.3 | 2.7 | >5.5 | >4.2 | 2.1 |
| p Value | 0.20 | 0.21 | 0.69 | 0.061 | 0.13 | 0.25 | <0.13 | <0.20 | 0.56 |
| 95% CI of OR Quart3 | 0.61 10 | 0.45 38 | 0.29 6.5 | 0.93 23 | 0.60 46 | 0.49 15 | >0.62 na | >0.46 na | 0.18 24 |
| OR Quart 4 | 5.0 | 8.7 | 2.1 | 6.8 | 7.5 | 2.7 | >6.8 | >5.3 | 3.1 |
| p Value | 0.020 | 0.044 | 0.32 | 0.017 | 0.063 | 0.25 | <0.082 | <0.13 | 0.34 |
| 95% CI of OR Quart4 | 1.3 19 | 1.1 71 | 0.49 9.0 | 1.4 33 | 0.90 63 | 0.49 15 | >0.79 na | >0.60 na | 0.31 31 |

**Peptide YY Urine**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 14.8 | 58.9 | 14.8 | 54.0 | 14.8 | 41.8 |
| Average | 49.6 | 98.3 | 49.6 | 97.9 | 49.6 | 72.1 |
| Stdev | 107 | 98.2 | 107 | 101 | 107 | 82.1 |
| p(t-test) |  | 0.026 |  | 0.030 |  | 0.41 |
| Min | 0.00976 | 0.0188 | 0.00976 | 0.0188 | 0.00976 | 0.0188 |
| Max | 936 | 317 | 936 | 317 | 936 | 290 |
| n (Samp) | 151 | 28 | 151 | 27 | 151 | 16 |
| n (Patient) | 151 | 28 | 151 | 27 | 151 | 16 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 18.5 | 81.5 | 18.5 | 81.5 | 18.5 | 46.1 |
| Average | 63.5 | 97.5 | 63.5 | 93.1 | 63.5 | 59.1 |
| Stdev | 147 | 75.6 | 147 | 79.1 | 147 | 48.9 |
| p(t-test) |  | 0.37 |  | 0.44 |  | 0.92 |
| Min | 0.00976 | 0.957 | 0.00976 | 0.0188 | 0.00976 | 0.0188 |
| Max | 1200 | 278 | 1200 | 278 | 1200 | 139 |
| n (Samp) | 290 | 15 | 290 | 15 | 290 | 11 |
| n (Patient) | 290 | 15 | 290 | 15 | 290 | 11 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 20.2 | 35.0 | 20.2 | 31.4 | 20.2 | 18.4 |
| Average | 51.3 | 85.5 | 51.3 | 88.0 | 51.3 | 69.9 |
| Stdev | 104 | 109 | 104 | 112 | 104 | 102 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.19 | | 0.17 | | 0.58 |
| Min | 0.00976 | 0.0188 | 0.00976 | 0.0188 | 0.00976 | 0.0188 |
| Max | 936 | 317 | 936 | 317 | 936 | 290 |
| n (Samp) | 155 | 18 | 155 | 17 | 155 | 10 |
| n (Patient) | 155 | 18 | 155 | 17 | 155 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.72 | 0.76 | 0.60 | 0.69 | 0.71 | 0.60 | 0.63 | 0.63 | 0.51 |
| SE | 0.058 | 0.073 | 0.074 | 0.060 | 0.076 | 0.076 | 0.078 | 0.092 | 0.095 |
| p | 1.9E-4 | 3.6E-4 | 0.17 | 0.0019 | 0.0051 | 0.20 | 0.094 | 0.17 | 0.92 |
| nCohort 1 | 151 | 290 | 155 | 151 | 290 | 155 | 151 | 290 | 155 |
| nCohort 2 | 28 | 15 | 18 | 27 | 15 | 17 | 16 | 11 | 10 |
| Cutoff 1 | 25.9 | 46.1 | 10.5 | 25.5 | 36.6 | 10.5 | 10.6 | 25.9 | 0.817 |
| Sens 1 | 71% | 73% | 72% | 70% | 73% | 71% | 75% | 73% | 80% |
| Spec 1 | 64% | 72% | 39% | 63% | 68% | 39% | 45% | 60% | 25% |
| Cutoff 2 | 10.6 | 36.6 | 1.71 | 2.87 | 25.9 | 1.71 | 0.817 | 18.6 | 0.817 |
| Sens 2 | 82% | 80% | 83% | 81% | 80% | 82% | 88% | 82% | 80% |
| Spec 2 | 45% | 68% | 32% | 38% | 60% | 32% | 26% | 51% | 25% |
| Cutoff 3 | 0.817 | 18.6 | 0.0188 | 0.817 | 0.817 | 0.0188 | 0.0131 | 0.0151 | 0.0188 |
| Sens 3 | 96% | 93% | 100% | 93% | 93% | 100% | 100% | 100% | 90% |
| Spec 3 | 26% | 51% | 6% | 26% | 27% | 6% | 5% | 6% | 6% |
| Cutoff 4 | 35.7 | 40.7 | 45.8 | 35.7 | 40.7 | 45.8 | 35.7 | 40.7 | 45.8 |
| Sens 4 | 64% | 73% | 39% | 59% | 67% | 41% | 56% | 55% | 30% |
| Spec 4 | 70% | 70% | 70% | 70% | 70% | 70% | 70% | 70% | 70% |
| Cutoff 5 | 59.4 | 67.7 | 63.6 | 59.4 | 67.7 | 63.6 | 59.4 | 67.7 | 63.6 |
| Sens 5 | 50% | 53% | 33% | 48% | 53% | 35% | 44% | 45% | 30% |
| Spec 5 | 80% | 80% | 80% | 80% | 80% | 80% | 80% | 80% | 80% |
| Cutoff 6 | 138 | 150 | 138 | 138 | 150 | 138 | 138 | 150 | 138 |
| Sens 6 | 29% | 20% | 28% | 30% | 20% | 29% | 19% | 0% | 30% |
| Spec 6 | 90% | 90% | 90% | 90% | 90% | 90% | 90% | 90% | 90% |
| OR Quart 2 | 5.4 | >2.1 | 2.7 | 1.3 | 2.0 | 1.4 | 1.5 | 0.49 | 1.5 |
| p Value | 0.13 | <0.56 | 0.25 | 0.72 | 0.57 | 0.69 | 0.67 | 0.57 | 0.65 |
| 95% CI of OR Quart2 | 0.60 | >0.18 | 0.49 | 0.28 | 0.18 | 0.29 | 0.24 | 0.044 | 0.24 |
| | 48 | na | 15 | 6.3 | 23 | 6.5 | 9.5 | 5.6 | 9.7 |
| OR Quart 3 | 9.3 | >4.2 | 2.7 | 2.6 | 4.2 | 1.4 | 2.1 | 1.5 | 1.0 |
| p Value | 0.040 | <0.20 | 0.25 | 0.19 | 0.21 | 0.69 | 0.42 | 0.65 | 1.0 |
| 95% CI of OR Quart3 | 1.1 | >0.46 | 0.49 | 0.62 | 0.45 | 0.29 | 0.35 | 0.25 | 0.13 |
| | 78 | na | 15 | 11 | 38 | 6.5 | 12 | 9.4 | 7.5 |
| OR Quart 4 | 19 | >10 | 3.2 | 5.6 | 8.7 | 2.2 | 3.9 | 2.6 | 1.5 |
| p Value | 0.0052 | <0.031 | 0.17 | 0.012 | 0.044 | 0.30 | 0.10 | 0.27 | 0.67 |
| 95% CI of OR Quart4 | 2.4 | >1.2 | 0.62 | 1.5 | 1.1 | 0.50 | 0.76 | 0.48 | 0.24 |
| | 160 | na | 17 | 21 | 71 | 9.3 | 20 | 14 | 9.5 |

Table 5: Comparison of marker levels in EDTA samples collected from Cohort 1 (patients that did not progress beyond RIFLE stage 0) and in EDTA samples collected from subjects at 0, 24 hours, and 48 hours prior to reaching stage R, I or F in Cohort 2.

**Agouti-related protein**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0547 | 0.0751 | 0.0547 | 0.0705 | 0.0547 | 0.0647 |
| Average | 0.0769 | 0.101 | 0.0769 | 0.0853 | 0.0769 | 0.0761 |
| Stdev | 0.0545 | 0.0797 | 0.0545 | 0.0725 | 0.0545 | 0.0539 |
| p(t-test) | | 0.014 | | 0.41 | | 0.96 |
| Min | 0.0115 | 0.00624 | 0.0115 | 0.00798 | 0.0115 | 0.0283 |
| Max | 0.285 | 0.527 | 0.285 | 0.485 | 0.285 | 0.221 |
| n (Samp) | 133 | 67 | 133 | 47 | 133 | 12 |
| n (Patient) | 65 | 67 | 65 | 47 | 65 | 12 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0613 | 0.0902 | 0.0613 | 0.0878 | 0.0613 | 0.138 |
| Average | 0.0813 | 0.119 | 0.0813 | 0.124 | 0.0813 | 0.146 |
| Stdev | 0.0619 | 0.122 | 0.0619 | 0.112 | 0.0619 | 0.101 |
| p(t-test) | | 0.019 | | 0.023 | | 0.0074 |
| Min | 0.00924 | 0.00624 | 0.00924 | 0.00798 | 0.00924 | 0.0415 |
| Max | 0.485 | 0.527 | 0.485 | 0.411 | 0.485 | 0.285 |
| n (Samp) | 326 | 18 | 326 | 12 | 326 | 7 |
| n (Patient) | 141 | 18 | 141 | 12 | 141 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0613 | 0.0818 | 0.0613 | 0.0707 | 0.0613 | 0.0622 |
| Average | 0.0802 | 0.109 | 0.0802 | 0.0896 | 0.0802 | 0.0788 |
| Stdev | 0.0531 | 0.0846 | 0.0531 | 0.0716 | 0.0531 | 0.0529 |
| p(t-test) | | 0.0030 | | 0.31 | | 0.92 |
| Min | 0.00624 | 0.0215 | 0.00624 | 0.0215 | 0.00624 | 0.0283 |
| Max | 0.285 | 0.527 | 0.285 | 0.485 | 0.285 | 0.221 |
| n (Samp) | 162 | 58 | 162 | 51 | 162 | 14 |
| n (Patient) | 72 | 58 | 72 | 51 | 72 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.60 | 0.61 | 0.55 | 0.64 | 0.54 | 0.51 | 0.69 | 0.50 |
| SE | 0.043 | 0.072 | 0.044 | 0.050 | 0.088 | 0.047 | 0.088 | 0.11 | 0.081 |
| p | 0.012 | 0.18 | 0.012 | 0.27 | 0.12 | 0.37 | 0.91 | 0.10 | 0.97 |
| nCohort 1 | 133 | 326 | 162 | 133 | 326 | 162 | 133 | 326 | 162 |
| nCohort 2 | 67 | 18 | 58 | 47 | 12 | 51 | 12 | 7 | 14 |
| Cutoff 1 | 0.0574 | 0.0596 | 0.0634 | 0.0497 | 0.0704 | 0.0498 | 0.0422 | 0.0616 | 0.0479 |
| Sens 1 | 70% | 72% | 71% | 70% | 75% | 71% | 75% | 71% | 71% |
| Spec 1 | 52% | 48% | 53% | 41% | 57% | 36% | 30% | 51% | 31% |
| Cutoff 2 | 0.0445 | 0.0399 | 0.0469 | 0.0434 | 0.0497 | 0.0442 | 0.0359 | 0.0422 | 0.0359 |
| Sens 2 | 81% | 83% | 81% | 81% | 83% | 80% | 83% | 86% | 86% |
| Spec 2 | 33% | 25% | 29% | 31% | 36% | 28% | 18% | 26% | 17% |
| Cutoff 3 | 0.0313 | 0.0300 | 0.0350 | 0.0305 | 0.0473 | 0.0369 | 0.0283 | 0.0413 | 0.0283 |
| Sens 3 | 91% | 94% | 91% | 91% | 92% | 90% | 92% | 100% | 93% |
| Spec 3 | 14% | 13% | 15% | 14% | 31% | 18% | 13% | 25% | 12% |
| Cutoff 4 | 0.0886 | 0.0886 | 0.100 | 0.0886 | 0.0886 | 0.100 | 0.0886 | 0.0886 | 0.100 |
| Sens 4 | 43% | 50% | 40% | 32% | 50% | 31% | 25% | 57% | 21% |
| Spec 4 | 71% | 70% | 70% | 71% | 70% | 70% | 71% | 70% | 70% |
| Cutoff 5 | 0.118 | 0.116 | 0.121 | 0.118 | 0.116 | 0.121 | 0.118 | 0.116 | 0.121 |
| Sens 5 | 28% | 28% | 33% | 19% | 25% | 25% | 17% | 57% | 14% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 5 | 80% | 80% | 80% | 80% | 80% | 80% | 80% | 80% | 80% |
| Cutoff 6 | 0.157 | 0.166 | 0.152 | 0.157 | 0.166 | 0.152 | 0.157 | 0.166 | 0.152 |
| Sens 6 | 18% | 11% | 22% | 4% | 25% | 12% | 8% | 43% | 7% |
| Spec 6 | 90% | 90% | 90% | 90% | 90% | 90% | 90% | 90% | 90% |
| OR Quart 2 | 0.89 | 0.49 | 1.6 | 2.2 | 2.0 | 1.3 | 0.65 | >2.0 | 1.4 |
| p Value | 0.81 | 0.41 | 0.34 | 0.13 | 0.57 | 0.63 | 0.65 | <0.56 | 0.69 |
| 95% CI of OR Quart2 | 0.35 | 0.087 | 0.61 | 0.79 | 0.18 | 0.49 | 0.10 | >0.18 | 0.29 |
| | 2.3 | 2.7 | 4.1 | 6.2 | 22 | 3.2 | 4.1 | na | 6.5 |
| OR Quart 3 | 2.7 | 1.0 | 2.1 | 2.5 | 5.3 | 1.5 | 1.8 | >1.0 | 1.4 |
| p Value | 0.023 | 1.0 | 0.12 | 0.086 | 0.13 | 0.36 | 0.46 | <0.99 | 0.69 |
| 95% CI of OR Quart3 | 1.1 | 0.24 | 0.83 | 0.88 | 0.60 | 0.62 | 0.39 | >0.062 | 0.29 |
| | 6.3 | 4.1 | 5.3 | 6.8 | 46 | 3.9 | 8.1 | na | 6.5 |
| OR Quart 4 | 2.3 | 2.1 | 2.9 | 2.2 | 4.1 | 1.7 | 0.63 | >4.2 | 1.0 |
| p Value | 0.058 | 0.24 | 0.020 | 0.13 | 0.21 | 0.28 | 0.62 | <0.21 | 1.0 |
| 95% CI of OR Quart4 | 0.97 | 0.61 | 1.2 | 0.79 | 0.45 | 0.67 | 0.099 | >0.45 | 0.19 |
| | 5.4 | 7.3 | 7.2 | 6.2 | 37 | 4.1 | 4.0 | na | 5.2 |

**Osteocalcin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1940 | 2060 | 1940 | 1710 | 1940 | 1520 |
| Average | 2410 | 2760 | 2410 | 2440 | 2410 | 1920 |
| Stdev | 2110 | 2520 | 2110 | 2350 | 2110 | 1740 |
| p(t-test) | | 0.22 | | 0.90 | | 0.23 |
| Min | 91.1 | 63.3 | 91.1 | 129 | 91.1 | 248 |
| Max | 14000 | 12800 | 14000 | 10900 | 14000 | 7760 |
| n (Samp) | 183 | 95 | 183 | 69 | 183 | 29 |
| n (Patient) | 107 | 95 | 107 | 69 | 107 | 29 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1960 | 1750 | 1960 | 1770 | 1960 | 2250 |
| Average | 2570 | 2820 | 2570 | 3150 | 2570 | 3180 |
| Stdev | 2350 | 3120 | 2350 | 2990 | 2350 | 2410 |
| p(t-test) | | 0.59 | | 0.29 | | 0.35 |
| Min | 0.729 | 129 | 0.729 | 129 | 0.729 | 263 |
| Max | 17700 | 12800 | 17700 | 10500 | 17700 | 7760 |
| n (Samp) | 451 | 27 | 451 | 19 | 451 | 13 |
| n (Patient) | 209 | 27 | 209 | 19 | 209 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2100 | 1980 | 2100 | 1430 | 2100 | 1440 |
| Average | 2710 | 2570 | 2710 | 2260 | 2710 | 1950 |
| Stdev | 2500 | 2190 | 2500 | 2210 | 2500 | 1680 |
| p(t-test) | | 0.64 | | 0.17 | | 0.13 |
| Min | 91.1 | 63.3 | 91.1 | 197 | 91.1 | 248 |
| Max | 14000 | 8900 | 14000 | 10900 | 14000 | 6730 |
| n (Samp) | 206 | 81 | 206 | 74 | 206 | 26 |
| n (Patient) | 104 | 81 | 104 | 74 | 104 | 26 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.52 | 0.47 | 0.49 | 0.46 | 0.54 | 0.42 | 0.41 | 0.58 | 0.40 |
| SE | 0.037 | 0.058 | 0.038 | 0.041 | 0.069 | 0.040 | 0.059 | 0.084 | 0.062 |
| p | 0.61 | 0.61 | 0.73 | 0.38 | 0.60 | 0.045 | 0.12 | 0.33 | 0.10 |
| nCohort 1 | 183 | 451 | 206 | 183 | 451 | 206 | 183 | 451 | 206 |
| nCohort 2 | 95 | 27 | 81 | 69 | 19 | 74 | 29 | 13 | 26 |
| Cutoff 1 | 1120 | 895 | 1120 | 1060 | 1180 | 951 | 684 | 1660 | 684 |
| Sens 1 | 71% | 70% | 70% | 71% | 74% | 70% | 72% | 77% | 73% |
| Spec 1 | 27% | 20% | 27% | 26% | 30% | 21% | 13% | 42% | 13% |
| Cutoff 2 | 878 | 813 | 883 | 865 | 1100 | 724 | 583 | 1020 | 622 |
| Sens 2 | 80% | 81% | 80% | 81% | 84% | 81% | 83% | 85% | 81% |
| Spec 2 | 19% | 16% | 18% | 18% | 27% | 13% | 11% | 24% | 11% |
| Cutoff 3 | 628 | 638 | 583 | 353 | 353 | 447 | 353 | 323 | 394 |
| Sens 3 | 91% | 93% | 90% | 91% | 95% | 91% | 93% | 92% | 92% |
| Spec 3 | 11% | 11% | 11% | 9% | 6% | 10% | 9% | 5% | 9% |
| Cutoff 4 | 2790 | 2810 | 2810 | 2790 | 2810 | 2810 | 2790 | 2810 | 2810 |
| Sens 4 | 33% | 22% | 33% | 25% | 32% | 23% | 24% | 38% | 27% |
| Spec 4 | 70% | 70% | 70% | 70% | 70% | 70% | 70% | 70% | 70% |
| Cutoff 5 | 3340 | 3720 | 3740 | 3340 | 3720 | 3740 | 3340 | 3720 | 3740 |
| Sens 5 | 24% | 22% | 22% | 22% | 26% | 19% | 17% | 38% | 12% |
| Spec 5 | 80% | 80% | 80% | 80% | 80% | 80% | 80% | 80% | 80% |
| Cutoff 6 | 4570 | 5090 | 6390 | 4570 | 5090 | 6390 | 4570 | 5090 | 6390 |
| Sens 6 | 16% | 19% | 10% | 19% | 21% | 8% | 10% | 15% | 4% |
| Spec 6 | 90% | 90% | 90% | 90% | 90% | 90% | 90% | 90% | 90% |
| OR Quart 2 | 0.75 | 1.2 | 0.81 | 0.69 | 2.4 | 0.84 | 0.68 | 0.33 | 0.47 |
| p Value | 0.43 | 0.76 | 0.57 | 0.39 | 0.21 | 0.67 | 0.54 | 0.34 | 0.31 |
| 95% CI of OR Quart2 | 0.37 1.5 | 0.39 3.6 | 0.39 1.7 | 0.30 1.6 | 0.60 9.5 | 0.36 1.9 | 0.20 2.3 | 0.034 3.2 | 0.11 2.0 |
| OR Quart 3 | 0.94 | 0.66 | 0.93 | 1.6 | 1.0 | 1.8 | 0.68 | 1.3 | 1.0 |
| p Value | 0.86 | 0.52 | 0.85 | 0.25 | 1.0 | 0.13 | 0.54 | 0.70 | 1.0 |
| 95% CI of OR Quart3 | 0.46 1.9 | 0.18 2.4 | 0.45 1.9 | 0.73 3.4 | 0.20 5.1 | 0.84 3.8 | 0.20 2.3 | 0.29 6.1 | 0.30 3.3 |
| OR Quart 4 | 1.2 | 1.7 | 1.1 | 1.3 | 2.0 | 1.8 | 1.9 | 1.7 | 2.0 |
| p Value | 0.53 | 0.30 | 0.81 | 0.55 | 0.32 | 0.13 | 0.21 | 0.48 | 0.20 |
| 95% CI of OR Quart4 | 0.63 2.5 | 0.61 5.0 | 0.53 2.2 | 0.58 2.8 | 0.50 8.3 | 0.84 3.8 | 0.69 5.3 | 0.40 7.3 | 0.70 5.9 |

**Complement factor H**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 182000 | 195000 | 182000 | 184000 | 182000 | 176000 |
| Average | 189000 | 187000 | 189000 | 193000 | 189000 | 193000 |
| Stdev | 66500 | 67800 | 66500 | 63800 | 66500 | 59000 |
| p(t-test) | | 0.86 | | 0.69 | | 0.83 |
| Min | 57700 | 14900 | 57700 | 101000 | 57700 | 75300 |
| Max | 486000 | 339000 | 486000 | 427000 | 486000 | 297000 |
| n (Samp) | 157 | 77 | 157 | 57 | 157 | 15 |
| n (Patient) | 89 | 77 | 89 | 57 | 89 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 183000 | 176000 | 183000 | 192000 | 183000 | 200000 |
| Average | 192000 | 169000 | 192000 | 187000 | 192000 | 221000 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 79700 | 66300 | 79700 | 58400 | 79700 | 74200 |
| p(t-test) | | 0.18 | | 0.84 | | 0.34 |
| Min | 14900 | 53600 | 14900 | 48200 | 14900 | 112000 |
| Max | 1070000 | 339000 | 1070000 | 285000 | 1070000 | 325000 |
| n (Samp) | 376 | 22 | 376 | 13 | 376 | 7 |
| n (Patient) | 178 | 22 | 178 | 13 | 178 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 184000 | 193000 | 184000 | 178000 | 184000 | 169000 |
| Average | 192000 | 188000 | 192000 | 189000 | 192000 | 184000 |
| Stdev | 67000 | 69200 | 67000 | 68000 | 67000 | 60400 |
| p(t-test) | | 0.68 | | 0.81 | | 0.64 |
| Min | 29300 | 14900 | 29300 | 53600 | 29300 | 75300 |
| Max | 486000 | 320000 | 486000 | 427000 | 486000 | 297000 |
| n (Samp) | 183 | 66 | 183 | 60 | 183 | 17 |
| n (Patient) | 91 | 66 | 91 | 60 | 91 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.42 | 0.51 | 0.52 | 0.53 | 0.48 | 0.54 | 0.63 | 0.47 |
| SE | 0.040 | 0.065 | 0.042 | 0.045 | 0.083 | 0.043 | 0.080 | 0.11 | 0.074 |
| p | 0.52 | 0.25 | 0.83 | 0.69 | 0.73 | 0.72 | 0.63 | 0.27 | 0.71 |
| nCohort 1 | 157 | 376 | 183 | 157 | 376 | 183 | 157 | 376 | 183 |
| nCohort 2 | 77 | 22 | 66 | 57 | 13 | 60 | 15 | 7 | 17 |
| Cutoff 1 | 154000 | 135000 | 143000 | 148000 | 178000 | 147000 | 162000 | 183000 | 159000 |
| Sens 1 | 70% | 73% | 71% | 70% | 77% | 70% | 73% | 71% | 71% |
| Spec 1 | 36% | 19% | 24% | 32% | 45% | 29% | 39% | 50% | 36% |
| Cutoff 2 | 133000 | 114000 | 128000 | 141000 | 149000 | 138000 | 159000 | 177000 | 154000 |
| Sens 2 | 81% | 82% | 80% | 81% | 85% | 80% | 80% | 86% | 82% |
| Spec 2 | 17% | 10% | 15% | 23% | 29% | 21% | 37% | 44% | 34% |
| Cutoff 3 | 89900 | 80000 | 93000 | 127000 | 141000 | 122000 | 154000 | 112000 | 115000 |
| Sens 3 | 91% | 91% | 91% | 91% | 92% | 90% | 93% | 100% | 94% |
| Spec 3 | 3% | 2% | 2% | 12% | 22% | 11% | 36% | 9% | 11% |
| Cutoff 4 | 208000 | 213000 | 214000 | 208000 | 213000 | 214000 | 208000 | 213000 | 214000 |
| Sens 4 | 39% | 27% | 35% | 32% | 15% | 27% | 27% | 43% | 24% |
| Spec 4 | 70% | 70% | 70% | 70% | 70% | 70% | 70% | 70% | 70% |
| Cutoff 5 | 236000 | 237000 | 241000 | 236000 | 237000 | 241000 | 236000 | 237000 | 241000 |
| Sens 5 | 21% | 5% | 26% | 23% | 15% | 17% | 20% | 43% | 18% |
| Spec 5 | 80% | 80% | 80% | 80% | 80% | 80% | 80% | 80% | 80% |
| Cutoff 6 | 281000 | 272000 | 281000 | 281000 | 272000 | 281000 | 281000 | 272000 | 281000 |
| Sens 6 | 8% | 5% | 9% | 9% | 15% | 10% | 13% | 29% | 12% |
| Spec 6 | 90% | 90% | 90% | 90% | 90% | 90% | 90% | 90% | 90% |
| OR Quart 2 | 0.45 | 1.3 | 0.49 | 0.98 | 1.5 | 1.0 | 8.2 | 0.99 | 0.48 |
| p Value | 0.059 | 0.72 | 0.097 | 0.95 | 0.65 | 1.0 | 0.055 | 0.99 | 0.41 |
| 95% CI of OR Quart2 | 0.20 | 0.33 | 0.21 | 0.41 | 0.25 | 0.43 | 0.96 | 0.061 | 0.084 |
| | 1.0 | 4.9 | 1.1 | 2.3 | 9.3 | 2.3 | 70 | 16 | 2.7 |
| OR Quart 3 | 1.1 | 1.3 | 0.85 | 1.1 | 3.1 | 1.2 | 3.1 | 2.0 | 2.2 |
| p Value | 0.85 | 0.73 | 0.69 | 0.83 | 0.17 | 0.67 | 0.33 | 0.57 | 0.23 |
| 95% CI of OR Quart3 | 0.51 | 0.33 | 0.39 | 0.47 | 0.62 | 0.52 | 0.31 | 0.18 | 0.61 |
| | 2.3 | 4.8 | 1.9 | 2.6 | 16 | 2.7 | 32 | 22 | 7.8 |
| OR Quart 4 | 1.0 | 2.1 | 0.98 | 0.98 | 0.99 | 1.2 | 4.3 | 3.0 | 0.73 |
| p Value | 0.90 | 0.24 | 0.95 | 0.95 | 0.99 | 0.64 | 0.20 | 0.34 | 0.70 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of OR Quart4 | 0.49 2.2 | 0.61 7.2 | 0.46 2.1 | 0.41 2.3 | 0.14 7.2 | 0.53 2.8 | 0.46 40 | 0.31 30 | 0.16 3.5 |

**Ciliary neurotrophic factor**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0767 | 0.00367 | 0.0767 | 0.0651 | 0.0767 | 0.0709 |
| Average | 0.404 | 0.151 | 0.404 | 0.177 | 0.404 | 0.186 |
| Stdev | 0.943 | 0.339 | 0.943 | 0.388 | 0.943 | 0.355 |
| p(t-test) |  | 0.035 |  | 0.11 |  | 0.43 |
| Min | 8.69E-5 | 8.69E-5 | 8.69E-5 | 8.69E-5 | 8.69E-5 | 0.000186 |
| Max | 5.36 | 2.02 | 5.36 | 2.52 | 5.36 | 1.29 |
| n (Samp) | 133 | 67 | 133 | 47 | 133 | 12 |
| n (Patient) | 65 | 67 | 65 | 47 | 65 | 12 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0129 | 0.0342 | 0.0129 | 0.165 | 0.0129 | 0.171 |
| Average | 0.228 | 0.370 | 0.228 | 0.308 | 0.228 | 0.199 |
| Stdev | 0.655 | 0.620 | 0.655 | 0.563 | 0.655 | 0.150 |
| p(t-test) |  | 0.37 |  | 0.68 |  | 0.90 |
| Min | 8.69E-5 | 8.69E-5 | 8.69E-5 | 0.000173 | 8.69E-5 | 0.0129 |
| Max | 5.36 | 2.22 | 5.36 | 2.02 | 5.36 | 0.397 |
| n (Samp) | 326 | 18 | 326 | 12 | 326 | 7 |
| n (Patient) | 141 | 18 | 141 | 12 | 141 | 7 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0651 | 0.00826 | 0.0651 | 0.0415 | 0.0651 | 0.136 |
| Average | 0.417 | 0.135 | 0.417 | 0.196 | 0.417 | 0.238 |
| Stdev | 1.02 | 0.315 | 1.02 | 0.458 | 1.02 | 0.335 |
| p(t-test) |  | 0.040 |  | 0.14 |  | 0.51 |
| Min | 8.69E-5 | 8.69E-5 | 8.69E-5 | 8.69E-5 | 8.69E-5 | 0.000186 |
| Max | 6.40 | 2.02 | 6.40 | 2.52 | 6.40 | 1.29 |
| n (Samp) | 162 | 58 | 162 | 51 | 162 | 14 |
| n (Patient) | 72 | 58 | 72 | 51 | 72 | 14 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.42 | 0.52 | 0.44 | 0.48 | 0.64 | 0.47 | 0.54 | 0.73 | 0.61 |
| SE | 0.043 | 0.071 | 0.045 | 0.049 | 0.088 | 0.047 | 0.089 | 0.11 | 0.083 |
| p | 0.066 | 0.74 | 0.18 | 0.68 | 0.11 | 0.58 | 0.63 | 0.035 | 0.17 |
| nCohort 1 | 133 | 326 | 162 | 133 | 326 | 162 | 133 | 326 | 162 |
| nCohort 2 | 67 | 18 | 58 | 47 | 12 | 51 | 12 | 7 | 14 |
| Cutoff 1 | 0.000179 | 0.000179 | 0.000179 | 0.00238 | 0.0233 | 0.00191 | 0.00367 | 0.128 | 0.0647 |
| Sens 1 | 76% | 78% | 76% | 74% | 75% | 73% | 83% | 71% | 71% |
| Spec 1 | 22% | 23% | 27% | 30% | 52% | 32% | 41% | 72% | 48% |
| Cutoff 2 | 0.000173 | 0 | 0.000173 | 0.000186 | 0.00238 | 0.000179 | 0.00367 | 0.0647 | 0.00367 |
| Sens 2 | 84% | 100% | 84% | 81% | 83% | 80% | 83% | 86% | 86% |
| Spec 2 | 14% | 0% | 17% | 26% | 35% | 27% | 41% | 54% | 44% |
| Cutoff 3 | 8.69E-5 | 0 | 8.69E-5 | 8.69E-5 | 0.00191 | 8.69E-5 | 0.00268 | 0.00367 | 0.00268 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 91% | 100% | 93% | 94% | 92% | 92% | 92% | 100% | 93% |
| Spec 3 | 5% | 0% | 9% | 5% | 34% | 9% | 34% | 49% | 36% |
| Cutoff 4 | 0.206 | 0.117 | 0.200 | 0.206 | 0.117 | 0.200 | 0.206 | 0.117 | 0.200 |
| Sens 4 | 16% | 44% | 16% | 17% | 58% | 18% | 25% | 71% | 43% |
| Spec 4 | 73% | 71% | 70% | 73% | 71% | 70% | 73% | 71% | 70% |
| Cutoff 5 | 0.397 | 0.206 | 0.361 | 0.397 | 0.206 | 0.361 | 0.397 | 0.206 | 0.361 |
| Sens 5 | 9% | 33% | 9% | 13% | 25% | 14% | 8% | 29% | 21% |
| Spec 5 | 80% | 82% | 80% | 80% | 82% | 80% | 80% | 82% | 80% |
| Cutoff 6 | 0.896 | 0.410 | 0.896 | 0.896 | 0.410 | 0.896 | 0.896 | 0.410 | 0.896 |
| Sens 6 | 4% | 28% | 3% | 2% | 17% | 4% | 8% | 0% | 7% |
| Spec 6 | 90% | 90% | 90% | 90% | 90% | 90% | 90% | 90% | 90% |
| OR Quart 2 | 2.0 | 0.27 | 2.3 | 2.3 | 2.0 | 1.8 | 5.6 | >1.0 | >6.9 |
| p Value | 0.13 | 0.11 | 0.076 | 0.095 | 0.57 | 0.22 | 0.12 | <0.99 | <0.079 |
| 95% CI of | 0.82 | 0.054 | 0.92 | 0.86 | 0.18 | 0.70 | 0.62 | >0.062 | >0.80 |
| OR Quart2 | 4.8 | 1.3 | 5.7 | 6.2 | 22 | 4.6 | 51 | na | na |
| OR Quart 3 | 2.4 | 0.27 | 2.5 | 2.1 | 3.1 | 2.6 | 4.4 | >3.1 | >4.4 |
| p Value | 0.054 | 0.11 | 0.050 | 0.15 | 0.34 | 0.043 | 0.20 | <0.33 | <0.19 |
| 95% CI of | 0.98 | 0.054 | 1.0 | 0.78 | 0.31 | 1.0 | 0.46 | >0.32 | >0.47 |
| OR Quart3 | 5.7 | 1.3 | 6.2 | 5.6 | 30 | 6.4 | 41 | na | na |
| OR Quart 4 | 2.0 | 1.0 | 1.7 | 1.3 | 6.3 | 1.2 | 2.0 | >3.1 | >4.4 |
| p Value | 0.13 | 1.0 | 0.24 | 0.60 | 0.092 | 0.77 | 0.58 | <0.34 | <0.19 |
| 95% CI of | 0.82 | 0.34 | 0.68 | 0.47 | 0.74 | 0.43 | 0.17 | >0.31 | >0.47 |
| OR Quart4 | 4.8 | 3.0 | 4.5 | 3.7 | 54 | 3.1 | 23 | na | na |

**Follitropin subunit beta**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 7.83 | 4.95 | 7.83 | 5.51 | 7.83 | 5.05 |
| Average | 15.9 | 10.3 | 15.9 | 12.7 | 15.9 | 9.59 |
| Stdev | 20.2 | 11.0 | 20.2 | 16.2 | 20.2 | 10.9 |
| p(t-test) | | 0.037 | | 0.33 | | 0.29 |
| Min | 0.138 | 0.316 | 0.138 | 0.0879 | 0.138 | 0.0600 |
| Max | 141 | 53.6 | 141 | 78.2 | 141 | 34.9 |
| n (Samp) | 133 | 67 | 133 | 47 | 133 | 12 |
| n (Patient) | 65 | 67 | 65 | 47 | 65 | 12 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.38 | 7.46 | 6.38 | 11.9 | 6.38 | 5.03 |
| Average | 12.8 | 12.7 | 12.8 | 18.1 | 12.8 | 7.09 |
| Stdev | 16.4 | 14.1 | 16.4 | 19.9 | 16.4 | 5.61 |
| p(t-test) | | 0.97 | | 0.28 | | 0.35 |
| Min | 0.0600 | 1.34 | 0.0600 | 1.30 | 0.0600 | 0.913 |
| Max | 141 | 53.6 | 141 | 61.8 | 141 | 16.6 |
| n (Samp) | 326 | 18 | 326 | 12 | 326 | 7 |
| n (Patient) | 141 | 18 | 141 | 12 | 141 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 7.19 | 4.90 | 7.19 | 5.55 | 7.19 | 7.16 |
| Average | 17.0 | 9.92 | 17.0 | 12.4 | 17.0 | 10.3 |
| Stdev | 23.8 | 10.5 | 23.8 | 15.1 | 23.8 | 10.2 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.029 | | 0.19 | | 0.29 |
| Min | 0.131 | 0.316 | 0.131 | 0.0879 | 0.131 | 0.0600 |
| Max | 141 | 48.5 | 141 | 78.2 | 141 | 34.9 |
| n (Samp) | 162 | 58 | 162 | 51 | 162 | 14 |
| n (Patient) | 72 | 58 | 72 | 51 | 72 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.42 | 0.53 | 0.43 | 0.44 | 0.58 | 0.47 | 0.40 | 0.45 | 0.45 |
| SE | 0.043 | 0.071 | 0.045 | 0.050 | 0.088 | 0.047 | 0.090 | 0.11 | 0.082 |
| p | 0.063 | 0.63 | 0.11 | 0.24 | 0.35 | 0.47 | 0.26 | 0.64 | 0.57 |
| nCohort 1 | 133 | 326 | 162 | 133 | 326 | 162 | 133 | 326 | 162 |
| nCohort 2 | 67 | 18 | 58 | 47 | 12 | 51 | 12 | 7 | 14 |
| Cutoff 1 | 3.22 | 3.48 | 3.18 | 2.95 | 3.44 | 3.65 | 2.83 | 4.13 | 4.13 |
| Sens 1 | 70% | 72% | 71% | 70% | 75% | 71% | 75% | 71% | 71% |
| Spec 1 | 23% | 33% | 25% | 22% | 32% | 29% | 20% | 37% | 33% |
| Cutoff 2 | 2.19 | 3.22 | 1.68 | 2.07 | 2.66 | 2.92 | 2.75 | 2.86 | 2.75 |
| Sens 2 | 81% | 83% | 81% | 81% | 83% | 80% | 83% | 86% | 86% |
| Spec 2 | 17% | 30% | 14% | 17% | 25% | 25% | 20% | 28% | 22% |
| Cutoff 3 | 1.39 | 2.34 | 1.39 | 1.02 | 2.07 | 1.06 | 0.138 | 0.905 | 0.141 |
| Sens 3 | 91% | 94% | 91% | 91% | 92% | 90% | 92% | 100% | 93% |
| Spec 3 | 10% | 23% | 12% | 7% | 21% | 7% | 1% | 8% | 2% |
| Cutoff 4 | 16.9 | 13.8 | 14.9 | 16.9 | 13.8 | 14.9 | 16.9 | 13.8 | 14.9 |
| Sens 4 | 22% | 28% | 26% | 28% | 50% | 31% | 17% | 14% | 21% |
| Spec 4 | 71% | 70% | 70% | 71% | 70% | 70% | 71% | 70% | 70% |
| Cutoff 5 | 25.7 | 21.1 | 25.9 | 25.7 | 21.1 | 25.9 | 25.7 | 21.1 | 25.9 |
| Sens 5 | 10% | 22% | 10% | 15% | 33% | 14% | 17% | 0% | 14% |
| Spec 5 | 80% | 80% | 80% | 80% | 80% | 80% | 80% | 80% | 80% |
| Cutoff 6 | 41.1 | 34.6 | 42.6 | 41.1 | 34.6 | 42.6 | 41.1 | 34.6 | 42.6 |
| Sens 6 | 1% | 11% | 2% | 6% | 17% | 6% | 0% | 0% | 0% |
| Spec 6 | 90% | 90% | 90% | 90% | 90% | 90% | 90% | 90% | 90% |
| OR Quart 2 | 1.0 | 3.7 | 1.0 | 1.3 | 0.65 | 1.3 | 1.6 | >3.1 | 2.7 |
| p Value | 1.0 | 0.11 | 1.0 | 0.62 | 0.64 | 0.61 | 0.62 | <0.32 | 0.25 |
| 95% CI of OR Quart2 | 0.42 2.4 | 0.75 18 | 0.40 2.5 | 0.49 3.3 | 0.11 4.0 | 0.51 3.2 | 0.25 10 | >0.32 na | 0.49 15 |
| OR Quart 3 | 1.6 | 2.6 | 1.7 | 1.0 | 1.0 | 1.4 | 1.0 | >3.1 | 1.5 |
| p Value | 0.29 | 0.26 | 0.20 | 1.0 | 1.0 | 0.46 | 0.98 | <0.32 | 0.65 |
| 95% CI of OR Quart3 | 0.68 3.7 | 0.49 14 | 0.74 4.1 | 0.37 2.7 | 0.20 5.1 | 0.57 3.5 | 0.14 7.7 | >0.32 na | 0.24 9.7 |
| OR Quart 4 | 1.7 | 2.0 | 1.5 | 1.8 | 1.3 | 1.3 | 2.8 | >1.0 | 2.1 |
| p Value | 0.21 | 0.41 | 0.38 | 0.24 | 0.71 | 0.61 | 0.23 | <0.99 | 0.41 |
| 95% CI of OR Quart4 | 0.74 4.0 | 0.37 11 | 0.62 3.5 | 0.69 4.5 | 0.29 6.1 | 0.51 3.2 | 0.51 16 | >0.063 na | 0.36 12 |

**Follistatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 810 | 772 | 810 | 778 | 810 | 1120 |
| Average | 2870 | 2730 | 2870 | 1970 | 2870 | 1960 |
| Stdev | 4740 | 4060 | 4740 | 3210 | 4740 | 2630 |
| p(t-test) | | 0.81 | | 0.15 | | 0.33 |
| Min | 0.0206 | 0.221 | 0.0206 | 0.0206 | 0.0206 | 5.88 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 19900 | 15300 | 19900 | 12100 | 19900 | 10900 |
| n (Samp) | 184 | 94 | 184 | 69 | 184 | 28 |
| n (Patient) | 107 | 94 | 107 | 69 | 107 | 28 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 816 | 689 | 816 | 808 | 816 | 962 |
| Average | 2990 | 2620 | 2990 | 2110 | 2990 | 842 |
| Stdev | 4680 | 4000 | 4680 | 3370 | 4680 | 538 |
| p(t-test) | | 0.69 | | 0.42 | | 0.11 |
| Min | 0.0206 | 13.2 | 0.0206 | 189 | 0.0206 | 1.35 |
| Max | 28900 | 11800 | 28900 | 11600 | 28900 | 1600 |
| n (Samp) | 451 | 28 | 451 | 19 | 451 | 12 |
| n (Patient) | 209 | 28 | 209 | 19 | 209 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 764 | 774 | 764 | 786 | 764 | 1110 |
| Average | 2910 | 2800 | 2910 | 1970 | 2910 | 1900 |
| Stdev | 4710 | 4250 | 4710 | 3170 | 4710 | 2720 |
| p(t-test) | | 0.86 | | 0.11 | | 0.28 |
| Min | 0.0206 | 0.000554 | 0.0206 | 0.0206 | 0.0206 | 5.88 |
| Max | 19900 | 15900 | 19900 | 12100 | 19900 | 10900 |
| n (Samp) | 209 | 80 | 209 | 74 | 209 | 27 |
| n (Patient) | 107 | 80 | 107 | 74 | 107 | 27 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | 0.48 | 0.48 | 0.48 | 0.52 | 0.49 | 0.61 | 0.46 | 0.57 |
| SE | 0.037 | 0.057 | 0.038 | 0.041 | 0.068 | 0.039 | 0.060 | 0.086 | 0.061 |
| p | 0.52 | 0.72 | 0.66 | 0.64 | 0.76 | 0.81 | 0.060 | 0.64 | 0.23 |
| nCohort 1 | 184 | 451 | 209 | 184 | 451 | 209 | 184 | 451 | 209 |
| nCohort 2 | 94 | 28 | 80 | 69 | 19 | 74 | 28 | 12 | 27 |
| Cutoff 1 | 497 | 493 | 456 | 601 | 583 | 594 | 855 | 600 | 778 |
| Sens 1 | 70% | 71% | 70% | 71% | 74% | 70% | 71% | 75% | 70% |
| Spec 1 | 26% | 28% | 26% | 33% | 33% | 37% | 54% | 35% | 51% |
| Cutoff 2 | 359 | 443 | 355 | 486 | 518 | 465 | 764 | 321 | 624 |
| Sens 2 | 81% | 82% | 80% | 81% | 84% | 81% | 82% | 83% | 81% |
| Spec 2 | 18% | 24% | 20% | 24% | 29% | 27% | 48% | 16% | 39% |
| Cutoff 3 | 10.8 | 352 | 6.62 | 247 | 247 | 195 | 609 | 1.29 | 195 |
| Sens 3 | 90% | 93% | 90% | 91% | 95% | 91% | 93% | 100% | 93% |
| Spec 3 | 9% | 18% | 9% | 14% | 14% | 14% | 33% | 2% | 14% |
| Cutoff 4 | 1230 | 1320 | 1330 | 1230 | 1320 | 1330 | 1230 | 1320 | 1330 |
| Sens 4 | 32% | 25% | 30% | 25% | 32% | 23% | 36% | 25% | 33% |
| Spec 4 | 70% | 70% | 70% | 70% | 70% | 70% | 70% | 70% | 70% |
| Cutoff 5 | 2320 | 6970 | 4140 | 2320 | 6970 | 4140 | 2320 | 6970 | 4140 |
| Sens 5 | 22% | 18% | 22% | 16% | 11% | 14% | 11% | 0% | 11% |
| Spec 5 | 80% | 80% | 80% | 80% | 80% | 80% | 80% | 80% | 80% |
| Cutoff 6 | 11500 | 10900 | 11600 | 11500 | 10900 | 11600 | 11500 | 10900 | 11600 |
| Sens 6 | 6% | 11% | 5% | 1% | 11% | 1% | 0% | 0% | 0% |
| Spec 6 | 90% | 90% | 90% | 90% | 90% | 90% | 90% | 90% | 90% |
| OR Quart 2 | 0.68 | 0.83 | 0.95 | 1.5 | 4.2 | 1.8 | 2.7 | 6.3 | 2.1 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value | 0.30 | 0.76 | 0.89 | 0.29 | 0.074 | 0.13 | 0.26 | 0.092 | 0.31 |
| 95% CI of OR Quart2 | 0.33 | 0.25 | 0.46 | 0.69 | 0.87 | 0.84 | 0.49 | 0.74 | 0.50 |
|  | 1.4 | 2.8 | 2.0 | 3.4 | 20 | 3.8 | 14 | 53 | 8.9 |
| OR Quart 3 | 0.94 | 1.9 | 0.77 | 1.8 | 1.5 | 1.7 | 9.2 | 2.0 | 4.8 |
| p Value | 0.86 | 0.21 | 0.48 | 0.15 | 0.65 | 0.18 | 0.0048 | 0.57 | 0.021 |
| 95% CI of OR Quart3 | 0.47 | 0.69 | 0.36 | 0.81 | 0.25 | 0.78 | 2.0 | 0.18 | 1.3 |
|  | 1.9 | 5.4 | 1.6 | 3.9 | 9.2 | 3.6 | 43 | 23 | 18 |
| OR Quart 4 | 1.1 | 1.0 | 1.1 | 1.1 | 3.1 | 0.93 | 3.9 | 3.1 | 2.1 |
| p Value | 0.81 | 0.99 | 0.81 | 0.80 | 0.17 | 0.87 | 0.10 | 0.33 | 0.31 |
| 95% CI of OR Quart4 | 0.55 | 0.32 | 0.53 | 0.49 | 0.61 | 0.41 | 0.77 | 0.32 | 0.50 |
|  | 2.2 | 3.2 | 2.2 | 2.6 | 16 | 2.1 | 20 | 30 | 8.9 |

**Vitamin D-binding protein**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 179000 | 181000 | 179000 | 150000 | 179000 | 170000 |
| Average | 201000 | 205000 | 201000 | 175000 | 201000 | 181000 |
| Stdev | 92200 | 109000 | 92200 | 90000 | 92200 | 65400 |
| p(t-test) |  | 0.80 |  | 0.060 |  | 0.42 |
| Min | 41500 | 24700 | 41500 | 54200 | 41500 | 48700 |
| Max | 563000 | 630000 | 563000 | 497000 | 563000 | 314000 |
| n (Samp) | 158 | 76 | 158 | 57 | 158 | 15 |
| n (Patient) | 88 | 76 | 88 | 57 | 88 | 15 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 172000 | 149000 | 172000 | 152000 | 172000 | 180000 |
| Average | 193000 | 200000 | 193000 | 159000 | 193000 | 218000 |
| Stdev | 89600 | 138000 | 89600 | 82100 | 89600 | 120000 |
| p(t-test) |  | 0.74 |  | 0.22 |  | 0.47 |
| Min | 41500 | 42000 | 41500 | 24700 | 41500 | 101000 |
| Max | 630000 | 507000 | 630000 | 354000 | 630000 | 442000 |
| n (Samp) | 378 | 21 | 378 | 11 | 378 | 7 |
| n (Patient) | 176 | 21 | 176 | 11 | 176 | 7 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 181000 | 185000 | 181000 | 151000 | 181000 | 170000 |
| Average | 201000 | 206000 | 201000 | 169000 | 201000 | 178000 |
| Stdev | 94200 | 108000 | 94200 | 86000 | 94200 | 62600 |
| p(t-test) |  | 0.70 |  | 0.020 |  | 0.32 |
| Min | 41500 | 24700 | 41500 | 50900 | 41500 | 48700 |
| Max | 563000 | 630000 | 563000 | 497000 | 563000 | 314000 |
| n (Samp) | 185 | 66 | 185 | 61 | 185 | 17 |
| n (Patient) | 90 | 66 | 90 | 61 | 90 | 17 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.50 | 0.45 | 0.51 | 0.39 | 0.38 | 0.38 | 0.47 | 0.55 | 0.46 |
| SE | 0.040 | 0.066 | 0.042 | 0.045 | 0.092 | 0.043 | 0.079 | 0.11 | 0.075 |
| p | 0.96 | 0.43 | 0.86 | 0.013 | 0.19 | 0.0072 | 0.68 | 0.68 | 0.55 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 158 | 378 | 185 | 158 | 378 | 185 | 158 | 378 | 185 |
| nCohort 2 | 76 | 21 | 66 | 57 | 11 | 61 | 15 | 7 | 17 |
| Cutoff 1 | 149000 | 133000 | 155000 | 128000 | 126000 | 128000 | 153000 | 154000 | 152000 |
| Sens 1 | 71% | 71% | 71% | 70% | 73% | 70% | 73% | 71% | 71% |
| Spec 1 | 30% | 24% | 36% | 17% | 20% | 22% | 30% | 37% | 32% |
| Cutoff 2 | 130000 | 88700 | 132000 | 107000 | 108000 | 101000 | 146000 | 107000 | 133000 |
| Sens 2 | 80% | 81% | 80% | 81% | 82% | 80% | 80% | 86% | 82% |
| Spec 2 | 19% | 5% | 25% | 7% | 11% | 9% | 30% | 11% | 26% |
| Cutoff 3 | 89100 | 72700 | 98100 | 92600 | 107000 | 89100 | 125000 | 101000 | 125000 |
| Sens 3 | 91% | 90% | 91% | 91% | 91% | 90% | 93% | 100% | 94% |
| Spec 3 | 3% | 2% | 9% | 4% | 11% | 6% | 16% | 10% | 19% |
| Cutoff 4 | 224000 | 212000 | 230000 | 224000 | 212000 | 230000 | 224000 | 212000 | 230000 |
| Sens 4 | 30% | 33% | 27% | 16% | 18% | 15% | 20% | 43% | 18% |
| Spec 4 | 70% | 70% | 70% | 70% | 70% | 70% | 70% | 70% | 70% |
| Cutoff 5 | 249000 | 242000 | 251000 | 249000 | 242000 | 251000 | 249000 | 242000 | 251000 |
| Sens 5 | 25% | 29% | 18% | 14% | 9% | 11% | 20% | 43% | 18% |
| Spec 5 | 80% | 80% | 80% | 80% | 80% | 80% | 80% | 80% | 80% |
| Cutoff 6 | 312000 | 300000 | 312000 | 312000 | 300000 | 312000 | 312000 | 300000 | 312000 |
| Sens 6 | 12% | 19% | 11% | 9% | 9% | 7% | 7% | 14% | 6% |
| Spec 6 | 91% | 90% | 90% | 91% | 90% | 90% | 91% | 90% | 90% |
| OR Quart 2 | 0.81 | 0.27 | 1.4 | 1.6 | 3.1 | 1.8 | 1.0 | 0.49 | 1.0 |
| p Value | 0.60 | 0.11 | 0.44 | 0.34 | 0.33 | 0.23 | 0.98 | 0.57 | 0.98 |
| 95% CI of OR Quart2 | 0.38 / 1.8 | 0.055 / 1.3 | 0.61 / 3.1 | 0.61 / 4.1 | 0.32 / 30 | 0.70 / 4.4 | 0.20 / 5.4 | 0.044 / 5.5 | 0.20 / 5.3 |
| OR Quart 3 | 0.73 | 0.70 | 1.4 | 1.8 | 2.0 | 2.6 | 2.2 | 0.49 | 3.0 |
| p Value | 0.43 | 0.55 | 0.44 | 0.24 | 0.56 | 0.035 | 0.28 | 0.57 | 0.12 |
| 95% CI of OR Quart3 | 0.34 / 1.6 | 0.21 / 2.3 | 0.61 / 3.1 | 0.68 / 4.5 | 0.18 / 23 | 1.1 / 6.3 | 0.52 / 9.5 | 0.044 / 5.5 | 0.74 / 12 |
| OR Quart 4 | 0.95 | 1.0 | 1.2 | 3.3 | 5.3 | 2.7 | 1.0 | 1.5 | 1.0 |
| p Value | 0.90 | 0.98 | 0.71 | 0.0099 | 0.13 | 0.031 | 0.98 | 0.66 | 0.98 |
| 95% CI of OR Quart4 | 0.45 / 2.0 | 0.34 / 3.0 | 0.51 / 2.7 | 1.3 / 8.1 | 0.60 / 46 | 1.1 / 6.5 | 0.20 / 5.4 | 0.25 / 9.2 | 0.20 / 5.3 |

**Glucagon**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 52.8 | 63.9 | 52.8 | 79.1 | 52.8 | 20200 |
| Average | 4350 | 10100 | 4350 | 8700 | 4350 | 20100 |
| Stdev | 12500 | 17600 | 12500 | 16600 | 12500 | 20400 |
| p(t-test) | | 0.045 | | 0.10 | | 5.7E-6 |
| Min | 4.03 | 20.4 | 4.03 | 20.9 | 4.03 | 14.6 |
| Max | 40100 | 40100 | 40100 | 40100 | 40100 | 40100 |
| n (Samp) | 84 | 36 | 84 | 42 | 84 | 26 |
| n (Patient) | 75 | 36 | 75 | 42 | 75 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 70.1 | 43.2 | 70.1 | 61.2 | 70.1 | 77.3 |
| Average | 9040 | 12100 | 9040 | 11000 | 9040 | 17800 |
| Stdev | 16700 | 19300 | 16700 | 18700 | 16700 | 21100 |
| p(t-test) | | 0.58 | | 0.71 | | 0.13 |
| Min | 1.01 | 21.2 | 1.01 | 11.1 | 1.01 | 14.6 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 40100 | 40100 | 40100 | 40100 | 40100 | 40100 |
| n (Samp) | 201 | 10 | 201 | 11 | 201 | 9 |
| n (Patient) | 132 | 10 | 132 | 11 | 132 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 60.1 | 70.7 | 60.1 | 77.3 | 60.1 | 216 |
| Average | 6020 | 10700 | 6020 | 9430 | 6020 | 18100 |
| Stdev | 14300 | 18000 | 14300 | 17100 | 14300 | 20400 |
| p(t-test) | | 0.16 | | 0.25 | | 0.0031 |
| Min | 4.03 | 20.4 | 4.03 | 20.9 | 4.03 | 14.6 |
| Max | 40100 | 40100 | 40100 | 40100 | 40100 | 40100 |
| n (Samp) | 74 | 30 | 74 | 43 | 74 | 20 |
| n (Patient) | 61 | 30 | 61 | 43 | 61 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.48 | 0.56 | 0.63 | 0.46 | 0.59 | 0.73 | 0.52 | 0.70 |
| SE | 0.058 | 0.095 | 0.063 | 0.054 | 0.091 | 0.055 | 0.061 | 0.100 | 0.071 |
| p | 0.11 | 0.86 | 0.32 | 0.018 | 0.67 | 0.11 | 1.3E-4 | 0.83 | 0.0058 |
| nCohort 1 | 84 | 201 | 74 | 84 | 201 | 74 | 84 | 201 | 74 |
| nCohort 2 | 36 | 10 | 30 | 42 | 11 | 43 | 26 | 9 | 20 |
| Cutoff 1 | 24.4 | 37.0 | 41.4 | 28.7 | 20.9 | 29.9 | 86.8 | 20.9 | 89.9 |
| Sens 1 | 72% | 70% | 70% | 71% | 91% | 72% | 73% | 89% | 70% |
| Spec 1 | 37% | 33% | 36% | 38% | 5% | 32% | 75% | 5% | 69% |
| Cutoff 2 | 20.4 | 20.9 | 20.4 | 24.4 | 20.9 | 23.7 | 28.4 | 20.9 | 71.5 |
| Sens 2 | 97% | 100% | 97% | 81% | 91% | 81% | 81% | 89% | 80% |
| Spec 2 | 10% | 5% | 7% | 37% | 5% | 30% | 38% | 5% | 59% |
| Cutoff 3 | 20.4 | 20.9 | 20.4 | 20.9 | 20.9 | 20.9 | 18.6 | 12.9 | 18.6 |
| Sens 3 | 97% | 100% | 97% | 98% | 91% | 98% | 96% | 100% | 95% |
| Spec 3 | 10% | 5% | 7% | 10% | 5% | 7% | 10% | 2% | 7% |
| Cutoff 4 | 79.0 | 167 | 98.8 | 79.0 | 167 | 98.8 | 79.0 | 167 | 98.8 |
| Sens 4 | 44% | 40% | 43% | 50% | 36% | 44% | 73% | 44% | 65% |
| Spec 4 | 70% | 70% | 70% | 70% | 70% | 70% | 70% | 70% | 70% |
| Cutoff 5 | 143 | 40100 | 154 | 143 | 40100 | 154 | 143 | 40100 | 154 |
| Sens 5 | 36% | 0% | 37% | 36% | 0% | 37% | 54% | 0% | 50% |
| Spec 5 | 81% | 100% | 81% | 81% | 100% | 81% | 81% | 100% | 81% |
| Cutoff 6 | 40100 | 40100 | 40100 | 40100 | 40100 | 40100 | 40100 | 40100 | 40100 |
| Sens 6 | 0% | 0% | 0% | 0% | 0% | 0% | 0% | 0% | 0% |
| Spec 6 | 100% | 100% | 100% | 100% | 100% | 100% | 100% | 100% | 100% |
| OR Quart 2 | 0.85 | 0.32 | 1.0 | 3.1 | 0.65 | 1.4 | 0.44 | 0.31 | 0 |
| p Value | 0.77 | 0.33 | 1.0 | 0.062 | 0.65 | 0.57 | 0.37 | 0.32 | na |
| 95% CI of OR Quart2 | 0.28 2.6 | 0.032 3.2 | 0.29 3.4 | 0.94 10 | 0.10 4.1 | 0.45 4.2 | 0.074 2.6 | 0.032 3.1 | na na |
| OR Quart 3 | 0.58 | 1.0 | 0.65 | 2.5 | 0.32 | 1.2 | 1.6 | 0.32 | 1.7 |
| p Value | 0.37 | 1.0 | 0.51 | 0.14 | 0.33 | 0.77 | 0.49 | 0.33 | 0.48 |
| 95% CI of OR Quart3 | 0.18 1.9 | 0.19 5.2 | 0.18 2.4 | 0.73 8.4 | 0.032 3.2 | 0.38 3.7 | 0.41 6.6 | 0.032 3.2 | 0.40 7.0 |
| OR Quart 4 | 1.8 | 1.0 | 2.0 | 4.6 | 1.7 | 3.0 | 5.8 | 1.3 | 3.4 |
| p Value | 0.29 | 0.98 | 0.25 | 0.012 | 0.47 | 0.047 | 0.0081 | 0.72 | 0.076 |
| 95% CI of OR Quart4 | 0.62 5.2 | 0.20 5.3 | 0.62 6.4 | 1.4 15 | 0.39 7.7 | 1.0 8.9 | 1.6 21 | 0.28 6.3 | 0.88 13 |

**Glucagon-like peptide 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 57.2 | 55.4 | 57.2 | 66.8 | 57.2 | 77.6 |
| Average | 60.4 | 65.6 | 60.4 | 78.5 | 60.4 | 89.1 |
| Stdev | 39.7 | 46.1 | 39.7 | 58.6 | 39.7 | 59.0 |
| p(t-test) | | 0.53 | | 0.042 | | 0.0052 |
| Min | 2.37 | 3.01 | 2.37 | 3.01 | 2.37 | 2.37 |
| Max | 185 | 208 | 185 | 197 | 185 | 205 |
| n (Samp) | 84 | 36 | 84 | 42 | 84 | 26 |
| n (Patient) | 75 | 36 | 75 | 42 | 75 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 57.8 | 70.1 | 57.8 | 103 | 57.8 | 90.6 |
| Average | 67.2 | 71.7 | 67.2 | 89.6 | 67.2 | 90.9 |
| Stdev | 48.4 | 35.4 | 48.4 | 57.2 | 48.4 | 62.1 |
| p(t-test) | | 0.77 | | 0.14 | | 0.16 |
| Min | 2.37 | 18.7 | 2.37 | 13.1 | 2.37 | 9.67 |
| Max | 240 | 134 | 240 | 171 | 240 | 186 |
| n (Samp) | 201 | 10 | 201 | 11 | 201 | 9 |
| n (Patient) | 132 | 10 | 132 | 11 | 132 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 50.6 | 50.8 | 50.6 | 66.3 | 50.6 | 74.1 |
| Average | 58.2 | 64.5 | 58.2 | 76.8 | 58.2 | 85.7 |
| Stdev | 41.1 | 50.1 | 41.1 | 58.1 | 41.1 | 63.5 |
| p(t-test) | | 0.51 | | 0.046 | | 0.021 |
| Min | 2.37 | 3.01 | 2.37 | 3.01 | 2.37 | 2.37 |
| Max | 185 | 208 | 185 | 202 | 185 | 205 |
| n (Samp) | 74 | 30 | 74 | 43 | 74 | 20 |
| n (Patient) | 61 | 30 | 61 | 43 | 61 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.57 | 0.52 | 0.56 | 0.61 | 0.57 | 0.65 | 0.61 | 0.63 |
| SE | 0.058 | 0.096 | 0.063 | 0.055 | 0.093 | 0.056 | 0.065 | 0.10 | 0.074 |
| p | 0.66 | 0.46 | 0.78 | 0.29 | 0.23 | 0.20 | 0.019 | 0.27 | 0.084 |
| nCohort 1 | 84 | 201 | 74 | 84 | 201 | 74 | 84 | 201 | 74 |
| nCohort 2 | 36 | 10 | 30 | 42 | 11 | 43 | 26 | 9 | 20 |
| Cutoff 1 | 35.0 | 60.4 | 34.8 | 33.1 | 47.5 | 33.1 | 45.2 | 32.0 | 44.0 |
| Sens 1 | 72% | 70% | 70% | 71% | 73% | 72% | 73% | 78% | 70% |
| Spec 1 | 27% | 53% | 31% | 24% | 44% | 28% | 45% | 23% | 49% |
| Cutoff 2 | 31.2 | 44.0 | 27.0 | 22.9 | 32.0 | 22.9 | 31.2 | 17.7 | 27.0 |
| Sens 2 | 81% | 80% | 80% | 81% | 82% | 81% | 81% | 89% | 80% |
| Spec 2 | 23% | 39% | 22% | 14% | 23% | 16% | 23% | 11% | 22% |
| Cutoff 3 | 14.6 | 32.4 | 14.6 | 17.7 | 24.0 | 17.7 | 9.67 | 6.97 | 9.67 |
| Sens 3 | 92% | 90% | 90% | 90% | 91% | 91% | 92% | 100% | 90% |
| Spec 3 | 10% | 24% | 11% | 10% | 17% | 11% | 8% | 8% | 9% |
| Cutoff 4 | 71.7 | 78.1 | 68.3 | 71.7 | 78.1 | 68.3 | 71.7 | 78.1 | 68.3 |
| Sens 4 | 33% | 40% | 33% | 43% | 55% | 47% | 65% | 56% | 60% |
| Spec 4 | 70% | 70% | 70% | 70% | 70% | 70% | 70% | 70% | 70% |
| Cutoff 5 | 81.8 | 96.7 | 79.8 | 81.8 | 96.7 | 79.8 | 81.8 | 96.7 | 79.8 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 5 | 33% | 20% | 33% | 38% | 55% | 37% | 42% | 44% | 40% |
| Spec 5 | 81% | 80% | 81% | 81% | 80% | 81% | 81% | 80% | 81% |
| Cutoff 6 | 119 | 138 | 119 | 119 | 138 | 119 | 119 | 138 | 119 |
| Sens 6 | 8% | 0% | 10% | 29% | 18% | 26% | 35% | 33% | 30% |
| Spec 6 | 90% | 90% | 91% | 90% | 90% | 91% | 90% | 90% | 91% |
| OR Quart 2 | 1.2 | 0.48 | 0.83 | 0.61 | 0.65 | 1.0 | 0.42 | 0 | 0.51 |
| p Value | 0.78 | 0.55 | 0.76 | 0.37 | 0.65 | 1.0 | 0.26 | na | 0.40 |
| 95% CI of OR Quart2 | 0.39 | 0.042 | 0.25 | 0.20 | 0.10 | 0.34 | 0.093 | na | 0.11 |
| | 3.5 | 5.5 | 2.8 | 1.8 | 4.1 | 3.0 | 1.9 | na | 2.5 |
| OR Quart 3 | 0.47 | 2.6 | 0.54 | 0.53 | 0 | 0.60 | 1.0 | 0.65 | 0.54 |
| p Value | 0.23 | 0.27 | 0.34 | 0.27 | na | 0.39 | 1.0 | 0.65 | 0.44 |
| 95% CI of OR Quart3 | 0.14 | 0.48 | 0.15 | 0.17 | na | 0.19 | 0.28 | 0.10 | 0.11 |
| | 1.6 | 14 | 1.9 | 1.6 | na | 1.9 | 3.6 | 4.1 | 2.6 |
| OR Quart 4 | 1.6 | 0.98 | 1.4 | 1.8 | 2.1 | 2.2 | 2.3 | 1.3 | 2.2 |
| p Value | 0.42 | 0.98 | 0.56 | 0.25 | 0.30 | 0.15 | 0.18 | 0.72 | 0.24 |
| 95% CI of OR Quart4 | 0.53 | 0.13 | 0.45 | 0.66 | 0.50 | 0.76 | 0.69 | 0.28 | 0.59 |
| | 4.5 | 7.2 | 4.4 | 5.0 | 9.0 | 6.2 | 7.4 | 6.3 | 7.8 |

**Appetite-regulating hormone**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 23.4 | 21.8 | 23.4 | 24.3 | 23.4 | 26.1 |
| Average | 28.8 | 28.8 | 28.8 | 31.6 | 28.8 | 33.2 |
| Stdev | 26.4 | 21.3 | 26.4 | 22.8 | 26.4 | 22.3 |
| p(t-test) | | 1.00 | | 0.56 | | 0.45 |
| Min | 0.251 | 3.18 | 0.251 | 1.29 | 0.251 | 0.251 |
| Max | 195 | 86.6 | 195 | 92.2 | 195 | 81.4 |
| n (Samp) | 84 | 36 | 84 | 42 | 84 | 26 |
| n (Patient) | 75 | 36 | 75 | 42 | 75 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.1 | 28.4 | 24.1 | 33.7 | 24.1 | 21.3 |
| Average | 30.4 | 30.4 | 30.4 | 33.7 | 30.4 | 33.2 |
| Stdev | 24.2 | 20.0 | 24.2 | 27.0 | 24.2 | 28.2 |
| p(t-test) | | 1.00 | | 0.66 | | 0.74 |
| Min | 0.251 | 6.94 | 0.251 | 6.30 | 0.251 | 1.29 |
| Max | 195 | 65.5 | 195 | 92.2 | 195 | 91.4 |
| n (Samp) | 201 | 10 | 201 | 11 | 201 | 9 |
| n (Patient) | 132 | 10 | 132 | 11 | 132 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22.9 | 19.9 | 22.9 | 24.8 | 22.9 | 26.1 |
| Average | 26.4 | 30.2 | 26.4 | 30.4 | 26.4 | 33.3 |
| Stdev | 21.6 | 25.1 | 21.6 | 21.5 | 21.6 | 24.2 |
| p(t-test) | | 0.44 | | 0.33 | | 0.22 |
| Min | 0.251 | 3.18 | 0.251 | 1.29 | 0.251 | 0.251 |
| Max | 119 | 90.4 | 119 | 91.4 | 119 | 81.4 |
| n (Samp) | 74 | 30 | 74 | 43 | 74 | 20 |
| n (Patient) | 61 | 30 | 61 | 43 | 61 | 20 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.50 | 0.53 | 0.52 | 0.54 | 0.51 | 0.56 | 0.58 | 0.51 | 0.60 |
| SE | 0.058 | 0.095 | 0.063 | 0.055 | 0.090 | 0.056 | 0.066 | 0.099 | 0.074 |
| p | 0.97 | 0.79 | 0.78 | 0.49 | 0.88 | 0.31 | 0.22 | 0.92 | 0.20 |
| nCohort 1 | 84 | 201 | 74 | 84 | 201 | 74 | 84 | 201 | 74 |
| nCohort 2 | 36 | 10 | 30 | 42 | 11 | 43 | 26 | 9 | 20 |
| Cutoff 1 | 14.6 | 22.1 | 14.6 | 12.6 | 11.0 | 13.2 | 20.1 | 17.9 | 20.1 |
| Sens 1 | 72% | 70% | 70% | 71% | 73% | 72% | 73% | 78% | 70% |
| Spec 1 | 26% | 43% | 30% | 23% | 16% | 30% | 39% | 34% | 45% |
| Cutoff 2 | 9.67 | 9.67 | 9.67 | 11.0 | 9.67 | 11.1 | 17.9 | 8.24 | 16.8 |
| Sens 2 | 83% | 80% | 80% | 81% | 82% | 81% | 81% | 89% | 80% |
| Spec 2 | 17% | 15% | 20% | 18% | 15% | 26% | 33% | 13% | 35% |
| Cutoff 3 | 6.30 | 8.02 | 6.30 | 7.35 | 8.65 | 7.35 | 3.18 | 0.251 | 3.18 |
| Sens 3 | 92% | 90% | 90% | 90% | 91% | 91% | 92% | 100% | 90% |
| Spec 3 | 8% | 12% | 11% | 11% | 14% | 14% | 5% | 2% | 7% |
| Cutoff 4 | 30.7 | 38.0 | 28.8 | 30.7 | 38.0 | 28.8 | 30.7 | 38.0 | 28.8 |
| Sens 4 | 42% | 30% | 40% | 45% | 45% | 40% | 38% | 33% | 35% |
| Spec 4 | 70% | 70% | 70% | 70% | 70% | 70% | 70% | 70% | 70% |
| Cutoff 5 | 41.0 | 47.5 | 37.2 | 41.0 | 47.5 | 37.2 | 41.0 | 47.5 | 37.2 |
| Sens 5 | 25% | 20% | 30% | 38% | 27% | 35% | 27% | 22% | 35% |
| Spec 5 | 81% | 80% | 81% | 81% | 80% | 81% | 81% | 80% | 81% |
| Cutoff 6 | 51.9 | 55.1 | 51.9 | 51.9 | 55.1 | 51.9 | 51.9 | 55.1 | 51.9 |
| Sens 6 | 17% | 20% | 20% | 19% | 18% | 19% | 27% | 22% | 30% |
| Spec 6 | 90% | 90% | 91% | 90% | 90% | 91% | 90% | 90% | 91% |
| OR Quart 2 | 1.2 | 0.31 | 1.7 | 0.53 | 0 | 1.2 | 1.9 | 1.5 | 0.68 |
| p Value | 0.78 | 0.32 | 0.38 | 0.25 | na | 0.78 | 0.35 | 0.66 | 0.64 |
| 95% CI of | 0.39 | 0.032 | 0.53 | 0.18 | na | 0.39 | 0.49 | 0.24 | 0.13 |
| OR Quart2 | 3.5 | 3.1 | 5.5 | 1.6 | na | 3.5 | 7.5 | 9.4 | 3.4 |
| OR Quart 3 | 0.71 | 1.3 | 0.35 | 0.38 | 0.38 | 1.0 | 1.6 | 1.0 | 1.7 |
| p Value | 0.56 | 0.72 | 0.17 | 0.098 | 0.26 | 1.0 | 0.49 | 1.0 | 0.48 |
| 95% CI of | 0.22 | 0.28 | 0.080 | 0.12 | 0.070 | 0.33 | 0.41 | 0.14 | 0.40 |
| OR Quart3 | 2.2 | 6.3 | 1.6 | 1.2 | 2.0 | 3.0 | 6.6 | 7.4 | 7.0 |
| OR Quart 4 | 1.2 | 0.64 | 1.7 | 1.6 | 0.78 | 2.2 | 2.7 | 0.98 | 2.0 |
| p Value | 0.78 | 0.63 | 0.38 | 0.37 | 0.73 | 0.14 | 0.14 | 0.98 | 0.34 |
| 95% CI of | 0.39 | 0.10 | 0.53 | 0.58 | 0.20 | 0.77 | 0.72 | 0.13 | 0.49 |
| OR Quart4 | 3.5 | 4.0 | 5.5 | 4.3 | 3.1 | 6.4 | 10 | 7.2 | 7.9 |

**Islet amyloid polypeptide**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 9.07 | 8.98 | 9.07 | 7.32 | 9.07 | 10.1 |
| Average | 14.5 | 14.3 | 14.5 | 11.0 | 14.5 | 11.0 |
| Stdev | 19.8 | 16.4 | 19.8 | 11.2 | 19.8 | 7.80 |
| p(t-test) |  | 0.96 |  | 0.29 |  | 0.38 |
| Min | 0.0141 | 0.0166 | 0.0141 | 0.0141 | 0.0141 | 0.0141 |
| Max | 121 | 81.1 | 121 | 39.6 | 121 | 23.7 |
| n (Samp) | 84 | 36 | 84 | 42 | 84 | 26 |
| n (Patient) | 75 | 36 | 75 | 42 | 75 | 26 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 8.70 | 18.1 | 8.70 | 6.92 | 8.70 | 10.5 |
| Average | 13.0 | 24.3 | 13.0 | 13.5 | 13.0 | 11.5 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 15.3 | 24.4 | 15.3 | 15.0 | 15.3 | 7.40 |
| p(t-test) | | 0.028 | | 0.91 | | 0.77 |
| Min | 0.0141 | 0.175 | 0.0141 | 0.0141 | 0.0141 | 0.0141 |
| Max | 121 | 81.1 | 121 | 39.6 | 121 | 23.5 |
| n (Samp) | 201 | 10 | 201 | 11 | 201 | 9 |
| n (Patient) | 132 | 10 | 132 | 11 | 132 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.44 | 7.87 | 9.44 | 7.67 | 9.44 | 6.33 |
| Average | 12.2 | 11.2 | 12.2 | 9.98 | 12.2 | 9.89 |
| Stdev | 13.3 | 11.4 | 13.3 | 9.45 | 13.3 | 8.09 |
| p(t-test) | | 0.72 | | 0.34 | | 0.46 |
| Min | 0.0141 | 0.0166 | 0.0141 | 0.0141 | 0.0141 | 0.0141 |
| Max | 70.6 | 49.8 | 70.6 | 36.4 | 70.6 | 23.7 |
| n (Samp) | 74 | 30 | 74 | 43 | 74 | 20 |
| n (Patient) | 61 | 30 | 61 | 43 | 61 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.65 | 0.48 | 0.43 | 0.46 | 0.44 | 0.50 | 0.55 | 0.47 |
| SE | 0.058 | 0.096 | 0.063 | 0.055 | 0.091 | 0.056 | 0.065 | 0.10 | 0.074 |
| p | 0.92 | 0.12 | 0.73 | 0.22 | 0.70 | 0.27 | 0.99 | 0.65 | 0.64 |
| nCohort 1 | 84 | 201 | 74 | 84 | 201 | 74 | 84 | 201 | 74 |
| nCohort 2 | 36 | 10 | 30 | 42 | 11 | 43 | 26 | 9 | 20 |
| Cutoff 1 | 6.02 | 8.70 | 5.53 | 3.02 | 2.53 | 3.73 | 4.92 | 9.79 | 4.41 |
| Sens 1 | 72% | 70% | 70% | 71% | 73% | 72% | 73% | 78% | 70% |
| Spec 1 | 32% | 50% | 32% | 13% | 14% | 20% | 24% | 56% | 26% |
| Cutoff 2 | 3.53 | 8.19 | 3.53 | 2.01 | 0.466 | 2.01 | 3.63 | 2.88 | 2.81 |
| Sens 2 | 81% | 80% | 80% | 81% | 82% | 81% | 81% | 89% | 80% |
| Spec 2 | 14% | 48% | 18% | 5% | 6% | 5% | 15% | 16% | 14% |
| Cutoff 3 | 0.897 | 2.81 | 0.897 | 1.11 | 0 | 1.11 | 2.19 | 0 | 2.19 |
| Sens 3 | 92% | 90% | 90% | 90% | 100% | 91% | 92% | 100% | 90% |
| Spec 3 | 5% | 15% | 5% | 5% | 0% | 5% | 8% | 0% | 9% |
| Cutoff 4 | 12.7 | 13.1 | 12.6 | 12.7 | 13.1 | 12.6 | 12.7 | 13.1 | 12.6 |
| Sens 4 | 39% | 50% | 37% | 26% | 36% | 21% | 38% | 33% | 35% |
| Spec 4 | 70% | 71% | 70% | 70% | 71% | 70% | 70% | 71% | 70% |
| Cutoff 5 | 17.9 | 17.9 | 15.9 | 17.9 | 17.9 | 15.9 | 17.9 | 17.9 | 15.9 |
| Sens 5 | 19% | 50% | 20% | 19% | 27% | 21% | 23% | 22% | 30% |
| Spec 5 | 81% | 80% | 81% | 81% | 80% | 81% | 81% | 80% | 81% |
| Cutoff 6 | 25.9 | 27.9 | 23.1 | 25.9 | 27.9 | 23.1 | 25.9 | 27.9 | 23.1 |
| Sens 6 | 14% | 40% | 10% | 14% | 18% | 9% | 0% | 0% | 10% |
| Spec 6 | 90% | 90% | 91% | 90% | 90% | 91% | 90% | 90% | 91% |
| OR Quart 2 | 1.0 | 0.98 | 0.45 | 0.90 | 0.65 | 1.2 | 0.48 | 0 | 0.23 |
| p Value | 1.0 | 0.98 | 0.22 | 0.85 | 0.65 | 0.71 | 0.29 | na | 0.091 |
| 95% CI of | 0.33 | 0.13 | 0.13 | 0.31 | 0.10 | 0.41 | 0.12 | na | 0.042 |
| OR Quart2 | 3.0 | 7.2 | 1.6 | 2.6 | 4.1 | 3.7 | 1.9 | na | 1.3 |
| OR Quart 3 | 1.0 | 0.48 | 1.0 | 0.86 | 0.65 | 1.4 | 1.0 | 2.7 | 0.64 |
| p Value | 1.0 | 0.55 | 1.0 | 0.78 | 0.65 | 0.52 | 1.0 | 0.26 | 0.51 |
| 95% CI of | 0.33 | 0.042 | 0.32 | 0.29 | 0.10 | 0.48 | 0.30 | 0.49 | 0.17 |
| OR Quart3 | 3.0 | 5.5 | 3.1 | 2.5 | 4.1 | 4.2 | 3.4 | 14 | 2.4 |
| OR Quart 4 | 1.0 | 2.6 | 0.70 | 1.8 | 1.4 | 1.9 | 1.1 | 0.98 | 0.86 |
| p Value | 1.0 | 0.27 | 0.55 | 0.26 | 0.70 | 0.24 | 0.83 | 0.98 | 0.81 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of OR Quart4 | 0.33 3.0 | 0.48 14 | 0.21 2.3 | 0.65 5.1 | 0.29 6.4 | 0.65 5.5 | 0.35 3.8 | 0.13 7.2 | 0.24 3.1 |

**Interleukin-17A**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.58 | 1.37 | 1.58 | 1.79 | 1.58 | 2.86 |
| Average | 3.40 | 2.81 | 3.40 | 3.36 | 3.40 | 3.89 |
| Stdev | 5.09 | 4.30 | 5.09 | 4.95 | 5.09 | 3.89 |
| p(t-test) |  | 0.36 |  | 0.96 |  | 0.66 |
| Min | 0.000362 | 0.000362 | 0.000362 | 0.000469 | 0.000362 | 0.000469 |
| Max | 33.6 | 29.6 | 33.6 | 33.0 | 33.6 | 18.0 |
| n (Samp) | 159 | 88 | 159 | 62 | 159 | 22 |
| n (Patient) | 87 | 88 | 87 | 62 | 87 | 22 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.51 | 1.90 | 1.51 | 2.19 | 1.51 | 2.54 |
| Average | 3.31 | 3.71 | 3.31 | 4.47 | 3.31 | 3.17 |
| Stdev | 5.49 | 5.99 | 5.49 | 6.78 | 5.49 | 2.20 |
| p(t-test) |  | 0.72 |  | 0.33 |  | 0.93 |
| Min | 0.000362 | 0.000532 | 0.000362 | 0.00408 | 0.000362 | 0.690 |
| Max | 53.4 | 29.6 | 53.4 | 33.0 | 53.4 | 8.69 |
| n (Samp) | 399 | 26 | 399 | 23 | 399 | 11 |
| n (Patient) | 184 | 26 | 184 | 23 | 184 | 11 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.72 | 1.22 | 1.72 | 1.56 | 1.72 | 3.30 |
| Average | 3.20 | 2.69 | 3.20 | 2.90 | 3.20 | 3.83 |
| Stdev | 4.75 | 4.48 | 4.75 | 3.65 | 4.75 | 4.05 |
| p(t-test) |  | 0.42 |  | 0.65 |  | 0.56 |
| Min | 0.000362 | 0.000362 | 0.000362 | 0.000362 | 0.000362 | 0.000469 |
| Max | 33.6 | 29.6 | 33.6 | 19.4 | 33.6 | 18.0 |
| n (Samp) | 189 | 76 | 189 | 65 | 189 | 21 |
| n (Patient) | 93 | 76 | 93 | 65 | 93 | 21 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.47 | 0.55 | 0.45 | 0.53 | 0.61 | 0.52 | 0.62 | 0.64 | 0.61 |
| SE | 0.039 | 0.060 | 0.040 | 0.044 | 0.064 | 0.042 | 0.067 | 0.091 | 0.068 |
| p | 0.44 | 0.44 | 0.17 | 0.45 | 0.089 | 0.70 | 0.069 | 0.13 | 0.096 |
| nCohort 1 | 159 | 399 | 189 | 159 | 399 | 189 | 159 | 399 | 189 |
| nCohort 2 | 88 | 26 | 76 | 62 | 23 | 65 | 22 | 11 | 21 |
| Cutoff 1 | 0.176 | 0.348 | 0.150 | 0.634 | 1.06 | 0.608 | 2.17 | 2.05 | 2.04 |
| Sens 1 | 73% | 73% | 71% | 71% | 74% | 71% | 73% | 73% | 71% |
| Spec 1 | 26% | 29% | 25% | 34% | 44% | 34% | 57% | 58% | 56% |
| Cutoff 2 | 0.00219 | 0.234 | 0.000532 | 0.373 | 0.608 | 0.348 | 2.04 | 2.04 | 0.790 |
| Sens 2 | 83% | 81% | 80% | 81% | 83% | 80% | 82% | 82% | 81% |
| Spec 2 | 14% | 27% | 13% | 31% | 35% | 29% | 55% | 58% | 37% |
| Cutoff 3 | 0.000469 | 0.00436 | 0.000362 | 0.00219 | 0.490 | 0.00219 | 0.780 | 1.47 | 0.690 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 92% | 96% | 93% | 94% | 91% | 92% | 91% | 91% | 90% |
| Spec 3 | 8% | 19% | 4% | 14% | 31% | 14% | 36% | 50% | 34% |
| Cutoff 4 | 3.75 | 3.33 | 3.47 | 3.75 | 3.33 | 3.47 | 3.75 | 3.33 | 3.47 |
| Sens 4 | 27% | 35% | 25% | 27% | 43% | 29% | 27% | 27% | 38% |
| Spec 4 | 70% | 70% | 70% | 70% | 70% | 70% | 70% | 70% | 70% |
| Cutoff 5 | 5.11 | 4.95 | 5.11 | 5.11 | 4.95 | 5.11 | 5.11 | 4.95 | 5.11 |
| Sens 5 | 17% | 23% | 16% | 21% | 26% | 18% | 14% | 18% | 14% |
| Spec 5 | 81% | 80% | 81% | 81% | 80% | 81% | 81% | 80% | 81% |
| Cutoff 6 | 9.31 | 8.85 | 8.35 | 9.31 | 8.85 | 8.35 | 9.31 | 8.85 | 8.35 |
| Sens 6 | 5% | 8% | 8% | 8% | 9% | 6% | 9% | 0% | 14% |
| Spec 6 | 91% | 90% | 90% | 91% | 90% | 90% | 91% | 90% | 90% |
| OR Quart 2 | 1.2 | 1.4 | 0.94 | 1.9 | 3.1 | 1.9 | 3.1 | >2.0 | 5.3 |
| p Value | 0.70 | 0.55 | 0.88 | 0.13 | 0.17 | 0.12 | 0.33 | <0.57 | 0.13 |
| 95% CI of | 0.55 | 0.44 | 0.43 | 0.82 | 0.61 | 0.84 | 0.31 | >0.18 | 0.60 |
| OR Quart2 | 2.4 | 4.7 | 2.1 | 4.6 | 16 | 4.2 | 31 | na | 47 |
| OR Quart 3 | 1.3 | 1.2 | 1.5 | 1.9 | 3.7 | 1.2 | 20 | >7.5 | 14 |
| p Value | 0.45 | 0.76 | 0.31 | 0.13 | 0.11 | 0.67 | 0.0049 | <0.061 | 0.014 |
| 95% CI of | 0.63 | 0.36 | 0.69 | 0.82 | 0.75 | 0.52 | 2.5 | >0.91 | 1.7 |
| OR Quart3 | 2.8 | 4.1 | 3.1 | 4.6 | 18 | 2.8 | 160 | na | 110 |
| OR Quart 4 | 1.2 | 1.6 | 1.4 | 1.5 | 4.2 | 1.3 | 4.2 | >2.0 | 4.2 |
| p Value | 0.66 | 0.40 | 0.41 | 0.40 | 0.074 | 0.56 | 0.21 | <0.57 | 0.21 |
| 95% CI of | 0.56 | 0.52 | 0.64 | 0.60 | 0.87 | 0.56 | 0.45 | >0.18 | 0.45 |
| OR Quart4 | 2.5 | 5.2 | 2.9 | 3.6 | 20 | 2.9 | 39 | na | 39 |

**Insulin receptor substrate 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0314 | 0.0263 | 0.0314 | 0.0316 | 0.0314 | 0.0106 |
| Average | 0.871 | 0.448 | 0.871 | 0.332 | 0.871 | 0.502 |
| Stdev | 5.30 | 1.54 | 5.30 | 0.912 | 5.30 | 1.37 |
| p(t-test) | | 0.49 | | 0.45 | | 0.78 |
| Min | 3.03E-6 | 0.000111 | 3.03E-6 | 0.000645 | 3.03E-6 | 0.000172 |
| Max | 49.5 | 10.5 | 49.5 | 4.76 | 49.5 | 5.12 |
| n (Samp) | 158 | 78 | 158 | 56 | 158 | 16 |
| n (Patient) | 89 | 78 | 89 | 56 | 89 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0288 | 0.0461 | 0.0288 | 0.0437 | 0.0288 | 0.00737 |
| Average | 0.562 | 0.643 | 0.562 | 0.174 | 0.562 | 0.0163 |
| Stdev | 3.53 | 1.76 | 3.53 | 0.233 | 3.53 | 0.0196 |
| p(t-test) | | 0.92 | | 0.72 | | 0.68 |
| Min | 3.03E-6 | 0.00276 | 3.03E-6 | 0.000645 | 3.03E-6 | 0.000645 |
| Max | 49.5 | 7.74 | 49.5 | 0.722 | 49.5 | 0.0477 |
| n (Samp) | 378 | 22 | 378 | 11 | 378 | 7 |
| n (Patient) | 177 | 22 | 177 | 11 | 177 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0345 | 0.0238 | 0.0345 | 0.0301 | 0.0345 | 0.0233 |
| Average | 0.793 | 0.484 | 0.793 | 0.379 | 0.793 | 0.487 |
| Stdev | 4.91 | 1.64 | 4.91 | 0.982 | 4.91 | 1.29 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.61 | | 0.52 | | 0.79 |
| Min | 3.03E-6 | 0.000111 | 3.03E-6 | 0.000645 | 3.03E-6 | 0.000172 |
| Max | 49.5 | 10.5 | 49.5 | 4.76 | 49.5 | 5.12 |
| n (Samp) | 185 | 67 | 185 | 60 | 185 | 18 |
| n (Patient) | 91 | 67 | 91 | 60 | 91 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.57 | 0.50 | 0.51 | 0.55 | 0.50 | 0.36 | 0.28 | 0.44 |
| SE | 0.040 | 0.065 | 0.041 | 0.045 | 0.091 | 0.043 | 0.078 | 0.11 | 0.073 |
| p | 0.86 | 0.26 | 0.98 | 0.89 | 0.55 | 0.96 | 0.081 | 0.048 | 0.41 |
| nCohort 1 | 158 | 378 | 185 | 158 | 378 | 185 | 158 | 378 | 185 |
| nCohort 2 | 78 | 22 | 67 | 56 | 11 | 60 | 16 | 7 | 18 |
| Cutoff 1 | 0.0143 | 0.0179 | 0.0143 | 0.0103 | 0.0154 | 0.0143 | 0.000172 | 0.000172 | 0.000645 |
| Sens 1 | 72% | 73% | 70% | 73% | 73% | 70% | 88% | 100% | 72% |
| Spec 1 | 32% | 38% | 33% | 25% | 36% | 33% | 4% | 3% | 10% |
| Cutoff 2 | 0.00925 | 0.00831 | 0.0103 | 0.00605 | 0.00689 | 0.00831 | 0.000172 | 0.000172 | 0.000172 |
| Sens 2 | 81% | 82% | 82% | 82% | 82% | 80% | 88% | 100% | 89% |
| Spec 2 | 25% | 22% | 25% | 18% | 20% | 23% | 4% | 3% | 3% |
| Cutoff 3 | 0.00323 | 0.00372 | 0.00323 | 0.00323 | 0.00372 | 0.00323 | 3.96E-5 | 0.000172 | 3.96E-5 |
| Sens 3 | 92% | 91% | 93% | 91% | 91% | 92% | 100% | 100% | 100% |
| Spec 3 | 17% | 16% | 17% | 17% | 16% | 17% | 3% | 3% | 3% |
| Cutoff 4 | 0.0973 | 0.0777 | 0.106 | 0.0973 | 0.0777 | 0.106 | 0.0973 | 0.0777 | 0.106 |
| Sens 4 | 29% | 36% | 28% | 32% | 45% | 28% | 25% | 0% | 28% |
| Spec 4 | 70% | 70% | 70% | 70% | 70% | 70% | 70% | 70% | 70% |
| Cutoff 5 | 0.148 | 0.147 | 0.175 | 0.148 | 0.147 | 0.175 | 0.148 | 0.147 | 0.175 |
| Sens 5 | 22% | 27% | 21% | 27% | 36% | 23% | 19% | 0% | 22% |
| Spec 5 | 80% | 81% | 80% | 80% | 81% | 80% | 80% | 81% | 80% |
| Cutoff 6 | 0.764 | 0.503 | 0.527 | 0.764 | 0.503 | 0.527 | 0.764 | 0.503 | 0.527 |
| Sens 6 | 10% | 18% | 12% | 9% | 9% | 13% | 12% | 0% | 11% |
| Spec 6 | 91% | 90% | 90% | 91% | 90% | 90% | 91% | 90% | 90% |
| OR Quart 2 | 1.4 | 0.59 | 1.0 | 0.98 | 0.66 | 0.77 | 0.67 | >2.1 | 0.57 |
| p Value | 0.44 | 0.48 | 1.0 | 0.95 | 0.65 | 0.55 | 0.67 | <0.56 | 0.47 |
| 95% CI of OR Quart2 | 0.63 2.9 | 0.14 2.5 | 0.45 2.2 | 0.41 2.3 | 0.11 4.0 | 0.33 1.8 | 0.11 4.2 | >0.18 na | 0.13 2.5 |
| OR Quart 3 | 0.92 | 1.4 | 1.5 | 0.91 | 0.33 | 1.4 | 1.4 | >1.0 | 0.78 |
| p Value | 0.84 | 0.55 | 0.33 | 0.82 | 0.34 | 0.39 | 0.69 | <0.99 | 0.73 |
| 95% CI of OR Quart3 | 0.42 2.0 | 0.44 4.7 | 0.68 3.2 | 0.38 2.2 | 0.033 3.2 | 0.64 3.1 | 0.29 6.5 | >0.063 na | 0.20 3.1 |
| OR Quart 4 | 1.3 | 1.4 | 0.84 | 1.1 | 1.7 | 0.93 | 2.7 | >4.2 | 1.3 |
| p Value | 0.56 | 0.55 | 0.68 | 0.87 | 0.48 | 0.87 | 0.18 | <0.20 | 0.72 |
| 95% CI of OR Quart4 | 0.58 2.7 | 0.44 4.7 | 0.37 1.9 | 0.46 2.5 | 0.39 7.3 | 0.41 2.1 | 0.64 11 | >0.46 na | 0.36 4.4 |

**Involucrin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.750 | 0.649 | 0.750 | 0.751 | 0.750 | 0.593 |
| Average | 0.946 | 0.949 | 0.946 | 1.20 | 0.946 | 0.789 |
| Stdev | 0.702 | 0.806 | 0.702 | 1.21 | 0.702 | 0.593 |
| p(t-test) | | 0.97 | | 0.063 | | 0.39 |
| Min | 0.0472 | 0.148 | 0.0472 | 0.159 | 0.0472 | 0.258 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 3.56 | 4.58 | 3.56 | 7.42 | 3.56 | 2.33 |
| n (Samp) | 158 | 78 | 158 | 56 | 158 | 16 |
| n (Patient) | 89 | 78 | 89 | 56 | 89 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.689 | 0.832 | 0.689 | 0.975 | 0.689 | 1.56 |
| Average | 0.966 | 1.05 | 0.966 | 1.25 | 0.966 | 1.44 |
| Stdev | 0.954 | 0.731 | 0.954 | 0.815 | 0.954 | 0.650 |
| p(t-test) | | 0.69 | | 0.32 | | 0.19 |
| Min | 0.0472 | 0.148 | 0.0472 | 0.291 | 0.0472 | 0.591 |
| Max | 12.7 | 3.11 | 12.7 | 3.01 | 12.7 | 2.33 |
| n (Samp) | 378 | 22 | 378 | 11 | 378 | 7 |
| n (Patient) | 177 | 22 | 177 | 11 | 177 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.770 | 0.697 | 0.770 | 0.682 | 0.770 | 0.648 |
| Average | 0.983 | 0.926 | 0.983 | 1.19 | 0.983 | 0.796 |
| Stdev | 0.703 | 0.788 | 0.703 | 1.22 | 0.703 | 0.557 |
| p(t-test) | | 0.58 | | 0.10 | | 0.28 |
| Min | 0.0472 | 0.185 | 0.0472 | 0.159 | 0.0472 | 0.258 |
| Max | 3.67 | 4.58 | 3.67 | 7.42 | 3.67 | 2.33 |
| n (Samp) | 185 | 67 | 185 | 60 | 185 | 18 |
| n (Patient) | 91 | 67 | 91 | 60 | 91 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.55 | 0.45 | 0.54 | 0.64 | 0.51 | 0.42 | 0.75 | 0.41 |
| SE | 0.040 | 0.065 | 0.042 | 0.045 | 0.091 | 0.043 | 0.078 | 0.11 | 0.073 |
| p | 0.50 | 0.44 | 0.21 | 0.41 | 0.14 | 0.86 | 0.28 | 0.019 | 0.22 |
| nCohort 1 | 158 | 378 | 185 | 158 | 378 | 185 | 158 | 378 | 185 |
| nCohort 2 | 78 | 22 | 67 | 56 | 11 | 60 | 16 | 7 | 18 |
| Cutoff 1 | 0.490 | 0.511 | 0.501 | 0.560 | 0.910 | 0.560 | 0.439 | 0.997 | 0.461 |
| Sens 1 | 71% | 73% | 70% | 71% | 73% | 70% | 75% | 71% | 72% |
| Spec 1 | 27% | 30% | 24% | 34% | 67% | 30% | 21% | 72% | 20% |
| Cutoff 2 | 0.401 | 0.419 | 0.446 | 0.428 | 0.571 | 0.446 | 0.428 | 0.824 | 0.428 |
| Sens 2 | 81% | 82% | 81% | 80% | 82% | 80% | 81% | 86% | 83% |
| Spec 2 | 18% | 19% | 18% | 20% | 35% | 18% | 20% | 62% | 17% |
| Cutoff 3 | 0.317 | 0.317 | 0.330 | 0.290 | 0.368 | 0.312 | 0.267 | 0.584 | 0.291 |
| Sens 3 | 91% | 91% | 91% | 91% | 91% | 90% | 94% | 100% | 94% |
| Spec 3 | 10% | 10% | 10% | 8% | 14% | 8% | 8% | 38% | 5% |
| Cutoff 4 | 0.994 | 0.976 | 1.05 | 0.994 | 0.976 | 1.05 | 0.994 | 0.976 | 1.05 |
| Sens 4 | 28% | 45% | 25% | 39% | 45% | 35% | 19% | 71% | 17% |
| Spec 4 | 70% | 70% | 70% | 70% | 70% | 70% | 70% | 70% | 70% |
| Cutoff 5 | 1.30 | 1.31 | 1.36 | 1.30 | 1.31 | 1.36 | 1.30 | 1.31 | 1.36 |
| Sens 5 | 24% | 36% | 19% | 30% | 36% | 28% | 19% | 57% | 11% |
| Spec 5 | 80% | 80% | 80% | 80% | 80% | 80% | 80% | 80% | 80% |
| Cutoff 6 | 2.03 | 1.98 | 2.03 | 2.03 | 1.98 | 2.03 | 2.03 | 1.98 | 2.03 |
| Sens 6 | 9% | 5% | 9% | 11% | 9% | 13% | 6% | 29% | 6% |
| Spec 6 | 91% | 90% | 90% | 91% | 90% | 90% | 91% | 90% | 90% |
| OR Quart 2 | 0.61 | 1.0 | 0.83 | 1.4 | 0.49 | 1.2 | 0.67 | >1.0 | 1.4 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.22 | 1.0 | 0.67 | 0.41 | 0.57 | 0.68 | 0.67 | <0.99 | 0.70 |
| 95% CI of OR Quart2 | 0.27 | 0.28 | 0.36 | 0.60 | 0.044 | 0.53 | 0.11 | >0.062 | 0.29 |
| | 1.4 | 3.6 | 1.9 | 3.4 | 5.5 | 2.7 | 4.2 | na | 6.4 |
| OR Quart 3 | 1.3 | 0.79 | 1.4 | 0.70 | 2.0 | 0.53 | 1.8 | >2.0 | 1.7 |
| p Value | 0.45 | 0.73 | 0.42 | 0.47 | 0.42 | 0.18 | 0.46 | <0.56 | 0.47 |
| 95% CI of OR Quart3 | 0.63 | 0.21 | 0.63 | 0.27 | 0.37 | 0.21 | 0.39 | >0.18 | 0.39 |
| | 2.8 | 3.0 | 3.0 | 1.8 | 11 | 1.3 | 7.8 | na | 7.7 |
| OR Quart 4 | 1.0 | 1.7 | 1.5 | 1.9 | 2.0 | 1.4 | 2.2 | >4.1 | 2.2 |
| p Value | 1.0 | 0.39 | 0.32 | 0.16 | 0.42 | 0.45 | 0.28 | <0.21 | 0.29 |
| 95% CI of OR Quart4 | 0.47 | 0.52 | 0.68 | 0.79 | 0.36 | 0.61 | 0.52 | >0.45 | 0.51 |
| | 2.1 | 5.2 | 3.3 | 4.3 | 11 | 3.0 | 9.5 | na | 9.3 |

**Keratin, type II cytoskeletal 6 (6A, -6B, -6C mix)**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.13E-5 | 5.13E-5 | 5.13E-5 | 5.13E-5 | 5.13E-5 | 0.000220 |
| Average | 1.74 | 2.74 | 1.74 | 0.000120 | 1.74 | 13.5 |
| Stdev | 11.3 | 13.5 | 11.3 | 8.36E-5 | 11.3 | 53.9 |
| p(t-test) | | 0.55 | | 0.25 | | 0.021 |
| Min | 5.13E-5 | 5.13E-5 | 5.13E-5 | 5.13E-5 | 5.13E-5 | 5.13E-5 |
| Max | 119 | 88.7 | 119 | 0.000220 | 119 | 216 |
| n (Samp) | 158 | 78 | 158 | 56 | 158 | 16 |
| n (Patient) | 89 | 78 | 89 | 56 | 89 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.13E-5 | 5.13E-5 | 5.13E-5 | 5.13E-5 | 5.13E-5 | 5.13E-5 |
| Average | 2.44 | 4.03 | 2.44 | 8.19E-5 | 2.44 | 0.000123 |
| Stdev | 18.0 | 18.9 | 18.0 | 6.81E-5 | 18.0 | 9.00E-5 |
| p(t-test) | | 0.69 | | 0.65 | | 0.72 |
| Min | 5.13E-5 | 5.13E-5 | 5.13E-5 | 5.13E-5 | 5.13E-5 | 5.13E-5 |
| Max | 216 | 88.7 | 216 | 0.000220 | 216 | 0.000220 |
| n (Samp) | 378 | 22 | 378 | 11 | 378 | 7 |
| n (Patient) | 177 | 22 | 177 | 11 | 177 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.13E-5 | 5.13E-5 | 5.13E-5 | 5.13E-5 | 5.13E-5 | 0.000135 |
| Average | 1.34 | 5.09 | 1.34 | 1.70 | 1.34 | 12.0 |
| Stdev | 9.11 | 28.0 | 9.11 | 13.1 | 9.11 | 50.9 |
| p(t-test) | | 0.11 | | 0.81 | | 0.013 |
| Min | 5.13E-5 | 5.13E-5 | 5.13E-5 | 5.13E-5 | 5.13E-5 | 5.13E-5 |
| Max | 94.9 | 216 | 94.9 | 102 | 94.9 | 216 |
| n (Samp) | 185 | 67 | 185 | 60 | 185 | 18 |
| n (Patient) | 91 | 67 | 91 | 60 | 91 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.43 | 0.44 | 0.47 | 0.51 | 0.43 | 0.54 | 0.63 | 0.55 | 0.61 |
| SE | 0.040 | 0.065 | 0.042 | 0.045 | 0.091 | 0.043 | 0.078 | 0.11 | 0.073 |
| p | 0.094 | 0.36 | 0.45 | 0.78 | 0.46 | 0.30 | 0.10 | 0.66 | 0.14 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| nCohort 1 | 158 | 378 | 185 | 158 | 378 | 185 | 158 | 378 | 185 |
| nCohort 2 | 78 | 22 | 67 | 56 | 11 | 60 | 16 | 7 | 18 |
| Cutoff 1 | 0 | 0 | 0 | 0 | 0 | 0 | 0 | 0 | 0 |
| Sens 1 | 100% | 100% | 100% | 100% | 100% | 100% | 100% | 100% | 100% |
| Spec 1 | 0% | 0% | 0% | 0% | 0% | 0% | 0% | 0% | 0% |
| Cutoff 2 | 0 | 0 | 0 | 0 | 0 | 0 | 0 | 0 | 0 |
| Sens 2 | 100% | 100% | 100% | 100% | 100% | 100% | 100% | 100% | 100% |
| Spec 2 | 0% | 0% | 0% | 0% | 0% | 0% | 0% | 0% | 0% |
| Cutoff 3 | 0 | 0 | 0 | 0 | 0 | 0 | 0 | 0 | 0 |
| Sens 3 | 100% | 100% | 100% | 100% | 100% | 100% | 100% | 100% | 100% |
| Spec 3 | 0% | 0% | 0% | 0% | 0% | 0% | 0% | 0% | 0% |
| Cutoff 4 | 0.000220 | 0.000220 | 5.13E-5 | 0.000220 | 0.000220 | 5.13E-5 | 0.000220 | 0.000220 | 5.13E-5 |
| Sens 4 | 6% | 5% | 21% | 0% | 0% | 38% | 6% | 0% | 50% |
| Spec 4 | 95% | 96% | 71% | 95% | 96% | 71% | 95% | 96% | 71% |
| Cutoff 5 | 0.000220 | 0.000220 | 0.000220 | 0.000220 | 0.000220 | 0.000220 | 0.000220 | 0.000220 | 0.000220 |
| Sens 5 | 6% | 5% | 7% | 0% | 0% | 2% | 6% | 0% | 6% |
| Spec 5 | 95% | 96% | 96% | 95% | 96% | 96% | 95% | 96% | 96% |
| Cutoff 6 | 0.000220 | 0.000220 | 0.000220 | 0.000220 | 0.000220 | 0.000220 | 0.000220 | 0.000220 | 0.000220 |
| Sens 6 | 6% | 5% | 7% | 0% | 0% | 2% | 6% | 0% | 6% |
| Spec 6 | 95% | 96% | 96% | 95% | 96% | 96% | 95% | 96% | 96% |
| OR Quart 2 | 0 | 0 | 0 | 6.1 | 0 | 7.5 | 5.4 | 3.1 | 9.1 |
| p Value | na | na | na | 2.1E-4 | na | 2.4E-5 | 0.13 | 0.34 | 0.041 |
| 95% CI of | na | na | na | 2.3 | na | 2.9 | 0.60 | 0.31 | 1.1 |
| OR Quart2 | na | na | na | 16 | na | 19 | 48 | 30 | 76 |
| OR Quart 3 | 11 | 4.9 | 11 | 1.7 | 4.3 | 0.40 | 4.3 | 0 | 0 |
| p Value | 6.9E-8 | 0.0057 | 1.2E-8 | 0.30 | 0.069 | 0.20 | 0.20 | na | na |
| 95% CI of | 4.5 | 1.6 | 4.9 | 0.61 | 0.89 | 0.098 | 0.46 | na | na |
| OR Quart3 | 26 | 15 | 26 | 4.8 | 21 | 1.6 | 40 | na | na |
| OR Quart 4 | 0.70 | 0.24 | 0.30 | 1.9 | 0.50 | 3.7 | 6.6 | 3.0 | 10 |
| p Value | 0.40 | 0.21 | 0.031 | 0.23 | 0.57 | 0.0073 | 0.086 | 0.34 | 0.029 |
| 95% CI of | 0.30 | 0.027 | 0.10 | 0.68 | 0.045 | 1.4 | 0.76 | 0.31 | 1.3 |
| OR Quart4 | 1.6 | 2.2 | 0.90 | 5.2 | 5.6 | 9.5 | 58 | 30 | 86 |

**Collagenase 3**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0305 | 0.0305 | 0.0305 | 0.0699 | 0.0305 | 0.102 |
| Average | 4.25 | 1.81 | 4.25 | 2.16 | 4.25 | 1.31 |
| Stdev | 29.8 | 9.45 | 29.8 | 15.4 | 29.8 | 4.47 |
| p(t-test) |  | 0.44 |  | 0.57 |  | 0.59 |
| Min | 0.00117 | 0.00117 | 0.00117 | 0.00117 | 0.00117 | 0.00117 |
| Max | 313 | 80.9 | 313 | 130 | 313 | 17.8 |
| n (Samp) | 186 | 95 | 186 | 72 | 186 | 30 |
| n (Patient) | 109 | 95 | 109 | 72 | 109 | 30 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0305 | 0.0288 | 0.0305 | 0.0699 | 0.0305 | 0.102 |
| Average | 3.17 | 3.35 | 3.17 | 5.75 | 3.17 | 5.18 |
| Stdev | 21.7 | 15.3 | 21.7 | 18.8 | 21.7 | 13.0 |
| p(t-test) |  | 0.96 |  | 0.60 |  | 0.75 |
| Min | 0.00117 | 0.00117 | 0.00117 | 0.00117 | 0.00117 | 0.00726 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 313 | 80.9 | 313 | 78.6 | 313 | 43.1 |
| n (Samp) | 460 | 28 | 460 | 20 | 460 | 12 |
| n (Patient) | 213 | 28 | 213 | 20 | 213 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0297 | 0.0305 | 0.0297 | 0.0699 | 0.0297 | 0.102 |
| Average | 3.74 | 1.68 | 3.74 | 2.76 | 3.74 | 0.742 |
| Stdev | 28.0 | 7.14 | 28.0 | 16.2 | 28.0 | 3.27 |
| p(t-test) | | 0.52 | | 0.77 | | 0.57 |
| Min | 0.00117 | 0.00117 | 0.00117 | 0.00117 | 0.00117 | 0.00117 |
| Max | 313 | 46.3 | 313 | 130 | 313 | 17.8 |
| n (Samp) | 211 | 81 | 211 | 77 | 211 | 29 |
| n (Patient) | 109 | 81 | 109 | 77 | 109 | 29 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.45 | 0.45 | 0.50 | 0.53 | 0.56 | 0.57 | 0.62 | 0.66 | 0.62 |
| SE | 0.037 | 0.058 | 0.038 | 0.040 | 0.068 | 0.039 | 0.058 | 0.087 | 0.059 |
| p | 0.15 | 0.39 | 0.97 | 0.47 | 0.40 | 0.076 | 0.041 | 0.060 | 0.033 |
| nCohort 1 | 186 | 460 | 211 | 186 | 460 | 211 | 186 | 460 | 211 |
| nCohort 2 | 95 | 28 | 81 | 72 | 20 | 77 | 30 | 12 | 29 |
| Cutoff 1 | 0.00455 | 0.00374 | 0.00455 | 0.00726 | 0.0297 | 0.00726 | 0.0699 | 0.0297 | 0.0297 |
| Sens 1 | 71% | 75% | 73% | 72% | 75% | 71% | 70% | 83% | 72% |
| Spec 1 | 21% | 15% | 27% | 32% | 44% | 39% | 62% | 44% | 50% |
| Cutoff 2 | 0.00374 | 0.00117 | 0.00374 | 0.00374 | 0.00117 | 0.00374 | 0.0297 | 0.0297 | 0.00455 |
| Sens 2 | 82% | 96% | 83% | 85% | 90% | 87% | 83% | 83% | 86% |
| Spec 2 | 13% | 9% | 20% | 13% | 9% | 20% | 42% | 44% | 27% |
| Cutoff 3 | 0.00117 | 0.00117 | 0.00117 | 0.00117 | 0.00117 | 0.00117 | 0.00455 | 0.00726 | 0.00117 |
| Sens 3 | 93% | 96% | 91% | 94% | 90% | 96% | 90% | 92% | 93% |
| Spec 3 | 9% | 9% | 9% | 9% | 9% | 9% | 21% | 34% | 9% |
| Cutoff 4 | 0.204 | 0.102 | 0.102 | 0.204 | 0.102 | 0.102 | 0.204 | 0.102 | 0.102 |
| Sens 4 | 13% | 21% | 22% | 19% | 30% | 32% | 20% | 33% | 34% |
| Spec 4 | 82% | 72% | 73% | 82% | 72% | 73% | 82% | 72% | 73% |
| Cutoff 5 | 0.204 | 0.204 | 0.204 | 0.204 | 0.204 | 0.204 | 0.204 | 0.204 | 0.204 |
| Sens 5 | 13% | 14% | 12% | 19% | 25% | 19% | 20% | 33% | 21% |
| Spec 5 | 82% | 83% | 85% | 82% | 83% | 85% | 82% | 83% | 85% |
| Cutoff 6 | 0.383 | 0.383 | 0.383 | 0.383 | 0.383 | 0.383 | 0.383 | 0.383 | 0.383 |
| Sens 6 | 5% | 7% | 6% | 4% | 10% | 5% | 7% | 17% | 3% |
| Spec 6 | 96% | 95% | 97% | 96% | 95% | 97% | 96% | 95% | 97% |
| OR Quart 2 | 1.1 | 0.83 | 1.5 | 1.1 | 0.39 | 1.0 | 1.0 | >4.1 | 1.7 |
| p Value | 0.81 | 0.76 | 0.27 | 0.88 | 0.27 | 1.0 | 1.0 | <0.21 | 0.47 |
| 95% CI of OR Quart2 | 0.53 | 0.25 | 0.73 | 0.48 | 0.074 | 0.46 | 0.24 | >0.46 | 0.39 |
| | 2.2 | 2.8 | 3.1 | 2.3 | 2.0 | 2.2 | 4.2 | na | 7.6 |
| OR Quart 3 | 1.2 | 1.2 | 1.2 | 1.5 | 1.4 | 1.8 | 4.4 | >4.1 | 5.8 |
| p Value | 0.67 | 0.78 | 0.71 | 0.33 | 0.56 | 0.14 | 0.015 | <0.21 | 0.0085 |
| 95% CI of OR Quart3 | 0.57 | 0.38 | 0.55 | 0.68 | 0.44 | 0.83 | 1.3 | >0.46 | 1.6 |
| | 2.4 | 3.6 | 2.4 | 3.2 | 4.6 | 3.7 | 14 | na | 21 |
| OR Quart 4 | 1.7 | 1.7 | 1.1 | 1.1 | 1.2 | 1.4 | 2.2 | >4.1 | 2.5 |
| p Value | 0.14 | 0.31 | 0.85 | 0.73 | 0.76 | 0.34 | 0.23 | <0.21 | 0.20 |
| 95% CI of OR Quart4 | 0.84 | 0.61 | 0.51 | 0.52 | 0.36 | 0.68 | 0.61 | >0.46 | 0.62 |
| | 3.4 | 4.9 | 2.3 | 2.5 | 4.1 | 3.1 | 7.7 | na | 10 |

**NF-kappa-B inhibitor alpha**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000560 | 0.00169 | 0.000560 | 0.00105 | 0.000560 | 3.67E-5 |
| Average | 0.0684 | 0.0159 | 0.0684 | 0.0248 | 0.0684 | 0.0211 |
| Stdev | 0.403 | 0.0440 | 0.403 | 0.0661 | 0.403 | 0.0689 |
| p(t-test) | | 0.25 | | 0.42 | | 0.64 |
| Min | 1.84E-7 | 1.84E-7 | 1.84E-7 | 1.84E-7 | 1.84E-7 | 1.84E-7 |
| Max | 3.62 | 0.270 | 3.62 | 0.384 | 3.62 | 0.276 |
| n (Samp) | 158 | 78 | 158 | 56 | 158 | 16 |
| n (Patient) | 89 | 78 | 89 | 56 | 89 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00127 | 0.00146 | 0.00127 | 0.00122 | 0.00127 | 3.67E-5 |
| Average | 0.0409 | 0.0159 | 0.0409 | 0.0256 | 0.0409 | 0.00465 |
| Stdev | 0.266 | 0.0377 | 0.266 | 0.0535 | 0.266 | 0.0114 |
| p(t-test) | | 0.66 | | 0.85 | | 0.72 |
| Min | 1.84E-7 | 2.41E-5 | 1.84E-7 | 1.84E-7 | 1.84E-7 | 3.20E-6 |
| Max | 3.62 | 0.166 | 3.62 | 0.150 | 3.62 | 0.0306 |
| n (Samp) | 378 | 22 | 378 | 11 | 378 | 7 |
| n (Patient) | 177 | 22 | 177 | 11 | 177 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00104 | 0.00166 | 0.00104 | 0.000976 | 0.00104 | 0.00203 |
| Average | 0.0657 | 0.0146 | 0.0657 | 0.0195 | 0.0657 | 0.0210 |
| Stdev | 0.376 | 0.0428 | 0.376 | 0.0606 | 0.376 | 0.0647 |
| p(t-test) | | 0.27 | | 0.34 | | 0.62 |
| Min | 1.84E-7 | 1.84E-7 | 1.84E-7 | 1.84E-7 | 1.84E-7 | 1.84E-7 |
| Max | 3.62 | 0.270 | 3.62 | 0.384 | 3.62 | 0.276 |
| n (Samp) | 185 | 67 | 185 | 60 | 185 | 18 |
| n (Patient) | 91 | 67 | 91 | 60 | 91 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.56 | 0.51 | 0.50 | 0.51 | 0.48 | 0.42 | 0.38 | 0.47 |
| SE | 0.040 | 0.065 | 0.041 | 0.045 | 0.089 | 0.043 | 0.078 | 0.11 | 0.073 |
| p | 0.31 | 0.34 | 0.83 | 0.99 | 0.89 | 0.63 | 0.30 | 0.29 | 0.63 |
| nCohort 1 | 158 | 378 | 185 | 158 | 378 | 185 | 158 | 378 | 185 |
| nCohort 2 | 78 | 22 | 67 | 56 | 11 | 60 | 16 | 7 | 18 |
| Cutoff 1 | 0.000425 | 0.000295 | 0.000425 | 3.31E-5 | 0.000156 | 3.31E-5 | 3.31E-5 | 3.31E-5 | 3.31E-5 |
| Sens 1 | 71% | 73% | 72% | 77% | 73% | 78% | 75% | 86% | 78% |
| Spec 1 | 44% | 37% | 39% | 20% | 33% | 18% | 20% | 21% | 18% |
| Cutoff 2 | 3.31E-5 | 0.000228 | 3.31E-5 | 3.20E-6 | 3.31E-5 | 2.41E-5 | 3.20E-6 | 3.31E-5 | 3.20E-6 |
| Sens 2 | 83% | 82% | 82% | 93% | 91% | 80% | 94% | 86% | 94% |
| Spec 2 | 20% | 35% | 18% | 3% | 21% | 15% | 3% | 21% | 4% |
| Cutoff 3 | 3.20E-6 | 3.31E-5 | 3.20E-6 | 3.20E-6 | 3.31E-5 | 3.20E-6 | 3.20E-6 | 9.39E-7 | 3.20E-6 |
| Sens 3 | 95% | 91% | 94% | 93% | 91% | 93% | 94% | 100% | 94% |
| Spec 3 | 3% | 21% | 4% | 3% | 21% | 4% | 3% | 6% | 4% |
| Cutoff 4 | 0.00495 | 0.00402 | 0.00597 | 0.00495 | 0.00402 | 0.00597 | 0.00495 | 0.00402 | 0.00597 |
| Sens 4 | 26% | 45% | 25% | 32% | 36% | 32% | 19% | 14% | 22% |
| Spec 4 | 70% | 70% | 70% | 70% | 70% | 70% | 70% | 70% | 70% |
| Cutoff 5 | 0.0147 | 0.0115 | 0.0150 | 0.0147 | 0.0115 | 0.0150 | 0.0147 | 0.0115 | 0.0150 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 5 | 18% | 23% | 16% | 20% | 18% | 18% | 12% | 14% | 22% |
| Spec 5 | 80% | 80% | 80% | 80% | 80% | 80% | 80% | 80% | 80% |
| Cutoff 6 | 0.0421 | 0.0421 | 0.0521 | 0.0421 | 0.0421 | 0.0521 | 0.0421 | 0.0421 | 0.0521 |
| Sens 6 | 9% | 9% | 7% | 16% | 18% | 7% | 12% | 0% | 6% |
| Spec 6 | 91% | 90% | 90% | 91% | 90% | 90% | 91% | 90% | 90% |
| OR Quart 2 | 1.2 | 4.8 | 1.1 | 1.0 | 6.3 | 1.2 | 2.8 | 0 | 1.6 |
| p Value | 0.68 | 0.047 | 0.84 | 0.95 | 0.090 | 0.64 | 0.24 | na | 0.51 |
| 95% CI of | 0.53 | 1.0 | 0.48 | 0.45 | 0.75 | 0.53 | 0.51 | na | 0.41 |
| OR Quart2 | 2.6 | 23 | 2.5 | 2.3 | 54 | 2.8 | 15 | na | 5.9 |
| OR Quart 3 | 2.0 | 2.0 | 1.5 | 0.19 | 1.0 | 1.0 | 0.49 | 4.2 | 0 |
| p Value | 0.084 | 0.42 | 0.32 | 0.0056 | 1.0 | 0.96 | 0.56 | 0.21 | na |
| 95% CI of | 0.91 | 0.37 | 0.68 | 0.059 | 0.062 | 0.44 | 0.043 | 0.46 | na |
| OR Quart3 | 4.3 | 11 | 3.3 | 0.61 | 16 | 2.4 | 5.6 | 38 | na |
| OR Quart 4 | 1.3 | 3.7 | 1.1 | 1.4 | 3.0 | 1.2 | 4.8 | 2.0 | 2.2 |
| p Value | 0.55 | 0.11 | 0.84 | 0.38 | 0.34 | 0.64 | 0.057 | 0.56 | 0.21 |
| 95% CI of | 0.58 | 0.75 | 0.48 | 0.64 | 0.31 | 0.53 | 0.96 | 0.18 | 0.63 |
| OR Quart4 | 2.8 | 18 | 2.5 | 3.2 | 30 | 2.8 | 24 | 23 | 8.0 |

**Beta-nerve growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.48 | 1.85 | 1.48 | 1.82 | 1.48 | 1.42 |
| Average | 2.42 | 2.30 | 2.42 | 2.35 | 2.42 | 1.84 |
| Stdev | 5.59 | 1.70 | 5.59 | 1.79 | 5.59 | 1.34 |
| p(t-test) | | 0.87 | | 0.93 | | 0.69 |
| Min | 0.259 | 0.482 | 0.259 | 0.453 | 0.259 | 0.995 |
| Max | 70.0 | 8.57 | 70.0 | 9.39 | 70.0 | 6.48 |
| n (Samp) | 158 | 74 | 158 | 56 | 158 | 15 |
| n (Patient) | 88 | 74 | 88 | 56 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.61 | 2.21 | 1.61 | 2.67 | 1.61 | 1.79 |
| Average | 2.16 | 2.94 | 2.16 | 3.12 | 2.16 | 1.97 |
| Stdev | 3.75 | 2.05 | 3.75 | 2.29 | 3.75 | 0.828 |
| p(t-test) | | 0.36 | | 0.40 | | 0.89 |
| Min | 0.221 | 0.951 | 0.221 | 1.28 | 0.221 | 0.983 |
| Max | 70.0 | 8.57 | 70.0 | 9.39 | 70.0 | 3.50 |
| n (Samp) | 378 | 20 | 378 | 11 | 378 | 7 |
| n (Patient) | 175 | 20 | 175 | 11 | 175 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.87 | 1.81 | 1.87 | 1.88 | 1.87 | 1.45 |
| Average | 2.67 | 2.25 | 2.67 | 2.37 | 2.67 | 1.97 |
| Stdev | 5.25 | 1.58 | 5.25 | 1.63 | 5.25 | 1.35 |
| p(t-test) | | 0.53 | | 0.65 | | 0.57 |
| Min | 0.259 | 0.482 | 0.259 | 0.453 | 0.259 | 0.995 |
| Max | 70.0 | 8.47 | 70.0 | 7.22 | 70.0 | 6.48 |
| n (Samp) | 184 | 65 | 184 | 62 | 184 | 18 |
| n (Patient) | 89 | 65 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.66 | 0.49 | 0.57 | 0.72 | 0.52 | 0.50 | 0.57 | 0.45 |
| SE | 0.041 | 0.068 | 0.042 | 0.045 | 0.088 | 0.043 | 0.078 | 0.11 | 0.073 |
| p | 0.20 | 0.015 | 0.83 | 0.13 | 0.014 | 0.72 | 0.98 | 0.56 | 0.52 |
| nCohort 1 | 158 | 378 | 184 | 158 | 378 | 184 | 158 | 378 | 184 |
| nCohort 2 | 74 | 20 | 65 | 56 | 11 | 62 | 15 | 7 | 18 |
| Cutoff 1 | 1.12 | 1.87 | 1.36 | 1.35 | 1.66 | 1.41 | 1.29 | 1.48 | 1.29 |
| Sens 1 | 72% | 70% | 71% | 71% | 73% | 71% | 80% | 86% | 78% |
| Spec 1 | 29% | 61% | 37% | 45% | 53% | 38% | 37% | 43% | 29% |
| Cutoff 2 | 1.07 | 1.54 | 1.07 | 1.24 | 1.61 | 1.22 | 1.29 | 1.48 | 1.28 |
| Sens 2 | 84% | 80% | 83% | 80% | 82% | 81% | 80% | 86% | 83% |
| Spec 2 | 23% | 48% | 18% | 35% | 51% | 26% | 37% | 43% | 28% |
| Cutoff 3 | 0.848 | 1.10 | 0.848 | 0.848 | 1.56 | 0.951 | 1.22 | 0.977 | 1.09 |
| Sens 3 | 92% | 90% | 91% | 91% | 91% | 90% | 93% | 100% | 94% |
| Spec 3 | 12% | 22% | 9% | 12% | 48% | 13% | 33% | 16% | 21% |
| Cutoff 4 | 2.36 | 2.09 | 2.64 | 2.36 | 2.09 | 2.64 | 2.36 | 2.09 | 2.64 |
| Sens 4 | 26% | 55% | 26% | 30% | 64% | 26% | 13% | 29% | 17% |
| Spec 4 | 70% | 70% | 71% | 70% | 70% | 71% | 70% | 70% | 71% |
| Cutoff 5 | 2.89 | 2.64 | 3.35 | 2.89 | 2.64 | 3.35 | 2.89 | 2.64 | 3.35 |
| Sens 5 | 23% | 30% | 17% | 23% | 55% | 18% | 7% | 14% | 11% |
| Spec 5 | 80% | 81% | 80% | 80% | 81% | 80% | 80% | 81% | 80% |
| Cutoff 6 | 3.87 | 3.66 | 4.41 | 3.87 | 3.66 | 4.41 | 3.87 | 3.66 | 4.41 |
| Sens 6 | 15% | 30% | 8% | 12% | 27% | 11% | 7% | 0% | 6% |
| Spec 6 | 91% | 90% | 90% | 91% | 90% | 90% | 91% | 90% | 90% |
| OR Quart 2 | 0.76 | 0.24 | 0.94 | 0.98 | >2.0 | 1.5 | 7.0 | 2.0 | 2.1 |
| p Value | 0.52 | 0.21 | 0.88 | 0.96 | <0.56 | 0.33 | 0.078 | 0.57 | 0.40 |
| 95% CI of OR Quart2 | 0.33 1.8 | 0.026 2.2 | 0.42 2.1 | 0.37 2.6 | >0.18 na | 0.66 3.4 | 0.80 61 | 0.18 23 | 0.37 12 |
| OR Quart 3 | 2.3 | 2.1 | 0.94 | 2.8 | >3.1 | 1.4 | 8.4 | 2.0 | 6.0 |
| p Value | 0.036 | 0.24 | 0.88 | 0.021 | <0.33 | 0.40 | 0.052 | 0.57 | 0.026 |
| 95% CI of OR Quart3 | 1.1 5.0 | 0.61 7.2 | 0.42 2.1 | 1.2 6.8 | >0.32 na | 0.62 3.3 | 0.98 71 | 0.18 23 | 1.2 29 |
| OR Quart 4 | 1.2 | 1.8 | 1.3 | 1.7 | >6.3 | 1.1 | 1.0 | 2.0 | 1.0 |
| p Value | 0.68 | 0.37 | 0.51 | 0.28 | <0.091 | 0.87 | 0.99 | 0.57 | 0.98 |
| 95% CI of OR Quart4 | 0.53 2.6 | 0.51 6.3 | 0.59 2.8 | 0.67 4.1 | >0.75 na | 0.46 2.5 | 0.062 17 | 0.18 22 | 0.14 7.5 |

**Pancreatic prohormone**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 51.1 | 59.9 | 51.1 | 61.9 | 51.1 | 63.0 |
| Average | 159 | 94.6 | 159 | 129 | 159 | 93.5 |
| Stdev | 247 | 107 | 247 | 182 | 247 | 77.2 |
| p(t-test) | | 0.13 | | 0.48 | | 0.18 |
| Min | 0.731 | 0.683 | 0.731 | 0.770 | 0.731 | 8.51 |
| Max | 1300 | 498 | 1300 | 877 | 1300 | 300 |
| n (Samp) | 84 | 36 | 84 | 42 | 84 | 26 |
| n (Patient) | 75 | 36 | 75 | 42 | 75 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 62.8 | 59.2 | 62.8 | 48.2 | 62.8 | 77.9 |
| Average | 170 | 77.8 | 170 | 90.9 | 170 | 82.8 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 276 | 73.6 | 276 | 126 | 276 | 43.0 |
| p(t-test) | | 0.30 | | 0.35 | | 0.35 |
| Min | 0.683 | 0.770 | 0.683 | 4.47 | 0.683 | 35.0 |
| Max | 1920 | 243 | 1920 | 443 | 1920 | 165 |
| n (Samp) | 201 | 10 | 201 | 11 | 201 | 9 |
| n (Patient) | 132 | 10 | 132 | 11 | 132 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 45.9 | 62.8 | 45.9 | 77.2 | 45.9 | 72.1 |
| Average | 128 | 125 | 128 | 162 | 128 | 100 |
| Stdev | 217 | 168 | 217 | 230 | 217 | 83.7 |
| p(t-test) | | 0.93 | | 0.43 | | 0.57 |
| Min | 0.731 | 0.683 | 0.731 | 0.770 | 0.731 | 8.51 |
| Max | 1300 | 784 | 1300 | 1080 | 1300 | 300 |
| n (Samp) | 74 | 30 | 74 | 43 | 74 | 20 |
| n (Patient) | 61 | 30 | 61 | 43 | 61 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | 0.44 | 0.55 | 0.52 | 0.43 | 0.58 | 0.54 | 0.52 | 0.58 |
| SE | 0.058 | 0.096 | 0.063 | 0.055 | 0.092 | 0.055 | 0.066 | 0.100 | 0.074 |
| p | 0.73 | 0.52 | 0.47 | 0.72 | 0.42 | 0.15 | 0.58 | 0.80 | 0.28 |
| nCohort 1 | 84 | 201 | 74 | 84 | 201 | 74 | 84 | 201 | 74 |
| nCohort 2 | 36 | 10 | 30 | 42 | 11 | 43 | 26 | 9 | 20 |
| Cutoff 1 | 30.1 | 31.4 | 31.4 | 37.9 | 33.4 | 35.9 | 35.9 | 47.7 | 38.4 |
| Sens 1 | 72% | 70% | 70% | 71% | 73% | 72% | 73% | 78% | 70% |
| Spec 1 | 33% | 27% | 36% | 39% | 28% | 39% | 38% | 43% | 43% |
| Cutoff 2 | 23.5 | 30.1 | 25.5 | 21.3 | 24.8 | 21.3 | 34.8 | 35.9 | 31.4 |
| Sens 2 | 81% | 80% | 80% | 81% | 82% | 81% | 81% | 89% | 80% |
| Spec 2 | 30% | 26% | 32% | 27% | 22% | 30% | 36% | 30% | 36% |
| Cutoff 3 | 0.731 | 13.7 | 0.731 | 14.9 | 18.9 | 14.9 | 21.3 | 34.8 | 21.3 |
| Sens 3 | 92% | 90% | 90% | 90% | 91% | 91% | 92% | 100% | 90% |
| Spec 3 | 2% | 10% | 3% | 20% | 16% | 22% | 27% | 29% | 30% |
| Cutoff 4 | 122 | 132 | 109 | 122 | 132 | 109 | 122 | 132 | 109 |
| Sens 4 | 28% | 20% | 37% | 29% | 18% | 37% | 27% | 11% | 35% |
| Spec 4 | 70% | 70% | 70% | 70% | 70% | 70% | 70% | 70% | 70% |
| Cutoff 5 | 218 | 211 | 151 | 218 | 211 | 151 | 218 | 211 | 151 |
| Sens 5 | 8% | 10% | 27% | 12% | 9% | 26% | 8% | 0% | 25% |
| Spec 5 | 81% | 80% | 81% | 81% | 80% | 81% | 81% | 80% | 81% |
| Cutoff 6 | 472 | 468 | 434 | 472 | 468 | 434 | 472 | 468 | 434 |
| Sens 6 | 3% | 0% | 7% | 7% | 0% | 12% | 0% | 0% | 0% |
| Spec 6 | 90% | 90% | 91% | 90% | 90% | 91% | 90% | 90% | 91% |
| OR Quart 2 | 1.6 | 4.2 | 1.9 | 1.7 | 1.0 | 1.6 | 3.8 | >3.1 | 3.5 |
| p Value | 0.41 | 0.20 | 0.34 | 0.32 | 1.0 | 0.40 | 0.070 | <0.33 | 0.15 |
| 95% CI of | 0.53 | 0.46 | 0.52 | 0.59 | 0.14 | 0.53 | 0.90 | >0.31 | 0.63 |
| OR Quart2 | 4.8 | 39 | 6.7 | 5.1 | 7.4 | 4.9 | 16 | na | 20 |
| OR Quart 3 | 1.4 | 3.1 | 1.9 | 2.1 | 2.1 | 1.4 | 3.4 | >6.8 | 3.7 |
| p Value | 0.57 | 0.33 | 0.34 | 0.18 | 0.41 | 0.57 | 0.10 | <0.082 | 0.14 |
| 95% CI of | 0.45 | 0.31 | 0.52 | 0.71 | 0.36 | 0.45 | 0.78 | >0.79 | 0.66 |
| OR Quart3 | 4.2 | 31 | 6.7 | 6.1 | 12 | 4.2 | 14 | na | 21 |
| OR Quart 4 | 0.84 | 2.1 | 2.2 | 1.1 | 1.5 | 2.3 | 2.2 | >0 | 3.5 |
| p Value | 0.77 | 0.55 | 0.22 | 0.84 | 0.65 | 0.13 | 0.31 | <na | 0.15 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of OR Quart4 | 0.26 2.7 | 0.18 24 | 0.63 7.9 | 0.37 3.4 | 0.25 9.5 | 0.78 6.8 | 0.49 9.8 | >na na | 0.63 20 |

**Transthyretin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 61700 | 52800 | 61700 | 52200 | 61700 | 54000 |
| Average | 64900 | 60600 | 64900 | 57000 | 64900 | 59000 |
| Stdev | 33300 | 42300 | 33300 | 36200 | 33300 | 42300 |
| p(t-test) |  | 0.39 |  | 0.14 |  | 0.52 |
| Min | 12800 | 6500 | 12800 | 8510 | 12800 | 14200 |
| Max | 242000 | 248000 | 242000 | 215000 | 242000 | 167000 |
| n (Samp) | 157 | 77 | 157 | 57 | 157 | 15 |
| n (Patient) | 89 | 77 | 89 | 57 | 89 | 15 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 56500 | 49800 | 56500 | 43500 | 56500 | 57500 |
| Average | 63200 | 64400 | 63200 | 60000 | 63200 | 54900 |
| Stdev | 38000 | 53400 | 38000 | 53300 | 38000 | 27700 |
| p(t-test) |  | 0.90 |  | 0.77 |  | 0.56 |
| Min | 6500 | 15700 | 6500 | 8510 | 6500 | 17700 |
| Max | 293000 | 248000 | 293000 | 215000 | 293000 | 102000 |
| n (Samp) | 376 | 22 | 376 | 13 | 376 | 7 |
| n (Patient) | 178 | 22 | 178 | 13 | 178 | 7 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 57800 | 49900 | 57800 | 52500 | 57800 | 54000 |
| Average | 65300 | 56700 | 65300 | 55500 | 65300 | 57700 |
| Stdev | 37700 | 35300 | 37700 | 28000 | 37700 | 38500 |
| p(t-test) |  | 0.11 |  | 0.066 |  | 0.43 |
| Min | 8940 | 6500 | 8940 | 9150 | 8940 | 14200 |
| Max | 248000 | 170000 | 248000 | 171000 | 248000 | 167000 |
| n (Samp) | 183 | 66 | 183 | 60 | 183 | 17 |
| n (Patient) | 91 | 66 | 91 | 60 | 91 | 17 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.43 | 0.45 | 0.42 | 0.41 | 0.42 | 0.43 | 0.41 | 0.45 | 0.42 |
| SE | 0.040 | 0.065 | 0.042 | 0.045 | 0.084 | 0.044 | 0.080 | 0.11 | 0.075 |
| p | 0.067 | 0.48 | 0.065 | 0.053 | 0.36 | 0.12 | 0.27 | 0.64 | 0.31 |
| nCohort 1 | 157 | 376 | 183 | 157 | 376 | 183 | 157 | 376 | 183 |
| nCohort 2 | 77 | 22 | 66 | 57 | 13 | 60 | 15 | 7 | 17 |
| Cutoff 1 | 34400 | 37200 | 32000 | 34200 | 27300 | 40100 | 28400 | 53000 | 34000 |
| Sens 1 | 70% | 73% | 71% | 70% | 77% | 70% | 73% | 71% | 71% |
| Spec 1 | 18% | 29% | 17% | 18% | 12% | 27% | 10% | 46% | 19% |
| Cutoff 2 | 29400 | 34400 | 28000 | 27000 | 26200 | 32800 | 22800 | 26200 | 20900 |
| Sens 2 | 81% | 82% | 80% | 81% | 85% | 80% | 80% | 86% | 82% |
| Spec 2 | 11% | 24% | 10% | 7% | 11% | 17% | 3% | 11% | 4% |
| Cutoff 3 | 20500 | 31700 | 19600 | 20900 | 16000 | 23200 | 14200 | 17400 | 16500 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 91% | 91% | 91% | 91% | 92% | 90% | 93% | 100% | 94% |
| Spec 3 | 3% | 20% | 3% | 3% | 3% | 4% | 1% | 4% | 2% |
| Cutoff 4 | 75500 | 73600 | 75200 | 75500 | 73600 | 75200 | 75500 | 73600 | 75200 |
| Sens 4 | 25% | 14% | 26% | 23% | 31% | 22% | 27% | 14% | 24% |
| Spec 4 | 70% | 70% | 70% | 70% | 70% | 70% | 70% | 70% | 70% |
| Cutoff 5 | 89900 | 85300 | 88500 | 89900 | 85300 | 88500 | 89900 | 85300 | 88500 |
| Sens 5 | 18% | 14% | 17% | 9% | 23% | 7% | 20% | 14% | 12% |
| Spec 5 | 80% | 80% | 80% | 80% | 80% | 80% | 80% | 80% | 80% |
| Cutoff 6 | 108000 | 114000 | 108000 | 108000 | 114000 | 108000 | 108000 | 114000 | 108000 |
| Sens 6 | 12% | 14% | 9% | 7% | 8% | 5% | 13% | 0% | 12% |
| Spec 6 | 90% | 90% | 90% | 90% | 90% | 90% | 90% | 90% | 90% |
| OR Quart 2 | 0.87 | 2.1 | 0.78 | 0.83 | 0.24 | 1.3 | 0.48 | 3.1 | 1.0 |
| p Value | 0.73 | 0.31 | 0.56 | 0.68 | 0.21 | 0.51 | 0.41 | 0.34 | 1.0 |
| 95% CI of OR Quart2 | 0.39 | 0.51 | 0.34 | 0.33 | 0.027 | 0.56 | 0.082 | 0.31 | 0.24 |
| | 1.9 | 8.6 | 1.8 | 2.1 | 2.2 | 3.1 | 2.7 | 30 | 4.2 |
| OR Quart 3 | 0.85 | 2.8 | 0.63 | 1.2 | 0.75 | 1.5 | 0.73 | 1.0 | 0.73 |
| p Value | 0.68 | 0.14 | 0.30 | 0.66 | 0.71 | 0.39 | 0.69 | 1.0 | 0.70 |
| 95% CI of OR Quart3 | 0.38 | 0.72 | 0.27 | 0.51 | 0.16 | 0.62 | 0.15 | 0.062 | 0.16 |
| | 1.9 | 11 | 1.5 | 2.9 | 3.4 | 3.4 | 3.5 | 16 | 3.5 |
| OR Quart 4 | 2.0 | 1.7 | 2.1 | 1.6 | 1.3 | 1.6 | 1.6 | 2.0 | 1.6 |
| p Value | 0.076 | 0.47 | 0.052 | 0.26 | 0.72 | 0.27 | 0.50 | 0.56 | 0.51 |
| 95% CI of OR Quart4 | 0.93 | 0.40 | 0.99 | 0.70 | 0.33 | 0.69 | 0.41 | 0.18 | 0.41 |
| | 4.2 | 7.4 | 4.5 | 3.8 | 4.9 | 3.8 | 6.1 | 23 | 5.9 |

**C-peptide EDTA**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2380 | 2820 | 2380 | 2170 | 2380 | 2020 |
| Average | 2850 | 3530 | 2850 | 2970 | 2850 | 2460 |
| Stdev | 2170 | 3160 | 2170 | 2610 | 2170 | 1860 |
| p(t-test) | | 0.035 | | 0.71 | | 0.34 |
| Min | 53.0 | 21.7 | 53.0 | 51.9 | 53.0 | 179 |
| Max | 13900 | 19500 | 13900 | 11800 | 13900 | 7060 |
| n (Samp) | 185 | 96 | 185 | 73 | 185 | 31 |
| n (Patient) | 109 | 96 | 109 | 73 | 109 | 31 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2400 | 3320 | 2400 | 2720 | 2400 | 3330 |
| Average | 3060 | 4060 | 3060 | 3630 | 3060 | 3330 |
| Stdev | 2480 | 4270 | 2480 | 3260 | 2480 | 2310 |
| p(t-test) | | 0.048 | | 0.32 | | 0.69 |
| Min | 7.96 | 157 | 7.96 | 114 | 7.96 | 88.8 |
| Max | 15400 | 19500 | 15400 | 11800 | 15400 | 7530 |
| n (Samp) | 461 | 28 | 461 | 20 | 461 | 13 |
| n (Patient) | 213 | 28 | 213 | 20 | 213 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2450 | 2590 | 2450 | 2090 | 2450 | 1490 |
| Average | 3130 | 3240 | 3130 | 2910 | 3130 | 2270 |
| Stdev | 2730 | 2530 | 2730 | 2470 | 2730 | 1810 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.76 | | 0.53 | | 0.11 |
| Min | 53.0 | 21.7 | 53.0 | 51.9 | 53.0 | 179 |
| Max | 21700 | 11300 | 21700 | 11100 | 21700 | 6630 |
| n (Samp) | 208 | 82 | 208 | 78 | 208 | 28 |
| n (Patient) | 106 | 82 | 106 | 78 | 106 | 28 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.54 | 0.52 | 0.49 | 0.53 | 0.47 | 0.44 | 0.55 | 0.40 |
| SE | 0.037 | 0.057 | 0.038 | 0.040 | 0.067 | 0.039 | 0.057 | 0.083 | 0.060 |
| p | 0.20 | 0.49 | 0.62 | 0.77 | 0.70 | 0.49 | 0.33 | 0.51 | 0.082 |
| nCohort 1 | 185 | 461 | 208 | 185 | 461 | 208 | 185 | 461 | 208 |
| nCohort 2 | 96 | 28 | 82 | 73 | 20 | 78 | 31 | 13 | 28 |
| Cutoff 1 | 1520 | 1290 | 1520 | 1140 | 1470 | 1210 | 929 | 1500 | 894 |
| Sens 1 | 71% | 71% | 71% | 71% | 70% | 71% | 71% | 77% | 71% |
| Spec 1 | 36% | 28% | 35% | 21% | 34% | 24% | 16% | 34% | 14% |
| Cutoff 2 | 1140 | 1030 | 1280 | 929 | 753 | 974 | 805 | 1310 | 786 |
| Sens 2 | 80% | 82% | 80% | 81% | 80% | 81% | 81% | 85% | 82% |
| Spec 2 | 21% | 18% | 25% | 16% | 10% | 15% | 14% | 28% | 13% |
| Cutoff 3 | 786 | 564 | 865 | 564 | 631 | 421 | 568 | 568 | 564 |
| Sens 3 | 91% | 93% | 90% | 90% | 90% | 91% | 90% | 92% | 93% |
| Spec 3 | 14% | 8% | 13% | 11% | 9% | 6% | 11% | 8% | 8% |
| Cutoff 4 | 3600 | 3770 | 4080 | 3600 | 3770 | 4080 | 3600 | 3770 | 4080 |
| Sens 4 | 36% | 36% | 27% | 32% | 40% | 29% | 26% | 38% | 14% |
| Spec 4 | 70% | 70% | 70% | 70% | 70% | 70% | 70% | 70% | 70% |
| Cutoff 5 | 4640 | 4720 | 4770 | 4640 | 4720 | 4770 | 4640 | 4720 | 4770 |
| Sens 5 | 23% | 25% | 20% | 22% | 35% | 19% | 13% | 23% | 7% |
| Spec 5 | 80% | 80% | 80% | 80% | 80% | 80% | 80% | 80% | 80% |
| Cutoff 6 | 5460 | 6090 | 6100 | 5460 | 6090 | 6100 | 5460 | 6090 | 6100 |
| Sens 6 | 19% | 14% | 15% | 11% | 20% | 6% | 6% | 15% | 7% |
| Spec 6 | 90% | 90% | 90% | 90% | 90% | 90% | 90% | 90% | 90% |
| OR Quart 2 | 1.2 | 0.61 | 1.5 | 0.53 | 0.49 | 0.48 | 1.4 | 1.5 | 1.9 |
| p Value | 0.59 | 0.40 | 0.29 | 0.12 | 0.32 | 0.063 | 0.57 | 0.66 | 0.35 |
| 95% CI of OR Quart2 | 0.60 2.5 | 0.19 1.9 | 0.71 3.1 | 0.24 1.2 | 0.12 2.0 | 0.22 1.0 | 0.45 4.3 | 0.25 9.1 | 0.51 6.7 |
| OR Quart 3 | 1.5 | 0.87 | 1.6 | 0.74 | 0.66 | 0.76 | 1.0 | 2.0 | 1.6 |
| p Value | 0.29 | 0.79 | 0.20 | 0.44 | 0.52 | 0.46 | 1.0 | 0.42 | 0.51 |
| 95% CI of OR Quart3 | 0.73 3.0 | 0.30 2.5 | 0.78 3.4 | 0.35 1.6 | 0.18 2.4 | 0.37 1.6 | 0.30 3.3 | 0.37 11 | 0.42 5.8 |
| OR Quart 4 | 1.2 | 0.99 | 1.1 | 1.1 | 1.2 | 1.0 | 2.0 | 2.0 | 3.2 |
| p Value | 0.63 | 0.99 | 0.88 | 0.80 | 0.79 | 0.95 | 0.19 | 0.42 | 0.063 |
| 95% CI of OR Quart4 | 0.59 2.4 | 0.36 2.7 | 0.49 2.3 | 0.53 2.3 | 0.38 3.6 | 0.51 2.1 | 0.70 6.0 | 0.36 11 | 0.94 11 |

**Gastric inhibitory polypeptide EDTA**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 28.0 | 27.2 | 28.0 | 22.8 | 28.0 | 17.2 |
| Average | 57.6 | 51.4 | 57.6 | 55.8 | 57.6 | 35.7 |
| Stdev | 101 | 62.1 | 101 | 72.2 | 101 | 41.2 |
| p(t-test) | | 0.59 | | 0.89 | | 0.24 |
| Min | 0.888 | 2.35 | 0.888 | 2.33 | 0.888 | 2.35 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 820 | 391 | 820 | 330 | 820 | 160 |
| n (Samp) | 185 | 96 | 185 | 73 | 185 | 31 |
| n (Patient) | 109 | 96 | 109 | 73 | 109 | 31 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 25.0 | 28.9 | 25.0 | 17.6 | 25.0 | 32.7 |
| Average | 55.3 | 61.7 | 55.3 | 59.8 | 55.3 | 57.0 |
| Stdev | 84.0 | 81.0 | 84.0 | 80.7 | 84.0 | 69.5 |
| p(t-test) | | 0.70 | | 0.81 | | 0.94 |
| Min | 0.888 | 4.43 | 0.888 | 7.47 | 0.888 | 16.6 |
| Max | 820 | 391 | 820 | 289 | 820 | 251 |
| n (Samp) | 461 | 28 | 461 | 20 | 461 | 13 |
| n (Patient) | 213 | 28 | 213 | 20 | 213 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 25.6 | 27.2 | 25.6 | 23.6 | 25.6 | 14.6 |
| Average | 59.7 | 48.0 | 59.7 | 46.2 | 59.7 | 30.3 |
| Stdev | 102 | 53.5 | 102 | 60.8 | 102 | 36.1 |
| p(t-test) | | 0.33 | | 0.27 | | 0.13 |
| Min | 0.888 | 2.35 | 0.888 | 2.33 | 0.888 | 2.35 |
| Max | 820 | 248 | 820 | 330 | 820 | 146 |
| n (Samp) | 208 | 82 | 208 | 78 | 208 | 28 |
| n (Patient) | 106 | 82 | 106 | 78 | 106 | 28 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.52 | 0.55 | 0.51 | 0.50 | 0.50 | 0.48 | 0.43 | 0.59 | 0.39 |
| SE | 0.036 | 0.058 | 0.038 | 0.040 | 0.066 | 0.039 | 0.057 | 0.084 | 0.060 |
| p | 0.62 | 0.38 | 0.80 | 0.94 | 0.95 | 0.64 | 0.19 | 0.28 | 0.058 |
| nCohort 1 | 185 | 461 | 208 | 185 | 461 | 208 | 185 | 461 | 208 |
| nCohort 2 | 96 | 28 | 82 | 73 | 20 | 78 | 31 | 13 | 28 |
| Cutoff 1 | 15.0 | 17.1 | 14.2 | 13.5 | 16.1 | 13.5 | 13.0 | 19.4 | 9.80 |
| Sens 1 | 71% | 71% | 71% | 71% | 70% | 71% | 71% | 77% | 71% |
| Spec 1 | 34% | 38% | 32% | 29% | 36% | 30% | 28% | 42% | 21% |
| Cutoff 2 | 11.1 | 14.2 | 9.32 | 11.1 | 12.8 | 10.6 | 8.09 | 17.1 | 7.52 |
| Sens 2 | 80% | 82% | 80% | 81% | 80% | 81% | 81% | 85% | 82% |
| Spec 2 | 22% | 31% | 20% | 22% | 26% | 23% | 14% | 38% | 13% |
| Cutoff 3 | 7.92 | 10.2 | 7.37 | 7.74 | 9.89 | 7.37 | 6.56 | 16.8 | 5.69 |
| Sens 3 | 91% | 93% | 90% | 90% | 90% | 91% | 90% | 92% | 93% |
| Spec 3 | 13% | 19% | 13% | 12% | 19% | 13% | 11% | 38% | 9% |
| Cutoff 4 | 42.7 | 48.7 | 43.5 | 42.7 | 48.7 | 43.5 | 42.7 | 48.7 | 43.5 |
| Sens 4 | 38% | 36% | 38% | 33% | 30% | 29% | 26% | 23% | 21% |
| Spec 4 | 70% | 70% | 70% | 70% | 70% | 70% | 70% | 70% | 70% |
| Cutoff 5 | 68.1 | 80.9 | 72.2 | 68.1 | 80.9 | 72.2 | 68.1 | 80.9 | 72.2 |
| Sens 5 | 24% | 25% | 18% | 25% | 25% | 17% | 19% | 15% | 18% |
| Spec 5 | 80% | 80% | 80% | 80% | 80% | 80% | 80% | 80% | 80% |
| Cutoff 6 | 132 | 132 | 165 | 132 | 132 | 165 | 132 | 132 | 165 |
| Sens 6 | 10% | 14% | 5% | 14% | 15% | 6% | 6% | 15% | 0% |
| Spec 6 | 90% | 90% | 90% | 90% | 90% | 90% | 90% | 90% | 90% |
| OR Quart 2 | 1.5 | 2.1 | 0.74 | 1.4 | 2.1 | 1.3 | 0.54 | >6.3 | 0.15 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value | 0.29 | 0.25 | 0.43 | 0.37 | 0.25 | 0.53 | 0.35 | <0.092 | 0.086 |
| 95% CI of | 0.73 | 0.61 | 0.36 | 0.66 | 0.61 | 0.60 | 0.15 | >0.74 | 0.018 |
| OR Quart2 | 3.0 | 7.1 | 1.5 | 3.0 | 7.1 | 2.7 | 2.0 | na | 1.3 |
| OR Quart 3 | 0.87 | 2.1 | 0.76 | 0.70 | 0.74 | 1.4 | 1.9 | >5.2 | 2.3 |
| p Value | 0.71 | 0.25 | 0.46 | 0.41 | 0.70 | 0.35 | 0.21 | <0.13 | 0.13 |
| 95% CI of | 0.42 | 0.61 | 0.36 | 0.31 | 0.16 | 0.68 | 0.69 | >0.60 | 0.78 |
| OR Quart3 | 1.8 | 7.1 | 1.6 | 1.6 | 3.4 | 3.0 | 5.3 | na | 6.5 |
| OR Quart 4 | 1.6 | 2.1 | 1.1 | 1.3 | 1.2 | 1.2 | 1.2 | >2.0 | 1.6 |
| p Value | 0.18 | 0.25 | 0.76 | 0.47 | 0.74 | 0.66 | 0.78 | <0.57 | 0.41 |
| 95% CI of | 0.80 | 0.60 | 0.55 | 0.62 | 0.33 | 0.55 | 0.39 | >0.18 | 0.53 |
| OR Quart4 | 3.2 | 7.0 | 2.2 | 2.8 | 4.8 | 2.5 | 3.5 | na | 4.8 |

**Peptide YY EDTA**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 38.2 | 39.6 | 38.2 | 44.3 | 38.2 | 50.0 |
| Average | 56.6 | 55.6 | 56.6 | 64.1 | 56.6 | 82.9 |
| Stdev | 85.0 | 53.0 | 85.0 | 62.2 | 85.0 | 80.0 |
| p(t-test) |  | 0.92 |  | 0.49 |  | 0.11 |
| Min | 0.0108 | 0.00999 | 0.0108 | 0.00999 | 0.0108 | 0.0108 |
| Max | 789 | 284 | 789 | 299 | 789 | 406 |
| n (Samp) | 185 | 96 | 185 | 73 | 185 | 31 |
| n (Patient) | 109 | 96 | 109 | 73 | 109 | 31 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 39.3 | 42.8 | 39.3 | 65.6 | 39.3 | 47.9 |
| Average | 59.1 | 51.1 | 59.1 | 75.6 | 59.1 | 64.5 |
| Stdev | 78.3 | 42.7 | 78.3 | 64.4 | 78.3 | 35.5 |
| p(t-test) |  | 0.59 |  | 0.36 |  | 0.80 |
| Min | 0.00999 | 0.153 | 0.00999 | 1.06 | 0.00999 | 34.8 |
| Max | 789 | 158 | 789 | 299 | 789 | 146 |
| n (Samp) | 461 | 28 | 461 | 20 | 461 | 13 |
| n (Patient) | 213 | 28 | 213 | 20 | 213 | 13 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 40.4 | 38.1 | 40.4 | 40.6 | 40.4 | 41.6 |
| Average | 57.0 | 54.7 | 57.0 | 56.2 | 57.0 | 73.3 |
| Stdev | 79.0 | 55.5 | 79.0 | 56.5 | 79.0 | 86.6 |
| p(t-test) |  | 0.80 |  | 0.93 |  | 0.31 |
| Min | 0.0108 | 0.00999 | 0.0108 | 0.00999 | 0.0108 | 0.00999 |
| Max | 789 | 284 | 789 | 270 | 789 | 406 |
| n (Samp) | 208 | 82 | 208 | 78 | 208 | 28 |
| n (Patient) | 106 | 82 | 106 | 78 | 106 | 28 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.54 | 0.51 | 0.50 | 0.57 | 0.65 | 0.50 | 0.64 | 0.65 | 0.53 |
| SE | 0.037 | 0.057 | 0.038 | 0.040 | 0.068 | 0.038 | 0.057 | 0.084 | 0.059 |
| p | 0.32 | 0.87 | 0.96 | 0.071 | 0.033 | 0.96 | 0.014 | 0.079 | 0.67 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 185 | 461 | 208 | 185 | 461 | 208 | 185 | 461 | 208 |
| nCohort 2 | 96 | 28 | 82 | 73 | 20 | 78 | 31 | 13 | 28 |
| Cutoff 1 | 25.2 | 20.4 | 24.3 | 32.7 | 43.7 | 25.5 | 34.7 | 37.8 | 28.9 |
| Sens 1 | 71% | 71% | 71% | 71% | 70% | 71% | 71% | 77% | 71% |
| Spec 1 | 35% | 26% | 31% | 43% | 56% | 33% | 45% | 47% | 35% |
| Cutoff 2 | 18.0 | 12.6 | 14.1 | 21.5 | 33.6 | 16.8 | 32.1 | 36.5 | 0.0512 |
| Sens 2 | 80% | 82% | 80% | 81% | 80% | 81% | 81% | 85% | 82% |
| Spec 2 | 25% | 18% | 19% | 29% | 41% | 21% | 43% | 46% | 5% |
| Cutoff 3 | 9.90 | 6.44 | 6.60 | 4.70 | 24.9 | 2.37 | 2.80 | 35.9 | 0.00999 |
| Sens 3 | 91% | 93% | 90% | 90% | 90% | 91% | 90% | 92% | 96% |
| Spec 3 | 17% | 13% | 13% | 13% | 31% | 10% | 11% | 45% | 0% |
| Cutoff 4 | 54.8 | 57.4 | 57.9 | 54.8 | 57.4 | 57.9 | 54.8 | 57.4 | 57.9 |
| Sens 4 | 36% | 36% | 32% | 40% | 60% | 31% | 48% | 38% | 39% |
| Spec 4 | 70% | 70% | 70% | 70% | 70% | 70% | 70% | 70% | 70% |
| Cutoff 5 | 70.6 | 82.5 | 78.8 | 70.6 | 82.5 | 78.8 | 70.6 | 82.5 | 78.8 |
| Sens 5 | 21% | 21% | 18% | 29% | 30% | 21% | 48% | 31% | 39% |
| Spec 5 | 80% | 80% | 80% | 80% | 80% | 80% | 80% | 80% | 80% |
| Cutoff 6 | 110 | 116 | 112 | 110 | 116 | 112 | 110 | 116 | 112 |
| Sens 6 | 10% | 7% | 10% | 15% | 15% | 14% | 29% | 15% | 25% |
| Spec 6 | 90% | 90% | 90% | 90% | 90% | 90% | 90% | 90% | 90% |
| OR Quart 2 | 1.3 | 0.74 | 0.83 | 1.4 | 2.0 | 1.2 | 1.6 | >5.2 | 1.0 |
| p Value | 0.47 | 0.58 | 0.61 | 0.43 | 0.42 | 0.61 | 0.51 | <0.14 | 1.0 |
| 95% CI of | 0.64 | 0.25 | 0.40 | 0.61 | 0.37 | 0.58 | 0.42 | >0.60 | 0.33 |
| OR Quart2 | 2.6 | 2.2 | 1.7 | 3.2 | 11 | 2.5 | 5.9 | na | 3.1 |
| OR Quart 3 | 1.1 | 0.74 | 1.1 | 1.8 | 2.6 | 1.1 | 1.6 | >3.1 | 0.40 |
| p Value | 0.72 | 0.58 | 0.86 | 0.16 | 0.27 | 0.85 | 0.51 | <0.33 | 0.20 |
| 95% CI of | 0.56 | 0.25 | 0.52 | 0.80 | 0.49 | 0.51 | 0.42 | >0.32 | 0.098 |
| OR Quart3 | 2.3 | 2.2 | 2.2 | 4.0 | 13 | 2.3 | 5.9 | na | 1.6 |
| OR Quart 4 | 1.4 | 0.99 | 1.0 | 2.1 | 4.7 | 1.1 | 4.8 | >5.2 | 1.7 |
| p Value | 0.32 | 0.99 | 0.96 | 0.059 | 0.050 | 0.74 | 0.0091 | <0.14 | 0.31 |
| 95% CI of | 0.71 | 0.36 | 0.50 | 0.97 | 1.0 | 0.54 | 1.5 | >0.60 | 0.61 |
| OR Quart4 | 2.9 | 2.7 | 2.1 | 4.7 | 22 | 2.4 | 16 | na | 4.7 |

Table 6: Comparison of marker levels in EDTA samples collected from Cohort 1 (patients that did not progress beyond RIFLE stage 0 or R) and in EDTA samples collected from subjects at 0, 24 hours, and 48 hours prior to reaching stage I or F in Cohort 2.

**Agouti-related protein**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0638 | 0.0818 | 0.0638 | 0.0707 | 0.0638 | 0.0710 |
| Average | 0.0814 | 0.154 | 0.0814 | 0.0898 | 0.0814 | 0.0865 |
| Stdev | 0.0564 | 0.248 | 0.0564 | 0.0667 | 0.0564 | 0.0555 |
| p(t-test) | | 4.4E-5 | | 0.45 | | 0.70 |
| Min | 0.00624 | 0.0215 | 0.00624 | 0.00924 | 0.00624 | 0.0253 |
| Max | 0.485 | 1.34 | 0.485 | 0.258 | 0.485 | 0.235 |
| n (Samp) | 306 | 28 | 306 | 29 | 306 | 19 |
| n (Patient) | 143 | 28 | 143 | 29 | 143 | 19 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0674 | 0.0818 | 0.0674 | 0.0768 | 0.0674 | 0.0710 |
| Average | 0.0837 | 0.157 | 0.0837 | 0.0949 | 0.0837 | 0.0805 |
| Stdev | 0.0573 | 0.248 | 0.0573 | 0.0712 | 0.0573 | 0.0439 |
| p(t-test) | | 4.8E-5 | | 0.32 | | 0.82 |
| Min | 0.00624 | 0.0215 | 0.00624 | 0.00924 | 0.00624 | 0.0253 |
| Max | 0.485 | 1.34 | 0.485 | 0.258 | 0.485 | 0.160 |
| n (Samp) | 305 | 28 | 305 | 30 | 305 | 17 |
| n (Patient) | 138 | 28 | 138 | 30 | 138 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | nd | 0.59 | 0.53 | nd | 0.53 | 0.54 | nd | 0.51 |
| SE | 0.059 | nd | 0.059 | 0.057 | nd | 0.056 | 0.070 | nd | 0.073 |
| p | 0.11 | nd | 0.14 | 0.63 | nd | 0.58 | 0.61 | nd | 0.87 |
| nCohort 1 | 306 | nd | 305 | 306 | nd | 305 | 306 | nd | 305 |
| nCohort 2 | 28 | nd | 28 | 29 | nd | 30 | 19 | nd | 17 |
| Cutoff 1 | 0.0574 | nd | 0.0574 | 0.0489 | nd | 0.0526 | 0.0500 | nd | 0.0531 |
| Sens 1 | 71% | nd | 71% | 72% | nd | 70% | 74% | nd | 71% |
| Spec 1 | 43% | nd | 41% | 33% | nd | 37% | 35% | nd | 37% |
| Cutoff 2 | 0.0357 | nd | 0.0357 | 0.0350 | nd | 0.0434 | 0.0387 | nd | 0.0387 |
| Sens 2 | 82% | nd | 82% | 83% | nd | 80% | 84% | nd | 82% |
| Spec 2 | 17% | nd | 15% | 16% | nd | 24% | 22% | nd | 20% |
| Cutoff 3 | 0.0262 | nd | 0.0262 | 0.0212 | nd | 0.0272 | 0.0271 | nd | 0.0271 |
| Sens 3 | 93% | nd | 93% | 93% | nd | 90% | 95% | nd | 94% |
| Spec 3 | 8% | nd | 8% | 5% | nd | 9% | 9% | nd | 9% |
| Cutoff 4 | 0.0951 | nd | 0.100 | 0.0951 | nd | 0.100 | 0.0951 | nd | 0.100 |
| Sens 4 | 43% | nd | 39% | 34% | nd | 37% | 32% | nd | 29% |
| Spec 4 | 70% | nd | 70% | 70% | nd | 70% | 70% | nd | 70% |
| Cutoff 5 | 0.123 | nd | 0.126 | 0.123 | nd | 0.126 | 0.123 | nd | 0.126 |
| Sens 5 | 36% | nd | 36% | 21% | nd | 23% | 21% | nd | 18% |
| Spec 5 | 80% | nd | 80% | 80% | nd | 80% | 80% | nd | 80% |
| Cutoff 6 | 0.154 | nd | 0.161 | 0.154 | nd | 0.161 | 0.154 | nd | 0.161 |
| Sens 6 | 29% | nd | 29% | 14% | nd | 17% | 16% | nd | 0% |
| Spec 6 | 90% | nd | 90% | 90% | nd | 90% | 90% | nd | 90% |
| OR Quart 2 | 0.40 | nd | 0.41 | 1.2 | nd | 0.59 | 0.58 | nd | 0.99 |
| p Value | 0.20 | nd | 0.20 | 0.79 | nd | 0.38 | 0.47 | nd | 0.99 |
| 95% CI of OR Quart2 | 0.10 1.6 | nd | 0.10 1.6 | 0.37 3.6 | nd | 0.19 1.9 | 0.13 2.5 | nd | 0.24 4.1 |
| OR Quart 3 | 1.2 | nd | 1.2 | 1.2 | nd | 0.85 | 1.0 | nd | 1.0 |
| p Value | 0.79 | nd | 0.79 | 0.79 | nd | 0.77 | 1.0 | nd | 1.0 |
| 95% CI of OR Quart3 | 0.40 3.4 | nd | 0.40 3.4 | 0.37 3.6 | nd | 0.29 2.5 | 0.28 3.6 | nd | 0.24 4.1 |
| OR Quart 4 | 1.5 | nd | 1.5 | 1.5 | nd | 1.3 | 1.2 | nd | 1.2 |
| p Value | 0.46 | nd | 0.46 | 0.43 | nd | 0.64 | 0.77 | nd | 0.75 |
| 95% CI of OR Quart4 | 0.53 4.1 | nd | 0.53 4.1 | 0.52 4.5 | nd | 0.47 3.4 | 0.35 4.1 | nd | 0.32 4.8 |

**Osteocalcin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2030 | 1980 | 2030 | 1260 | 2030 | 1480 |
| Average | 2700 | 2480 | 2700 | 2430 | 2700 | 1970 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 2620 | 1960 | 2620 | 3000 | 2620 | 1770 |
| p(t-test) | | 0.63 | | 0.53 | | 0.13 |
| Min | 0.729 | 213 | 0.729 | 59.3 | 0.729 | 221 |
| Max | 21800 | 8950 | 21800 | 17700 | 21800 | 6930 |
| n (Samp) | 426 | 35 | 426 | 42 | 426 | 31 |
| n (Patient) | 209 | 35 | 209 | 42 | 209 | 31 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | nd | nd | 1960 | 1350 |
| Average | nd | nd | nd | nd | 2650 | 1740 |
| Stdev | nd | nd | nd | nd | 2560 | 1400 |
| p(t-test) | nd | nd | nd | nd | | 0.32 |
| Min | nd | nd | nd | nd | 0.729 | 336 |
| Max | nd | nd | nd | nd | 21800 | 4840 |
| n (Samp) | nd | nd | nd | nd | 571 | 8 |
| n (Patient) | nd | nd | nd | nd | 256 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2050 | 2160 | 2050 | 1650 | 2050 | 1240 |
| Average | 2750 | 2530 | 2750 | 2560 | 2750 | 1910 |
| Stdev | 2680 | 1970 | 2680 | 3020 | 2680 | 1800 |
| p(t-test) | | 0.65 | | 0.66 | | 0.11 |
| Min | 0.729 | 213 | 0.729 | 59.3 | 0.729 | 221 |
| Max | 21800 | 8950 | 21800 | 17700 | 21800 | 6930 |
| n (Samp) | 407 | 32 | 407 | 42 | 407 | 27 |
| n (Patient) | 189 | 32 | 189 | 42 | 189 | 27 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | nd | 0.50 | 0.43 | nd | 0.45 | 0.40 | 0.39 | 0.38 |
| SE | 0.051 | nd | 0.053 | 0.048 | nd | 0.048 | 0.055 | 0.11 | 0.059 |
| p | 0.98 | nd | 0.95 | 0.12 | nd | 0.27 | 0.079 | 0.30 | 0.052 |
| nCohort 1 | 426 | nd | 407 | 426 | nd | 407 | 426 | 571 | 407 |
| nCohort 2 | 35 | nd | 32 | 42 | nd | 42 | 31 | 8 | 27 |
| Cutoff 1 | 1220 | nd | 1220 | 1020 | nd | 1020 | 865 | 1020 | 813 |
| Sens 1 | 71% | nd | 72% | 71% | nd | 71% | 71% | 75% | 70% |
| Spec 1 | 30% | nd | 30% | 23% | nd | 23% | 18% | 25% | 17% |
| Cutoff 2 | 909 | nd | 909 | 686 | nd | 686 | 686 | 865 | 660 |
| Sens 2 | 80% | nd | 81% | 81% | nd | 81% | 81% | 88% | 81% |
| Spec 2 | 20% | nd | 20% | 12% | nd | 13% | 12% | 19% | 12% |
| Cutoff 3 | 555 | nd | 555 | 553 | nd | 553 | 443 | 323 | 443 |
| Sens 3 | 91% | nd | 91% | 90% | nd | 90% | 90% | 100% | 93% |
| Spec 3 | 10% | nd | 10% | 9% | nd | 10% | 8% | 5% | 8% |
| Cutoff 4 | 2890 | nd | 2930 | 2890 | nd | 2930 | 2890 | 2810 | 2930 |
| Sens 4 | 29% | nd | 31% | 21% | nd | 24% | 19% | 12% | 19% |
| Spec 4 | 70% | nd | 70% | 70% | nd | 70% | 70% | 70% | 70% |
| Cutoff 5 | 3800 | nd | 3980 | 3800 | nd | 3980 | 3800 | 3780 | 3980 |
| Sens 5 | 17% | nd | 16% | 19% | nd | 21% | 13% | 12% | 11% |
| Spec 5 | 80% | nd | 80% | 80% | nd | 80% | 80% | 80% | 80% |
| Cutoff 6 | 5550 | nd | 6010 | 5550 | nd | 6010 | 5550 | 5550 | 6010 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 6 | 6% | nd | 6% | 12% | nd | 7% | 6% | 0% | 7% |
| Spec 6 | 90% | nd | 90% | 90% | nd | 90% | 90% | 90% | 90% |
| OR Quart 2 | 0.69 | nd | 0.99 | 0.65 | nd | 0.69 | 1.4 | 1.0 | 2.4 |
| p Value | 0.46 | nd | 0.98 | 0.43 | nd | 0.46 | 0.54 | 1.0 | 0.20 |
| 95% CI of OR Quart2 | 0.25 1.9 | nd | 0.36 2.7 | 0.22 1.9 | nd | 0.25 1.9 | 0.44 4.7 | 0.062 16 | 0.62 9.7 |
| OR Quart 3 | 0.90 | nd | 0.73 | 1.6 | nd | 1.2 | 1.4 | 3.0 | 2.1 |
| p Value | 0.83 | nd | 0.57 | 0.28 | nd | 0.64 | 0.54 | 0.34 | 0.32 |
| 95% CI of OR Quart3 | 0.35 2.3 | nd | 0.24 2.2 | 0.68 3.9 | nd | 0.51 3.0 | 0.44 4.7 | 0.31 30 | 0.50 8.4 |
| OR Quart 4 | 0.90 | nd | 1.3 | 1.5 | nd | 1.4 | 2.6 | 3.1 | 4.0 |
| p Value | 0.83 | nd | 0.64 | 0.37 | nd | 0.50 | 0.084 | 0.33 | 0.037 |
| 95% CI of OR Quart4 | 0.35 2.3 | nd | 0.48 3.3 | 0.62 3.7 | nd | 0.57 3.2 | 0.88 7.6 | 0.31 30 | 1.1 15 |

**Complement factor H**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 185000 | 179000 | 185000 | 182000 | 185000 | 185000 |
| Average | 193000 | 186000 | 193000 | 187000 | 193000 | 198000 |
| Stdev | 82900 | 67500 | 82900 | 51400 | 82900 | 64100 |
| p(t-test) | | 0.67 | | 0.70 | | 0.77 |
| Min | 14900 | 48200 | 14900 | 92800 | 14900 | 75300 |
| Max | 1070000 | 394000 | 1070000 | 331000 | 1070000 | 347000 |
| n (Samp) | 356 | 28 | 356 | 35 | 356 | 20 |
| n (Patient) | 178 | 28 | 178 | 35 | 178 | 20 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 184000 | 179000 | 184000 | 179000 | 184000 | 181000 |
| Average | 192000 | 184000 | 192000 | 185000 | 192000 | 191000 |
| Stdev | 83400 | 69200 | 83400 | 52500 | 83400 | 60000 |
| p(t-test) | | 0.61 | | 0.60 | | 0.93 |
| Min | 14900 | 48200 | 14900 | 92800 | 14900 | 75300 |
| Max | 1070000 | 394000 | 1070000 | 331000 | 1070000 | 347000 |
| n (Samp) | 347 | 28 | 347 | 35 | 347 | 18 |
| n (Patient) | 165 | 28 | 165 | 35 | 165 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | nd | 0.48 | 0.50 | nd | 0.49 | 0.53 | nd | 0.51 |
| SE | 0.057 | nd | 0.057 | 0.051 | nd | 0.052 | 0.068 | nd | 0.070 |
| p | 0.82 | nd | 0.79 | 0.99 | nd | 0.85 | 0.63 | nd | 0.92 |
| nCohort 1 | 356 | nd | 347 | 356 | nd | 347 | 356 | nd | 347 |
| nCohort 2 | 28 | nd | 28 | 35 | nd | 35 | 20 | nd | 18 |
| Cutoff 1 | 156000 | nd | 156000 | 150000 | nd | 147000 | 165000 | nd | 163000 |
| Sens 1 | 71% | nd | 71% | 71% | nd | 71% | 70% | nd | 72% |
| Spec 1 | 34% | nd | 34% | 31% | nd | 29% | 38% | nd | 37% |
| Cutoff 2 | 126000 | nd | 126000 | 143000 | nd | 143000 | 159000 | nd | 154000 |
| Sens 2 | 82% | nd | 82% | 80% | nd | 80% | 80% | nd | 83% |
| Spec 2 | 15% | nd | 15% | 26% | nd | 25% | 35% | nd | 34% |
| Cutoff 3 | 95800 | nd | 89900 | 128000 | nd | 123000 | 146000 | nd | 135000 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 3 | 93% | nd | 93% | 91% | nd | 91% | 90% | nd | 94% |
| Spec 3 | 5% | nd | 4% | 17% | nd | 15% | 28% | nd | 20% |
| Cutoff 4 | 214000 | nd | 214000 | 214000 | nd | 214000 | 214000 | nd | 214000 |
| Sens 4 | 36% | nd | 36% | 29% | nd | 31% | 25% | nd | 22% |
| Spec 4 | 70% | nd | 70% | 70% | nd | 70% | 70% | nd | 70% |
| Cutoff 5 | 241000 | nd | 242000 | 241000 | nd | 242000 | 241000 | nd | 242000 |
| Sens 5 | 14% | nd | 14% | 14% | nd | 14% | 20% | nd | 17% |
| Spec 5 | 80% | nd | 80% | 80% | nd | 80% | 80% | nd | 80% |
| Cutoff 6 | 286000 | nd | 291000 | 286000 | nd | 291000 | 286000 | nd | 291000 |
| Sens 6 | 4% | nd | 4% | 3% | nd | 3% | 10% | nd | 6% |
| Spec 6 | 90% | nd | 90% | 90% | nd | 90% | 90% | nd | 90% |
| OR Quart 2 | 0.70 | nd | 0.70 | 2.1 | nd | 1.0 | 4.3 | nd | 3.7 |
| p Value | 0.55 | nd | 0.55 | 0.15 | nd | 0.98 | 0.071 | nd | 0.11 |
| 95% CI of OR Quart2 | 0.21 | nd | 0.21 | 0.76 | nd | 0.36 | 0.88 | nd | 0.75 |
|  | 2.3 | nd | 2.3 | 5.9 | nd | 2.8 | 21 | nd | 18 |
| OR Quart 3 | 1.3 | nd | 1.3 | 1.5 | nd | 1.6 | 2.6 | nd | 2.6 |
| p Value | 0.60 | nd | 0.60 | 0.43 | nd | 0.35 | 0.26 | nd | 0.26 |
| 95% CI of OR Quart3 | 0.47 | nd | 0.47 | 0.52 | nd | 0.61 | 0.49 | nd | 0.49 |
|  | 3.7 | nd | 3.7 | 4.5 | nd | 4.0 | 14 | nd | 14 |
| OR Quart 4 | 1.0 | nd | 1.0 | 1.3 | nd | 0.88 | 2.6 | nd | 2.0 |
| p Value | 1.0 | nd | 0.98 | 0.59 | nd | 0.80 | 0.26 | nd | 0.42 |
| 95% CI of OR Quart4 | 0.34 | nd | 0.34 | 0.45 | nd | 0.30 | 0.49 | nd | 0.36 |
|  | 3.0 | nd | 3.0 | 4.0 | nd | 2.5 | 14 | nd | 11 |

**Ciliary neurotrophic factor**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0129 | 0.00367 | 0.0129 | 0.0129 | 0.0129 | 0.0767 |
| Average | 0.282 | 0.224 | 0.282 | 0.104 | 0.282 | 0.181 |
| Stdev | 0.785 | 0.470 | 0.785 | 0.152 | 0.785 | 0.301 |
| p(t-test) |  | 0.70 |  | 0.22 |  | 0.58 |
| Min | 8.69E-5 | 8.69E-5 | 8.69E-5 | 8.69E-5 | 8.69E-5 | 8.69E-5 |
| Max | 6.40 | 2.02 | 6.40 | 0.548 | 6.40 | 1.29 |
| n (Samp) | 306 | 28 | 306 | 29 | 306 | 19 |
| n (Patient) | 143 | 28 | 143 | 29 | 143 | 19 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0129 | 0.00367 | 0.0129 | 0.0448 | 0.0129 | 0.0651 |
| Average | 0.283 | 0.210 | 0.283 | 0.123 | 0.283 | 0.167 |
| Stdev | 0.786 | 0.449 | 0.786 | 0.183 | 0.786 | 0.314 |
| p(t-test) |  | 0.63 |  | 0.27 |  | 0.55 |
| Min | 8.69E-5 | 8.69E-5 | 8.69E-5 | 8.69E-5 | 8.69E-5 | 8.69E-5 |
| Max | 6.40 | 2.02 | 6.40 | 0.686 | 6.40 | 1.29 |
| n (Samp) | 305 | 28 | 305 | 30 | 305 | 17 |
| n (Patient) | 138 | 28 | 138 | 30 | 138 | 17 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.46 | nd | 0.46 | 0.47 | nd | 0.49 | 0.54 | nd | 0.50 |
| SE | 0.058 | nd | 0.058 | 0.057 | nd | 0.056 | 0.070 | nd | 0.072 |
| p | 0.53 | nd | 0.53 | 0.65 | nd | 0.84 | 0.59 | nd | 0.95 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 306 | nd | 305 | 306 | nd | 305 | 306 | nd | 305 |
| nCohort 2 | 28 | nd | 28 | 29 | nd | 30 | 19 | nd | 17 |
| Cutoff 1 | 0.000179 | nd | 0.000179 | 0.000173 | nd | 0.000179 | 0.000179 | nd | 0.000179 |
| Sens 1 | 71% | nd | 71% | 76% | nd | 70% | 79% | nd | 76% |
| Spec 1 | 24% | nd | 25% | 16% | nd | 25% | 24% | nd | 25% |
| Cutoff 2 | 8.69E-5 | nd | 8.69E-5 | 8.69E-5 | nd | 8.69E-5 | 0.000173 | nd | 0.000173 |
| Sens 2 | 89% | nd | 89% | 90% | nd | 90% | 89% | nd | 88% |
| Spec 2 | 8% | nd | 9% | 8% | nd | 9% | 16% | nd | 17% |
| Cutoff 3 | 0 | nd | 0 | 0 | nd | 8.69E-5 | 0 | nd | 0 |
| Sens 3 | 100% | nd | 100% | 100% | nd | 90% | 100% | nd | 100% |
| Spec 3 | 0% | nd | 0% | 0% | nd | 9% | 0% | nd | 0% |
| Cutoff 4 | 0.151 | nd | 0.151 | 0.151 | nd | 0.151 | 0.151 | nd | 0.151 |
| Sens 4 | 29% | nd | 29% | 21% | nd | 23% | 37% | nd | 29% |
| Spec 4 | 72% | nd | 71% | 72% | nd | 71% | 72% | nd | 71% |
| Cutoff 5 | 0.226 | nd | 0.226 | 0.226 | nd | 0.226 | 0.226 | nd | 0.226 |
| Sens 5 | 21% | nd | 21% | 14% | nd | 17% | 21% | nd | 18% |
| Spec 5 | 80% | nd | 80% | 80% | nd | 80% | 80% | nd | 80% |
| Cutoff 6 | 0.560 | nd | 0.548 | 0.560 | nd | 0.548 | 0.560 | nd | 0.548 |
| Sens 6 | 11% | nd | 11% | 0% | nd | 3% | 5% | nd | 6% |
| Spec 6 | 90% | nd | 90% | 90% | nd | 90% | 90% | nd | 90% |
| OR Quart 2 | 0.36 | nd | 0.36 | 1.6 | nd | 2.0 | 0.74 | nd | 0.73 |
| p Value | 0.14 | nd | 0.14 | 0.42 | nd | 0.21 | 0.70 | nd | 0.69 |
| 95% CI of OR Quart2 | 0.091 1.4 | nd | 0.091 1.4 | 0.53 4.6 | nd | 0.69 5.6 | 0.16 3.4 | nd | 0.16 3.4 |
| OR Quart 3 | 1.1 | nd | 1.2 | 0.82 | nd | 0.65 | 1.5 | nd | 1.5 |
| p Value | 0.80 | nd | 0.78 | 0.76 | nd | 0.52 | 0.52 | nd | 0.52 |
| 95% CI of OR Quart3 | 0.42 3.1 | nd | 0.42 3.2 | 0.24 2.8 | nd | 0.18 2.4 | 0.42 5.7 | nd | 0.42 5.7 |
| OR Quart 4 | 1.0 | nd | 1.0 | 1.6 | nd | 1.6 | 1.5 | nd | 0.99 |
| p Value | 0.98 | nd | 0.98 | 0.41 | nd | 0.41 | 0.53 | nd | 0.99 |
| 95% CI of OR Quart4 | 0.36 2.8 | nd | 0.36 2.8 | 0.54 4.7 | nd | 0.54 4.7 | 0.41 5.6 | nd | 0.24 4.1 |

**Follitropin subunit beta**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.19 | 6.77 | 6.19 | 6.50 | 6.19 | 11.5 |
| Average | 14.6 | 9.26 | 14.6 | 9.35 | 14.6 | 12.2 |
| Stdev | 20.6 | 7.59 | 20.6 | 8.24 | 20.6 | 8.69 |
| p(t-test) | | 0.18 | | 0.18 | | 0.61 |
| Min | 0.0600 | 0.733 | 0.0600 | 0.0879 | 0.0600 | 0.913 |
| Max | 141 | 27.3 | 141 | 32.6 | 141 | 26.4 |
| n (Samp) | 306 | 28 | 306 | 29 | 306 | 19 |
| n (Patient) | 143 | 28 | 143 | 29 | 143 | 19 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.97 | 6.77 | 5.97 | 6.09 | 5.97 | 12.5 |
| Average | 14.1 | 9.25 | 14.1 | 9.21 | 14.1 | 13.1 |
| Stdev | 20.3 | 7.59 | 20.3 | 8.13 | 20.3 | 8.64 |
| p(t-test) | | 0.21 | | 0.19 | | 0.84 |
| Min | 0.0600 | 0.733 | 0.0600 | 0.0879 | 0.0600 | 2.55 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 141 | 27.3 | 141 | 32.6 | 141 | 26.4 |
| n (Samp) | 305 | 28 | 305 | 30 | 305 | 17 |
| n (Patient) | 138 | 28 | 138 | 30 | 138 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | nd | 0.50 | 0.50 | nd | 0.50 | 0.58 | nd | 0.61 |
| SE | 0.057 | nd | 0.057 | 0.056 | nd | 0.055 | 0.070 | nd | 0.074 |
| p | 0.91 | nd | 0.95 | 0.93 | nd | 0.95 | 0.29 | nd | 0.12 |
| nCohort 1 | 306 | nd | 305 | 306 | nd | 305 | 306 | nd | 305 |
| nCohort 2 | 28 | nd | 28 | 29 | nd | 30 | 19 | nd | 17 |
| Cutoff 1 | 3.44 | nd | 3.44 | 4.20 | nd | 4.25 | 5.41 | nd | 5.68 |
| Sens 1 | 71% | nd | 71% | 72% | nd | 70% | 74% | nd | 71% |
| Spec 1 | 33% | nd | 34% | 39% | nd | 40% | 46% | nd | 48% |
| Cutoff 2 | 2.12 | nd | 2.12 | 2.75 | nd | 3.22 | 3.65 | nd | 4.48 |
| Sens 2 | 82% | nd | 82% | 83% | nd | 80% | 84% | nd | 82% |
| Spec 2 | 20% | nd | 22% | 26% | nd | 32% | 34% | nd | 43% |
| Cutoff 3 | 1.21 | nd | 1.21 | 1.21 | nd | 2.12 | 2.50 | nd | 2.83 |
| Sens 3 | 93% | nd | 93% | 93% | nd | 90% | 95% | nd | 94% |
| Spec 3 | 12% | nd | 12% | 12% | nd | 22% | 24% | nd | 28% |
| Cutoff 4 | 13.2 | nd | 12.9 | 13.2 | nd | 12.9 | 13.2 | nd | 12.9 |
| Sens 4 | 29% | nd | 29% | 21% | nd | 20% | 42% | nd | 47% |
| Spec 4 | 70% | nd | 70% | 70% | nd | 70% | 70% | nd | 70% |
| Cutoff 5 | 22.8 | nd | 22.0 | 22.8 | nd | 22.0 | 22.8 | nd | 22.0 |
| Sens 5 | 7% | nd | 7% | 7% | nd | 7% | 21% | nd | 24% |
| Spec 5 | 80% | nd | 80% | 80% | nd | 80% | 80% | nd | 80% |
| Cutoff 6 | 39.2 | nd | 38.4 | 39.2 | nd | 38.4 | 39.2 | nd | 38.4 |
| Sens 6 | 0% | nd | 0% | 0% | nd | 0% | 0% | nd | 0% |
| Spec 6 | 90% | nd | 90% | 90% | nd | 90% | 90% | nd | 90% |
| OR Quart 2 | 2.2 | nd | 1.2 | 1.6 | nd | 2.1 | 3.2 | nd | 6.3 |
| p Value | 0.18 | nd | 0.77 | 0.42 | nd | 0.19 | 0.17 | nd | 0.091 |
| 95% CI of OR Quart2 | 0.71 6.6 | nd | 0.38 3.7 | 0.53 4.6 | nd | 0.69 6.5 | 0.62 16 | nd | 0.74 54 |
| OR Quart 3 | 1.4 | nd | 1.4 | 1.6 | nd | 1.9 | 3.2 | nd | 5.3 |
| p Value | 0.55 | nd | 0.58 | 0.42 | nd | 0.28 | 0.17 | nd | 0.13 |
| 95% CI of OR Quart3 | 0.44 4.7 | nd | 0.45 4.1 | 0.53 4.6 | nd | 0.60 5.8 | 0.62 16 | nd | 0.60 46 |
| OR Quart 4 | 1.2 | nd | 1.2 | 0.83 | nd | 1.2 | 2.6 | nd | 5.2 |
| p Value | 0.74 | nd | 0.79 | 0.77 | nd | 0.77 | 0.27 | nd | 0.14 |
| 95% CI of OR Quart4 | 0.36 4.2 | nd | 0.37 3.6 | 0.24 2.8 | nd | 0.35 4.1 | 0.48 14 | nd | 0.59 46 |

**Follistatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 808 | 678 | 808 | 813 | 808 | 832 |
| Average | 2700 | 3740 | 2700 | 2670 | 2700 | 2050 |
| Stdev | 4450 | 4750 | 4450 | 3940 | 4450 | 3320 |
| p(t-test) | | 0.19 | | 0.96 | | 0.43 |
| Min | 0.0206 | 1.69 | 0.0206 | 0.000554 | 0.0206 | 1.35 |
| Max | 28900 | 15900 | 28900 | 15600 | 28900 | 11700 |
| n (Samp) | 426 | 35 | 426 | 44 | 426 | 30 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 210 | 35 | 210 | 44 | 210 | 30 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 807 | 608 | 807 | 601 |
| Average | nd | nd | 2940 | 926 | 2940 | 640 |
| Stdev | nd | nd | 4570 | 983 | 4570 | 407 |
| p(t-test) | nd | nd | | 0.25 | | 0.13 |
| Min | nd | nd | 0.000554 | 13.2 | 0.000554 | 1.35 |
| Max | nd | nd | 28900 | 3050 | 28900 | 1280 |
| n (Samp) | nd | nd | 570 | 7 | 570 | 9 |
| n (Patient) | nd | nd | 256 | 7 | 256 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 795 | 874 | 795 | 846 | 795 | 973 |
| Average | 2830 | 4220 | 2830 | 2800 | 2830 | 2360 |
| Stdev | 4570 | 5080 | 4570 | 4030 | 4570 | 3510 |
| p(t-test) | | 0.10 | | 0.96 | | 0.60 |
| Min | 0.0206 | 1.69 | 0.0206 | 0.000554 | 0.0206 | 2.41 |
| Max | 28900 | 15900 | 28900 | 15600 | 28900 | 11700 |
| n (Samp) | 409 | 32 | 409 | 43 | 409 | 26 |
| n (Patient) | 193 | 32 | 193 | 43 | 193 | 26 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | nd | 0.52 | 0.53 | 0.42 | 0.52 | 0.48 | 0.37 | 0.51 |
| SE | 0.051 | nd | 0.054 | 0.046 | 0.11 | 0.047 | 0.055 | 0.10 | 0.059 |
| p | 0.92 | nd | 0.67 | 0.56 | 0.47 | 0.60 | 0.70 | 0.21 | 0.89 |
| nCohort 1 | 426 | nd | 409 | 426 | 570 | 409 | 426 | 570 | 409 |
| nCohort 2 | 35 | nd | 32 | 44 | 7 | 43 | 30 | 9 | 26 |
| Cutoff 1 | 441 | nd | 394 | 594 | 594 | 518 | 442 | 518 | 394 |
| Sens 1 | 71% | nd | 72% | 70% | 71% | 72% | 70% | 78% | 73% |
| Spec 1 | 22% | nd | 20% | 34% | 36% | 30% | 22% | 31% | 20% |
| Cutoff 2 | 312 | nd | 257 | 472 | 460 | 461 | 257 | 180 | 257 |
| Sens 2 | 80% | nd | 81% | 82% | 86% | 81% | 80% | 89% | 81% |
| Spec 2 | 14% | nd | 14% | 24% | 26% | 25% | 13% | 11% | 14% |
| Cutoff 3 | 29.3 | nd | 29.3 | 312 | 13.0 | 312 | 13.0 | 1.29 | 13.0 |
| Sens 3 | 91% | nd | 91% | 91% | 100% | 91% | 90% | 100% | 92% |
| Spec 3 | 10% | nd | 11% | 14% | 9% | 15% | 8% | 2% | 9% |
| Cutoff 4 | 1210 | nd | 1230 | 1210 | 1340 | 1230 | 1210 | 1340 | 1230 |
| Sens 4 | 40% | nd | 44% | 41% | 14% | 40% | 33% | 0% | 42% |
| Spec 4 | 70% | nd | 70% | 70% | 70% | 70% | 70% | 70% | 70% |
| Cutoff 5 | 2310 | nd | 4140 | 2310 | 6480 | 4140 | 2310 | 6480 | 4140 |
| Sens 5 | 34% | nd | 38% | 23% | 0% | 23% | 13% | 0% | 15% |
| Spec 5 | 80% | nd | 80% | 80% | 80% | 80% | 80% | 80% | 80% |
| Cutoff 6 | 10500 | nd | 10700 | 10500 | 10900 | 10700 | 10500 | 10900 | 10700 |
| Sens 6 | 14% | nd | 16% | 7% | 0% | 7% | 7% | 0% | 8% |
| Spec 6 | 90% | nd | 90% | 90% | 90% | 90% | 90% | 90% | 90% |
| OR Quart 2 | 0.20 | nd | 0.48 | 1.2 | 1.0 | 1.4 | 0.48 | >3.1 | 0.31 |
| p Value | 0.012 | nd | 0.19 | 0.67 | 1.00 | 0.49 | 0.19 | <0.33 | 0.087 |
| 95% CI of | 0.055 | nd | 0.16 | 0.50 | 0.062 | 0.55 | 0.16 | >0.31 | 0.082 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart2 | 0.70 | nd | 1.4 | 2.9 | 16 | 3.4 | 1.4 | na | 1.2 |
| OR Quart 3 | 0.26 | nd | 0.28 | 0.89 | 4.1 | 1.0 | 0.48 | >4.1 | 0.31 |
| p Value | 0.022 | nd | 0.059 | 0.81 | 0.21 | 1.0 | 0.19 | <0.21 | 0.087 |
| 95% CI of | 0.084 | nd | 0.075 | 0.35 | 0.45 | 0.38 | 0.16 | >0.45 | 0.082 |
| OR Quart3 | 0.82 | nd | 1.0 | 2.3 | 37 | 2.6 | 1.4 | na | 1.2 |
| OR Quart 4 | 1.0 | nd | 1.4 | 1.3 | 1.0 | 1.5 | 1.0 | >2.0 | 1.2 |
| p Value | 0.98 | nd | 0.40 | 0.53 | 1.00 | 0.37 | 1.0 | <0.56 | 0.65 |
| 95% CI of | 0.46 | nd | 0.61 | 0.56 | 0.062 | 0.61 | 0.40 | >0.18 | 0.49 |
| OR Quart4 | 2.2 | nd | 3.4 | 3.2 | 16 | 3.7 | 2.5 | na | 3.1 |

**Vitamin D-binding protein**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 174000 | 178000 | 174000 | 164000 | 174000 | 161000 |
| Average | 197000 | 182000 | 197000 | 181000 | 197000 | 158000 |
| Stdev | 94500 | 82200 | 94500 | 99400 | 94500 | 67700 |
| p(t-test) | | 0.41 | | 0.34 | | 0.071 |
| Min | 41500 | 24700 | 41500 | 54200 | 41500 | 48700 |
| Max | 630000 | 422000 | 630000 | 556000 | 630000 | 278000 |
| n (Samp) | 358 | 28 | 358 | 34 | 358 | 20 |
| n (Patient) | 175 | 28 | 175 | 34 | 175 | 20 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 174000 | 178000 | 174000 | 163000 | 174000 | 161000 |
| Average | 197000 | 179000 | 197000 | 177000 | 197000 | 154000 |
| Stdev | 93600 | 84900 | 93600 | 99700 | 93600 | 64200 |
| p(t-test) | | 0.33 | | 0.25 | | 0.056 |
| Min | 41500 | 24700 | 41500 | 54200 | 41500 | 48700 |
| Max | 630000 | 422000 | 630000 | 556000 | 630000 | 273000 |
| n (Samp) | 350 | 28 | 350 | 35 | 350 | 18 |
| n (Patient) | 163 | 28 | 163 | 35 | 163 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | nd | 0.46 | 0.43 | nd | 0.42 | 0.39 | nd | 0.38 |
| SE | 0.057 | nd | 0.058 | 0.053 | nd | 0.053 | 0.069 | nd | 0.072 |
| p | 0.68 | nd | 0.54 | 0.18 | nd | 0.11 | 0.11 | nd | 0.088 |
| nCohort 1 | 358 | nd | 350 | 358 | nd | 350 | 358 | nd | 350 |
| nCohort 2 | 28 | nd | 28 | 34 | nd | 35 | 20 | nd | 18 |
| Cutoff 1 | 148000 | nd | 148000 | 139000 | nd | 128000 | 108000 | nd | 107000 |
| Sens 1 | 71% | nd | 71% | 71% | nd | 71% | 70% | nd | 72% |
| Spec 1 | 32% | nd | 33% | 27% | nd | 22% | 12% | nd | 12% |
| Cutoff 2 | 120000 | nd | 103000 | 121000 | nd | 121000 | 102000 | nd | 77400 |
| Sens 2 | 82% | nd | 82% | 82% | nd | 80% | 80% | nd | 83% |
| Spec 2 | 18% | nd | 11% | 18% | nd | 18% | 10% | nd | 3% |
| Cutoff 3 | 76300 | nd | 68600 | 98100 | nd | 89100 | 76700 | nd | 76300 |
| Sens 3 | 93% | nd | 93% | 91% | nd | 91% | 90% | nd | 94% |
| Spec 3 | 3% | nd | 2% | 9% | nd | 6% | 3% | nd | 3% |
| Cutoff 4 | 220000 | nd | 222000 | 220000 | nd | 222000 | 220000 | nd | 222000 |
| Sens 4 | 29% | nd | 29% | 18% | nd | 17% | 20% | nd | 17% |
| Spec 4 | 70% | nd | 70% | 70% | nd | 70% | 70% | nd | 70% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 5 | 250000 | nd | 252000 | 250000 | nd | 252000 | 250000 | nd | 252000 |
| Sens 5 | 14% | nd | 14% | 12% | nd | 11% | 10% | nd | 6% |
| Spec 5 | 80% | nd | 80% | 80% | nd | 80% | 80% | nd | 80% |
| Cutoff 6 | 310000 | nd | 307000 | 310000 | nd | 307000 | 310000 | nd | 307000 |
| Sens 6 | 4% | nd | 7% | 6% | nd | 6% | 0% | nd | 0% |
| Spec 6 | 90% | nd | 90% | 90% | nd | 90% | 90% | nd | 90% |
| OR Quart 2 | 2.1 | nd | 2.1 | 1.0 | nd | 1.0 | 1.7 | nd | 2.6 |
| p Value | 0.18 | nd | 0.18 | 1.0 | nd | 0.99 | 0.47 | nd | 0.26 |
| 95% CI of OR Quart2 | 0.70 6.5 | nd | 0.70 6.5 | 0.31 3.2 | nd | 0.31 3.3 | 0.40 7.4 | nd | 0.49 14 |
| OR Quart 3 | 1.2 | nd | 1.0 | 2.1 | nd | 2.2 | 1.3 | nd | 2.0 |
| p Value | 0.76 | nd | 1.0 | 0.15 | nd | 0.14 | 0.70 | nd | 0.42 |
| 95% CI of OR Quart3 | 0.36 4.1 | nd | 0.28 3.6 | 0.77 6.0 | nd | 0.78 6.0 | 0.29 6.2 | nd | 0.37 11 |
| OR Quart 4 | 1.4 | nd | 1.7 | 1.7 | nd | 2.0 | 2.9 | nd | 3.7 |
| p Value | 0.54 | nd | 0.38 | 0.30 | nd | 0.20 | 0.13 | nd | 0.11 |
| 95% CI of OR Quart4 | 0.44 4.7 | nd | 0.53 5.3 | 0.61 5.0 | nd | 0.70 5.5 | 0.73 11 | nd | 0.75 18 |

**Glucagon**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 61.3 | 21.2 | 61.3 | 113 | 61.3 | 297 |
| Average | 8600 | 5800 | 8600 | 6900 | 8600 | 16600 |
| Stdev | 16400 | 15100 | 16400 | 15300 | 16400 | 20300 |
| p(t-test) | | 0.66 | | 0.67 | | 0.061 |
| Min | 1.01 | 21.2 | 1.01 | 21.2 | 1.01 | 21.2 |
| Max | 40100 | 40100 | 40100 | 40100 | 40100 | 40100 |
| n (Samp) | 197 | 7 | 197 | 18 | 197 | 17 |
| n (Patient) | 131 | 7 | 131 | 18 | 131 | 17 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 68.7 | 212 | 68.7 | 20200 |
| Average | nd | nd | 9190 | 9660 | 9190 | 20100 |
| Stdev | nd | nd | 16800 | 17400 | 16800 | 20700 |
| p(t-test) | nd | nd | | 0.91 | | 0.023 |
| Min | nd | nd | 1.01 | 21.2 | 1.01 | 21.2 |
| Max | nd | nd | 40100 | 40100 | 40100 | 40100 |
| n (Samp) | nd | nd | 171 | 17 | 171 | 14 |
| n (Patient) | nd | nd | 108 | 17 | 108 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.39 | nd | nd | 0.59 | nd | 0.61 | 0.63 | nd | 0.65 |
| SE | 0.12 | nd | nd | 0.073 | nd | 0.076 | 0.075 | nd | 0.082 |
| p | 0.35 | nd | nd | 0.23 | nd | 0.14 | 0.092 | nd | 0.061 |
| nCohort 1 | 197 | nd | nd | 197 | nd | 171 | 197 | nd | 171 |
| nCohort 2 | 7 | nd | nd | 18 | nd | 17 | 17 | nd | 14 |
| Cutoff 1 | 20.9 | nd | nd | 50.4 | nd | 104 | 65.0 | nd | 104 |
| Sens 1 | 100% | nd | nd | 72% | nd | 71% | 71% | nd | 71% |
| Spec 1 | 7% | nd | nd | 44% | nd | 64% | 52% | nd | 64% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 2 | 20.9 | nd | nd | 20.9 | nd | 36.0 | 24.7 | nd | 24.7 |
| Sens 2 | 100% | nd | nd | 100% | nd | 82% | 82% | nd | 86% |
| Spec 2 | 7% | nd | nd | 7% | nd | 32% | 29% | nd | 26% |
| Cutoff 3 | 20.9 | nd | nd | 20.9 | nd | 20.9 | 20.9 | nd | 20.9 |
| Sens 3 | 100% | nd | nd | 100% | nd | 100% | 100% | nd | 100% |
| Spec 3 | 7% | nd | nd | 7% | nd | 6% | 7% | nd | 6% |
| Cutoff 4 | 140 | nd | nd | 140 | nd | 148 | 140 | nd | 148 |
| Sens 4 | 29% | nd | nd | 44% | nd | 53% | 53% | nd | 64% |
| Spec 4 | 70% | nd | nd | 70% | nd | 70% | 70% | nd | 70% |
| Cutoff 5 | 40100 | nd | nd | 40100 | nd | 40100 | 40100 | nd | 40100 |
| Sens 5 | 0% | nd | nd | 0% | nd | 0% | 0% | nd | 0% |
| Spec 5 | 100% | nd | nd | 100% | nd | 100% | 100% | nd | 100% |
| Cutoff 6 | 40100 | nd | nd | 40100 | nd | 40100 | 40100 | nd | 40100 |
| Sens 6 | 0% | nd | nd | 0% | nd | 0% | 0% | nd | 0% |
| Spec 6 | 100% | nd | nd | 100% | nd | 100% | 100% | nd | 100% |
| OR Quart 2 | 0 | nd | nd | 0.47 | nd | 0.65 | 0.64 | nd | 1.0 |
| p Value | na | nd | nd | 0.40 | nd | 0.65 | 0.63 | nd | 1.0 |
| 95% CI of | na | nd | nd | 0.083 | nd | 0.10 | 0.10 | nd | 0.13 |
| OR Quart2 | na | nd | nd | 2.7 | nd | 4.1 | 4.0 | nd | 7.4 |
| OR Quart 3 | 0.49 | nd | nd | 1.2 | nd | 1.7 | 1.0 | nd | 1.5 |
| p Value | 0.57 | nd | nd | 0.75 | nd | 0.46 | 1.0 | nd | 0.65 |
| 95% CI of | 0.043 | nd | nd | 0.32 | nd | 0.39 | 0.19 | nd | 0.24 |
| OR Quart3 | 5.6 | nd | nd | 4.9 | nd | 7.8 | 5.2 | nd | 9.6 |
| OR Quart 4 | 2.1 | nd | nd | 1.8 | nd | 2.6 | 3.3 | nd | 3.8 |
| p Value | 0.41 | nd | nd | 0.36 | nd | 0.19 | 0.084 | nd | 0.10 |
| 95% CI of | 0.36 | nd | nd | 0.50 | nd | 0.62 | 0.85 | nd | 0.76 |
| OR Quart4 | 12 | nd | nd | 6.6 | nd | 11 | 13 | nd | 20 |

**Glucagon-like peptide 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 57.2 | 58.6 | 57.2 | 78.5 | 57.2 | 90.6 |
| Average | 64.8 | 49.1 | 64.8 | 87.8 | 64.8 | 95.1 |
| Stdev | 43.9 | 32.5 | 43.9 | 59.1 | 43.9 | 68.1 |
| p(t-test) | | 0.35 | | 0.040 | | 0.010 |
| Min | 2.37 | 2.52 | 2.37 | 13.1 | 2.37 | 2.81 |
| Max | 208 | 87.1 | 208 | 197 | 208 | 205 |
| n (Samp) | 197 | 7 | 197 | 18 | 197 | 17 |
| n (Patient) | 131 | 7 | 131 | 18 | 131 | 17 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 52.4 | 67.1 | 52.4 | 70.8 |
| Average | nd | nd | 62.5 | 85.3 | 62.5 | 89.1 |
| Stdev | nd | nd | 43.9 | 65.5 | 43.9 | 70.6 |
| p(t-test) | nd | nd | | 0.053 | | 0.040 |
| Min | nd | nd | 2.37 | 13.1 | 2.37 | 2.81 |
| Max | nd | nd | 208 | 202 | 208 | 205 |
| n (Samp) | nd | nd | 171 | 17 | 171 | 14 |
| n (Patient) | nd | nd | 108 | 17 | 108 | 14 |

| | 0hr prior to AKI stage | 24hr prior to AKI stage | 48hr prior to AKI stage |
|---|---|---|---|
| | | | |

|  | sCr or UO | sCr only | UO only | sCr or UO | sCr only | UO only | sCr or UO | sCr only | UO only |
|---|---|---|---|---|---|---|---|---|---|
| AUC | 0.42 | nd | nd | 0.61 | nd | 0.58 | 0.61 | nd | 0.58 |
| SE | 0.11 | nd | nd | 0.073 | nd | 0.076 | 0.075 | nd | 0.083 |
| p | 0.47 | nd | nd | 0.14 | nd | 0.30 | 0.13 | nd | 0.34 |
| nCohort 1 | 197 | nd | nd | 197 | nd | 171 | 197 | nd | 171 |
| nCohort 2 | 7 | nd | nd | 18 | nd | 17 | 17 | nd | 14 |
| Cutoff 1 | 27.3 | nd | nd | 50.7 | nd | 35.0 | 40.5 | nd | 34.8 |
| Sens 1 | 71% | nd | nd | 72% | nd | 71% | 71% | nd | 71% |
| Spec 1 | 19% | nd | nd | 47% | nd | 32% | 34% | nd | 31% |
| Cutoff 2 | 17.7 | nd | nd | 28.3 | nd | 27.0 | 27.3 | nd | 18.7 |
| Sens 2 | 86% | nd | nd | 83% | nd | 82% | 82% | nd | 86% |
| Spec 2 | 11% | nd | nd | 20% | nd | 22% | 19% | nd | 13% |
| Cutoff 3 | 2.37 | nd | nd | 14.6 | nd | 14.6 | 17.7 | nd | 17.7 |
| Sens 3 | 100% | nd | nd | 94% | nd | 94% | 94% | nd | 93% |
| Spec 3 | 1% | nd | nd | 10% | nd | 12% | 11% | nd | 12% |
| Cutoff 4 | 77.2 | nd | nd | 77.2 | nd | 75.4 | 77.2 | nd | 75.4 |
| Sens 4 | 29% | nd | nd | 50% | nd | 41% | 53% | nd | 50% |
| Spec 4 | 70% | nd | nd | 70% | nd | 71% | 70% | nd | 71% |
| Cutoff 5 | 96.4 | nd | nd | 96.4 | nd | 92.8 | 96.4 | nd | 92.8 |
| Sens 5 | 0% | nd | nd | 39% | nd | 35% | 47% | nd | 43% |
| Spec 5 | 80% | nd | nd | 80% | nd | 80% | 80% | nd | 80% |
| Cutoff 6 | 132 | nd | nd | 132 | nd | 124 | 132 | nd | 124 |
| Sens 6 | 0% | nd | nd | 28% | nd | 29% | 35% | nd | 36% |
| Spec 6 | 90% | nd | nd | 90% | nd | 90% | 90% | nd | 90% |
| OR Quart 2 | >4.3 | nd | nd | 0.72 | nd | 0.57 | 0.72 | nd | 0.48 |
| p Value | <0.20 | nd | nd | 0.68 | nd | 0.46 | 0.68 | nd | 0.41 |
| 95% CI of | >0.47 | nd | nd | 0.15 | nd | 0.13 | 0.15 | nd | 0.083 |
| OR Quart2 | na | nd | nd | 3.4 | nd | 2.5 | 3.4 | nd | 2.7 |
| OR Quart 3 | >0 | nd | nd | 0.98 | nd | 0.57 | 0.48 | nd | 0.48 |
| p Value | <na | nd | nd | 0.98 | nd | 0.46 | 0.41 | nd | 0.41 |
| 95% CI of | >na | nd | nd | 0.23 | nd | 0.13 | 0.084 | nd | 0.083 |
| OR Quart3 | na | nd | nd | 4.1 | nd | 2.5 | 2.7 | nd | 2.7 |
| OR Quart 4 | >3.2 | nd | nd | 1.8 | nd | 1.2 | 2.1 | nd | 1.5 |
| p Value | <0.32 | nd | nd | 0.36 | nd | 0.75 | 0.24 | nd | 0.53 |
| 95% CI of | >0.32 | nd | nd | 0.50 | nd | 0.35 | 0.60 | nd | 0.40 |
| OR Quart4 | na | nd | nd | 6.6 | nd | 4.3 | 7.6 | nd | 5.8 |

**Appetite-regulating hormone**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 24.8 | 14.3 | 24.8 | 23.6 | 24.8 | 36.6 |
| Average | 30.5 | 15.2 | 30.5 | 31.7 | 30.5 | 39.3 |
| Stdev | 24.9 | 9.22 | 24.9 | 21.8 | 24.9 | 24.3 |
| p(t-test) |  | 0.11 |  | 0.84 |  | 0.16 |
| Min | 0.251 | 6.30 | 0.251 | 6.30 | 0.251 | 3.18 |
| Max | 195 | 31.3 | 195 | 92.2 | 195 | 91.4 |
| n (Samp) | 197 | 7 | 197 | 18 | 197 | 17 |
| n (Patient) | 131 | 7 | 131 | 18 | 131 | 17 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 23.7 | 23.4 | 23.7 | 29.6 |
| Average | nd | nd | 28.9 | 29.1 | 28.9 | 35.7 |
| Stdev | nd | nd | 22.7 | 19.5 | 22.7 | 22.0 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | nd | nd | | 0.97 | | 0.28 |
| Min | nd | nd | 0.251 | 6.30 | 0.251 | 3.18 |
| Max | nd | nd | 119 | 67.7 | 119 | 72.6 |
| n (Samp) | nd | nd | 171 | 17 | 171 | 14 |
| n (Patient) | nd | nd | 108 | 17 | 108 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.28 | nd | nd | 0.53 | nd | 0.52 | 0.62 | nd | 0.61 |
| SE | 0.11 | nd | nd | 0.072 | nd | 0.074 | 0.075 | nd | 0.083 |
| p | 0.052 | nd | nd | 0.67 | nd | 0.82 | 0.11 | nd | 0.20 |
| nCohort 1 | 197 | nd | nd | 197 | nd | 171 | 197 | nd | 171 |
| nCohort 2 | 7 | nd | nd | 18 | nd | 17 | 17 | nd | 14 |
| Cutoff 1 | 8.02 | nd | nd | 17.3 | nd | 16.1 | 21.5 | nd | 21.5 |
| Sens 1 | 71% | nd | nd | 72% | nd | 71% | 71% | nd | 71% |
| Spec 1 | 13% | nd | nd | 34% | nd | 34% | 43% | nd | 46% |
| Cutoff 2 | 6.64 | nd | nd | 15.4 | nd | 13.6 | 20.0 | nd | 15.2 |
| Sens 2 | 86% | nd | nd | 83% | nd | 82% | 82% | nd | 86% |
| Spec 2 | 11% | nd | nd | 30% | nd | 29% | 39% | nd | 32% |
| Cutoff 3 | 5.26 | nd | nd | 11.1 | nd | 8.02 | 8.24 | nd | 8.24 |
| Sens 3 | 100% | nd | nd | 94% | nd | 94% | 94% | nd | 93% |
| Spec 3 | 9% | nd | nd | 20% | nd | 15% | 14% | nd | 16% |
| Cutoff 4 | 38.6 | nd | nd | 38.6 | nd | 35.7 | 38.6 | nd | 35.7 |
| Sens 4 | 0% | nd | nd | 28% | nd | 29% | 47% | nd | 43% |
| Spec 4 | 71% | nd | nd | 71% | nd | 70% | 71% | nd | 70% |
| Cutoff 5 | 47.1 | nd | nd | 47.1 | nd | 46.7 | 47.1 | nd | 46.7 |
| Sens 5 | 0% | nd | nd | 22% | nd | 24% | 47% | nd | 43% |
| Spec 5 | 80% | nd | nd | 80% | nd | 80% | 80% | nd | 80% |
| Cutoff 6 | 55.1 | nd | nd | 55.1 | nd | 55.1 | 55.1 | nd | 55.1 |
| Sens 6 | 0% | nd | nd | 11% | nd | 12% | 29% | nd | 29% |
| Spec 6 | 90% | nd | nd | 90% | nd | 90% | 90% | nd | 90% |
| OR Quart 2 | >1.0 | nd | nd | 4.4 | nd | 2.1 | 2.6 | nd | 2.1 |
| p Value | <0.99 | nd | nd | 0.068 | nd | 0.30 | 0.27 | nd | 0.41 |
| 95% CI of | >0.062 | nd | nd | 0.90 | nd | 0.50 | 0.48 | nd | 0.36 |
| OR Quart2 | na | nd | nd | 22 | nd | 9.1 | 14 | nd | 12 |
| OR Quart 3 | >3.2 | nd | nd | 1.5 | nd | 1.0 | 1.0 | nd | 1.0 |
| p Value | <0.32 | nd | nd | 0.66 | nd | 1.0 | 1.0 | nd | 1.0 |
| 95% CI of | >0.32 | nd | nd | 0.24 | nd | 0.19 | 0.14 | nd | 0.13 |
| OR Quart3 | na | nd | nd | 9.4 | nd | 5.2 | 7.4 | nd | 7.4 |
| OR Quart 4 | >3.2 | nd | nd | 2.6 | nd | 1.7 | 4.4 | nd | 3.2 |
| p Value | <0.32 | nd | nd | 0.27 | nd | 0.46 | 0.068 | nd | 0.17 |
| 95% CI of | >0.32 | nd | nd | 0.48 | nd | 0.39 | 0.90 | nd | 0.61 |
| OR Quart4 | na | nd | nd | 14 | nd | 7.8 | 22 | nd | 17 |

**Islet amyloid polypeptide**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.19 | 6.23 | 9.19 | 7.51 | 9.19 | 10.4 |
| Average | 13.7 | 9.06 | 13.7 | 11.3 | 13.7 | 11.1 |
| Stdev | 16.5 | 10.4 | 16.5 | 11.0 | 16.5 | 8.26 |
| p(t-test) | | 0.46 | | 0.53 | | 0.52 |
| Min | 0.0141 | 0.175 | 0.0141 | 0.338 | 0.0141 | 0.0166 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 121 | 31.4 | 121 | 38.0 | 121 | 30.2 |
| n (Samp) | 197 | 7 | 197 | 18 | 197 | 17 |
| n (Patient) | 131 | 7 | 131 | 18 | 131 | 17 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 8.70 | 6.02 | 8.70 | 8.16 |
| Average | nd | nd | 11.5 | 9.90 | 11.5 | 10.4 |
| Stdev | nd | nd | 11.9 | 9.56 | 11.9 | 9.01 |
| p(t-test) | nd | nd | | 0.58 | | 0.73 |
| Min | nd | nd | 0.0141 | 0.338 | 0.0141 | 0.0166 |
| Max | nd | nd | 70.6 | 29.5 | 70.6 | 30.2 |
| n (Samp) | nd | nd | 171 | 17 | 171 | 14 |
| n (Patient) | nd | nd | 108 | 17 | 108 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.39 | nd | nd | 0.46 | nd | 0.45 | 0.51 | nd | 0.49 |
| SE | 0.12 | nd | nd | 0.073 | nd | 0.075 | 0.073 | nd | 0.081 |
| p | 0.35 | nd | nd | 0.55 | nd | 0.47 | 0.92 | nd | 0.91 |
| nCohort 1 | 197 | nd | nd | 197 | nd | 171 | 197 | nd | 171 |
| nCohort 2 | 7 | nd | nd | 18 | nd | 17 | 17 | nd | 14 |
| Cutoff 1 | 4.48 | nd | nd | 3.53 | nd | 3.53 | 6.99 | nd | 5.96 |
| Sens 1 | 71% | nd | nd | 72% | nd | 71% | 71% | nd | 71% |
| Spec 1 | 24% | nd | nd | 20% | nd | 22% | 40% | nd | 36% |
| Cutoff 2 | 2.88 | nd | nd | 1.42 | nd | 1.42 | 2.88 | nd | 2.01 |
| Sens 2 | 86% | nd | nd | 83% | nd | 82% | 82% | nd | 86% |
| Spec 2 | 16% | nd | nd | 8% | nd | 9% | 16% | nd | 11% |
| Cutoff 3 | 0.133 | nd | nd | 0.338 | nd | 0.338 | 0.0166 | nd | 0.0166 |
| Sens 3 | 100% | nd | nd | 94% | nd | 94% | 94% | nd | 93% |
| Spec 3 | 6% | nd | nd | 6% | nd | 7% | 6% | nd | 6% |
| Cutoff 4 | 13.9 | nd | nd | 13.9 | nd | 12.6 | 13.9 | nd | 12.6 |
| Sens 4 | 14% | nd | nd | 33% | nd | 29% | 35% | nd | 29% |
| Spec 4 | 70% | nd | nd | 70% | nd | 70% | 70% | nd | 70% |
| Cutoff 5 | 19.0 | nd | nd | 19.0 | nd | 16.5 | 19.0 | nd | 16.5 |
| Sens 5 | 14% | nd | nd | 28% | nd | 29% | 18% | nd | 21% |
| Spec 5 | 80% | nd | nd | 80% | nd | 80% | 80% | nd | 80% |
| Cutoff 6 | 27.9 | nd | nd | 27.9 | nd | 23.7 | 27.9 | nd | 23.7 |
| Sens 6 | 14% | nd | nd | 11% | nd | 12% | 6% | nd | 7% |
| Spec 6 | 90% | nd | nd | 90% | nd | 90% | 90% | nd | 90% |
| OR Quart 2 | 1.0 | nd | nd | 0.38 | nd | 0.18 | 0.72 | nd | 0.75 |
| p Value | 1.0 | nd | nd | 0.26 | nd | 0.13 | 0.68 | nd | 0.72 |
| 95% CI of OR Quart2 | 0.061 16 | nd | nd | 0.070 2.0 | nd | 0.020 1.6 | 0.15 3.4 | nd | 0.16 3.6 |
| OR Quart 3 | 2.0 | nd | nd | 1.0 | nd | 1.0 | 1.9 | nd | 0.75 |
| p Value | 0.57 | nd | nd | 1.0 | nd | 1.0 | 0.35 | nd | 0.72 |
| 95% CI of OR Quart3 | 0.18 23 | nd | nd | 0.27 3.7 | nd | 0.27 3.7 | 0.51 6.8 | nd | 0.16 3.6 |
| OR Quart 4 | 3.1 | nd | nd | 1.3 | nd | 1.2 | 0.72 | nd | 1.0 |
| p Value | 0.33 | nd | nd | 0.73 | nd | 0.75 | 0.68 | nd | 0.97 |
| 95% CI of OR Quart4 | 0.31 31 | nd | nd | 0.36 4.4 | nd | 0.35 4.3 | 0.15 3.4 | nd | 0.24 4.4 |

**Interleukin-17A**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.86 | 0.787 | 1.86 | 1.97 | 1.86 | 2.41 |
| Average | 3.39 | 4.47 | 3.39 | 4.11 | 3.39 | 4.04 |
| Stdev | 5.30 | 11.7 | 5.30 | 6.26 | 5.30 | 6.10 |
| p(t-test) | | 0.32 | | 0.40 | | 0.54 |
| Min | 0.000362 | 0.000362 | 0.000362 | 0.000362 | 0.000362 | 0.000362 |
| Max | 53.4 | 61.7 | 53.4 | 28.8 | 53.4 | 28.6 |
| n (Samp) | 370 | 35 | 370 | 45 | 370 | 28 |
| n (Patient) | 185 | 35 | 185 | 45 | 185 | 28 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.53 | 4.40 | 1.53 | 2.82 |
| Average | nd | nd | 3.47 | 4.59 | 3.47 | 3.50 |
| Stdev | nd | nd | 6.02 | 2.84 | 6.02 | 1.77 |
| p(t-test) | nd | nd | | 0.62 | | 0.99 |
| Min | nd | nd | 0.000362 | 0.901 | 0.000362 | 1.18 |
| Max | nd | nd | 61.7 | 9.53 | 61.7 | 6.18 |
| n (Samp) | nd | nd | 516 | 7 | 516 | 7 |
| n (Patient) | nd | nd | 231 | 7 | 231 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.74 | 0.655 | 1.74 | 1.97 | 1.74 | 1.60 |
| Average | 3.24 | 4.67 | 3.24 | 4.09 | 3.24 | 4.03 |
| Stdev | 5.09 | 12.3 | 5.09 | 6.15 | 5.09 | 6.48 |
| p(t-test) | | 0.20 | | 0.30 | | 0.46 |
| Min | 0.000362 | 0.000362 | 0.000362 | 0.000362 | 0.000362 | 0.000362 |
| Max | 53.4 | 61.7 | 53.4 | 28.8 | 53.4 | 28.6 |
| n (Samp) | 359 | 32 | 359 | 47 | 359 | 25 |
| n (Patient) | 173 | 32 | 173 | 47 | 173 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.40 | nd | 0.40 | 0.53 | 0.72 | 0.53 | 0.55 | 0.68 | 0.53 |
| SE | 0.053 | nd | 0.055 | 0.046 | 0.11 | 0.045 | 0.058 | 0.11 | 0.061 |
| p | 0.065 | nd | 0.060 | 0.58 | 0.049 | 0.45 | 0.44 | 0.12 | 0.63 |
| nCohort 1 | 370 | nd | 359 | 370 | 516 | 359 | 370 | 516 | 359 |
| nCohort 2 | 35 | nd | 32 | 45 | 7 | 47 | 28 | 7 | 25 |
| Cutoff 1 | 0.0873 | nd | 0.0705 | 0.857 | 3.19 | 0.790 | 1.17 | 2.51 | 1.03 |
| Sens 1 | 71% | nd | 72% | 71% | 71% | 70% | 71% | 71% | 72% |
| Spec 1 | 21% | nd | 21% | 36% | 68% | 36% | 42% | 62% | 41% |
| Cutoff 2 | 0.00219 | nd | 0.000482 | 0.570 | 2.54 | 0.537 | 0.690 | 2.39 | 0.690 |
| Sens 2 | 80% | nd | 81% | 80% | 86% | 81% | 82% | 86% | 80% |
| Spec 2 | 12% | nd | 10% | 30% | 63% | 31% | 32% | 61% | 33% |
| Cutoff 3 | 0.000469 | nd | 0.000469 | 0.00219 | 0.857 | 0.000482 | 0.00436 | 1.17 | 0.00436 |
| Sens 3 | 94% | nd | 94% | 91% | 100% | 91% | 93% | 100% | 92% |
| Spec 3 | 6% | nd | 7% | 12% | 39% | 10% | 16% | 44% | 17% |
| Cutoff 4 | 3.68 | nd | 3.55 | 3.68 | 3.33 | 3.55 | 3.68 | 3.33 | 3.55 |
| Sens 4 | 17% | nd | 19% | 27% | 57% | 30% | 32% | 43% | 32% |
| Spec 4 | 70% | nd | 70% | 70% | 70% | 70% | 70% | 70% | 70% |
| Cutoff 5 | 5.11 | nd | 5.11 | 5.11 | 5.04 | 5.11 | 5.11 | 5.04 | 5.11 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 5 | 11% | nd | 12% | 22% | 29% | 23% | 18% | 29% | 16% |
| Spec 5 | 80% | nd | 80% | 80% | 80% | 80% | 80% | 80% | 80% |
| Cutoff 6 | 8.31 | nd | 8.22 | 8.31 | 8.85 | 8.22 | 8.31 | 8.85 | 8.22 |
| Sens 6 | 11% | nd | 12% | 13% | 14% | 13% | 14% | 0% | 16% |
| Spec 6 | 90% | nd | 90% | 90% | 90% | 90% | 90% | 90% | 90% |
| OR Quart 2 | 1.0 | nd | 0.59 | 2.1 | >1.0 | 1.7 | 2.3 | >1.0 | 2.7 |
| p Value | 0.99 | nd | 0.47 | 0.12 | <1.0 | 0.26 | 0.17 | <1.0 | 0.11 |
| 95% CI of OR Quart2 | 0.28 3.6 | nd | 0.14 2.5 | 0.82 5.5 | >0.062 na | 0.67 4.3 | 0.70 7.9 | >0.062 na | 0.81 8.8 |
| OR Quart 3 | 2.9 | nd | 2.6 | 2.1 | >2.0 | 1.9 | 2.7 | >4.1 | 1.8 |
| p Value | 0.054 | nd | 0.085 | 0.12 | <0.57 | 0.18 | 0.11 | <0.21 | 0.36 |
| 95% CI of OR Quart3 | 0.98 8.4 | nd | 0.88 7.7 | 0.82 5.5 | >0.18 na | 0.75 4.7 | 0.81 8.8 | >0.45 na | 0.51 6.4 |
| OR Quart 4 | 2.6 | nd | 2.6 | 1.5 | >4.1 | 1.6 | 1.2 | >2.0 | 1.0 |
| p Value | 0.082 | nd | 0.081 | 0.46 | <0.21 | 0.36 | 0.75 | <0.57 | 1.0 |
| 95% CI of OR Quart4 | 0.89 7.7 | nd | 0.89 7.8 | 0.53 4.0 | >0.45 na | 0.61 4.0 | 0.33 4.8 | >0.18 na | 0.24 4.1 |

**Insulin receptor substrate 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0314 | 0.0336 | 0.0314 | 0.0499 | 0.0314 | 0.0389 |
| Average | 0.564 | 0.425 | 0.564 | 0.234 | 0.564 | 0.297 |
| Stdev | 3.59 | 1.49 | 3.59 | 0.641 | 3.59 | 0.796 |
| p(t-test) | | 0.84 | | 0.59 | | 0.74 |
| Min | 3.03E-6 | 0.000111 | 3.03E-6 | 0.000645 | 3.03E-6 | 0.000645 |
| Max | 49.5 | 7.74 | 49.5 | 3.67 | 49.5 | 3.51 |
| n (Samp) | 358 | 28 | 358 | 34 | 358 | 20 |
| n (Patient) | 177 | 28 | 177 | 34 | 177 | 20 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0305 | 0.0336 | 0.0305 | 0.0506 | 0.0305 | 0.0435 |
| Average | 0.579 | 0.153 | 0.579 | 0.242 | 0.579 | 0.328 |
| Stdev | 3.64 | 0.402 | 3.64 | 0.633 | 3.64 | 0.836 |
| p(t-test) | | 0.54 | | 0.59 | | 0.77 |
| Min | 3.03E-6 | 0.000111 | 3.03E-6 | 0.000645 | 3.03E-6 | 0.000645 |
| Max | 49.5 | 2.10 | 49.5 | 3.67 | 49.5 | 3.51 |
| n (Samp) | 349 | 28 | 349 | 35 | 349 | 18 |
| n (Patient) | 164 | 28 | 164 | 35 | 164 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.52 | nd | 0.52 | 0.54 | nd | 0.55 | 0.51 | nd | 0.54 |
| SE | 0.057 | nd | 0.057 | 0.053 | nd | 0.052 | 0.067 | nd | 0.071 |
| p | 0.71 | nd | 0.77 | 0.45 | nd | 0.32 | 0.82 | nd | 0.57 |
| nCohort 1 | 358 | nd | 349 | 358 | nd | 349 | 358 | nd | 349 |
| nCohort 2 | 28 | nd | 28 | 34 | nd | 35 | 20 | nd | 18 |
| Cutoff 1 | 0.0191 | nd | 0.0191 | 0.0154 | nd | 0.0154 | 0.0144 | nd | 0.0144 |
| Sens 1 | 71% | nd | 71% | 71% | nd | 71% | 70% | nd | 72% |
| Spec 1 | 38% | nd | 39% | 34% | nd | 34% | 32% | nd | 33% |
| Cutoff 2 | 0.0106 | nd | 0.0106 | 0.0106 | nd | 0.0106 | 0.0106 | nd | 0.0106 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 2 | 82% | nd | 82% | 82% | nd | 80% | 80% | nd | 83% |
| Spec 2 | 27% | nd | 26% | 25% | nd | 26% | 25% | nd | 25% |
| Cutoff 3 | 0.00323 | nd | 0.00323 | 0.00276 | nd | 0.00276 | 0.000645 | nd | 0.000645 |
| Sens 3 | 93% | nd | 93% | 91% | nd | 91% | 90% | nd | 94% |
| Spec 3 | 14% | nd | 13% | 11% | nd | 11% | 9% | nd | 9% |
| Cutoff 4 | 0.0883 | nd | 0.0827 | 0.0883 | nd | 0.0827 | 0.0883 | nd | 0.0827 |
| Sens 4 | 29% | nd | 29% | 38% | nd | 40% | 30% | nd | 33% |
| Spec 4 | 70% | nd | 70% | 70% | nd | 70% | 70% | nd | 70% |
| Cutoff 5 | 0.170 | nd | 0.170 | 0.170 | nd | 0.170 | 0.170 | nd | 0.170 |
| Sens 5 | 18% | nd | 14% | 26% | nd | 29% | 25% | nd | 28% |
| Spec 5 | 80% | nd | 80% | 80% | nd | 80% | 80% | nd | 80% |
| Cutoff 6 | 0.527 | nd | 0.614 | 0.527 | nd | 0.614 | 0.527 | nd | 0.614 |
| Sens 6 | 11% | nd | 4% | 9% | nd | 9% | 10% | nd | 11% |
| Spec 6 | 90% | nd | 90% | 90% | nd | 90% | 90% | nd | 90% |
| OR Quart 2 | 2.4 | nd | 2.4 | 1.6 | nd | 1.6 | 1.2 | nd | 0.99 |
| p Value | 0.17 | nd | 0.16 | 0.42 | nd | 0.42 | 0.75 | nd | 0.99 |
| 95% CI of OR Quart2 | 0.70 7.9 | nd | 0.71 8.0 | 0.53 4.5 | nd | 0.53 4.5 | 0.33 4.8 | nd | 0.24 4.1 |
| OR Quart 3 | 1.8 | nd | 1.8 | 1.7 | nd | 1.7 | 1.3 | nd | 0.99 |
| p Value | 0.36 | nd | 0.36 | 0.30 | nd | 0.30 | 0.73 | nd | 0.99 |
| 95% CI of OR Quart3 | 0.51 6.4 | nd | 0.51 6.4 | 0.61 5.0 | nd | 0.61 5.0 | 0.33 4.9 | nd | 0.24 4.1 |
| OR Quart 4 | 2.1 | nd | 2.1 | 1.6 | nd | 1.7 | 1.5 | nd | 1.5 |
| p Value | 0.25 | nd | 0.25 | 0.42 | nd | 0.30 | 0.53 | nd | 0.53 |
| 95% CI of OR Quart4 | 0.60 7.1 | nd | 0.60 7.1 | 0.53 4.5 | nd | 0.61 5.0 | 0.41 5.6 | nd | 0.41 5.6 |

**Keratin, type II cytoskeletal 6 (6A, -6B, -6C mix)**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.13E-5 | 5.13E-5 | 5.13E-5 | 5.13E-5 | 5.13E-5 | 5.13E-5 |
| Average | 3.42 | 5.13E-5 | 3.42 | 6.35 | 3.42 | 9.34E-5 |
| Stdev | 20.3 | 0 | 20.3 | 37.0 | 20.3 | 7.48E-5 |
| p(t-test) | | 0.37 | | 0.46 | | 0.45 |
| Min | 5.13E-5 | 5.13E-5 | 5.13E-5 | 5.13E-5 | 5.13E-5 | 5.13E-5 |
| Max | 216 | 5.13E-5 | 216 | 216 | 216 | 0.000220 |
| n (Samp) | 358 | 28 | 358 | 34 | 358 | 20 |
| n (Patient) | 177 | 28 | 177 | 34 | 177 | 20 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.13E-5 | 5.13E-5 | 5.13E-5 | 5.13E-5 | 5.13E-5 | 5.13E-5 |
| Average | 2.85 | 5.13E-5 | 2.85 | 6.16 | 2.85 | 8.87E-5 |
| Stdev | 19.1 | 0 | 19.1 | 36.5 | 19.1 | 7.21E-5 |
| p(t-test) | | 0.43 | | 0.38 | | 0.53 |
| Min | 5.13E-5 | 5.13E-5 | 5.13E-5 | 5.13E-5 | 5.13E-5 | 5.13E-5 |
| Max | 216 | 5.13E-5 | 216 | 216 | 216 | 0.000220 |
| n (Samp) | 349 | 28 | 349 | 35 | 349 | 18 |
| n (Patient) | 164 | 28 | 164 | 35 | 164 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.33 | nd | 0.35 | 0.46 | nd | 0.48 | 0.45 | nd | 0.46 |
| SE | 0.058 | nd | 0.058 | 0.053 | nd | 0.052 | 0.068 | nd | 0.071 |
| p | 0.0040 | nd | 0.0096 | 0.50 | nd | 0.66 | 0.48 | nd | 0.53 |
| nCohort 1 | 358 | nd | 349 | 358 | nd | 349 | 358 | nd | 349 |
| nCohort 2 | 28 | nd | 28 | 34 | nd | 35 | 20 | nd | 18 |
| Cutoff 1 | 0 | nd | 0 | 0 | nd | 0 | 0 | nd | 0 |
| Sens 1 | 100% | nd | 100% | 100% | nd | 100% | 100% | nd | 100% |
| Spec 1 | 0% | nd | 0% | 0% | nd | 0% | 0% | nd | 0% |
| Cutoff 2 | 0 | nd | 0 | 0 | nd | 0 | 0 | nd | 0 |
| Sens 2 | 100% | nd | 100% | 100% | nd | 100% | 100% | nd | 100% |
| Spec 2 | 0% | nd | 0% | 0% | nd | 0% | 0% | nd | 0% |
| Cutoff 3 | 0 | nd | 0 | 0 | nd | 0 | 0 | nd | 0 |
| Sens 3 | 100% | nd | 100% | 100% | nd | 100% | 100% | nd | 100% |
| Spec 3 | 0% | nd | 0% | 0% | nd | 0% | 0% | nd | 0% |
| Cutoff 4 | 0.000220 | nd | 0.000135 | 0.000220 | nd | 0.000135 | 0.000220 | nd | 0.000135 |
| Sens 4 | 0% | nd | 0% | 3% | nd | 26% | 0% | nd | 22% |
| Spec 4 | 94% | nd | 70% | 94% | nd | 70% | 94% | nd | 70% |
| Cutoff 5 | 0.000220 | nd | 0.000220 | 0.000220 | nd | 0.000220 | 0.000220 | nd | 0.000220 |
| Sens 5 | 0% | nd | 0% | 3% | nd | 3% | 0% | nd | 0% |
| Spec 5 | 94% | nd | 95% | 94% | nd | 95% | 94% | nd | 95% |
| Cutoff 6 | 0.000220 | nd | 0.000220 | 0.000220 | nd | 0.000220 | 0.000220 | nd | 0.000220 |
| Sens 6 | 0% | nd | 0% | 3% | nd | 3% | 0% | nd | 0% |
| Spec 6 | 94% | nd | 95% | 94% | nd | 95% | 94% | nd | 95% |
| OR Quart 2 | >0 | nd | >0 | 0 | nd | 0.12 | 0.24 | nd | 0 |
| p Value | <na | nd | <na | na | nd | 0.044 | 0.21 | nd | na |
| 95% CI of | >na | nd | >na | na | nd | 0.014 | 0.027 | nd | na |
| OR Quart2 | na | nd | na | na | nd | 0.94 | 2.2 | nd | na |
| OR Quart 3 | >37 | nd | >38 | 3.2 | nd | 3.9 | 3.9 | nd | 3.6 |
| p Value | <4.4E-4 | nd | <4.1E-4 | 0.0057 | nd | 0.0019 | 0.020 | nd | 0.030 |
| 95% CI of | >5.0 | nd | >5.1 | 1.4 | nd | 1.6 | 1.2 | nd | 1.1 |
| OR Quart3 | na | nd | na | 7.3 | nd | 9.1 | 12 | nd | 12 |
| OR Quart 4 | >1.0 | nd | >1.0 | 0.10 | nd | 0.12 | 0.24 | nd | 0.24 |
| p Value | <0.99 | nd | <0.99 | 0.032 | nd | 0.044 | 0.21 | nd | 0.21 |
| 95% CI of | >0.063 | nd | >0.063 | 0.013 | nd | 0.014 | 0.027 | nd | 0.027 |
| OR Quart4 | na | nd | na | 0.82 | nd | 0.94 | 2.2 | nd | 2.2 |

**Collagenase 3**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0305 | 0.0288 | 0.0305 | 0.0699 | 0.0305 | 0.0305 |
| Average | 2.63 | 6.31 | 2.63 | 5.18 | 2.63 | 3.31 |
| Stdev | 20.9 | 20.9 | 20.9 | 21.0 | 20.9 | 12.6 |
| p(t-test) |  | 0.32 |  | 0.44 |  | 0.86 |
| Min | 0.00117 | 0.00117 | 0.00117 | 0.00117 | 0.00117 | 0.00117 |
| Max | 313 | 84.7 | 313 | 130 | 313 | 57.0 |
| n (Samp) | 433 | 35 | 433 | 45 | 433 | 31 |
| n (Patient) | 212 | 35 | 212 | 45 | 212 | 31 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 0.0305 | 0.0305 | 0.0305 | 0.0699 |
| Average | nd | nd | 3.09 | 0.109 | 3.09 | 4.91 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | nd | nd | 20.1 | 0.140 | 20.1 | 14.3 |
| p(t-test) | nd | nd | | 0.70 | | 0.79 |
| Min | nd | nd | 0.00117 | 0.00374 | 0.00117 | 0.00374 |
| Max | nd | nd | 313 | 0.383 | 313 | 43.1 |
| n (Samp) | nd | nd | 581 | 7 | 581 | 9 |
| n (Patient) | nd | nd | 261 | 7 | 261 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0305 | 0.0180 | 0.0305 | 0.0502 | 0.0305 | 0.0305 |
| Average | 2.95 | 4.37 | 2.95 | 5.29 | 2.95 | 2.18 |
| Stdev | 21.5 | 17.2 | 21.5 | 21.2 | 21.5 | 10.9 |
| p(t-test) | | 0.72 | | 0.49 | | 0.85 |
| Min | 0.00117 | 0.00117 | 0.00117 | 0.00117 | 0.00117 | 0.00117 |
| Max | 313 | 84.7 | 313 | 130 | 313 | 57.0 |
| n (Samp) | 416 | 32 | 416 | 44 | 416 | 27 |
| n (Patient) | 195 | 32 | 195 | 44 | 195 | 27 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.40 | nd | 0.38 | 0.53 | 0.53 | 0.52 | 0.51 | 0.59 | 0.49 |
| SE | 0.052 | nd | 0.055 | 0.046 | 0.11 | 0.046 | 0.054 | 0.10 | 0.058 |
| p | 0.054 | nd | 0.035 | 0.49 | 0.76 | 0.66 | 0.91 | 0.36 | 0.82 |
| nCohort 1 | 433 | nd | 416 | 433 | 581 | 416 | 433 | 581 | 416 |
| nCohort 2 | 35 | nd | 32 | 45 | 7 | 44 | 31 | 9 | 27 |
| Cutoff 1 | 0.00374 | nd | 0.00374 | 0.00455 | 0.00726 | 0.00455 | 0.00726 | 0.0297 | 0.00455 |
| Sens 1 | 80% | nd | 81% | 73% | 71% | 70% | 71% | 78% | 74% |
| Spec 1 | 16% | nd | 17% | 24% | 36% | 26% | 33% | 46% | 26% |
| Cutoff 2 | 0.00374 | nd | 0.00374 | 0.00374 | 0.00455 | 0.00117 | 0.00374 | 0.00117 | 0.00374 |
| Sens 2 | 80% | nd | 81% | 82% | 86% | 91% | 84% | 100% | 81% |
| Spec 2 | 16% | nd | 17% | 16% | 27% | 9% | 16% | 9% | 17% |
| Cutoff 3 | 0.00117 | nd | 0.00117 | 0.00117 | 0.00117 | 0.00117 | 0.00117 | 0.00117 | 0.00117 |
| Sens 3 | 91% | nd | 91% | 91% | 100% | 91% | 94% | 100% | 93% |
| Spec 3 | 9% | nd | 9% | 9% | 9% | 9% | 9% | 9% | 9% |
| Cutoff 4 | 0.102 | nd | 0.102 | 0.102 | 0.102 | 0.102 | 0.102 | 0.102 | 0.102 |
| Sens 4 | 11% | nd | 9% | 36% | 29% | 32% | 19% | 33% | 15% |
| Spec 4 | 72% | nd | 73% | 72% | 74% | 73% | 72% | 74% | 73% |
| Cutoff 5 | 0.204 | nd | 0.204 | 0.204 | 0.204 | 0.204 | 0.204 | 0.204 | 0.204 |
| Sens 5 | 11% | nd | 9% | 24% | 14% | 23% | 16% | 33% | 11% |
| Spec 5 | 84% | nd | 84% | 84% | 84% | 84% | 84% | 84% | 84% |
| Cutoff 6 | 0.383 | nd | 0.383 | 0.383 | 0.383 | 0.383 | 0.383 | 0.383 | 0.383 |
| Sens 6 | 11% | nd | 9% | 11% | 0% | 11% | 6% | 11% | 4% |
| Spec 6 | 97% | nd | 96% | 97% | 95% | 96% | 97% | 95% | 96% |
| OR Quart 2 | 1.5 | nd | 2.8 | 0.81 | 2.0 | 0.43 | 1.5 | 0.49 | 2.1 |
| p Value | 0.52 | nd | 0.14 | 0.64 | 0.57 | 0.10 | 0.45 | 0.57 | 0.24 |
| 95% CI of OR Quart2 | 0.42 | nd | 0.72 | 0.34 | 0.18 | 0.16 | 0.54 | 0.044 | 0.61 |
| | 5.6 | nd | 11 | 2.0 | 22 | 1.2 | 4.0 | 5.5 | 7.1 |
| OR Quart 3 | 3.5 | nd | 4.8 | 0.56 | 2.0 | 1.1 | 1.2 | 1.5 | 2.1 |
| p Value | 0.032 | nd | 0.017 | 0.24 | 0.57 | 0.84 | 0.79 | 0.65 | 0.24 |
| 95% CI of OR Quart3 | 1.1 | nd | 1.3 | 0.21 | 0.18 | 0.49 | 0.40 | 0.25 | 0.61 |
| | 11 | nd | 17 | 1.5 | 22 | 2.4 | 3.3 | 9.2 | 7.1 |
| OR Quart 4 | 3.2 | nd | 2.8 | 1.4 | 2.0 | 0.83 | 0.85 | 1.5 | 1.8 |
| p Value | 0.048 | nd | 0.14 | 0.44 | 0.57 | 0.67 | 0.78 | 0.66 | 0.35 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of OR Quart4 | 1.0 | nd | 0.72 | 0.62 | 0.18 | 0.36 | 0.28 | 0.25 | 0.52 |
|  | 10 | nd | 11 | 3.0 | 22 | 1.9 | 2.6 | 9.1 | 6.4 |

**NF-kappa-B inhibitor alpha**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.000953 | 0.00173 | 0.000953 | 0.00163 | 0.000953 | 0.00194 |
| Average | 0.0425 | 0.0194 | 0.0425 | 0.0116 | 0.0425 | 0.0148 |
| Stdev | 0.274 | 0.0431 | 0.274 | 0.0206 | 0.274 | 0.0346 |
| p(t-test) |  | 0.66 |  | 0.51 |  | 0.65 |
| Min | 1.84E-7 | 1.84E-7 | 1.84E-7 | 1.84E-7 | 1.84E-7 | 1.84E-7 |
| Max | 3.62 | 0.208 | 3.62 | 0.0836 | 3.62 | 0.152 |
| n (Samp) | 358 | 28 | 358 | 34 | 358 | 20 |
| n (Patient) | 177 | 28 | 177 | 34 | 177 | 20 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.00106 | 0.00173 | 0.00106 | 0.00166 | 0.00106 | 0.00194 |
| Average | 0.0434 | 0.0190 | 0.0434 | 0.0114 | 0.0434 | 0.0148 |
| Stdev | 0.278 | 0.0430 | 0.278 | 0.0203 | 0.278 | 0.0362 |
| p(t-test) |  | 0.64 |  | 0.50 |  | 0.66 |
| Min | 1.84E-7 | 1.84E-7 | 1.84E-7 | 1.84E-7 | 1.84E-7 | 1.84E-7 |
| Max | 3.62 | 0.208 | 3.62 | 0.0836 | 3.62 | 0.152 |
| n (Samp) | 349 | 28 | 349 | 35 | 349 | 18 |
| n (Patient) | 164 | 28 | 164 | 35 | 164 | 18 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.58 | nd | 0.57 | 0.55 | nd | 0.55 | 0.57 | nd | 0.56 |
| SE | 0.058 | nd | 0.058 | 0.053 | nd | 0.052 | 0.068 | nd | 0.072 |
| p | 0.18 | nd | 0.24 | 0.33 | nd | 0.38 | 0.33 | nd | 0.43 |
| nCohort 1 | 358 | nd | 349 | 358 | nd | 349 | 358 | nd | 349 |
| nCohort 2 | 28 | nd | 28 | 34 | nd | 35 | 20 | nd | 18 |
| Cutoff 1 | 0.000936 | nd | 0.000936 | 0.000480 | nd | 0.000480 | 0.000543 | nd | 0.000543 |
| Sens 1 | 71% | nd | 71% | 71% | nd | 71% | 70% | nd | 72% |
| Spec 1 | 50% | nd | 49% | 43% | nd | 42% | 44% | nd | 42% |
| Cutoff 2 | 0.000309 | nd | 0.000309 | 3.31E-5 | nd | 3.31E-5 | 3.31E-5 | nd | 3.31E-5 |
| Sens 2 | 82% | nd | 82% | 82% | nd | 83% | 90% | nd | 89% |
| Spec 2 | 40% | nd | 38% | 21% | nd | 19% | 21% | nd | 19% |
| Cutoff 3 | 0 | nd | 0 | 3.20E-6 | nd | 3.20E-6 | 3.31E-5 | nd | 9.39E-7 |
| Sens 3 | 100% | nd | 100% | 94% | nd | 94% | 90% | nd | 94% |
| Spec 3 | 0% | nd | 0% | 6% | nd | 6% | 21% | nd | 5% |
| Cutoff 4 | 0.00418 | nd | 0.00423 | 0.00418 | nd | 0.00423 | 0.00418 | nd | 0.00423 |
| Sens 4 | 36% | nd | 36% | 38% | nd | 37% | 35% | nd | 33% |
| Spec 4 | 70% | nd | 70% | 70% | nd | 70% | 70% | nd | 70% |
| Cutoff 5 | 0.00930 | nd | 0.0115 | 0.00930 | nd | 0.0115 | 0.00930 | nd | 0.0115 |
| Sens 5 | 32% | nd | 32% | 26% | nd | 26% | 30% | nd | 22% |
| Spec 5 | 80% | nd | 80% | 80% | nd | 80% | 80% | nd | 80% |
| Cutoff 6 | 0.0387 | nd | 0.0381 | 0.0387 | nd | 0.0381 | 0.0387 | nd | 0.0381 |
| Sens 6 | 18% | nd | 14% | 12% | nd | 11% | 10% | nd | 11% |
| Spec 6 | 90% | nd | 90% | 90% | nd | 90% | 90% | nd | 90% |
| OR Quart 2 | 1.5 | nd | 1.8 | 1.0 | nd | 1.4 | 2.6 | nd | 0.99 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value | 0.53 | nd | 0.36 | 1.0 | nd | 0.58 | 0.27 | nd | 0.99 |
| 95% CI of | 0.41 | nd | 0.51 | 0.34 | nd | 0.45 | 0.48 | nd | 0.19 |
| OR Quart2 | 5.6 | nd | 6.4 | 3.0 | nd | 4.1 | 14 | nd | 5.0 |
| OR Quart 3 | 2.1 | nd | 2.1 | 1.3 | nd | 1.7 | 3.1 | nd | 2.0 |
| p Value | 0.24 | nd | 0.24 | 0.60 | nd | 0.30 | 0.17 | nd | 0.32 |
| 95% CI of | 0.61 | nd | 0.61 | 0.47 | nd | 0.61 | 0.62 | nd | 0.50 |
| OR Quart3 | 7.2 | nd | 7.2 | 3.7 | nd | 5.0 | 16 | nd | 8.4 |
| OR Quart 4 | 2.6 | nd | 2.4 | 1.6 | nd | 1.9 | 3.7 | nd | 2.0 |
| p Value | 0.11 | nd | 0.17 | 0.33 | nd | 0.21 | 0.11 | nd | 0.32 |
| 95% CI of | 0.80 | nd | 0.70 | 0.61 | nd | 0.69 | 0.74 | nd | 0.50 |
| OR Quart4 | 8.7 | nd | 7.9 | 4.4 | nd | 5.5 | 18 | nd | 8.4 |

**Beta-nerve growth factor**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.62 | 1.82 | 1.62 | 1.89 | 1.62 | 2.06 |
| Average | 2.32 | 2.29 | 2.32 | 2.23 | 2.32 | 2.53 |
| Stdev | 3.91 | 1.71 | 3.91 | 1.42 | 3.91 | 1.39 |
| p(t-test) |  | 0.97 |  | 0.89 |  | 0.80 |
| Min | 0.221 | 0.681 | 0.221 | 0.453 | 0.221 | 1.23 |
| Max | 70.0 | 8.70 | 70.0 | 6.73 | 70.0 | 6.48 |
| n (Samp) | 358 | 28 | 358 | 34 | 358 | 22 |
| n (Patient) | 175 | 28 | 175 | 34 | 175 | 22 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.70 | 1.70 | 1.70 | 1.87 | 1.70 | 2.06 |
| Average | 2.36 | 2.28 | 2.36 | 2.22 | 2.36 | 2.56 |
| Stdev | 3.95 | 1.72 | 3.95 | 1.40 | 3.95 | 1.46 |
| p(t-test) |  | 0.91 |  | 0.83 |  | 0.83 |
| Min | 0.221 | 0.681 | 0.221 | 0.453 | 0.221 | 1.23 |
| Max | 70.0 | 8.70 | 70.0 | 6.73 | 70.0 | 6.48 |
| n (Samp) | 349 | 28 | 349 | 35 | 349 | 20 |
| n (Patient) | 161 | 28 | 161 | 35 | 161 | 20 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.54 | nd | 0.52 | 0.54 | nd | 0.53 | 0.64 | nd | 0.63 |
| SE | 0.058 | nd | 0.057 | 0.053 | nd | 0.052 | 0.065 | nd | 0.069 |
| p | 0.53 | nd | 0.75 | 0.45 | nd | 0.58 | 0.027 | nd | 0.066 |
| nCohort 1 | 358 | nd | 349 | 358 | nd | 349 | 358 | nd | 349 |
| nCohort 2 | 28 | nd | 28 | 34 | nd | 35 | 22 | nd | 20 |
| Cutoff 1 | 1.36 | nd | 1.36 | 1.30 | nd | 1.30 | 1.82 | nd | 1.82 |
| Sens 1 | 71% | nd | 71% | 74% | nd | 74% | 73% | nd | 70% |
| Spec 1 | 39% | nd | 37% | 35% | nd | 33% | 56% | nd | 54% |
| Cutoff 2 | 1.12 | nd | 1.12 | 1.07 | nd | 1.16 | 1.49 | nd | 1.49 |
| Sens 2 | 86% | nd | 86% | 82% | nd | 80% | 82% | nd | 80% |
| Spec 2 | 26% | nd | 25% | 20% | nd | 25% | 45% | nd | 43% |
| Cutoff 3 | 0.865 | nd | 0.865 | 0.786 | nd | 0.786 | 1.42 | nd | 1.42 |
| Sens 3 | 93% | nd | 93% | 94% | nd | 94% | 91% | nd | 90% |
| Spec 3 | 9% | nd | 9% | 8% | nd | 7% | 40% | nd | 38% |
| Cutoff 4 | 2.25 | nd | 2.31 | 2.25 | nd | 2.31 | 2.25 | nd | 2.31 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 4 | 29% | nd | 29% | 38% | nd | 34% | 41% | nd | 35% |
| Spec 4 | 70% | nd | 70% | 70% | nd | 70% | 70% | nd | 70% |
| Cutoff 5 | 2.92 | nd | 3.05 | 2.92 | nd | 3.05 | 2.92 | nd | 3.05 |
| Sens 5 | 18% | nd | 18% | 24% | nd | 20% | 27% | nd | 25% |
| Spec 5 | 80% | nd | 80% | 80% | nd | 80% | 80% | nd | 80% |
| Cutoff 6 | 4.06 | nd | 4.07 | 4.06 | nd | 4.07 | 4.06 | nd | 4.07 |
| Sens 6 | 18% | nd | 18% | 9% | nd | 9% | 14% | nd | 15% |
| Spec 6 | 90% | nd | 90% | 90% | nd | 90% | 90% | nd | 90% |
| OR Quart 2 | 2.4 | nd | 1.4 | 1.0 | nd | 0.87 | >6.4 | nd | >6.4 |
| p Value | 0.17 | nd | 0.58 | 1.0 | nd | 0.79 | <0.088 | nd | <0.088 |
| 95% CI of | 0.70 | nd | 0.45 | 0.34 | nd | 0.30 | >0.76 | nd | >0.76 |
| OR Quart2 | 7.9 | nd | 4.1 | 3.0 | nd | 2.5 | na | nd | na |
| OR Quart 3 | 1.8 | nd | 1.4 | 1.6 | nd | 1.4 | >11 | nd | >8.8 |
| p Value | 0.36 | nd | 0.58 | 0.33 | nd | 0.47 | <0.023 | nd | <0.043 |
| 95% CI of | 0.51 | nd | 0.45 | 0.61 | nd | 0.55 | >1.4 | nd | >1.1 |
| OR Quart3 | 6.4 | nd | 4.1 | 4.4 | nd | 3.7 | na | nd | na |
| OR Quart 4 | 2.1 | nd | 0.99 | 1.3 | nd | 1.1 | >6.4 | nd | >6.3 |
| p Value | 0.25 | nd | 0.98 | 0.60 | nd | 0.80 | <0.088 | nd | <0.090 |
| 95% CI of | 0.60 | nd | 0.31 | 0.47 | nd | 0.42 | >0.76 | nd | >0.75 |
| OR Quart4 | 7.1 | nd | 3.2 | 3.7 | nd | 3.1 | na | nd | na |

**Pancreatic prohormone**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 60.9 | 152 | 60.9 | 112 | 60.9 | 104 |
| Average | 162 | 243 | 162 | 166 | 162 | 125 |
| Stdev | 270 | 343 | 270 | 211 | 270 | 140 |
| p(t-test) | | 0.44 | | 0.94 | | 0.58 |
| Min | 0.683 | 32.3 | 0.683 | 3.78 | 0.683 | 0.731 |
| Max | 1920 | 1000 | 1920 | 869 | 1920 | 455 |
| n (Samp) | 197 | 7 | 197 | 18 | 197 | 17 |
| n (Patient) | 131 | 7 | 131 | 18 | 131 | 17 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 58.4 | 126 | 58.4 | 119 |
| Average | nd | nd | 157 | 225 | 157 | 151 |
| Stdev | nd | nd | 272 | 308 | 272 | 149 |
| p(t-test) | nd | nd | | 0.33 | | 0.93 |
| Min | nd | nd | 0.683 | 3.78 | 0.683 | 0.731 |
| Max | nd | nd | 1920 | 1080 | 1920 | 455 |
| n (Samp) | nd | nd | 171 | 17 | 171 | 14 |
| n (Patient) | nd | nd | 108 | 17 | 108 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.65 | nd | nd | 0.56 | nd | 0.58 | 0.52 | nd | 0.57 |
| SE | 0.11 | nd | nd | 0.073 | nd | 0.076 | 0.074 | nd | 0.083 |
| p | 0.18 | nd | nd | 0.40 | nd | 0.28 | 0.82 | nd | 0.41 |
| nCohort 1 | 197 | nd | nd | 197 | nd | 171 | 197 | nd | 171 |
| nCohort 2 | 7 | nd | nd | 18 | nd | 17 | 17 | nd | 14 |
| Cutoff 1 | 63.8 | nd | nd | 33.4 | nd | 33.4 | 42.5 | nd | 45.3 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 1 | 71% | nd | nd | 72% | nd | 71% | 71% | nd | 71% |
| Spec 1 | 53% | nd | nd | 30% | nd | 29% | 38% | nd | 42% |
| Cutoff 2 | 51.2 | nd | nd | 27.7 | nd | 27.7 | 21.4 | nd | 18.5 |
| Sens 2 | 86% | nd | nd | 83% | nd | 82% | 82% | nd | 86% |
| Spec 2 | 45% | nd | nd | 25% | nd | 24% | 20% | nd | 16% |
| Cutoff 3 | 31.4 | nd | nd | 8.51 | nd | 8.51 | 0.731 | nd | 0.731 |
| Sens 3 | 100% | nd | nd | 94% | nd | 94% | 94% | nd | 93% |
| Spec 3 | 29% | nd | nd | 8% | nd | 8% | 3% | nd | 2% |
| Cutoff 4 | 116 | nd | nd | 116 | nd | 112 | 116 | nd | 112 |
| Sens 4 | 57% | nd | nd | 50% | nd | 53% | 35% | nd | 57% |
| Spec 4 | 70% | nd | nd | 70% | nd | 70% | 70% | nd | 70% |
| Cutoff 5 | 207 | nd | nd | 207 | nd | 176 | 207 | nd | 176 |
| Sens 5 | 29% | nd | nd | 22% | nd | 29% | 18% | nd | 29% |
| Spec 5 | 80% | nd | nd | 80% | nd | 80% | 80% | nd | 80% |
| Cutoff 6 | 468 | nd | nd | 468 | nd | 443 | 468 | nd | 443 |
| Sens 6 | 14% | nd | nd | 6% | nd | 12% | 0% | nd | 14% |
| Spec 6 | 90% | nd | nd | 90% | nd | 90% | 90% | nd | 90% |
| OR Quart 2 | >2.1 | nd | nd | 0.72 | nd | 0.18 | 0.98 | nd | 0.23 |
| p Value | <0.55 | nd | nd | 0.68 | nd | 0.13 | 0.98 | nd | 0.20 |
| 95% CI of | >0.18 | nd | nd | 0.15 | nd | 0.020 | 0.23 | nd | 0.025 |
| OR Quart2 | na | nd | nd | 3.4 | nd | 1.6 | 4.1 | nd | 2.2 |
| OR Quart 3 | >2.1 | nd | nd | 1.5 | nd | 1.0 | 1.3 | nd | 1.0 |
| p Value | <0.55 | nd | nd | 0.53 | nd | 1.0 | 0.73 | nd | 1.0 |
| 95% CI of | >0.18 | nd | nd | 0.41 | nd | 0.27 | 0.32 | nd | 0.23 |
| OR Quart3 | na | nd | nd | 5.8 | nd | 3.7 | 5.0 | nd | 4.3 |
| OR Quart 4 | >3.2 | nd | nd | 1.2 | nd | 1.2 | 0.98 | nd | 1.2 |
| p Value | <0.32 | nd | nd | 0.75 | nd | 0.75 | 0.98 | nd | 0.75 |
| 95% CI of | >0.32 | nd | nd | 0.32 | nd | 0.35 | 0.23 | nd | 0.31 |
| OR Quart4 | na | nd | nd | 4.9 | nd | 4.3 | 4.1 | nd | 5.0 |

**Transthyretin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 57600 | 56100 | 57600 | 56500 | 57600 | 50800 |
| Average | 64500 | 65800 | 64500 | 65800 | 64500 | 54000 |
| Stdev | 40500 | 35600 | 40500 | 37900 | 40500 | 31600 |
| p(t-test) | | 0.87 | | 0.86 | | 0.26 |
| Min | 6500 | 25500 | 6500 | 8510 | 6500 | 14200 |
| Max | 293000 | 165000 | 293000 | 168000 | 293000 | 147000 |
| n (Samp) | 356 | 28 | 356 | 35 | 356 | 20 |
| n (Patient) | 178 | 28 | 178 | 35 | 178 | 20 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 56600 | 54200 | 56600 | 56600 | 56600 | 50800 |
| Average | 62900 | 65300 | 62900 | 67200 | 62900 | 55300 |
| Stdev | 38100 | 35900 | 38100 | 36700 | 38100 | 32700 |
| p(t-test) | | 0.75 | | 0.53 | | 0.41 |
| Min | 6500 | 25500 | 6500 | 20000 | 6500 | 14200 |
| Max | 293000 | 165000 | 293000 | 168000 | 293000 | 147000 |
| n (Samp) | 347 | 28 | 347 | 35 | 347 | 18 |
| n (Patient) | 165 | 28 | 165 | 35 | 165 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.52 | nd | 0.52 | 0.51 | nd | 0.54 | 0.42 | nd | 0.44 |
| SE | 0.057 | nd | 0.057 | 0.052 | nd | 0.052 | 0.068 | nd | 0.072 |
| p | 0.73 | nd | 0.71 | 0.77 | nd | 0.47 | 0.22 | nd | 0.37 |
| nCohort 1 | 356 | nd | 347 | 356 | nd | 347 | 356 | nd | 347 |
| nCohort 2 | 28 | nd | 28 | 35 | nd | 35 | 20 | nd | 18 |
| Cutoff 1 | 44100 | nd | 42600 | 43300 | nd | 44900 | 36600 | nd | 36800 |
| Sens 1 | 71% | nd | 71% | 71% | nd | 71% | 70% | nd | 72% |
| Spec 1 | 35% | nd | 34% | 34% | nd | 37% | 26% | nd | 25% |
| Cutoff 2 | 31000 | nd | 31000 | 36200 | nd | 36800 | 29000 | nd | 29000 |
| Sens 2 | 82% | nd | 82% | 80% | nd | 80% | 80% | nd | 83% |
| Spec 2 | 18% | nd | 18% | 25% | nd | 25% | 16% | nd | 16% |
| Cutoff 3 | 29400 | nd | 29400 | 21600 | nd | 29900 | 24600 | nd | 16500 |
| Sens 3 | 93% | nd | 93% | 91% | nd | 91% | 90% | nd | 94% |
| Spec 3 | 17% | nd | 17% | 6% | nd | 17% | 9% | nd | 3% |
| Cutoff 4 | 73800 | nd | 73400 | 73800 | nd | 73400 | 73800 | nd | 73400 |
| Sens 4 | 32% | nd | 32% | 37% | nd | 37% | 15% | nd | 17% |
| Spec 4 | 70% | nd | 70% | 70% | nd | 70% | 70% | nd | 70% |
| Cutoff 5 | 85300 | nd | 83800 | 85300 | nd | 83800 | 85300 | nd | 83800 |
| Sens 5 | 29% | nd | 29% | 26% | nd | 29% | 10% | nd | 17% |
| Spec 5 | 80% | nd | 80% | 80% | nd | 80% | 80% | nd | 80% |
| Cutoff 6 | 111000 | nd | 108000 | 111000 | nd | 108000 | 111000 | nd | 108000 |
| Sens 6 | 11% | nd | 11% | 14% | nd | 14% | 5% | nd | 11% |
| Spec 6 | 90% | nd | 90% | 90% | nd | 90% | 90% | nd | 90% |
| OR Quart 2 | 1.2 | nd | 1.1 | 1.4 | nd | 1.5 | 1.7 | nd | 1.7 |
| p Value | 0.79 | nd | 0.80 | 0.48 | nd | 0.46 | 0.47 | nd | 0.46 |
| 95% CI of OR Quart2 | 0.40 | nd | 0.40 | 0.54 | nd | 0.53 | 0.40 | nd | 0.40 |
| | 3.3 | nd | 3.3 | 3.7 | nd | 4.0 | 7.3 | nd | 7.4 |
| OR Quart 3 | 0.70 | nd | 0.69 | 0.60 | nd | 0.70 | 2.1 | nd | 1.7 |
| p Value | 0.55 | nd | 0.54 | 0.38 | nd | 0.55 | 0.31 | nd | 0.46 |
| 95% CI of OR Quart3 | 0.21 | nd | 0.21 | 0.19 | nd | 0.21 | 0.50 | nd | 0.40 |
| | 2.3 | nd | 2.3 | 1.9 | nd | 2.3 | 8.5 | nd | 7.4 |
| OR Quart 4 | 1.2 | nd | 1.1 | 1.4 | nd | 2.0 | 2.1 | nd | 1.7 |
| p Value | 0.79 | nd | 0.80 | 0.48 | nd | 0.17 | 0.31 | nd | 0.46 |
| 95% CI of OR Quart4 | 0.40 | nd | 0.40 | 0.54 | nd | 0.75 | 0.50 | nd | 0.40 |
| | 3.3 | nd | 3.3 | 3.7 | nd | 5.2 | 8.5 | nd | 7.4 |

**C-peptide EDTA**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2540 | 3170 | 2540 | 2120 | 2540 | 2430 |
| Average | 3180 | 4620 | 3180 | 2850 | 3180 | 2900 |
| Stdev | 2730 | 4410 | 2730 | 2110 | 2730 | 1960 |
| p(t-test) | | 0.0049 | | 0.43 | | 0.56 |
| Min | 39.8 | 21.7 | 39.8 | 51.9 | 39.8 | 88.8 |
| Max | 21700 | 19500 | 21700 | 8450 | 21700 | 7570 |
| n (Samp) | 434 | 35 | 434 | 43 | 434 | 32 |
| n (Patient) | 212 | 35 | 212 | 43 | 212 | 32 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 2660 | 2210 | 2660 | 1800 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | nd | nd | 3270 | 2640 | 3270 | 2620 |
| Stdev | nd | nd | 2700 | 1950 | 2700 | 2610 |
| p(t-test) | nd | nd | | 0.57 | | 0.50 |
| Min | nd | nd | 7.96 | 878 | 7.96 | 137 |
| Max | nd | nd | 21700 | 5360 | 21700 | 7530 |
| n (Samp) | nd | nd | 583 | 6 | 583 | 8 |
| n (Patient) | nd | nd | 262 | 6 | 262 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2540 | 3070 | 2540 | 2110 | 2540 | 2430 |
| Average | 3230 | 4270 | 3230 | 2740 | 3230 | 2720 |
| Stdev | 2780 | 3710 | 2780 | 2110 | 2780 | 1820 |
| p(t-test) | | 0.050 | | 0.26 | | 0.34 |
| Min | 39.8 | 21.7 | 39.8 | 51.9 | 39.8 | 88.8 |
| Max | 21700 | 17300 | 21700 | 8450 | 21700 | 7570 |
| n (Samp) | 415 | 32 | 415 | 43 | 415 | 28 |
| n (Patient) | 192 | 32 | 192 | 43 | 192 | 28 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | 0.59 | 0.48 | 0.44 | 0.46 | 0.50 | 0.41 | 0.48 |
| SE | 0.052 | nd | 0.055 | 0.047 | 0.12 | 0.047 | 0.053 | 0.11 | 0.057 |
| p | 0.064 | nd | 0.12 | 0.66 | 0.60 | 0.38 | 0.98 | 0.40 | 0.71 |
| nCohort 1 | 434 | nd | 415 | 434 | 583 | 415 | 434 | 583 | 415 |
| nCohort 2 | 35 | nd | 32 | 43 | 6 | 43 | 32 | 8 | 28 |
| Cutoff 1 | 1920 | nd | 1920 | 1330 | 894 | 1290 | 1650 | 959 | 1630 |
| Sens 1 | 71% | nd | 72% | 72% | 83% | 72% | 72% | 75% | 71% |
| Spec 1 | 42% | nd | 42% | 30% | 13% | 27% | 38% | 15% | 37% |
| Cutoff 2 | 1500 | nd | 1400 | 998 | 894 | 934 | 1270 | 159 | 974 |
| Sens 2 | 80% | nd | 81% | 81% | 83% | 81% | 81% | 88% | 82% |
| Spec 2 | 35% | nd | 31% | 17% | 13% | 16% | 27% | 3% | 17% |
| Cutoff 3 | 904 | nd | 904 | 754 | 875 | 708 | 564 | 114 | 163 |
| Sens 3 | 91% | nd | 91% | 91% | 100% | 91% | 91% | 100% | 93% |
| Spec 3 | 15% | nd | 15% | 11% | 13% | 10% | 8% | 2% | 3% |
| Cutoff 4 | 4080 | nd | 4110 | 4080 | 4110 | 4110 | 4080 | 4110 | 4110 |
| Sens 4 | 37% | nd | 38% | 23% | 33% | 21% | 28% | 25% | 25% |
| Spec 4 | 70% | nd | 70% | 70% | 70% | 70% | 70% | 70% | 70% |
| Cutoff 5 | 4920 | nd | 4930 | 4920 | 4920 | 4930 | 4920 | 4920 | 4930 |
| Sens 5 | 31% | nd | 31% | 14% | 17% | 14% | 12% | 25% | 7% |
| Spec 5 | 80% | nd | 80% | 80% | 80% | 80% | 80% | 80% | 80% |
| Cutoff 6 | 6170 | nd | 6430 | 6170 | 6360 | 6430 | 6170 | 6360 | 6430 |
| Sens 6 | 26% | nd | 25% | 9% | 0% | 9% | 6% | 12% | 4% |
| Spec 6 | 90% | nd | 90% | 90% | 90% | 90% | 90% | 90% | 90% |
| OR Quart 2 | 2.6 | nd | 2.3 | 1.3 | 2.0 | 1.1 | 1.9 | 0.50 | 1.2 |
| p Value | 0.11 | nd | 0.17 | 0.63 | 0.57 | 0.80 | 0.22 | 0.57 | 0.78 |
| 95% CI of OR Quart2 | 0.80 | nd | 0.70 | 0.50 | 0.18 | 0.44 | 0.68 | 0.045 | 0.38 |
| | 8.7 | nd | 7.8 | 3.2 | 23 | 2.9 | 5.3 | 5.5 | 3.6 |
| OR Quart 3 | 2.4 | nd | 2.1 | 1.4 | 0 | 1.4 | 1.2 | 1.0 | 1.5 |
| p Value | 0.16 | nd | 0.25 | 0.48 | na | 0.49 | 0.78 | 1.0 | 0.43 |
| 95% CI of OR Quart3 | 0.70 | nd | 0.60 | 0.56 | na | 0.55 | 0.38 | 0.14 | 0.53 |
| | 7.9 | nd | 7.0 | 3.4 | na | 3.4 | 3.6 | 7.2 | 4.5 |
| OR Quart 4 | 3.2 | nd | 2.9 | 1.3 | 3.1 | 1.4 | 1.3 | 1.5 | 1.0 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.050 | nd | 0.075 | 0.63 | 0.33 | 0.48 | 0.59 | 0.65 | 0.99 |
| 95% CI of | 1.0 | nd | 0.90 | 0.50 | 0.31 | 0.56 | 0.45 | 0.25 | 0.32 |
| OR Quart4 | 10 | nd | 9.4 | 3.2 | 30 | 3.4 | 4.0 | 9.2 | 3.2 |

**Gastric inhibitory polypeptide EDTA**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 25.6 | 41.6 | 25.6 | 16.9 | 25.6 | 19.7 |
| Average | 54.7 | 70.9 | 54.7 | 35.9 | 54.7 | 44.1 |
| Stdev | 82.4 | 80.8 | 82.4 | 59.3 | 82.4 | 59.3 |
| p(t-test) | | 0.26 | | 0.15 | | 0.48 |
| Min | 0.888 | 3.61 | 0.888 | 2.33 | 0.888 | 4.13 |
| Max | 820 | 405 | 820 | 287 | 820 | 251 |
| n (Samp) | 434 | 35 | 434 | 43 | 434 | 32 |
| n (Patient) | 212 | 35 | 212 | 43 | 212 | 32 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 26.2 | 19.1 | 26.2 | 28.1 |
| Average | nd | nd | 55.2 | 39.6 | 55.2 | 55.8 |
| Stdev | nd | nd | 81.0 | 40.3 | 81.0 | 80.2 |
| p(t-test) | nd | nd | | 0.64 | | 0.98 |
| Min | nd | nd | 0.888 | 7.99 | 0.888 | 8.68 |
| Max | nd | nd | 820 | 92.9 | 820 | 251 |
| n (Samp) | nd | nd | 583 | 6 | 583 | 8 |
| n (Patient) | nd | nd | 262 | 6 | 262 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.5 | 47.3 | 24.5 | 17.2 | 24.5 | 15.8 |
| Average | 55.9 | 71.2 | 55.9 | 37.1 | 55.9 | 37.8 |
| Stdev | 85.0 | 82.4 | 85.0 | 60.4 | 85.0 | 49.2 |
| p(t-test) | | 0.33 | | 0.16 | | 0.27 |
| Min | 0.888 | 3.61 | 0.888 | 2.33 | 0.888 | 4.13 |
| Max | 820 | 405 | 820 | 287 | 820 | 192 |
| n (Samp) | 415 | 32 | 415 | 43 | 415 | 28 |
| n (Patient) | 192 | 32 | 192 | 43 | 192 | 28 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | nd | 0.59 | 0.39 | 0.45 | 0.40 | 0.46 | 0.53 | 0.43 |
| SE | 0.052 | nd | 0.055 | 0.047 | 0.12 | 0.048 | 0.054 | 0.10 | 0.058 |
| p | 0.091 | nd | 0.084 | 0.021 | 0.71 | 0.038 | 0.43 | 0.75 | 0.20 |
| nCohort 1 | 434 | nd | 415 | 434 | 583 | 415 | 434 | 583 | 415 |
| nCohort 2 | 35 | nd | 32 | 43 | 6 | 43 | 32 | 8 | 28 |
| Cutoff 1 | 17.3 | nd | 17.3 | 9.01 | 9.01 | 9.01 | 14.2 | 19.5 | 13.0 |
| Sens 1 | 71% | nd | 72% | 72% | 83% | 72% | 72% | 75% | 71% |
| Spec 1 | 37% | nd | 38% | 16% | 15% | 17% | 30% | 41% | 28% |
| Cutoff 2 | 14.2 | nd | 14.2 | 7.47 | 9.01 | 7.37 | 12.6 | 17.6 | 8.52 |
| Sens 2 | 80% | nd | 81% | 81% | 83% | 81% | 81% | 88% | 82% |
| Spec 2 | 29% | nd | 31% | 11% | 15% | 11% | 26% | 38% | 15% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 3 | 7.95 | nd | 9.29 | 6.25 | 7.95 | 6.25 | 6.93 | 8.52 | 5.69 |
| Sens 3 | 91% | nd | 91% | 91% | 100% | 91% | 91% | 100% | 93% |
| Spec 3 | 13% | nd | 19% | 9% | 13% | 9% | 10% | 13% | 7% |
| Cutoff 4 | 47.6 | nd | 48.4 | 47.6 | 48.7 | 48.4 | 47.6 | 48.7 | 48.4 |
| Sens 4 | 49% | nd | 50% | 16% | 33% | 16% | 25% | 25% | 25% |
| Spec 4 | 70% | nd | 70% | 70% | 70% | 70% | 70% | 70% | 70% |
| Cutoff 5 | 75.0 | nd | 80.9 | 75.0 | 77.2 | 80.9 | 75.0 | 77.2 | 80.9 |
| Sens 5 | 34% | nd | 28% | 9% | 33% | 9% | 16% | 12% | 14% |
| Spec 5 | 80% | nd | 80% | 80% | 80% | 80% | 80% | 80% | 80% |
| Cutoff 6 | 145 | nd | 146 | 145 | 145 | 146 | 145 | 145 | 146 |
| Sens 6 | 17% | nd | 16% | 5% | 0% | 5% | 9% | 12% | 7% |
| Spec 6 | 90% | nd | 90% | 90% | 90% | 90% | 90% | 90% | 90% |
| OR Quart 2 | 1.0 | nd | 0.99 | 1.3 | 0 | 1.5 | 0.49 | 3.0 | 0.41 |
| p Value | 1.0 | nd | 0.99 | 0.59 | na | 0.44 | 0.25 | 0.34 | 0.21 |
| 95% CI of | 0.34 | nd | 0.31 | 0.48 | na | 0.54 | 0.14 | 0.31 | 0.10 |
| OR Quart2 | 2.9 | nd | 3.2 | 3.7 | na | 4.0 | 1.7 | 29 | 1.6 |
| OR Quart 3 | 0.85 | nd | 0.99 | 1.6 | 1.0 | 1.5 | 1.9 | 2.0 | 1.8 |
| p Value | 0.78 | nd | 0.99 | 0.32 | 0.99 | 0.45 | 0.18 | 0.57 | 0.24 |
| 95% CI of | 0.28 | nd | 0.31 | 0.61 | 0.14 | 0.54 | 0.75 | 0.18 | 0.68 |
| OR Quart3 | 2.6 | nd | 3.2 | 4.4 | 7.2 | 4.0 | 4.6 | 22 | 4.8 |
| OR Quart 4 | 2.3 | nd | 2.5 | 2.5 | 1.0 | 2.5 | 0.74 | 2.0 | 0.86 |
| p Value | 0.083 | nd | 0.071 | 0.052 | 0.99 | 0.051 | 0.59 | 0.57 | 0.79 |
| 95% CI of | 0.90 | nd | 0.92 | 0.99 | 0.14 | 0.99 | 0.25 | 0.18 | 0.28 |
| OR Quart4 | 5.8 | nd | 6.8 | 6.3 | 7.2 | 6.4 | 2.2 | 22 | 2.6 |

**Peptide YY EDTA**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 39.6 | 41.0 | 39.6 | 42.8 | 39.6 | 46.2 |
| Average | 56.6 | 56.9 | 56.6 | 63.2 | 56.6 | 79.8 |
| Stdev | 73.1 | 63.2 | 73.1 | 65.3 | 73.1 | 87.2 |
| p(t-test) | | 0.98 | | 0.57 | | 0.089 |
| Min | 0.00999 | 0.0108 | 0.00999 | 0.00999 | 0.00999 | 0.0108 |
| Max | 789 | 284 | 789 | 270 | 789 | 406 |
| n (Samp) | 434 | 35 | 434 | 43 | 434 | 32 |
| n (Patient) | 212 | 35 | 212 | 43 | 212 | 32 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 40.2 | 56.3 | 40.2 | 57.6 |
| Average | nd | nd | 59.4 | 51.0 | 59.4 | 81.8 |
| Stdev | nd | nd | 74.6 | 24.5 | 74.6 | 65.7 |
| p(t-test) | nd | nd | | 0.78 | | 0.40 |
| Min | nd | nd | 0.00999 | 21.6 | 0.00999 | 13.4 |
| Max | nd | nd | 789 | 78.3 | 789 | 211 |
| n (Samp) | nd | nd | 583 | 6 | 583 | 8 |
| n (Patient) | nd | nd | 262 | 6 | 262 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 40.2 | 40.1 | 40.2 | 42.5 | 40.2 | 45.0 |
| Average | 56.6 | 57.7 | 56.6 | 64.0 | 56.6 | 75.0 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 72.7 | 65.7 | 72.7 | 67.8 | 72.7 | 88.3 |
| p(t-test) | | 0.93 | | 0.52 | | 0.20 |
| Min | 0.00999 | 0.0108 | 0.00999 | 0.00999 | 0.00999 | 0.0108 |
| Max | 789 | 284 | 789 | 270 | 789 | 406 |
| n (Samp) | 415 | 32 | 415 | 43 | 415 | 28 |
| n (Patient) | 192 | 32 | 192 | 43 | 192 | 28 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | nd | 0.48 | 0.53 | 0.57 | 0.52 | 0.56 | 0.65 | 0.53 |
| SE | 0.051 | nd | 0.053 | 0.047 | 0.12 | 0.047 | 0.054 | 0.11 | 0.057 |
| p | 0.90 | nd | 0.78 | 0.57 | 0.58 | 0.73 | 0.25 | 0.15 | 0.57 |
| nCohort 1 | 434 | nd | 415 | 434 | 583 | 415 | 434 | 583 | 415 |
| nCohort 2 | 35 | nd | 32 | 43 | 6 | 43 | 32 | 8 | 28 |
| Cutoff 1 | 18.0 | nd | 18.0 | 21.6 | 22.6 | 21.6 | 26.7 | 43.7 | 23.7 |
| Sens 1 | 71% | nd | 72% | 72% | 83% | 72% | 72% | 75% | 71% |
| Spec 1 | 22% | nd | 22% | 26% | 28% | 27% | 32% | 54% | 30% |
| Cutoff 2 | 14.0 | nd | 14.0 | 10.5 | 22.6 | 10.5 | 13.4 | 36.5 | 13.4 |
| Sens 2 | 80% | nd | 81% | 81% | 83% | 81% | 84% | 88% | 82% |
| Spec 2 | 18% | nd | 18% | 15% | 28% | 15% | 18% | 45% | 18% |
| Cutoff 3 | 10.5 | nd | 10.5 | 0.0512 | 21.6 | 0.0512 | 7.32 | 13.4 | 0.0108 |
| Sens 3 | 91% | nd | 91% | 91% | 100% | 91% | 91% | 100% | 93% |
| Spec 3 | 15% | nd | 15% | 6% | 27% | 6% | 14% | 19% | 2% |
| Cutoff 4 | 58.7 | nd | 60.4 | 58.7 | 60.4 | 60.4 | 58.7 | 60.4 | 60.4 |
| Sens 4 | 29% | nd | 25% | 40% | 50% | 33% | 41% | 50% | 39% |
| Spec 4 | 70% | nd | 70% | 70% | 70% | 70% | 70% | 70% | 70% |
| Cutoff 5 | 79.5 | nd | 79.5 | 79.5 | 85.3 | 79.5 | 79.5 | 85.3 | 79.5 |
| Sens 5 | 20% | nd | 22% | 26% | 0% | 28% | 34% | 38% | 29% |
| Spec 5 | 80% | nd | 80% | 80% | 80% | 80% | 80% | 80% | 80% |
| Cutoff 6 | 111 | nd | 110 | 111 | 123 | 110 | 111 | 123 | 110 |
| Sens 6 | 11% | nd | 12% | 16% | 0% | 19% | 28% | 25% | 25% |
| Spec 6 | 90% | nd | 90% | 90% | 90% | 90% | 90% | 90% | 90% |
| OR Quart 2 | 1.0 | nd | 1.0 | 0.71 | >2.0 | 0.89 | 0.99 | 0.99 | 0.84 |
| p Value | 0.99 | nd | 1.0 | 0.47 | <0.57 | 0.80 | 0.99 | 1.00 | 0.76 |
| 95% CI of OR Quart2 | 0.39 | nd | 0.36 | 0.27 | >0.18 | 0.36 | 0.34 | 0.062 | 0.27 |
| | 2.6 | nd | 2.8 | 1.8 | na | 2.2 | 2.9 | 16 | 2.6 |
| OR Quart 3 | 0.65 | nd | 0.74 | 0.90 | >2.0 | 0.80 | 0.85 | 3.0 | 0.69 |
| p Value | 0.44 | nd | 0.58 | 0.82 | <0.57 | 0.64 | 0.78 | 0.34 | 0.54 |
| 95% CI of OR Quart3 | 0.23 | nd | 0.25 | 0.37 | >0.18 | 0.32 | 0.28 | 0.31 | 0.21 |
| | 1.9 | nd | 2.2 | 2.2 | na | 2.0 | 2.6 | 29 | 2.3 |
| OR Quart 4 | 1.3 | nd | 1.3 | 1.3 | >2.0 | 1.2 | 1.8 | 3.0 | 1.5 |
| p Value | 0.63 | nd | 0.61 | 0.54 | <0.57 | 0.68 | 0.24 | 0.34 | 0.46 |
| 95% CI of OR Quart4 | 0.50 | nd | 0.49 | 0.56 | >0.18 | 0.51 | 0.67 | 0.31 | 0.53 |
| | 3.2 | nd | 3.4 | 3.0 | na | 2.8 | 4.7 | 29 | 4.0 |

Table 7: Comparison of marker levels in EDTA samples collected within 12 hours of reaching stage R from Cohort 1 (patients that reached, but did not progress beyond, RIFLE stage R) and from Cohort 2 (patients that reached RIFLE stage I or F).

**Osteocalcin**

| | sCr or UO | sCr only | UO only |
|---|---|---|---|
| | | | |

|  | Cohort 1 | Cohort 2 | Cohort 1 | Cohort 2 | Cohort 1 | Cohort 2 |
|---|---|---|---|---|---|---|
| Median | 2080 | 1220 | 1590 | 827 | 2080 | 1240 |
| Average | 3060 | 2150 | 3400 | 953 | 2640 | 2350 |
| Stdev | 3400 | 1990 | 4600 | 410 | 2320 | 2140 |
| p(t-test) |  | 0.13 |  | 0.21 |  | 0.56 |
| Min | 0.729 | 59.3 | 129 | 648 | 0.729 | 59.3 |
| Max | 21800 | 8560 | 21800 | 1750 | 10500 | 8560 |
| n (Samp) | 83 | 36 | 31 | 6 | 65 | 31 |
| n (Patient) | 83 | 36 | 31 | 6 | 65 | 31 |

|  | At Enrollment | | |
|---|---|---|---|
|  | sCr or UO | sCr only | UO only |
| AUC | 0.41 | 0.24 | 0.45 |
| SE | 0.058 | 0.12 | 0.064 |
| p | 0.13 | 0.029 | 0.39 |
| nCohort 1 | 83 | 31 | 65 |
| nCohort 2 | 36 | 6 | 31 |
| Cutoff 1 | 860 | 654 | 888 |
| Sens 1 | 72% | 83% | 71% |
| Spec 1 | 20% | 10% | 20% |
| Cutoff 2 | 785 | 654 | 790 |
| Sens 2 | 81% | 83% | 81% |
| Spec 2 | 17% | 10% | 18% |
| Cutoff 3 | 628 | 586 | 628 |
| Sens 3 | 94% | 100% | 90% |
| Spec 3 | 11% | 6% | 15% |
| Cutoff 4 | 2980 | 2400 | 2980 |
| Sens 4 | 25% | 0% | 29% |
| Spec 4 | 71% | 71% | 71% |
| Cutoff 5 | 3980 | 4150 | 3320 |
| Sens 5 | 14% | 0% | 29% |
| Spec 5 | 81% | 81% | 80% |
| Cutoff 6 | 8250 | 8370 | 5110 |
| Sens 6 | 3% | 0% | 13% |
| Spec 6 | 90% | 90% | 91% |
| OR Quart 2 | 0.47 | >1.2 | 0.33 |
| p Value | 0.23 | <0.88 | 0.11 |
| 95% CI of | 0.14 | >0.067 | 0.086 |
| OR Quart2 | 1.6 | na | 1.3 |
| OR Quart 3 | 1.2 | >2.9 | 0.69 |
| p Value | 0.78 | <0.43 | 0.54 |
| 95% CI of | 0.39 | >0.21 | 0.21 |
| OR Quart3 | 3.5 | na | 2.3 |
| OR Quart 4 | 1.6 | >5.0 | 1.4 |
| p Value | 0.36 | <0.20 | 0.56 |
| 95% CI of | 0.56 | >0.42 | 0.45 |
| OR Quart4 | 4.8 | na | 4.5 |

**Follistatin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 766 | 840 | 748 | 519 | 806 | 886 |
| Average | 2150 | 3450 | 2410 | 839 | 2250 | 3770 |
| Stdev | 3480 | 4980 | 3790 | 1000 | 3750 | 5250 |

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| p(t-test) | | 0.11 | | 0.29 | | 0.11 |
| Min | 0.221 | 2.51 | 2.00 | 13.2 | 0.221 | 0.000554 |
| Max | 11800 | 15600 | 11800 | 3050 | 15900 | 15600 |
| n (Samp) | 83 | 36 | 32 | 7 | 64 | 31 |
| n (Patient) | 83 | 36 | 32 | 7 | 64 | 31 |

| | At Enrollment | | |
|---|---|---|---|
| | sCr or UO | sCr only | UO only |
| AUC | 0.53 | 0.37 | 0.52 |
| SE | 0.058 | 0.12 | 0.064 |
| p | 0.57 | 0.28 | 0.70 |
| nCohort 1 | 83 | 32 | 64 |
| nCohort 2 | 36 | 7 | 31 |
| Cutoff 1 | 502 | 502 | 371 |
| Sens 1 | 72% | 71% | 71% |
| Spec 1 | 30% | 31% | 20% |
| Cutoff 2 | 314 | 460 | 310 |
| Sens 2 | 81% | 86% | 81% |
| Spec 2 | 14% | 22% | 16% |
| Cutoff 3 | 9.59 | 9.59 | 3.75 |
| Sens 3 | 92% | 100% | 90% |
| Spec 3 | 8% | 6% | 5% |
| Cutoff 4 | 1190 | 1170 | 1180 |
| Sens 4 | 39% | 14% | 42% |
| Spec 4 | 71% | 72% | 70% |
| Cutoff 5 | 1430 | 1430 | 1450 |
| Sens 5 | 36% | 14% | 39% |
| Spec 5 | 81% | 81% | 81% |
| Cutoff 6 | 9880 | 10300 | 9880 |
| Sens 6 | 17% | 0% | 19% |
| Spec 6 | 90% | 91% | 91% |
| OR Quart 2 | 0.58 | 1.0 | 0.34 |
| p Value | 0.35 | 1.0 | 0.10 |
| 95% CI of | 0.18 | 0.054 | 0.095 |
| OR Quart2 | 1.8 | 19 | 1.2 |
| OR Quart 3 | 0.48 | 3.9 | 0.26 |
| p Value | 0.22 | 0.28 | 0.051 |
| 95% CI of | 0.15 | 0.33 | 0.067 |
| OR Quart3 | 1.5 | 46 | 1.0 |
| OR Quart 4 | 1.5 | 2.6 | 1.3 |
| p Value | 0.49 | 0.48 | 0.65 |
| 95% CI of | 0.51 | 0.19 | 0.41 |
| OR Quart4 | 4.2 | 34 | 4.1 |

**Islet amyloid polypeptide**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 8.57 | 8.76 | nd | nd | 8.57 | 10.4 |
| Average | 13.3 | 10.7 | nd | nd | 11.7 | 10.9 |
| Stdev | 15.5 | 8.47 | nd | nd | 11.4 | 9.82 |
| p(t-test) | | 0.57 | nd | nd | | 0.85 |
| Min | 0.0141 | 0.338 | nd | nd | 0.0141 | 0.338 |

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Max | 81.1 | 29.5 | nd | nd | 49.8 | 29.5 |
| n (Samp) | 42 | 13 | nd | nd | 34 | 9 |
| n (Patient) | 42 | 13 | nd | nd | 34 | 9 |

|  | At Enrollment | | |
|---|---|---|---|
|  | sCr or UO | sCr only | UO only |
| AUC | 0.51 | nd | 0.50 |
| SE | 0.093 | nd | 0.11 |
| p | 0.95 | nd | 0.98 |
| nCohort 1 | 42 | nd | 34 |
| nCohort 2 | 13 | nd | 9 |
| Cutoff 1 | 3.61 | nd | 3.21 |
| Sens 1 | 77% | nd | 78% |
| Spec 1 | 26% | nd | 26% |
| Cutoff 2 | 3.21 | nd | 0.338 |
| Sens 2 | 85% | nd | 89% |
| Spec 2 | 26% | nd | 12% |
| Cutoff 3 | 0.338 | nd | 0.0166 |
| Sens 3 | 92% | nd | 100% |
| Spec 3 | 10% | nd | 12% |
| Cutoff 4 | 14.1 | nd | 14.1 |
| Sens 4 | 31% | nd | 33% |
| Spec 4 | 71% | nd | 71% |
| Cutoff 5 | 17.5 | nd | 17.5 |
| Sens 5 | 23% | nd | 22% |
| Spec 5 | 81% | nd | 82% |
| Cutoff 6 | 29.3 | nd | 24.8 |
| Sens 6 | 8% | nd | 11% |
| Spec 6 | 90% | nd | 91% |
| OR Quart 2 | 2.2 | nd | 0.89 |
| p Value | 0.42 | nd | 0.92 |
| 95% CI of | 0.33 | nd | 0.10 |
| OR Quart2 | 15 | nd | 7.9 |
| OR Quart 3 | 1.5 | nd | 0.89 |
| p Value | 0.69 | nd | 0.92 |
| 95% CI of | 0.21 | nd | 0.10 |
| OR Quart3 | 11 | nd | 7.9 |
| OR Quart 4 | 2.2 | nd | 1.5 |
| p Value | 0.42 | nd | 0.70 |
| 95% CI of | 0.33 | nd | 0.20 |
| OR Quart4 | 15 | nd | 12 |

**Pancreatic prohormone**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 68.6 | 126 | nd | nd | 80.8 | 126 |
| Average | 166 | 199 | nd | nd | 193 | 246 |
| Stdev | 316 | 247 | nd | nd | 360 | 286 |
| p(t-test) |  | 0.73 | nd | nd |  | 0.69 |
| Min | 0.683 | 0.731 | nd | nd | 0.683 | 0.731 |
| Max | 1920 | 869 | nd | nd | 1920 | 869 |
| n (Samp) | 42 | 13 | nd | nd | 34 | 9 |

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| n (Patient) | 42 | 13 | nd | nd | 34 | 9 |

|  | At Enrollment | | |
|---|---|---|---|
|  | sCr or UO | sCr only | UO only |
| AUC | 0.58 | nd | 0.58 |
| SE | 0.094 | nd | 0.11 |
| p | 0.41 | nd | 0.49 |
| nCohort 1 | 42 | nd | 34 |
| nCohort 2 | 13 | nd | 9 |
| Cutoff 1 | 31.4 | nd | 31.4 |
| Sens 1 | 77% | nd | 78% |
| Spec 1 | 31% | nd | 26% |
| Cutoff 2 | 20.4 | nd | 4.47 |
| Sens 2 | 85% | nd | 89% |
| Spec 2 | 19% | nd | 15% |
| Cutoff 3 | 13.7 | nd | 0.683 |
| Sens 3 | 92% | nd | 100% |
| Spec 3 | 19% | nd | 3% |
| Cutoff 4 | 137 | nd | 137 |
| Sens 4 | 31% | nd | 44% |
| Spec 4 | 71% | nd | 71% |
| Cutoff 5 | 211 | nd | 211 |
| Sens 5 | 31% | nd | 44% |
| Spec 5 | 81% | nd | 82% |
| Cutoff 6 | 361 | nd | 498 |
| Sens 6 | 23% | nd | 11% |
| Spec 6 | 90% | nd | 91% |
| OR Quart 2 | 0.56 | nd | 0.89 |
| p Value | 0.56 | nd | 0.92 |
| 95% CI of | 0.077 | nd | 0.10 |
| OR Quart2 | 4.0 | nd | 7.9 |
| OR Quart 3 | 1.3 | nd | 0.40 |
| p Value | 0.75 | nd | 0.49 |
| 95% CI of | 0.24 | nd | 0.030 |
| OR Quart3 | 7.6 | nd | 5.2 |
| OR Quart 4 | 1.3 | nd | 2.3 |
| p Value | 0.75 | nd | 0.41 |
| 95% CI of | 0.24 | nd | 0.32 |
| OR Quart4 | 7.6 | nd | 17 |

**Transthyretin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 56300 | 52800 | nd | nd | 51000 | 57400 |
| Average | 61900 | 66500 | nd | nd | 56700 | 68900 |
| Stdev | 44300 | 41000 | nd | nd | 35900 | 43300 |
| p(t-test) |  | 0.63 | nd | nd |  | 0.20 |
| Min | 6500 | 22300 | nd | nd | 6500 | 22300 |
| Max | 248000 | 170000 | nd | nd | 165000 | 170000 |
| n (Samp) | 67 | 29 | nd | nd | 52 | 25 |
| n (Patient) | 67 | 29 | nd | nd | 52 | 25 |

|  | At Enrollment | | |
|---|---|---|---|
|  | sCr or UO | sCr only | UO only |
| AUC | 0.54 | nd | 0.57 |
| SE | 0.065 | nd | 0.071 |
| p | 0.49 | nd | 0.32 |
| nCohort 1 | 67 | nd | 52 |
| nCohort 2 | 29 | nd | 25 |
| Cutoff 1 | 35600 | nd | 34900 |
| Sens 1 | 72% | nd | 72% |
| Spec 1 | 33% | nd | 35% |
| Cutoff 2 | 32000 | nd | 31400 |
| Sens 2 | 83% | nd | 80% |
| Spec 2 | 28% | nd | 29% |
| Cutoff 3 | 25400 | nd | 25400 |
| Sens 3 | 93% | nd | 92% |
| Spec 3 | 16% | nd | 19% |
| Cutoff 4 | 68600 | nd | 69000 |
| Sens 4 | 38% | nd | 40% |
| Spec 4 | 70% | nd | 71% |
| Cutoff 5 | 85900 | nd | 84200 |
| Sens 5 | 24% | nd | 28% |
| Spec 5 | 81% | nd | 81% |
| Cutoff 6 | 120000 | nd | 107000 |
| Sens 6 | 14% | nd | 20% |
| Spec 6 | 91% | nd | 90% |
| OR Quart 2 | 2.7 | nd | 1.6 |
| p Value | 0.13 | nd | 0.49 |
| 95% CI of | 0.76 | nd | 0.41 |
| OR Quart2 | 9.7 | nd | 6.5 |
| OR Quart 3 | 1.0 | nd | 0.75 |
| p Value | 1.0 | nd | 0.70 |
| 95% CI of | 0.25 | nd | 0.17 |
| OR Quart3 | 4.0 | nd | 3.4 |
| OR Quart 4 | 2.3 | nd | 2.3 |
| p Value | 0.21 | nd | 0.23 |
| 95% CI of | 0.63 | nd | 0.59 |
| OR Quart4 | 8.2 | nd | 8.8 |

**C-peptide EDTA**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 2770 | 2630 | 3500 | 1160 | 2480 | 2490 |
| Average | 3320 | 3300 | 3930 | 2700 | 3150 | 2980 |
| Stdev | 2700 | 2540 | 2990 | 3770 | 2450 | 2140 |
| p(t-test) |  | 0.96 |  | 0.35 |  | 0.74 |
| Min | 39.8 | 137 | 157 | 163 | 39.8 | 137 |
| Max | 11400 | 11000 | 11400 | 11000 | 10200 | 10600 |
| n (Samp) | 84 | 37 | 32 | 7 | 66 | 31 |
| n (Patient) | 84 | 37 | 32 | 7 | 66 | 31 |

|  | At Enrollment | | |
|---|---|---|---|
|  | sCr or UO | sCr only | UO only |
| AUC | 0.51 | 0.30 | 0.51 |
| SE | 0.057 | 0.12 | 0.063 |

|  | At Enrollment |  |  |
|---|---|---|---|
|  | sCr or UO | sCr only | UO only |
| p | 0.80 | 0.10 | 0.91 |
| nCohort 1 | 84 | 32 | 66 |
| nCohort 2 | 37 | 7 | 31 |
| Cutoff 1 | 2050 | 1050 | 2050 |
| Sens 1 | 70% | 71% | 71% |
| Spec 1 | 43% | 16% | 44% |
| Cutoff 2 | 1520 | 865 | 1710 |
| Sens 2 | 81% | 86% | 81% |
| Spec 2 | 33% | 12% | 39% |
| Cutoff 3 | 1050 | 157 | 1030 |
| Sens 3 | 92% | 100% | 90% |
| Spec 3 | 21% | 3% | 20% |
| Cutoff 4 | 4030 | 4370 | 3890 |
| Sens 4 | 24% | 14% | 16% |
| Spec 4 | 70% | 72% | 71% |
| Cutoff 5 | 5230 | 6070 | 4660 |
| Sens 5 | 16% | 14% | 10% |
| Spec 5 | 81% | 81% | 80% |
| Cutoff 6 | 6840 | 8360 | 6470 |
| Sens 6 | 8% | 14% | 6% |
| Spec 6 | 90% | 91% | 91% |
| OR Quart 2 | 2.2 | 1.0 | 2.7 |
| p Value | 0.17 | 1.0 | 0.13 |
| 95% CI of | 0.72 | 0.054 | 0.76 |
| OR Quart2 | 6.7 | 19 | 9.7 |
| OR Quart 3 | 1.6 | 2.2 | 3.2 |
| p Value | 0.39 | 0.54 | 0.072 |
| 95% CI of | 0.53 | 0.17 | 0.90 |
| OR Quart3 | 5.1 | 30 | 11 |
| OR Quart 4 | 1.1 | 4.5 | 0.95 |
| p Value | 0.82 | 0.24 | 0.94 |
| 95% CI of | 0.36 | 0.37 | 0.24 |
| OR Quart4 | 3.7 | 54 | 3.8 |

**Peptide YY EDTA**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 40.5 | 48.2 | 42.8 | 48.0 | 39.6 | 37.8 |
| Average | 52.5 | 60.5 | 57.3 | 47.6 | 52.5 | 53.7 |
| Stdev | 42.2 | 57.0 | 53.8 | 28.8 | 42.8 | 53.1 |
| p(t-test) |  | 0.39 |  | 0.65 |  | 0.91 |
| Min | 0.00999 | 0.00999 | 0.00999 | 13.4 | 0.00999 | 0.0108 |
| Max | 189 | 234 | 234 | 79.9 | 189 | 229 |
| n (Samp) | 84 | 37 | 32 | 7 | 66 | 31 |
| n (Patient) | 84 | 37 | 32 | 7 | 66 | 31 |

|  | At Enrollment |  |  |
|---|---|---|---|
|  | sCr or UO | sCr only | UO only |
| AUC | 0.52 | 0.50 | 0.47 |
| SE | 0.057 | 0.12 | 0.063 |
| p | 0.75 | 0.97 | 0.62 |
| nCohort 1 | 84 | 32 | 66 |

| | At Enrollment | | |
|---|---|---|---|
| | sCr or UO | sCr only | UO only |
| nCohort 2 | 37 | 7 | 31 |
| Cutoff 1 | 24.6 | 21.0 | 19.3 |
| Sens 1 | 70% | 71% | 71% |
| Spec 1 | 23% | 28% | 21% |
| Cutoff 2 | 14.6 | 18.0 | 11.4 |
| Sens 2 | 81% | 86% | 81% |
| Spec 2 | 18% | 28% | 12% |
| Cutoff 3 | 6.60 | 12.1 | 6.66 |
| Sens 3 | 92% | 100% | 90% |
| Spec 3 | 11% | 22% | 11% |
| Cutoff 4 | 63.3 | 65.8 | 64.6 |
| Sens 4 | 38% | 43% | 29% |
| Spec 4 | 70% | 72% | 71% |
| Cutoff 5 | 79.8 | 97.1 | 74.9 |
| Sens 5 | 22% | 0% | 19% |
| Spec 5 | 81% | 81% | 80% |
| Cutoff 6 | 104 | 129 | 98.8 |
| Sens 6 | 14% | 0% | 16% |
| Spec 6 | 90% | 91% | 91% |
| OR Quart 2 | 0.43 | 2.0 | 0.88 |
| p Value | 0.16 | 0.60 | 0.83 |
| 95% CI of | 0.14 | 0.15 | 0.26 |
| OR Quart2 | 1.4 | 27 | 3.0 |
| OR Quart 3 | 0.63 | 2.0 | 0.71 |
| p Value | 0.41 | 0.60 | 0.59 |
| 95% CI of | 0.21 | 0.15 | 0.20 |
| OR Quart3 | 1.9 | 27 | 2.5 |
| OR Quart 4 | 1.1 | 2.0 | 1.5 |
| p Value | 0.87 | 0.60 | 0.48 |
| 95% CI of | 0.39 | 0.15 | 0.47 |
| OR Quart4 | 3.1 | 27 | 4.9 |

Table 8: Comparison of the maximum marker levels in EDTA samples collected from Cohort 1 (patients that did not progress beyond RIFLE stage 0) and the maximum values in EDTA samples collected from subjects between enrollment and 0, 24 hours, and 48 hours prior to reaching stage F in Cohort 2.

**Agouti-related protein**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0554 | 0.235 | 0.0554 | 0.235 | 0.0554 | 0.238 |
| Average | 0.0814 | 0.338 | 0.0814 | 0.243 | 0.0814 | 0.242 |
| Stdev | 0.0613 | 0.391 | 0.0613 | 0.152 | 0.0613 | 0.102 |
| p(t-test) | | 3.2E-6 | | 1.0E-7 | | 1.9E-7 |
| Min | 0.0164 | 0.0923 | 0.0164 | 0.0670 | 0.0164 | 0.0923 |
| Max | 0.285 | 1.34 | 0.285 | 0.527 | 0.285 | 0.411 |
| n (Samp) | 65 | 9 | 65 | 9 | 65 | 6 |
| n (Patient) | 65 | 9 | 65 | 9 | 65 | 6 |

| | 0hr prior to AKI stage | 24hr prior to AKI stage | 48hr prior to AKI stage |
|---|---|---|---|

| | sCr or UO | sCr only | UO only | sCr or UO | sCr only | UO only | sCr or UO | sCr only | UO only |
|---|---|---|---|---|---|---|---|---|---|
| AUC | 0.90 | nd | nd | 0.89 | nd | nd | 0.94 | nd | nd |
| SE | 0.069 | nd | nd | 0.074 | nd | nd | 0.070 | nd | nd |
| p | 4.4E-9 | nd | nd | 2.2E-7 | nd | nd | 6.0E-10 | nd | nd |
| nCohort 1 | 65 | nd | nd | 65 | nd | nd | 65 | nd | nd |
| nCohort 2 | 9 | nd | nd | 9 | nd | nd | 6 | nd | nd |
| Cutoff 1 | 0.100 | nd | nd | 0.0923 | nd | nd | 0.220 | nd | nd |
| Sens 1 | 78% | nd | nd | 78% | nd | nd | 83% | nd | nd |
| Spec 1 | 75% | nd | nd | 75% | nd | nd | 95% | nd | nd |
| Cutoff 2 | 0.0923 | nd | nd | 0.0904 | nd | nd | 0.220 | nd | nd |
| Sens 2 | 89% | nd | nd | 89% | nd | nd | 83% | nd | nd |
| Spec 2 | 75% | nd | nd | 75% | nd | nd | 95% | nd | nd |
| Cutoff 3 | 0.0904 | nd | nd | 0.0634 | nd | nd | 0.0904 | nd | nd |
| Sens 3 | 100% | nd | nd | 100% | nd | nd | 100% | nd | nd |
| Spec 3 | 75% | nd | nd | 58% | nd | nd | 75% | nd | nd |
| Cutoff 4 | 0.0837 | nd | nd | 0.0837 | nd | nd | 0.0837 | nd | nd |
| Sens 4 | 100% | nd | nd | 89% | nd | nd | 100% | nd | nd |
| Spec 4 | 71% | nd | nd | 71% | nd | nd | 71% | nd | nd |
| Cutoff 5 | 0.118 | nd | nd | 0.118 | nd | nd | 0.118 | nd | nd |
| Sens 5 | 67% | nd | nd | 67% | nd | nd | 83% | nd | nd |
| Spec 5 | 80% | nd | nd | 80% | nd | nd | 80% | nd | nd |
| Cutoff 6 | 0.184 | nd | nd | 0.184 | nd | nd | 0.184 | nd | nd |
| Sens 6 | 67% | nd | nd | 67% | nd | nd | 83% | nd | nd |
| Spec 6 | 91% | nd | nd | 91% | nd | nd | 91% | nd | nd |
| OR Quart 2 | >0 | nd | nd | >0 | nd | nd | >0 | nd | nd |
| p Value | <na | nd | nd | <na | nd | nd | <na | nd | nd |
| 95% CI of | >na | nd | nd | >na | nd | nd | >na | nd | nd |
| OR Quart2 | na | nd | nd | na | nd | nd | na | nd | nd |
| OR Quart 3 | >3.6 | nd | nd | >3.6 | nd | nd | >1.0 | nd | nd |
| p Value | <0.29 | nd | nd | <0.29 | nd | nd | <1.0 | nd | nd |
| 95% CI of | >0.34 | nd | nd | >0.34 | nd | nd | >0.058 | nd | nd |
| OR Quart3 | na | nd | nd | na | nd | nd | na | nd | nd |
| OR Quart 4 | >8.3 | nd | nd | >8.3 | nd | nd | >6.5 | nd | nd |
| p Value | <0.063 | nd | nd | <0.063 | nd | nd | <0.10 | nd | nd |
| 95% CI of | >0.89 | nd | nd | >0.89 | nd | nd | >0.68 | nd | nd |
| OR Quart4 | na | nd | nd | na | nd | nd | na | nd | nd |

**Osteocalcin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2120 | 2030 | 2120 | 2030 | 2120 | 2250 |
| Average | 2640 | 2830 | 2640 | 2800 | 2640 | 3280 |
| Stdev | 2410 | 2040 | 2410 | 2060 | 2410 | 2440 |
| p(t-test) | | 0.78 | | 0.81 | | 0.44 |
| Min | 91.1 | 674 | 91.1 | 674 | 91.1 | 691 |
| Max | 14000 | 7230 | 14000 | 7230 | 14000 | 7230 |
| n (Samp) | 107 | 14 | 107 | 14 | 107 | 9 |
| n (Patient) | 107 | 14 | 107 | 14 | 107 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2140 | 1820 | 2140 | 1820 | 2140 | 2030 |
| Average | 2850 | 3010 | 2850 | 3010 | 2850 | 3400 |
| Stdev | 2770 | 2680 | 2770 | 2680 | 2770 | 2710 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.88 | | 0.88 | | 0.63 |
| Min | 0.729 | 674 | 0.729 | 674 | 0.729 | 1100 |
| Max | 17700 | 7230 | 17700 | 7230 | 17700 | 7230 |
| n (Samp) | 209 | 7 | 209 | 7 | 209 | 6 |
| n (Patient) | 209 | 7 | 209 | 7 | 209 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2270 | 2550 | 2270 | 2550 | nd | nd |
| Average | 3050 | 3060 | 3050 | 3030 | nd | nd |
| Stdev | 2890 | 1960 | 2890 | 2010 | nd | nd |
| p(t-test) | | 0.99 | | 0.98 | nd | nd |
| Min | 91.1 | 1230 | 91.1 | 900 | nd | nd |
| Max | 14000 | 7230 | 14000 | 7230 | nd | nd |
| n (Samp) | 104 | 9 | 104 | 9 | nd | nd |
| n (Patient) | 104 | 9 | 104 | 9 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.49 | 0.56 | 0.53 | 0.49 | 0.55 | 0.58 | 0.56 | nd |
| SE | 0.084 | 0.11 | 0.10 | 0.084 | 0.11 | 0.10 | 0.10 | 0.12 | nd |
| p | 0.62 | 0.94 | 0.55 | 0.68 | 0.94 | 0.62 | 0.46 | 0.64 | nd |
| nCohort 1 | 107 | 209 | 104 | 107 | 209 | 104 | 107 | 209 | nd |
| nCohort 2 | 14 | 7 | 9 | 14 | 7 | 9 | 9 | 6 | nd |
| Cutoff 1 | 1770 | 1530 | 1790 | 1770 | 1530 | 1780 | 1510 | 1530 | nd |
| Sens 1 | 71% | 71% | 78% | 71% | 71% | 78% | 78% | 83% | nd |
| Spec 1 | 42% | 34% | 38% | 42% | 34% | 38% | 36% | 34% | nd |
| Cutoff 2 | 1210 | 1100 | 1770 | 1020 | 1100 | 1770 | 1020 | 1530 | nd |
| Sens 2 | 86% | 86% | 89% | 86% | 86% | 89% | 89% | 83% | nd |
| Spec 2 | 28% | 22% | 38% | 23% | 22% | 38% | 23% | 34% | nd |
| Cutoff 3 | 1020 | 622 | 1210 | 855 | 622 | 874 | 622 | 1100 | nd |
| Sens 3 | 93% | 100% | 100% | 93% | 100% | 100% | 100% | 100% | nd |
| Spec 3 | 23% | 10% | 27% | 19% | 10% | 17% | 12% | 22% | nd |
| Cutoff 4 | 2880 | 3210 | 3290 | 2880 | 3210 | 3290 | 2880 | 3210 | nd |
| Sens 4 | 29% | 29% | 22% | 29% | 29% | 22% | 44% | 33% | nd |
| Spec 4 | 70% | 70% | 70% | 70% | 70% | 70% | 70% | 70% | nd |
| Cutoff 5 | 3780 | 4020 | 4200 | 3780 | 4020 | 4200 | 3780 | 4020 | nd |
| Sens 5 | 21% | 29% | 22% | 21% | 29% | 22% | 33% | 33% | nd |
| Spec 5 | 80% | 80% | 81% | 80% | 80% | 81% | 80% | 80% | nd |
| Cutoff 6 | 4720 | 5140 | 7160 | 4720 | 5140 | 7160 | 4720 | 5140 | nd |
| Sens 6 | 21% | 29% | 11% | 21% | 29% | 11% | 33% | 33% | nd |
| Spec 6 | 91% | 90% | 90% | 91% | 90% | 90% | 91% | 90% | nd |
| OR Quart 2 | 2.8 | 0.49 | >4.7 | 1.4 | 0.49 | 3.2 | 1.0 | 2.0 | nd |
| p Value | 0.24 | 0.57 | <0.18 | 0.69 | 0.57 | 0.32 | 1.0 | 0.58 | nd |
| 95% CI of OR Quart2 | 0.50 | 0.043 | >0.49 | 0.28 | 0.043 | 0.32 | 0.13 | 0.18 | nd |
| | 16 | 5.6 | na | 6.8 | 5.6 | 33 | 7.6 | 23 | nd |
| OR Quart 3 | 2.2 | 1.0 | >3.4 | 1.4 | 1.0 | 3.2 | 1.0 | 0.98 | nd |
| p Value | 0.40 | 1.0 | <0.31 | 0.69 | 1.0 | 0.32 | 1.0 | 0.99 | nd |
| 95% CI of OR Quart3 | 0.36 | 0.14 | >0.33 | 0.28 | 0.14 | 0.32 | 0.13 | 0.060 | nd |
| | 13 | 7.4 | na | 6.8 | 7.4 | 33 | 7.6 | 16 | nd |
| OR Quart 4 | 1.5 | 1.0 | >2.1 | 0.96 | 1.0 | 2.0 | 1.6 | 2.0 | nd |
| p Value | 0.67 | 1.0 | <0.56 | 0.97 | 1.0 | 0.58 | 0.64 | 0.58 | nd |
| 95% CI of | 0.23 | 0.14 | >0.18 | 0.18 | 0.14 | 0.17 | 0.24 | 0.18 | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart4 | 9.7 | 7.4 | na | 5.2 | 7.4 | 23 | 10 | 23 | nd |

**Complement factor H**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 191000 | 187000 | 191000 | 187000 | 191000 | 268000 |
| Average | 203000 | 206000 | 203000 | 206000 | 203000 | 259000 |
| Stdev | 69100 | 92100 | 69100 | 92100 | 69100 | 79900 |
| p(t-test) | | 0.90 | | 0.90 | | 0.060 |
| Min | 57700 | 92800 | 57700 | 92800 | 57700 | 125000 |
| Max | 486000 | 347000 | 486000 | 347000 | 486000 | 347000 |
| n (Samp) | 89 | 10 | 89 | 10 | 89 | 6 |
| n (Patient) | 89 | 10 | 89 | 10 | 89 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 191000 | 138000 | 191000 | 138000 | nd | nd |
| Average | 206000 | 152000 | 206000 | 152000 | nd | nd |
| Stdev | 70900 | 66700 | 70900 | 66700 | nd | nd |
| p(t-test) | | 0.072 | | 0.072 | nd | nd |
| Min | 29300 | 92800 | 29300 | 92800 | nd | nd |
| Max | 486000 | 281000 | 486000 | 281000 | nd | nd |
| n (Samp) | 91 | 6 | 91 | 6 | nd | nd |
| n (Patient) | 91 | 6 | 91 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | nd | 0.24 | 0.49 | nd | 0.24 | 0.73 | nd | nd |
| SE | 0.097 | nd | 0.12 | 0.097 | nd | 0.12 | 0.12 | nd | nd |
| p | 0.91 | nd | 0.025 | 0.91 | nd | 0.025 | 0.052 | nd | nd |
| nCohort 1 | 89 | nd | 91 | 89 | nd | 91 | 89 | nd | nd |
| nCohort 2 | 10 | nd | 6 | 10 | nd | 6 | 6 | nd | nd |
| Cutoff 1 | 147000 | nd | 110000 | 147000 | nd | 110000 | 223000 | nd | nd |
| Sens 1 | 70% | nd | 83% | 70% | nd | 83% | 83% | nd | nd |
| Spec 1 | 19% | nd | 5% | 19% | nd | 5% | 72% | nd | nd |
| Cutoff 2 | 115000 | nd | 110000 | 115000 | nd | 110000 | 223000 | nd | nd |
| Sens 2 | 80% | nd | 83% | 80% | nd | 83% | 83% | nd | nd |
| Spec 2 | 7% | nd | 5% | 7% | nd | 5% | 72% | nd | nd |
| Cutoff 3 | 110000 | nd | 29300 | 110000 | nd | 29300 | 115000 | nd | nd |
| Sens 3 | 90% | nd | 100% | 90% | nd | 100% | 100% | nd | nd |
| Spec 3 | 6% | nd | 1% | 6% | nd | 1% | 7% | nd | nd |
| Cutoff 4 | 223000 | nd | 228000 | 223000 | nd | 228000 | 223000 | nd | nd |
| Sens 4 | 50% | nd | 17% | 50% | nd | 17% | 83% | nd | nd |
| Spec 4 | 71% | nd | 70% | 71% | nd | 70% | 71% | nd | nd |
| Cutoff 5 | 251000 | nd | 254000 | 251000 | nd | 254000 | 251000 | nd | nd |
| Sens 5 | 40% | nd | 17% | 40% | nd | 17% | 67% | nd | nd |
| Spec 5 | 81% | nd | 80% | 81% | nd | 80% | 81% | nd | nd |
| Cutoff 6 | 306000 | nd | 299000 | 306000 | nd | 299000 | 306000 | nd | nd |
| Sens 6 | 20% | nd | 0% | 20% | nd | 0% | 33% | nd | nd |
| Spec 6 | 91% | nd | 90% | 91% | nd | 90% | 91% | nd | nd |
| OR Quart 2 | 0.22 | nd | 0 | 0.22 | nd | 0 | 0 | nd | nd |
| p Value | 0.19 | nd | na | 0.19 | nd | na | na | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | 0.023 | nd | na | 0.023 | nd | na | na | nd | nd |
| OR Quart2 | 2.1 | nd | na | 2.1 | nd | na | na | nd | nd |
| OR Quart 3 | 0.22 | nd | 1.0 | 0.22 | nd | 1.0 | 0.96 | nd | nd |
| p Value | 0.19 | nd | 0.98 | 0.19 | nd | 0.98 | 0.98 | nd | nd |
| 95% CI of | 0.023 | nd | 0.062 | 0.023 | nd | 0.062 | 0.056 | nd | nd |
| OR Quart3 | 2.1 | nd | 18 | 2.1 | nd | 18 | 16 | nd | nd |
| OR Quart 4 | 1.0 | nd | 4.8 | 1.0 | nd | 4.8 | 4.4 | nd | nd |
| p Value | 0.95 | nd | 0.18 | 0.95 | nd | 0.18 | 0.20 | nd | nd |
| 95% CI of | 0.23 | nd | 0.50 | 0.23 | nd | 0.50 | 0.45 | nd | nd |
| OR Quart4 | 4.8 | nd | 46 | 4.8 | nd | 46 | 43 | nd | nd |

**Follistatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 896 | 1280 | 896 | 1280 | 896 | 942 |
| Average | 2500 | 4650 | 2500 | 4380 | 2500 | 3010 |
| Stdev | 4480 | 4920 | 4480 | 4890 | 4480 | 4210 |
| p(t-test) | | 0.088 | | 0.13 | | 0.75 |
| Min | 8.52 | 328 | 8.52 | 328 | 8.52 | 328 |
| Max | 19900 | 12900 | 19900 | 12900 | 19900 | 11100 |
| n (Samp) | 107 | 15 | 107 | 15 | 107 | 9 |
| n (Patient) | 107 | 15 | 107 | 15 | 107 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1010 | 1160 | 1010 | 1160 | 1010 | 1050 |
| Average | 3020 | 3990 | 3020 | 3490 | 3020 | 3550 |
| Stdev | 4950 | 4500 | 4950 | 4330 | 4950 | 4670 |
| p(t-test) | | 0.58 | | 0.79 | | 0.78 |
| Min | 8.52 | 328 | 8.52 | 328 | 8.52 | 328 |
| Max | 28900 | 11100 | 28900 | 11100 | 28900 | 11100 |
| n (Samp) | 209 | 8 | 209 | 8 | 209 | 7 |
| n (Patient) | 209 | 8 | 209 | 8 | 209 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 921 | 1160 | 921 | 1160 | nd | nd |
| Average | 2870 | 4080 | 2870 | 4080 | nd | nd |
| Stdev | 4720 | 5070 | 4720 | 5070 | nd | nd |
| p(t-test) | | 0.44 | | 0.44 | nd | nd |
| Min | 8.52 | 570 | 8.52 | 570 | nd | nd |
| Max | 19900 | 12900 | 19900 | 12900 | nd | nd |
| n (Samp) | 107 | 10 | 107 | 10 | nd | nd |
| n (Patient) | 107 | 10 | 107 | 10 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.55 | 0.60 | 0.63 | 0.55 | 0.60 | 0.49 | 0.51 | nd |
| SE | 0.082 | 0.11 | 0.099 | 0.082 | 0.11 | 0.099 | 0.10 | 0.11 | nd |
| p | 0.12 | 0.61 | 0.32 | 0.12 | 0.62 | 0.32 | 0.93 | 0.90 | nd |
| nCohort 1 | 107 | 209 | 107 | 107 | 209 | 107 | 107 | 209 | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 2 | 15 | 8 | 10 | 15 | 8 | 10 | 9 | 7 | nd |
| Cutoff 1 | 834 | 921 | 834 | 834 | 921 | 834 | 541 | 921 | nd |
| Sens 1 | 73% | 75% | 70% | 73% | 75% | 70% | 78% | 71% | nd |
| Spec 1 | 46% | 48% | 45% | 46% | 48% | 45% | 15% | 48% | nd |
| Cutoff 2 | 764 | 554 | 764 | 764 | 554 | 764 | 429 | 554 | nd |
| Sens 2 | 80% | 88% | 80% | 80% | 88% | 80% | 89% | 86% | nd |
| Spec 2 | 36% | 15% | 36% | 36% | 15% | 36% | 9% | 15% | nd |
| Cutoff 3 | 541 | 254 | 690 | 541 | 254 | 690 | 195 | 254 | nd |
| Sens 3 | 93% | 100% | 90% | 93% | 100% | 90% | 100% | 100% | nd |
| Spec 3 | 15% | 5% | 29% | 15% | 5% | 29% | 5% | 5% | nd |
| Cutoff 4 | 1210 | 1380 | 1360 | 1210 | 1380 | 1360 | 1210 | 1380 | nd |
| Sens 4 | 53% | 38% | 40% | 53% | 38% | 40% | 33% | 29% | nd |
| Spec 4 | 70% | 70% | 70% | 70% | 70% | 70% | 70% | 70% | nd |
| Cutoff 5 | 1470 | 1890 | 2050 | 1470 | 1890 | 2050 | 1470 | 1890 | nd |
| Sens 5 | 47% | 38% | 40% | 47% | 38% | 40% | 33% | 29% | nd |
| Spec 5 | 80% | 80% | 80% | 80% | 80% | 80% | 80% | 80% | nd |
| Cutoff 6 | 10300 | 12400 | 11900 | 10300 | 12400 | 11900 | 10300 | 12400 | nd |
| Sens 6 | 20% | 0% | 20% | 20% | 0% | 20% | 11% | 0% | nd |
| Spec 6 | 91% | 90% | 91% | 91% | 90% | 91% | 91% | 90% | nd |
| OR Quart 2 | 1.5 | 0.49 | 3.2 | 1.5 | 0.49 | 3.2 | 0.64 | 0.49 | nd |
| p Value | 0.67 | 0.57 | 0.32 | 0.67 | 0.57 | 0.32 | 0.64 | 0.57 | nd |
| 95% CI of | 0.23 | 0.043 | 0.32 | 0.23 | 0.043 | 0.32 | 0.099 | 0.043 | nd |
| OR Quart2 | 9.7 | 5.6 | 33 | 9.7 | 5.6 | 33 | 4.2 | 5.6 | nd |
| OR Quart 3 | 1.6 | 1.0 | 2.1 | 1.6 | 1.0 | 2.1 | 0.31 | 1.0 | nd |
| p Value | 0.64 | 1.0 | 0.56 | 0.64 | 1.0 | 0.56 | 0.32 | 1.0 | nd |
| 95% CI of | 0.24 | 0.14 | 0.18 | 0.24 | 0.14 | 0.18 | 0.030 | 0.14 | nd |
| OR Quart3 | 10 | 7.4 | 24 | 10 | 7.4 | 24 | 3.2 | 7.4 | nd |
| OR Quart 4 | 4.1 | 1.5 | 4.3 | 4.1 | 1.5 | 4.3 | 1.0 | 1.0 | nd |
| p Value | 0.097 | 0.66 | 0.20 | 0.097 | 0.66 | 0.20 | 1.0 | 1.0 | nd |
| 95% CI of | 0.77 | 0.24 | 0.45 | 0.77 | 0.24 | 0.45 | 0.18 | 0.14 | nd |
| OR Quart4 | 22 | 9.4 | 41 | 22 | 9.4 | 41 | 5.4 | 7.4 | nd |

**Vitamin D-binding protein**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 197000 | 136000 | 197000 | 136000 | 197000 | 191000 |
| Average | 226000 | 189000 | 226000 | 188000 | 226000 | 246000 |
| Stdev | 102000 | 124000 | 102000 | 125000 | 102000 | 122000 |
| p(t-test) | | 0.28 | | 0.26 | | 0.62 |
| Min | 72400 | 54200 | 72400 | 54200 | 72400 | 125000 |
| Max | 563000 | 442000 | 563000 | 442000 | 563000 | 442000 |
| n (Samp) | 88 | 11 | 88 | 11 | 88 | 7 |
| n (Patient) | 88 | 11 | 88 | 11 | 88 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 196000 | 234000 | 196000 | 234000 | 196000 | 234000 |
| Average | 222000 | 264000 | 222000 | 264000 | 222000 | 264000 |
| Stdev | 105000 | 122000 | 105000 | 122000 | 105000 | 122000 |
| p(t-test) | | 0.34 | | 0.34 | | 0.34 |
| Min | 54200 | 125000 | 54200 | 125000 | 54200 | 125000 |
| Max | 630000 | 442000 | 630000 | 442000 | 630000 | 442000 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 176 | 6 | 176 | 6 | 176 | 6 |
| n (Patient) | 176 | 6 | 176 | 6 | 176 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 214000 | 112000 | 214000 | 106000 | nd | nd |
| Average | 234000 | 144000 | 234000 | 142000 | nd | nd |
| Stdev | 105000 | 114000 | 105000 | 115000 | nd | nd |
| p(t-test) | | 0.046 | | 0.041 | nd | nd |
| Min | 68000 | 54200 | 68000 | 54200 | nd | nd |
| Max | 563000 | 370000 | 563000 | 370000 | nd | nd |
| n (Samp) | 90 | 6 | 90 | 6 | nd | nd |
| n (Patient) | 90 | 6 | 90 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.35 | 0.60 | 0.20 | 0.34 | 0.60 | 0.19 | 0.53 | 0.60 | nd |
| SE | 0.094 | 0.12 | 0.11 | 0.094 | 0.12 | 0.11 | 0.12 | 0.12 | nd |
| p | 0.10 | 0.41 | 0.0067 | 0.089 | 0.41 | 0.0042 | 0.81 | 0.41 | nd |
| nCohort 1 | 88 | 176 | 90 | 88 | 176 | 90 | 88 | 176 | nd |
| nCohort 2 | 11 | 6 | 6 | 11 | 6 | 6 | 7 | 6 | nd |
| Cutoff 1 | 125000 | 180000 | 72400 | 98900 | 180000 | 72400 | 179000 | 180000 | nd |
| Sens 1 | 73% | 83% | 83% | 73% | 83% | 83% | 71% | 83% | nd |
| Spec 1 | 10% | 41% | 2% | 2% | 41% | 2% | 38% | 41% | nd |
| Cutoff 2 | 81500 | 180000 | 72400 | 78700 | 180000 | 72400 | 134000 | 180000 | nd |
| Sens 2 | 82% | 83% | 83% | 82% | 83% | 83% | 86% | 83% | nd |
| Spec 2 | 1% | 41% | 2% | 1% | 41% | 2% | 15% | 41% | nd |
| Cutoff 3 | 72400 | 125000 | 0 | 72400 | 125000 | 0 | 125000 | 125000 | nd |
| Sens 3 | 91% | 100% | 100% | 91% | 100% | 100% | 100% | 100% | nd |
| Spec 3 | 1% | 12% | 0% | 1% | 12% | 0% | 10% | 12% | nd |
| Cutoff 4 | 247000 | 249000 | 251000 | 247000 | 249000 | 251000 | 247000 | 249000 | nd |
| Sens 4 | 27% | 50% | 17% | 27% | 50% | 17% | 43% | 50% | nd |
| Spec 4 | 70% | 70% | 70% | 70% | 70% | 70% | 70% | 70% | nd |
| Cutoff 5 | 296000 | 278000 | 302000 | 296000 | 278000 | 302000 | 296000 | 278000 | nd |
| Sens 5 | 18% | 33% | 17% | 18% | 33% | 17% | 29% | 33% | nd |
| Spec 5 | 81% | 80% | 80% | 81% | 80% | 80% | 81% | 80% | nd |
| Cutoff 6 | 388000 | 388000 | 388000 | 388000 | 388000 | 388000 | 388000 | 388000 | nd |
| Sens 6 | 9% | 17% | 0% | 9% | 17% | 0% | 14% | 17% | nd |
| Spec 6 | 91% | 90% | 90% | 91% | 90% | 90% | 91% | 90% | nd |
| OR Quart 2 | 0 | 2.0 | 0 | 0 | 2.0 | 0 | 0.95 | 2.0 | nd |
| p Value | na | 0.58 | na | na | 0.58 | na | 0.96 | 0.58 | nd |
| 95% CI of | na | 0.17 | na | na | 0.17 | na | 0.12 | 0.17 | nd |
| OR Quart2 | na | 23 | na | na | 23 | na | 7.4 | 23 | nd |
| OR Quart 3 | 0.64 | 0 | 0 | 0.64 | 0 | 0 | 0 | 0 | nd |
| p Value | 0.64 | na | na | 0.64 | na | na | na | na | nd |
| 95% CI of | 0.097 | na | na | 0.097 | na | na | na | na | nd |
| OR Quart3 | 4.2 | na | na | 4.2 | na | na | na | na | nd |
| OR Quart 4 | 2.4 | 3.1 | 6.1 | 2.4 | 3.1 | 6.1 | 1.5 | 3.1 | nd |
| p Value | 0.25 | 0.34 | 0.11 | 0.25 | 0.34 | 0.11 | 0.67 | 0.34 | nd |
| 95% CI of | 0.53 | 0.31 | 0.65 | 0.53 | 0.31 | 0.65 | 0.23 | 0.31 | nd |
| OR Quart4 | 11 | 31 | 56 | 11 | 31 | 56 | 9.9 | 31 | nd |

**Glucagon**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 61.3 | 40100 | 61.3 | 40100 | nd | nd |
| Average | 4870 | 27000 | 4870 | 27000 | nd | nd |
| Stdev | 13100 | 20200 | 13100 | 20200 | nd | nd |
| p(t-test) | | 2.6E-4 | | 2.6E-4 | nd | nd |
| Min | 10.8 | 66.0 | 10.8 | 66.0 | nd | nd |
| Max | 40100 | 40100 | 40100 | 40100 | nd | nd |
| n (Samp) | 75 | 6 | 75 | 6 | nd | nd |
| n (Patient) | 75 | 6 | 75 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.86 | nd | nd | 0.86 | nd | nd | nd | nd | nd |
| SE | 0.097 | nd | nd | 0.097 | nd | nd | nd | nd | nd |
| p | 2.0E-4 | nd | nd | 2.0E-4 | nd | nd | nd | nd | nd |
| nCohort 1 | 75 | nd | nd | 75 | nd | nd | nd | nd | nd |
| nCohort 2 | 6 | nd | nd | 6 | nd | nd | nd | nd | nd |
| Cutoff 1 | 562 | nd | nd | 562 | nd | nd | nd | nd | nd |
| Sens 1 | 83% | nd | nd | 83% | nd | nd | nd | nd | nd |
| Spec 1 | 88% | nd | nd | 88% | nd | nd | nd | nd | nd |
| Cutoff 2 | 562 | nd | nd | 562 | nd | nd | nd | nd | nd |
| Sens 2 | 83% | nd | nd | 83% | nd | nd | nd | nd | nd |
| Spec 2 | 88% | nd | nd | 88% | nd | nd | nd | nd | nd |
| Cutoff 3 | 64.3 | nd | nd | 64.3 | nd | nd | nd | nd | nd |
| Sens 3 | 100% | nd | nd | 100% | nd | nd | nd | nd | nd |
| Spec 3 | 53% | nd | nd | 53% | nd | nd | nd | nd | nd |
| Cutoff 4 | 83.0 | nd | nd | 83.0 | nd | nd | nd | nd | nd |
| Sens 4 | 83% | nd | nd | 83% | nd | nd | nd | nd | nd |
| Spec 4 | 71% | nd | nd | 71% | nd | nd | nd | nd | nd |
| Cutoff 5 | 148 | nd | nd | 148 | nd | nd | nd | nd | nd |
| Sens 5 | 83% | nd | nd | 83% | nd | nd | nd | nd | nd |
| Spec 5 | 80% | nd | nd | 80% | nd | nd | nd | nd | nd |
| Cutoff 6 | 40100 | nd | nd | 40100 | nd | nd | nd | nd | nd |
| Sens 6 | 0% | nd | nd | 0% | nd | nd | nd | nd | nd |
| Spec 6 | 100% | nd | nd | 100% | nd | nd | nd | nd | nd |
| OR Quart 2 | >0 | nd | nd | >0 | nd | nd | nd | nd | nd |
| p Value | <na | nd | nd | <na | nd | nd | nd | nd | nd |
| 95% CI of OR Quart2 | >na | nd | nd | >na | nd | nd | nd | nd | nd |
| | na | nd | nd | na | nd | nd | nd | nd | nd |
| OR Quart 3 | >1.1 | nd | nd | >1.1 | nd | nd | nd | nd | nd |
| p Value | <0.97 | nd | nd | <0.97 | nd | nd | nd | nd | nd |
| 95% CI of OR Quart3 | >0.061 | nd | nd | >0.061 | nd | nd | nd | nd | nd |
| | na | nd | nd | na | nd | nd | nd | nd | nd |
| OR Quart 4 | >6.2 | nd | nd | >6.2 | nd | nd | nd | nd | nd |
| p Value | <0.11 | nd | nd | <0.11 | nd | nd | nd | nd | nd |
| 95% CI of OR Quart4 | >0.66 | nd | nd | >0.66 | nd | nd | nd | nd | nd |
| | na | nd | nd | na | nd | nd | nd | nd | nd |

**Glucagon-like peptide 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 52.4 | 177 | 52.4 | 177 | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 59.2 | 169 | 59.2 | 169 | nd | nd |
| Stdev | 39.7 | 55.0 | 39.7 | 55.0 | nd | nd |
| p(t-test) | | 1.3E-8 | | 1.3E-8 | nd | nd |
| Min | 2.37 | 87.4 | 2.37 | 87.4 | nd | nd |
| Max | 185 | 240 | 185 | 240 | nd | nd |
| n (Samp) | 75 | 6 | 75 | 6 | nd | nd |
| n (Patient) | 75 | 6 | 75 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.95 | nd | nd | 0.95 | nd | nd | nd | nd | nd |
| SE | 0.062 | nd | nd | 0.062 | nd | nd | nd | nd | nd |
| p | 3.7E-13 | nd | nd | 3.7E-13 | nd | nd | nd | nd | nd |
| nCohort 1 | 75 | nd | nd | 75 | nd | nd | nd | nd | nd |
| nCohort 2 | 6 | nd | nd | 6 | nd | nd | nd | nd | nd |
| Cutoff 1 | 129 | nd | nd | 129 | nd | nd | nd | nd | nd |
| Sens 1 | 83% | nd | nd | 83% | nd | nd | nd | nd | nd |
| Spec 1 | 93% | nd | nd | 93% | nd | nd | nd | nd | nd |
| Cutoff 2 | 129 | nd | nd | 129 | nd | nd | nd | nd | nd |
| Sens 2 | 83% | nd | nd | 83% | nd | nd | nd | nd | nd |
| Spec 2 | 93% | nd | nd | 93% | nd | nd | nd | nd | nd |
| Cutoff 3 | 81.8 | nd | nd | 81.8 | nd | nd | nd | nd | nd |
| Sens 3 | 100% | nd | nd | 100% | nd | nd | nd | nd | nd |
| Spec 3 | 80% | nd | nd | 80% | nd | nd | nd | nd | nd |
| Cutoff 4 | 69.7 | nd | nd | 69.7 | nd | nd | nd | nd | nd |
| Sens 4 | 100% | nd | nd | 100% | nd | nd | nd | nd | nd |
| Spec 4 | 71% | nd | nd | 71% | nd | nd | nd | nd | nd |
| Cutoff 5 | 81.8 | nd | nd | 81.8 | nd | nd | nd | nd | nd |
| Sens 5 | 100% | nd | nd | 100% | nd | nd | nd | nd | nd |
| Spec 5 | 80% | nd | nd | 80% | nd | nd | nd | nd | nd |
| Cutoff 6 | 119 | nd | nd | 119 | nd | nd | nd | nd | nd |
| Sens 6 | 83% | nd | nd | 83% | nd | nd | nd | nd | nd |
| Spec 6 | 91% | nd | nd | 91% | nd | nd | nd | nd | nd |
| OR Quart 2 | >0 | nd | nd | >0 | nd | nd | nd | nd | nd |
| p Value | <na | nd | nd | <na | nd | nd | nd | nd | nd |
| 95% CI of | >na | nd | nd | >na | nd | nd | nd | nd | nd |
| OR Quart2 | na | nd | nd | na | nd | nd | nd | nd | nd |
| OR Quart 3 | >0 | nd | nd | >0 | nd | nd | nd | nd | nd |
| p Value | <na | nd | nd | <na | nd | nd | nd | nd | nd |
| 95% CI of | >na | nd | nd | >na | nd | nd | nd | nd | nd |
| OR Quart3 | na | nd | nd | na | nd | nd | nd | nd | nd |
| OR Quart 4 | >8.0 | nd | nd | >8.0 | nd | nd | nd | nd | nd |
| p Value | <0.066 | nd | nd | <0.066 | nd | nd | nd | nd | nd |
| 95% CI of | >0.87 | nd | nd | >0.87 | nd | nd | nd | nd | nd |
| OR Quart4 | na | nd | nd | na | nd | nd | nd | nd | nd |

**Appetite-regulating hormone**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.1 | 57.7 | 24.1 | 57.7 | nd | nd |
| Average | 29.0 | 54.3 | 29.0 | 54.3 | nd | nd |
| Stdev | 27.4 | 26.6 | 27.4 | 26.6 | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.032 | | 0.032 | nd | nd |
| Min | 0.251 | 22.3 | 0.251 | 22.3 | nd | nd |
| Max | 195 | 88.8 | 195 | 88.8 | nd | nd |
| n (Samp) | 75 | 6 | 75 | 6 | nd | nd |
| n (Patient) | 75 | 6 | 75 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.79 | nd | nd | 0.79 | nd | nd | nd | nd | nd |
| SE | 0.11 | nd | nd | 0.11 | nd | nd | nd | nd | nd |
| p | 0.0090 | nd | nd | 0.0090 | nd | nd | nd | nd | nd |
| nCohort 1 | 75 | nd | nd | 75 | nd | nd | nd | nd | nd |
| nCohort 2 | 6 | nd | nd | 6 | nd | nd | nd | nd | nd |
| Cutoff 1 | 26.2 | nd | nd | 26.2 | nd | nd | nd | nd | nd |
| Sens 1 | 83% | nd | nd | 83% | nd | nd | nd | nd | nd |
| Spec 1 | 61% | nd | nd | 61% | nd | nd | nd | nd | nd |
| Cutoff 2 | 26.2 | nd | nd | 26.2 | nd | nd | nd | nd | nd |
| Sens 2 | 83% | nd | nd | 83% | nd | nd | nd | nd | nd |
| Spec 2 | 61% | nd | nd | 61% | nd | nd | nd | nd | nd |
| Cutoff 3 | 21.3 | nd | nd | 21.3 | nd | nd | nd | nd | nd |
| Sens 3 | 100% | nd | nd | 100% | nd | nd | nd | nd | nd |
| Spec 3 | 41% | nd | nd | 41% | nd | nd | nd | nd | nd |
| Cutoff 4 | 31.1 | nd | nd | 31.1 | nd | nd | nd | nd | nd |
| Sens 4 | 67% | nd | nd | 67% | nd | nd | nd | nd | nd |
| Spec 4 | 71% | nd | nd | 71% | nd | nd | nd | nd | nd |
| Cutoff 5 | 40.8 | nd | nd | 40.8 | nd | nd | nd | nd | nd |
| Sens 5 | 67% | nd | nd | 67% | nd | nd | nd | nd | nd |
| Spec 5 | 80% | nd | nd | 80% | nd | nd | nd | nd | nd |
| Cutoff 6 | 51.9 | nd | nd | 51.9 | nd | nd | nd | nd | nd |
| Sens 6 | 50% | nd | nd | 50% | nd | nd | nd | nd | nd |
| Spec 6 | 91% | nd | nd | 91% | nd | nd | nd | nd | nd |
| OR Quart 2 | >1.1 | nd | nd | >1.1 | nd | nd | nd | nd | nd |
| p Value | <0.97 | nd | nd | <0.97 | nd | nd | nd | nd | nd |
| 95% CI of | >0.061 | nd | nd | >0.061 | nd | nd | nd | nd | nd |
| OR Quart2 | na | nd | nd | na | nd | nd | nd | nd | nd |
| OR Quart 3 | >1.1 | nd | nd | >1.1 | nd | nd | nd | nd | nd |
| p Value | <0.97 | nd | nd | <0.97 | nd | nd | nd | nd | nd |
| 95% CI of | >0.061 | nd | nd | >0.061 | nd | nd | nd | nd | nd |
| OR Quart3 | na | nd | nd | na | nd | nd | nd | nd | nd |
| OR Quart 4 | >4.7 | nd | nd | >4.7 | nd | nd | nd | nd | nd |
| p Value | <0.18 | nd | nd | <0.18 | nd | nd | nd | nd | nd |
| 95% CI of | >0.48 | nd | nd | >0.48 | nd | nd | nd | nd | nd |
| OR Quart4 | na | nd | nd | na | nd | nd | nd | nd | nd |

**Islet amyloid polypeptide**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.67 | 19.0 | 9.67 | 19.0 | nd | nd |
| Average | 15.3 | 18.7 | 15.3 | 18.7 | nd | nd |
| Stdev | 20.8 | 8.44 | 20.8 | 8.44 | nd | nd |
| p(t-test) | | 0.69 | | 0.69 | nd | nd |
| Min | 0.0141 | 8.76 | 0.0141 | 8.76 | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 121 | 31.6 | 121 | 31.6 | nd | nd |
| n (Samp) | 75 | 6 | 75 | 6 | nd | nd |
| n (Patient) | 75 | 6 | 75 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.74 | nd | nd | 0.74 | nd | nd | nd | nd | nd |
| SE | 0.12 | nd | nd | 0.12 | nd | nd | nd | nd | nd |
| p | 0.047 | nd | nd | 0.047 | nd | nd | nd | nd | nd |
| nCohort 1 | 75 | nd | nd | 75 | nd | nd | nd | nd | nd |
| nCohort 2 | 6 | nd | nd | 6 | nd | nd | nd | nd | nd |
| Cutoff 1 | 11.0 | nd | nd | 11.0 | nd | nd | nd | nd | nd |
| Sens 1 | 83% | nd | nd | 83% | nd | nd | nd | nd | nd |
| Spec 1 | 59% | nd | nd | 59% | nd | nd | nd | nd | nd |
| Cutoff 2 | 11.0 | nd | nd | 11.0 | nd | nd | nd | nd | nd |
| Sens 2 | 83% | nd | nd | 83% | nd | nd | nd | nd | nd |
| Spec 2 | 59% | nd | nd | 59% | nd | nd | nd | nd | nd |
| Cutoff 3 | 8.38 | nd | nd | 8.38 | nd | nd | nd | nd | nd |
| Sens 3 | 100% | nd | nd | 100% | nd | nd | nd | nd | nd |
| Spec 3 | 47% | nd | nd | 47% | nd | nd | nd | nd | nd |
| Cutoff 4 | 13.1 | nd | nd | 13.1 | nd | nd | nd | nd | nd |
| Sens 4 | 67% | nd | nd | 67% | nd | nd | nd | nd | nd |
| Spec 4 | 71% | nd | nd | 71% | nd | nd | nd | nd | nd |
| Cutoff 5 | 18.4 | nd | nd | 18.4 | nd | nd | nd | nd | nd |
| Sens 5 | 50% | nd | nd | 50% | nd | nd | nd | nd | nd |
| Spec 5 | 80% | nd | nd | 80% | nd | nd | nd | nd | nd |
| Cutoff 6 | 26.6 | nd | nd | 26.6 | nd | nd | nd | nd | nd |
| Sens 6 | 17% | nd | nd | 17% | nd | nd | nd | nd | nd |
| Spec 6 | 91% | nd | nd | 91% | nd | nd | nd | nd | nd |
| OR Quart 2 | >1.1 | nd | nd | >1.1 | nd | nd | nd | nd | nd |
| p Value | <0.97 | nd | nd | <0.97 | nd | nd | nd | nd | nd |
| 95% CI of | >0.061 | nd | nd | >0.061 | nd | nd | nd | nd | nd |
| OR Quart2 | na | nd | nd | na | nd | nd | nd | nd | nd |
| OR Quart 3 | >2.2 | nd | nd | >2.2 | nd | nd | nd | nd | nd |
| p Value | <0.53 | nd | nd | <0.53 | nd | nd | nd | nd | nd |
| 95% CI of | >0.19 | nd | nd | >0.19 | nd | nd | nd | nd | nd |
| OR Quart3 | na | nd | nd | na | nd | nd | nd | nd | nd |
| OR Quart 4 | >3.3 | nd | nd | >3.3 | nd | nd | nd | nd | nd |
| p Value | <0.32 | nd | nd | <0.32 | nd | nd | nd | nd | nd |
| 95% CI of | >0.32 | nd | nd | >0.32 | nd | nd | nd | nd | nd |
| OR Quart4 | na | nd | nd | na | nd | nd | nd | nd | nd |

**Interleukin-17A**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.90 | 4.49 | 2.90 | 4.49 | 2.90 | 5.24 |
| Average | 4.63 | 10.1 | 4.63 | 8.46 | 4.63 | 9.12 |
| Stdev | 5.51 | 14.5 | 5.51 | 9.05 | 5.51 | 8.67 |
| p(t-test) | | 0.0066 | | 0.018 | | 0.016 |
| Min | 0.000362 | 0.000482 | 0.000362 | 0.000469 | 0.000362 | 0.000532 |
| Max | 33.6 | 61.7 | 33.6 | 29.6 | 33.6 | 28.6 |
| n (Samp) | 87 | 19 | 87 | 19 | 87 | 12 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 87 | 19 | 87 | 19 | 87 | 12 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.91 | 4.18 | 2.91 | 4.18 | 2.91 | 4.49 |
| Average | 4.94 | 6.98 | 4.94 | 6.98 | 4.94 | 8.43 |
| Stdev | 6.61 | 8.09 | 6.61 | 8.09 | 6.61 | 9.34 |
| p(t-test) | | 0.35 | | 0.35 | | 0.18 |
| Min | 0.000362 | 0.901 | 0.000362 | 0.901 | 0.000362 | 2.56 |
| Max | 53.4 | 28.6 | 53.4 | 28.6 | 53.4 | 28.6 |
| n (Samp) | 184 | 10 | 184 | 10 | 184 | 7 |
| n (Patient) | 184 | 10 | 184 | 10 | 184 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.51 | 4.73 | 2.51 | 4.73 | 2.51 | 5.24 |
| Average | 4.37 | 11.3 | 4.37 | 8.67 | 4.37 | 7.96 |
| Stdev | 5.33 | 17.1 | 5.33 | 9.14 | 5.33 | 7.19 |
| p(t-test) | | 0.0031 | | 0.018 | | 0.079 |
| Min | 0.000362 | 0.000482 | 0.000362 | 0.000469 | 0.000362 | 0.000532 |
| Max | 33.6 | 61.7 | 33.6 | 29.6 | 33.6 | 19.4 |
| n (Samp) | 93 | 12 | 93 | 12 | 93 | 8 |
| n (Patient) | 93 | 12 | 93 | 12 | 93 | 8 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | 0.63 | 0.65 | 0.64 | 0.63 | 0.65 | 0.69 | 0.69 | 0.68 |
| SE | 0.074 | 0.097 | 0.090 | 0.074 | 0.097 | 0.090 | 0.089 | 0.11 | 0.11 |
| p | 0.056 | 0.18 | 0.088 | 0.059 | 0.18 | 0.093 | 0.034 | 0.10 | 0.098 |
| nCohort 1 | 87 | 184 | 93 | 87 | 184 | 93 | 87 | 184 | 93 |
| nCohort 2 | 19 | 10 | 12 | 19 | 10 | 12 | 12 | 7 | 8 |
| Cutoff 1 | 3.27 | 3.27 | 3.33 | 3.27 | 3.27 | 3.33 | 3.33 | 3.84 | 3.33 |
| Sens 1 | 74% | 70% | 75% | 74% | 70% | 75% | 75% | 71% | 75% |
| Spec 1 | 54% | 53% | 58% | 54% | 53% | 58% | 55% | 60% | 58% |
| Cutoff 2 | 2.51 | 2.66 | 2.59 | 2.51 | 2.66 | 2.59 | 2.59 | 2.66 | 2.59 |
| Sens 2 | 84% | 80% | 83% | 84% | 80% | 83% | 83% | 86% | 88% |
| Spec 2 | 48% | 49% | 52% | 48% | 49% | 52% | 49% | 49% | 52% |
| Cutoff 3 | 0.000482 | 2.51 | 0.000482 | 0.000482 | 2.51 | 0.000482 | 2.51 | 2.51 | 0.000482 |
| Sens 3 | 95% | 90% | 92% | 95% | 90% | 92% | 92% | 100% | 100% |
| Spec 3 | 5% | 46% | 4% | 5% | 46% | 4% | 48% | 46% | 4% |
| Cutoff 4 | 5.11 | 5.19 | 5.04 | 5.11 | 5.19 | 5.04 | 5.11 | 5.19 | 5.04 |
| Sens 4 | 42% | 40% | 42% | 42% | 40% | 42% | 50% | 43% | 50% |
| Spec 4 | 70% | 70% | 71% | 70% | 70% | 71% | 70% | 70% | 71% |
| Cutoff 5 | 7.72 | 7.64 | 7.66 | 7.72 | 7.64 | 7.66 | 7.72 | 7.64 | 7.66 |
| Sens 5 | 32% | 20% | 33% | 32% | 20% | 33% | 42% | 29% | 38% |
| Spec 5 | 80% | 80% | 81% | 80% | 80% | 81% | 80% | 80% | 81% |
| Cutoff 6 | 11.0 | 11.2 | 10.4 | 11.0 | 11.2 | 10.4 | 11.0 | 11.2 | 10.4 |
| Sens 6 | 26% | 10% | 33% | 26% | 10% | 33% | 25% | 14% | 25% |
| Spec 6 | 91% | 90% | 90% | 91% | 90% | 90% | 91% | 90% | 90% |
| OR Quart 2 | 0.96 | 2.0 | 0.48 | 0.96 | 2.0 | 0.48 | 2.0 | >2.0 | 1.0 |
| p Value | 0.96 | 0.58 | 0.56 | 0.96 | 0.58 | 0.56 | 0.58 | <0.57 | 1.0 |
| 95% CI of | 0.18 | 0.18 | 0.041 | 0.18 | 0.18 | 0.041 | 0.17 | >0.18 | 0.059 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart2 | 5.2 | 23 | 5.6 | 5.2 | 23 | 5.6 | 24 | na | 17 |
| OR Quart 3 | 2.8 | 4.3 | 2.2 | 2.8 | 4.3 | 2.2 | 4.4 | >3.1 | 2.1 |
| p Value | 0.17 | 0.20 | 0.39 | 0.17 | 0.20 | 0.39 | 0.20 | <0.33 | 0.56 |
| 95% CI of | 0.64 | 0.46 | 0.36 | 0.64 | 0.46 | 0.36 | 0.45 | >0.31 | 0.18 |
| OR Quart3 | 12 | 40 | 13 | 12 | 40 | 13 | 42 | na | 25 |
| OR Quart 4 | 2.2 | 3.1 | 2.7 | 2.2 | 3.1 | 2.7 | 5.8 | >2.0 | 4.4 |
| p Value | 0.31 | 0.34 | 0.26 | 0.31 | 0.34 | 0.26 | 0.12 | <0.57 | 0.20 |
| 95% CI of | 0.49 | 0.31 | 0.48 | 0.49 | 0.31 | 0.48 | 0.62 | >0.18 | 0.45 |
| OR Quart4 | 9.9 | 31 | 16 | 9.9 | 31 | 16 | 53 | na | 42 |

**Insulin receptor substrate 1**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0512 | 1.17 | 0.0512 | 1.17 | 0.0512 | 1.54 |
| Average | 0.912 | 1.72 | 0.912 | 1.72 | 0.912 | 2.78 |
| Stdev | 5.31 | 2.37 | 5.31 | 2.37 | 5.31 | 2.59 |
| p(t-test) |  | 0.64 |  | 0.64 |  | 0.40 |
| Min | 0.000172 | 0.0342 | 0.000172 | 0.0289 | 0.000172 | 1.09 |
| Max | 49.5 | 7.74 | 49.5 | 7.74 | 49.5 | 7.74 |
| n (Samp) | 89 | 10 | 89 | 10 | 89 | 6 |
| n (Patient) | 89 | 10 | 89 | 10 | 89 | 6 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0512 | 0.196 | 0.0512 | 0.196 | nd | nd |
| Average | 0.949 | 1.59 | 0.949 | 1.59 | nd | nd |
| Stdev | 5.27 | 3.05 | 5.27 | 3.05 | nd | nd |
| p(t-test) |  | 0.77 |  | 0.77 | nd | nd |
| Min | 0.000172 | 0.0342 | 0.000172 | 0.0289 | nd | nd |
| Max | 49.5 | 7.74 | 49.5 | 7.74 | nd | nd |
| n (Samp) | 91 | 6 | 91 | 6 | nd | nd |
| n (Patient) | 91 | 6 | 91 | 6 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.83 | nd | 0.76 | 0.82 | nd | 0.75 | 0.93 | nd | nd |
| SE | 0.082 | nd | 0.12 | 0.083 | nd | 0.12 | 0.073 | nd | nd |
| p | 5.9E-5 | nd | 0.025 | 1.1E-4 | nd | 0.033 | 3.1E-9 | nd | nd |
| nCohort 1 | 89 | nd | 91 | 89 | nd | 91 | 89 | nd | nd |
| nCohort 2 | 10 | nd | 6 | 10 | nd | 6 | 6 | nd | nd |
| Cutoff 1 | 0.212 | nd | 0.114 | 0.212 | nd | 0.114 | 1.09 | nd | nd |
| Sens 1 | 70% | nd | 83% | 70% | nd | 83% | 83% | nd | nd |
| Spec 1 | 82% | nd | 70% | 82% | nd | 70% | 90% | nd | nd |
| Cutoff 2 | 0.170 | nd | 0.114 | 0.170 | nd | 0.114 | 1.09 | nd | nd |
| Sens 2 | 80% | nd | 83% | 80% | nd | 83% | 83% | nd | nd |
| Spec 2 | 79% | nd | 70% | 79% | nd | 70% | 90% | nd | nd |
| Cutoff 3 | 0.114 | nd | 0.0332 | 0.114 | nd | 0.0271 | 0.929 | nd | nd |
| Sens 3 | 90% | nd | 100% | 90% | nd | 100% | 100% | nd | nd |
| Spec 3 | 69% | nd | 41% | 69% | nd | 34% | 90% | nd | nd |
| Cutoff 4 | 0.134 | nd | 0.114 | 0.134 | nd | 0.114 | 0.134 | nd | nd |
| Sens 4 | 80% | nd | 83% | 80% | nd | 83% | 100% | nd | nd |
| Spec 4 | 71% | nd | 70% | 71% | nd | 70% | 71% | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 5 | 0.186 | nd | 0.186 | 0.186 | nd | 0.186 | 0.186 | nd | nd |
| Sens 5 | 70% | nd | 50% | 70% | nd | 50% | 100% | nd | nd |
| Spec 5 | 81% | nd | 80% | 81% | nd | 80% | 81% | nd | nd |
| Cutoff 6 | 1.27 | nd | 1.19 | 1.27 | nd | 1.19 | 1.27 | nd | nd |
| Sens 6 | 30% | nd | 33% | 30% | nd | 33% | 50% | nd | nd |
| Spec 6 | 91% | nd | 90% | 91% | nd | 90% | 91% | nd | nd |
| OR Quart 2 | >1.0 | nd | >1.0 | >1.0 | nd | >1.0 | >0 | nd | nd |
| p Value | <1.0 | nd | <0.98 | <1.0 | nd | <0.98 | <na | nd | nd |
| 95% CI of OR Quart2 | >0.059 na | nd | >0.062 na | >0.059 na | nd | >0.062 na | >na na | nd | nd |
| OR Quart 3 | >2.1 | nd | >1.0 | >2.1 | nd | >1.0 | >0 | nd | nd |
| p Value | <0.56 | nd | <0.98 | <0.56 | nd | <0.98 | <na | nd | nd |
| 95% CI of OR Quart3 | >0.18 na | nd | >0.062 na | >0.18 na | nd | >0.062 na | >na na | nd | nd |
| OR Quart 4 | >9.3 | nd | >4.6 | >9.3 | nd | >4.6 | >7.7 | nd | nd |
| p Value | <0.045 | nd | <0.19 | <0.045 | nd | <0.19 | <0.070 | nd | nd |
| 95% CI of OR Quart4 | >1.1 na | nd | >0.47 na | >1.1 na | nd | >0.47 na | >0.85 na | nd | nd |

**Involucrin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.751 | 3.27 | 0.751 | 2.93 | 0.751 | 3.39 |
| Average | 0.949 | 3.05 | 0.949 | 2.98 | 0.949 | 3.31 |
| Stdev | 0.724 | 2.13 | 0.724 | 2.13 | 0.724 | 2.55 |
| p(t-test) |  | 1.7E-9 |  | 4.4E-9 |  | 2.5E-8 |
| Min | 0.0472 | 0.278 | 0.0472 | 0.278 | 0.0472 | 0.278 |
| Max | 3.56 | 7.42 | 3.56 | 7.42 | 3.56 | 7.42 |
| n (Samp) | 89 | 10 | 89 | 10 | 89 | 6 |
| n (Patient) | 89 | 10 | 89 | 10 | 89 | 6 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.800 | 3.56 | 0.800 | 3.23 | nd | nd |
| Average | 1.04 | 3.62 | 1.04 | 3.51 | nd | nd |
| Stdev | 0.779 | 2.25 | 0.779 | 2.28 | nd | nd |
| p(t-test) |  | 1.6E-9 |  | 6.7E-9 | nd | nd |
| Min | 0.0472 | 1.26 | 0.0472 | 1.26 | nd | nd |
| Max | 3.67 | 7.42 | 3.67 | 7.42 | nd | nd |
| n (Samp) | 91 | 6 | 91 | 6 | nd | nd |
| n (Patient) | 91 | 6 | 91 | 6 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.84 | nd | 0.92 | 0.84 | nd | 0.92 | 0.80 | nd | nd |
| SE | 0.080 | nd | 0.077 | 0.081 | nd | 0.079 | 0.11 | nd | nd |
| p | 2.3E-5 | nd | 4.3E-8 | 3.2E-5 | nd | 1.5E-7 | 0.0074 | nd | nd |
| nCohort 1 | 89 | nd | 91 | 89 | nd | 91 | 89 | nd | nd |
| nCohort 2 | 10 | nd | 6 | 10 | nd | 6 | 6 | nd | nd |
| Cutoff 1 | 1.33 | nd | 1.33 | 1.33 | nd | 1.33 | 1.02 | nd | nd |
| Sens 1 | 70% | nd | 83% | 70% | nd | 83% | 83% | nd | nd |
| Spec 1 | 83% | nd | 80% | 83% | nd | 80% | 73% | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 2 | 1.25 | nd | 1.33 | 1.25 | nd | 1.33 | 1.02 | nd | nd |
| Sens 2 | 80% | nd | 83% | 80% | nd | 83% | 83% | nd | nd |
| Spec 2 | 79% | nd | 80% | 79% | nd | 80% | 73% | nd | nd |
| Cutoff 3 | 1.02 | nd | 1.25 | 1.02 | nd | 1.25 | 0.240 | nd | nd |
| Sens 3 | 90% | nd | 100% | 90% | nd | 100% | 100% | nd | nd |
| Spec 3 | 73% | nd | 75% | 73% | nd | 75% | 9% | nd | nd |
| Cutoff 4 | 0.983 | nd | 1.16 | 0.983 | nd | 1.16 | 0.983 | nd | nd |
| Sens 4 | 90% | nd | 100% | 90% | nd | 100% | 83% | nd | nd |
| Spec 4 | 71% | nd | 70% | 71% | nd | 70% | 71% | nd | nd |
| Cutoff 5 | 1.30 | nd | 1.33 | 1.30 | nd | 1.33 | 1.30 | nd | nd |
| Sens 5 | 70% | nd | 83% | 70% | nd | 83% | 67% | nd | nd |
| Spec 5 | 81% | nd | 80% | 81% | nd | 80% | 81% | nd | nd |
| Cutoff 6 | 1.73 | nd | 2.12 | 1.73 | nd | 2.12 | 1.73 | nd | nd |
| Sens 6 | 60% | nd | 67% | 60% | nd | 67% | 67% | nd | nd |
| Spec 6 | 91% | nd | 90% | 91% | nd | 90% | 91% | nd | nd |
| OR Quart 2 | 0 | nd | >0 | 0 | nd | >0 | 0 | nd | nd |
| p Value | na | nd | <na | na | nd | <na | na | nd | nd |
| 95% CI of | na | nd | >na | na | nd | >na | na | nd | nd |
| OR Quart2 | na | nd | na | na | nd | na | na | nd | nd |
| OR Quart 3 | 2.0 | nd | >1.0 | 2.0 | nd | >1.0 | 0.96 | nd | nd |
| p Value | 0.58 | nd | <0.98 | 0.58 | nd | <0.98 | 0.98 | nd | nd |
| 95% CI of | 0.17 | nd | >0.062 | 0.17 | nd | >0.062 | 0.056 | nd | nd |
| OR Quart3 | 24 | nd | na | 24 | nd | na | 16 | nd | nd |
| OR Quart 4 | 8.9 | nd | >6.0 | 8.9 | nd | >6.0 | 4.4 | nd | nd |
| p Value | 0.049 | nd | <0.11 | 0.049 | nd | <0.11 | 0.20 | nd | nd |
| 95% CI of | 1.0 | nd | >0.65 | 1.0 | nd | >0.65 | 0.45 | nd | nd |
| OR Quart4 | 79 | nd | na | 79 | nd | na | 43 | nd | nd |

**Keratin, type II cytoskeletal 6 (6A, -6B, -6C mix)**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000220 | 5.13E-5 | 0.000220 | 5.13E-5 | 0.000220 | 5.13E-5 |
| Average | 2.60 | 8.50E-5 | 2.60 | 8.50E-5 | 2.60 | 5.13E-5 |
| Stdev | 14.8 | 7.10E-5 | 14.8 | 7.10E-5 | 14.8 | 0 |
| p(t-test) | | 0.58 | | 0.58 | | 0.67 |
| Min | 5.13E-5 | 5.13E-5 | 5.13E-5 | 5.13E-5 | 5.13E-5 | 5.13E-5 |
| Max | 119 | 0.000220 | 119 | 0.000220 | 119 | 5.13E-5 |
| n (Samp) | 89 | 10 | 89 | 10 | 89 | 6 |
| n (Patient) | 89 | 10 | 89 | 10 | 89 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000220 | 5.13E-5 | 0.000220 | 5.13E-5 | nd | nd |
| Average | 2.24 | 0.000107 | 2.24 | 0.000107 | nd | nd |
| Stdev | 12.7 | 8.70E-5 | 12.7 | 8.70E-5 | nd | nd |
| p(t-test) | | 0.67 | | 0.67 | nd | nd |
| Min | 5.13E-5 | 5.13E-5 | 5.13E-5 | 5.13E-5 | nd | nd |
| Max | 94.9 | 0.000220 | 94.9 | 0.000220 | nd | nd |
| n (Samp) | 91 | 6 | 91 | 6 | nd | nd |
| n (Patient) | 91 | 6 | 91 | 6 | nd | nd |

| | 0hr prior to AKI stage | 24hr prior to AKI stage | 48hr prior to AKI stage |
|---|---|---|---|

|  | sCr or UO | sCr only | UO only | sCr or UO | sCr only | UO only | sCr or UO | sCr only | UO only |
|---|---|---|---|---|---|---|---|---|---|
| AUC | 0.29 | nd | 0.40 | 0.29 | nd | 0.40 | 0.19 | nd | nd |
| SE | 0.095 | nd | 0.13 | 0.095 | nd | 0.13 | 0.11 | nd | nd |
| p | 0.025 | nd | 0.44 | 0.025 | nd | 0.44 | 0.0046 | nd | nd |
| nCohort 1 | 89 | nd | 91 | 89 | nd | 91 | 89 | nd | nd |
| nCohort 2 | 10 | nd | 6 | 10 | nd | 6 | 6 | nd | nd |
| Cutoff 1 | 0 | nd | 0 | 0 | nd | 0 | 0 | nd | nd |
| Sens 1 | 100% | nd | 100% | 100% | nd | 100% | 100% | nd | nd |
| Spec 1 | 0% | nd | 0% | 0% | nd | 0% | 0% | nd | nd |
| Cutoff 2 | 0 | nd | 0 | 0 | nd | 0 | 0 | nd | nd |
| Sens 2 | 100% | nd | 100% | 100% | nd | 100% | 100% | nd | nd |
| Spec 2 | 0% | nd | 0% | 0% | nd | 0% | 0% | nd | nd |
| Cutoff 3 | 0 | nd | 0 | 0 | nd | 0 | 0 | nd | nd |
| Sens 3 | 100% | nd | 100% | 100% | nd | 100% | 100% | nd | nd |
| Spec 3 | 0% | nd | 0% | 0% | nd | 0% | 0% | nd | nd |
| Cutoff 4 | 0.000220 | nd | 0.000220 | 0.000220 | nd | 0.000220 | 0.000220 | nd | nd |
| Sens 4 | 0% | nd | 0% | 0% | nd | 0% | 0% | nd | nd |
| Spec 4 | 94% | nd | 96% | 94% | nd | 96% | 94% | nd | nd |
| Cutoff 5 | 0.000220 | nd | 0.000220 | 0.000220 | nd | 0.000220 | 0.000220 | nd | nd |
| Sens 5 | 0% | nd | 0% | 0% | nd | 0% | 0% | nd | nd |
| Spec 5 | 94% | nd | 96% | 94% | nd | 96% | 94% | nd | nd |
| Cutoff 6 | 0.000220 | nd | 0.000220 | 0.000220 | nd | 0.000220 | 0.000220 | nd | nd |
| Sens 6 | 0% | nd | 0% | 0% | nd | 0% | 0% | nd | nd |
| Spec 6 | 94% | nd | 96% | 94% | nd | 96% | 94% | nd | nd |
| OR Quart 2 | >0 | nd | >2.3 | >0 | nd | >2.3 | >0 | nd | nd |
| p Value | <na | nd | <0.51 | <na | nd | <0.51 | <na | nd | nd |
| 95% CI of | >na | nd | >0.19 | >na | nd | >0.19 | >na | nd | nd |
| OR Quart2 | na | nd | na | na | nd | na | na | nd | nd |
| OR Quart 3 | >14 | nd | >3.6 | >14 | nd | >3.6 | >6.3 | nd | nd |
| p Value | <0.016 | nd | <0.29 | <0.016 | nd | <0.29 | <0.11 | nd | nd |
| 95% CI of | >1.6 | nd | >0.35 | >1.6 | nd | >0.35 | >0.68 | nd | nd |
| OR Quart3 | na | nd | na | na | nd | na | na | nd | nd |
| OR Quart 4 | >1.1 | nd | >1.1 | >1.1 | nd | >1.1 | >1.1 | nd | nd |
| p Value | <0.95 | nd | <0.95 | <0.95 | nd | <0.95 | <0.95 | nd | nd |
| 95% CI of | >0.064 | nd | >0.064 | >0.064 | nd | >0.064 | >0.064 | nd | nd |
| OR Quart4 | na | nd | na | na | nd | na | na | nd | nd |

**Lipopolysaccharide (serotypes -K,-O)**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0185 | 0.0572 | 0.0185 | 0.0488 | 0.0185 | 0.0323 |
| Average | 0.0813 | 0.103 | 0.0813 | 0.0893 | 0.0813 | 0.0326 |
| Stdev | 0.303 | 0.129 | 0.303 | 0.128 | 0.303 | 0.0231 |
| p(t-test) |  | 0.82 |  | 0.93 |  | 0.70 |
| Min | 6.10E-6 | 6.10E-6 | 6.10E-6 | 6.10E-6 | 6.10E-6 | 6.10E-6 |
| Max | 2.81 | 0.436 | 2.81 | 0.436 | 2.81 | 0.0575 |
| n (Samp) | 89 | 10 | 89 | 10 | 89 | 6 |
| n (Patient) | 89 | 10 | 89 | 10 | 89 | 6 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0185 | 0.112 | 0.0185 | 0.0716 | nd | nd |
| Average | 0.0827 | 0.149 | 0.0827 | 0.126 | nd | nd |
| Stdev | 0.304 | 0.153 | 0.304 | 0.159 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.60 | | 0.73 | nd | nd |
| Min | 6.10E-6 | 6.10E-6 | 6.10E-6 | 6.10E-6 | nd | nd |
| Max | 2.81 | 0.436 | 2.81 | 0.436 | nd | nd |
| n (Samp) | 91 | 6 | 91 | 6 | nd | nd |
| n (Patient) | 91 | 6 | 91 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.68 | nd | 0.75 | 0.66 | nd | 0.70 | 0.54 | nd | nd |
| SE | 0.097 | nd | 0.12 | 0.098 | nd | 0.12 | 0.12 | nd | nd |
| p | 0.060 | nd | 0.038 | 0.11 | nd | 0.095 | 0.74 | nd | nd |
| nCohort 1 | 89 | nd | 91 | 89 | nd | 91 | 89 | nd | nd |
| nCohort 2 | 10 | nd | 6 | 10 | nd | 6 | 6 | nd | nd |
| Cutoff 1 | 0.0401 | nd | 0.0542 | 0.0354 | nd | 0.0354 | 0.0159 | nd | nd |
| Sens 1 | 70% | nd | 83% | 70% | nd | 83% | 83% | nd | nd |
| Spec 1 | 66% | nd | 76% | 64% | nd | 65% | 47% | nd | nd |
| Cutoff 2 | 0.0205 | nd | 0.0542 | 0.0205 | nd | 0.0354 | 0.0159 | nd | nd |
| Sens 2 | 80% | nd | 83% | 80% | nd | 83% | 83% | nd | nd |
| Spec 2 | 53% | nd | 76% | 53% | nd | 65% | 47% | nd | nd |
| Cutoff 3 | 0.0159 | nd | 0 | 0.0159 | nd | 0 | 0 | nd | nd |
| Sens 3 | 90% | nd | 100% | 90% | nd | 100% | 100% | nd | nd |
| Spec 3 | 47% | nd | 0% | 47% | nd | 0% | 0% | nd | nd |
| Cutoff 4 | 0.0506 | nd | 0.0469 | 0.0506 | nd | 0.0469 | 0.0506 | nd | nd |
| Sens 4 | 60% | nd | 83% | 50% | nd | 67% | 33% | nd | nd |
| Spec 4 | 71% | nd | 70% | 71% | nd | 70% | 71% | nd | nd |
| Cutoff 5 | 0.0951 | nd | 0.0829 | 0.0951 | nd | 0.0829 | 0.0951 | nd | nd |
| Sens 5 | 30% | nd | 67% | 20% | nd | 50% | 0% | nd | nd |
| Spec 5 | 81% | nd | 80% | 81% | nd | 80% | 81% | nd | nd |
| Cutoff 6 | 0.121 | nd | 0.121 | 0.121 | nd | 0.121 | 0.121 | nd | nd |
| Sens 6 | 30% | nd | 50% | 20% | nd | 33% | 0% | nd | nd |
| Spec 6 | 91% | nd | 90% | 91% | nd | 90% | 91% | nd | nd |
| OR Quart 2 | 0.96 | nd | 0 | 0.96 | nd | 0 | 0.96 | nd | nd |
| p Value | 0.98 | nd | na | 0.98 | nd | na | 0.98 | nd | nd |
| 95% CI of OR Quart2 | 0.057 16 | nd | na na | 0.057 16 | nd | na na | 0.056 16 | nd | nd |
| OR Quart 3 | 4.4 | nd | 1.0 | 5.8 | nd | 2.1 | 4.4 | nd | nd |
| p Value | 0.20 | nd | 1.0 | 0.12 | nd | 0.56 | 0.20 | nd | nd |
| 95% CI of OR Quart3 | 0.45 42 | nd | 0.059 17 | 0.62 53 | nd | 0.18 25 | 0.45 43 | nd | nd |
| OR Quart 4 | 4.4 | nd | 4.4 | 3.1 | nd | 3.1 | 0 | nd | nd |
| p Value | 0.20 | nd | 0.20 | 0.34 | nd | 0.34 | na | nd | nd |
| 95% CI of OR Quart4 | 0.45 42 | nd | 0.45 42 | 0.30 32 | nd | 0.30 32 | na na | nd | nd |

**Collagenase 3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.204 | 0.102 | 0.204 | 0.102 | 0.204 | 0.0305 |
| Average | 4.13 | 17.3 | 4.13 | 12.4 | 4.13 | 4.83 |
| Stdev | 30.7 | 39.2 | 30.7 | 34.5 | 30.7 | 14.4 |
| p(t-test) | | 0.14 | | 0.34 | | 0.95 |
| Min | 0.00117 | 0.00455 | 0.00117 | 0.00117 | 0.00117 | 0.00374 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 313 | 130 | 313 | 130 | 313 | 43.1 |
| n (Samp) | 109 | 15 | 109 | 15 | 109 | 9 |
| n (Patient) | 109 | 15 | 109 | 15 | 109 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.102 | 0.0502 | 0.102 | 0.0502 | 0.102 | 0.0305 |
| Average | 4.35 | 5.47 | 4.35 | 5.47 | 4.35 | 6.24 |
| Stdev | 26.0 | 15.2 | 26.0 | 15.2 | 26.0 | 16.3 |
| p(t-test) | | 0.90 | | 0.90 | | 0.85 |
| Min | 0.00117 | 0.00726 | 0.00117 | 0.00726 | 0.00117 | 0.00726 |
| Max | 313 | 43.1 | 313 | 43.1 | 313 | 43.1 |
| n (Samp) | 213 | 8 | 213 | 8 | 213 | 7 |
| n (Patient) | 213 | 8 | 213 | 8 | 213 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.102 | 0.102 | 0.102 | 0.102 | nd | nd |
| Average | 4.11 | 21.6 | 4.11 | 14.2 | nd | nd |
| Stdev | 30.7 | 46.5 | 30.7 | 40.9 | nd | nd |
| p(t-test) | | 0.10 | | 0.33 | nd | nd |
| Min | 0.00117 | 0.00455 | 0.00117 | 0.00117 | nd | nd |
| Max | 313 | 130 | 313 | 130 | nd | nd |
| n (Samp) | 109 | 10 | 109 | 10 | nd | nd |
| n (Patient) | 109 | 10 | 109 | 10 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | 0.43 | 0.54 | 0.47 | 0.43 | 0.53 | 0.32 | 0.43 | nd |
| SE | 0.081 | 0.11 | 0.098 | 0.081 | 0.11 | 0.097 | 0.10 | 0.11 | nd |
| p | 0.79 | 0.53 | 0.67 | 0.74 | 0.53 | 0.75 | 0.079 | 0.51 | nd |
| nCohort 1 | 109 | 213 | 109 | 109 | 213 | 109 | 109 | 213 | nd |
| nCohort 2 | 15 | 8 | 10 | 15 | 8 | 10 | 9 | 7 | nd |
| Cutoff 1 | 0.00455 | 0.00455 | 0.00726 | 0.00455 | 0.00455 | 0.00726 | 0.00455 | 0.00455 | nd |
| Sens 1 | 93% | 100% | 70% | 93% | 100% | 70% | 89% | 100% | nd |
| Spec 1 | 9% | 13% | 25% | 9% | 13% | 25% | 9% | 13% | nd |
| Cutoff 2 | 0.00455 | 0.00455 | 0.00455 | 0.00455 | 0.00455 | 0.00455 | 0.00455 | 0.00455 | nd |
| Sens 2 | 93% | 100% | 90% | 93% | 100% | 90% | 89% | 100% | nd |
| Spec 2 | 9% | 13% | 14% | 9% | 13% | 14% | 9% | 13% | nd |
| Cutoff 3 | 0.00455 | 0.00455 | 0.00455 | 0.00455 | 0.00455 | 0.00455 | 0.00117 | 0.00455 | nd |
| Sens 3 | 93% | 100% | 90% | 93% | 100% | 90% | 100% | 100% | nd |
| Spec 3 | 9% | 13% | 14% | 9% | 13% | 14% | 2% | 13% | nd |
| Cutoff 4 | 0.204 | 0.204 | 0.204 | 0.204 | 0.204 | 0.204 | 0.204 | 0.204 | nd |
| Sens 4 | 33% | 25% | 40% | 33% | 25% | 40% | 11% | 29% | nd |
| Spec 4 | 72% | 70% | 74% | 72% | 70% | 74% | 72% | 70% | nd |
| Cutoff 5 | 0.383 | 0.383 | 0.383 | 0.383 | 0.383 | 0.383 | 0.383 | 0.383 | nd |
| Sens 5 | 20% | 12% | 20% | 20% | 12% | 20% | 11% | 14% | nd |
| Spec 5 | 96% | 94% | 96% | 96% | 94% | 96% | 96% | 94% | nd |
| Cutoff 6 | 0.383 | 0.383 | 0.383 | 0.383 | 0.383 | 0.383 | 0.383 | 0.383 | nd |
| Sens 6 | 20% | 12% | 20% | 20% | 12% | 20% | 11% | 14% | nd |
| Spec 6 | 96% | 94% | 96% | 96% | 94% | 96% | 96% | 94% | nd |
| OR Quart 2 | 0.64 | 1.0 | 0.96 | 0.64 | 1.0 | 0.96 | 0 | 0 | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.64 | 0.99 | 0.97 | 0.64 | 0.99 | 0.97 | na | na | nd |
| 95% CI of | 0.100 | 0.062 | 0.18 | 0.100 | 0.062 | 0.18 | na | na | nd |
| OR Quart2 | 4.1 | 17 | 5.2 | 4.1 | 17 | 5.2 | na | na | nd |
| OR Quart 3 | 1.8 | 3.2 | 0 | 1.8 | 3.2 | 0 | 3.2 | 0.49 | nd |
| p Value | 0.45 | 0.32 | na | 0.45 | 0.32 | na | 0.32 | 0.57 | nd |
| 95% CI of | 0.39 | 0.32 | na | 0.39 | 0.32 | na | 0.32 | 0.043 | nd |
| OR Quart3 | 8.3 | 31 | na | 8.3 | 31 | na | 33 | 5.6 | nd |
| OR Quart 4 | 1.8 | 3.2 | 1.3 | 1.8 | 3.2 | 1.3 | 6.0 | 2.1 | nd |
| p Value | 0.45 | 0.32 | 0.72 | 0.45 | 0.32 | 0.72 | 0.11 | 0.41 | nd |
| 95% CI of | 0.39 | 0.32 | 0.27 | 0.39 | 0.32 | 0.27 | 0.66 | 0.36 | nd |
| OR Quart4 | 8.3 | 31 | 6.6 | 8.3 | 31 | 6.6 | 55 | 12 | nd |

**NF-kappa-B inhibitor alpha**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000295 | 0.0117 | 0.000295 | 0.0107 | 0.000295 | 0.0145 |
| Average | 0.0562 | 0.0848 | 0.0562 | 0.0846 | 0.0562 | 0.0184 |
| Stdev | 0.384 | 0.156 | 0.384 | 0.156 | 0.384 | 0.0169 |
| p(t-test) | | 0.82 | | 0.82 | | 0.81 |
| Min | 2.41E-5 | 3.67E-5 | 2.41E-5 | 3.67E-5 | 2.41E-5 | 0.00131 |
| Max | 3.62 | 0.400 | 3.62 | 0.400 | 3.62 | 0.0395 |
| n (Samp) | 89 | 10 | 89 | 10 | 89 | 6 |
| n (Patient) | 89 | 10 | 89 | 10 | 89 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000560 | 0.00902 | 0.000560 | 0.00801 | nd | nd |
| Average | 0.0635 | 0.130 | 0.0635 | 0.130 | nd | nd |
| Stdev | 0.387 | 0.194 | 0.387 | 0.194 | nd | nd |
| p(t-test) | | 0.68 | | 0.68 | nd | nd |
| Min | 1.84E-7 | 3.67E-5 | 1.84E-7 | 3.67E-5 | nd | nd |
| Max | 3.62 | 0.400 | 3.62 | 0.400 | nd | nd |
| n (Samp) | 91 | 6 | 91 | 6 | nd | nd |
| n (Patient) | 91 | 6 | 91 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.77 | nd | 0.73 | 0.76 | nd | 0.73 | 0.78 | nd | nd |
| SE | 0.091 | nd | 0.12 | 0.091 | nd | 0.12 | 0.11 | nd | nd |
| p | 0.0034 | nd | 0.050 | 0.0036 | nd | 0.055 | 0.014 | nd | nd |
| nCohort 1 | 89 | nd | 91 | 89 | nd | 91 | 89 | nd | nd |
| nCohort 2 | 10 | nd | 6 | 10 | nd | 6 | 6 | nd | nd |
| Cutoff 1 | 0.00457 | nd | 0.00266 | 0.00457 | nd | 0.00266 | 0.00266 | nd | nd |
| Sens 1 | 70% | nd | 83% | 70% | nd | 83% | 83% | nd | nd |
| Spec 1 | 72% | nd | 66% | 72% | nd | 66% | 70% | nd | nd |
| Cutoff 2 | 0.00266 | nd | 0.00266 | 0.00266 | nd | 0.00266 | 0.00266 | nd | nd |
| Sens 2 | 80% | nd | 83% | 80% | nd | 83% | 83% | nd | nd |
| Spec 2 | 70% | nd | 66% | 70% | nd | 66% | 70% | nd | nd |
| Cutoff 3 | 0.00126 | nd | 2.41E-5 | 0.00126 | nd | 2.41E-5 | 0.00126 | nd | nd |
| Sens 3 | 90% | nd | 100% | 90% | nd | 100% | 100% | nd | nd |
| Spec 3 | 60% | nd | 19% | 60% | nd | 19% | 60% | nd | nd |
| Cutoff 4 | 0.00423 | nd | 0.00465 | 0.00423 | nd | 0.00465 | 0.00423 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 4 | 70% | nd | 67% | 70% | nd | 67% | 67% | nd | nd |
| Spec 4 | 71% | nd | 70% | 71% | nd | 70% | 71% | nd | nd |
| Cutoff 5 | 0.0115 | nd | 0.0138 | 0.0115 | nd | 0.0138 | 0.0115 | nd | nd |
| Sens 5 | 50% | nd | 33% | 40% | nd | 33% | 50% | nd | nd |
| Spec 5 | 81% | nd | 80% | 81% | nd | 80% | 81% | nd | nd |
| Cutoff 6 | 0.0521 | nd | 0.0479 | 0.0521 | nd | 0.0479 | 0.0521 | nd | nd |
| Sens 6 | 20% | nd | 33% | 20% | nd | 33% | 0% | nd | nd |
| Spec 6 | 91% | nd | 90% | 91% | nd | 90% | 91% | nd | nd |
| OR Quart 2 | 0 | nd | >1.0 | 0 | nd | >1.0 | >0 | nd | nd |
| p Value | na | nd | <0.98 | na | nd | <0.98 | <na | nd | nd |
| 95% CI of | na | nd | >0.062 | na | nd | >0.062 | >na | nd | nd |
| OR Quart2 | na | nd | na | na | nd | na | na | nd | nd |
| OR Quart 3 | 3.1 | nd | >2.2 | 3.1 | nd | >2.2 | >2.1 | nd | nd |
| p Value | 0.34 | nd | <0.54 | 0.34 | nd | <0.54 | <0.56 | nd | nd |
| 95% CI of | 0.30 | nd | >0.18 | 0.30 | nd | >0.18 | >0.18 | nd | nd |
| OR Quart3 | 32 | nd | na | 32 | nd | na | na | nd | nd |
| OR Quart 4 | 7.3 | nd | >3.3 | 7.3 | nd | >3.3 | >4.6 | nd | nd |
| p Value | 0.078 | nd | <0.32 | 0.078 | nd | <0.32 | <0.19 | nd | nd |
| 95% CI of | 0.80 | nd | >0.32 | 0.80 | nd | >0.32 | >0.47 | nd | nd |
| OR Quart4 | 66 | nd | na | 66 | nd | na | na | nd | nd |

**Beta-nerve growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.71 | 3.44 | 1.71 | 3.44 | 1.71 | 3.44 |
| Average | 3.00 | 4.15 | 3.00 | 3.86 | 3.00 | 3.59 |
| Stdev | 7.39 | 2.13 | 7.39 | 1.60 | 7.39 | 1.32 |
| p(t-test) | | 0.61 | | 0.70 | | 0.83 |
| Min | 0.717 | 1.92 | 0.717 | 1.92 | 0.717 | 1.81 |
| Max | 70.0 | 8.70 | 70.0 | 6.73 | 70.0 | 5.85 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.00 | 3.44 | 2.00 | 3.44 | nd | nd |
| Average | 3.36 | 4.52 | 3.36 | 4.07 | nd | nd |
| Stdev | 7.36 | 2.60 | 7.36 | 1.95 | nd | nd |
| p(t-test) | | 0.68 | | 0.80 | nd | nd |
| Min | 0.774 | 1.92 | 0.774 | 1.92 | nd | nd |
| Max | 70.0 | 8.70 | 70.0 | 6.73 | nd | nd |
| n (Samp) | 89 | 7 | 89 | 7 | nd | nd |
| n (Patient) | 89 | 7 | 89 | 7 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.82 | nd | 0.76 | 0.82 | nd | 0.75 | 0.82 | nd | nd |
| SE | 0.079 | nd | 0.11 | 0.080 | nd | 0.11 | 0.100 | nd | nd |
| p | 4.5E-5 | nd | 0.019 | 5.9E-5 | nd | 0.023 | 0.0015 | nd | nd |
| nCohort 1 | 88 | nd | 89 | 88 | nd | 89 | 88 | nd | nd |
| nCohort 2 | 11 | nd | 7 | 11 | nd | 7 | 7 | nd | nd |
| Cutoff 1 | 2.79 | nd | 2.79 | 2.79 | nd | 2.79 | 3.05 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 1 | 73% | nd | 71% | 73% | nd | 71% | 71% | nd | nd |
| Spec 1 | 76% | nd | 69% | 76% | nd | 69% | 83% | nd | nd |
| Cutoff 2 | 2.64 | nd | 2.09 | 2.64 | nd | 2.09 | 2.64 | nd | nd |
| Sens 2 | 82% | nd | 86% | 82% | nd | 86% | 86% | nd | nd |
| Spec 2 | 75% | nd | 53% | 75% | nd | 53% | 75% | nd | nd |
| Cutoff 3 | 2.06 | nd | 1.90 | 2.06 | nd | 1.90 | 1.78 | nd | nd |
| Sens 3 | 91% | nd | 100% | 91% | nd | 100% | 100% | nd | nd |
| Spec 3 | 62% | nd | 44% | 62% | nd | 44% | 52% | nd | nd |
| Cutoff 4 | 2.57 | nd | 2.91 | 2.57 | nd | 2.91 | 2.57 | nd | nd |
| Sens 4 | 82% | nd | 57% | 82% | nd | 57% | 86% | nd | nd |
| Spec 4 | 70% | nd | 72% | 70% | nd | 72% | 70% | nd | nd |
| Cutoff 5 | 2.92 | nd | 3.63 | 2.92 | nd | 3.63 | 2.92 | nd | nd |
| Sens 5 | 64% | nd | 43% | 64% | nd | 43% | 71% | nd | nd |
| Spec 5 | 81% | nd | 81% | 81% | nd | 81% | 81% | nd | nd |
| Cutoff 6 | 4.01 | nd | 4.99 | 4.01 | nd | 4.99 | 4.01 | nd | nd |
| Sens 6 | 36% | nd | 43% | 36% | nd | 43% | 29% | nd | nd |
| Spec 6 | 91% | nd | 91% | 91% | nd | 91% | 91% | nd | nd |
| OR Quart 2 | >1.0 | nd | >1.0 | >1.0 | nd | >1.0 | >1.0 | nd | nd |
| p Value | <1.0 | nd | <0.98 | <1.0 | nd | <0.98 | <1.0 | nd | nd |
| 95% CI of OR Quart2 | >0.059 | nd | >0.062 | >0.059 | nd | >0.062 | >0.059 | nd | nd |
| | na | nd | na | na | nd | na | na | nd | nd |
| OR Quart 3 | >3.3 | nd | >2.2 | >3.3 | nd | >2.2 | >1.0 | nd | nd |
| p Value | <0.32 | nd | <0.54 | <0.32 | nd | <0.54 | <1.0 | nd | nd |
| 95% CI of OR Quart3 | >0.32 | nd | >0.18 | >0.32 | nd | >0.18 | >0.059 | nd | nd |
| | na | nd | na | na | nd | na | na | nd | nd |
| OR Quart 4 | >9.3 | nd | >4.8 | >9.3 | nd | >4.8 | >6.1 | nd | nd |
| p Value | <0.045 | nd | <0.18 | <0.045 | nd | <0.18 | <0.11 | nd | nd |
| 95% CI of OR Quart4 | >1.1 | nd | >0.50 | >1.1 | nd | >0.50 | >0.65 | nd | nd |
| | na | nd | na | na | nd | na | na | nd | nd |

**Pancreatic prohormone**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 59.6 | 182 | 59.6 | 182 | nd | nd |
| Average | 171 | 313 | 171 | 313 | nd | nd |
| Stdev | 256 | 379 | 256 | 379 | nd | nd |
| p(t-test) | | 0.21 | | 0.21 | nd | nd |
| Min | 0.731 | 55.6 | 0.731 | 55.6 | nd | nd |
| Max | 1300 | 1080 | 1300 | 1080 | nd | nd |
| n (Samp) | 75 | 6 | 75 | 6 | nd | nd |
| n (Patient) | 75 | 6 | 75 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.74 | nd | nd | 0.74 | nd | nd | nd | nd | nd |
| SE | 0.12 | nd | nd | 0.12 | nd | nd | nd | nd | nd |
| p | 0.043 | nd | nd | 0.043 | nd | nd | nd | nd | nd |
| nCohort 1 | 75 | nd | nd | 75 | nd | nd | nd | nd | nd |
| nCohort 2 | 6 | nd | nd | 6 | nd | nd | nd | nd | nd |
| Cutoff 1 | 132 | nd | nd | 132 | nd | nd | nd | nd | nd |
| Sens 1 | 83% | nd | nd | 83% | nd | nd | nd | nd | nd |
| Spec 1 | 71% | nd | nd | 71% | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 2 | 132 | nd | nd | 132 | nd | nd | nd | nd | nd |
| Sens 2 | 83% | nd | nd | 83% | nd | nd | nd | nd | nd |
| Spec 2 | 71% | nd | nd | 71% | nd | nd | nd | nd | nd |
| Cutoff 3 | 55.2 | nd | nd | 55.2 | nd | nd | nd | nd | nd |
| Sens 3 | 100% | nd | nd | 100% | nd | nd | nd | nd | nd |
| Spec 3 | 48% | nd | nd | 48% | nd | nd | nd | nd | nd |
| Cutoff 4 | 132 | nd | nd | 132 | nd | nd | nd | nd | nd |
| Sens 4 | 83% | nd | nd | 83% | nd | nd | nd | nd | nd |
| Spec 4 | 71% | nd | nd | 71% | nd | nd | nd | nd | nd |
| Cutoff 5 | 218 | nd | nd | 218 | nd | nd | nd | nd | nd |
| Sens 5 | 33% | nd | nd | 33% | nd | nd | nd | nd | nd |
| Spec 5 | 80% | nd | nd | 80% | nd | nd | nd | nd | nd |
| Cutoff 6 | 504 | nd | nd | 504 | nd | nd | nd | nd | nd |
| Sens 6 | 17% | nd | nd | 17% | nd | nd | nd | nd | nd |
| Spec 6 | 91% | nd | nd | 91% | nd | nd | nd | nd | nd |
| OR Quart 2 | >1.1 | nd | nd | >1.1 | nd | nd | nd | nd | nd |
| p Value | <0.97 | nd | nd | <0.97 | nd | nd | nd | nd | nd |
| 95% CI of OR Quart2 | >0.061 na | nd | nd | >0.061 na | nd | nd | nd | nd | nd |
| OR Quart 3 | >2.2 | nd | nd | >2.2 | nd | nd | nd | nd | nd |
| p Value | <0.53 | nd | nd | <0.53 | nd | nd | nd | nd | nd |
| 95% CI of OR Quart3 | >0.19 na | nd | nd | >0.19 na | nd | nd | nd | nd | nd |
| OR Quart 4 | >3.3 | nd | nd | >3.3 | nd | nd | nd | nd | nd |
| p Value | <0.32 | nd | nd | <0.32 | nd | nd | nd | nd | nd |
| 95% CI of OR Quart4 | >0.32 na | nd | nd | >0.32 na | nd | nd | nd | nd | nd |

**Transthyretin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 70200 | 61300 | 70200 | 61300 | 70200 | 74200 |
| Average | 73000 | 63100 | 73000 | 63100 | 73000 | 73900 |
| Stdev | 37700 | 20700 | 37700 | 20700 | 37700 | 15600 |
| p(t-test) | | 0.42 | | 0.42 | | 0.95 |
| Min | 12800 | 30100 | 12800 | 30100 | 12800 | 57400 |
| Max | 242000 | 91800 | 242000 | 91800 | 242000 | 91800 |
| n (Samp) | 89 | 10 | 89 | 10 | 89 | 6 |
| n (Patient) | 89 | 10 | 89 | 10 | 89 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 72000 | 55400 | 72000 | 55400 | nd | nd |
| Average | 74900 | 56100 | 74900 | 56100 | nd | nd |
| Stdev | 41800 | 22300 | 41800 | 22300 | nd | nd |
| p(t-test) | | 0.28 | | 0.28 | nd | nd |
| Min | 8940 | 30100 | 8940 | 30100 | nd | nd |
| Max | 248000 | 91800 | 248000 | 91800 | nd | nd |
| n (Samp) | 91 | 6 | 91 | 6 | nd | nd |
| n (Patient) | 91 | 6 | 91 | 6 | nd | nd |

| | 0hr prior to AKI stage | 24hr prior to AKI stage | 48hr prior to AKI stage |
|---|---|---|---|

|  | sCr or UO | sCr only | UO only | sCr or UO | sCr only | UO only | sCr or UO | sCr only | UO only |
|---|---|---|---|---|---|---|---|---|---|
| AUC | 0.42 | nd | 0.35 | 0.42 | nd | 0.35 | 0.53 | nd | nd |
| SE | 0.099 | nd | 0.13 | 0.099 | nd | 0.13 | 0.12 | nd | nd |
| p | 0.45 | nd | 0.23 | 0.45 | nd | 0.23 | 0.79 | nd | nd |
| nCohort 1 | 89 | nd | 91 | 89 | nd | 91 | 89 | nd | nd |
| nCohort 2 | 10 | nd | 6 | 10 | nd | 6 | 6 | nd | nd |
| Cutoff 1 | 55600 | nd | 34000 | 55600 | nd | 34000 | 57800 | nd | nd |
| Sens 1 | 70% | nd | 83% | 70% | nd | 83% | 83% | nd | nd |
| Spec 1 | 34% | nd | 14% | 34% | nd | 14% | 37% | nd | nd |
| Cutoff 2 | 53300 | nd | 34000 | 53300 | nd | 34000 | 57800 | nd | nd |
| Sens 2 | 80% | nd | 83% | 80% | nd | 83% | 83% | nd | nd |
| Spec 2 | 33% | nd | 14% | 33% | nd | 14% | 37% | nd | nd |
| Cutoff 3 | 36200 | nd | 29300 | 36200 | nd | 29300 | 55600 | nd | nd |
| Sens 3 | 90% | nd | 100% | 90% | nd | 100% | 100% | nd | nd |
| Spec 3 | 17% | nd | 11% | 17% | nd | 11% | 34% | nd | nd |
| Cutoff 4 | 89900 | nd | 88500 | 89900 | nd | 88500 | 89900 | nd | nd |
| Sens 4 | 10% | nd | 17% | 10% | nd | 17% | 17% | nd | nd |
| Spec 4 | 71% | nd | 70% | 71% | nd | 70% | 71% | nd | nd |
| Cutoff 5 | 98900 | nd | 97800 | 98900 | nd | 97800 | 98900 | nd | nd |
| Sens 5 | 0% | nd | 0% | 0% | nd | 0% | 0% | nd | nd |
| Spec 5 | 81% | nd | 80% | 81% | nd | 80% | 81% | nd | nd |
| Cutoff 6 | 120000 | nd | 123000 | 120000 | nd | 123000 | 120000 | nd | nd |
| Sens 6 | 0% | nd | 0% | 0% | nd | 0% | 0% | nd | nd |
| Spec 6 | 91% | nd | 90% | 91% | nd | 90% | 91% | nd | nd |
| OR Quart 2 | >3.4 | nd | 0 | >3.4 | nd | 0 | >3.3 | nd | nd |
| p Value | <0.30 | nd | na | <0.30 | nd | na | <0.32 | nd | nd |
| 95% CI of | >0.33 | nd | na | >0.33 | nd | na | >0.32 | nd | nd |
| OR Quart2 | na | nd | na | na | nd | na | na | nd | nd |
| OR Quart 3 | >6.2 | nd | 3.4 | >6.2 | nd | 3.4 | >3.3 | nd | nd |
| p Value | <0.11 | nd | 0.30 | <0.11 | nd | 0.30 | <0.32 | nd | nd |
| 95% CI of | >0.67 | nd | 0.33 | >0.67 | nd | 0.33 | >0.32 | nd | nd |
| OR Quart3 | na | nd | 36 | na | nd | 36 | na | nd | nd |
| OR Quart 4 | >2.3 | nd | 2.2 | >2.3 | nd | 2.2 | >0 | nd | nd |
| p Value | <0.51 | nd | 0.54 | <0.51 | nd | 0.54 | <na | nd | nd |
| 95% CI of | >0.19 | nd | 0.18 | >0.19 | nd | 0.18 | >na | nd | nd |
| OR Quart4 | na | nd | 26 | na | nd | 26 | na | nd | nd |

**C-peptide EDTA**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 2640 | 3360 | 2640 | 3360 | 2640 | 2520 |
| Average | 3140 | 4210 | 3140 | 4080 | 3140 | 3740 |
| Stdev | 2280 | 2920 | 2280 | 2780 | 2280 | 3320 |
| p(t-test) |  | 0.11 |  | 0.16 |  | 0.46 |
| Min | 65.0 | 878 | 65.0 | 878 | 65.0 | 878 |
| Max | 13900 | 11000 | 13900 | 11000 | 13900 | 11000 |
| n (Samp) | 109 | 14 | 109 | 14 | 109 | 9 |
| n (Patient) | 109 | 14 | 109 | 14 | 109 | 9 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 2940 | 2880 | 2940 | 2880 | 2940 | 2450 |
| Average | 3530 | 4170 | 3530 | 4170 | 3530 | 4320 |
| Stdev | 2810 | 3700 | 2810 | 3700 | 2810 | 4030 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.56 | | 0.56 | | 0.50 |
| Min | 51.9 | 878 | 51.9 | 878 | 51.9 | 878 |
| Max | 15400 | 11000 | 15400 | 11000 | 15400 | 11000 |
| n (Samp) | 213 | 7 | 213 | 7 | 213 | 6 |
| n (Patient) | 213 | 7 | 213 | 7 | 213 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3080 | 3420 | 3080 | 3420 | nd | nd |
| Average | 3620 | 3840 | 3620 | 3640 | nd | nd |
| Stdev | 3160 | 2070 | 3160 | 1670 | nd | nd |
| p(t-test) | | 0.84 | | 0.99 | nd | nd |
| Min | 65.0 | 999 | 65.0 | 999 | nd | nd |
| Max | 21700 | 7900 | 21700 | 6070 | nd | nd |
| n (Samp) | 106 | 9 | 106 | 9 | nd | nd |
| n (Patient) | 106 | 9 | 106 | 9 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.53 | 0.58 | 0.60 | 0.53 | 0.57 | 0.52 | 0.52 | nd |
| SE | 0.084 | 0.11 | 0.10 | 0.084 | 0.11 | 0.10 | 0.10 | 0.12 | nd |
| p | 0.21 | 0.81 | 0.45 | 0.23 | 0.81 | 0.49 | 0.81 | 0.87 | nd |
| nCohort 1 | 109 | 213 | 106 | 109 | 213 | 106 | 109 | 213 | nd |
| nCohort 2 | 14 | 7 | 9 | 14 | 7 | 9 | 9 | 6 | nd |
| Cutoff 1 | 2570 | 2010 | 2570 | 2570 | 2010 | 2570 | 1730 | 1620 | nd |
| Sens 1 | 71% | 71% | 78% | 71% | 71% | 78% | 78% | 83% | nd |
| Spec 1 | 50% | 36% | 46% | 50% | 36% | 46% | 33% | 29% | nd |
| Cutoff 2 | 1460 | 1620 | 1970 | 1460 | 1620 | 1970 | 1460 | 1620 | nd |
| Sens 2 | 86% | 86% | 89% | 86% | 86% | 89% | 89% | 83% | nd |
| Spec 2 | 28% | 29% | 37% | 28% | 29% | 37% | 28% | 29% | nd |
| Cutoff 3 | 894 | 875 | 894 | 894 | 875 | 894 | 776 | 875 | nd |
| Sens 3 | 93% | 100% | 100% | 93% | 100% | 100% | 100% | 100% | nd |
| Spec 3 | 13% | 10% | 11% | 13% | 10% | 11% | 12% | 10% | nd |
| Cutoff 4 | 4260 | 4400 | 4360 | 4260 | 4400 | 4360 | 4260 | 4400 | nd |
| Sens 4 | 43% | 29% | 44% | 43% | 29% | 44% | 22% | 33% | nd |
| Spec 4 | 71% | 70% | 71% | 71% | 70% | 71% | 71% | 70% | nd |
| Cutoff 5 | 5000 | 5250 | 5370 | 5000 | 5250 | 5370 | 5000 | 5250 | nd |
| Sens 5 | 29% | 29% | 11% | 29% | 29% | 11% | 22% | 33% | nd |
| Spec 5 | 81% | 80% | 80% | 81% | 80% | 80% | 81% | 80% | nd |
| Cutoff 6 | 5940 | 6840 | 6420 | 5940 | 6840 | 6420 | 5940 | 6840 | nd |
| Sens 6 | 21% | 29% | 11% | 21% | 29% | 0% | 22% | 33% | nd |
| Spec 6 | 91% | 90% | 91% | 91% | 90% | 91% | 91% | 90% | nd |
| OR Quart 2 | 1.5 | 3.1 | 3.1 | 1.5 | 3.1 | 3.1 | 4.3 | 3.1 | nd |
| p Value | 0.67 | 0.33 | 0.34 | 0.67 | 0.33 | 0.34 | 0.20 | 0.34 | nd |
| 95% CI of OR Quart2 | 0.23 | 0.31 | 0.30 | 0.23 | 0.31 | 0.30 | 0.45 | 0.31 | nd |
| | 9.7 | 31 | 32 | 9.7 | 31 | 32 | 41 | 30 | nd |
| OR Quart 3 | 2.1 | 1.0 | 2.0 | 2.1 | 1.0 | 2.0 | 2.1 | 0 | nd |
| p Value | 0.42 | 1.0 | 0.58 | 0.42 | 1.0 | 0.58 | 0.56 | na | nd |
| 95% CI of OR Quart3 | 0.35 | 0.061 | 0.17 | 0.35 | 0.061 | 0.17 | 0.18 | na | nd |
| | 12 | 16 | 23 | 12 | 16 | 23 | 24 | na | nd |
| OR Quart 4 | 2.7 | 2.0 | 3.1 | 2.7 | 2.0 | 3.1 | 2.0 | 2.0 | nd |
| p Value | 0.26 | 0.57 | 0.34 | 0.26 | 0.57 | 0.34 | 0.58 | 0.58 | nd |
| 95% CI of | 0.48 | 0.18 | 0.30 | 0.48 | 0.18 | 0.30 | 0.17 | 0.18 | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart4 | 15 | 23 | 32 | 15 | 23 | 32 | 23 | 23 | nd |

**Gastric inhibitory polypeptide EDTA**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 39.5 | 52.3 | 39.5 | 47.0 | 39.5 | 95.8 |
| Average | 74.0 | 74.7 | 74.0 | 73.9 | 74.0 | 103 |
| Stdev | 110 | 70.8 | 110 | 71.0 | 110 | 73.0 |
| p(t-test) |  | 0.98 |  | 1.00 |  | 0.43 |
| Min | 0.888 | 7.04 | 0.888 | 7.04 | 0.888 | 18.2 |
| Max | 820 | 251 | 820 | 251 | 820 | 251 |
| n (Samp) | 109 | 14 | 109 | 14 | 109 | 9 |
| n (Patient) | 109 | 14 | 109 | 14 | 109 | 9 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 41.6 | 95.8 | 41.6 | 95.8 | 41.6 | 116 |
| Average | 77.3 | 96.1 | 77.3 | 96.1 | 77.3 | 111 |
| Stdev | 99.3 | 88.6 | 99.3 | 88.6 | 99.3 | 87.2 |
| p(t-test) |  | 0.62 |  | 0.62 |  | 0.41 |
| Min | 0.888 | 7.99 | 0.888 | 7.99 | 0.888 | 18.2 |
| Max | 820 | 251 | 820 | 251 | 820 | 251 |
| n (Samp) | 213 | 7 | 213 | 7 | 213 | 6 |
| n (Patient) | 213 | 7 | 213 | 7 | 213 | 6 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 40.8 | 45.1 | 40.8 | 45.1 | nd | nd |
| Average | 82.5 | 58.7 | 82.5 | 57.5 | nd | nd |
| Stdev | 117 | 49.5 | 117 | 49.6 | nd | nd |
| p(t-test) |  | 0.55 |  | 0.53 | nd | nd |
| Min | 0.888 | 7.04 | 0.888 | 7.04 | nd | nd |
| Max | 820 | 137 | 820 | 137 | nd | nd |
| n (Samp) | 106 | 9 | 106 | 9 | nd | nd |
| n (Patient) | 106 | 9 | 106 | 9 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.55 | 0.58 | 0.51 | 0.54 | 0.58 | 0.50 | 0.69 | 0.66 | nd |
| SE | 0.084 | 0.11 | 0.10 | 0.084 | 0.11 | 0.10 | 0.10 | 0.12 | nd |
| p | 0.55 | 0.49 | 0.93 | 0.59 | 0.49 | 0.98 | 0.057 | 0.20 | nd |
| nCohort 1 | 109 | 213 | 106 | 109 | 213 | 106 | 109 | 213 | nd |
| nCohort 2 | 14 | 7 | 9 | 14 | 7 | 9 | 9 | 6 | nd |
| Cutoff 1 | 20.5 | 20.5 | 18.1 | 20.5 | 20.5 | 18.1 | 43.5 | 20.5 | nd |
| Sens 1 | 71% | 71% | 78% | 71% | 71% | 78% | 78% | 83% | nd |
| Spec 1 | 29% | 30% | 28% | 29% | 30% | 28% | 60% | 30% | nd |
| Cutoff 2 | 15.0 | 18.2 | 15.0 | 15.0 | 18.2 | 15.0 | 20.5 | 20.5 | nd |
| Sens 2 | 86% | 86% | 89% | 86% | 86% | 89% | 89% | 83% | nd |
| Spec 2 | 24% | 29% | 25% | 24% | 29% | 25% | 29% | 30% | nd |
| Cutoff 3 | 7.37 | 7.95 | 6.56 | 7.37 | 7.95 | 6.56 | 18.1 | 18.2 | nd |
| Sens 3 | 93% | 100% | 100% | 93% | 100% | 100% | 100% | 100% | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 10% | 11% | 10% | 10% | 11% | 10% | 28% | 29% | nd |
| Cutoff 4 | 61.5 | 89.4 | 80.9 | 61.5 | 89.4 | 80.9 | 61.5 | 89.4 | nd |
| Sens 4 | 43% | 57% | 33% | 43% | 57% | 33% | 67% | 67% | nd |
| Spec 4 | 71% | 70% | 71% | 71% | 70% | 71% | 71% | 70% | nd |
| Cutoff 5 | 106 | 118 | 116 | 106 | 118 | 116 | 106 | 118 | nd |
| Sens 5 | 29% | 43% | 22% | 29% | 43% | 22% | 44% | 50% | nd |
| Spec 5 | 81% | 80% | 80% | 81% | 80% | 80% | 81% | 80% | nd |
| Cutoff 6 | 204 | 195 | 215 | 204 | 195 | 215 | 204 | 195 | nd |
| Sens 6 | 7% | 14% | 0% | 7% | 14% | 0% | 11% | 17% | nd |
| Spec 6 | 91% | 90% | 91% | 91% | 90% | 91% | 91% | 90% | nd |
| OR Quart 2 | 0.96 | 2.0 | 3.1 | 0.96 | 2.0 | 3.1 | >2.1 | >2.0 | nd |
| p Value | 0.97 | 0.57 | 0.34 | 0.97 | 0.57 | 0.34 | <0.56 | <0.57 | nd |
| 95% CI of OR Quart2 | 0.18 5.2 | 0.18 23 | 0.30 32 | 0.18 5.2 | 0.18 23 | 0.30 32 | >0.18 na | >0.18 na | nd nd |
| OR Quart 3 | 0.96 | 1.0 | 3.1 | 0.96 | 1.0 | 3.1 | >2.1 | >1.0 | nd |
| p Value | 0.97 | 1.0 | 0.34 | 0.97 | 1.0 | 0.34 | <0.54 | <1.0 | nd |
| 95% CI of OR Quart3 | 0.18 5.2 | 0.061 16 | 0.30 32 | 0.18 5.2 | 0.061 16 | 0.30 32 | >0.18 na | >0.061 na | nd nd |
| OR Quart 4 | 1.7 | 3.1 | 2.0 | 1.7 | 3.1 | 2.0 | >5.8 | >3.1 | nd |
| p Value | 0.48 | 0.33 | 0.58 | 0.48 | 0.33 | 0.58 | <0.12 | <0.33 | nd |
| 95% CI of OR Quart4 | 0.38 8.0 | 0.31 31 | 0.17 23 | 0.38 8.0 | 0.31 31 | 0.17 23 | >0.63 na | >0.31 na | nd nd |

**Peptide YY EDTA**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 44.5 | 98.1 | 44.5 | 98.1 | 44.5 | 89.2 |
| Average | 67.9 | 139 | 67.9 | 135 | 67.9 | 125 |
| Stdev | 97.9 | 113 | 97.9 | 108 | 97.9 | 132 |
| p(t-test) | | 0.013 | | 0.018 | | 0.10 |
| Min | 0.0512 | 21.6 | 0.0512 | 21.6 | 0.0512 | 21.6 |
| Max | 789 | 427 | 789 | 427 | 789 | 427 |
| n (Samp) | 109 | 14 | 109 | 14 | 109 | 9 |
| n (Patient) | 109 | 14 | 109 | 14 | 109 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 48.2 | 78.3 | 48.2 | 78.3 | 48.2 | 68.6 |
| Average | 72.8 | 83.4 | 72.8 | 83.4 | 72.8 | 84.3 |
| Stdev | 87.4 | 65.3 | 87.4 | 65.3 | 87.4 | 71.5 |
| p(t-test) | | 0.75 | | 0.75 | | 0.75 |
| Min | 0.0143 | 21.6 | 0.0143 | 21.6 | 0.0143 | 21.6 |
| Max | 789 | 216 | 789 | 216 | 789 | 216 |
| n (Samp) | 213 | 7 | 213 | 7 | 213 | 6 |
| n (Patient) | 213 | 7 | 213 | 7 | 213 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 48.3 | 172 | 48.3 | 172 | nd | nd |
| Average | 69.8 | 187 | 69.8 | 181 | nd | nd |
| Stdev | 95.4 | 114 | 95.4 | 110 | nd | nd |
| p(t-test) | | 6.9E-4 | | 0.0012 | | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.0512 | 44.4 | 0.0512 | 44.4 | nd | nd |
| Max | 789 | 427 | 789 | 427 | nd | nd |
| n (Samp) | 106 | 9 | 106 | 9 | nd | nd |
| n (Patient) | 106 | 9 | 106 | 9 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.75 | 0.59 | 0.88 | 0.75 | 0.59 | 0.87 | 0.65 | 0.57 | nd |
| SE | 0.078 | 0.11 | 0.076 | 0.078 | 0.11 | 0.077 | 0.10 | 0.12 | nd |
| p | 0.0013 | 0.43 | 8.8E-7 | 0.0013 | 0.43 | 1.0E-6 | 0.16 | 0.56 | nd |
| nCohort 1 | 109 | 213 | 106 | 109 | 213 | 106 | 109 | 213 | nd |
| nCohort 2 | 14 | 7 | 9 | 14 | 7 | 9 | 9 | 6 | nd |
| Cutoff 1 | 76.2 | 48.0 | 98.5 | 76.2 | 48.0 | 98.5 | 29.6 | 30.9 | nd |
| Sens 1 | 71% | 71% | 78% | 71% | 71% | 78% | 78% | 83% | nd |
| Spec 1 | 75% | 49% | 83% | 75% | 49% | 83% | 29% | 25% | nd |
| Cutoff 2 | 44.1 | 30.9 | 93.7 | 44.1 | 30.9 | 93.7 | 22.5 | 30.9 | nd |
| Sens 2 | 86% | 86% | 89% | 86% | 86% | 89% | 89% | 83% | nd |
| Spec 2 | 50% | 25% | 82% | 50% | 25% | 82% | 23% | 25% | nd |
| Cutoff 3 | 29.6 | 21.5 | 41.0 | 29.6 | 21.5 | 41.0 | 21.5 | 21.5 | nd |
| Sens 3 | 93% | 100% | 100% | 93% | 100% | 100% | 100% | 100% | nd |
| Spec 3 | 29% | 15% | 45% | 29% | 15% | 45% | 20% | 15% | nd |
| Cutoff 4 | 65.8 | 78.8 | 70.6 | 65.8 | 78.8 | 70.6 | 65.8 | 78.8 | nd |
| Sens 4 | 71% | 43% | 89% | 71% | 43% | 89% | 56% | 50% | nd |
| Spec 4 | 71% | 70% | 71% | 71% | 70% | 71% | 71% | 70% | nd |
| Cutoff 5 | 88.7 | 98.5 | 90.2 | 88.7 | 98.5 | 90.2 | 88.7 | 98.5 | nd |
| Sens 5 | 64% | 29% | 89% | 64% | 29% | 89% | 56% | 33% | nd |
| Spec 5 | 81% | 80% | 80% | 81% | 80% | 80% | 81% | 80% | nd |
| Cutoff 6 | 113 | 128 | 114 | 113 | 128 | 114 | 113 | 128 | nd |
| Sens 6 | 43% | 14% | 67% | 43% | 14% | 67% | 33% | 17% | nd |
| Spec 6 | 91% | 90% | 91% | 91% | 90% | 91% | 91% | 90% | nd |
| OR Quart 2 | 2.0 | 2.0 | >1.0 | 2.0 | 2.0 | >1.0 | 0.47 | 2.0 | nd |
| p Value | 0.58 | 0.57 | <1.0 | 0.58 | 0.57 | <1.0 | 0.54 | 0.58 | nd |
| 95% CI of OR Quart2 | 0.17 23 | 0.18 23 | >0.060 na | 0.17 23 | 0.18 23 | >0.060 na | 0.040 5.4 | 0.18 23 | nd |
| OR Quart 3 | 2.0 | 1.0 | >0 | 2.0 | 1.0 | >0 | 0.48 | 0 | nd |
| p Value | 0.58 | 1.0 | <na | 0.58 | 1.0 | <na | 0.56 | na | nd |
| 95% CI of OR Quart3 | 0.17 23 | 0.061 16 | >na na | 0.17 23 | 0.061 16 | >na na | 0.041 5.6 | na na | nd |
| OR Quart 4 | 12 | 3.1 | >11 | 12 | 3.1 | >11 | 2.7 | 3.1 | nd |
| p Value | 0.023 | 0.33 | <0.031 | 0.023 | 0.33 | <0.031 | 0.26 | 0.34 | nd |
| 95% CI of OR Quart4 | 1.4 100 | 0.31 31 | >1.2 na | 1.4 100 | 0.31 31 | >1.2 na | 0.48 15 | 0.31 30 | nd |

Table 9: Comparison of marker levels in urine samples collected from Cohort 1 (patients that did not progress beyond RIFLE stage 0, R, or I) and in urine samples collected from Cohort 2 (subjects who progress to RIFLE stage F) at 0, 24 hours, and 48 hours prior to the subject reaching RIFLE stage I.

**Agouti-related protein**

| sCr or UO | 0hr prior to AKI stage | 24hr prior to AKI stage | 48hr prior to AKI stage |
|---|---|---|---|

|  | Cohort 1 | Cohort 2 | Cohort 1 | Cohort 2 | Cohort 1 | Cohort 2 |
|---|---|---|---|---|---|---|
| Median | 0.839 | 1.61 | 0.839 | 1.48 | 0.839 | 0.686 |
| Average | 1.38 | 2.09 | 1.38 | 1.67 | 1.38 | 0.831 |
| Stdev | 1.92 | 1.69 | 1.92 | 1.46 | 1.92 | 0.802 |
| p(t-test) |  | 0.18 |  | 0.53 |  | 0.42 |
| Min | 0.00198 | 0.183 | 0.00198 | 0.213 | 0.00198 | 0.00498 |
| Max | 20.0 | 6.53 | 20.0 | 6.12 | 20.0 | 2.33 |
| n (Samp) | 807 | 13 | 807 | 18 | 807 | 8 |
| n (Patient) | 298 | 13 | 298 | 18 | 298 | 8 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 0.847 | 0.978 | nd | nd |
| Average | nd | nd | 1.38 | 1.70 | nd | nd |
| Stdev | nd | nd | 1.90 | 1.91 | nd | nd |
| p(t-test) | nd | nd |  | 0.61 | nd | nd |
| Min | nd | nd | 0.00198 | 0.213 | nd | nd |
| Max | nd | nd | 20.0 | 6.53 | nd | nd |
| n (Samp) | nd | nd | 833 | 9 | nd | nd |
| n (Patient) | nd | nd | 309 | 9 | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.871 | 0.867 | 0.871 | 1.44 | nd | nd |
| Average | 1.39 | 1.29 | 1.39 | 1.65 | nd | nd |
| Stdev | 1.89 | 1.12 | 1.89 | 1.62 | nd | nd |
| p(t-test) |  | 0.89 |  | 0.62 | nd | nd |
| Min | 0.00198 | 0.183 | 0.00198 | 0.126 | nd | nd |
| Max | 20.0 | 3.44 | 20.0 | 6.12 | nd | nd |
| n (Samp) | 833 | 7 | 833 | 13 | nd | nd |
| n (Patient) | 292 | 7 | 292 | 13 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.68 | nd | 0.54 | 0.61 | 0.59 | 0.58 | 0.40 | nd | nd |
| SE | 0.083 | nd | 0.11 | 0.071 | 0.10 | 0.084 | 0.11 | nd | nd |
| p | 0.025 | nd | 0.75 | 0.12 | 0.39 | 0.33 | 0.33 | nd | nd |
| nCohort 1 | 807 | nd | 833 | 807 | 833 | 833 | 807 | nd | nd |
| nCohort 2 | 13 | nd | 7 | 18 | 9 | 13 | 8 | nd | nd |
| Cutoff 1 | 0.867 | nd | 0.767 | 0.761 | 0.770 | 0.701 | 0.218 | nd | nd |
| Sens 1 | 77% | nd | 71% | 72% | 78% | 77% | 75% | nd | nd |
| Spec 1 | 51% | nd | 45% | 46% | 46% | 41% | 15% | nd | nd |
| Cutoff 2 | 0.786 | nd | 0.638 | 0.600 | 0.600 | 0.488 | 0.140 | nd | nd |
| Sens 2 | 85% | nd | 86% | 83% | 89% | 85% | 88% | nd | nd |
| Spec 2 | 47% | nd | 39% | 38% | 37% | 31% | 9% | nd | nd |
| Cutoff 3 | 0.767 | nd | 0.182 | 0.366 | 0.211 | 0.366 | 0.00318 | nd | nd |
| Sens 3 | 92% | nd | 100% | 94% | 100% | 92% | 100% | nd | nd |
| Spec 3 | 46% | nd | 12% | 25% | 15% | 24% | 0% | nd | nd |
| Cutoff 4 | 1.48 | nd | 1.54 | 1.48 | 1.50 | 1.54 | 1.48 | nd | nd |
| Sens 4 | 54% | nd | 29% | 50% | 44% | 38% | 25% | nd | nd |
| Spec 4 | 70% | nd | 70% | 70% | 70% | 70% | 70% | nd | nd |
| Cutoff 5 | 2.05 | nd | 2.10 | 2.05 | 2.05 | 2.10 | 2.05 | nd | nd |
| Sens 5 | 46% | nd | 29% | 22% | 22% | 15% | 12% | nd | nd |
| Spec 5 | 80% | nd | 80% | 80% | 80% | 80% | 80% | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 6 | 2.99 | nd | 2.99 | 2.99 | 2.95 | 2.99 | 2.99 | nd | nd |
| Sens 6 | 23% | nd | 14% | 17% | 11% | 15% | 0% | nd | nd |
| Spec 6 | 90% | nd | 90% | 90% | 90% | 90% | 90% | nd | nd |
| OR Quart 2 | 2.0 | nd | 3.0 | 3.1 | 3.0 | 2.0 | 3.0 | nd | nd |
| p Value | 0.57 | nd | 0.34 | 0.17 | 0.34 | 0.42 | 0.34 | nd | nd |
| 95% CI of OR Quart2 | 0.18 | nd | 0.31 | 0.61 | 0.31 | 0.36 | 0.31 | nd | nd |
| | 22 | nd | 29 | 15 | 29 | 11 | 29 | nd | nd |
| OR Quart 3 | 4.1 | nd | 1.0 | 1.5 | 3.0 | 1.0 | 1.0 | nd | nd |
| p Value | 0.21 | nd | 1.0 | 0.65 | 0.34 | 1.0 | 1.0 | nd | nd |
| 95% CI of OR Quart3 | 0.45 | nd | 0.062 | 0.25 | 0.31 | 0.14 | 0.062 | nd | nd |
| | 37 | nd | 16 | 9.1 | 29 | 7.2 | 16 | nd | nd |
| OR Quart 4 | 6.2 | nd | 2.0 | 3.6 | 2.0 | 2.5 | 3.0 | nd | nd |
| p Value | 0.094 | nd | 0.57 | 0.12 | 0.57 | 0.27 | 0.34 | nd | nd |
| 95% CI of OR Quart4 | 0.73 | nd | 0.18 | 0.73 | 0.18 | 0.48 | 0.31 | nd | nd |
| | 52 | nd | 22 | 17 | 22 | 13 | 30 | nd | nd |

**Complement factor H**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.38 | 6.86 | 9.38 | 5.31 | 9.38 | 8.96 |
| Average | 18.5 | 11.7 | 18.5 | 19.3 | 18.5 | 15.4 |
| Stdev | 37.6 | 15.9 | 37.6 | 35.9 | 37.6 | 18.0 |
| p(t-test) | | 0.46 | | 0.92 | | 0.78 |
| Min | 0.0620 | 2.25 | 0.0620 | 1.61 | 0.0620 | 0.121 |
| Max | 772 | 70.6 | 772 | 142 | 772 | 59.2 |
| n (Samp) | 892 | 17 | 892 | 20 | 892 | 11 |
| n (Patient) | 370 | 17 | 370 | 20 | 370 | 11 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 9.32 | 20.2 | 9.32 | 13.3 |
| Average | nd | nd | 18.3 | 38.3 | 18.3 | 20.0 |
| Stdev | nd | nd | 37.2 | 48.1 | 37.2 | 19.2 |
| p(t-test) | nd | nd | | 0.11 | | 0.90 |
| Min | nd | nd | 0.0620 | 4.62 | 0.0620 | 2.52 |
| Max | nd | nd | 772 | 142 | 772 | 59.2 |
| n (Samp) | nd | nd | 922 | 9 | 922 | 8 |
| n (Patient) | nd | nd | 382 | 9 | 382 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.32 | 6.55 | 9.32 | 6.01 | nd | nd |
| Average | 18.1 | 15.4 | 18.1 | 9.37 | nd | nd |
| Stdev | 36.6 | 29.4 | 36.6 | 10.0 | nd | nd |
| p(t-test) | | 0.81 | | 0.36 | nd | nd |
| Min | 0.0620 | 2.25 | 0.0620 | 1.61 | nd | nd |
| Max | 772 | 98.7 | 772 | 35.1 | nd | nd |
| n (Samp) | 886 | 10 | 886 | 15 | nd | nd |
| n (Patient) | 345 | 10 | 345 | 15 | nd | nd |

| | 0hr prior to AKI stage | 24hr prior to AKI stage | 48hr prior to AKI stage |
|---|---|---|---|

| | sCr or UO | sCr only | UO only | sCr or UO | sCr only | UO only | sCr or UO | sCr only | UO only |
|---|---|---|---|---|---|---|---|---|---|
| AUC | 0.42 | nd | 0.38 | 0.40 | 0.68 | 0.37 | 0.46 | 0.58 | nd |
| SE | 0.073 | nd | 0.095 | 0.068 | 0.099 | 0.078 | 0.090 | 0.11 | nd |
| p | 0.29 | nd | 0.21 | 0.15 | 0.077 | 0.084 | 0.63 | 0.48 | nd |
| nCohort 1 | 892 | nd | 886 | 892 | 922 | 886 | 892 | 922 | nd |
| nCohort 2 | 17 | nd | 10 | 20 | 9 | 15 | 11 | 8 | nd |
| Cutoff 1 | 6.04 | nd | 5.90 | 3.91 | 8.99 | 3.89 | 3.30 | 6.01 | nd |
| Sens 1 | 71% | nd | 70% | 70% | 78% | 73% | 73% | 75% | nd |
| Spec 1 | 31% | nd | 30% | 18% | 49% | 18% | 14% | 31% | nd |
| Cutoff 2 | 4.88 | nd | 4.76 | 3.28 | 6.57 | 3.28 | 2.83 | 5.33 | nd |
| Sens 2 | 82% | nd | 80% | 80% | 89% | 80% | 82% | 88% | nd |
| Spec 2 | 24% | nd | 23% | 14% | 36% | 14% | 11% | 27% | nd |
| Cutoff 3 | 2.60 | nd | 2.60 | 2.41 | 4.58 | 2.41 | 2.72 | 2.41 | nd |
| Sens 3 | 94% | nd | 90% | 90% | 100% | 93% | 91% | 100% | nd |
| Spec 3 | 10% | nd | 10% | 10% | 23% | 9% | 11% | 10% | nd |
| Cutoff 4 | 15.6 | nd | 15.4 | 15.6 | 15.4 | 15.4 | 15.6 | 15.4 | nd |
| Sens 4 | 12% | nd | 10% | 25% | 56% | 20% | 36% | 50% | nd |
| Spec 4 | 70% | nd | 70% | 70% | 70% | 70% | 70% | 70% | nd |
| Cutoff 5 | 22.2 | nd | 22.0 | 22.2 | 22.2 | 22.0 | 22.2 | 22.2 | nd |
| Sens 5 | 12% | nd | 10% | 15% | 33% | 13% | 27% | 38% | nd |
| Spec 5 | 80% | nd | 80% | 80% | 80% | 80% | 80% | 80% | nd |
| Cutoff 6 | 38.7 | nd | 37.8 | 38.7 | 37.8 | 37.8 | 38.7 | 37.8 | nd |
| Sens 6 | 6% | nd | 10% | 10% | 22% | 0% | 9% | 12% | nd |
| Spec 6 | 90% | nd | 90% | 90% | 90% | 90% | 90% | 90% | nd |
| OR Quart 2 | 1.0 | nd | 1.0 | 0.50 | 2.0 | 1.0 | 0.66 | 2.0 | nd |
| p Value | 1.00 | nd | 1.0 | 0.42 | 0.57 | 1.00 | 0.66 | 0.57 | nd |
| 95% CI of | 0.14 | nd | 0.062 | 0.090 | 0.18 | 0.14 | 0.11 | 0.18 | nd |
| OR Quart2 | 7.2 | nd | 16 | 2.7 | 22 | 7.2 | 4.0 | 22 | nd |
| OR Quart 3 | 4.7 | nd | 5.1 | 1.0 | 1.00 | 2.0 | 0.66 | 2.0 | nd |
| p Value | 0.050 | nd | 0.14 | 1.0 | 1.00 | 0.42 | 0.66 | 0.57 | nd |
| 95% CI of | 1.00 | nd | 0.59 | 0.25 | 0.062 | 0.37 | 0.11 | 0.18 | nd |
| OR Quart3 | 22 | nd | 44 | 4.0 | 16 | 11 | 4.0 | 22 | nd |
| OR Quart 4 | 2.0 | nd | 3.0 | 2.6 | 5.1 | 3.6 | 1.3 | 3.0 | nd |
| p Value | 0.42 | nd | 0.34 | 0.12 | 0.14 | 0.11 | 0.70 | 0.34 | nd |
| 95% CI of | 0.37 | nd | 0.31 | 0.79 | 0.59 | 0.74 | 0.30 | 0.31 | nd |
| OR Quart4 | 11 | nd | 29 | 8.3 | 44 | 18 | 6.1 | 29 | nd |

**Ciliary neurotrophic factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0325 | 0.000166 | 0.0325 | 0.267 | 0.0325 | 0.102 |
| Average | 0.216 | 0.156 | 0.216 | 0.233 | 0.216 | 0.143 |
| Stdev | 0.679 | 0.215 | 0.679 | 0.218 | 0.679 | 0.158 |
| p(t-test) | | 0.75 | | 0.92 | | 0.76 |
| Min | 0.000103 | 0.000103 | 0.000103 | 0.000119 | 0.000103 | 0.000119 |
| Max | 14.0 | 0.657 | 14.0 | 0.778 | 14.0 | 0.397 |
| n (Samp) | 807 | 13 | 807 | 18 | 807 | 8 |
| n (Patient) | 298 | 13 | 298 | 18 | 298 | 8 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.0325 | 0.0194 | nd | nd |
| Average | nd | nd | 0.218 | 0.152 | nd | nd |
| Stdev | nd | nd | 0.674 | 0.195 | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | nd | nd | | 0.77 | nd | nd |
| Min | nd | nd | 0.000103 | 0.000123 | nd | nd |
| Max | nd | nd | 14.0 | 0.521 | nd | nd |
| n (Samp) | nd | nd | 833 | 9 | nd | nd |
| n (Patient) | nd | nd | 309 | 9 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0325 | 0.0175 | 0.0325 | 0.290 | nd | nd |
| Average | 0.209 | 0.176 | 0.209 | 0.250 | nd | nd |
| Stdev | 0.671 | 0.264 | 0.671 | 0.226 | nd | nd |
| p(t-test) | | 0.90 | | 0.83 | nd | nd |
| Min | 0.000103 | 0.000103 | 0.000103 | 0.000119 | nd | nd |
| Max | 14.0 | 0.657 | 14.0 | 0.778 | nd | nd |
| n (Samp) | 833 | 7 | 833 | 13 | nd | nd |
| n (Patient) | 292 | 7 | 292 | 13 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | nd | 0.51 | 0.63 | 0.55 | 0.66 | 0.52 | nd | nd |
| SE | 0.081 | nd | 0.11 | 0.071 | 0.099 | 0.083 | 0.10 | nd | nd |
| p | 1.00 | nd | 0.92 | 0.065 | 0.63 | 0.056 | 0.82 | nd | nd |
| nCohort 1 | 807 | nd | 833 | 807 | 833 | 833 | 807 | nd | nd |
| nCohort 2 | 13 | nd | 7 | 18 | 9 | 13 | 8 | nd | nd |
| Cutoff 1 | 0.000123 | nd | 0.000161 | 0.0175 | 0.000166 | 0.0175 | 0.000121 | nd | nd |
| Sens 1 | 85% | nd | 71% | 78% | 78% | 85% | 88% | nd | nd |
| Spec 1 | 27% | nd | 31% | 44% | 36% | 45% | 22% | nd | nd |
| Cutoff 2 | 0.000123 | nd | 0.000123 | 0.000123 | 0.000123 | 0.0175 | 0.000121 | nd | nd |
| Sens 2 | 85% | nd | 86% | 83% | 89% | 85% | 88% | nd | nd |
| Spec 2 | 27% | nd | 27% | 27% | 27% | 45% | 22% | nd | nd |
| Cutoff 3 | 0.000121 | nd | 0 | 0.000121 | 0.000121 | 0.000119 | 0.000118 | nd | nd |
| Sens 3 | 92% | nd | 100% | 94% | 100% | 92% | 100% | nd | nd |
| Spec 3 | 22% | nd | 0% | 22% | 22% | 18% | 11% | nd | nd |
| Cutoff 4 | 0.229 | nd | 0.191 | 0.229 | 0.229 | 0.191 | 0.229 | nd | nd |
| Sens 4 | 31% | nd | 29% | 56% | 33% | 62% | 25% | nd | nd |
| Spec 4 | 72% | nd | 72% | 72% | 72% | 72% | 72% | nd | nd |
| Cutoff 5 | 0.327 | nd | 0.290 | 0.327 | 0.327 | 0.290 | 0.327 | nd | nd |
| Sens 5 | 23% | nd | 29% | 28% | 22% | 46% | 25% | nd | nd |
| Spec 5 | 81% | nd | 80% | 81% | 81% | 80% | 81% | nd | nd |
| Cutoff 6 | 0.459 | nd | 0.434 | 0.459 | 0.459 | 0.434 | 0.459 | nd | nd |
| Sens 6 | 8% | nd | 14% | 11% | 11% | 8% | 0% | nd | nd |
| Spec 6 | 91% | nd | 90% | 91% | 91% | 90% | 91% | nd | nd |
| OR Quart 2 | 2.5 | nd | 3.0 | 1.0 | >5.1 | 1.00 | 0.50 | nd | nd |
| p Value | 0.27 | nd | 0.34 | 1.0 | <0.14 | 1.00 | 0.57 | nd | nd |
| 95% CI of | 0.49 | nd | 0.31 | 0.20 | >0.59 | 0.14 | 0.045 | nd | nd |
| OR Quart2 | 13 | nd | 29 | 5.0 | na | 7.1 | 5.5 | nd | nd |
| OR Quart 3 | 1.0 | nd | 1.0 | 1.0 | >1.0 | 0.50 | 1.5 | nd | nd |
| p Value | 1.0 | nd | 1.0 | 1.0 | <1.00 | 0.57 | 0.66 | nd | nd |
| 95% CI of | 0.14 | nd | 0.062 | 0.20 | >0.062 | 0.045 | 0.25 | nd | nd |
| OR Quart3 | 7.2 | nd | 16 | 5.0 | na | 5.5 | 9.1 | nd | nd |
| OR Quart 4 | 2.0 | nd | 2.0 | 3.1 | >3.0 | 4.1 | 1.00 | nd | nd |
| p Value | 0.42 | nd | 0.57 | 0.096 | <0.34 | 0.077 | 1.00 | nd | nd |
| 95% CI of | 0.37 | nd | 0.18 | 0.82 | >0.31 | 0.86 | 0.14 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart4 | 11 | nd | 22 | 12 | na | 20 | 7.1 | nd | nd |

**Follistatin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 7.39 | 15.8 | 7.39 | 14.8 | 7.39 | 12.0 |
| Average | 26.7 | 23.8 | 26.7 | 56.9 | 26.7 | 21.2 |
| Stdev | 225 | 20.6 | 225 | 169 | 225 | 28.8 |
| p(t-test) |  | 0.95 |  | 0.51 |  | 0.93 |
| Min | 0.000347 | 2.82 | 0.000347 | 0.0412 | 0.000347 | 0.0767 |
| Max | 8290 | 63.0 | 8290 | 840 | 8290 | 98.2 |
| n (Samp) | 1487 | 23 | 1487 | 24 | 1487 | 14 |
| n (Patient) | 518 | 23 | 518 | 24 | 518 | 14 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 7.79 | 15.8 | 7.79 | 11.1 | 7.79 | 26.6 |
| Average | 27.9 | 32.4 | 27.9 | 12.7 | 27.9 | 30.0 |
| Stdev | 223 | 39.1 | 223 | 8.34 | 223 | 30.9 |
| p(t-test) |  | 0.95 |  | 0.83 |  | 0.98 |
| Min | 0.000347 | 4.14 | 0.000347 | 0.299 | 0.000347 | 1.26 |
| Max | 8290 | 125 | 8290 | 28.6 | 8290 | 98.2 |
| n (Samp) | 1540 | 9 | 1540 | 10 | 1540 | 9 |
| n (Patient) | 534 | 9 | 534 | 10 | 534 | 9 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 7.38 | 18.9 | 7.38 | 21.8 | 7.38 | 20.9 |
| Average | 27.0 | 166 | 27.0 | 129 | 27.0 | 22.0 |
| Stdev | 232 | 532 | 232 | 322 | 232 | 24.0 |
| p(t-test) |  | 0.029 |  | 0.048 |  | 0.96 |
| Min | 0.000347 | 2.66 | 0.000347 | 0.0412 | 0.000347 | 0.0767 |
| Max | 8290 | 2010 | 8290 | 1300 | 8290 | 66.2 |
| n (Samp) | 1391 | 14 | 1391 | 21 | 1391 | 6 |
| n (Patient) | 444 | 14 | 444 | 21 | 444 | 6 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.68 | 0.70 | 0.69 | 0.64 | 0.58 | 0.75 | 0.50 | 0.65 | 0.58 |
| SE | 0.062 | 0.098 | 0.079 | 0.062 | 0.095 | 0.062 | 0.078 | 0.10 | 0.12 |
| p | 0.0044 | 0.042 | 0.017 | 0.019 | 0.39 | 4.2E-5 | 0.97 | 0.14 | 0.53 |
| nCohort 1 | 1487 | 1540 | 1391 | 1487 | 1540 | 1391 | 1487 | 1540 | 1391 |
| nCohort 2 | 23 | 9 | 14 | 24 | 10 | 21 | 14 | 9 | 6 |
| Cutoff 1 | 6.24 | 12.0 | 9.70 | 9.60 | 9.60 | 12.2 | 1.26 | 3.83 | 1.02 |
| Sens 1 | 74% | 78% | 71% | 71% | 70% | 71% | 71% | 78% | 83% |
| Spec 1 | 44% | 66% | 60% | 59% | 58% | 67% | 11% | 29% | 9% |
| Cutoff 2 | 5.79 | 5.45 | 5.79 | 4.95 | 9.36 | 9.60 | 0.820 | 3.36 | 1.02 |
| Sens 2 | 83% | 89% | 86% | 83% | 80% | 81% | 86% | 89% | 83% |
| Spec 2 | 42% | 39% | 42% | 37% | 57% | 59% | 9% | 26% | 9% |
| Cutoff 3 | 4.75 | 4.11 | 2.82 | 1.70 | 3.98 | 8.92 | 0.301 | 1.26 | 0.0615 |
| Sens 3 | 91% | 100% | 93% | 92% | 90% | 90% | 93% | 100% | 100% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 36% | 31% | 23% | 14% | 30% | 57% | 6% | 10% | 4% |
| Cutoff 4 | 13.6 | 14.4 | 13.7 | 13.6 | 14.4 | 13.7 | 13.6 | 14.4 | 13.7 |
| Sens 4 | 57% | 67% | 57% | 54% | 40% | 67% | 50% | 67% | 67% |
| Spec 4 | 70% | 70% | 70% | 70% | 70% | 70% | 70% | 70% | 70% |
| Cutoff 5 | 21.8 | 23.1 | 21.7 | 21.8 | 23.1 | 21.7 | 21.8 | 23.1 | 21.7 |
| Sens 5 | 43% | 33% | 50% | 38% | 10% | 52% | 36% | 56% | 33% |
| Spec 5 | 80% | 80% | 80% | 80% | 80% | 80% | 80% | 80% | 80% |
| Cutoff 6 | 42.7 | 45.0 | 42.3 | 42.7 | 45.0 | 42.3 | 42.7 | 45.0 | 42.3 |
| Sens 6 | 22% | 22% | 29% | 25% | 0% | 38% | 14% | 22% | 17% |
| Spec 6 | 90% | 90% | 90% | 90% | 90% | 90% | 90% | 90% | 90% |
| OR Quart 2 | 6.1 | >2.0 | 1.0 | 1.00 | 1.00 | 1.0 | 0 | 2.0 | 0 |
| p Value | 0.096 | <0.57 | 1.0 | 1.00 | 1.00 | 1.0 | na | 0.57 | na |
| 95% CI of OR Quart2 | 0.73 / 51 | >0.18 / na | 0.14 / 7.1 | 0.20 / 5.0 | 0.062 / 16 | 0.062 / 16 | na | 0.18 / 22 | na |
| OR Quart 3 | 6.1 | >4.0 | 1.5 | 3.0 | 6.1 | 8.2 | 0.28 | 0 | 0 |
| p Value | 0.095 | <0.21 | 0.66 | 0.097 | 0.095 | 0.048 | 0.12 | na | na |
| 95% CI of OR Quart3 | 0.73 / 51 | >0.45 / na | 0.25 / 9.1 | 0.82 / 11 | 0.73 / 51 | 1.0 / 66 | 0.058 / 1.4 | na | na |
| OR Quart 4 | 10 | >3.0 | 3.5 | 3.0 | 2.0 | 11 | 0.71 | 6.1 | 2.0 |
| p Value | 0.027 | <0.34 | 0.12 | 0.097 | 0.57 | 0.020 | 0.57 | 0.096 | 0.42 |
| 95% CI of OR Quart4 | 1.3 / 80 | >0.31 / na | 0.73 / 17 | 0.82 / 11 | 0.18 / 22 | 1.5 / 88 | 0.22 / 2.3 | 0.73 / 51 | 0.36 / 11 |

**Vitamin D-binding protein**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 220 | 2710 | 220 | 968 | 220 | 10.2 |
| Average | 7580 | 54300 | 7580 | 2190 | 7580 | 49500 |
| Stdev | 34700 | 99900 | 34700 | 3120 | 34700 | 131000 |
| p(t-test) | | 2.8E-5 | | 0.52 | | 0.0027 |
| Min | 0.259 | 5.93 | 0.259 | 52.6 | 0.259 | 0.337 |
| Max | 379000 | 283000 | 379000 | 11500 | 379000 | 347000 |
| n (Samp) | 684 | 11 | 684 | 17 | 684 | 7 |
| n (Patient) | 274 | 11 | 274 | 17 | 274 | 7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 224 | 567 | nd | nd |
| Average | nd | nd | 7740 | 2250 | nd | nd |
| Stdev | nd | nd | 35100 | 3790 | nd | nd |
| p(t-test) | nd | nd | | 0.64 | nd | nd |
| Min | nd | nd | 0.259 | 79.9 | nd | nd |
| Max | nd | nd | 379000 | 11000 | nd | nd |
| n (Samp) | nd | nd | 705 | 9 | nd | nd |
| n (Patient) | nd | nd | 284 | 9 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 218 | 1530 | 218 | 1330 | nd | nd |
| Average | 8340 | 84500 | 8340 | 2940 | nd | nd |
| Stdev | 37200 | 131000 | 37200 | 3470 | nd | nd |
| p(t-test) | | 1.9E-6 | | 0.62 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.200 | 5.93 | 0.200 | 31.9 | nd | nd |
| Max | 379000 | 283000 | 379000 | 11500 | nd | nd |
| n (Samp) | 703 | 6 | 703 | 12 | nd | nd |
| n (Patient) | 267 | 6 | 267 | 12 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.75 | nd | 0.64 | 0.64 | 0.59 | 0.69 | 0.21 | nd | nd |
| SE | 0.086 | nd | 0.12 | 0.073 | 0.10 | 0.086 | 0.10 | nd | nd |
| p | 0.0036 | nd | 0.24 | 0.056 | 0.38 | 0.031 | 0.0040 | nd | nd |
| nCohort 1 | 684 | nd | 703 | 684 | 705 | 703 | 684 | nd | nd |
| nCohort 2 | 11 | nd | 6 | 17 | 9 | 12 | 7 | nd | nd |
| Cutoff 1 | 575 | nd | 143 | 190 | 112 | 467 | 0.892 | nd | nd |
| Sens 1 | 73% | nd | 83% | 71% | 78% | 75% | 71% | nd | nd |
| Spec 1 | 72% | nd | 39% | 47% | 33% | 69% | 1% | nd | nd |
| Cutoff 2 | 284 | nd | 143 | 112 | 94.9 | 190 | 0.611 | nd | nd |
| Sens 2 | 82% | nd | 83% | 82% | 89% | 83% | 86% | nd | nd |
| Spec 2 | 58% | nd | 39% | 33% | 29% | 47% | 1% | nd | nd |
| Cutoff 3 | 222 | nd | 5.82 | 79.5 | 79.5 | 80.8 | 0.333 | nd | nd |
| Sens 3 | 91% | nd | 100% | 94% | 100% | 92% | 100% | nd | nd |
| Spec 3 | 50% | nd | 4% | 26% | 26% | 26% | 0% | nd | nd |
| Cutoff 4 | 477 | nd | 510 | 477 | 498 | 510 | 477 | nd | nd |
| Sens 4 | 73% | nd | 50% | 59% | 56% | 67% | 14% | nd | nd |
| Spec 4 | 70% | nd | 70% | 70% | 70% | 70% | 70% | nd | nd |
| Cutoff 5 | 1310 | nd | 1500 | 1310 | 1490 | 1500 | 1310 | nd | nd |
| Sens 5 | 55% | nd | 50% | 35% | 22% | 42% | 14% | nd | nd |
| Spec 5 | 80% | nd | 80% | 80% | 80% | 80% | 80% | nd | nd |
| Cutoff 6 | 10300 | nd | 12300 | 10300 | 10400 | 12300 | 10300 | nd | nd |
| Sens 6 | 36% | nd | 33% | 6% | 11% | 0% | 14% | nd | nd |
| Spec 6 | 90% | nd | 90% | 90% | 90% | 90% | 90% | nd | nd |
| OR Quart 2 | 0.99 | nd | 1.0 | 5.1 | >4.1 | 2.0 | 0 | nd | nd |
| p Value | 1.00 | nd | 1.0 | 0.14 | <0.21 | 0.57 | na | nd | nd |
| 95% CI of OR Quart2 | 0.062 16 | nd | 0.062 16 | 0.59 44 | >0.45 na | 0.18 22 | na na | nd | nd |
| OR Quart 3 | 2.0 | nd | 1.0 | 2.0 | >1.0 | 0.99 | 1.0 | nd | nd |
| p Value | 0.57 | nd | 1.0 | 0.57 | <1.00 | 1.00 | 1.0 | nd | nd |
| 95% CI of OR Quart3 | 0.18 22 | nd | 0.062 16 | 0.18 22 | >0.062 na | 0.062 16 | 0.062 16 | nd | nd |
| OR Quart 4 | 7.2 | nd | 3.0 | 9.4 | >4.1 | 8.3 | 5.1 | nd | nd |
| p Value | 0.066 | nd | 0.34 | 0.035 | <0.21 | 0.047 | 0.14 | nd | nd |
| 95% CI of OR Quart4 | 0.88 59 | nd | 0.31 29 | 1.2 75 | >0.45 na | 1.0 67 | 0.60 45 | nd | nd |

**Glucagon**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 238 | 9330 | 238 | 20.5 | nd | nd |
| Average | 1840 | 5460 | 1840 | 1900 | nd | nd |
| Stdev | 3210 | 4830 | 3210 | 3920 | nd | nd |
| p(t-test) | | 0.0037 | | 0.95 | nd | nd |
| Min | 0.0247 | 174 | 0.0247 | 4.85 | nd | nd |
| Max | 9330 | 9330 | 9330 | 9330 | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 331 | 7 | 331 | 10 | nd | nd |
| n (Patient) | 191 | 7 | 191 | 10 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 238 | 27.4 | nd | nd |
| Average | nd | nd | 1660 | 2370 | nd | nd |
| Stdev | nd | nd | 3020 | 4300 | nd | nd |
| p(t-test) | nd | nd | | 0.51 | nd | nd |
| Min | nd | nd | 0.0247 | 4.85 | nd | nd |
| Max | nd | nd | 9330 | 9330 | nd | nd |
| n (Samp) | nd | nd | 289 | 8 | nd | nd |
| n (Patient) | nd | nd | 160 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.75 | nd | nd | 0.36 | nd | 0.39 | nd | nd | nd |
| SE | 0.11 | nd | nd | 0.096 | nd | 0.11 | nd | nd | nd |
| p | 0.018 | nd | nd | 0.15 | nd | 0.32 | nd | nd | nd |
| nCohort 1 | 331 | nd | nd | 331 | nd | 289 | nd | nd | nd |
| nCohort 2 | 7 | nd | nd | 10 | nd | 8 | nd | nd | nd |
| Cutoff 1 | 275 | nd | nd | 8.98 | nd | 8.64 | nd | nd | nd |
| Sens 1 | 86% | nd | nd | 70% | nd | 75% | nd | nd | nd |
| Spec 1 | 53% | nd | nd | 21% | nd | 20% | nd | nd | nd |
| Cutoff 2 | 275 | nd | nd | 8.64 | nd | 5.82 | nd | nd | nd |
| Sens 2 | 86% | nd | nd | 80% | nd | 88% | nd | nd | nd |
| Spec 2 | 53% | nd | nd | 20% | nd | 9% | nd | nd | nd |
| Cutoff 3 | 174 | nd | nd | 5.82 | nd | 4.46 | nd | nd | nd |
| Sens 3 | 100% | nd | nd | 90% | nd | 100% | nd | nd | nd |
| Spec 3 | 34% | nd | nd | 9% | nd | 6% | nd | nd | nd |
| Cutoff 4 | 347 | nd | nd | 347 | nd | 347 | nd | nd | nd |
| Sens 4 | 57% | nd | nd | 20% | nd | 25% | nd | nd | nd |
| Spec 4 | 70% | nd | nd | 70% | nd | 72% | nd | nd | nd |
| Cutoff 5 | 2420 | nd | nd | 2420 | nd | 2420 | nd | nd | nd |
| Sens 5 | 57% | nd | nd | 20% | nd | 25% | nd | nd | nd |
| Spec 5 | 85% | nd | nd | 85% | nd | 87% | nd | nd | nd |
| Cutoff 6 | 9330 | nd | nd | 9330 | nd | 9330 | nd | nd | nd |
| Sens 6 | 0% | nd | nd | 0% | nd | 0% | nd | nd | nd |
| Spec 6 | 100% | nd | nd | 100% | nd | 100% | nd | nd | nd |
| OR Quart 2 | >1.0 | nd | nd | 0 | nd | 0 | nd | nd | nd |
| p Value | <1.0 | nd | nd | na | nd | na | nd | nd | nd |
| 95% CI of OR Quart2 | >0.062 | nd | nd | na | nd | na | nd | nd | nd |
| | na | nd | nd | na | nd | na | nd | nd | nd |
| OR Quart 3 | >2.0 | nd | nd | 1.5 | nd | 1.0 | nd | nd | nd |
| p Value | <0.56 | nd | nd | 0.64 | nd | 0.99 | nd | nd | nd |
| 95% CI of OR Quart3 | >0.18 | nd | nd | 0.25 | nd | 0.14 | nd | nd | nd |
| | na | nd | nd | 9.4 | nd | 7.4 | nd | nd | nd |
| OR Quart 4 | >4.1 | nd | nd | 2.6 | nd | 2.1 | nd | nd | nd |
| p Value | <0.21 | nd | nd | 0.26 | nd | 0.40 | nd | nd | nd |
| 95% CI of OR Quart4 | >0.45 | nd | nd | 0.50 | nd | 0.37 | nd | nd | nd |
| | na | nd | nd | 14 | nd | 12 | nd | nd | nd |

**Glucagon-like peptide 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.10 | 5.53 | 3.10 | 5.53 | nd | nd |
| Average | 8.40 | 4.22 | 8.40 | 17.9 | nd | nd |
| Stdev | 40.0 | 3.67 | 40.0 | 32.1 | nd | nd |
| p(t-test) | | 0.78 | | 0.46 | nd | nd |
| Min | 0.407 | 0.407 | 0.407 | 0.685 | nd | nd |
| Max | 519 | 9.39 | 519 | 107 | nd | nd |
| n (Samp) | 331 | 7 | 331 | 10 | nd | nd |
| n (Patient) | 191 | 7 | 191 | 10 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 3.10 | 7.57 | nd | nd |
| Average | nd | nd | 8.83 | 21.3 | nd | nd |
| Stdev | nd | nd | 42.7 | 35.5 | nd | nd |
| p(t-test) | nd | nd | | 0.41 | nd | nd |
| Min | nd | nd | 0.407 | 0.685 | nd | nd |
| Max | nd | nd | 519 | 107 | nd | nd |
| n (Samp) | nd | nd | 289 | 8 | nd | nd |
| n (Patient) | nd | nd | 160 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.45 | nd | nd | 0.70 | nd | 0.74 | nd | nd | nd |
| SE | 0.11 | nd | nd | 0.094 | nd | 0.10 | nd | nd | nd |
| p | 0.67 | nd | nd | 0.038 | nd | 0.018 | nd | nd | nd |
| nCohort 1 | 331 | nd | nd | 331 | nd | 289 | nd | nd | nd |
| nCohort 2 | 7 | nd | nd | 10 | nd | 8 | nd | nd | nd |
| Cutoff 1 | 0.407 | nd | nd | 5.14 | nd | 5.14 | nd | nd | nd |
| Sens 1 | 71% | nd | nd | 80% | nd | 88% | nd | nd | nd |
| Spec 1 | 14% | nd | nd | 56% | nd | 58% | nd | nd | nd |
| Cutoff 2 | 0 | nd | nd | 5.14 | nd | 5.14 | nd | nd | nd |
| Sens 2 | 100% | nd | nd | 80% | nd | 88% | nd | nd | nd |
| Spec 2 | 0% | nd | nd | 56% | nd | 58% | nd | nd | nd |
| Cutoff 3 | 0 | nd | nd | 2.30 | nd | 0.407 | nd | nd | nd |
| Sens 3 | 100% | nd | nd | 90% | nd | 100% | nd | nd | nd |
| Spec 3 | 0% | nd | nd | 36% | nd | 16% | nd | nd | nd |
| Cutoff 4 | 5.53 | nd | nd | 5.53 | nd | 5.53 | nd | nd | nd |
| Sens 4 | 43% | nd | nd | 40% | nd | 50% | nd | nd | nd |
| Spec 4 | 71% | nd | nd | 71% | nd | 72% | nd | nd | nd |
| Cutoff 5 | 9.60 | nd | nd | 9.60 | nd | 9.60 | nd | nd | nd |
| Sens 5 | 0% | nd | nd | 30% | nd | 38% | nd | nd | nd |
| Spec 5 | 94% | nd | nd | 94% | nd | 94% | nd | nd | nd |
| Cutoff 6 | 9.60 | nd | nd | 9.60 | nd | 9.60 | nd | nd | nd |
| Sens 6 | 0% | nd | nd | 30% | nd | 38% | nd | nd | nd |
| Spec 6 | 94% | nd | nd | 94% | nd | 94% | nd | nd | nd |
| OR Quart 2 | 3.1 | nd | nd | 1.0 | nd | 0 | nd | nd | nd |
| p Value | 0.33 | nd | nd | 1.0 | nd | na | nd | nd | nd |
| 95% CI of | 0.32 | nd | nd | 0.062 | nd | na | nd | nd | nd |
| OR Quart2 | 31 | nd | nd | 16 | nd | na | nd | nd | nd |
| OR Quart 3 | 0 | nd | nd | 4.1 | nd | 3.1 | nd | nd | nd |
| p Value | na | nd | nd | 0.21 | nd | 0.33 | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of OR Quart3 | na | nd | nd | 0.45 | nd | 0.31 | nd | nd | nd |
| | na | nd | nd | 38 | nd | 30 | nd | nd | nd |
| OR Quart 4 | 3.1 | nd | nd | 4.1 | nd | 4.1 | nd | nd | nd |
| p Value | 0.33 | nd | nd | 0.21 | nd | 0.21 | nd | nd | nd |
| 95% CI of OR Quart4 | 0.32 | nd | nd | 0.45 | nd | 0.45 | nd | nd | nd |
| | 31 | nd | nd | 37 | nd | 38 | nd | nd | nd |

**Interleukin-17A**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00337 | 0.00492 | 0.00337 | 0.00409 | 0.00337 | 0.00348 |
| Average | 0.945 | 1.06 | 0.945 | 0.859 | 0.945 | 0.332 |
| Stdev | 7.23 | 3.01 | 7.23 | 2.96 | 7.23 | 0.952 |
| p(t-test) | | 0.93 | | 0.95 | | 0.72 |
| Min | 0.000344 | 0.000381 | 0.000344 | 0.000381 | 0.000344 | 0.000521 |
| Max | 157 | 12.5 | 157 | 13.0 | 157 | 4.03 |
| n (Samp) | 1925 | 33 | 1925 | 29 | 1925 | 18 |
| n (Patient) | 639 | 33 | 639 | 29 | 639 | 18 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00360 | 0.00337 | 0.00360 | 0.00492 | 0.00360 | 0.00190 |
| Average | 0.963 | 0.0491 | 0.963 | 0.162 | 0.963 | 0.145 |
| Stdev | 7.10 | 0.104 | 7.10 | 0.278 | 7.10 | 0.284 |
| p(t-test) | | 0.64 | | 0.71 | | 0.70 |
| Min | 0.000344 | 0.000344 | 0.000344 | 0.000381 | 0.000344 | 0.000521 |
| Max | 157 | 0.363 | 157 | 0.710 | 157 | 0.814 |
| n (Samp) | 2013 | 13 | 2013 | 11 | 2013 | 11 |
| n (Patient) | 660 | 13 | 660 | 11 | 660 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00337 | 0.00847 | 0.00337 | 0.00427 | 0.00337 | 0.00360 |
| Average | 0.926 | 3.25 | 0.926 | 2.45 | 0.926 | 0.0596 |
| Stdev | 7.22 | 8.12 | 7.22 | 5.23 | 7.22 | 0.111 |
| p(t-test) | | 0.14 | | 0.28 | | 0.72 |
| Min | 0.000344 | 0.000603 | 0.000344 | 0.000380 | 0.000344 | 0.000763 |
| Max | 157 | 33.1 | 157 | 18.8 | 157 | 0.292 |
| n (Samp) | 1825 | 21 | 1825 | 26 | 1825 | 9 |
| n (Patient) | 561 | 21 | 561 | 26 | 561 | 9 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.46 | 0.62 | 0.51 | 0.45 | 0.57 | 0.50 | 0.41 | 0.57 |
| SE | 0.052 | 0.082 | 0.066 | 0.054 | 0.089 | 0.059 | 0.069 | 0.091 | 0.100 |
| p | 0.17 | 0.63 | 0.064 | 0.83 | 0.61 | 0.21 | 0.95 | 0.32 | 0.47 |
| nCohort 1 | 1925 | 2013 | 1825 | 1925 | 2013 | 1825 | 1925 | 2013 | 1825 |
| nCohort 2 | 33 | 13 | 21 | 29 | 11 | 26 | 18 | 11 | 9 |
| Cutoff 1 | 0.00190 | 0.000662 | 0.00190 | 0.00221 | 0.000597 | 0.00263 | 0.00190 | 0.000603 | 0.00322 |
| Sens 1 | 73% | 77% | 76% | 72% | 73% | 73% | 72% | 73% | 78% |
| Spec 1 | 32% | 18% | 34% | 36% | 11% | 40% | 32% | 14% | 47% |

EP 3 249 402 A1

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 2 | 0.000662 | 0.000380 | 0.00182 | 0.000410 | 0.000410 | 0.000747 | 0.000603 | 0.000597 | 0.00221 |
| Sens 2 | 85% | 92% | 81% | 86% | 82% | 81% | 83% | 82% | 89% |
| Spec 2 | 18% | 4% | 32% | 7% | 7% | 25% | 14% | 11% | 38% |
| Cutoff 3 | 0.000597 | 0.000380 | 0.000662 | 0.000380 | 0.000380 | 0.000380 | 0.000410 | 0.000410 | 0.000747 |
| Sens 3 | 91% | 92% | 90% | 100% | 100% | 96% | 100% | 100% | 100% |
| Spec 3 | 12% | 4% | 20% | 4% | 4% | 4% | 7% | 7% | 25% |
| Cutoff 4 | 0.00875 | 0.00988 | 0.00875 | 0.00875 | 0.00988 | 0.00875 | 0.00875 | 0.00988 | 0.00875 |
| Sens 4 | 45% | 31% | 48% | 28% | 27% | 35% | 28% | 27% | 22% |
| Spec 4 | 70% | 71% | 70% | 70% | 71% | 70% | 70% | 71% | 70% |
| Cutoff 5 | 0.152 | 0.160 | 0.155 | 0.152 | 0.160 | 0.155 | 0.152 | 0.160 | 0.155 |
| Sens 5 | 27% | 8% | 38% | 21% | 27% | 31% | 28% | 27% | 22% |
| Spec 5 | 80% | 80% | 80% | 80% | 80% | 80% | 80% | 80% | 80% |
| Cutoff 6 | 0.830 | 0.950 | 0.855 | 0.830 | 0.950 | 0.855 | 0.830 | 0.950 | 0.855 |
| Sens 6 | 15% | 0% | 24% | 7% | 0% | 19% | 6% | 0% | 0% |
| Spec 6 | 90% | 90% | 90% | 90% | 90% | 90% | 90% | 90% | 90% |
| OR Quart 2 | 0.71 | 0.50 | 0.75 | 0.49 | 1.0 | 1.00 | 0.80 | 0 | >3.0 |
| p Value | 0.56 | 0.42 | 0.70 | 0.25 | 1.0 | 1.00 | 0.74 | na | <0.34 |
| 95% CI of OR Quart2 | 0.22 2.3 | 0.091 2.7 | 0.17 3.4 | 0.15 1.7 | 0.20 5.0 | 0.29 3.5 | 0.21 3.0 | na na | >0.31 na |
| OR Quart 3 | 1.1 | 0.75 | 1.3 | 1.3 | 0 | 1.4 | 0.80 | 1.3 | >4.0 |
| p Value | 0.79 | 0.71 | 0.74 | 0.63 | na | 0.57 | 0.74 | 0.71 | <0.21 |
| 95% CI of OR Quart3 | 0.41 3.2 | 0.17 3.4 | 0.33 4.7 | 0.49 3.2 | na na | 0.44 4.5 | 0.21 3.0 | 0.30 6.0 | >0.45 na |
| OR Quart 4 | 1.9 | 1.0 | 2.3 | 0.87 | 1.7 | 1.8 | 1.00 | 1.3 | >2.0 |
| p Value | 0.18 | 1.00 | 0.18 | 0.79 | 0.48 | 0.29 | 1.00 | 0.71 | <0.57 |
| 95% CI of OR Quart4 | 0.74 4.7 | 0.25 4.0 | 0.69 7.4 | 0.31 2.4 | 0.40 7.0 | 0.60 5.4 | 0.29 3.5 | 0.30 6.0 | >0.18 na |

**Insulin receptor substrate 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.78E-5 | 0.00148 | 2.78E-5 | 0.00243 | 2.78E-5 | 0.000546 |
| Average | 0.00276 | 0.00234 | 0.00276 | 0.0183 | 0.00276 | 0.000975 |
| Stdev | 0.0271 | 0.00292 | 0.0271 | 0.0658 | 0.0271 | 0.00130 |
| p(t-test) | | 0.96 | | 0.022 | | 0.85 |
| Min | 1.25E-9 | 1.25E-9 | 1.25E-9 | 7.25E-7 | 1.25E-9 | 2.32E-6 |
| Max | 0.735 | 0.00914 | 0.735 | 0.282 | 0.735 | 0.00334 |
| n (Samp) | 805 | 13 | 805 | 18 | 805 | 8 |
| n (Patient) | 298 | 13 | 298 | 18 | 298 | 8 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 2.78E-5 | 0.00224 | nd | nd |
| Average | nd | nd | 0.00308 | 0.00172 | nd | nd |
| Stdev | nd | nd | 0.0283 | 0.00156 | nd | nd |
| p(t-test) | nd | nd | | 0.89 | nd | nd |
| Min | nd | nd | 1.25E-9 | 7.25E-7 | nd | nd |
| Max | nd | nd | 0.735 | 0.00456 | nd | nd |
| n (Samp) | nd | nd | 831 | 9 | nd | nd |
| n (Patient) | nd | nd | 309 | 9 | nd | nd |

| UO only | 0hr prior to AKI stage | 24hr prior to AKI stage | 48hr prior to AKI stage |
|---|---|---|---|

|  | Cohort 1 | Cohort 2 | Cohort 1 | Cohort 2 | Cohort 1 | Cohort 2 |
|---|---|---|---|---|---|---|
| Median | 2.78E-5 | 1.50E-6 | 2.78E-5 | 0.00243 | nd | nd |
| Average | 0.00271 | 0.000980 | 0.00271 | 0.0245 | nd | nd |
| Stdev | 0.0266 | 0.00180 | 0.0266 | 0.0774 | nd | nd |
| p(t-test) |  | 0.86 |  | 0.0055 | nd | nd |
| Min | 1.25E-9 | 1.25E-9 | 1.25E-9 | 7.25E-7 | nd | nd |
| Max | 0.735 | 0.00456 | 0.735 | 0.282 | nd | nd |
| n (Samp) | 831 | 7 | 831 | 13 | nd | nd |
| n (Patient) | 292 | 7 | 292 | 13 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.55 | nd | 0.33 | 0.72 | 0.63 | 0.69 | 0.57 | nd | nd |
| SE | 0.083 | nd | 0.11 | 0.069 | 0.10 | 0.082 | 0.11 | nd | nd |
| p | 0.54 | nd | 0.14 | 0.0012 | 0.19 | 0.021 | 0.49 | nd | nd |
| nCohort 1 | 805 | nd | 831 | 805 | 831 | 831 | 805 | nd | nd |
| nCohort 2 | 13 | nd | 7 | 18 | 9 | 13 | 8 | nd | nd |
| Cutoff 1 | 1.25E-9 | nd | 0 | 0.00101 | 3.73E-6 | 2.78E-5 | 3.73E-6 | nd | nd |
| Sens 1 | 85% | nd | 100% | 72% | 78% | 77% | 88% | nd | nd |
| Spec 1 | 7% | nd | 0% | 64% | 34% | 52% | 34% | nd | nd |
| Cutoff 2 | 1.25E-9 | nd | 0 | 3.73E-6 | 1.78E-6 | 3.73E-6 | 3.73E-6 | nd | nd |
| Sens 2 | 85% | nd | 100% | 89% | 89% | 85% | 88% | nd | nd |
| Spec 2 | 7% | nd | 0% | 34% | 27% | 34% | 34% | nd | nd |
| Cutoff 3 | 0 | nd | 0 | 1.25E-9 | 1.25E-9 | 1.25E-9 | 1.78E-6 | nd | nd |
| Sens 3 | 100% | nd | 100% | 100% | 100% | 100% | 100% | nd | nd |
| Spec 3 | 0% | nd | 0% | 7% | 7% | 7% | 27% | nd | nd |
| Cutoff 4 | 0.00110 | nd | 0.00110 | 0.00110 | 0.00110 | 0.00110 | 0.00110 | nd | nd |
| Sens 4 | 54% | nd | 29% | 67% | 56% | 62% | 25% | nd | nd |
| Spec 4 | 71% | nd | 71% | 71% | 70% | 71% | 71% | nd | nd |
| Cutoff 5 | 0.00229 | nd | 0.00229 | 0.00229 | 0.00229 | 0.00229 | 0.00229 | nd | nd |
| Sens 5 | 38% | nd | 14% | 67% | 44% | 62% | 25% | nd | nd |
| Spec 5 | 82% | nd | 82% | 82% | 81% | 82% | 82% | nd | nd |
| Cutoff 6 | 0.00281 | nd | 0.00281 | 0.00281 | 0.00281 | 0.00281 | 0.00281 | nd | nd |
| Sens 6 | 31% | nd | 14% | 22% | 11% | 31% | 12% | nd | nd |
| Spec 6 | 92% | nd | 91% | 92% | 91% | 91% | 92% | nd | nd |
| OR Quart 2 | 0.49 | nd | 0 | 1.00 | 2.0 | 0.50 | >3.0 | nd | nd |
| p Value | 0.42 | nd | na | 1.00 | 0.57 | 0.57 | <0.34 | nd | nd |
| 95% CI of | 0.089 | nd | na | 0.14 | 0.18 | 0.045 | >0.31 | nd | nd |
| OR Quart2 | 2.7 | nd | na | 7.1 | 22 | 5.5 | na | nd | nd |
| OR Quart 3 | 0.25 | nd | 0.50 | 1.00 | 2.0 | 1.0 | >3.0 | nd | nd |
| p Value | 0.21 | nd | 0.57 | 1.00 | 0.57 | 1.0 | <0.34 | nd | nd |
| 95% CI of | 0.027 | nd | 0.045 | 0.14 | 0.18 | 0.14 | >0.31 | nd | nd |
| OR Quart3 | 2.2 | nd | 5.5 | 7.1 | 22 | 7.2 | na | nd | nd |
| OR Quart 4 | 1.5 | nd | 2.0 | 6.3 | 4.1 | 4.1 | >2.0 | nd | nd |
| p Value | 0.53 | nd | 0.42 | 0.017 | 0.21 | 0.076 | <0.57 | nd | nd |
| 95% CI of | 0.42 | nd | 0.37 | 1.4 | 0.45 | 0.86 | >0.18 | nd | nd |
| OR Quart4 | 5.4 | nd | 11 | 28 | 37 | 20 | na | nd | nd |

**Involucrin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.499 | 0.604 | 0.499 | 1.15 | 0.499 | 0.420 |
| Average | 1.33 | 2.11 | 1.33 | 2.58 | 1.33 | 0.526 |
| Stdev | 3.76 | 2.84 | 3.76 | 4.14 | 3.76 | 0.521 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.32 | | 0.099 | | 0.41 |
| Min | 0.00244 | 0.0767 | 0.00244 | 0.151 | 0.00244 | 0.0268 |
| Max | 102 | 9.05 | 102 | 17.0 | 102 | 2.13 |
| n (Samp) | 1363 | 23 | 1363 | 25 | 1363 | 15 |
| n (Patient) | 521 | 23 | 521 | 25 | 521 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.508 | 0.380 | 0.508 | 0.473 | 0.508 | 0.696 |
| Average | 1.38 | 0.646 | 1.38 | 1.18 | 1.38 | 2.51 |
| Stdev | 3.78 | 0.786 | 3.78 | 1.46 | 3.78 | 4.63 |
| p(t-test) | | 0.61 | | 0.87 | | 0.37 |
| Min | 0.00244 | 0.0767 | 0.00244 | 0.193 | 0.00244 | 0.119 |
| Max | 102 | 2.34 | 102 | 4.04 | 102 | 14.5 |
| n (Samp) | 1407 | 7 | 1407 | 10 | 1407 | 9 |
| n (Patient) | 536 | 7 | 536 | 10 | 536 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.507 | 1.43 | 0.507 | 1.36 | 0.507 | 0.528 |
| Average | 1.35 | 2.91 | 1.35 | 3.09 | 1.35 | 0.481 |
| Stdev | 3.85 | 3.15 | 3.85 | 4.51 | 3.85 | 0.296 |
| p(t-test) | | 0.12 | | 0.036 | | 0.55 |
| Min | 0.00244 | 0.144 | 0.00244 | 0.124 | 0.00244 | 0.0268 |
| Max | 102 | 9.05 | 102 | 17.0 | 102 | 0.811 |
| n (Samp) | 1287 | 15 | 1287 | 22 | 1287 | 7 |
| n (Patient) | 448 | 15 | 448 | 22 | 448 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.42 | 0.68 | 0.69 | 0.53 | 0.68 | 0.41 | 0.55 | 0.42 |
| SE | 0.063 | 0.11 | 0.077 | 0.059 | 0.093 | 0.063 | 0.078 | 0.099 | 0.11 |
| p | 0.14 | 0.46 | 0.020 | 0.0014 | 0.71 | 0.0036 | 0.25 | 0.58 | 0.50 |
| nCohort 1 | 1363 | 1407 | 1287 | 1363 | 1407 | 1287 | 1363 | 1407 | 1287 |
| nCohort 2 | 23 | 7 | 15 | 25 | 10 | 22 | 15 | 9 | 7 |
| Cutoff 1 | 0.380 | 0.234 | 0.502 | 0.683 | 0.357 | 0.591 | 0.178 | 0.255 | 0.419 |
| Sens 1 | 74% | 71% | 73% | 72% | 70% | 73% | 73% | 78% | 71% |
| Spec 1 | 41% | 26% | 49% | 60% | 38% | 55% | 21% | 29% | 43% |
| Cutoff 2 | 0.357 | 0.142 | 0.398 | 0.485 | 0.230 | 0.457 | 0.162 | 0.178 | 0.162 |
| Sens 2 | 83% | 86% | 80% | 80% | 80% | 82% | 80% | 89% | 86% |
| Spec 2 | 39% | 15% | 42% | 49% | 26% | 46% | 18% | 20% | 17% |
| Cutoff 3 | 0.234 | 0.0763 | 0.273 | 0.225 | 0.225 | 0.225 | 0.0270 | 0.118 | 0.0265 |
| Sens 3 | 91% | 100% | 93% | 92% | 90% | 91% | 93% | 100% | 100% |
| Spec 3 | 27% | 6% | 30% | 26% | 26% | 25% | 1% | 12% | 1% |
| Cutoff 4 | 0.979 | 1.00 | 1.00 | 0.979 | 1.00 | 1.00 | 0.979 | 1.00 | 1.00 |
| Sens 4 | 35% | 14% | 53% | 60% | 30% | 55% | 7% | 33% | 0% |
| Spec 4 | 70% | 70% | 70% | 70% | 70% | 70% | 70% | 70% | 70% |
| Cutoff 5 | 1.56 | 1.59 | 1.57 | 1.56 | 1.59 | 1.57 | 1.56 | 1.59 | 1.57 |
| Sens 5 | 30% | 14% | 47% | 36% | 20% | 41% | 7% | 33% | 0% |
| Spec 5 | 80% | 80% | 80% | 80% | 80% | 80% | 80% | 80% | 80% |
| Cutoff 6 | 2.88 | 2.99 | 2.90 | 2.88 | 2.99 | 2.90 | 2.88 | 2.99 | 2.90 |
| Sens 6 | 22% | 0% | 33% | 20% | 20% | 27% | 0% | 22% | 0% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 6 | 90% | 90% | 90% | 90% | 90% | 90% | 90% | 90% | 90% |
| OR Quart 2 | 3.5 | 2.0 | 4.0 | 2.0 | 5.1 | 1.0 | 6.1 | 1.0 | >4.1 |
| p Value | 0.12 | 0.57 | 0.21 | 0.42 | 0.14 | 1.0 | 0.095 | 1.0 | <0.21 |
| 95% CI of OR Quart2 | 0.73 17 | 0.18 22 | 0.45 36 | 0.37 11 | 0.59 44 | 0.20 5.0 | 0.73 51 | 0.14 7.1 | >0.45 na |
| OR Quart 3 | 3.0 | 2.0 | 2.0 | 3.6 | 2.0 | 1.3 | 3.0 | 1.0 | >1.0 |
| p Value | 0.18 | 0.57 | 0.57 | 0.12 | 0.57 | 0.70 | 0.34 | 1.0 | <1.00 |
| 95% CI of OR Quart3 | 0.61 15 | 0.18 22 | 0.18 22 | 0.73 17 | 0.18 22 | 0.30 6.0 | 0.31 29 | 0.14 7.1 | >0.062 na |
| OR Quart 4 | 4.1 | 2.0 | 8.2 | 6.2 | 2.0 | 4.1 | 5.1 | 1.5 | >2.0 |
| p Value | 0.078 | 0.57 | 0.049 | 0.018 | 0.57 | 0.030 | 0.14 | 0.66 | <0.57 |
| 95% CI of OR Quart4 | 0.86 19 | 0.18 22 | 1.0 66 | 1.4 28 | 0.18 22 | 1.1 15 | 0.59 44 | 0.25 9.1 | >0.18 na |

**Keratin, type II cytoskeletal 1; type1 cytoskeletal 10 (Keratin-1,-10 mix)**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0174 | 0.00790 | 0.0174 | 0.0502 | 0.0174 | 0.0174 |
| Average | 2.77 | 0.269 | 2.77 | 13.3 | 2.77 | 0.839 |
| Stdev | 14.5 | 0.884 | 14.5 | 61.6 | 14.5 | 1.30 |
| p(t-test) | | 0.41 | | 0.0016 | | 0.61 |
| Min | 0.000410 | 0.000410 | 0.000410 | 0.000410 | 0.000410 | 0.000410 |
| Max | 367 | 3.62 | 367 | 309 | 367 | 4.35 |
| n (Samp) | 1363 | 23 | 1363 | 25 | 1363 | 15 |
| n (Patient) | 521 | 23 | 521 | 25 | 521 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0174 | 0.00432 | 0.0174 | 0.0502 | 0.0174 | 0.0174 |
| Average | 2.95 | 0.00582 | 2.95 | 1.77 | 2.95 | 0.749 |
| Stdev | 16.4 | 0.00474 | 16.4 | 2.44 | 16.4 | 1.02 |
| p(t-test) | | 0.64 | | 0.82 | | 0.69 |
| Min | 0.000410 | 0.000410 | 0.000410 | 0.00694 | 0.000410 | 0.000410 |
| Max | 367 | 0.0113 | 367 | 6.14 | 367 | 2.40 |
| n (Samp) | 1407 | 7 | 1407 | 10 | 1407 | 9 |
| n (Patient) | 536 | 7 | 536 | 10 | 536 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0174 | 0.00694 | 0.0174 | 0.0174 | 0.0174 | 0.0153 |
| Average | 2.43 | 3.15 | 2.43 | 15.2 | 2.43 | 0.825 |
| Stdev | 10.7 | 10.8 | 10.7 | 65.6 | 10.7 | 1.64 |
| p(t-test) | | 0.79 | | 1.1E-5 | | 0.69 |
| Min | 0.000410 | 0.000410 | 0.000410 | 0.000410 | 0.000410 | 0.000410 |
| Max | 217 | 42.0 | 217 | 309 | 217 | 4.35 |
| n (Samp) | 1287 | 15 | 1287 | 22 | 1287 | 7 |
| n (Patient) | 448 | 15 | 448 | 22 | 448 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.30 | 0.21 | 0.33 | 0.54 | 0.62 | 0.51 | 0.47 | 0.43 | 0.38 |
| SE | 0.062 | 0.10 | 0.077 | 0.059 | 0.095 | 0.062 | 0.076 | 0.100 | 0.11 |
| p | 9.5E-4 | 0.0056 | 0.023 | 0.52 | 0.21 | 0.90 | 0.74 | 0.49 | 0.31 |
| nCohort 1 | 1363 | 1407 | 1287 | 1363 | 1407 | 1287 | 1363 | 1407 | 1287 |
| nCohort 2 | 23 | 7 | 15 | 25 | 10 | 22 | 15 | 9 | 7 |
| Cutoff 1 | 0.00380 | 0.00380 | 0.00408 | 0.00926 | 0.0128 | 0.00892 | 0.00787 | 0 | 0.00303 |
| Sens 1 | 74% | 71% | 73% | 76% | 70% | 73% | 73% | 100% | 71% |
| Spec 1 | 11% | 11% | 12% | 40% | 45% | 36% | 33% | 0% | 10% |
| Cutoff 2 | 0 | 0 | 0 | 0.00694 | 0.00926 | 0.00562 | 0.00380 | 0 | 0 |
| Sens 2 | 100% | 100% | 100% | 80% | 90% | 82% | 80% | 100% | 100% |
| Spec 2 | 0% | 0% | 0% | 30% | 40% | 26% | 11% | 0% | 0% |
| Cutoff 3 | 0 | 0 | 0 | 0.000693 | 0.00926 | 0.000693 | 0 | 0 | 0 |
| Sens 3 | 100% | 100% | 100% | 92% | 90% | 91% | 100% | 100% | 100% |
| Spec 3 | 0% | 0% | 0% | 6% | 40% | 6% | 0% | 0% | 0% |
| Cutoff 4 | 1.21 | 1.21 | 1.39 | 1.21 | 1.21 | 1.39 | 1.21 | 1.21 | 1.39 |
| Sens 4 | 9% | 0% | 13% | 28% | 40% | 23% | 27% | 22% | 14% |
| Spec 4 | 70% | 70% | 72% | 70% | 70% | 72% | 70% | 70% | 72% |
| Cutoff 5 | 2.40 | 2.40 | 2.40 | 2.40 | 2.40 | 2.40 | 2.40 | 2.40 | 2.40 |
| Sens 5 | 4% | 0% | 13% | 16% | 30% | 18% | 7% | 0% | 14% |
| Spec 5 | 80% | 80% | 80% | 80% | 80% | 80% | 80% | 80% | 80% |
| Cutoff 6 | 5.19 | 5.20 | 4.71 | 5.19 | 5.20 | 4.71 | 5.19 | 5.20 | 4.71 |
| Sens 6 | 0% | 0% | 13% | 4% | 10% | 14% | 0% | 0% | 0% |
| Spec 6 | 90% | 90% | 90% | 90% | 90% | 90% | 90% | 90% | 90% |
| OR Quart 2 | 0.50 | >0 | 0 | 2.0 | >4.0 | 1.8 | 1.7 | 1.0 | 1.0 |
| p Value | 0.57 | <na | na | 0.25 | <0.21 | 0.37 | 0.48 | 1.0 | 1.00 |
| 95% CI of | 0.045 | >na | na | 0.60 | >0.45 | 0.51 | 0.40 | 0.14 | 0.062 |
| OR Quart2 | 5.5 | na | na | 6.8 | na | 6.1 | 7.1 | 7.1 | 16 |
| OR Quart 3 | 5.6 | >3.0 | 4.1 | 1.5 | >2.0 | 1.5 | 1.0 | 1.0 | 2.0 |
| p Value | 0.025 | <0.34 | 0.077 | 0.53 | <0.57 | 0.53 | 1.0 | 1.0 | 0.57 |
| 95% CI of | 1.2 | >0.31 | 0.86 | 0.42 | >0.18 | 0.42 | 0.20 | 0.14 | 0.18 |
| OR Quart3 | 26 | na | 19 | 5.4 | na | 5.4 | 5.0 | 7.1 | 22 |
| OR Quart 4 | 4.6 | >4.1 | 2.5 | 1.8 | >4.0 | 1.2 | 1.3 | 1.5 | 3.0 |
| p Value | 0.052 | <0.21 | 0.27 | 0.37 | <0.21 | 0.74 | 0.70 | 0.66 | 0.34 |
| 95% CI of | 0.99 | >0.45 | 0.49 | 0.51 | >0.45 | 0.33 | 0.30 | 0.25 | 0.31 |
| OR Quart4 | 21 | na | 13 | 6.1 | na | 4.7 | 6.0 | 9.1 | 29 |

**Lipopolysaccharide (serotypes -K,-O)**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.53E-5 | 9.72E-5 | 2.53E-5 | 9.77E-5 | 2.53E-5 | 9.77E-5 |
| Average | 0.115 | 0.0760 | 0.115 | 0.0268 | 0.115 | 0.00189 |
| Stdev | 0.634 | 0.241 | 0.634 | 0.116 | 0.634 | 0.00425 |
| p(t-test) | | 0.77 | | 0.49 | | 0.49 |
| Min | 1.40E-8 | 1.14E-5 | 1.40E-8 | 4.54E-6 | 1.40E-8 | 6.10E-6 |
| Max | 6.42 | 1.04 | 6.42 | 0.579 | 6.42 | 0.0162 |
| n (Samp) | 1363 | 23 | 1363 | 25 | 1363 | 15 |
| n (Patient) | 521 | 23 | 521 | 25 | 521 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.53E-5 | 1.85E-5 | 2.53E-5 | 9.74E-5 | 2.53E-5 | 9.72E-5 |
| Average | 0.119 | 0.152 | 0.119 | 0.00587 | 0.119 | 0.00144 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 0.641 | 0.394 | 0.641 | 0.0149 | 0.641 | 0.00346 |
| p(t-test) | | 0.89 | | 0.58 | | 0.58 |
| Min | 1.40E-8 | 1.18E-5 | 1.40E-8 | 1.18E-5 | 1.40E-8 | 6.10E-6 |
| Max | 6.42 | 1.04 | 6.42 | 0.0478 | 6.42 | 0.0105 |
| n (Samp) | 1407 | 7 | 1407 | 10 | 1407 | 9 |
| n (Patient) | 536 | 7 | 536 | 10 | 536 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.53E-5 | 0.00215 | 2.53E-5 | 0.000150 | 2.53E-5 | 0.00160 |
| Average | 0.0998 | 0.318 | 0.0998 | 0.278 | 0.0998 | 0.00367 |
| Stdev | 0.584 | 0.830 | 0.584 | 0.815 | 0.584 | 0.00588 |
| p(t-test) | | 0.15 | | 0.16 | | 0.66 |
| Min | 1.40E-8 | 1.14E-5 | 1.40E-8 | 4.54E-6 | 1.40E-8 | 6.10E-6 |
| Max | 6.40 | 2.77 | 6.40 | 3.10 | 6.40 | 0.0162 |
| n (Samp) | 1287 | 15 | 1287 | 22 | 1287 | 7 |
| n (Patient) | 448 | 15 | 448 | 22 | 448 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.58 | 0.57 | 0.64 | 0.51 | 0.55 | 0.57 | 0.51 | 0.45 | 0.54 |
| SE | 0.063 | 0.11 | 0.078 | 0.059 | 0.094 | 0.064 | 0.075 | 0.099 | 0.11 |
| p | 0.18 | 0.55 | 0.077 | 0.81 | 0.61 | 0.31 | 0.92 | 0.64 | 0.70 |
| nCohort 1 | 1363 | 1407 | 1287 | 1363 | 1407 | 1287 | 1363 | 1407 | 1287 |
| nCohort 2 | 23 | 7 | 15 | 25 | 10 | 22 | 15 | 9 | 7 |
| Cutoff 1 | 1.57E-5 | 1.57E-5 | 1.80E-5 | 1.48E-5 | 1.65E-5 | 1.49E-5 | 1.49E-5 | 1.14E-5 | 1.65E-5 |
| Sens 1 | 74% | 71% | 73% | 76% | 70% | 77% | 73% | 89% | 71% |
| Spec 1 | 38% | 37% | 44% | 32% | 40% | 36% | 34% | 19% | 42% |
| Cutoff 2 | 1.48E-5 | 1.48E-5 | 1.57E-5 | 1.14E-5 | 1.48E-5 | 1.48E-5 | 1.14E-5 | 1.14E-5 | 1.14E-5 |
| Sens 2 | 87% | 86% | 80% | 88% | 90% | 82% | 93% | 89% | 86% |
| Spec 2 | 32% | 31% | 38% | 20% | 31% | 32% | 20% | 19% | 19% |
| Cutoff 3 | 1.38E-5 | 1.14E-5 | 1.38E-5 | 5.32E-6 | 1.48E-5 | 5.32E-6 | 1.14E-5 | 5.32E-6 | 5.32E-6 |
| Sens 3 | 91% | 100% | 93% | 96% | 90% | 95% | 93% | 100% | 100% |
| Spec 3 | 28% | 19% | 29% | 4% | 31% | 4% | 20% | 4% | 4% |
| Cutoff 4 | 0.00160 | 0.00196 | 0.00159 | 0.00160 | 0.00196 | 0.00159 | 0.00160 | 0.00196 | 0.00159 |
| Sens 4 | 43% | 43% | 53% | 32% | 40% | 36% | 27% | 22% | 57% |
| Spec 4 | 70% | 70% | 70% | 70% | 70% | 70% | 70% | 70% | 70% |
| Cutoff 5 | 0.00755 | 0.00766 | 0.00671 | 0.00755 | 0.00766 | 0.00671 | 0.00755 | 0.00766 | 0.00671 |
| Sens 5 | 22% | 29% | 33% | 16% | 10% | 23% | 7% | 11% | 14% |
| Spec 5 | 80% | 81% | 80% | 80% | 81% | 80% | 80% | 81% | 80% |
| Cutoff 6 | 0.0331 | 0.0381 | 0.0289 | 0.0331 | 0.0381 | 0.0289 | 0.0331 | 0.0381 | 0.0289 |
| Sens 6 | 13% | 14% | 20% | 8% | 10% | 14% | 0% | 0% | 0% |
| Spec 6 | 90% | 90% | 90% | 90% | 90% | 90% | 90% | 90% | 90% |
| OR Quart 2 | 4.6 | 3.0 | 5.0 | 0.83 | 3.0 | 1.3 | 0.50 | 4.0 | 0.50 |
| p Value | 0.053 | 0.34 | 0.14 | 0.76 | 0.34 | 0.74 | 0.42 | 0.21 | 0.57 |
| 95% CI of OR Quart2 | 0.98 | 0.31 | 0.59 | 0.25 | 0.31 | 0.33 | 0.090 | 0.45 | 0.045 |
| | 21 | 29 | 43 | 2.7 | 29 | 4.7 | 2.7 | 36 | 5.5 |
| OR Quart 3 | 2.0 | 0 | 2.0 | 1.3 | 4.0 | 1.5 | 1.8 | 1.0 | 1.0 |
| p Value | 0.42 | na | 0.57 | 0.59 | 0.21 | 0.53 | 0.37 | 1.0 | 1.0 |
| 95% CI of OR Quart3 | 0.37 | na | 0.18 | 0.46 | 0.45 | 0.42 | 0.51 | 0.062 | 0.14 |
| | 11 | na | 22 | 3.9 | 36 | 5.4 | 6.1 | 16 | 7.1 |
| OR Quart 4 | 4.1 | 3.0 | 7.1 | 1.0 | 2.0 | 1.8 | 0.50 | 3.0 | 1.00 |
| p Value | 0.078 | 0.34 | 0.067 | 1.0 | 0.57 | 0.37 | 0.42 | 0.34 | 1.00 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of OR Quart4 | 0.86 19 | 0.31 29 | 0.87 58 | 0.32 3.1 | 0.18 22 | 0.51 6.1 | 0.090 2.7 | 0.31 29 | 0.14 7.1 |

**Collagenase 3**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 3.12 | 0.202 | 3.12 | 0.230 | 3.12 | 1.55 |
| Average | 12.7 | 14.8 | 12.7 | 9.77 | 12.7 | 7.58 |
| Stdev | 25.8 | 24.4 | 25.8 | 16.5 | 25.8 | 12.3 |
| p(t-test) |  | 0.79 |  | 0.64 |  | 0.60 |
| Min | 0.00479 | 0.00479 | 0.00479 | 0.00479 | 0.00479 | 0.00588 |
| Max | 340 | 70.5 | 340 | 55.0 | 340 | 32.7 |
| n (Samp) | 687 | 11 | 687 | 17 | 687 | 7 |
| n (Patient) | 275 | 11 | 275 | 17 | 275 | 7 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 3.10 | 7.51 | nd | nd |
| Average | nd | nd | 12.5 | 20.3 | nd | nd |
| Stdev | nd | nd | 25.5 | 25.2 | nd | nd |
| p(t-test) | nd | nd |  | 0.36 | nd | nd |
| Min | nd | nd | 0.00479 | 0.202 | nd | nd |
| Max | nd | nd | 340 | 58.5 | nd | nd |
| n (Samp) | nd | nd | 708 | 9 | nd | nd |
| n (Patient) | nd | nd | 285 | 9 | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 3.54 | 0.0149 | 3.54 | 0.718 | nd | nd |
| Average | 13.3 | 4.01 | 13.3 | 7.31 | nd | nd |
| Stdev | 26.0 | 7.38 | 26.0 | 12.9 | nd | nd |
| p(t-test) |  | 0.38 |  | 0.42 | nd | nd |
| Min | 0.00479 | 0.00479 | 0.00479 | 0.00479 | nd | nd |
| Max | 340 | 18.4 | 340 | 41.9 | nd | nd |
| n (Samp) | 706 | 6 | 706 | 12 | nd | nd |
| n (Patient) | 268 | 6 | 268 | 12 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.42 | nd | 0.29 | 0.49 | 0.63 | 0.46 | 0.48 | nd | nd |
| SE | 0.091 | nd | 0.12 | 0.071 | 0.10 | 0.086 | 0.11 | nd | nd |
| p | 0.35 | nd | 0.078 | 0.92 | 0.18 | 0.61 | 0.85 | nd | nd |
| nCohort 1 | 687 | nd | 706 | 687 | 708 | 706 | 687 | nd | nd |
| nCohort 2 | 11 | nd | 6 | 17 | 9 | 12 | 7 | nd | nd |
| Cutoff 1 | 0.00479 | nd | 0.00479 | 0.0466 | 0.202 | 0.0466 | 1.39 | nd | nd |
| Sens 1 | 73% | nd | 83% | 88% | 78% | 92% | 71% | nd | nd |
| Spec 1 | 2% | nd | 3% | 29% | 37% | 29% | 46% | nd | nd |
| Cutoff 2 | 0 | nd | 0.00479 | 0.0466 | 0.0466 | 0.0466 | 0.0425 | nd | nd |
| Sens 2 | 100% | nd | 83% | 88% | 100% | 92% | 86% | nd | nd |
| Spec 2 | 0% | nd | 3% | 29% | 29% | 29% | 27% | nd | nd |
| Cutoff 3 | 0 | nd | 0 | 0.0200 | 0.0466 | 0.0466 | 0.00519 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 100% | nd | 100% | 94% | 100% | 92% | 100% | nd | nd |
| Spec 3 | 0% | nd | 0% | 23% | 29% | 29% | 5% | nd | nd |
| Cutoff 4 | 13.0 | nd | 13.5 | 13.0 | 12.3 | 13.5 | 13.0 | nd | nd |
| Sens 4 | 36% | nd | 17% | 24% | 44% | 17% | 29% | nd | nd |
| Spec 4 | 71% | nd | 70% | 71% | 70% | 70% | 71% | nd | nd |
| Cutoff 5 | 20.9 | nd | 22.4 | 20.9 | 20.4 | 22.4 | 20.9 | nd | nd |
| Sens 5 | 18% | nd | 0% | 18% | 33% | 17% | 14% | nd | nd |
| Spec 5 | 80% | nd | 80% | 80% | 80% | 80% | 80% | nd | nd |
| Cutoff 6 | 35.5 | nd | 38.2 | 35.5 | 34.2 | 38.2 | 35.5 | nd | nd |
| Sens 6 | 18% | nd | 0% | 12% | 33% | 8% | 0% | nd | nd |
| Spec 6 | 90% | nd | 90% | 90% | 90% | 90% | 90% | nd | nd |
| OR Quart 2 | 0 | nd | 1.0 | 1.3 | >4.1 | 1.0 | 1.0 | nd | nd |
| p Value | na | nd | 1.0 | 0.70 | <0.21 | 1.00 | 1.00 | nd | nd |
| 95% CI of | na | nd | 0.062 | 0.30 | >0.45 | 0.14 | 0.062 | nd | nd |
| OR Quart2 | na | nd | 16 | 6.1 | na | 7.2 | 16 | nd | nd |
| OR Quart 3 | 0.49 | nd | 0 | 3.1 | >2.0 | 3.6 | 4.1 | nd | nd |
| p Value | 0.42 | nd | na | 0.093 | <0.57 | 0.11 | 0.21 | nd | nd |
| 95% CI of | 0.089 | nd | na | 0.83 | >0.18 | 0.74 | 0.45 | nd | nd |
| OR Quart3 | 2.7 | nd | na | 12 | na | 18 | 37 | nd | nd |
| OR Quart 4 | 1.3 | nd | 4.1 | 0.33 | >3.0 | 0.50 | 1.0 | nd | nd |
| p Value | 0.73 | nd | 0.21 | 0.34 | <0.34 | 0.57 | 1.00 | nd | nd |
| 95% CI of | 0.33 | nd | 0.45 | 0.034 | >0.31 | 0.045 | 0.062 | nd | nd |
| OR Quart4 | 4.8 | nd | 37 | 3.2 | na | 5.6 | 16 | nd | nd |

**NF-kappa-B inhibitor alpha**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000108 | 0.000422 | 0.000108 | 0.000127 | 0.000108 | 1.06E-6 |
| Average | 0.00508 | 0.0131 | 0.00508 | 0.00193 | 0.00508 | 0.000344 |
| Stdev | 0.0267 | 0.0266 | 0.0267 | 0.00423 | 0.0267 | 0.000884 |
| p(t-test) | | 0.28 | | 0.62 | | 0.62 |
| Min | 3.52E-7 | 3.52E-7 | 3.52E-7 | 9.58E-7 | 3.52E-7 | 5.23E-7 |
| Max | 0.326 | 0.0725 | 0.326 | 0.0159 | 0.326 | 0.00252 |
| n (Samp) | 805 | 13 | 805 | 18 | 805 | 8 |
| n (Patient) | 298 | 13 | 298 | 18 | 298 | 8 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.000108 | 3.20E-5 | nd | nd |
| Average | nd | nd | 0.00556 | 0.000260 | nd | nd |
| Stdev | nd | nd | 0.0281 | 0.000443 | nd | nd |
| p(t-test) | nd | nd | | 0.57 | nd | nd |
| Min | nd | nd | 3.52E-7 | 5.23E-7 | nd | nd |
| Max | nd | nd | 0.326 | 0.00125 | nd | nd |
| n (Samp) | nd | nd | 831 | 9 | nd | nd |
| n (Patient) | nd | nd | 309 | 9 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000108 | 0.000422 | 0.000108 | 0.000566 | nd | nd |
| Average | 0.00503 | 0.00828 | 0.00503 | 0.0224 | nd | nd |
| Stdev | 0.0264 | 0.0208 | 0.0264 | 0.0707 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.75 | | 0.025 | nd | nd |
| Min | 3.52E-7 | 3.52E-7 | 3.52E-7 | 2.25E-5 | nd | nd |
| Max | 0.326 | 0.0554 | 0.326 | 0.257 | nd | nd |
| n (Samp) | 831 | 7 | 831 | 13 | nd | nd |
| n (Patient) | 292 | 7 | 292 | 13 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | 0.55 | 0.58 | 0.47 | 0.70 | 0.37 | nd | nd |
| SE | 0.084 | nd | 0.11 | 0.071 | 0.098 | 0.082 | 0.11 | nd | nd |
| p | 0.23 | nd | 0.66 | 0.24 | 0.77 | 0.017 | 0.23 | nd | nd |
| nCohort 1 | 805 | nd | 831 | 805 | 831 | 831 | 805 | nd | nd |
| nCohort 2 | 13 | nd | 7 | 18 | 9 | 13 | 8 | nd | nd |
| Cutoff 1 | 9.77E-5 | nd | 0.000219 | 2.25E-5 | 2.18E-5 | 2.25E-5 | 5.23E-7 | nd | nd |
| Sens 1 | 77% | nd | 71% | 83% | 78% | 92% | 88% | nd | nd |
| Spec 1 | 49% | nd | 60% | 39% | 36% | 40% | 23% | nd | nd |
| Cutoff 2 | 9.58E-7 | nd | 0 | 2.25E-5 | 1.03E-6 | 2.25E-5 | 5.23E-7 | nd | nd |
| Sens 2 | 85% | nd | 100% | 83% | 89% | 92% | 88% | nd | nd |
| Spec 2 | 26% | nd | 0% | 39% | 27% | 40% | 23% | nd | nd |
| Cutoff 3 | 0 | nd | 0 | 1.03E-6 | 5.05E-7 | 2.25E-5 | 5.05E-7 | nd | nd |
| Sens 3 | 100% | nd | 100% | 94% | 100% | 92% | 100% | nd | nd |
| Spec 3 | 0% | nd | 0% | 27% | 19% | 40% | 19% | nd | nd |
| Cutoff 4 | 0.000566 | nd | 0.000532 | 0.000566 | 0.000566 | 0.000532 | 0.000566 | nd | nd |
| Sens 4 | 46% | nd | 43% | 33% | 22% | 54% | 12% | nd | nd |
| Spec 4 | 71% | nd | 70% | 71% | 71% | 70% | 71% | nd | nd |
| Cutoff 5 | 0.00113 | nd | 0.00110 | 0.00113 | 0.00121 | 0.00110 | 0.00113 | nd | nd |
| Sens 5 | 31% | nd | 29% | 22% | 11% | 31% | 12% | nd | nd |
| Spec 5 | 80% | nd | 80% | 80% | 80% | 80% | 80% | nd | nd |
| Cutoff 6 | 0.00351 | nd | 0.00343 | 0.00351 | 0.00381 | 0.00343 | 0.00351 | nd | nd |
| Sens 6 | 23% | nd | 14% | 17% | 0% | 31% | 0% | nd | nd |
| Spec 6 | 90% | nd | 90% | 90% | 90% | 90% | 90% | nd | nd |
| OR Quart 2 | 1.00 | nd | 0 | 7.2 | 2.0 | >4.1 | 1.0 | nd | nd |
| p Value | 1.00 | nd | na | 0.066 | 0.57 | <0.21 | 1.00 | nd | nd |
| 95% CI of OR Quart2 | 0.14 | nd | na | 0.87 | 0.18 | >0.45 | 0.062 | nd | nd |
| | 7.1 | nd | na | 59 | 22 | na | 16 | nd | nd |
| OR Quart 3 | 2.5 | nd | 1.5 | 4.0 | 5.1 | >3.0 | 2.0 | nd | nd |
| p Value | 0.27 | nd | 0.65 | 0.21 | 0.14 | <0.34 | 0.57 | nd | nd |
| 95% CI of OR Quart3 | 0.49 | nd | 0.25 | 0.45 | 0.59 | >0.31 | 0.18 | nd | nd |
| | 13 | nd | 9.1 | 36 | 44 | na | 22 | nd | nd |
| OR Quart 4 | 2.0 | nd | 1.00 | 6.1 | 1.0 | >6.2 | 4.1 | nd | nd |
| p Value | 0.42 | nd | 1.00 | 0.095 | 1.0 | <0.093 | 0.21 | nd | nd |
| 95% CI of OR Quart4 | 0.36 | nd | 0.14 | 0.73 | 0.062 | >0.74 | 0.45 | nd | nd |
| | 11 | nd | 7.1 | 51 | 16 | na | 37 | nd | nd |

**Beta-nerve growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.36 | 1.85 | 2.36 | 4.01 | 2.36 | 1.75 |
| Average | 3.81 | 3.82 | 3.81 | 5.40 | 3.81 | 2.52 |
| Stdev | 5.01 | 5.92 | 5.01 | 5.87 | 5.01 | 2.76 |
| p(t-test) | | 0.99 | | 0.19 | | 0.47 |
| Min | 0.000201 | 0.000201 | 0.000201 | 0.000605 | 0.000201 | 0.000201 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 46.1 | 22.1 | 46.1 | 25.2 | 46.1 | 8.38 |
| n (Samp) | 807 | 13 | 807 | 18 | 807 | 8 |
| n (Patient) | 300 | 13 | 300 | 18 | 300 | 8 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 2.32 | 3.88 | nd | nd |
| Average | nd | nd | 3.79 | 3.76 | nd | nd |
| Stdev | nd | nd | 5.02 | 1.92 | nd | nd |
| p(t-test) | nd | nd | | 0.99 | nd | nd |
| Min | nd | nd | 0.000201 | 0.731 | nd | nd |
| Max | nd | nd | 46.1 | 6.35 | nd | nd |
| n (Samp) | nd | nd | 833 | 9 | nd | nd |
| n (Patient) | nd | nd | 311 | 9 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.31 | 1.68 | 2.31 | 3.79 | nd | nd |
| Average | 3.67 | 1.97 | 3.67 | 5.95 | nd | nd |
| Stdev | 4.74 | 2.28 | 4.74 | 6.74 | nd | nd |
| p(t-test) | | 0.34 | | 0.089 | nd | nd |
| Min | 0.000201 | 0.000605 | 0.000201 | 0.000605 | nd | nd |
| Max | 46.1 | 6.80 | 46.1 | 25.2 | nd | nd |
| n (Samp) | 833 | 7 | 833 | 13 | nd | nd |
| n (Patient) | 294 | 7 | 294 | 13 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.45 | nd | 0.37 | 0.62 | 0.63 | 0.63 | 0.42 | nd | nd |
| SE | 0.083 | nd | 0.11 | 0.071 | 0.10 | 0.084 | 0.11 | nd | nd |
| p | 0.57 | nd | 0.27 | 0.10 | 0.21 | 0.12 | 0.45 | nd | nd |
| nCohort 1 | 807 | nd | 833 | 807 | 833 | 833 | 807 | nd | nd |
| nCohort 2 | 13 | nd | 7 | 18 | 9 | 13 | 8 | nd | nd |
| Cutoff 1 | 0.942 | nd | 1.02 | 2.39 | 2.75 | 1.91 | 1.04 | nd | nd |
| Sens 1 | 77% | nd | 71% | 72% | 78% | 77% | 75% | nd | nd |
| Spec 1 | 25% | nd | 27% | 50% | 55% | 45% | 27% | nd | nd |
| Cutoff 2 | 0.886 | nd | 0.261 | 1.30 | 1.53 | 1.30 | 0.147 | nd | nd |
| Sens 2 | 85% | nd | 86% | 83% | 89% | 85% | 88% | nd | nd |
| Spec 2 | 24% | nd | 11% | 33% | 37% | 33% | 9% | nd | nd |
| Cutoff 3 | 0.000506 | nd | 0.000506 | 0.397 | 0.717 | 0.798 | 0 | nd | nd |
| Sens 3 | 92% | nd | 100% | 94% | 100% | 92% | 100% | nd | nd |
| Spec 3 | 4% | nd | 4% | 13% | 20% | 22% | 0% | nd | nd |
| Cutoff 4 | 3.85 | nd | 3.84 | 3.85 | 3.84 | 3.84 | 3.85 | nd | nd |
| Sens 4 | 31% | nd | 14% | 50% | 56% | 46% | 25% | nd | nd |
| Spec 4 | 70% | nd | 70% | 70% | 70% | 70% | 70% | nd | nd |
| Cutoff 5 | 5.38 | nd | 5.18 | 5.38 | 5.37 | 5.18 | 5.38 | nd | nd |
| Sens 5 | 23% | nd | 14% | 33% | 22% | 31% | 12% | nd | nd |
| Spec 5 | 80% | nd | 80% | 80% | 80% | 80% | 80% | nd | nd |
| Cutoff 6 | 8.82 | nd | 8.33 | 8.82 | 8.78 | 8.33 | 8.82 | nd | nd |
| Sens 6 | 8% | nd | 0% | 17% | 0% | 23% | 0% | nd | nd |
| Spec 6 | 90% | nd | 90% | 90% | 90% | 90% | 90% | nd | nd |
| OR Quart 2 | 0.33 | nd | 0 | 1.0 | 1.00 | 1.00 | 2.0 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value | 0.34 | nd | na | 1.0 | 1.00 | 1.00 | 0.57 | nd | nd |
| 95% CI of | 0.034 | nd | na | 0.20 | 0.062 | 0.14 | 0.18 | nd | nd |
| OR Quart2 | 3.2 | nd | na | 5.0 | 16 | 7.1 | 22 | nd | nd |
| OR Quart 3 | 2.0 | nd | 4.1 | 1.7 | 4.1 | 2.0 | 3.0 | nd | nd |
| p Value | 0.32 | nd | 0.21 | 0.48 | 0.21 | 0.42 | 0.34 | nd | nd |
| 95% CI of | 0.50 | nd | 0.45 | 0.40 | 0.45 | 0.37 | 0.31 | nd | nd |
| OR Quart3 | 8.2 | nd | 37 | 7.1 | 37 | 11 | 29 | nd | nd |
| OR Quart 4 | 1.0 | nd | 2.0 | 2.4 | 3.0 | 2.5 | 2.0 | nd | nd |
| p Value | 1.0 | nd | 0.57 | 0.22 | 0.34 | 0.27 | 0.57 | nd | nd |
| 95% CI of | 0.20 | nd | 0.18 | 0.60 | 0.31 | 0.48 | 0.18 | nd | nd |
| OR Quart4 | 5.0 | nd | 22 | 9.3 | 29 | 13 | 22 | nd | nd |

**Transthyretin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 24.7 | 51.6 | 24.7 | 47.5 | 24.7 | 15.5 |
| Average | 53.7 | 101 | 53.7 | 60.5 | 53.7 | 44.6 |
| Stdev | 124 | 121 | 124 | 57.8 | 124 | 70.8 |
| p(t-test) |  | 0.12 |  | 0.81 |  | 0.81 |
| Min | 0.000148 | 3.29 | 0.000148 | 5.08 | 0.000148 | 1.67 |
| Max | 2000 | 461 | 2000 | 238 | 2000 | 241 |
| n (Samp) | 892 | 17 | 892 | 20 | 892 | 11 |
| n (Patient) | 370 | 17 | 370 | 20 | 370 | 11 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 25.7 | 31.4 | 25.7 | 31.1 |
| Average | nd | nd | 55.5 | 42.0 | 55.5 | 70.6 |
| Stdev | nd | nd | 124 | 35.5 | 124 | 79.0 |
| p(t-test) | nd | nd |  | 0.74 |  | 0.73 |
| Min | nd | nd | 0.000148 | 6.54 | 0.000148 | 9.84 |
| Max | nd | nd | 2000 | 127 | 2000 | 241 |
| n (Samp) | nd | nd | 922 | 9 | 922 | 8 |
| n (Patient) | nd | nd | 382 | 9 | 382 | 8 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 25.2 | 61.5 | 25.2 | 50.5 | nd | nd |
| Average | 54.9 | 114 | 54.9 | 63.2 | nd | nd |
| Stdev | 125 | 140 | 125 | 62.7 | nd | nd |
| p(t-test) |  | 0.14 |  | 0.80 | nd | nd |
| Min | 0.000148 | 9.87 | 0.000148 | 5.08 | nd | nd |
| Max | 2000 | 461 | 2000 | 238 | nd | nd |
| n (Samp) | 886 | 10 | 886 | 15 | nd | nd |
| n (Patient) | 345 | 10 | 345 | 15 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.67 | nd | 0.70 | 0.63 | 0.56 | 0.62 | 0.43 | 0.61 | nd |
| SE | 0.073 | nd | 0.093 | 0.068 | 0.100 | 0.078 | 0.090 | 0.11 | nd |
| p | 0.022 | nd | 0.031 | 0.059 | 0.54 | 0.12 | 0.46 | 0.29 | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 892 | nd | 886 | 892 | 922 | 886 | 892 | 922 | nd |
| nCohort 2 | 17 | nd | 10 | 20 | 9 | 15 | 11 | 8 | nd |
| Cutoff 1 | 35.5 | nd | 39.8 | 31.1 | 18.9 | 22.0 | 9.69 | 26.8 | nd |
| Sens 1 | 71% | nd | 70% | 70% | 78% | 73% | 73% | 75% | nd |
| Spec 1 | 63% | nd | 65% | 58% | 40% | 45% | 21% | 51% | nd |
| Cutoff 2 | 12.9 | nd | 35.5 | 16.2 | 17.0 | 19.2 | 8.79 | 15.4 | nd |
| Sens 2 | 82% | nd | 80% | 80% | 89% | 80% | 82% | 88% | nd |
| Spec 2 | 28% | nd | 62% | 35% | 35% | 41% | 19% | 32% | nd |
| Cutoff 3 | 6.65 | nd | 12.9 | 12.4 | 6.53 | 10.7 | 5.71 | 9.69 | nd |
| Sens 3 | 94% | nd | 90% | 90% | 100% | 93% | 91% | 100% | nd |
| Spec 3 | 15% | nd | 27% | 27% | 14% | 23% | 13% | 21% | nd |
| Cutoff 4 | 46.1 | nd | 47.0 | 46.1 | 47.3 | 47.0 | 46.1 | 47.3 | nd |
| Sens 4 | 59% | nd | 60% | 50% | 33% | 53% | 18% | 38% | nd |
| Spec 4 | 70% | nd | 70% | 70% | 70% | 70% | 70% | 70% | nd |
| Cutoff 5 | 63.5 | nd | 65.0 | 63.5 | 66.5 | 65.0 | 63.5 | 66.5 | nd |
| Sens 5 | 47% | nd | 50% | 30% | 11% | 33% | 18% | 38% | nd |
| Spec 5 | 80% | nd | 80% | 80% | 80% | 80% | 80% | 80% | nd |
| Cutoff 6 | 108 | nd | 109 | 108 | 111 | 109 | 108 | 111 | nd |
| Sens 6 | 29% | nd | 30% | 15% | 11% | 13% | 9% | 25% | nd |
| Spec 6 | 90% | nd | 90% | 90% | 90% | 90% | 90% | 90% | nd |
| OR Quart 2 | 0.33 | nd | 1.0 | 2.0 | 2.0 | 1.5 | 1.5 | 1.00 | nd |
| p Value | 0.34 | nd | 1.0 | 0.42 | 0.57 | 0.66 | 0.66 | 1.00 | nd |
| 95% CI of | 0.034 | nd | 0.062 | 0.37 | 0.18 | 0.25 | 0.25 | 0.062 | nd |
| OR Quart2 | 3.2 | nd | 16 | 11 | 22 | 9.1 | 9.1 | 16 | nd |
| OR Quart 3 | 1.7 | nd | 3.0 | 3.6 | 5.1 | 2.5 | 1.0 | 3.0 | nd |
| p Value | 0.48 | nd | 0.34 | 0.11 | 0.14 | 0.27 | 1.0 | 0.34 | nd |
| 95% CI of | 0.40 | nd | 0.31 | 0.74 | 0.59 | 0.49 | 0.14 | 0.31 | nd |
| OR Quart3 | 7.1 | nd | 29 | 17 | 44 | 13 | 7.2 | 29 | nd |
| OR Quart 4 | 2.7 | nd | 5.1 | 3.6 | 1.00 | 2.5 | 2.0 | 3.0 | nd |
| p Value | 0.14 | nd | 0.14 | 0.11 | 1.00 | 0.27 | 0.42 | 0.34 | nd |
| 95% CI of | 0.71 | nd | 0.59 | 0.74 | 0.062 | 0.48 | 0.37 | 0.31 | nd |
| OR Quart4 | 10 | nd | 44 | 17 | 16 | 13 | 11 | 29 | nd |

**C-peptide Urine**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22200 | 6700 | 22200 | 14400 | 22200 | 22000 |
| Average | 32300 | 16200 | 32300 | 22300 | 32300 | 33900 |
| Stdev | 29300 | 24400 | 29300 | 25900 | 29300 | 31600 |
| p(t-test) | | 0.024 | | 0.13 | | 0.86 |
| Min | 0.00978 | 734 | 0.00978 | 419 | 0.00978 | 936 |
| Max | 114000 | 100000 | 114000 | 100000 | 114000 | 100000 |
| n (Samp) | 893 | 17 | 893 | 20 | 893 | 11 |
| n (Patient) | 370 | 17 | 370 | 20 | 370 | 11 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 21700 | 18000 | 21700 | 14600 |
| Average | nd | nd | 31400 | 24100 | 31400 | 28200 |
| Stdev | nd | nd | 29200 | 17800 | 29200 | 33300 |
| p(t-test) | nd | nd | | 0.45 | | 0.75 |
| Min | nd | nd | 0.00978 | 4640 | 0.00978 | 936 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | nd | nd | 114000 | 59400 | 114000 | 100000 |
| n (Samp) | nd | nd | 923 | 9 | 923 | 8 |
| n (Patient) | nd | nd | 382 | 9 | 382 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22000 | 3460 | 22000 | 5980 | nd | nd |
| Average | 31900 | 14600 | 31900 | 16800 | nd | nd |
| Stdev | 29100 | 26900 | 29100 | 25300 | nd | nd |
| p(t-test) | | 0.062 | | 0.046 | nd | nd |
| Min | 0.00978 | 734 | 0.00978 | 419 | nd | nd |
| Max | 114000 | 87500 | 114000 | 99100 | nd | nd |
| n (Samp) | 887 | 10 | 887 | 15 | nd | nd |
| n (Patient) | 345 | 10 | 345 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.29 | nd | 0.23 | 0.38 | 0.48 | 0.29 | 0.50 | 0.44 | nd |
| SE | 0.071 | nd | 0.088 | 0.068 | 0.098 | 0.076 | 0.088 | 0.11 | nd |
| p | 0.0025 | nd | 0.0026 | 0.066 | 0.82 | 0.0063 | 0.98 | 0.54 | nd |
| nCohort 1 | 893 | nd | 887 | 893 | 923 | 887 | 893 | 923 | nd |
| nCohort 2 | 17 | nd | 10 | 20 | 9 | 15 | 11 | 8 | nd |
| Cutoff 1 | 2160 | nd | 2000 | 5480 | 12500 | 1870 | 7980 | 5860 | nd |
| Sens 1 | 71% | nd | 70% | 70% | 78% | 73% | 73% | 75% | nd |
| Spec 1 | 6% | nd | 6% | 14% | 36% | 6% | 22% | 16% | nd |
| Cutoff 2 | 1580 | nd | 1580 | 2700 | 10800 | 1460 | 5980 | 4910 | nd |
| Sens 2 | 82% | nd | 80% | 80% | 89% | 80% | 82% | 88% | nd |
| Spec 2 | 4% | nd | 5% | 7% | 31% | 4% | 15% | 14% | nd |
| Cutoff 3 | 1240 | nd | 1240 | 1400 | 4620 | 936 | 4100 | 800 | nd |
| Sens 3 | 94% | nd | 90% | 90% | 100% | 93% | 91% | 100% | nd |
| Spec 3 | 3% | nd | 4% | 4% | 13% | 3% | 10% | 3% | nd |
| Cutoff 4 | 40700 | nd | 39400 | 40700 | 38600 | 39400 | 40700 | 38600 | nd |
| Sens 4 | 6% | nd | 10% | 15% | 22% | 7% | 36% | 38% | nd |
| Spec 4 | 70% | nd | 70% | 70% | 70% | 70% | 70% | 70% | nd |
| Cutoff 5 | 58100 | nd | 56700 | 58100 | 56900 | 56700 | 58100 | 56900 | nd |
| Sens 5 | 6% | nd | 10% | 15% | 11% | 7% | 27% | 12% | nd |
| Spec 5 | 80% | nd | 80% | 80% | 80% | 80% | 80% | 80% | nd |
| Cutoff 6 | 83300 | nd | 83300 | 83300 | 82300 | 83300 | 83300 | 82300 | nd |
| Sens 6 | 6% | nd | 10% | 5% | 0% | 7% | 9% | 12% | nd |
| Spec 6 | 90% | nd | 90% | 90% | 90% | 90% | 90% | 90% | nd |
| OR Quart 2 | 3.0 | nd | 1.0 | 1.0 | 2.0 | 3.0 | 0.50 | 2.0 | nd |
| p Value | 0.34 | nd | 1.00 | 1.00 | 0.57 | 0.34 | 0.42 | 0.57 | nd |
| 95% CI of OR Quart2 | 0.31 29 | nd | 0.062 16 | 0.20 5.0 | 0.18 22 | 0.31 29 | 0.090 2.7 | 0.18 22 | nd |
| OR Quart 3 | 3.0 | nd | 1.0 | 2.0 | 5.1 | 3.0 | 0.50 | 1.0 | nd |
| p Value | 0.34 | nd | 1.00 | 0.32 | 0.14 | 0.34 | 0.42 | 1.0 | nd |
| 95% CI of OR Quart3 | 0.31 29 | nd | 0.062 16 | 0.50 8.2 | 0.59 44 | 0.31 29 | 0.090 2.7 | 0.062 16 | nd |
| OR Quart 4 | 10 | nd | 7.2 | 2.7 | 1.0 | 8.3 | 0.75 | 4.1 | nd |
| p Value | 0.026 | nd | 0.065 | 0.14 | 1.0 | 0.047 | 0.70 | 0.21 | nd |
| 95% CI of OR Quart4 | 1.3 82 | nd | 0.88 59 | 0.72 10 | 0.062 16 | 1.0 67 | 0.17 3.4 | 0.45 37 | nd |

**Gastric inhibitory polypeptide  Urine**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.376 | 0.262 | 0.376 | 0.626 | 0.376 | 1.39 |
| Average | 23.7 | 38.1 | 23.7 | 64.2 | 23.7 | 3.44 |
| Stdev | 209 | 147 | 209 | 232 | 209 | 4.64 |
| p(t-test) | | 0.78 | | 0.39 | | 0.75 |
| Min | 9.60E-5 | 9.60E-5 | 9.60E-5 | 0.0273 | 9.60E-5 | 0.137 |
| Max | 3840 | 606 | 3840 | 1040 | 3840 | 12.7 |
| n (Samp) | 894 | 17 | 894 | 20 | 894 | 11 |
| n (Patient) | 370 | 17 | 370 | 20 | 370 | 11 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.372 | 0.815 | 0.372 | 2.07 |
| Average | nd | nd | 24.2 | 76.5 | 24.2 | 8.10 |
| Stdev | nd | nd | 209 | 199 | 209 | 11.7 |
| p(t-test) | nd | nd | | 0.45 | | 0.83 |
| Min | nd | nd | 9.60E-5 | 0.0273 | 9.60E-5 | 0.137 |
| Max | nd | nd | 3840 | 606 | 3840 | 34.1 |
| n (Samp) | nd | nd | 924 | 9 | 924 | 8 |
| n (Patient) | nd | nd | 382 | 9 | 382 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.397 | 0.228 | 0.397 | 0.990 | nd | nd |
| Average | 23.7 | 3.88 | 23.7 | 81.3 | nd | nd |
| Stdev | 210 | 11.7 | 210 | 268 | nd | nd |
| p(t-test) | | 0.76 | | 0.30 | nd | nd |
| Min | 9.60E-5 | 0.000104 | 9.60E-5 | 0.109 | nd | nd |
| Max | 3840 | 37.2 | 3840 | 1040 | nd | nd |
| n (Samp) | 888 | 10 | 888 | 15 | nd | nd |
| n (Patient) | 345 | 10 | 345 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.44 | nd | 0.35 | 0.63 | 0.68 | 0.66 | 0.68 | 0.76 | nd |
| SE | 0.073 | nd | 0.095 | 0.068 | 0.099 | 0.077 | 0.090 | 0.100 | nd |
| p | 0.39 | nd | 0.10 | 0.046 | 0.068 | 0.034 | 0.045 | 0.0098 | nd |
| nCohort 1 | 894 | nd | 888 | 894 | 924 | 888 | 894 | 924 | nd |
| nCohort 2 | 17 | nd | 10 | 20 | 9 | 15 | 11 | 8 | nd |
| Cutoff 1 | 0.0766 | nd | 0.0766 | 0.304 | 0.393 | 0.304 | 0.462 | 1.09 | nd |
| Sens 1 | 71% | nd | 70% | 70% | 78% | 73% | 73% | 75% | nd |
| Spec 1 | 25% | nd | 24% | 45% | 51% | 43% | 55% | 75% | nd |
| Cutoff 2 | 0.00847 | nd | 0.000161 | 0.206 | 0.108 | 0.261 | 0.246 | 0.462 | nd |
| Sens 2 | 82% | nd | 80% | 80% | 89% | 80% | 82% | 88% | nd |
| Spec 2 | 18% | nd | 7% | 36% | 28% | 41% | 41% | 55% | nd |
| Cutoff 3 | 9.60E-5 | nd | 9.60E-5 | 0.108 | 0.0269 | 0.119 | 0.222 | 0.136 | nd |
| Sens 3 | 94% | nd | 100% | 90% | 100% | 93% | 91% | 100% | nd |
| Spec 3 | 0% | nd | 0% | 27% | 21% | 28% | 37% | 31% | nd |
| Cutoff 4 | 0.828 | nd | 0.880 | 0.828 | 0.828 | 0.880 | 0.828 | 0.828 | nd |
| Sens 4 | 24% | nd | 10% | 45% | 44% | 53% | 55% | 75% | nd |
| Spec 4 | 70% | nd | 70% | 70% | 70% | 70% | 70% | 70% | nd |
| Cutoff 5 | 1.58 | nd | 1.58 | 1.58 | 1.58 | 1.58 | 1.58 | 1.58 | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 5 | 12% | nd | 10% | 40% | 44% | 40% | 45% | 50% | nd |
| Spec 5 | 80% | nd | 80% | 80% | 80% | 80% | 80% | 80% | nd |
| Cutoff 6 | 5.82 | nd | 5.85 | 5.82 | 5.66 | 5.85 | 5.82 | 5.66 | nd |
| Sens 6 | 12% | nd | 10% | 25% | 44% | 20% | 18% | 38% | nd |
| Spec 6 | 90% | nd | 90% | 90% | 90% | 90% | 90% | 90% | nd |
| OR Quart 2 | 2.0 | nd | 1.0 | 2.5 | 1.0 | >5.1 | >3.0 | >1.0 | nd |
| p Value | 0.42 | nd | 1.00 | 0.27 | 1.0 | <0.14 | <0.34 | <1.00 | nd |
| 95% CI of | 0.37 | nd | 0.062 | 0.48 | 0.062 | >0.59 | >0.31 | >0.062 | nd |
| OR Quart2 | 11 | nd | 16 | 13 | 16 | na | na | na | nd |
| OR Quart 3 | 3.1 | nd | 4.1 | 2.0 | 3.0 | >3.0 | >2.0 | >2.0 | nd |
| p Value | 0.17 | nd | 0.21 | 0.42 | 0.34 | <0.34 | <0.57 | <0.57 | nd |
| 95% CI of | 0.61 | nd | 0.45 | 0.37 | 0.31 | >0.31 | >0.18 | >0.18 | nd |
| OR Quart3 | 15 | nd | 37 | 11 | 29 | na | na | na | nd |
| OR Quart 4 | 2.5 | nd | 4.1 | 4.6 | 4.0 | >7.2 | >6.1 | >5.1 | nd |
| p Value | 0.27 | nd | 0.21 | 0.052 | 0.21 | <0.066 | <0.094 | <0.14 | nd |
| 95% CI of | 0.49 | nd | 0.45 | 0.99 | 0.45 | >0.88 | >0.73 | >0.59 | nd |
| OR Quart4 | 13 | nd | 37 | 22 | 36 | na | na | na | nd |

**Peptide YY Urine**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 5.26 | 14.6 | 5.26 | 22.7 | 5.26 | 0.957 |
| Average | 43.1 | 44.4 | 43.1 | 78.3 | 43.1 | 46.1 |
| Stdev | 115 | 61.7 | 115 | 105 | 115 | 86.4 |
| p(t-test) |  | 0.96 |  | 0.17 |  | 0.93 |
| Min | 0.00976 | 0.0188 | 0.00976 | 0.00976 | 0.00976 | 0.0188 |
| Max | 1200 | 196 | 1200 | 317 | 1200 | 290 |
| n (Samp) | 892 | 17 | 892 | 20 | 892 | 11 |
| n (Patient) | 370 | 17 | 370 | 20 | 370 | 11 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 5.61 | 98.0 | 5.61 | 37.7 |
| Average | nd | nd | 43.7 | 102 | 43.7 | 41.4 |
| Stdev | nd | nd | 114 | 93.9 | 114 | 35.4 |
| p(t-test) | nd | nd |  | 0.13 |  | 0.95 |
| Min | nd | nd | 0.00976 | 0.00976 | 0.00976 | 0.0188 |
| Max | nd | nd | 1200 | 278 | 1200 | 82.4 |
| n (Samp) | nd | nd | 922 | 9 | 922 | 8 |
| n (Patient) | nd | nd | 382 | 9 | 382 | 8 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 6.34 | 21.5 | 6.34 | 18.9 | nd | nd |
| Average | 43.5 | 30.3 | 43.5 | 72.2 | nd | nd |
| Stdev | 114 | 46.0 | 114 | 104 | nd | nd |
| p(t-test) |  | 0.71 |  | 0.33 | nd | nd |
| Min | 0.00976 | 0.0128 | 0.00976 | 0.0188 | nd | nd |
| Max | 1200 | 153 | 1200 | 317 | nd | nd |
| n (Samp) | 886 | 10 | 886 | 15 | nd | nd |
| n (Patient) | 345 | 10 | 345 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | nd | 0.54 | 0.65 | 0.71 | 0.65 | 0.52 | 0.64 | nd |
| SE | 0.073 | nd | 0.094 | 0.067 | 0.098 | 0.078 | 0.089 | 0.11 | nd |
| p | 0.10 | nd | 0.68 | 0.028 | 0.033 | 0.056 | 0.82 | 0.19 | nd |
| nCohort 1 | 892 | nd | 886 | 892 | 922 | 886 | 892 | 922 | nd |
| nCohort 2 | 17 | nd | 10 | 20 | 9 | 15 | 11 | 8 | nd |
| Cutoff 1 | 10.7 | nd | 0.817 | 3.26 | 25.9 | 3.26 | 0.0196 | 18.8 | nd |
| Sens 1 | 71% | nd | 70% | 70% | 78% | 73% | 73% | 75% | nd |
| Spec 1 | 55% | nd | 40% | 49% | 69% | 48% | 26% | 62% | nd |
| Cutoff 2 | 0.817 | nd | 0.0196 | 0.963 | 0.0151 | 2.77 | 0.0151 | 0.0151 | nd |
| Sens 2 | 82% | nd | 80% | 80% | 89% | 80% | 100% | 100% | nd |
| Spec 2 | 40% | nd | 27% | 43% | 18% | 47% | 18% | 18% | nd |
| Cutoff 3 | 0.0196 | nd | 0.0128 | 0.0188 | 0 | 0.0188 | 0.0151 | 0.0151 | nd |
| Sens 3 | 94% | nd | 90% | 90% | 100% | 93% | 100% | 100% | nd |
| Spec 3 | 26% | nd | 12% | 24% | 0% | 26% | 18% | 18% | nd |
| Cutoff 4 | 27.1 | nd | 28.3 | 27.1 | 27.9 | 28.3 | 27.1 | 27.9 | nd |
| Sens 4 | 47% | nd | 50% | 45% | 67% | 40% | 36% | 50% | nd |
| Spec 4 | 70% | nd | 70% | 70% | 70% | 70% | 70% | 70% | nd |
| Cutoff 5 | 44.2 | nd | 47.0 | 44.2 | 45.6 | 47.0 | 44.2 | 45.6 | nd |
| Sens 5 | 24% | nd | 10% | 45% | 67% | 40% | 27% | 50% | nd |
| Spec 5 | 80% | nd | 80% | 80% | 80% | 80% | 80% | 80% | nd |
| Cutoff 6 | 103 | nd | 104 | 103 | 104 | 104 | 103 | 104 | nd |
| Sens 6 | 18% | nd | 10% | 25% | 44% | 20% | 9% | 0% | nd |
| Spec 6 | 90% | nd | 90% | 90% | 90% | 90% | 90% | 90% | nd |
| OR Quart 2 | 4.1 | nd | 1.0 | 2.5 | 0 | 4.1 | 1.00 | 0 | nd |
| p Value | 0.21 | nd | 1.0 | 0.27 | na | 0.21 | 1.00 | na | nd |
| 95% CI of | 0.45 | nd | 0.14 | 0.49 | na | 0.45 | 0.20 | na | nd |
| OR Quart2 | 37 | nd | 7.2 | 13 | na | 37 | 5.0 | na | nd |
| OR Quart 3 | 7.2 | nd | 2.0 | 2.0 | 0.50 | 4.1 | 0.33 | 1.0 | nd |
| p Value | 0.066 | nd | 0.42 | 0.42 | 0.57 | 0.21 | 0.34 | 1.0 | nd |
| 95% CI of | 0.88 | nd | 0.37 | 0.37 | 0.045 | 0.45 | 0.034 | 0.14 | nd |
| OR Quart3 | 59 | nd | 11 | 11 | 5.5 | 37 | 3.2 | 7.2 | nd |
| OR Quart 4 | 5.1 | nd | 1.0 | 4.6 | 3.0 | 6.1 | 1.3 | 2.0 | nd |
| p Value | 0.14 | nd | 1.0 | 0.051 | 0.18 | 0.095 | 0.71 | 0.42 | nd |
| 95% CI of | 0.59 | nd | 0.14 | 0.99 | 0.61 | 0.73 | 0.29 | 0.36 | nd |
| OR Quart4 | 44 | nd | 7.2 | 22 | 15 | 51 | 6.0 | 11 | nd |

Table 10: Comparison of marker levels in EDTA samples collected from Cohort 1 (patients that did not progress beyond RIFLE stage 0, R, or I) and in EDTA samples collected from Cohort 2 (subjects who progress to RIFLE stage F) at 0, 24 hours, and 48 hours prior to the subject reaching RIFLE stage I.

**Complement factor H**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 183000 | 169000 | nd | nd |
| Average | nd | nd | 191000 | 171000 | nd | nd |
| Stdev | nd | nd | 76200 | 64200 | nd | nd |
| p(t-test) | nd | nd | | 0.52 | nd | nd |
| Min | nd | nd | 14900 | 92800 | nd | nd |
| Max | nd | nd | 1070000 | 255000 | nd | nd |
| n (Samp) | nd | nd | 488 | 6 | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | nd | nd | 222 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.43 | nd | nd | nd | nd | nd |
| SE | nd | nd | nd | 0.12 | nd | nd | nd | nd | nd |
| p | nd | nd | nd | 0.57 | nd | nd | nd | nd | nd |
| nCohort 1 | nd | nd | nd | 488 | nd | nd | nd | nd | nd |
| nCohort 2 | nd | nd | nd | 6 | nd | nd | nd | nd | nd |
| Cutoff 1 | nd | nd | nd | 112000 | nd | nd | nd | nd | nd |
| Sens 1 | nd | nd | nd | 83% | nd | nd | nd | nd | nd |
| Spec 1 | nd | nd | nd | 9% | nd | nd | nd | nd | nd |
| Cutoff 2 | nd | nd | nd | 112000 | nd | nd | nd | nd | nd |
| Sens 2 | nd | nd | nd | 83% | nd | nd | nd | nd | nd |
| Spec 2 | nd | nd | nd | 9% | nd | nd | nd | nd | nd |
| Cutoff 3 | nd | nd | nd | 92200 | nd | nd | nd | nd | nd |
| Sens 3 | nd | nd | nd | 100% | nd | nd | nd | nd | nd |
| Spec 3 | nd | nd | nd | 5% | nd | nd | nd | nd | nd |
| Cutoff 4 | nd | nd | nd | 213000 | nd | nd | nd | nd | nd |
| Sens 4 | nd | nd | nd | 33% | nd | nd | nd | nd | nd |
| Spec 4 | nd | nd | nd | 70% | nd | nd | nd | nd | nd |
| Cutoff 5 | nd | nd | nd | 236000 | nd | nd | nd | nd | nd |
| Sens 5 | nd | nd | nd | 17% | nd | nd | nd | nd | nd |
| Spec 5 | nd | nd | nd | 80% | nd | nd | nd | nd | nd |
| Cutoff 6 | nd | nd | nd | 273000 | nd | nd | nd | nd | nd |
| Sens 6 | nd | nd | nd | 0% | nd | nd | nd | nd | nd |
| Spec 6 | nd | nd | nd | 90% | nd | nd | nd | nd | nd |
| OR Quart 2 | nd | nd | nd | 0.50 | nd | nd | nd | nd | nd |
| p Value | nd | nd | nd | 0.57 | nd | nd | nd | nd | nd |
| 95% CI of OR Quart2 | nd | nd | nd | 0.045 | nd | nd | nd | nd | nd |
| | nd | nd | nd | 5.6 | nd | nd | nd | nd | nd |
| OR Quart 3 | nd | nd | nd | 0.50 | nd | nd | nd | nd | nd |
| p Value | nd | nd | nd | 0.57 | nd | nd | nd | nd | nd |
| 95% CI of OR Quart3 | nd | nd | nd | 0.044 | nd | nd | nd | nd | nd |
| | nd | nd | nd | 5.5 | nd | nd | nd | nd | nd |
| OR Quart 4 | nd | nd | nd | 1.0 | nd | nd | nd | nd | nd |
| p Value | nd | nd | nd | 0.99 | nd | nd | nd | nd | nd |
| 95% CI of OR Quart4 | nd | nd | nd | 0.14 | nd | nd | nd | nd | nd |
| | nd | nd | nd | 7.3 | nd | nd | nd | nd | nd |

**Follistatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 796 | 942 | 796 | 736 | 796 | 529 |
| Average | 2910 | 3920 | 2910 | 2380 | 2910 | 754 |
| Stdev | 4540 | 4600 | 4540 | 3980 | 4540 | 759 |
| p(t-test) | | 0.56 | | 0.73 | | 0.21 |
| Min | 0.000554 | 2.75 | 0.000554 | 570 | 0.000554 | 1.35 |
| Max | 28900 | 12100 | 28900 | 12800 | 28900 | 2290 |
| n (Samp) | 588 | 7 | 588 | 9 | 588 | 7 |
| n (Patient) | 263 | 7 | 263 | 9 | 263 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 778 | 781 | nd | nd |
| Average | nd | nd | 3000 | 2280 | nd | nd |
| Stdev | nd | nd | 4620 | 4260 | nd | nd |
| p(t-test) | nd | nd | | 0.66 | nd | nd |
| Min | nd | nd | 0.000554 | 366 | nd | nd |
| Max | nd | nd | 28900 | 12800 | nd | nd |
| n (Samp) | nd | nd | 571 | 8 | nd | nd |
| n (Patient) | nd | nd | 243 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | nd | nd | 0.56 | nd | 0.54 | 0.36 | nd | nd |
| SE | 0.11 | nd | nd | 0.10 | nd | 0.10 | 0.11 | nd | nd |
| p | 0.59 | nd | nd | 0.52 | nd | 0.74 | 0.23 | nd | nd |
| nCohort 1 | 588 | nd | nd | 588 | nd | 571 | 588 | nd | nd |
| nCohort 2 | 7 | nd | nd | 9 | nd | 8 | 7 | nd | nd |
| Cutoff 1 | 839 | nd | nd | 702 | nd | 702 | 394 | nd | nd |
| Sens 1 | 71% | nd | nd | 78% | nd | 75% | 71% | nd | nd |
| Spec 1 | 54% | nd | nd | 45% | nd | 46% | 21% | nd | nd |
| Cutoff 2 | 441 | nd | nd | 594 | nd | 563 | 240 | nd | nd |
| Sens 2 | 86% | nd | nd | 89% | nd | 88% | 86% | nd | nd |
| Spec 2 | 24% | nd | nd | 36% | nd | 37% | 13% | nd | nd |
| Cutoff 3 | 2.51 | nd | nd | 563 | nd | 360 | 1.29 | nd | nd |
| Sens 3 | 100% | nd | nd | 100% | nd | 100% | 100% | nd | nd |
| Spec 3 | 4% | nd | nd | 34% | nd | 21% | 2% | nd | nd |
| Cutoff 4 | 1320 | nd | nd | 1320 | nd | 1370 | 1320 | nd | nd |
| Sens 4 | 43% | nd | nd | 22% | nd | 12% | 14% | nd | nd |
| Spec 4 | 70% | nd | nd | 70% | nd | 70% | 70% | nd | nd |
| Cutoff 5 | 6590 | nd | nd | 6590 | nd | 7330 | 6590 | nd | nd |
| Sens 5 | 29% | nd | nd | 11% | nd | 12% | 0% | nd | nd |
| Spec 5 | 80% | nd | nd | 80% | nd | 80% | 80% | nd | nd |
| Cutoff 6 | 10800 | nd | nd | 10800 | nd | 10900 | 10800 | nd | nd |
| Sens 6 | 14% | nd | nd | 11% | nd | 12% | 0% | nd | nd |
| Spec 6 | 90% | nd | nd | 90% | nd | 90% | 90% | nd | nd |
| OR Quart 2 | 0 | nd | nd | >5.2 | nd | 3.0 | 1.0 | nd | nd |
| p Value | na | nd | nd | <0.14 | nd | 0.34 | 1.0 | nd | nd |
| 95% CI of OR Quart2 | na | nd | nd | >0.60 | nd | 0.31 | 0.062 | nd | nd |
| | na | nd | nd | na | nd | 29 | 16 | nd | nd |
| OR Quart 3 | 0.99 | nd | nd | >2.0 | nd | 3.0 | 2.0 | nd | nd |
| p Value | 0.99 | nd | nd | <0.57 | nd | 0.34 | 0.57 | nd | nd |
| 95% CI of OR Quart3 | 0.14 | nd | nd | >0.18 | nd | 0.31 | 0.18 | nd | nd |
| | 7.1 | nd | nd | na | nd | 29 | 22 | nd | nd |
| OR Quart 4 | 1.5 | nd | nd | >2.0 | nd | 0.99 | 3.1 | nd | nd |
| p Value | 0.66 | nd | nd | <0.57 | nd | 1.00 | 0.33 | nd | nd |
| 95% CI of OR Quart4 | 0.25 | nd | nd | >0.18 | nd | 0.062 | 0.31 | nd | nd |
| | 9.1 | nd | nd | na | nd | 16 | 30 | nd | nd |

**Vitamin D-binding protein**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 172000 | 102000 | nd | nd |
| Average | nd | nd | 193000 | 115000 | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | nd | nd | 92800 | 54200 | nd | nd |
| p(t-test) | nd | nd | | 0.042 | nd | nd |
| Min | nd | nd | 22500 | 54200 | nd | nd |
| Max | nd | nd | 630000 | 183000 | nd | nd |
| n (Samp) | nd | nd | 489 | 6 | nd | nd |
| n (Patient) | nd | nd | 218 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.23 | nd | nd | nd | nd | nd |
| SE | nd | nd | nd | 0.11 | nd | nd | nd | nd | nd |
| p | nd | nd | nd | 0.016 | nd | nd | nd | nd | nd |
| nCohort 1 | nd | nd | nd | 489 | nd | nd | nd | nd | nd |
| nCohort 2 | nd | nd | nd | 6 | nd | nd | nd | nd | nd |
| Cutoff 1 | nd | nd | nd | 68600 | nd | nd | nd | nd | nd |
| Sens 1 | nd | nd | nd | 83% | nd | nd | nd | nd | nd |
| Spec 1 | nd | nd | nd | 2% | nd | nd | nd | nd | nd |
| Cutoff 2 | nd | nd | nd | 68600 | nd | nd | nd | nd | nd |
| Sens 2 | nd | nd | nd | 83% | nd | nd | nd | nd | nd |
| Spec 2 | nd | nd | nd | 2% | nd | nd | nd | nd | nd |
| Cutoff 3 | nd | nd | nd | 50900 | nd | nd | nd | nd | nd |
| Sens 3 | nd | nd | nd | 100% | nd | nd | nd | nd | nd |
| Spec 3 | nd | nd | nd | 1% | nd | nd | nd | nd | nd |
| Cutoff 4 | nd | nd | nd | 215000 | nd | nd | nd | nd | nd |
| Sens 4 | nd | nd | nd | 0% | nd | nd | nd | nd | nd |
| Spec 4 | nd | nd | nd | 70% | nd | nd | nd | nd | nd |
| Cutoff 5 | nd | nd | nd | 249000 | nd | nd | nd | nd | nd |
| Sens 5 | nd | nd | nd | 0% | nd | nd | nd | nd | nd |
| Spec 5 | nd | nd | nd | 80% | nd | nd | nd | nd | nd |
| Cutoff 6 | nd | nd | nd | 304000 | nd | nd | nd | nd | nd |
| Sens 6 | nd | nd | nd | 0% | nd | nd | nd | nd | nd |
| Spec 6 | nd | nd | nd | 90% | nd | nd | nd | nd | nd |
| OR Quart 2 | nd | nd | nd | >2.0 | nd | nd | nd | nd | nd |
| p Value | nd | nd | nd | <0.56 | nd | nd | nd | nd | nd |
| 95% CI of OR Quart2 | nd | nd | nd | >0.18 na | nd | nd | nd | nd | nd |
| OR Quart 3 | nd | nd | nd | >0 | nd | nd | nd | nd | nd |
| p Value | nd | nd | nd | <na | nd | nd | nd | nd | nd |
| 95% CI of OR Quart3 | nd | nd | nd | >na na | nd | nd | nd | nd | nd |
| OR Quart 4 | nd | nd | nd | >4.2 | nd | nd | nd | nd | nd |
| p Value | nd | nd | nd | <0.20 | nd | nd | nd | nd | nd |
| 95% CI of OR Quart4 | nd | nd | nd | >0.46 na | nd | nd | nd | nd | nd |

**Interleukin-17A**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.59 | 3.29 | 1.59 | 2.67 | 1.59 | 5.39 |
| Average | 3.15 | 9.06 | 3.15 | 7.88 | 3.15 | 9.95 |
| Stdev | 5.05 | 19.9 | 5.05 | 8.58 | 5.05 | 9.99 |
| p(t-test) | | 0.0017 | | 0.0062 | | 5.4E-4 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.000362 | 0.000482 | 0.000362 | 0.901 | 0.000362 | 1.18 |
| Max | 53.4 | 61.7 | 53.4 | 24.2 | 53.4 | 28.6 |
| n (Samp) | 532 | 9 | 532 | 9 | 532 | 7 |
| n (Patient) | 238 | 9 | 238 | 9 | 238 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.51 | 5.48 | nd | nd |
| Average | nd | nd | 3.10 | 8.82 | nd | nd |
| Stdev | nd | nd | 5.02 | 8.77 | nd | nd |
| p(t-test) | nd | nd | | 0.0017 | nd | nd |
| Min | nd | nd | 0.000362 | 0.000532 | nd | nd |
| Max | nd | nd | 53.4 | 24.2 | nd | nd |
| n (Samp) | nd | nd | 525 | 8 | nd | nd |
| n (Patient) | nd | nd | 224 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | nd | nd | 0.73 | nd | 0.74 | 0.80 | nd | nd |
| SE | 0.100 | nd | nd | 0.097 | nd | 0.10 | 0.10 | nd | nd |
| p | 0.61 | nd | nd | 0.020 | nd | 0.017 | 0.0035 | nd | nd |
| nCohort 1 | 532 | nd | nd | 532 | nd | 525 | 532 | nd | nd |
| nCohort 2 | 9 | nd | nd | 9 | nd | 8 | 7 | nd | nd |
| Cutoff 1 | 0.490 | nd | nd | 2.07 | nd | 2.66 | 3.84 | nd | nd |
| Sens 1 | 78% | nd | nd | 78% | nd | 75% | 71% | nd | nd |
| Spec 1 | 29% | nd | nd | 57% | nd | 66% | 75% | nd | nd |
| Cutoff 2 | 0.000482 | nd | nd | 1.80 | nd | 2.07 | 2.82 | nd | nd |
| Sens 2 | 89% | nd | nd | 89% | nd | 88% | 86% | nd | nd |
| Spec 2 | 10% | nd | nd | 53% | nd | 58% | 66% | nd | nd |
| Cutoff 3 | 0.000469 | nd | nd | 0.857 | nd | 0.000482 | 1.17 | nd | nd |
| Sens 3 | 100% | nd | nd | 100% | nd | 100% | 100% | nd | nd |
| Spec 3 | 7% | nd | nd | 38% | nd | 10% | 43% | nd | nd |
| Cutoff 4 | 3.30 | nd | nd | 3.30 | nd | 3.29 | 3.30 | nd | nd |
| Sens 4 | 44% | nd | nd | 44% | nd | 62% | 71% | nd | nd |
| Spec 4 | 70% | nd | nd | 70% | nd | 70% | 70% | nd | nd |
| Cutoff 5 | 4.67 | nd | nd | 4.67 | nd | 4.86 | 4.67 | nd | nd |
| Sens 5 | 33% | nd | nd | 44% | nd | 62% | 57% | nd | nd |
| Spec 5 | 80% | nd | nd | 80% | nd | 80% | 80% | nd | nd |
| Cutoff 6 | 7.86 | nd | nd | 7.86 | nd | 7.86 | 7.86 | nd | nd |
| Sens 6 | 11% | nd | nd | 33% | nd | 38% | 43% | nd | nd |
| Spec 6 | 90% | nd | nd | 90% | nd | 90% | 90% | nd | nd |
| OR Quart 2 | 1.0 | nd | nd | >1.0 | nd | 0 | >1.0 | nd | nd |
| p Value | 1.0 | nd | nd | <1.00 | nd | na | <1.0 | nd | nd |
| 95% CI of | 0.14 | nd | nd | >0.062 | nd | na | >0.062 | nd | nd |
| OR Quart2 | 7.2 | nd | nd | na | nd | na | na | nd | nd |
| OR Quart 3 | 1.0 | nd | nd | >4.1 | nd | 2.0 | >2.0 | nd | nd |
| p Value | 1.0 | nd | nd | <0.21 | nd | 0.57 | <0.57 | nd | nd |
| 95% CI of | 0.14 | nd | nd | >0.45 | nd | 0.18 | >0.18 | nd | nd |
| OR Quart3 | 7.2 | nd | nd | na | nd | 22 | na | nd | nd |
| OR Quart 4 | 1.5 | nd | nd | >4.1 | nd | 5.1 | >4.1 | nd | nd |
| p Value | 0.66 | nd | nd | <0.21 | nd | 0.14 | <0.21 | nd | nd |
| 95% CI of | 0.25 | nd | nd | >0.45 | nd | 0.59 | >0.45 | nd | nd |
| OR Quart4 | 9.1 | nd | nd | na | nd | 44 | na | nd | nd |

**Insulin receptor substrate 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.0289 | 0.170 | nd | nd |
| Average | nd | nd | 0.476 | 0.352 | nd | nd |
| Stdev | nd | nd | 3.12 | 0.380 | nd | nd |
| p(t-test) | nd | nd | | 0.92 | nd | nd |
| Min | nd | nd | 3.03E-6 | 0.0217 | nd | nd |
| Max | nd | nd | 49.5 | 0.934 | nd | nd |
| n (Samp) | nd | nd | 490 | 6 | nd | nd |
| n (Patient) | nd | nd | 220 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.77 | nd | nd | nd | nd | nd |
| SE | nd | nd | nd | 0.11 | nd | nd | nd | nd | nd |
| p | nd | nd | nd | 0.020 | nd | nd | nd | nd | nd |
| nCohort 1 | nd | nd | nd | 490 | nd | nd | nd | nd | nd |
| nCohort 2 | nd | nd | nd | 6 | nd | nd | nd | nd | nd |
| Cutoff 1 | nd | nd | nd | 0.0883 | nd | nd | nd | nd | nd |
| Sens 1 | nd | nd | nd | 83% | nd | nd | nd | nd | nd |
| Spec 1 | nd | nd | nd | 72% | nd | nd | nd | nd | nd |
| Cutoff 2 | nd | nd | nd | 0.0883 | nd | nd | nd | nd | nd |
| Sens 2 | nd | nd | nd | 83% | nd | nd | nd | nd | nd |
| Spec 2 | nd | nd | nd | 72% | nd | nd | nd | nd | nd |
| Cutoff 3 | nd | nd | nd | 0.0216 | nd | nd | nd | nd | nd |
| Sens 3 | nd | nd | nd | 100% | nd | nd | nd | nd | nd |
| Spec 3 | nd | nd | nd | 42% | nd | nd | nd | nd | nd |
| Cutoff 4 | nd | nd | nd | 0.0807 | nd | nd | nd | nd | nd |
| Sens 4 | nd | nd | nd | 83% | nd | nd | nd | nd | nd |
| Spec 4 | nd | nd | nd | 70% | nd | nd | nd | nd | nd |
| Cutoff 5 | nd | nd | nd | 0.163 | nd | nd | nd | nd | nd |
| Sens 5 | nd | nd | nd | 50% | nd | nd | nd | nd | nd |
| Spec 5 | nd | nd | nd | 80% | nd | nd | nd | nd | nd |
| Cutoff 6 | nd | nd | nd | 0.395 | nd | nd | nd | nd | nd |
| Sens 6 | nd | nd | nd | 33% | nd | nd | nd | nd | nd |
| Spec 6 | nd | nd | nd | 90% | nd | nd | nd | nd | nd |
| OR Quart 2 | nd | nd | nd | >1.0 | nd | nd | nd | nd | nd |
| p Value | nd | nd | nd | <1.00 | nd | nd | nd | nd | nd |
| 95% CI of OR Quart2 | nd | nd | nd | >0.062 na | nd | nd | nd | nd | nd |
| OR Quart 3 | nd | nd | nd | >1.0 | nd | nd | nd | nd | nd |
| p Value | nd | nd | nd | <1.00 | nd | nd | nd | nd | nd |
| 95% CI of OR Quart3 | nd | nd | nd | >0.062 na | nd | nd | nd | nd | nd |
| OR Quart 4 | nd | nd | nd | >4.1 | nd | nd | nd | nd | nd |
| p Value | nd | nd | nd | <0.21 | nd | nd | nd | nd | nd |
| 95% CI of OR Quart4 | nd | nd | nd | >0.46 na | nd | nd | nd | nd | nd |

**Involucrin**

| sCr or UO | 0hr prior to AKI stage | 24hr prior to AKI stage | 48hr prior to AKI stage |
|---|---|---|---|

| | Cohort 1 | Cohort 2 | Cohort 1 | Cohort 2 | Cohort 1 | Cohort 2 |
|---|---|---|---|---|---|---|
| Median | nd | nd | 0.696 | 2.03 | nd | nd |
| Average | nd | nd | 0.932 | 2.46 | nd | nd |
| Stdev | nd | nd | 0.855 | 1.85 | nd | nd |
| p(t-test) | nd | nd | | 2.3E-5 | nd | nd |
| Min | nd | nd | 0.0472 | 0.185 | nd | nd |
| Max | nd | nd | 12.7 | 4.83 | nd | nd |
| n (Samp) | nd | nd | 490 | 6 | nd | nd |
| n (Patient) | nd | nd | 220 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.77 | nd | nd | nd | nd | nd |
| SE | nd | nd | nd | 0.11 | nd | nd | nd | nd | nd |
| p | nd | nd | nd | 0.018 | nd | nd | nd | nd | nd |
| nCohort 1 | nd | nd | nd | 490 | nd | nd | nd | nd | nd |
| nCohort 2 | nd | nd | nd | 6 | nd | nd | nd | nd | nd |
| Cutoff 1 | nd | nd | nd | 1.25 | nd | nd | nd | nd | nd |
| Sens 1 | nd | nd | nd | 83% | nd | nd | nd | nd | nd |
| Spec 1 | nd | nd | nd | 80% | nd | nd | nd | nd | nd |
| Cutoff 2 | nd | nd | nd | 1.25 | nd | nd | nd | nd | nd |
| Sens 2 | nd | nd | nd | 83% | nd | nd | nd | nd | nd |
| Spec 2 | nd | nd | nd | 80% | nd | nd | nd | nd | nd |
| Cutoff 3 | nd | nd | nd | 0.173 | nd | nd | nd | nd | nd |
| Sens 3 | nd | nd | nd | 100% | nd | nd | nd | nd | nd |
| Spec 3 | nd | nd | nd | 2% | nd | nd | nd | nd | nd |
| Cutoff 4 | nd | nd | nd | 0.959 | nd | nd | nd | nd | nd |
| Sens 4 | nd | nd | nd | 83% | nd | nd | nd | nd | nd |
| Spec 4 | nd | nd | nd | 70% | nd | nd | nd | nd | nd |
| Cutoff 5 | nd | nd | nd | 1.26 | nd | nd | nd | nd | nd |
| Sens 5 | nd | nd | nd | 67% | nd | nd | nd | nd | nd |
| Spec 5 | nd | nd | nd | 80% | nd | nd | nd | nd | nd |
| Cutoff 6 | nd | nd | nd | 1.82 | nd | nd | nd | nd | nd |
| Sens 6 | nd | nd | nd | 50% | nd | nd | nd | nd | nd |
| Spec 6 | nd | nd | nd | 90% | nd | nd | nd | nd | nd |
| OR Quart 2 | nd | nd | nd | 0 | nd | nd | nd | nd | nd |
| p Value | nd | nd | nd | na | nd | nd | nd | nd | nd |
| 95% CI of | nd | nd | nd | na | nd | nd | nd | nd | nd |
| OR Quart2 | nd | nd | nd | na | nd | nd | nd | nd | nd |
| OR Quart 3 | nd | nd | nd | 0 | nd | nd | nd | nd | nd |
| p Value | nd | nd | nd | na | nd | nd | nd | nd | nd |
| 95% CI of | nd | nd | nd | na | nd | nd | nd | nd | nd |
| OR Quart3 | nd | nd | nd | na | nd | nd | nd | nd | nd |
| OR Quart 4 | nd | nd | nd | 5.2 | nd | nd | nd | nd | nd |
| p Value | nd | nd | nd | 0.14 | nd | nd | nd | nd | nd |
| 95% CI of | nd | nd | nd | 0.59 | nd | nd | nd | nd | nd |
| OR Quart4 | nd | nd | nd | 45 | nd | nd | nd | nd | nd |

**NF-kappa-B inhibitor alpha**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.00106 | 0.00387 | nd | nd |
| Average | nd | nd | 0.0344 | 0.00393 | nd | nd |
| Stdev | nd | nd | 0.235 | 0.00297 | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | nd | nd | | 0.75 | nd | nd |
| Min | nd | nd | 1.84E-7 | 3.67E-5 | nd | nd |
| Max | nd | nd | 3.62 | 0.00890 | nd | nd |
| n (Samp) | nd | nd | 490 | 6 | nd | nd |
| n (Patient) | nd | nd | 220 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.63 | nd | nd | nd | nd | nd |
| SE | nd | nd | nd | 0.12 | nd | nd | nd | nd | nd |
| p | nd | nd | nd | 0.30 | nd | nd | nd | nd | nd |
| nCohort 1 | nd | nd | nd | 490 | nd | nd | nd | nd | nd |
| nCohort 2 | nd | nd | nd | 6 | nd | nd | nd | nd | nd |
| Cutoff 1 | nd | nd | nd | 0.00217 | nd | nd | nd | nd | nd |
| Sens 1 | nd | nd | nd | 83% | nd | nd | nd | nd | nd |
| Spec 1 | nd | nd | nd | 62% | nd | nd | nd | nd | nd |
| Cutoff 2 | nd | nd | nd | 0.00217 | nd | nd | nd | nd | nd |
| Sens 2 | nd | nd | nd | 83% | nd | nd | nd | nd | nd |
| Spec 2 | nd | nd | nd | 62% | nd | nd | nd | nd | nd |
| Cutoff 3 | nd | nd | nd | 3.31E-5 | nd | nd | nd | nd | nd |
| Sens 3 | nd | nd | nd | 100% | nd | nd | nd | nd | nd |
| Spec 3 | nd | nd | nd | 20% | nd | nd | nd | nd | nd |
| Cutoff 4 | nd | nd | nd | 0.00413 | nd | nd | nd | nd | nd |
| Sens 4 | nd | nd | nd | 50% | nd | nd | nd | nd | nd |
| Spec 4 | nd | nd | nd | 70% | nd | nd | nd | nd | nd |
| Cutoff 5 | nd | nd | nd | 0.0122 | nd | nd | nd | nd | nd |
| Sens 5 | nd | nd | nd | 0% | nd | nd | nd | nd | nd |
| Spec 5 | nd | nd | nd | 80% | nd | nd | nd | nd | nd |
| Cutoff 6 | nd | nd | nd | 0.0421 | nd | nd | nd | nd | nd |
| Sens 6 | nd | nd | nd | 0% | nd | nd | nd | nd | nd |
| Spec 6 | nd | nd | nd | 90% | nd | nd | nd | nd | nd |
| OR Quart 2 | nd | nd | nd | 0 | nd | nd | nd | nd | nd |
| p Value | nd | nd | nd | na | nd | nd | nd | nd | nd |
| 95% CI of OR Quart2 | nd | nd | nd | na | nd | nd | nd | nd | nd |
| | nd | nd | nd | na | nd | nd | nd | nd | nd |
| OR Quart 3 | nd | nd | nd | 4.1 | nd | nd | nd | nd | nd |
| p Value | nd | nd | nd | 0.21 | nd | nd | nd | nd | nd |
| 95% CI of OR Quart3 | nd | nd | nd | 0.45 | nd | nd | nd | nd | nd |
| | nd | nd | nd | 37 | nd | nd | nd | nd | nd |
| OR Quart 4 | nd | nd | nd | 1.0 | nd | nd | nd | nd | nd |
| p Value | nd | nd | nd | 1.0 | nd | nd | nd | nd | nd |
| 95% CI of OR Quart4 | nd | nd | nd | 0.062 | nd | nd | nd | nd | nd |
| | nd | nd | nd | 16 | nd | nd | nd | nd | nd |

**Beta-nerve growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.71 | 2.96 | nd | nd |
| Average | nd | nd | 2.28 | 3.76 | nd | nd |
| Stdev | nd | nd | 3.40 | 1.74 | nd | nd |
| p(t-test) | nd | nd | | 0.29 | nd | nd |
| Min | nd | nd | 0.221 | 2.36 | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | nd | nd | 70.0 | 6.73 | nd | nd |
| n (Samp) | nd | nd | 489 | 6 | nd | nd |
| n (Patient) | nd | nd | 218 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.83 | nd | nd | nd | nd | nd |
| SE | nd | nd | nd | 0.10 | nd | nd | nd | nd | nd |
| p | nd | nd | nd | 0.0014 | nd | nd | nd | nd | nd |
| nCohort 1 | nd | nd | nd | 489 | nd | nd | nd | nd | nd |
| nCohort 2 | nd | nd | nd | 6 | nd | nd | nd | nd | nd |
| Cutoff 1 | nd | nd | nd | 2.53 | nd | nd | nd | nd | nd |
| Sens 1 | nd | nd | nd | 83% | nd | nd | nd | nd | nd |
| Spec 1 | nd | nd | nd | 74% | nd | nd | nd | nd | nd |
| Cutoff 2 | nd | nd | nd | 2.53 | nd | nd | nd | nd | nd |
| Sens 2 | nd | nd | nd | 83% | nd | nd | nd | nd | nd |
| Spec 2 | nd | nd | nd | 74% | nd | nd | nd | nd | nd |
| Cutoff 3 | nd | nd | nd | 2.35 | nd | nd | nd | nd | nd |
| Sens 3 | nd | nd | nd | 100% | nd | nd | nd | nd | nd |
| Spec 3 | nd | nd | nd | 71% | nd | nd | nd | nd | nd |
| Cutoff 4 | nd | nd | nd | 2.30 | nd | nd | nd | nd | nd |
| Sens 4 | nd | nd | nd | 100% | nd | nd | nd | nd | nd |
| Spec 4 | nd | nd | nd | 70% | nd | nd | nd | nd | nd |
| Cutoff 5 | nd | nd | nd | 2.91 | nd | nd | nd | nd | nd |
| Sens 5 | nd | nd | nd | 50% | nd | nd | nd | nd | nd |
| Spec 5 | nd | nd | nd | 80% | nd | nd | nd | nd | nd |
| Cutoff 6 | nd | nd | nd | 4.06 | nd | nd | nd | nd | nd |
| Sens 6 | nd | nd | nd | 33% | nd | nd | nd | nd | nd |
| Spec 6 | nd | nd | nd | 90% | nd | nd | nd | nd | nd |
| OR Quart 2 | nd | nd | nd | >0 | nd | nd | nd | nd | nd |
| p Value | nd | nd | nd | <na | nd | nd | nd | nd | nd |
| 95% CI of | nd | nd | nd | >na | nd | nd | nd | nd | nd |
| OR Quart2 | nd | nd | nd | na | nd | nd | nd | nd | nd |
| OR Quart 3 | nd | nd | nd | >2.0 | nd | nd | nd | nd | nd |
| p Value | nd | nd | nd | <0.57 | nd | nd | nd | nd | nd |
| 95% CI of | nd | nd | nd | >0.18 | nd | nd | nd | nd | nd |
| OR Quart3 | nd | nd | nd | na | nd | nd | nd | nd | nd |
| OR Quart 4 | nd | nd | nd | >4.1 | nd | nd | nd | nd | nd |
| p Value | nd | nd | nd | <0.21 | nd | nd | nd | nd | nd |
| 95% CI of | nd | nd | nd | >0.45 | nd | nd | nd | nd | nd |
| OR Quart4 | nd | nd | nd | na | nd | nd | nd | nd | nd |

**Transthyretin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 55200 | 45500 | nd | nd |
| Average | nd | nd | 63200 | 55200 | nd | nd |
| Stdev | nd | nd | 39800 | 25800 | nd | nd |
| p(t-test) | nd | nd | | 0.62 | nd | nd |
| Min | nd | nd | 6500 | 30100 | nd | nd |
| Max | nd | nd | 293000 | 89900 | nd | nd |
| n (Samp) | nd | nd | 488 | 6 | nd | nd |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | nd | nd | 222 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.47 | nd | nd | nd | nd | nd |
| SE | nd | nd | nd | 0.12 | nd | nd | nd | nd | nd |
| p | nd | nd | nd | 0.83 | nd | nd | nd | nd | nd |
| nCohort 1 | nd | nd | nd | 488 | nd | nd | nd | nd | nd |
| nCohort 2 | nd | nd | nd | 6 | nd | nd | nd | nd | nd |
| Cutoff 1 | nd | nd | nd | 36200 | nd | nd | nd | nd | nd |
| Sens 1 | nd | nd | nd | 83% | nd | nd | nd | nd | nd |
| Spec 1 | nd | nd | nd | 27% | nd | nd | nd | nd | nd |
| Cutoff 2 | nd | nd | nd | 36200 | nd | nd | nd | nd | nd |
| Sens 2 | nd | nd | nd | 83% | nd | nd | nd | nd | nd |
| Spec 2 | nd | nd | nd | 27% | nd | nd | nd | nd | nd |
| Cutoff 3 | nd | nd | nd | 29900 | nd | nd | nd | nd | nd |
| Sens 3 | nd | nd | nd | 100% | nd | nd | nd | nd | nd |
| Spec 3 | nd | nd | nd | 18% | nd | nd | nd | nd | nd |
| Cutoff 4 | nd | nd | nd | 73300 | nd | nd | nd | nd | nd |
| Sens 4 | nd | nd | nd | 33% | nd | nd | nd | nd | nd |
| Spec 4 | nd | nd | nd | 70% | nd | nd | nd | nd | nd |
| Cutoff 5 | nd | nd | nd | 85300 | nd | nd | nd | nd | nd |
| Sens 5 | nd | nd | nd | 17% | nd | nd | nd | nd | nd |
| Spec 5 | nd | nd | nd | 80% | nd | nd | nd | nd | nd |
| Cutoff 6 | nd | nd | nd | 118000 | nd | nd | nd | nd | nd |
| Sens 6 | nd | nd | nd | 0% | nd | nd | nd | nd | nd |
| Spec 6 | nd | nd | nd | 90% | nd | nd | nd | nd | nd |
| OR Quart 2 | nd | nd | nd | 0 | nd | nd | nd | nd | nd |
| p Value | nd | nd | nd | na | nd | nd | nd | nd | nd |
| 95% CI of OR Quart2 | nd | nd | nd | na | nd | nd | nd | nd | nd |
| | nd | nd | nd | na | nd | nd | nd | nd | nd |
| OR Quart 3 | nd | nd | nd | 1.5 | nd | nd | nd | nd | nd |
| p Value | nd | nd | nd | 0.65 | nd | nd | nd | nd | nd |
| 95% CI of OR Quart3 | nd | nd | nd | 0.25 | nd | nd | nd | nd | nd |
| | nd | nd | nd | 9.2 | nd | nd | nd | nd | nd |
| OR Quart 4 | nd | nd | nd | 0.50 | nd | nd | nd | nd | nd |
| p Value | nd | nd | nd | 0.57 | nd | nd | nd | nd | nd |
| 95% CI of OR Quart4 | nd | nd | nd | 0.045 | nd | nd | nd | nd | nd |
| | nd | nd | nd | 5.6 | nd | nd | nd | nd | nd |

**C-peptide EDTA**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2660 | 3260 | 2660 | 2410 | 2660 | 2360 |
| Average | 3310 | 4600 | 3310 | 3140 | 3310 | 3480 |
| Stdev | 2840 | 3170 | 2840 | 2630 | 2840 | 2950 |
| p(t-test) | | 0.23 | | 0.87 | | 0.88 |
| Min | 7.96 | 1740 | 7.96 | 726 | 7.96 | 163 |
| Max | 21700 | 9770 | 21700 | 8140 | 21700 | 7570 |
| n (Samp) | 601 | 7 | 601 | 8 | 601 | 7 |
| n (Patient) | 269 | 7 | 269 | 8 | 269 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 2600 | 1520 | nd | nd |
| Average | nd | nd | 3300 | 2670 | nd | nd |
| Stdev | nd | nd | 2850 | 2130 | nd | nd |
| p(t-test) | nd | nd | | 0.56 | nd | nd |
| Min | nd | nd | 7.96 | 674 | nd | nd |
| Max | nd | nd | 21700 | 5230 | nd | nd |
| n (Samp) | nd | nd | 582 | 7 | nd | nd |
| n (Patient) | nd | nd | 246 | 7 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | nd | nd | 0.48 | nd | 0.44 | 0.52 | nd | nd |
| SE | 0.11 | nd | nd | 0.10 | nd | 0.11 | 0.11 | nd | nd |
| p | 0.20 | nd | nd | 0.81 | nd | 0.60 | 0.85 | nd | nd |
| nCohort 1 | 601 | nd | nd | 601 | nd | 582 | 601 | nd | nd |
| nCohort 2 | 7 | nd | nd | 8 | nd | 7 | 7 | nd | nd |
| Cutoff 1 | 2450 | nd | nd | 998 | nd | 998 | 1650 | nd | nd |
| Sens 1 | 71% | nd | nd | 75% | nd | 71% | 71% | nd | nd |
| Spec 1 | 47% | nd | nd | 16% | nd | 17% | 34% | nd | nd |
| Cutoff 2 | 1790 | nd | nd | 875 | nd | 708 | 1630 | nd | nd |
| Sens 2 | 86% | nd | nd | 88% | nd | 86% | 86% | nd | nd |
| Spec 2 | 36% | nd | nd | 13% | nd | 10% | 34% | nd | nd |
| Cutoff 3 | 1730 | nd | nd | 708 | nd | 656 | 159 | nd | nd |
| Sens 3 | 100% | nd | nd | 100% | nd | 100% | 100% | nd | nd |
| Spec 3 | 36% | nd | nd | 10% | nd | 10% | 3% | nd | nd |
| Cutoff 4 | 4100 | nd | nd | 4100 | nd | 4090 | 4100 | nd | nd |
| Sens 4 | 43% | nd | nd | 38% | nd | 43% | 29% | nd | nd |
| Spec 4 | 70% | nd | nd | 70% | nd | 70% | 70% | nd | nd |
| Cutoff 5 | 4920 | nd | nd | 4920 | nd | 4940 | 4920 | nd | nd |
| Sens 5 | 43% | nd | nd | 12% | nd | 14% | 29% | nd | nd |
| Spec 5 | 80% | nd | nd | 80% | nd | 80% | 80% | nd | nd |
| Cutoff 6 | 6430 | nd | nd | 6430 | nd | 6630 | 6430 | nd | nd |
| Sens 6 | 29% | nd | nd | 12% | nd | 0% | 29% | nd | nd |
| Spec 6 | 90% | nd | nd | 90% | nd | 90% | 90% | nd | nd |
| OR Quart 2 | >3.1 | nd | nd | 0.33 | nd | 0 | 3.0 | nd | nd |
| p Value | <0.34 | nd | nd | 0.34 | nd | na | 0.34 | nd | nd |
| 95% CI of | >0.31 | nd | nd | 0.034 | nd | na | 0.31 | nd | nd |
| OR Quart2 | na | nd | nd | 3.2 | nd | na | 30 | nd | nd |
| OR Quart 3 | >1.0 | nd | nd | 0.33 | nd | 0.33 | 1.0 | nd | nd |
| p Value | <1.00 | nd | nd | 0.34 | nd | 0.34 | 1.0 | nd | nd |
| 95% CI of | >0.062 | nd | nd | 0.034 | nd | 0.034 | 0.062 | nd | nd |
| OR Quart3 | na | nd | nd | 3.2 | nd | 3.2 | 16 | nd | nd |
| OR Quart 4 | >3.1 | nd | nd | 1.0 | nd | 1.0 | 2.0 | nd | nd |
| p Value | <0.34 | nd | nd | 0.99 | nd | 0.99 | 0.57 | nd | nd |
| 95% CI of | >0.31 | nd | nd | 0.20 | nd | 0.20 | 0.18 | nd | nd |
| OR Quart4 | na | nd | nd | 5.1 | nd | 5.1 | 22 | nd | nd |

**Gastric inhibitory polypeptide EDTA**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 26.8 | 14.2 | 26.8 | 8.52 | 26.8 | 19.4 |
| Average | 56.3 | 22.6 | 56.3 | 23.4 | 56.3 | 59.1 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 81.4 | 19.5 | 81.4 | 24.2 | 81.4 | 89.2 |
| p(t-test) | | 0.27 | | 0.25 | | 0.93 |
| Min | 0.888 | 7.46 | 0.888 | 6.35 | 0.888 | 7.18 |
| Max | 820 | 59.5 | 820 | 72.4 | 820 | 251 |
| n (Samp) | 601 | 7 | 601 | 8 | 601 | 7 |
| n (Patient) | 269 | 7 | 269 | 8 | 269 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 24.8 | 37.0 | nd | nd |
| Average | nd | nd | 55.8 | 45.7 | nd | nd |
| Stdev | nd | nd | 82.5 | 43.3 | nd | nd |
| p(t-test) | nd | nd | | 0.75 | nd | nd |
| Min | nd | nd | 0.888 | 7.04 | nd | nd |
| Max | nd | nd | 820 | 130 | nd | nd |
| n (Samp) | nd | nd | 582 | 7 | nd | nd |
| n (Patient) | nd | nd | 246 | 7 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.35 | nd | nd | 0.32 | nd | 0.52 | 0.45 | nd | nd |
| SE | 0.11 | nd | nd | 0.11 | nd | 0.11 | 0.11 | nd | nd |
| p | 0.19 | nd | nd | 0.094 | nd | 0.84 | 0.68 | nd | nd |
| nCohort 1 | 601 | nd | nd | 601 | nd | 582 | 601 | nd | nd |
| nCohort 2 | 7 | nd | nd | 8 | nd | 7 | 7 | nd | nd |
| Cutoff 1 | 7.95 | nd | nd | 7.83 | nd | 25.8 | 14.3 | nd | nd |
| Sens 1 | 71% | nd | nd | 75% | nd | 71% | 71% | nd | nd |
| Spec 1 | 12% | nd | nd | 12% | nd | 51% | 29% | nd | nd |
| Cutoff 2 | 7.56 | nd | nd | 6.99 | nd | 7.83 | 8.52 | nd | nd |
| Sens 2 | 86% | nd | nd | 88% | nd | 86% | 86% | nd | nd |
| Spec 2 | 11% | nd | nd | 10% | nd | 13% | 13% | nd | nd |
| Cutoff 3 | 7.37 | nd | nd | 6.25 | nd | 6.99 | 6.99 | nd | nd |
| Sens 3 | 100% | nd | nd | 100% | nd | 100% | 100% | nd | nd |
| Spec 3 | 10% | nd | nd | 8% | nd | 10% | 10% | nd | nd |
| Cutoff 4 | 49.7 | nd | nd | 49.7 | nd | 49.1 | 49.7 | nd | nd |
| Sens 4 | 14% | nd | nd | 12% | nd | 29% | 29% | nd | nd |
| Spec 4 | 70% | nd | nd | 70% | nd | 70% | 70% | nd | nd |
| Cutoff 5 | 81.4 | nd | nd | 81.4 | nd | 80.9 | 81.4 | nd | nd |
| Sens 5 | 0% | nd | nd | 0% | nd | 14% | 29% | nd | nd |
| Spec 5 | 80% | nd | nd | 80% | nd | 80% | 80% | nd | nd |
| Cutoff 6 | 147 | nd | nd | 147 | nd | 147 | 147 | nd | nd |
| Sens 6 | 0% | nd | nd | 0% | nd | 0% | 14% | nd | nd |
| Spec 6 | 90% | nd | nd | 90% | nd | 90% | 90% | nd | nd |
| OR Quart 2 | >2.0 | nd | nd | 2.0 | nd | 0 | 0 | nd | nd |
| p Value | <0.57 | nd | nd | 0.57 | nd | na | na | nd | nd |
| 95% CI of OR Quart2 | >0.18 na | nd | nd | 0.18 23 | nd | na na | na na | nd | nd |
| OR Quart 3 | >2.0 | nd | nd | 0 | nd | 1.5 | 1.5 | nd | nd |
| p Value | <0.57 | nd | nd | na | nd | 0.65 | 0.65 | nd | nd |
| 95% CI of OR Quart3 | >0.18 na | nd | nd | na na | nd | 0.25 9.2 | 0.25 9.2 | nd | nd |
| OR Quart 4 | >3.1 | nd | nd | 5.2 | nd | 0.99 | 1.0 | nd | nd |
| p Value | <0.34 | nd | nd | 0.14 | nd | 0.99 | 1.0 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | >0.31 | nd | nd | 0.60 | nd | 0.14 | 0.14 | nd | nd |
| OR Quart4 | na | nd | nd | 45 | nd | 7.1 | 7.2 | nd | nd |

**Peptide YY  EDTA**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 40.2 | 95.2 | 40.2 | 87.1 | 40.2 | 89.2 |
| Average | 56.3 | 111 | 56.3 | 102 | 56.3 | 135 |
| Stdev | 68.1 | 96.7 | 68.1 | 85.9 | 68.1 | 143 |
| p(t-test) | | 0.037 | | 0.063 | | 0.0029 |
| Min | 0.00999 | 12.7 | 0.00999 | 12.0 | 0.00999 | 8.31 |
| Max | 789 | 284 | 789 | 270 | 789 | 406 |
| n (Samp) | 601 | 7 | 601 | 8 | 601 | 7 |
| n (Patient) | 269 | 7 | 269 | 8 | 269 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 39.4 | 118 | nd | nd |
| Average | nd | nd | 54.6 | 128 | nd | nd |
| Stdev | nd | nd | 67.4 | 84.8 | nd | nd |
| p(t-test) | nd | nd | | 0.0044 | nd | nd |
| Min | nd | nd | 0.00999 | 22.6 | nd | nd |
| Max | nd | nd | 789 | 270 | nd | nd |
| n (Samp) | nd | nd | 582 | 7 | nd | nd |
| n (Patient) | nd | nd | 246 | 7 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | nd | nd | 0.69 | nd | 0.79 | 0.66 | nd | nd |
| SE | 0.11 | nd | nd | 0.11 | nd | 0.10 | 0.11 | nd | nd |
| p | 0.098 | nd | nd | 0.076 | nd | 0.0039 | 0.16 | nd | nd |
| nCohort 1 | 601 | nd | nd | 601 | nd | 582 | 601 | nd | nd |
| nCohort 2 | 7 | nd | nd | 8 | nd | 7 | 7 | nd | nd |
| Cutoff 1 | 55.3 | nd | nd | 45.0 | nd | 95.9 | 44.1 | nd | nd |
| Sens 1 | 71% | nd | nd | 75% | nd | 71% | 71% | nd | nd |
| Spec 1 | 67% | nd | nd | 56% | nd | 86% | 55% | nd | nd |
| Cutoff 2 | 18.0 | nd | nd | 21.6 | nd | 45.0 | 13.4 | nd | nd |
| Sens 2 | 86% | nd | nd | 88% | nd | 86% | 86% | nd | nd |
| Spec 2 | 23% | nd | nd | 27% | nd | 58% | 19% | nd | nd |
| Cutoff 3 | 12.6 | nd | nd | 11.7 | nd | 22.6 | 7.32 | nd | nd |
| Sens 3 | 100% | nd | nd | 100% | nd | 100% | 100% | nd | nd |
| Spec 3 | 18% | nd | nd | 17% | nd | 30% | 14% | nd | nd |
| Cutoff 4 | 60.3 | nd | nd | 60.3 | nd | 58.5 | 60.3 | nd | nd |
| Sens 4 | 57% | nd | nd | 62% | nd | 71% | 57% | nd | nd |
| Spec 4 | 70% | nd | nd | 70% | nd | 70% | 70% | nd | nd |
| Cutoff 5 | 81.2 | nd | nd | 81.2 | nd | 79.0 | 81.2 | nd | nd |
| Sens 5 | 57% | nd | nd | 50% | nd | 71% | 57% | nd | nd |
| Spec 5 | 80% | nd | nd | 80% | nd | 80% | 80% | nd | nd |
| Cutoff 6 | 113 | nd | nd | 113 | nd | 110 | 113 | nd | nd |
| Sens 6 | 43% | nd | nd | 38% | nd | 57% | 43% | nd | nd |
| Spec 6 | 90% | nd | nd | 90% | nd | 90% | 90% | nd | nd |
| OR Quart 2 | 0 | nd | nd | 1.0 | nd | >1.0 | 0 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | na | nd | nd | 1.0 | nd | <1.00 | na | nd | nd |
| 95% CI of | na | nd | nd | 0.062 | nd | >0.062 | na | nd | nd |
| OR Quart2 | na | nd | nd | 16 | nd | na | na | nd | nd |
| OR Quart 3 | 0.50 | nd | nd | 1.0 | nd | >1.0 | 0.50 | nd | nd |
| p Value | 0.57 | nd | nd | 1.0 | nd | <1.00 | 0.57 | nd | nd |
| 95% CI of | 0.045 | nd | nd | 0.062 | nd | >0.062 | 0.045 | nd | nd |
| OR Quart3 | 5.5 | nd | nd | 16 | nd | na | 5.5 | nd | nd |
| OR Quart 4 | 2.0 | nd | nd | 5.1 | nd | >5.1 | 2.0 | nd | nd |
| p Value | 0.42 | nd | nd | 0.14 | nd | <0.14 | 0.42 | nd | nd |
| 95% CI of | 0.37 | nd | nd | 0.59 | nd | >0.59 | 0.37 | nd | nd |
| OR Quart4 | 11 | nd | nd | 44 | nd | na | 11 | nd | nd |

Table 11: Comparison of marker levels in enroll urine samples collected from Cohort 1 (patients that did not progress beyond RIFLE stage 0 or R within 48hrs) and in enroll urine samples collected from Cohort 2 (subjects reaching RIFLE stage I or F within 48hrs). Enroll samples from patients already at RIFLE stage I or F were included in Cohort 2.

**Agouti-related protein**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 0.698 | 0.922 | 0.729 | 0.982 | 0.698 | 0.949 |
| Average | 1.25 | 1.43 | 1.25 | 1.91 | 1.24 | 1.43 |
| Stdev | 2.34 | 1.46 | 2.20 | 1.93 | 2.32 | 1.49 |
| p(t-test) | | 0.59 | | 0.33 | | 0.57 |
| Min | 0.00198 | 0.00498 | 0.00198 | 0.126 | 0.00198 | 0.00498 |
| Max | 20.0 | 6.53 | 20.0 | 6.53 | 20.0 | 6.53 |
| n (Samp) | 210 | 57 | 250 | 11 | 208 | 50 |
| n (Patient) | 210 | 57 | 250 | 11 | 208 | 50 |

| | At Enrollment | | |
|---|---|---|---|
| | sCr or UO | sCr only | UO only |
| AUC | 0.60 | 0.63 | 0.60 |
| SE | 0.044 | 0.092 | 0.046 |
| p | 0.030 | 0.14 | 0.037 |
| nCohort 1 | 210 | 250 | 208 |
| nCohort 2 | 57 | 11 | 50 |
| Cutoff 1 | 0.579 | 0.720 | 0.549 |
| Sens 1 | 70% | 73% | 70% |
| Spec 1 | 45% | 50% | 44% |
| Cutoff 2 | 0.301 | 0.579 | 0.301 |
| Sens 2 | 81% | 82% | 80% |
| Spec 2 | 29% | 43% | 28% |
| Cutoff 3 | 0.156 | 0.129 | 0.202 |
| Sens 3 | 91% | 91% | 90% |
| Spec 3 | 16% | 12% | 19% |
| Cutoff 4 | 1.16 | 1.24 | 1.16 |
| Sens 4 | 42% | 45% | 44% |
| Spec 4 | 70% | 70% | 70% |
| Cutoff 5 | 1.51 | 1.60 | 1.51 |

|  | At Enrollment | | |
|---|---|---|---|
|  | sCr or UO | sCr only | UO only |
| Sens 5 | 37% | 45% | 38% |
| Spec 5 | 80% | 80% | 80% |
| Cutoff 6 | 2.33 | 2.33 | 2.34 |
| Sens 6 | 18% | 36% | 16% |
| Spec 6 | 90% | 90% | 90% |
| OR Quart 2 | 1.5 | 1.0 | 1.4 |
| p Value | 0.37 | 1.0 | 0.48 |
| 95% CI of | 0.60 | 0.14 | 0.53 |
| OR Quart2 | 3.9 | 7.3 | 3.8 |
| OR Quart 3 | 1.5 | 1.0 | 1.5 |
| p Value | 0.37 | 1.0 | 0.46 |
| 95% CI of | 0.60 | 0.14 | 0.54 |
| OR Quart3 | 3.9 | 7.3 | 3.9 |
| OR Quart 4 | 3.1 | 2.6 | 3.1 |
| p Value | 0.011 | 0.27 | 0.014 |
| 95% CI of | 1.3 | 0.48 | 1.3 |
| OR Quart4 | 7.4 | 14 | 7.7 |

**Osteocalcin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 6300 | 8150 | 6550 | 9180 | 6450 | 7750 |
| Average | 8120 | 10500 | 8500 | 12300 | 8410 | 9760 |
| Stdev | 7530 | 9290 | 7980 | 9120 | 7880 | 9070 |
| p(t-test) |  | 0.0090 |  | 0.033 |  | 0.17 |
| Min | 73.4 | 123 | 73.4 | 699 | 73.4 | 123 |
| Max | 83000 | 49400 | 83000 | 33400 | 83000 | 49400 |
| n (Samp) | 341 | 101 | 413 | 21 | 324 | 86 |
| n (Patient) | 341 | 101 | 413 | 21 | 324 | 86 |

|  | At Enrollment | | |
|---|---|---|---|
|  | sCr or UO | sCr only | UO only |
| AUC | 0.59 | 0.65 | 0.55 |
| SE | 0.033 | 0.066 | 0.036 |
| p | 0.0086 | 0.024 | 0.12 |
| nCohort 1 | 341 | 413 | 324 |
| nCohort 2 | 101 | 21 | 86 |
| Cutoff 1 | 5010 | 7030 | 4960 |
| Sens 1 | 70% | 71% | 71% |
| Spec 1 | 40% | 54% | 40% |
| Cutoff 2 | 4190 | 5090 | 3570 |
| Sens 2 | 80% | 81% | 80% |
| Spec 2 | 33% | 40% | 26% |
| Cutoff 3 | 2770 | 4190 | 2240 |
| Sens 3 | 90% | 90% | 91% |
| Spec 3 | 18% | 31% | 11% |
| Cutoff 4 | 9160 | 9550 | 9550 |
| Sens 4 | 41% | 48% | 31% |
| Spec 4 | 70% | 70% | 70% |
| Cutoff 5 | 11400 | 12000 | 12000 |
| Sens 5 | 32% | 38% | 23% |
| Spec 5 | 80% | 80% | 80% |

|  | At Enrollment | | |
|---|---|---|---|
|  | sCr or UO | sCr only | UO only |
| Cutoff 6 | 16700 | 17000 | 17300 |
| Sens 6 | 15% | 24% | 10% |
| Spec 6 | 90% | 90% | 90% |
| OR Quart 2 | 0.99 | 1.5 | 1.1 |
| p Value | 0.98 | 0.66 | 0.88 |
| 95% CI of | 0.49 | 0.25 | 0.51 |
| OR Quart2 | 2.0 | 9.2 | 2.2 |
| OR Quart 3 | 1.9 | 4.2 | 1.8 |
| p Value | 0.055 | 0.072 | 0.091 |
| 95% CI of | 0.99 | 0.88 | 0.91 |
| OR Quart3 | 3.6 | 20 | 3.6 |
| OR Quart 4 | 1.9 | 4.2 | 1.5 |
| p Value | 0.043 | 0.074 | 0.24 |
| 95% CI of | 1.0 | 0.87 | 0.76 |
| OR Quart4 | 3.7 | 20 | 3.0 |

**Follitropin subunit beta**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 15.5 | 15.4 | 15.9 | 8.19 | 15.9 | 16.6 |
| Average | 34.6 | 28.3 | 33.4 | 39.7 | 36.1 | 24.2 |
| Stdev | 44.9 | 43.7 | 42.6 | 87.2 | 48.5 | 25.0 |
| p(t-test) |  | 0.34 |  | 0.66 |  | 0.095 |
| Min | 0.342 | 0.716 | 0.342 | 1.46 | 0.342 | 0.716 |
| Max | 246 | 300 | 246 | 300 | 300 | 119 |
| n (Samp) | 210 | 57 | 250 | 11 | 208 | 50 |
| n (Patient) | 210 | 57 | 250 | 11 | 208 | 50 |

|  | At Enrollment | | |
|---|---|---|---|
|  | sCr or UO | sCr only | UO only |
| AUC | 0.49 | 0.43 | 0.48 |
| SE | 0.043 | 0.092 | 0.046 |
| p | 0.82 | 0.41 | 0.67 |
| nCohort 1 | 210 | 250 | 208 |
| nCohort 2 | 57 | 11 | 50 |
| Cutoff 1 | 8.14 | 5.26 | 8.50 |
| Sens 1 | 70% | 73% | 70% |
| Spec 1 | 34% | 23% | 36% |
| Cutoff 2 | 6.06 | 5.23 | 6.34 |
| Sens 2 | 81% | 82% | 80% |
| Spec 2 | 27% | 23% | 28% |
| Cutoff 3 | 2.98 | 2.84 | 4.72 |
| Sens 3 | 91% | 91% | 90% |
| Spec 3 | 15% | 13% | 22% |
| Cutoff 4 | 38.4 | 36.6 | 38.4 |
| Sens 4 | 21% | 9% | 22% |
| Spec 4 | 70% | 70% | 70% |
| Cutoff 5 | 56.4 | 53.3 | 57.0 |
| Sens 5 | 9% | 9% | 8% |
| Spec 5 | 80% | 80% | 80% |
| Cutoff 6 | 86.0 | 84.9 | 97.1 |
| Sens 6 | 4% | 9% | 2% |

| | At Enrollment | | |
|---|---|---|---|
| | sCr or UO | sCr only | UO only |
| Spec 6 | 90% | 90% | 90% |
| OR Quart 2 | 1.9 | 3.1 | 1.7 |
| p Value | 0.15 | 0.33 | 0.25 |
| 95% CI of | 0.81 | 0.32 | 0.69 |
| OR Quart2 | 4.3 | 31 | 4.1 |
| OR Quart 3 | 1.7 | 3.1 | 1.8 |
| p Value | 0.21 | 0.33 | 0.19 |
| 95% CI of | 0.74 | 0.32 | 0.75 |
| OR Quart3 | 4.0 | 31 | 4.3 |
| OR Quart 4 | 1.0 | 4.3 | 0.90 |
| p Value | 0.97 | 0.20 | 0.83 |
| 95% CI of | 0.41 | 0.46 | 0.34 |
| OR Quart4 | 2.5 | 39 | 2.4 |

**Follistatin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 6.79 | 10.6 | 7.20 | 19.0 | 6.69 | 9.59 |
| Average | 38.8 | 43.3 | 37.8 | 94.8 | 41.7 | 46.2 |
| Stdev | 402 | 150 | 371 | 279 | 441 | 159 |
| p(t-test) | | 0.91 | | 0.49 | | 0.92 |
| Min | 0.000347 | 0.224 | 0.000347 | 4.40 | 0.000347 | 0.224 |
| Max | 8290 | 1300 | 8290 | 1300 | 8290 | 1300 |
| n (Samp) | 428 | 108 | 507 | 21 | 355 | 96 |
| n (Patient) | 428 | 108 | 507 | 21 | 355 | 96 |

| | At Enrollment | | |
|---|---|---|---|
| | sCr or UO | sCr only | UO only |
| AUC | 0.60 | 0.73 | 0.59 |
| SE | 0.032 | 0.064 | 0.034 |
| p | 0.0023 | 2.7E-4 | 0.0054 |
| nCohort 1 | 428 | 507 | 355 |
| nCohort 2 | 108 | 21 | 96 |
| Cutoff 1 | 4.96 | 11.9 | 4.69 |
| Sens 1 | 70% | 71% | 71% |
| Spec 1 | 39% | 66% | 38% |
| Cutoff 2 | 4.39 | 8.95 | 4.00 |
| Sens 2 | 81% | 81% | 80% |
| Spec 2 | 36% | 57% | 34% |
| Cutoff 3 | 2.44 | 5.51 | 2.40 |
| Sens 3 | 91% | 90% | 91% |
| Spec 3 | 23% | 42% | 22% |
| Cutoff 4 | 13.1 | 13.7 | 12.8 |
| Sens 4 | 43% | 67% | 43% |
| Spec 4 | 70% | 70% | 70% |
| Cutoff 5 | 22.8 | 23.6 | 19.6 |
| Sens 5 | 27% | 33% | 32% |
| Spec 5 | 80% | 80% | 80% |
| Cutoff 6 | 45.9 | 47.8 | 40.0 |
| Sens 6 | 18% | 29% | 19% |
| Spec 6 | 90% | 90% | 90% |
| OR Quart 2 | 1.9 | >4.1 | 1.5 |

|  | At Enrollment |  |  |
|---|---|---|---|
|  | sCr or UO | sCr only | UO only |
| p Value | 0.070 | <0.21 | 0.23 |
| 95% CI of | 0.95 | >0.45 | 0.76 |
| OR Quart2 | 3.6 | na | 3.1 |
| OR Quart 3 | 1.9 | >7.4 | 1.5 |
| p Value | 0.050 | <0.063 | 0.23 |
| 95% CI of | 1.00 | >0.90 | 0.76 |
| OR Quart3 | 3.8 | na | 3.1 |
| OR Quart 4 | 2.8 | >11 | 2.6 |
| p Value | 0.0017 | <0.024 | 0.0051 |
| 95% CI of | 1.5 | >1.4 | 1.3 |
| OR Quart4 | 5.4 | na | 5.0 |

**Vitamin D-binding protein**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 139 | 425 | 162 | 567 | 152 | 383 |
| Average | 1730 | 8720 | 2980 | 12000 | 2400 | 7380 |
| Stdev | 7110 | 31500 | 16300 | 22200 | 9120 | 32200 |
| p(t-test) |  | 0.0051 |  | 0.078 |  | 0.064 |
| Min | 0.200 | 0.337 | 0.259 | 31.9 | 0.200 | 0.337 |
| Max | 63300 | 221000 | 221000 | 62500 | 63300 | 221000 |
| n (Samp) | 189 | 56 | 229 | 11 | 187 | 49 |
| n (Patient) | 189 | 56 | 229 | 11 | 187 | 49 |

|  | At Enrollment |  |  |
|---|---|---|---|
|  | sCr or UO | sCr only | UO only |
| AUC | 0.67 | 0.70 | 0.63 |
| SE | 0.043 | 0.090 | 0.047 |
| p | 1.4E-4 | 0.024 | 0.0057 |
| nCohort 1 | 189 | 229 | 187 |
| nCohort 2 | 56 | 11 | 49 |
| Cutoff 1 | 160 | 301 | 121 |
| Sens 1 | 71% | 73% | 71% |
| Spec 1 | 54% | 64% | 45% |
| Cutoff 2 | 80.3 | 229 | 78.7 |
| Sens 2 | 80% | 82% | 82% |
| Spec 2 | 35% | 59% | 34% |
| Cutoff 3 | 41.5 | 80.3 | 32.2 |
| Sens 3 | 91% | 91% | 92% |
| Spec 3 | 16% | 32% | 11% |
| Cutoff 4 | 325 | 435 | 350 |
| Sens 4 | 57% | 55% | 53% |
| Spec 4 | 70% | 70% | 70% |
| Cutoff 5 | 662 | 876 | 683 |
| Sens 5 | 41% | 45% | 41% |
| Spec 5 | 80% | 80% | 80% |
| Cutoff 6 | 1670 | 4210 | 3130 |
| Sens 6 | 32% | 36% | 22% |
| Spec 6 | 90% | 90% | 90% |
| OR Quart 2 | 0.87 | 1.0 | 0.87 |
| p Value | 0.79 | 1.0 | 0.79 |
| 95% CI of | 0.31 | 0.061 | 0.31 |

|  | At Enrollment |  |  |
|---|---|---|---|
|  | sCr or UO | sCr only | UO only |
| OR Quart2 | 2.4 | 16 | 2.4 |
| OR Quart 3 | 2.1 | 4.2 | 1.4 |
| p Value | 0.12 | 0.20 | 0.47 |
| 95% CI of OR Quart3 | 0.83 5.1 | 0.46 39 | 0.55 3.7 |
| OR Quart 4 | 3.4 | 5.4 | 2.8 |
| p Value | 0.0061 | 0.13 | 0.021 |
| 95% CI of OR Quart4 | 1.4 8.2 | 0.61 47 | 1.2 6.9 |

**Glucagon**

|  | sCr or UO |  | sCr only |  | UO only |  |
|---|---|---|---|---|---|---|
|  | Cohort 1 | Cohort 2 | Cohort 1 | Cohort 2 | Cohort 1 | Cohort 2 |
| Median | 238 | 347 | nd | nd | 238 | 347 |
| Average | 1410 | 3470 | nd | nd | 1260 | 3360 |
| Stdev | 2870 | 4300 | nd | nd | 2630 | 4250 |
| p(t-test) |  | 0.0045 | nd | nd |  | 0.0037 |
| Min | 1.98 | 5.61 | nd | nd | 1.98 | 5.61 |
| Max | 9330 | 9330 | nd | nd | 9330 | 9330 |
| n (Samp) | 111 | 24 | nd | nd | 92 | 22 |
| n (Patient) | 111 | 24 | nd | nd | 92 | 22 |

|  | At Enrollment |  |  |
|---|---|---|---|
|  | sCr or UO | sCr only | UO only |
| AUC | 0.63 | nd | 0.63 |
| SE | 0.066 | nd | 0.069 |
| p | 0.057 | nd | 0.060 |
| nCohort 1 | 111 | nd | 92 |
| nCohort 2 | 24 | nd | 22 |
| Cutoff 1 | 41.7 | nd | 41.7 |
| Sens 1 | 71% | nd | 73% |
| Spec 1 | 39% | nd | 39% |
| Cutoff 2 | 8.21 | nd | 8.21 |
| Sens 2 | 83% | nd | 82% |
| Spec 2 | 27% | nd | 28% |
| Cutoff 3 | 6.53 | nd | 6.52 |
| Sens 3 | 92% | nd | 91% |
| Spec 3 | 20% | nd | 21% |
| Cutoff 4 | 347 | nd | 347 |
| Sens 4 | 46% | nd | 45% |
| Spec 4 | 76% | nd | 76% |
| Cutoff 5 | 2420 | nd | 2420 |
| Sens 5 | 33% | nd | 32% |
| Spec 5 | 89% | nd | 91% |
| Cutoff 6 | 9330 | nd | 2420 |
| Sens 6 | 0% | nd | 32% |
| Spec 6 | 100% | nd | 91% |
| OR Quart 2 | 1.2 | nd | 0.96 |
| p Value | 0.76 | nd | 0.96 |
| 95% CI of OR Quart2 | 0.30 5.1 | nd | 0.22 4.3 |
| OR Quart 3 | 0.97 | nd | 1.0 |

|  | At Enrollment | | |
|---|---|---|---|
|  | sCr or UO | sCr only | UO only |
| p Value | 0.96 | nd | 1.0 |
| 95% CI of OR Quart3 | 0.22 4.2 | nd nd | 0.22 4.5 |
| OR Quart 4 | 3.5 | nd | 3.2 |
| p Value | 0.055 | nd | 0.085 |
| 95% CI of OR Quart4 | 0.98 12 | nd nd | 0.86 12 |

**Interleukin-17A**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.00360 | 0.00409 | 0.00360 | 0.00537 | 0.00360 | 0.00409 |
| Average | 0.858 | 1.17 | 0.906 | 1.50 | 0.743 | 1.35 |
| Stdev | 6.05 | 4.58 | 5.89 | 4.22 | 5.58 | 4.91 |
| p(t-test) |  | 0.57 |  | 0.59 |  | 0.28 |
| Min | 0.000344 | 0.000344 | 0.000344 | 0.000380 | 0.000344 | 0.000344 |
| Max | 105 | 39.1 | 105 | 18.8 | 105 | 39.1 |
| n (Samp) | 525 | 138 | 623 | 30 | 448 | 119 |
| n (Patient) | 525 | 138 | 623 | 30 | 448 | 119 |

|  | At Enrollment | | |
|---|---|---|---|
|  | sCr or UO | sCr only | UO only |
| AUC | 0.54 | 0.52 | 0.56 |
| SE | 0.028 | 0.055 | 0.030 |
| p | 0.19 | 0.66 | 0.045 |
| nCohort 1 | 525 | 623 | 448 |
| nCohort 2 | 138 | 30 | 119 |
| Cutoff 1 | 0.00287 | 0.00172 | 0.00303 |
| Sens 1 | 71% | 70% | 71% |
| Spec 1 | 38% | 22% | 44% |
| Cutoff 2 | 0.000734 | 0.000734 | 0.000734 |
| Sens 2 | 82% | 80% | 82% |
| Spec 2 | 14% | 15% | 18% |
| Cutoff 3 | 0.000410 | 0.000380 | 0.000410 |
| Sens 3 | 91% | 97% | 92% |
| Spec 3 | 5% | 3% | 6% |
| Cutoff 4 | 0.00988 | 0.0127 | 0.00988 |
| Sens 4 | 33% | 33% | 35% |
| Spec 4 | 70% | 70% | 70% |
| Cutoff 5 | 0.121 | 0.150 | 0.129 |
| Sens 5 | 28% | 23% | 30% |
| Spec 5 | 80% | 80% | 80% |
| Cutoff 6 | 0.682 | 0.766 | 0.668 |
| Sens 6 | 17% | 17% | 19% |
| Spec 6 | 90% | 90% | 90% |
| OR Quart 2 | 0.55 | 0.085 | 1.1 |
| p Value | 0.042 | 0.019 | 0.78 |
| 95% CI of OR Quart2 | 0.31 0.98 | 0.011 0.67 | 0.59 2.0 |
| OR Quart 3 | 1.1 | 0.71 | 1.4 |
| p Value | 0.72 | 0.48 | 0.25 |
| 95% CI of | 0.66 | 0.28 | 0.79 |

| | At Enrollment | | |
|---|---|---|---|
| | sCr or UO | sCr only | UO only |
| OR Quart3 | 1.8 | 1.8 | 2.6 |
| OR Quart 4 | 1.2 | 0.90 | 1.7 |
| p Value | 0.54 | 0.81 | 0.066 |
| 95% CI of | 0.71 | 0.37 | 0.96 |
| OR Quart4 | 2.0 | 2.2 | 3.1 |

**Insulin receptor substrate 1**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 0.000546 | 0.00110 | 0.000546 | 0.00148 | 0.000546 | 0.000578 |
| Average | 0.00521 | 0.00831 | 0.00618 | 0.00178 | 0.00522 | 0.00930 |
| Stdev | 0.0509 | 0.0384 | 0.0501 | 0.00164 | 0.0512 | 0.0409 |
| p(t-test) | | 0.67 | | 0.77 | | 0.60 |
| Min | 1.25E-9 | 1.25E-9 | 1.25E-9 | 7.25E-7 | 1.25E-9 | 1.25E-9 |
| Max | 0.735 | 0.282 | 0.735 | 0.00563 | 0.735 | 0.282 |
| n (Samp) | 209 | 57 | 249 | 11 | 207 | 50 |
| n (Patient) | 209 | 57 | 249 | 11 | 207 | 50 |

| | At Enrollment | | |
|---|---|---|---|
| | sCr or UO | sCr only | UO only |
| AUC | 0.55 | 0.62 | 0.55 |
| SE | 0.044 | 0.092 | 0.046 |
| p | 0.26 | 0.19 | 0.25 |
| nCohort 1 | 209 | 249 | 207 |
| nCohort 2 | 57 | 11 | 50 |
| Cutoff 1 | 2.32E-6 | 0.000646 | 2.32E-6 |
| Sens 1 | 75% | 73% | 74% |
| Spec 1 | 30% | 55% | 31% |
| Cutoff 2 | 9.90E-7 | 2.78E-5 | 9.90E-7 |
| Sens 2 | 84% | 82% | 84% |
| Spec 2 | 13% | 45% | 14% |
| Cutoff 3 | 1.25E-9 | 2.32E-6 | 1.25E-9 |
| Sens 3 | 96% | 91% | 96% |
| Spec 3 | 7% | 30% | 7% |
| Cutoff 4 | 0.00229 | 0.00229 | 0.00148 |
| Sens 4 | 35% | 36% | 42% |
| Spec 4 | 75% | 73% | 71% |
| Cutoff 5 | 0.00243 | 0.00243 | 0.00243 |
| Sens 5 | 25% | 18% | 26% |
| Spec 5 | 85% | 83% | 85% |
| Cutoff 6 | 0.00303 | 0.00334 | 0.00303 |
| Sens 6 | 18% | 9% | 20% |
| Spec 6 | 90% | 91% | 90% |
| OR Quart 2 | 1.1 | 2.0 | 1.0 |
| p Value | 0.86 | 0.57 | 1.0 |
| 95% CI of | 0.46 | 0.18 | 0.41 |
| OR Quart2 | 2.5 | 23 | 2.4 |
| OR Quart 3 | 1.3 | 4.2 | 0.62 |
| p Value | 0.53 | 0.21 | 0.33 |
| 95% CI of | 0.57 | 0.46 | 0.23 |
| OR Quart3 | 3.0 | 39 | 1.6 |
| OR Quart 4 | 1.1 | 4.2 | 1.7 |

|  | At Enrollment | | |
|---|---|---|---|
|  | sCr or UO | sCr only | UO only |
| p Value | 0.86 | 0.21 | 0.23 |
| 95% CI of | 0.46 | 0.46 | 0.72 |
| OR Quart4 | 2.5 | 39 | 3.8 |

**Involucrin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.462 | 0.811 | 0.507 | 0.612 | 0.487 | 1.05 |
| Average | 1.05 | 2.51 | 1.26 | 2.28 | 1.10 | 2.56 |
| Stdev | 2.17 | 4.01 | 2.61 | 3.12 | 2.35 | 4.10 |
| p(t-test) |  | 2.2E-6 |  | 0.11 |  | 3.3E-5 |
| Min | 0.0119 | 0.0294 | 0.0119 | 0.0767 | 0.0191 | 0.0294 |
| Max | 31.3 | 19.7 | 31.3 | 9.64 | 31.3 | 19.7 |
| n (Samp) | 433 | 84 | 491 | 17 | 362 | 74 |
| n (Patient) | 433 | 84 | 491 | 17 | 362 | 74 |

|  | At Enrollment | | |
|---|---|---|---|
|  | sCr or UO | sCr only | UO only |
| AUC | 0.66 | 0.56 | 0.67 |
| SE | 0.035 | 0.073 | 0.037 |
| p | 6.8E-6 | 0.44 | 5.9E-6 |
| nCohort 1 | 433 | 491 | 362 |
| nCohort 2 | 84 | 17 | 74 |
| Cutoff 1 | 0.450 | 0.246 | 0.489 |
| Sens 1 | 70% | 71% | 70% |
| Spec 1 | 49% | 29% | 50% |
| Cutoff 2 | 0.274 | 0.191 | 0.357 |
| Sens 2 | 81% | 82% | 81% |
| Spec 2 | 34% | 22% | 42% |
| Cutoff 3 | 0.185 | 0.114 | 0.185 |
| Sens 3 | 90% | 94% | 91% |
| Spec 3 | 23% | 13% | 21% |
| Cutoff 4 | 0.905 | 1.00 | 0.916 |
| Sens 4 | 49% | 35% | 51% |
| Spec 4 | 70% | 70% | 70% |
| Cutoff 5 | 1.36 | 1.59 | 1.36 |
| Sens 5 | 45% | 35% | 47% |
| Spec 5 | 80% | 80% | 80% |
| Cutoff 6 | 2.24 | 2.64 | 2.19 |
| Sens 6 | 29% | 29% | 30% |
| Spec 6 | 90% | 90% | 90% |
| OR Quart 2 | 1.6 | 1.0 | 1.8 |
| p Value | 0.23 | 1.0 | 0.20 |
| 95% CI of | 0.73 | 0.24 | 0.74 |
| OR Quart2 | 3.6 | 4.1 | 4.2 |
| OR Quart 3 | 1.7 | 0.74 | 1.8 |
| p Value | 0.17 | 0.70 | 0.20 |
| 95% CI of | 0.79 | 0.16 | 0.74 |
| OR Quart3 | 3.8 | 3.4 | 4.2 |
| OR Quart 4 | 4.4 | 1.5 | 5.3 |
| p Value | 5.6E-5 | 0.52 | 4.0E-5 |
| 95% CI of | 2.1 | 0.42 | 2.4 |

|  | At Enrollment | | |
|---|---|---|---|
|  | sCr or UO | sCr only | UO only |
| OR Quart4 | 9.1 | 5.5 | 12 |

**Lipopolysaccharide (serotypes -K,-O)**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 2.53E-5 | 9.99E-5 | 2.53E-5 | 0.00200 | 4.34E-5 | 9.88E-5 |
| Average | 0.136 | 0.107 | 0.124 | 0.420 | 0.118 | 0.0993 |
| Stdev | 0.690 | 0.444 | 0.650 | 0.920 | 0.603 | 0.459 |
| p(t-test) |  | 0.71 |  | 0.069 |  | 0.80 |
| Min | 1.40E-8 | 4.54E-6 | 1.40E-8 | 4.54E-6 | 2.09E-8 | 6.10E-6 |
| Max | 6.42 | 3.10 | 6.42 | 3.10 | 5.44 | 3.10 |
| n (Samp) | 433 | 84 | 491 | 17 | 362 | 74 |
| n (Patient) | 433 | 84 | 491 | 17 | 362 | 74 |

|  | At Enrollment | | |
|---|---|---|---|
|  | sCr or UO | sCr only | UO only |
| AUC | 0.54 | 0.63 | 0.52 |
| SE | 0.035 | 0.074 | 0.037 |
| p | 0.30 | 0.071 | 0.56 |
| nCohort 1 | 433 | 491 | 362 |
| nCohort 2 | 84 | 17 | 74 |
| Cutoff 1 | 1.48E-5 | 1.80E-5 | 1.48E-5 |
| Sens 1 | 70% | 71% | 70% |
| Spec 1 | 30% | 42% | 31% |
| Cutoff 2 | 1.14E-5 | 1.48E-5 | 1.14E-5 |
| Sens 2 | 81% | 82% | 81% |
| Spec 2 | 18% | 30% | 18% |
| Cutoff 3 | 4.54E-6 | 2.09E-8 | 4.54E-6 |
| Sens 3 | 98% | 100% | 100% |
| Spec 3 | 5% | 1% | 4% |
| Cutoff 4 | 0.00110 | 0.00134 | 0.00159 |
| Sens 4 | 37% | 53% | 34% |
| Spec 4 | 70% | 70% | 70% |
| Cutoff 5 | 0.00662 | 0.00713 | 0.00713 |
| Sens 5 | 26% | 41% | 23% |
| Spec 5 | 80% | 80% | 80% |
| Cutoff 6 | 0.0358 | 0.0396 | 0.0396 |
| Sens 6 | 17% | 35% | 14% |
| Spec 6 | 90% | 90% | 90% |
| OR Quart 2 | 0.53 | 1.0 | 0.66 |
| p Value | 0.082 | 1.0 | 0.26 |
| 95% CI of | 0.26 | 0.20 | 0.31 |
| OR Quart2 | 1.1 | 5.1 | 1.4 |
| OR Quart 3 | 0.76 | 1.0 | 1.0 |
| p Value | 0.40 | 1.0 | 1.0 |
| 95% CI of | 0.39 | 0.20 | 0.50 |
| OR Quart3 | 1.5 | 5.1 | 2.0 |
| OR Quart 4 | 1.1 | 2.8 | 1.0 |
| p Value | 0.66 | 0.14 | 1.0 |
| 95% CI of | 0.62 | 0.72 | 0.50 |
| OR Quart4 | 2.1 | 11 | 2.0 |

**Collagenase 3**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.230 | 3.88 | 1.29 | 0.202 | 1.23 | 6.71 |
| Average | 10.1 | 10.6 | 9.83 | 4.52 | 10.1 | 12.0 |
| Stdev | 22.3 | 16.2 | 20.7 | 12.5 | 22.4 | 16.8 |
| p(t-test) |  | 0.86 |  | 0.40 |  | 0.59 |
| Min | 0.00479 | 0.00479 | 0.00479 | 0.00479 | 0.00479 | 0.00479 |
| Max | 182 | 79.6 | 182 | 41.9 | 182 | 79.6 |
| n (Samp) | 189 | 56 | 229 | 11 | 187 | 49 |
| n (Patient) | 189 | 56 | 229 | 11 | 187 | 49 |

|  | At Enrollment | | |
|---|---|---|---|
|  | sCr or UO | sCr only | UO only |
| AUC | 0.54 | 0.35 | 0.58 |
| SE | 0.044 | 0.092 | 0.047 |
| p | 0.42 | 0.11 | 0.091 |
| nCohort 1 | 189 | 229 | 187 |
| nCohort 2 | 56 | 11 | 49 |
| Cutoff 1 | 0.0466 | 0.00741 | 0.0466 |
| Sens 1 | 75% | 73% | 80% |
| Spec 1 | 29% | 12% | 32% |
| Cutoff 2 | 0.0198 | 0 | 0.0425 |
| Sens 2 | 84% | 100% | 82% |
| Spec 2 | 19% | 0% | 29% |
| Cutoff 3 | 0 | 0 | 0.00519 |
| Sens 3 | 100% | 100% | 92% |
| Spec 3 | 0% | 0% | 4% |
| Cutoff 4 | 7.62 | 8.97 | 7.51 |
| Sens 4 | 43% | 9% | 49% |
| Spec 4 | 72% | 70% | 70% |
| Cutoff 5 | 15.2 | 15.2 | 15.2 |
| Sens 5 | 23% | 9% | 27% |
| Spec 5 | 80% | 80% | 80% |
| Cutoff 6 | 29.8 | 29.8 | 29.8 |
| Sens 6 | 12% | 9% | 14% |
| Spec 6 | 90% | 90% | 90% |
| OR Quart 2 | 0.90 | 3.1 | 1.1 |
| p Value | 0.82 | 0.33 | 0.80 |
| 95% CI of | 0.36 | 0.31 | 0.42 |
| OR Quart2 | 2.2 | 31 | 3.0 |
| OR Quart 3 | 1.6 | 3.1 | 1.9 |
| p Value | 0.29 | 0.33 | 0.17 |
| 95% CI of | 0.68 | 0.31 | 0.75 |
| OR Quart3 | 3.7 | 31 | 4.8 |
| OR Quart 4 | 1.4 | 4.2 | 1.9 |
| p Value | 0.42 | 0.20 | 0.17 |
| 95% CI of | 0.61 | 0.46 | 0.75 |
| OR Quart4 | 3.3 | 39 | 4.8 |

**NF-kappa-B inhibitor alpha**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 3.20E-5 | 0.000211 | 9.81E-5 | 0.000566 | 3.20E-5 | 0.000202 |

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Average | 0.00582 | 0.00754 | 0.00516 | 0.0325 | 0.00631 | 0.00660 |
| Stdev | 0.0293 | 0.0351 | 0.0269 | 0.0776 | 0.0298 | 0.0363 |
| p(t-test) |  | 0.71 |  | 0.0039 |  | 0.95 |
| Min | 3.52E-7 | 3.52E-7 | 3.52E-7 | 9.58E-7 | 3.52E-7 | 3.52E-7 |
| Max | 0.268 | 0.257 | 0.268 | 0.257 | 0.268 | 0.257 |
| n (Samp) | 209 | 57 | 249 | 11 | 207 | 50 |
| n (Patient) | 209 | 57 | 249 | 11 | 207 | 50 |

|  | At Enrollment | | |
|---|---|---|---|
|  | sCr or UO | sCr only | UO only |
| AUC | 0.58 | 0.63 | 0.56 |
| SE | 0.044 | 0.092 | 0.046 |
| p | 0.077 | 0.14 | 0.17 |
| nCohort 1 | 209 | 249 | 207 |
| nCohort 2 | 57 | 11 | 50 |
| Cutoff 1 | 2.62E-5 | 2.94E-5 | 2.62E-5 |
| Sens 1 | 70% | 73% | 70% |
| Spec 1 | 37% | 38% | 37% |
| Cutoff 2 | 1.03E-6 | 2.25E-5 | 1.03E-6 |
| Sens 2 | 82% | 82% | 82% |
| Spec 2 | 21% | 33% | 21% |
| Cutoff 3 | 5.23E-7 | 6.66E-6 | 5.23E-7 |
| Sens 3 | 93% | 91% | 92% |
| Spec 3 | 17% | 31% | 17% |
| Cutoff 4 | 0.000493 | 0.000532 | 0.000523 |
| Sens 4 | 42% | 55% | 40% |
| Spec 4 | 71% | 71% | 71% |
| Cutoff 5 | 0.00113 | 0.00128 | 0.00113 |
| Sens 5 | 25% | 27% | 24% |
| Spec 5 | 81% | 80% | 81% |
| Cutoff 6 | 0.00351 | 0.00427 | 0.00381 |
| Sens 6 | 19% | 27% | 16% |
| Spec 6 | 90% | 90% | 90% |
| OR Quart 2 | 0.79 | 4.2 | 0.69 |
| p Value | 0.61 | 0.21 | 0.46 |
| 95% CI of | 0.32 | 0.46 | 0.26 |
| OR Quart2 | 2.0 | 39 | 1.8 |
| OR Quart 3 | 1.7 | 1.0 | 1.9 |
| p Value | 0.22 | 1.0 | 0.14 |
| 95% CI of | 0.74 | 0.061 | 0.81 |
| OR Quart3 | 3.9 | 16 | 4.4 |
| OR Quart 4 | 1.5 | 5.3 | 1.2 |
| p Value | 0.32 | 0.13 | 0.68 |
| 95% CI of | 0.67 | 0.61 | 0.49 |
| OR Quart4 | 3.5 | 47 | 2.9 |

**Beta-nerve growth factor**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 2.84 | 1.92 | 2.82 | 2.35 | 2.83 | 1.61 |
| Average | 4.22 | 3.48 | 4.21 | 2.38 | 4.19 | 3.65 |
| Stdev | 4.78 | 4.95 | 4.92 | 1.82 | 4.77 | 5.25 |

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| p(t-test) |  | 0.31 |  | 0.22 |  | 0.48 |
| Min | 0.000201 | 0.000506 | 0.000201 | 0.0483 | 0.000201 | 0.000506 |
| Max | 29.2 | 25.2 | 29.2 | 6.35 | 29.2 | 25.2 |
| n (Samp) | 210 | 57 | 250 | 11 | 209 | 50 |
| n (Patient) | 210 | 57 | 250 | 11 | 209 | 50 |

|  | At Enrollment | | |
|---|---|---|---|
|  | sCr or UO | sCr only | UO only |
| AUC | 0.41 | 0.41 | 0.42 |
| SE | 0.044 | 0.092 | 0.046 |
| p | 0.046 | 0.31 | 0.067 |
| nCohort 1 | 210 | 250 | 209 |
| nCohort 2 | 57 | 11 | 50 |
| Cutoff 1 | 0.937 | 0.937 | 0.869 |
| Sens 1 | 70% | 73% | 70% |
| Spec 1 | 19% | 20% | 17% |
| Cutoff 2 | 0.676 | 0.781 | 0.676 |
| Sens 2 | 81% | 82% | 80% |
| Spec 2 | 13% | 16% | 13% |
| Cutoff 3 | 0.000506 | 0.771 | 0.000506 |
| Sens 3 | 96% | 91% | 96% |
| Spec 3 | 2% | 16% | 2% |
| Cutoff 4 | 4.15 | 4.15 | 4.02 |
| Sens 4 | 23% | 9% | 26% |
| Spec 4 | 70% | 70% | 70% |
| Cutoff 5 | 5.99 | 5.99 | 5.99 |
| Sens 5 | 16% | 9% | 18% |
| Spec 5 | 80% | 80% | 80% |
| Cutoff 6 | 9.80 | 9.80 | 11.0 |
| Sens 6 | 7% | 0% | 8% |
| Spec 6 | 90% | 90% | 90% |
| OR Quart 2 | 1.2 | 2.1 | 0.79 |
| p Value | 0.65 | 0.56 | 0.63 |
| 95% CI of | 0.51 | 0.18 | 0.30 |
| OR Quart2 | 3.0 | 23 | 2.1 |
| OR Quart 3 | 0.89 | 4.3 | 0.89 |
| p Value | 0.81 | 0.20 | 0.81 |
| 95% CI of | 0.35 | 0.46 | 0.35 |
| OR Quart3 | 2.3 | 39 | 2.3 |
| OR Quart 4 | 2.7 | 4.3 | 2.2 |
| p Value | 0.017 | 0.20 | 0.060 |
| 95% CI of | 1.2 | 0.46 | 0.97 |
| OR Quart4 | 6.2 | 39 | 5.2 |

**Transthyretin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 24.4 | 46.1 | 26.7 | 54.5 | 25.1 | 38.0 |
| Average | 47.5 | 112 | 60.1 | 120 | 50.7 | 115 |
| Stdev | 78.1 | 259 | 141 | 179 | 80.5 | 274 |
| p(t-test) |  | 7.5E-4 |  | 0.16 |  | 0.0021 |
| Min | 0.164 | 1.67 | 0.164 | 3.29 | 0.430 | 1.67 |

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Max | 648 | 2000 | 2000 | 638 | 648 | 2000 |
| n (Samp) | 247 | 69 | 298 | 12 | 234 | 61 |
| n (Patient) | 247 | 69 | 298 | 12 | 234 | 61 |

|  | At Enrollment | | |
|---|---|---|---|
|  | sCr or UO | sCr only | UO only |
| AUC | 0.64 | 0.64 | 0.61 |
| SE | 0.039 | 0.088 | 0.042 |
| p | 4.7E-4 | 0.10 | 0.0067 |
| nCohort 1 | 247 | 298 | 234 |
| nCohort 2 | 69 | 12 | 61 |
| Cutoff 1 | 19.2 | 21.7 | 19.2 |
| Sens 1 | 71% | 75% | 70% |
| Spec 1 | 41% | 42% | 39% |
| Cutoff 2 | 14.2 | 19.2 | 14.6 |
| Sens 2 | 81% | 83% | 80% |
| Spec 2 | 32% | 38% | 31% |
| Cutoff 3 | 8.67 | 10.0 | 11.0 |
| Sens 3 | 91% | 92% | 90% |
| Spec 3 | 20% | 21% | 24% |
| Cutoff 4 | 43.4 | 48.3 | 46.2 |
| Sens 4 | 51% | 58% | 46% |
| Spec 4 | 70% | 70% | 70% |
| Cutoff 5 | 60.7 | 73.6 | 67.7 |
| Sens 5 | 41% | 42% | 39% |
| Spec 5 | 80% | 80% | 80% |
| Cutoff 6 | 108 | 120 | 116 |
| Sens 6 | 25% | 33% | 20% |
| Spec 6 | 90% | 90% | 90% |
| OR Quart 2 | 1.8 | 0.99 | 1.8 |
| p Value | 0.20 | 0.99 | 0.20 |
| 95% CI of | 0.74 | 0.14 | 0.74 |
| OR Quart2 | 4.1 | 7.2 | 4.4 |
| OR Quart 3 | 1.6 | 1.5 | 1.5 |
| p Value | 0.28 | 0.65 | 0.38 |
| 95% CI of | 0.68 | 0.25 | 0.60 |
| OR Quart3 | 3.9 | 9.4 | 3.8 |
| OR Quart 4 | 3.8 | 2.6 | 3.4 |
| p Value | 0.0012 | 0.27 | 0.0047 |
| 95% CI of | 1.7 | 0.48 | 1.5 |
| OR Quart4 | 8.5 | 14 | 8.0 |

Table 12: Comparison of marker levels in enroll EDTA samples collected from Cohort 1 (patients that did not progress beyond RIFLE stage 0 or R within 48hrs) and in enroll EDTA samples collected from Cohort 2 (subjects reaching RIFLE stage I or F within 48hrs). Enroll samples from patients already at stage I or F were included in Cohort 2.

**Agouti-related protein**

|  | sCr or UO | sCr only | UO only |
|---|---|---|---|

| | Cohort 1 | Cohort 2 | Cohort 1 | Cohort 2 | Cohort 1 | Cohort 2 |
|---|---|---|---|---|---|---|
| Median | 0.0557 | 0.0789 | nd | nd | 0.0560 | 0.0855 |
| Average | 0.0721 | 0.108 | nd | nd | 0.0730 | 0.111 |
| Stdev | 0.0486 | 0.0802 | nd | nd | 0.0489 | 0.0811 |
| p(t-test) | | 0.0036 | nd | nd | | 0.0031 |
| Min | 0.00892 | 0.0112 | nd | nd | 0.00892 | 0.0112 |
| Max | 0.224 | 0.335 | nd | nd | 0.224 | 0.335 |
| n (Samp) | 120 | 24 | nd | nd | 117 | 23 |
| n (Patient) | 120 | 24 | nd | nd | 117 | 23 |

| | At Enrollment | | |
|---|---|---|---|
| | sCr or UO | sCr only | UO only |
| AUC | 0.66 | nd | 0.66 |
| SE | 0.065 | nd | 0.066 |
| p | 0.014 | nd | 0.015 |
| nCohort 1 | 120 | nd | 117 |
| nCohort 2 | 24 | nd | 23 |
| Cutoff 1 | 0.0560 | nd | 0.0560 |
| Sens 1 | 71% | nd | 74% |
| Spec 1 | 52% | nd | 50% |
| Cutoff 2 | 0.0500 | nd | 0.0500 |
| Sens 2 | 83% | nd | 83% |
| Spec 2 | 44% | nd | 43% |
| Cutoff 3 | 0.0434 | nd | 0.0434 |
| Sens 3 | 92% | nd | 91% |
| Spec 3 | 35% | nd | 33% |
| Cutoff 4 | 0.0845 | nd | 0.0846 |
| Sens 4 | 50% | nd | 52% |
| Spec 4 | 70% | nd | 70% |
| Cutoff 5 | 0.104 | nd | 0.104 |
| Sens 5 | 38% | nd | 39% |
| Spec 5 | 80% | nd | 80% |
| Cutoff 6 | 0.141 | nd | 0.141 |
| Sens 6 | 25% | nd | 26% |
| Spec 6 | 90% | nd | 91% |
| OR Quart 2 | 8.4 | nd | 7.0 |
| p Value | 0.052 | nd | 0.079 |
| 95% CI of | 0.98 | nd | 0.80 |
| OR Quart2 | 73 | nd | 62 |
| OR Quart 3 | 8.4 | nd | 8.5 |
| p Value | 0.052 | nd | 0.052 |
| 95% CI of | 0.98 | nd | 0.99 |
| OR Quart3 | 73 | nd | 73 |
| OR Quart 4 | 12 | nd | 12 |
| p Value | 0.024 | nd | 0.023 |
| 95% CI of | 1.4 | nd | 1.4 |
| OR Quart4 | 98 | nd | 99 |

**Osteocalcin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 2150 | 1740 | 2080 | 1270 | 2110 | 1850 |
| Average | 3030 | 2770 | 3030 | 1970 | 3070 | 2990 |
| Stdev | 3240 | 3170 | 3290 | 1550 | 3320 | 3290 |

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| p(t-test) |  | 0.66 |  | 0.39 |  | 0.89 |
| Min | 0.729 | 59.3 | 0.729 | 674 | 0.729 | 59.3 |
| Max | 21800 | 17700 | 21800 | 5090 | 21800 | 17700 |
| n (Samp) | 160 | 38 | 187 | 7 | 150 | 34 |
| n (Patient) | 160 | 38 | 187 | 7 | 150 | 34 |

|  | At Enrollment | | |
|---|---|---|---|
|  | sCr or UO | sCr only | UO only |
| AUC | 0.46 | 0.41 | 0.49 |
| SE | 0.053 | 0.12 | 0.055 |
| p | 0.46 | 0.45 | 0.88 |
| nCohort 1 | 160 | 187 | 150 |
| nCohort 2 | 38 | 7 | 34 |
| Cutoff 1 | 1100 | 991 | 1230 |
| Sens 1 | 71% | 71% | 71% |
| Spec 1 | 26% | 24% | 31% |
| Cutoff 2 | 855 | 860 | 854 |
| Sens 2 | 82% | 86% | 82% |
| Spcc 2 | 18% | 19% | 19% |
| Cutoff 3 | 672 | 672 | 686 |
| Sens 3 | 92% | 100% | 91% |
| Spec 3 | 12% | 12% | 15% |
| Cutoff 4 | 3280 | 3280 | 3290 |
| Sens 4 | 24% | 14% | 26% |
| Spec 4 | 70% | 70% | 70% |
| Cutoff 5 | 4300 | 4420 | 4300 |
| Sens 5 | 21% | 14% | 24% |
| Spec 5 | 80% | 80% | 80% |
| Cutoff 6 | 5740 | 6460 | 5810 |
| Sens 6 | 13% | 0% | 12% |
| Spec 6 | 90% | 90% | 90% |
| OR Quart 2 | 1.0 | 2.1 | 1.0 |
| p Value | 0.96 | 0.55 | 1.0 |
| 95% CI of | 0.35 | 0.18 | 0.34 |
| OR Quart2 | 3.0 | 24 | 2.9 |
| OR Quart 3 | 1.5 | 1.0 | 1.3 |
| p Value | 0.45 | 1.0 | 0.60 |
| 95% CI of | 0.54 | 0.061 | 0.47 |
| OR Quart3 | 4.1 | 16 | 3.7 |
| OR Quart 4 | 1.5 | 3.2 | 1.0 |
| p Value | 0.42 | 0.32 | 1.0 |
| 95% CI of | 0.55 | 0.32 | 0.34 |
| OR Quart4 | 4.2 | 32 | 2.9 |

**Follitropin subunit beta**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 7.89 | 4.24 | nd | nd | 7.48 | 4.21 |
| Average | 18.7 | 8.87 | nd | nd | 18.4 | 8.97 |
| Stdev | 25.7 | 11.5 | nd | nd | 25.4 | 11.8 |
| p(t-test) |  | 0.068 | nd | nd |  | 0.085 |
| Min | 0.0600 | 1.14 | nd | nd | 0.0600 | 1.14 |

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Max | 141 | 47.7 | nd | nd | 141 | 47.7 |
| n (Samp) | 120 | 24 | nd | nd | 117 | 23 |
| n (Patient) | 120 | 24 | nd | nd | 117 | 23 |

|  | At Enrollment | | |
|---|---|---|---|
|  | sCr or UO | sCr only | UO only |
| AUC | 0.38 | nd | 0.38 |
| SE | 0.066 | nd | 0.067 |
| p | 0.060 | nd | 0.065 |
| nCohort 1 | 120 | nd | 117 |
| nCohort 2 | 24 | nd | 23 |
| Cutoff 1 | 3.33 | nd | 3.05 |
| Sens 1 | 71% | nd | 74% |
| Spec 1 | 28% | nd | 26% |
| Cutoff 2 | 2.23 | nd | 2.23 |
| Sens 2 | 83% | nd | 83% |
| Spec 2 | 18% | nd | 18% |
| Cutoff 3 | 1.29 | nd | 1.29 |
| Sens 3 | 92% | nd | 91% |
| Spec 3 | 8% | nd | 8% |
| Cutoff 4 | 16.5 | nd | 16.1 |
| Sens 4 | 17% | nd | 17% |
| Spec 4 | 70% | nd | 70% |
| Cutoff 5 | 27.5 | nd | 27.5 |
| Sens 5 | 8% | nd | 9% |
| Spec 5 | 80% | nd | 80% |
| Cutoff 6 | 51.5 | nd | 51.5 |
| Sens 6 | 0% | nd | 0% |
| Spec 6 | 90% | nd | 91% |
| OR Quart 2 | 0.47 | nd | 0.47 |
| p Value | 0.40 | nd | 0.40 |
| 95% CI of | 0.081 | nd | 0.080 |
| OR Quart2 | 2.7 | nd | 2.7 |
| OR Quart 3 | 4.0 | nd | 3.6 |
| p Value | 0.030 | nd | 0.049 |
| 95% CI of | 1.1 | nd | 1.0 |
| OR Quart3 | 14 | nd | 13 |
| OR Quart 4 | 1.6 | nd | 1.6 |
| p Value | 0.50 | nd | 0.50 |
| 95% CI of | 0.41 | nd | 0.41 |
| OR Quart4 | 6.2 | nd | 6.3 |

**Follistatin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 912 | 864 | 912 | 846 | 962 | 864 |
| Average | 2970 | 2520 | 2870 | 1220 | 3120 | 2660 |
| Stdev | 4670 | 4030 | 4540 | 975 | 4790 | 4230 |
| p(t-test) |  | 0.58 |  | 0.34 |  | 0.61 |
| Min | 0.0206 | 8.66 | 0.0206 | 366 | 0.0206 | 8.66 |
| Max | 28900 | 15600 | 28900 | 3050 | 28900 | 15600 |
| n (Samp) | 163 | 38 | 190 | 7 | 153 | 34 |

|  | sCr or UO |  | sCr only |  | UO only |  |
|---|---|---|---|---|---|---|
|  | Cohort 1 | Cohort 2 | Cohort 1 | Cohort 2 | Cohort 1 | Cohort 2 |
| n (Patient) | 163 | 38 | 190 | 7 | 153 | 34 |

|  | At Enrollment |  |  |
|---|---|---|---|
|  | sCr or UO | sCr only | UO only |
| AUC | 0.49 | 0.48 | 0.48 |
| SE | 0.052 | 0.11 | 0.055 |
| p | 0.78 | 0.87 | 0.76 |
| nCohort 1 | 163 | 190 | 153 |
| nCohort 2 | 38 | 7 | 34 |
| Cutoff 1 | 486 | 609 | 486 |
| Sens 1 | 71% | 71% | 71% |
| Spec 1 | 23% | 32% | 24% |
| Cutoff 2 | 466 | 466 | 398 |
| Sens 2 | 82% | 86% | 82% |
| Spec 2 | 21% | 21% | 17% |
| Cutoff 3 | 314 | 360 | 314 |
| Sens 3 | 92% | 100% | 91% |
| Spec 3 | 12% | 14% | 12% |
| Cutoff 4 | 1390 | 1380 | 1430 |
| Sens 4 | 29% | 29% | 26% |
| Spec 4 | 71% | 70% | 71% |
| Cutoff 5 | 6420 | 2310 | 7700 |
| Sens 5 | 16% | 14% | 15% |
| Spec 5 | 80% | 80% | 80% |
| Cutoff 6 | 10900 | 10900 | 10900 |
| Sens 6 | 11% | 0% | 12% |
| Spec 6 | 90% | 90% | 90% |
| OR Quart 2 | 1.0 | 0.50 | 1.0 |
| p Value | 0.96 | 0.58 | 1.0 |
| 95% CI of | 0.37 | 0.044 | 0.34 |
| OR Quart2 | 2.8 | 5.7 | 2.9 |
| OR Quart 3 | 0.89 | 1.0 | 1.0 |
| p Value | 0.82 | 0.98 | 1.0 |
| 95% CI of | 0.31 | 0.14 | 0.34 |
| OR Quart3 | 2.5 | 7.6 | 2.9 |
| OR Quart 4 | 1.5 | 1.0 | 1.4 |
| p Value | 0.43 | 0.98 | 0.57 |
| 95% CI of | 0.56 | 0.14 | 0.48 |
| OR Quart4 | 3.9 | 7.6 | 3.8 |

**Vitamin D-binding protein**

|  | sCr or UO |  | sCr only |  | UO only |  |
|---|---|---|---|---|---|---|
|  | Cohort 1 | Cohort 2 | Cohort 1 | Cohort 2 | Cohort 1 | Cohort 2 |
| Median | 154000 | 148000 | nd | nd | 153000 | 145000 |
| Average | 166000 | 153000 | nd | nd | 167000 | 149000 |
| Stdev | 75600 | 70500 | nd | nd | 77200 | 68600 |
| p(t-test) |  | 0.39 | nd | nd |  | 0.25 |
| Min | 54900 | 22500 | nd | nd | 54900 | 22500 |
| Max | 531000 | 276000 | nd | nd | 531000 | 276000 |
| n (Samp) | 138 | 29 | nd | nd | 132 | 28 |
| n (Patient) | 138 | 29 | nd | nd | 132 | 28 |

| | At Enrollment | | |
|---|---|---|---|
| | sCr or UO | sCr only | UO only |
| AUC | 0.46 | nd | 0.45 |
| SE | 0.060 | nd | 0.061 |
| p | 0.55 | nd | 0.38 |
| nCohort 1 | 138 | nd | 132 |
| nCohort 2 | 29 | nd | 28 |
| Cutoff 1 | 102000 | nd | 102000 |
| Sens 1 | 72% | nd | 71% |
| Spec 1 | 14% | nd | 14% |
| Cutoff 2 | 77400 | nd | 77400 |
| Sens 2 | 83% | nd | 82% |
| Spec 2 | 5% | nd | 5% |
| Cutoff 3 | 69700 | nd | 69700 |
| Sens 3 | 93% | nd | 93% |
| Spec 3 | 4% | nd | 5% |
| Cutoff 4 | 176000 | nd | 178000 |
| Sens 4 | 31% | nd | 29% |
| Spec 4 | 70% | nd | 70% |
| Cutoff 5 | 215000 | nd | 217000 |
| Sens 5 | 24% | nd | 21% |
| Spec 5 | 80% | nd | 80% |
| Cutoff 6 | 250000 | nd | 250000 |
| Sens 6 | 14% | nd | 11% |
| Spec 6 | 91% | nd | 90% |
| OR Quart 2 | 1.0 | nd | 1.2 |
| p Value | 1.0 | nd | 0.76 |
| 95% CI of | 0.32 | nd | 0.37 |
| OR Quart2 | 3.2 | nd | 4.0 |
| OR Quart 3 | 0.68 | nd | 0.81 |
| p Value | 0.53 | nd | 0.75 |
| 95% CI of | 0.20 | nd | 0.23 |
| OR Quart3 | 2.3 | nd | 2.9 |
| OR Quart 4 | 1.6 | nd | 1.9 |
| p Value | 0.39 | nd | 0.27 |
| 95% CI of | 0.55 | nd | 0.61 |
| OR Quart4 | 4.8 | nd | 5.8 |

**Glucagon**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 74.3 | 206 | nd | nd | 74.4 | 297 |
| Average | 9680 | 12200 | nd | nd | 10100 | 12000 |
| Stdev | 17200 | 18700 | nd | nd | 17500 | 18700 |
| p(t-test) | | 0.57 | nd | nd | | 0.69 |
| Min | 11.1 | 21.2 | nd | nd | 11.1 | 21.2 |
| Max | 40100 | 40100 | nd | nd | 40100 | 40100 |
| n (Samp) | 75 | 20 | nd | nd | 64 | 17 |
| n (Patient) | 75 | 20 | nd | nd | 64 | 17 |

| | At Enrollment | | |
|---|---|---|---|
| | sCr or UO | sCr only | UO only |
| AUC | 0.56 | nd | 0.58 |
| SE | 0.074 | nd | 0.080 |

|  | At Enrollment | | |
|---|---|---|---|
|  | sCr or UO | sCr only | UO only |
| p | 0.44 | nd | 0.30 |
| nCohort 1 | 75 | nd | 64 |
| nCohort 2 | 20 | nd | 17 |
| Cutoff 1 | 48.5 | nd | 104 |
| Sens 1 | 70% | nd | 71% |
| Spec 1 | 37% | nd | 59% |
| Cutoff 2 | 14.6 | nd | 23.1 |
| Sens 2 | 100% | nd | 82% |
| Spec 2 | 3% | nd | 19% |
| Cutoff 3 | 14.6 | nd | 14.6 |
| Sens 3 | 100% | nd | 100% |
| Spec 3 | 3% | nd | 3% |
| Cutoff 4 | 219 | nd | 243 |
| Sens 4 | 50% | nd | 53% |
| Spec 4 | 71% | nd | 70% |
| Cutoff 5 | 40100 | nd | 40100 |
| Sens 5 | 0% | nd | 0% |
| Spec 5 | 100% | nd | 100% |
| Cutoff 6 | 40100 | nd | 40100 |
| Sens 6 | 0% | nd | 0% |
| Spec 6 | 100% | nd | 100% |
| OR Quart 2 | 0.12 | nd | 0.21 |
| p Value | 0.063 | nd | 0.18 |
| 95% CI of | 0.014 | nd | 0.021 |
| OR Quart2 | 1.1 | nd | 2.1 |
| OR Quart 3 | 1.2 | nd | 2.2 |
| p Value | 0.81 | nd | 0.29 |
| 95% CI of | 0.32 | nd | 0.52 |
| OR Quart3 | 4.2 | nd | 9.0 |
| OR Quart 4 | 0.94 | nd | 1.2 |
| p Value | 0.93 | nd | 0.77 |
| 95% CI of | 0.25 | nd | 0.28 |
| OR Quart4 | 3.5 | nd | 5.5 |

**Interleukin-17A**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 1.88 | 2.41 | 1.89 | 2.45 | 1.85 | 2.51 |
| Average | 3.71 | 4.94 | 4.05 | 2.70 | 3.52 | 5.47 |
| Stdev | 6.39 | 7.60 | 6.84 | 2.50 | 6.06 | 8.03 |
| p(t-test) |  | 0.31 |  | 0.56 |  | 0.12 |
| Min | 0.000362 | 0.000469 | 0.000362 | 0.000532 | 0.000362 | 0.000469 |
| Max | 53.4 | 28.8 | 53.4 | 6.61 | 53.4 | 28.8 |
| n (Samp) | 149 | 38 | 175 | 9 | 142 | 33 |
| n (Patient) | 149 | 38 | 175 | 9 | 142 | 33 |

|  | At Enrollment | | |
|---|---|---|---|
|  | sCr or UO | sCr only | UO only |
| AUC | 0.53 | 0.50 | 0.56 |
| SE | 0.053 | 0.099 | 0.057 |
| p | 0.55 | 0.97 | 0.27 |
| nCohort 1 | 149 | 175 | 142 |

|  | At Enrollment | | |
|---|---|---|---|
|  | sCr or UO | sCr only | UO only |
| nCohort 2 | 38 | 9 | 33 |
| Cutoff 1 | 0.787 | 0.520 | 0.787 |
| Sens 1 | 71% | 78% | 73% |
| Spec 1 | 30% | 23% | 32% |
| Cutoff 2 | 0.520 | 0.0656 | 0.520 |
| Sens 2 | 82% | 89% | 82% |
| Spec 2 | 24% | 18% | 26% |
| Cutoff 3 | 0.00436 | 0.000482 | 0.00436 |
| Sens 3 | 92% | 100% | 91% |
| Spec 3 | 15% | 7% | 16% |
| Cutoff 4 | 4.00 | 3.87 | 3.77 |
| Sens 4 | 29% | 33% | 36% |
| Spec 4 | 71% | 70% | 70% |
| Cutoff 5 | 5.11 | 5.11 | 5.11 |
| Sens 5 | 26% | 22% | 30% |
| Spec 5 | 81% | 80% | 81% |
| Cutoff 6 | 8.85 | 9.07 | 8.84 |
| Sens 6 | 13% | 0% | 15% |
| Spec 6 | 91% | 90% | 90% |
| OR Quart 2 | 0.84 | 0.65 | 1.4 |
| p Value | 0.75 | 0.65 | 0.59 |
| 95% CI of | 0.29 | 0.10 | 0.43 |
| OR Quart2 | 2.4 | 4.1 | 4.3 |
| OR Quart 3 | 1.1 | 0.32 | 1.4 |
| p Value | 0.84 | 0.33 | 0.59 |
| 95% CI of | 0.40 | 0.032 | 0.43 |
| OR Quart3 | 3.0 | 3.2 | 4.3 |
| OR Quart 4 | 1.3 | 1.0 | 2.1 |
| p Value | 0.65 | 1.0 | 0.20 |
| 95% CI of | 0.47 | 0.19 | 0.68 |
| OR Quart4 | 3.4 | 5.2 | 6.2 |

**Insulin receptor substrate 1**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.0314 | 0.0824 | nd | nd | 0.0314 | 0.0816 |
| Average | 0.643 | 0.263 | nd | nd | 0.662 | 0.267 |
| Stdev | 4.30 | 0.471 | nd | nd | 4.39 | 0.479 |
| p(t-test) |  | 0.64 | nd | nd |  | 0.64 |
| Min | 3.96E-5 | 0.000645 | nd | nd | 3.96E-5 | 0.000645 |
| Max | 49.5 | 2.31 | nd | nd | 49.5 | 2.31 |
| n (Samp) | 137 | 29 | nd | nd | 131 | 28 |
| n (Patient) | 137 | 29 | nd | nd | 131 | 28 |

|  | At Enrollment | | |
|---|---|---|---|
|  | sCr or UO | sCr only | UO only |
| AUC | 0.60 | nd | 0.60 |
| SE | 0.060 | nd | 0.061 |
| p | 0.091 | nd | 0.11 |
| nCohort 1 | 137 | nd | 131 |
| nCohort 2 | 29 | nd | 28 |
| Cutoff 1 | 0.0238 | nd | 0.0238 |

|  | At Enrollment | | |
|---|---|---|---|
|  | sCr or UO | sCr only | UO only |
| Sens 1 | 72% | nd | 71% |
| Spec 1 | 42% | nd | 42% |
| Cutoff 2 | 0.00696 | nd | 0.00696 |
| Sens 2 | 83% | nd | 82% |
| Spec 2 | 20% | nd | 19% |
| Cutoff 3 | 0.00372 | nd | 0.00372 |
| Sens 3 | 93% | nd | 93% |
| Spec 3 | 15% | nd | 15% |
| Cutoff 4 | 0.0827 | nd | 0.0810 |
| Sens 4 | 48% | nd | 50% |
| Spec 4 | 70% | nd | 70% |
| Cutoff 5 | 0.165 | nd | 0.147 |
| Sens 5 | 41% | nd | 43% |
| Spec 5 | 80% | nd | 80% |
| Cutoff 6 | 0.614 | nd | 0.503 |
| Sens 6 | 10% | nd | 11% |
| Spec 6 | 91% | nd | 90% |
| OR Quart 2 | 0.79 | nd | 0.79 |
| p Value | 0.71 | nd | 0.71 |
| 95% CI of | 0.22 | nd | 0.22 |
| OR Quart2 | 2.8 | nd | 2.8 |
| OR Quart 3 | 0.81 | nd | 0.79 |
| p Value | 0.75 | nd | 0.71 |
| 95% CI of | 0.23 | nd | 0.22 |
| OR Quart3 | 2.9 | nd | 2.8 |
| OR Quart 4 | 2.6 | nd | 2.4 |
| p Value | 0.083 | nd | 0.13 |
| 95% CI of | 0.88 | nd | 0.78 |
| OR Quart4 | 7.7 | nd | 7.1 |

**Involucrin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.749 | 1.11 | nd | nd | 0.732 | 1.18 |
| Average | 1.02 | 2.10 | nd | nd | 1.02 | 2.15 |
| Stdev | 0.839 | 2.59 | nd | nd | 0.847 | 2.62 |
| p(t-test) |  | 8.7E-5 | nd | nd |  | 7.5E-5 |
| Min | 0.0472 | 0.339 | nd | nd | 0.0472 | 0.339 |
| Max | 5.41 | 12.7 | nd | nd | 5.41 | 12.7 |
| n (Samp) | 137 | 29 | nd | nd | 131 | 28 |
| n (Patient) | 137 | 29 | nd | nd | 131 | 28 |

|  | At Enrollment | | |
|---|---|---|---|
|  | sCr or UO | sCr only | UO only |
| AUC | 0.67 | nd | 0.68 |
| SE | 0.059 | nd | 0.060 |
| p | 0.0038 | nd | 0.0026 |
| nCohort 1 | 137 | nd | 131 |
| nCohort 2 | 29 | nd | 28 |
| Cutoff 1 | 0.767 | nd | 0.805 |
| Sens 1 | 72% | nd | 71% |
| Spec 1 | 53% | nd | 56% |

| | At Enrollment | | |
| --- | --- | --- | --- |
| | sCr or UO | sCr only | UO only |
| Cutoff 2 | 0.658 | nd | 0.658 |
| Sens 2 | 83% | nd | 82% |
| Spec 2 | 41% | nd | 41% |
| Cutoff 3 | 0.477 | nd | 0.477 |
| Sens 3 | 93% | nd | 93% |
| Spec 3 | 26% | nd | 25% |
| Cutoff 4 | 1.07 | nd | 1.05 |
| Sens 4 | 52% | nd | 54% |
| Spec 4 | 70% | nd | 70% |
| Cutoff 5 | 1.57 | nd | 1.44 |
| Sens 5 | 31% | nd | 39% |
| Spec 5 | 80% | nd | 80% |
| Cutoff 6 | 2.29 | nd | 2.29 |
| Sens 6 | 31% | nd | 32% |
| Spec 6 | 91% | nd | 90% |
| OR Quart 2 | 2.1 | nd | 1.7 |
| p Value | 0.32 | nd | 0.48 |
| 95% CI of | 0.49 | nd | 0.38 |
| OR Quart2 | 9.1 | nd | 7.7 |
| OR Quart 3 | 2.6 | nd | 2.5 |
| p Value | 0.19 | nd | 0.20 |
| 95% CI of | 0.62 | nd | 0.61 |
| OR Quart3 | 11 | nd | 11 |
| OR Quart 4 | 5.7 | nd | 5.8 |
| p Value | 0.011 | nd | 0.011 |
| 95% CI of | 1.5 | nd | 1.5 |
| OR Quart4 | 22 | nd | 22 |

**NF-kappa-B inhibitor alpha**

| | sCr or UO | | sCr only | | UO only | |
| --- | --- | --- | --- | --- | --- | --- |
| | Cohort 1 | Cohort 2 | Cohort 1 | Cohort 2 | Cohort 1 | Cohort 2 |
| Median | 0.00122 | 0.00279 | nd | nd | 0.00140 | 0.00374 |
| Average | 0.0448 | 0.0324 | nd | nd | 0.0459 | 0.0335 |
| Stdev | 0.317 | 0.0783 | nd | nd | 0.324 | 0.0795 |
| p(t-test) | | 0.84 | nd | nd | | 0.84 |
| Min | 1.84E-7 | 1.84E-7 | nd | nd | 1.84E-7 | 1.84E-7 |
| Max | 3.62 | 0.400 | nd | nd | 3.62 | 0.400 |
| n (Samp) | 137 | 29 | nd | nd | 131 | 28 |
| n (Patient) | 137 | 29 | nd | nd | 131 | 28 |

| | At Enrollment | | |
| --- | --- | --- | --- |
| | sCr or UO | sCr only | UO only |
| AUC | 0.60 | nd | 0.60 |
| SE | 0.060 | nd | 0.061 |
| p | 0.098 | nd | 0.10 |
| nCohort 1 | 137 | nd | 131 |
| nCohort 2 | 29 | nd | 28 |
| Cutoff 1 | 0.000457 | nd | 0.000457 |
| Sens 1 | 72% | nd | 71% |
| Spec 1 | 42% | nd | 39% |
| Cutoff 2 | 3.31E-5 | nd | 3.31E-5 |
| Sens 2 | 83% | nd | 82% |

|  | At Enrollment |  |  |
|---|---|---|---|
|  | sCr or UO | sCr only | UO only |
| Spec 2 | 18% | nd | 17% |
| Cutoff 3 | 3.20E-6 | nd | 3.20E-6 |
| Sens 3 | 93% | nd | 93% |
| Spec 3 | 6% | nd | 6% |
| Cutoff 4 | 0.00393 | nd | 0.00402 |
| Sens 4 | 48% | nd | 50% |
| Spec 4 | 70% | nd | 70% |
| Cutoff 5 | 0.00743 | nd | 0.00743 |
| Sens 5 | 38% | nd | 39% |
| Spec 5 | 80% | nd | 80% |
| Cutoff 6 | 0.0360 | nd | 0.0266 |
| Sens 6 | 24% | nd | 29% |
| Spec 6 | 91% | nd | 90% |
| OR Quart 2 | 0.66 | nd | 0.51 |
| p Value | 0.51 | nd | 0.31 |
| 95% CI of | 0.19 | nd | 0.14 |
| OR Quart2 | 2.3 | nd | 1.9 |
| OR Quart 3 | 0.53 | nd | 0.51 |
| p Value | 0.34 | nd | 0.31 |
| 95% CI of | 0.14 | nd | 0.14 |
| OR Quart3 | 2.0 | nd | 1.9 |
| OR Quart 4 | 2.2 | nd | 2.2 |
| p Value | 0.14 | nd | 0.14 |
| 95% CI of | 0.77 | nd | 0.77 |
| OR Quart4 | 6.2 | nd | 6.3 |

**Beta-nerve growth factor**

|  | sCr or UO |  | sCr only |  | UO only |  |
|---|---|---|---|---|---|---|
|  | Cohort 1 | Cohort 2 | Cohort 1 | Cohort 2 | Cohort 1 | Cohort 2 |
| Median | 1.53 | 2.07 | nd | nd | 1.53 | 2.00 |
| Average | 2.43 | 2.39 | nd | nd | 2.48 | 2.36 |
| Stdev | 5.88 | 1.37 | nd | nd | 6.01 | 1.39 |
| p(t-test) |  | 0.97 | nd | nd |  | 0.92 |
| Min | 0.221 | 0.983 | nd | nd | 0.221 | 0.983 |
| Max | 70.0 | 6.73 | nd | nd | 70.0 | 6.73 |
| n (Samp) | 141 | 30 | nd | nd | 135 | 29 |
| n (Patient) | 141 | 30 | nd | nd | 135 | 29 |

|  | At Enrollment |  |  |
|---|---|---|---|
|  | sCr or UO | sCr only | UO only |
| AUC | 0.63 | nd | 0.62 |
| SE | 0.059 | nd | 0.060 |
| p | 0.027 | nd | 0.055 |
| nCohort 1 | 141 | nd | 135 |
| nCohort 2 | 30 | nd | 29 |
| Cutoff 1 | 1.77 | nd | 1.63 |
| Sens 1 | 70% | nd | 72% |
| Spec 1 | 61% | nd | 55% |
| Cutoff 2 | 1.30 | nd | 1.30 |
| Sens 2 | 80% | nd | 83% |
| Spec 2 | 38% | nd | 35% |
| Cutoff 3 | 1.08 | nd | 1.07 |

|  | At Enrollment | | |
|---|---|---|---|
|  | sCr or UO | sCr only | UO only |
| Sens 3 | 90% | nd | 93% |
| Spec 3 | 22% | nd | 21% |
| Cutoff 4 | 2.07 | nd | 2.22 |
| Sens 4 | 50% | nd | 38% |
| Spec 4 | 70% | nd | 70% |
| Cutoff 5 | 2.76 | nd | 2.89 |
| Sens 5 | 30% | nd | 17% |
| Spec 5 | 80% | nd | 80% |
| Cutoff 6 | 3.97 | nd | 3.97 |
| Sens 6 | 10% | nd | 10% |
| Spec 6 | 91% | nd | 90% |
| OR Quart 2 | 0.97 | nd | 1.0 |
| p Value | 0.97 | nd | 1.0 |
| 95% CI of | 0.23 | nd | 0.23 |
| OR Quart2 | 4.2 | nd | 4.3 |
| OR Quart 3 | 4.1 | nd | 4.3 |
| p Value | 0.023 | nd | 0.020 |
| 95% CI of | 1.2 | nd | 1.3 |
| OR Quart3 | 14 | nd | 15 |
| OR Quart 4 | 2.5 | nd | 2.2 |
| p Value | 0.15 | nd | 0.22 |
| 95% CI of | 0.71 | nd | 0.62 |
| OR Quart4 | 8.9 | nd | 8.1 |

**Transthyretin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 62800 | 58200 | nd | nd | 61700 | 61300 |
| Average | 64800 | 70100 | nd | nd | 64700 | 70900 |
| Stdev | 33600 | 38500 | nd | nd | 33000 | 39000 |
| p(t-test) |  | 0.45 | nd | nd |  | 0.38 |
| Min | 12700 | 10600 | nd | nd | 12700 | 10600 |
| Max | 165000 | 156000 | nd | nd | 165000 | 156000 |
| n (Samp) | 137 | 29 | nd | nd | 131 | 28 |
| n (Patient) | 137 | 29 | nd | nd | 131 | 28 |

|  | At Enrollment | | |
|---|---|---|---|
|  | sCr or UO | sCr only | UO only |
| AUC | 0.53 | nd | 0.54 |
| SE | 0.060 | nd | 0.061 |
| p | 0.61 | nd | 0.56 |
| nCohort 1 | 137 | nd | 131 |
| nCohort 2 | 29 | nd | 28 |
| Cutoff 1 | 48500 | nd | 48500 |
| Sens 1 | 72% | nd | 71% |
| Spec 1 | 39% | nd | 38% |
| Cutoff 2 | 35400 | nd | 35400 |
| Sens 2 | 83% | nd | 82% |
| Spec 2 | 21% | nd | 20% |
| Cutoff 3 | 25200 | nd | 24600 |
| Sens 3 | 93% | nd | 93% |
| Spec 3 | 8% | nd | 6% |

| | At Enrollment | | |
|---|---|---|---|
| | sCr or UO | sCr only | UO only |
| Cutoff 4 | 76200 | nd | 75900 |
| Sens 4 | 34% | nd | 36% |
| Spec 4 | 70% | nd | 70% |
| Cutoff 5 | 84400 | nd | 83800 |
| Sens 5 | 24% | nd | 25% |
| Spec 5 | 80% | nd | 80% |
| Cutoff 6 | 109000 | nd | 106000 |
| Sens 6 | 17% | nd | 18% |
| Spec 6 | 91% | nd | 90% |
| OR Quart 2 | 1.6 | nd | 1.4 |
| p Value | 0.42 | nd | 0.59 |
| 95% CI of | 0.51 | nd | 0.43 |
| OR Quart2 | 5.0 | nd | 4.4 |
| OR Quart 3 | 0.81 | nd | 0.79 |
| p Value | 0.75 | nd | 0.71 |
| 95% CI of | 0.23 | nd | 0.22 |
| OR Quart3 | 2.9 | nd | 2.8 |
| OR Quart 4 | 1.6 | nd | 1.6 |
| p Value | 0.42 | nd | 0.42 |
| 95% CI of | 0.51 | nd | 0.51 |
| OR Quart4 | 5.0 | nd | 5.0 |

While the invention has been described and exemplified in sufficient detail for those skilled in this art to make and use it, various alternatives, modifications, and improvements should be apparent without departing from the spirit and scope of the invention. The examples provided herein are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

It will be readily apparent to a person skilled in the art that varying substitutions and modifications may be made to the invention disclosed herein without departing from the scope and spirit of the invention.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

The present invention is further characterized by the following items:

1. A method for evaluating renal status in a subject, comprising:

performing one or more assaysconfigured to detect one or more biomarkers selected from the group consisting of Beta-nerve growth factor, Interleukin-17A, Follitropin subunit beta, Collagenase 3, Follistatin, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha on a body fluid sample obtained from the subject to provide an assay result; and

correlating the assay result(s) to the renal status of the subject.

2. A method according to item 1, wherein said correlation step comprises correlating the assay result(s) to one or more of risk stratification, diagnosis, staging, prognosis, classifying and monitoring of the renal status of the subject.

3. A method according to item 1, wherein said correlating step comprises assigning a likelihood of one or more future changes in renal status to the subject based on the assay result(s).

4. A method according to item 3, wherein said one or more future changes in renal status comprise one or more of a future injury to renal function, future reduced renal function, future improvement in renal function, and future acute renal failure (ARF).

5. A method according to one of items 1-4, wherein said assay results comprise at least 2, 3, or 4 of:

a measured urine or plasma concentration of Beta-nerve growth factor,
a measured urine or plasma concentration of Interleukin-17A,
a measured urine or plasma concentration of Follitropin subunit beta,
a measured urine or plasma concentration of Collagenase 3,
a measured urine or plasma concentration of Follistatin,
a measured urine or plasma concentration of Vitamin D Binding Protein,
a measured urine or plasma concentration of Islet amyloid polypeptide,
a measured urine or plasma concentration of Insulin C-peptide,
a measured urine or plasma concentration of Complement Factor H,
a measured urine or plasma concentration of Gastric inhibitory polypeptide,
a measured urine or plasma concentration of Glucagon-like peptide 1,
a measured urine or plasma concentration of Glucagon,
a measured urine or plasma concentration of Involucrin,
a measured urine or plasma concentration of type II cytoskeletal Keratin-1/10,
a measured urine or plasma concentration of type II cytoskeletal 6A/6B/6C,
a measured urine or plasma concentration of Osteocalcin,
a measured urine or plasma concentration of Lipopolysaccharide,
a measured urine or plasma concentration of Pancreatic prohormone,
a measured urine or plasma concentration of Peptide YY,
a measured urine or plasma concentration of Agouti-related protein,
a measured urine or plasma concentration of Ciliary neurotrophic factor,
a measured urine or plasma concentration of Appetite-regulating hormone,
a measured urine or plasma concentration of Transthyretin,
a measured urine or plasma concentration of Insulin receptor substrate 1, and a measured urine or plasma concentration of and NF-kappa-B inhibitor alpha.

6. A method according to one of items 1-5, wherein a plurality of assay results are combined using a function that converts the plurality of assay results into a single composite result.

7. A method according to item 3, wherein said one or more future changes in renal status comprise a clinical outcome related to a renal injury suffered by the subject.

8. A method according to item 3, wherein the likelihood of one or more future changes in renal status is that an event of interest is more or less likely to occur within 30 days of the time at which the body fluid sample is obtained from the subject.

9. A method according to item 8, wherein the likelihood of one or more future changes in renal status is that an event of interest is more or less likely to occur within a period selected from the group consisting of 21 days, 14 days, 7 days, 5 days, 96 hours, 72 hours, 48 hours, 36 hours, 24 hours, and 12 hours.

10. A method according to one of items 1-5, wherein the subject is selected for evaluation of renal status based on the pre-existence in the subject of one or more known risk factors for prerenal, intrinsic renal, or postrenal ARF.

11. A method according to one of items 1-5, wherein the subject is selected for evaluation of renal status based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

12. A method according to one of items 1-5, wherein said correlating step comprises assigning a diagnosis of the occurrence or nonoccurrence of one or more of an injury to renal function, reduced renal function, or ARF to the subject based on the assay result(s).

13. A method according to one of items 1-5, wherein said correlating step comprises assessing whether or not renal function is improving or worsening in a subject who has suffered from an injury to renal function, reduced renal function, or ARF based on the assay result(s).

14. A method according to one of items 1-5, wherein said method is a method of diagnosing the occurrence or nonoccurrence of an injury to renal function in said subject.

15. A method according to one of items 1-5, wherein said method is a method of diagnosing the occurrence or nonoccurrence of reduced renal function in said subject.

16. A method according to one of items 1-5, wherein said method is a method of diagnosing the occurrence or nonoccurrence of acute renal failure in said subject.

17. A method according to one of items 1-5, wherein said method is a method of diagnosing the occurrence or nonoccurrence of a need for renal replacement therapy in said subject.

18. A method according to one of items 1-5, wherein said method is a method of diagnosing the occurrence or nonoccurrence of a need for renal transplantation in said subject.

19. A method according to one of items 1-5, wherein said method is a method of assigning a risk of the future occurrence or nonoccurrence of an injury to renal function in said subject.

20. A method according to one of items 1-5, wherein said method is a method of assigning a risk of the future occurrence or nonoccurrence of reduced renal function in said subject.

21. A method according to one of items 1-5, wherein said method is a method of assigning a risk of the future occurrence or nonoccurrence of acute renal failure in said subject.

22. A method according to one of items 1-5, wherein said method is a method of assigning a risk of the future occurrence or nonoccurrence of a need for renal replacement therapy in said subject.

23. A method according to one of items 1-5, wherein said method is a method of assigning a risk of the future occurrence or nonoccurrence of a need for renal transplantation in said subject.

24. A method according to one of items 1-5, wherein said one or more future changes in renal status comprise one or more of a future injury to renal function, future reduced renal function, future improvement in renal function, and future acute renal failure (ARF) within 72 hours of the time at which the body fluid sample is obtained.

25. A method according to one of items 1-5, wherein said one or more future changes in renal status comprise one or more of a future injury to renal function, future reduced renal function, future improvement in renal function, and future acute renal failure (ARF) within 48 hours of the time at which the body fluid sample is obtained.

26. A method according to one of items 1-5, wherein said one or more future changes in renal status comprise one or more of a future injury to renal function, future reduced renal function, future improvement in renal function, and future acute renal failure (ARF) within 24 hours of the time at which the body fluid sample is obtained.

27. A method according to one of items 1-5, wherein the subject is in RIFLE stage 0 or R.

28. A method according to item 27, wherein the subject is in RIFLE stage 0, and said correlating step comprises assigning a likelihood that the subject will reach RIFLE stage R, I or F within 72 hours.

29. A method according to item 28, wherein the subject is in RIFLE stage 0, and said correlating step comprises assigning a likelihood that the subject will reach RIFLE stage I or F within 72 hours.

30. A method according to item 28, wherein the subject is in RIFLE stage 0, and said correlating step comprises assigning a likelihood that the subject will reach RIFLE stage F within 72 hours.

31. A method according to item 27, wherein the subject is in RIFLE stage 0 or R, and said correlating step comprises assigning a likelihood that the subject will reach RIFLE stage I or F within 72 hours.

32. A method according to item 31, wherein the subject is in RIFLE stage 0 or R, and said correlating step comprises assigning a likelihood that the subject will reach RIFLE stage F within 72 hours.

33. A method according to item 27, wherein the subject is in RIFLE stage R, and said correlating step comprises assigning a likelihood that the subject will reach RIFLE stage I or F within 72 hours.

34. A method according to item 33, wherein the subject is in RIFLE stage R, and said correlating step comprises assigning a likelihood that the subject will reach RIFLE stage F within 72 hours.

35. A method according to one of items 1-5, wherein the subject is in RIFLE stage 0, R, or I, and said correlating step comprises assigning a likelihood that the subject will reach RIFLE stage F within 72 hours.

36. A method according to item 35, wherein the subject is in RIFLE stage I, and said correlating step comprises assigning a likelihood that the subject will reach RIFLE stage F within 72 hours.

37. A method according to item 28, wherein said correlating step comprises assigning a likelihood that the subject will reach RIFLE stage R, I or F within 48 hours.

38. A method according to item 29, wherein said correlating step comprises assigning a likelihood that the subject will reach RIFLE stage I or F within 48 hours.

39. A method according to item 30, wherein said correlating step comprises assigning a likelihood that the subject will reach RIFLE stage F within 48 hours.

40. A method according to item 31, wherein said correlating step comprises assigning a likelihood that the subject will reach RIFLE stage I or F within 48 hours.

41. A method according to item 32, wherein said correlating step comprises assigning a likelihood that the subject will reach RIFLE stage F within 48 hours.

42. A method according to item 33, wherein said correlating step comprises assigning a likelihood that the subject will reach RIFLE stage I or F within 48 hours.

43. A method according to item 34, wherein said correlating step comprises assigning a likelihood that the subject will reach RIFLE stage F within 48 hours.

44. A method according to item 35, wherein said correlating step comprises assigning a likelihood that the subject will reach RIFLE stage F within 48 hours.

45. A method according to item 36, wherein said correlating step comprises assigning a likelihood that the subject will reach RIFLE stage F within 48 hours.

46. A method according to item 28, wherein said correlating step comprises assigning a likelihood that the subject will reach RIFLE stage R, I or F within 24 hours.

47. A method according to item 29, wherein said correlating step comprises assigning a likelihood that the subject will reach RIFLE stage I or F within 24 hours.

48. A method according to item 30, wherein said correlating step comprises assigning a likelihood that the subject will reach RIFLE stage F within 24 hours.

49. A method according to item 31, wherein said correlating step comprises assigning a likelihood that the subject will reach RIFLE stage I or F within 24 hours.

50. A method according to item 32, wherein said correlating step comprises assigning a likelihood that the subject will reach RIFLE stage F within 24 hours.

51. A method according to item 33, wherein said correlating step comprises assigning a likelihood that the subject will reach RIFLE stage I or F within 24 hours.

52. A method according to item 34, wherein said correlating step comprises assigning a likelihood that the subject will reach RIFLE stage F within 24 hours.

53. A method according to item 35, wherein said correlating step comprises assigning a likelihood that the subject will reach RIFLE stage F within 24 hours.

54. A method according to item 36, wherein said correlating step comprises assigning a likelihood that the subject will reach RIFLE stage F within 24 hours.

55. A method according to one of items 1-5, wherein the subject is not in acute renal failure.

56. A method according to one of items 1-5, wherein the subject has not experienced a 1.5-fold or greater increase in serum creatinine over a baseline value determined prior to the time at which the body fluid sample is obtained.

57. A method according to one of items 1-5, wherein the subject has a urine output of at least 0.5 ml/kg/hr over the 6 hours preceding the time at which the body fluid sample is obtained.

58. A method according to one of items 1-5, wherein the subject has not experienced an increase of 0.3 mg/dL or greater in serum creatinine over a baseline value determined prior to the time at which the body fluid sample is obtained.

59. A method according to one of items 1-5, wherein the subject (i) has not experienced a 1.5-fold or greater increase in serum creatinine over a baseline value determined prior to the time at which the body fluid sample is obtained, (ii) has a urine output of at least 0.5 ml/kg/hr over the 6 hours preceding the time at which the body fluid sample is obtained, and (iii) has not experienced an increase of 0.3 mg/dL or greater in serum creatinine over a baseline value determined prior to the time at which the body fluid sample is obtained.

60. A method according to one of items 1-5, wherein the subject has not experienced a 1.5-fold or greater increase in serum creatinine over a baseline value determined prior to the time at which the body fluid sample is obtained.

61. A method according to one of items 1-5, wherein the subject has a urine output of at least 0.5 ml/kg/hr over the 6 hours preceding the time at which the body fluid sample is obtained.

62. A method according to one of items 1-5, wherein the subject (i) has not experienced a 1.5-fold or greater increase in serum creatinine over a baseline value determined prior to the time at which the body fluid sample is obtained, (ii) has a urine output of at least 0.5 ml/kg/hr over the 12 hours preceding the time at which the body fluid sample is obtained, and (iii) has not experienced an increase of 0.3 mg/dL or greater in serum creatinine over a baseline value determined prior to the time at which the body fluid sample is obtained.

63. A method according to one of items 1-5, wherein said correlating step comprises assigning one or more of: a likelihood that within 72 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.

64. A method according to item 63, wherein said correlating step comprises assigning one or more of: a likelihood that within 48 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.

65. A method according to item 63, wherein said correlating step comprises assigning one or more of: a likelihood that within 24 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.

66. A method according to item 63, wherein said correlating step comprises assigning a likelihood that within 72 hours the subject will experience a 1.5-fold or greater increase in serum creatinine.

67. A method according to item 63, wherein said correlating step comprises assigning a likelihood that within 72 hours the subject will have a urine output of less than 0.5 ml/kg/hr over a 6 hour period.

68. A method according to item 63, wherein said correlating step comprises assigning a likelihood that within 72 hours the subject will experience an increase of 0.3 mg/dL or greater in serum creatinine.

69. A method according to item 63, wherein said correlating step comprises assigning a likelihood that within 48 hours the subject will experience a 1.5-fold or greater increase in serum creatinine.

70. A method according to item 63, wherein said correlating step comprises assigning a likelihood that within 48 hours the subject will have a urine output of less than 0.5 ml/kg/hr over a 6 hour period.

71. A method according to item 63, wherein said correlating step comprises assigning a likelihood that within 48 hours the subject will experience an increase of 0.3 mg/dL or greater in serum creatinine.

72. A method according to item 63, wherein said correlating step comprises assigning a likelihood that within 24 hours the subject will experience a 1.5-fold or greater increase in serum creatinine.

73. A method according to item 63, wherein said correlating step comprises assigning a likelihood that within 24 hours the subject will have a urine output of less than 0.5 ml/kg/hr over a 6 hour period.

74. A method according to item 63, wherein said correlating step comprises assigning a likelihood that within 24 hours the subject will experience an increase of 0.3 mg/dL or greater in serum creatinine.

75. A method according to one of items 1-5, wherein the subject has not experienced a 2-fold or greater increase in serum creatinine over a baseline value determined prior to the time at which the body fluid sample is obtained.

76. A method according to one of items 1-5, wherein the subject has a urine output of at least 0.5 ml/kg/hr over the 12 hours preceding the time at which the body fluid sample is obtained.

77. A method according to one of items 1-5, wherein the subject (i) has not experienced a 2-fold or greater increase in serum creatinine over a baseline value determined prior to the time at which the body fluid sample is obtained, (ii) has a urine output of at least 0.5 ml/kg/hr over the 2 hours preceding the time at which the body fluid sample is obtained, and (iii) has not experienced an increase of 0.3 mg/dL or greater in serum creatinine over a baseline value determined prior to the time at which the body fluid sample is obtained.

78. A method according to one of items 1-5, wherein the subject has not experienced a 3-fold or greater increase in serum creatinine over a baseline value determined prior to the time at which the body fluid sample is obtained.

79. A method according to one of items 1-5, wherein the subject has a urine output of at least 0.3 ml/kg/hr over the 24 hours preceding the time at which the body fluid sample is obtained, or anuria over the 12 hours preceding the time at which the body fluid sample is obtained.

80. A method according to one of items 1-5, wherein the subject (i) has not experienced a 3-fold or greater increase in serum creatinine over a baseline value determined prior to the time at which the body fluid sample is obtained, (ii) has a urine output of at least 0.3 ml/kg/hr over the 24 hours preceding the time at which the body fluid sample is obtained, or anuria over the 12 hours preceding the time at which the body fluid sample is obtained, and (iii) has not experienced an increase of 0.3 mg/dL or greater in serum creatinine over a baseline value determined prior to the time at which the body fluid sample is obtained.

81. A method according to one of items 1-5, wherein said correlating step comprises assigning one or more of: a likelihood that within 72 hours the subject will (i) experience a 2-fold or greater increase in serum creatinine (ii) have a urine output of less than 0.5 ml/kg/hr over a 12 hour period, or (iii) experience an increase of 0.3 mg/dL or greater in serum creatinine.

82. A method according to item 81, wherein said correlating step comprises assigning one or more of: a likelihood that within 48 hours the subject will (i) experience a 2-fold or greater increase in serum creatinine (ii) have a urine output of less than 0.5 ml/kg/hr over a 6 hour period, or (iii) experience an increase of 0.3 mg/dL or greater in serum creatinine.

83. A method according to item 81, wherein said correlating step comprises assigning one or more of: a likelihood that within 24 hours the subject will (i) experience a 2-fold or greater increase in serum creatinine, or (ii) have a urine output of less than 0.5 ml/kg/hr over a 6 hour period.

84. A method according to item 81, wherein said correlating step comprises assigning a likelihood that within 72 hours the subject will experience a 2-fold or greater increase in serum creatinine.

85. A method according to item 81, wherein said correlating step comprises assigning a likelihood that within 72 hours the subject will have a urine output of less than 0.5 ml/kg/hr over a 6 hour period.

86. A method according to item 81, wherein said correlating step comprises assigning a likelihood that within 48 hours the subject will experience a 2-fold or greater increase in serum creatinine.

87. A method according to item 81, wherein said correlating step comprises assigning a likelihood that within 48 hours the subject will have a urine output of less than 0.5 ml/kg/hr over a 6 hour period.

88. A method according to item 81, wherein said correlating step comprises assigning a likelihood that within 24 hours the subject will experience a 2-fold or greater increase in serum creatinine.

89. A method according to item 81, wherein said correlating step comprises assigning a likelihood that within 24 hours the subject will have a urine output of less than 0.5 ml/kg/hr over a 6 hour period.

90. A method according to one of items 1-5, wherein said correlating step comprises assigning one or more of: a likelihood that within 72 hours the subject will (i) experience a 3-fold or greater increase in serum creatinine, or (ii) have a urine output of less than 0.3 ml/kg/hr over a 24 hour period or anuria over a 12 hour period.

91. A method according to item 90, wherein said correlating step comprises assigning one or more of: a likelihood that within 48 hours the subject will (i) experience a 3-fold or greater increase in serum creatinine, or (ii) have a urine output of less than 0.3 ml/kg/hr over a 24 hour period or anuria over a 12 hour period.

92. A method according to item 90, wherein said correlating step comprises assigning one or more of: a likelihood that within 24 hours the subject will (i) experience a 3-fold or greater increase in serum creatinine, or (ii) have a urine output of less than 0.3 ml/kg/hr over a 24 hour period or anuria over a 12 hour period.

93. A method according to item 90, wherein said correlating step comprises assigning a likelihood that within 72 hours the subject will experience a 3-fold or greater increase in serum creatinine.

94. A method according to item 90, wherein said correlating step comprises assigning a likelihood that within 72 hours the subject will have a urine output of less than 0.3 ml/kg/hr over a 24 hour period or anuria over a 12 hour period.

95. A method according to item 90, wherein said correlating step comprises assigning a likelihood that within 48 hours the subject will experience a 3-fold or greater increase in serum creatinine.

96. A method according to item 90, wherein said correlating step comprises assigning a likelihood that within 48 hours the subject will have a urine output of less than 0.3 ml/kg/hr over a 24 hour period or anuria over a 12 hour period.

97. A method according to item 90, wherein said correlating step comprises assigning a likelihood that within 24 hours the subject will experience a 3-fold or greater increase in serum creatinine.

98. A method according to item 90, wherein said correlating step comprises assigning a likelihood that within 24 hours the subject will have a urine output of less than 0.3 ml/kg/hr over a 24 hour period or anuria over a 12 hour period.

99. A method according to one of items 1-98, wherein the body fluid sample is a urine sample.

100. A method according to one of items 1-99, wherein said method comprises performing assays that detect one, two or three, or more of Beta-nerve growth factor, Interleukin-17A, Follitropin subunit beta, Collagenase 3, Follistatin, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha.

101. Measurement of one or more biomarkers selected from the group consisting of Beta-nerve growth factor, Interleukin-17A, Follitropin subunit beta, Collagenase 3, Follistatin, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha for the evaluation of renal injury.

102. Measurement of one or more biomarkers selected from the group consisting of Beta-nerve growth factor, Interleukin-17A, Follitropin subunit beta, Collagenase 3, Follistatin, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha for the evaluation of acute renal injury.

103. A kit, comprising:

reagents for performing one or more assays configured to detect one or more kidney injury markers selected from the group consisting of Beta-nerve growth factor, Interleukin-17A, Follitropin subunit beta, Collagenase 3, Follistatin, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha.

104. A kit according to item 103, wherein said reagents comprise one or more binding reagents, each of which specifically binds one of said of kidney injury markers.

105. A kit according to item 104, wherein a plurality of binding reagents are contained in a single assay device.

106. A kit according to item 103, wherein at least one of said assays is configured as a sandwich binding assay.

107. A kit according to item 103, wherein at least one of said assays is configured as a competitive binding assay.

108. A kit according to one of items 103-107, wherein said one or more assays comprise assays that detect one, two or three, or more of Beta-nerve growth factor, Interleukin-17A, Follitropin subunit beta, Collagenase 3, Follistatin, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha.

Other embodiments are set forth within the following claims.

## Claims

1. A method for evaluating renal status in a subject, comprising:

performing one or more assays configured to detect one or more biomarkers on a body fluid sample obtained from the subject to provide an assay result; and

correlating the assay result(s) to the renal status of the subject, wherein said correlating step comprises assigning a likelihood of a future change in renal status to the subject based on the assay result(s),

wherein said future change in renal status comprises future acute renal failure (ARF), and the likelihood of the future change in renal status is that an event of interest is more or less likely to occur within a period of 24 hours or less of the time at which the body fluid sample is obtained from the subject and

wherein the one or more biomarkers comprise Follistatin, and optionally further one or more of Beta-nerve growth factor, Interleukin-17A, Follitropin subunit beta, Collagenase 3, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha.

2. The method of claim 1, wherein said correlating step comprises

(a) assigning a diagnosis of the occurrence or nonoccurrence of one or more of an injury to renal function, reduced renal function, or ARF to the subject based on the assay result(s),

(b) assessing whether or not renal function is improving or worsening in a subject who has suffered from an injury to renal function, reduced renal function, or ARF based on the assay result(s) or

(c) assigning one or more of: a likelihood that within 24 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine or

(d) assigning one or more of: a likelihood that within 24 hours the subject will (i) experience a 2-fold or greater increase in serum creatinine (ii) have a urine output of less than 0.5 ml/kg/hr over a 12 hour period, or (iii) experience an increase of 0.3 mg/dL or greater in serum creatinine or

(e) assigning one or more of: a likelihood that within 24 hours the subject will (i) experience a 3-fold or greater increase in serum creatinine, or (ii) have a urine output of less than 0.3 ml/kg/hr over a 24 hour period or anuria over a 12 hour period.

3. The method of claims 1 or 2, wherein said assay results comprise a measured urine or plasma concentration of Follistatin, and optionally further at least 1, 2, 3, or 4 of:

a measured urine or plasma concentration of Beta-nerve growth factor,
a measured urine or plasma concentration of Interleukin-17A,
a measured urine or plasma concentration of Follitropin subunit beta,
a measured urine or plasma concentration of Collagenase 3,
a measured urine or plasma concentration of Vitamin D Binding Protein,
a measured urine or plasma concentration of Islet amyloid polypeptide,
a measured urine or plasma concentration of Insulin C-peptide,
a measured urine or plasma concentration of Complement Factor H,
a measured urine or plasma concentration of Gastric inhibitory polypeptide,
a measured urine or plasma concentration of Glucagon-like peptide 1,
a measured urine or plasma concentration of Glucagon,
a measured urine or plasma concentration of Involucrin,
a measured urine or plasma concentration of type II cytoskeletal Keratin-1/10,
a measured urine or plasma concentration of type II cytoskeletal 6A/6B/6C,
a measured urine or plasma concentration of Osteocalcin,
a measured urine or plasma concentration of Lipopolysaccharide,
a measured urine or plasma concentration of Pancreatic prohormone,
a measured urine or plasma concentration of Peptide YY,
a measured urine or plasma concentration of Agouti-related protein,
a measured urine or plasma concentration of Ciliary neurotrophic factor,
a measured urine or plasma concentration of Appetite-regulating hormone,
a measured urine or plasma concentration of Transthyretin,
a measured urine or plasma concentration of Insulin receptor substrate 1, and
a measured urine or plasma concentration of and NF-kappa-B inhibitor alpha.

**4.** A method according to one of claims 1-3, wherein a plurality of assay results are combined using a function that converts the plurality of assay results into a single composite result.

**5.** A method according to claim 1, wherein said future change in renal status comprises a clinical outcome related to a renal injury suffered by the subject.

**6.** A method according to one of claims 1-3, wherein the subject is selected for evaluation of renal status based on

(i) the pre-existence in the subject of one or more known risk factors for prerenal, intrinsic renal, or postrenal ARF or
(ii) an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

**7.** A method according to one of claims 1-3, wherein said method is a method of diagnosing the occurrence or nonoccurrence of acute renal failure in said subject, a method of diagnosing the occurrence or nonoccurrence of a need for renal replacement therapy in said subject, a method of diagnosing the occurrence or nonoccurrence of a need for renal transplantation in said subject, a method of assigning a risk of the future occurrence or nonoccurrence of a need for renal replacement therapy in said subject or a method of assigning a risk of the future occurrence or nonoccurrence of a need for renal transplantation in said subject.

**8.** A method according to one of claims 1-3, wherein said future change in renal status comprises future ARF within 12 hours of the time at which the body fluid sample is obtained.

**9.** A method according to one of claims 1-3, wherein

(A) the subject is in RIFLE stage 0 or R, wherein

(i) the subject optionally is in RIFLE stage 0, and said correlating step comprises assigning a likelihood that the subject will reach RIFLE stage R, I or F within 24 hours, wherein the subject is in RIFLE stage 0, and said correlating step comprises assigning a likelihood that the subject will reach RIFLE stage I or F within 24 hours or
wherein the subject optionally is in RIFLE stage 0, and said correlating step comprises assigning a likelihood that the subject will reach RIFLE stage F within 24 hours,
(ii) wherein the subject optionally is in RIFLE stage 0 or R, and said correlating step comprises assigning a likelihood that the subject will reach RIFLE stage I or F within 24 hours,
wherein the subject optionally is in RIFLE stage 0 or R, and said correlating step comprises assigning a likelihood that the subject will reach RIFLE stage F within 24 hours,
(iii) wherein the subject optionally is in RIFLE stage R, and said correlating step comprises assigning a likelihood that the subject will reach RIFLE stage I or F within 24 hours,
wherein the subject optionally is in RIFLE stage R, and said correlating step comprises assigning a likelihood that the subject will reach RIFLE stage F within 24 hours or

(B) wherein the subject is in RIFLE stage 0, R, or I, and said correlating step comprises assigning a likelihood that the subject will reach RIFLE stage F within 24 hours.

**10.** A method according to one of claims 1-4, wherein

(a) the subject is not in acute renal failure,
(b) the subject has not experienced a 1.5-fold or greater increase in serum creatinine over a baseline value determined prior to the time at which the body fluid sample is obtained,
(c) the subject has a urine output of at least 0.5 ml/kg/hr over the 6 hours preceding the time at which the body fluid sample is obtained,
(d) the subject has not experienced an increase of 0.3 mg/dL or greater in serum creatinine over a baseline value determined prior to the time at which the body fluid sample is obtained,

(e) the subject (i) has not experienced a 1.5-fold or greater increase in serum creatinine over a baseline value determined prior to the time at which the body fluid sample is obtained, (ii) has a urine output of at least 0.5 ml/kg/hr over the 6 hours preceding the time at which the body fluid sample is obtained, and (iii) has not experienced an increase of 0.3 mg/dL or greater in serum creatinine over a baseline value determined prior to the time at which the body fluid sample is obtained,

(f) the subject has not experienced a 1.5-fold or greater increase in serum creatinine over a baseline value determined prior to the time at which the body fluid sample is obtained,

(g) the subject has a urine output of at least 0.5 ml/kg/hr over the 6 hours preceding the time at which the body fluid sample is obtained,

(h) the subject (i) has not experienced a 1.5-fold or greater increase in serum creatinine over a baseline value determined prior to the time at which the body fluid sample is obtained, (ii) has a urine output of at least 0.5 ml/kg/hr over the 12 hours preceding the time at which the body fluid sample is obtained, and (iii) has not experienced an increase of 0.3 mg/dL or greater in serum creatinine over a baseline value determined prior to the time at which the body fluid sample is obtained,

(i) the subject has not experienced a 2-fold or greater increase in serum creatinine over a baseline value determined prior to the time at which the body fluid sample is obtained,

(j) the subject has a urine output of at least 0.5 ml/kg/hr over the 12 hours preceding the time at which the body fluid sample is obtained,

(k) the subject (i) has not experienced a 2-fold or greater increase in serum creatinine over a baseline value determined prior to the time at which the body fluid sample is obtained, (ii) has a urine output of at least 0.5 ml/kg/hr over the 2 hours preceding the time at which the body fluid sample is obtained, and (iii) has not experienced an increase of 0.3 mg/dL or greater in serum creatinine over a baseline value determined prior to the time at which the body fluid sample is obtained,

(l) the subject has not experienced a 3-fold or greater increase in serum creatinine over a baseline value determined prior to the time at which the body fluid sample is obtained,

(m) the subject has a urine output of at least 0.3 ml/kg/hr over the 24 hours preceding the time at which the body fluid sample is obtained, or anuria over the 12 hours preceding the time at which the body fluid sample is obtained or

(n) the subject (i) has not experienced a 3-fold or greater increase in serum creatinine over a baseline value determined prior to the time at which the body fluid sample is obtained, (ii) has a urine output of at least 0.3 ml/kg/hr over the 24 hours preceding the time at which the body fluid sample is obtained, or anuria over the 12 hours preceding the time at which the body fluid sample is obtained, and (iii) has not experienced an increase of 0.3 mg/dL or greater in serum creatinine over a baseline value determined prior to the time at which the body fluid sample is obtained.

**11.** A method according to one of claims 1-10, wherein the body fluid sample is a urine sample.

**12.** A method according to one of claims 1-11, wherein said method comprises performing assays that detect Follistatin, and optionally further one, two or three, or more of Beta-nerve growth factor, Interleukin-17A, Follitropin subunit beta, Collagenase 3, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha.

**13.** The use of Follistatin, and optionally further of one or more biomarkers of Beta-nerve growth factor, Interleukin-17A, Follitropin subunit beta, Collagenase 3, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha for the evaluation of acute renal failure.

**14.** A kit, comprising:

reagents for performing one or more assays configured to detect Follistatin, and optionally further one or more kidney injury markers of Beta-nerve growth factor, Interleukin-17A, Follitropin subunit beta, Collagenase 3, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha.

15. The kit of claim 14, wherein

(a) said reagents comprise one or more binding reagents, each of which specifically binds one of said of kidney injury markers,
wherein optionally a plurality of binding reagents are contained in a single assay device,
(b) at least one of said assays is configured as a sandwich binding assay, or
(c) at least one of said assays is configured as a competitive binding assay,

wherein said one or more assays optionally comprise assays that detect Follistatin, and optionally further one, two or three, or more of Beta-nerve growth factor, Interleukin-17A, Follitropin subunit beta, Collagenase 3, Vitamin D Binding Protein, Islet amyloid polypeptide, Insulin C-peptide, Complement Factor H, Gastric inhibitory polypeptide, Glucagon-like peptide 1, Glucagon, Involucrin, Type II cytoskeletal Keratin-1/Keratin-10, Type II cytoskeletal Keratin-6A/6B/6C, Osteocalcin, Lipopolysaccharide, Pancreatic prohormone, Peptide YY, Agouti-related protein, Ciliary neurotrophic factor, Appetite-regulating hormone, Transthyretin, Insulin receptor substrate 1, and NF-kappa-B inhibitor alpha.

Europäisches
Patentamt

European
Patent Office

Office européen
des brevets

## EUROPEAN SEARCH REPORT

Application Number

EP 17 17 5396

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SAKAMOTO Y ET AL: "DETERMINATION OF FREE FOLLISTATIN LEVELS IN SERA OF NORMAL SUBJECTSAND PATIENTS WITH VARIOUS DISEASES", EUROPEAN JOURNAL OF ENDOCRINOLOGY, BIOSCIENTIFICA LTD, GB, vol. 135, no. 3, 1 September 1996 (1996-09-01), pages 345-351, XP000987180, ISSN: 0804-4643 * abstract, page 346, 1left-hand column, last paragraph, right-hand column, 2nd paragraph, page 349, figure 5; * | 1-15 | INV. G01N33/53 |
| Y | US 2002/028762 A1 (KOJIMA ITARU [JP]) 7 March 2002 (2002-03-07) * paragraphs 10, 14, 16,38-39, 56, claims 1-6; * | 1-15 | |
| Y | AKITO MAESHIMA ET AL: "Activin A: Autocrine Regulator of Kidney Development and Repair", ENDOCRINE JOURNAL, vol. 55, no. 1, 1 January 2008 (2008-01-01), pages 1-9, XP055414474, JP ISSN: 0918-8959, DOI: 10.1507/endocrj.KR-113 * page 5, left-hand column paragraph 2 through right-hand column paragraph to * | 1-15 | |

TECHNICAL FIELDS
SEARCHED (IPC)

G01N

-/--

The present search report has been drawn up for all claims

| Place of search | Date of completion of the search | Examiner |
|---|---|---|
| Munich | 11 October 2017 | Pilch, Bartosz |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 17 17 5396

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | A Maeshima ET AL: "Involvement of the activin-follistatin system in tubular regeneration after renal ischemia in rats", Journal of the American Society of Nephrology : JASN, 1 August 2001 (2001-08-01), page 1685, XP055414517, United States Retrieved from the Internet: URL:http://jasn.asnjournals.org/content/12/8/1685.full.pdf#page=1&view=FitH * page 1694, left-hand column; * | 1-15 | |
| Y | DEREK C. ADAMS: "Follistatin-like 1 regulates renal IL-1[beta] expression in cisplatin nephrotoxicity", AM J PHYSIOL RENAL PHYSIOL., vol. 299, no. 6, 29 September 2010 (2010-09-29), pages F1320-F1327, XP055163044, * abstract; * | 1-15 | |

TECHNICAL FIELDS
SEARCHED    (IPC)

The present search report has been drawn up for all claims

| Place of search | Date of completion of the search | Examiner |
|---|---|---|
| Munich | 11 October 2017 | Pilch, Bartosz |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 17 17 5396

This annex lists the patent family members relating to the patent documents cited in the above-mentioned European search report.
The members are as contained in the European Patent Office EDP file on
The European Patent Office is in no way liable for these particulars which are merely given for the purpose of information.

11-10-2017

| Patent document cited in search report | Publication date | Patent family member(s) | Publication date |
|---|---|---|---|
| US 2002028762 A1 | 07-03-2002 | JP 4487376 B2<br>JP 2001288111 A<br>US 2002028762 A1 | 23-06-2010<br>16-10-2001<br>07-03-2002 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61390999 A
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

**Non-patent literature cited in the description**

- Harrison's Principles of Internal Medicine. McGraw Hill, 1741-1830
- Current Medical Diagnosis & Treatment. McGraw Hill, 2008, 785-815
- **PRAUGHT ; SHLIPAK.** *Curr Opin Nephrol Hypertens,* 2005, vol. 14, 265-270
- **CHERTOW et al.** *J Am Soc Nephrol,* 2005, vol. 16, 3365-3370
- **LASSNIGG.** *J Am Soc Nephrol,* 2004, vol. 15, 1597-1605
- **BELLOMO et al.** *Crit Care.,* 2004, vol. 8 (4), R204-12
- **KELLUM.** *Crit. Care Med.,* 2008, vol. 36, 141-45
- **RICCI et al.** *Kidney Int.,* 2008, vol. 73, 538-546
- **MEHTA et al.** *Crit. Care,* 2007, vol. 11, R31
- **MCCOLLOUGH et al.** *Rev Cardiovasc Med.,* 2006, vol. 7 (4), 177-197
- The Immunoassay Handbook. Stockton Press, 1994
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **NELSON ; GRISWOLD.** *Comput. Methods Programs Biomed,* 1988, vol. 27, 65-8
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **FISCHER et al.** *Intensive Care Med,* 2003, vol. 29, 1043-51
- **BAGSHAW et al.** *Nephrol. Dial. Transplant,* 2008, vol. 23, 1203-1210
- Merck Manual of Diagnosis and Therapy. Merck Research Laboratories, 1999
- **WIJEYSUNDERA et al.** *JAMA,* 2007, vol. 297, 1801-9
- **HANLEY, J. A. ; MCNEIL, B.J.** The meaning and use of the area under a receiver operating characteristic (ROC) curve. *Radiology,* 1982, vol. 143, 29-36